# EUROPEAN PATENT APPLICATION

(11) **EP 4 772 514 A1**
(43) Date of publication of application: **08.07.2026**
(21) Application number: 24858762.8
(22) Date of filing: 30.08.2024
(51) Int. Cl.: C07D 498/18, C07D 519/00, A61K 31/504, A61P 35/00

(54) **MACROCYCLIC COMPOUNDS, PREPARATION METHOD THEREFOR, AND USE THEREOF**

(30) Priority: 01.09.2023 CN 202311130834; 17.04.2024 CN 202410466283; 22.05.2024 CN 202410644266; 26.06.2024 CN 202410844971; 23.08.2024 CN 202411171444
(71) Applicant: Genfleet Therapeutics (Shanghai) Inc., Shanghai 201203 (CN)
(72) Inventor: ZHAO, Jichen, Shanghai 201203 (CN); ZHOU, Fusheng, Shanghai 201203 (CN); LIANG, Tao, Shanghai 201203 (CN); LU, Yandong, Shanghai 201203 (CN); JIANG, Tao, Shanghai 201203 (CN); LIN, Chonglan, Shanghai 201203 (CN); PENG, Ling, Shanghai 201203 (CN); LANG, Jiong, Shanghai 201203 (CN); LU, Qiang, Shanghai 201203 (CN)
(74) Representative: Elzaburu S.L.P.
(86) International application number: PCT/CN2024/116083
(87) International publication number: WO 2025/045233

(57) **Abstract**

Compounds shown as formula (I) or stereoisomers thereof, or pharmaceutically acceptable salts, solvates or prodrugs thereof, a pharmaceutical composition comprising same, and the use of same in the preparation of drugs for preventing and/or treating diseases or disorders related to RAS protein activity, such as cancer.

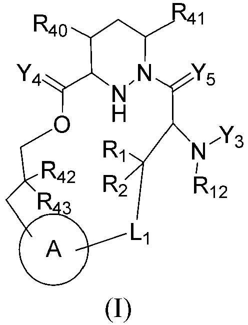

## Description

The present application claims priority to the prior application with the Patent Application No. 202311130834X filed with China National Intellectual Property Administration on September 1, 2023, the prior application with the Patent Application No. 2024104662832 filed with China National Intellectual Property Administration on April 17, 2024, the prior application with the Patent Application No. 2024106442663 filed with China National Intellectual Property Administration on May 22, 2024, the prior application with the Patent Application No. 2024108449718 filed with China National Intellectual Property Administration on June 26, 2024, and the prior application with the Patent Application No. 2024111714441 filed with China National Intellectual Property Administration on August 23, 2024. These five prior applications are incorporated herein by reference in their entirety.

### TECHNICAL FIELD

The present disclosure pertains to the field of pharmaceuticals, and particularly relates to a macrocyclic compound capable of inhibiting the activity of the RAS protein, a preparation method therefor, and use thereof.

### BACKGROUND

The RAS gene (rat sarcoma viral oncogene homolog) encodes a class of small GTPase family proteins with a molecular weight of about 21 kDa. The mammalian RAS family includes three members: HRAS, KRAS, and NRAS, which serve as molecular switches in a variety of cell signaling processes. RAS is in an inactivated state (dormant or turned off) when bound to GDP. When cells are exposed to certain growthpromoting stimuli, RAS is induced to exchange its bound GDP for GTP, thereby becoming activated. Subsequently, it recruits and activates other downstream target proteins (e.g., Raf and PI3K), thereby promoting cell proliferation. The RAS protein endogenously possesses the activity of hydrolyzing GTP to GDP (thereby converting itself into an inactivated state), but the conversion rate is low. However, once it binds to a GTPase-activating protein (GAP), the interaction between the two greatly accelerates the rate at which the RAS protein converts GTP to GDP, such that RAS can revert to an inactivated state rapidly upon cessation of cell stimulation, thereby shutting down the signaling pathway transduction.

RAS gene mutations can be found in approximately 30% of human tumors, in which KRAS mutations are the most common and account for about 85%, while NRAS and HRAS mutations account for 12% and 3%, respectively. KRAS mutations are predominantly found in pancreatic cancer (86%), colorectal cancer (41%), and lung cancer (32%); NRAS mutations are common in melanoma and acute myeloid leukemia; HRAS mutations are common in bladder cancer and head and neck cancer. In tumors, RAS gene mutations occur primarily via point mutations. More than 150 distinct RAS point mutations have been identified, with mutations at positions G12, G13, and Q61 being the most common. Generally, these pathogenic mutations affect the interaction between RAS and GAP and the endogenous GTPase activity of RAS. Consequently, the mutated RAS protein exists mainly in a GTP-bound activated form within cells, such that the signaling pathway mediated by this protein is in a continuously activated state, which in turn gives rise to uncontrolled cell proliferation and ultimately promotes tumor formation.

Currently, for KRAS G12C mutation, targeted drugs AMG510 and MRTX849 have been approved for marketing for the treatment of non-small cell lung cancer with KRAS G12C mutation. However, for KRAS G12D and KRAS G12V mutations that commonly occur in pancreatic cancer and colorectal cancer, as well as NRAS and HRAS mutations commonly found in melanoma and head and neck cancer, no corresponding targeted drugs have been approved. Therefore, the research and development of highly effective inhibitors targeting various RAS mutations is of great clinical significance, and also represents both a focus and a challenge in the field of new drug research and development.

### SUMMARY

The present disclosure provides a class of RAS inhibitors, which drive the formation of a high-affinity ternary complex between an RAS protein and a cytoplasmic chaperonin (e.g., cyclophilinA). The non-covalent interactions between these inhibitors and the RAS protein and cytoplasmic chaperonin can inhibit RAS activity.

The RAS inhibitors of the present disclosure have the advantages of high activity, low toxic and side effects, and the like, and are expected to possess good physicochemical properties and have great potential to be manufactured as a drug.

A first aspect of the present disclosure provides a compound represented by formula (I), or a stable deuterated derivative thereof, or a stereoisomer thereof, or a pharmaceutically acceptable salt thereof, a solvate thereof, or a prodrug thereof: 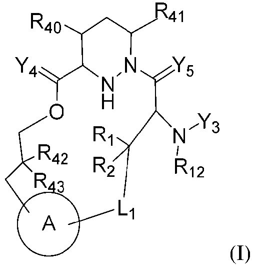 wherein,
ring A is 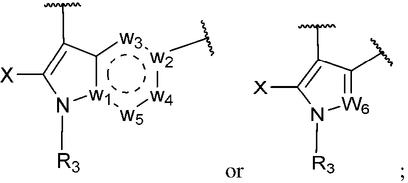 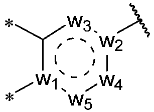 is phenyl, 5- or 6-membered heteroaryl, or 5- or 6-membered heterocyclyl; 1 or 2 ring atoms of the 5- or 6-membered heteroaryl are heteroatoms selected from nitrogen, oxygen, and sulfur; 1 or 2 ring atoms of the 5- or 6-membered heterocyclyl are heteroatoms selected from nitrogen, oxygen, and sulfur; the 5- or 6-membered heterocyclyl is fully saturated heterocyclyl or partially unsaturated heterocyclyl; or wherein, W₁ is CH, C, or N; W₂ is CH, C, or N; W₃ is CR₁₃, O, S, N, or NH; W₄ is CR₁₃, O, S, N, or NH; W₅ is absent, O, or CR_{14;} 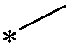 indicates a linking site to the moiety on the left;
W₆ is selected from CR₁₃ and N;
X is 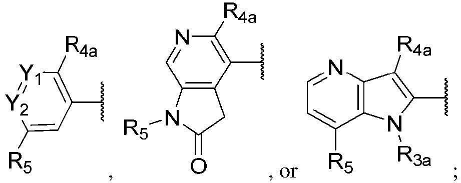
Y₁ is N or CH, wherein N can be oxidized (N⁺-O⁻);
Y₂ is N or CH, wherein N can be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
R₄₀ is hydrogen, halogen, or substituted or unsubstituted C₁₋₆ alkyl; R₄₁ is hydrogen, halogen, or substituted or unsubstituted C₁₋₆ alkyl; or
R₄₀ and R₄₁ are linked to form -CH₂- or -CH₂CH₂-, wherein the -CH₂- or -CH₂CH₂- is optionally substituted with one or more groups selected from deuterium, halogen, C₁₋₆ alkyl, C₁₋₆ haloalkyl, and deuterated C₁₋₆ alkyl;
when ring A is 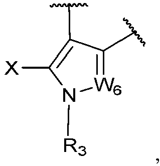 L₁ is -N(R₀₀)C(=O)-, -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, -L₂-substituted or unsubstituted 8- or 10-membered bicyclic heteroaryl-, or -L₂-substituted or unsubstituted 3- to 8-membered heterocyclyl-; L₂ is C₃₋₆ cycloalkyl, 3- to 8-membered heterocyclyl, -O-, - C₁₋₄ alkyl-, -N(R₀₀)-, -C(=O)-, -CR₀₉=CR₁₀-, -N(R₀₀)C(=O)-, or -N(R₀₀)C(=S)-;
when ring A is 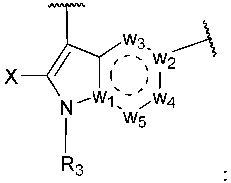
when at least one of W₁, W₂, W₃, W₄, and W₅ is a heteroatom selected from O, S, N, and NH, L₁ is - N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered heterocyclyl-;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
   or,
when W₁ and W₂ are both C, and W₃, W₄, and W₅ are all CH, L₁ is -N(R₀₀)C(=O)-O-, -C₁₋₄ alkyl-N(R₀₀)C(=O)-, -N(R₀₀)C(=O)-C₁₋₄ alkyl-, -C₁₋₄ alkyl-N(R₀₀)C(=S)-, -substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, -O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁ R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂,-C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7-to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), - S(=O)(-C₁₋₄ alkyl-NR₀₁R₀₂), -S(=O)₂(-C₁₋₄ alkyl-C(R₀₅)R₀₃R₀₄), -S(=O)(-C₁₋₄ alkyl-C(R₀₅)R₀₃R₀₄), -C₂₋₄ alkynyl-substituted or unsubstituted 5- or 6-membered monocyclic heteroaryl, -C₂₋₄ alkynyl-substituted or unsubstituted 8- to 10-membered bicyclic heteroaryl, -C₂₋₄ alkynyl-substituted or unsubstituted 6- to 12-membered heteroaryl-fused heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, substituted or unsubstituted C₆₋₁₀ aryl-substituted or unsubstituted 3- to 8-membered monocyclic heterocyclyl, substituted or unsubstituted 5- or 6-membered monocyclic heteroaryl-substituted or unsubstituted 3- to 8-membered monocyclic heterocyclyl, substituted or unsubstituted C₆₋₁₀ aryl-C₁₋₄ alkyl-substituted or unsubstituted 3- to 8-membered monocyclic heterocyclyl, substituted or unsubstituted 5- or 6-membered monocyclic heteroaryl-C₁₋₄ alkyl-substituted or unsubstituted 3- to 8-membered monocyclic heterocyclyl, substituted or unsubstituted C₆₋₁₀ aryl-fused substituted or unsubstituted 3- to 8-membered monocyclic heterocyclyl, substituted or unsubstituted 5- or 6-membered monocyclic heteroaryl-fused substituted or unsubstituted 3- to 8-membered monocyclic heterocyclyl, substituted or unsubstituted C₆₋₁₀ aryl-C₁₋₄ alkyl-NR₀₁R₀₂, or substituted or unsubstituted 5- or 6-membered monocyclic heteroaryl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein 2 hydrogen atoms on the same carbon atom of the C₁₋₆ alkyl or C₁₋₆ alkoxy are optionally both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 5-to 15-membered tricyclic heterocyclyl, substituted or unsubstituted 7- to 12-membered bicyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or
Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6-to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
Y₅ is 0 or S;
Y₄ is O or S;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₃ is hydrogen, halogen, substituted or unsubstituted C₁₋₆ alkyl, or substituted or unsubstituted C₁₋₆ alkoxy; R₁₄ is hydrogen, halogen, or substituted or unsubstituted C₁₋₆ alkyl; or
in W₄ and W₅, R₁₃ and R₁₄, together with the carbon atom to which they are attached, form substituted or unsubstituted C₃₋₆ cycloalkyl, substituted or unsubstituted C₆₋₁₀ aryl, substituted or unsubstituted 5- or 6-membered monocyclic heteroaryl, or substituted or unsubstituted 3- to 8-membered heterocyclyl; or
in W₅, R₁₄ and R₃, together with the atom to which they are attached, form substituted or unsubstituted nitrogen-containing 4- to 8-membered monocyclic heterocyclyl; in the nitrogen-containing 4- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus; or
in W₆, R₁₃ and R₃, together with the atom to which they are attached, form substituted or unsubstituted nitrogen-containing 3- to 8-membered monocyclic heterocyclyl or substituted or unsubstituted 5- or 6-membered monocyclic heteroaryl; in the nitrogen-containing 3- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus;
in the groups described above, the substitutions each independently refer to that the groups described above are each independently substituted with 1, 2, 3, 4, 5, or 6 groups selected from group S1; the groups of group S1 are each independently selected from the following group consisting of: -SF₅, deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C ₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, -C₁₋₄ alkyl-hydroxy, -C₁₋₄ alkyl-NR^{a1}R^{b1}, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, - C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₃₋₆ monocyclic cycloalkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, or -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C ₁₋₆ alkyl)₂, -C₁₋₄ alkyl-P(=O)-(C ₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, phenyl, and C₃₋₆ monocyclic cycloalkyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

Herein, to further illustrate the linking positions of 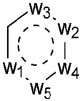 to other moieties of the molecule, it can be represented as 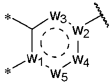 ; the asterisk in 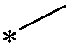 is linked to the moiety on the left; the wavy line in 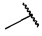 is linked to rest of the molecule.

In one embodiment, R₄₀ is hydrogen; R₄₁ is hydrogen.

The first aspect of the present disclosure also provides a compound represented by formula (ID), or a stable deuterated derivative thereof, or a stereoisomer thereof, or a pharmaceutically acceptable salt thereof, a solvate thereof, or a prodrug thereof: 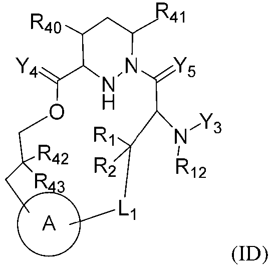 wherein,
ring A is 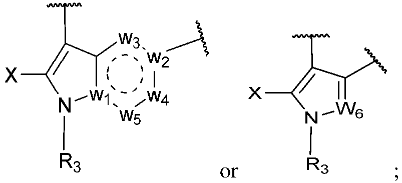 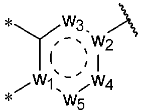 is phenyl, 5- or 6-membered monocyclic heteroaryl, or 5- or 6-membered monocyclic heterocyclyl; 1 or 2 ring atoms of the 5- or 6-membered monocyclic heteroaryl are heteroatoms selected from nitrogen, oxygen, and sulfur; 1 or 2 ring atoms of the 5- or 6-membered monocyclic heterocyclyl are heteroatoms selected from nitrogen, oxygen, and sulfur; the 5- or 6-membered monocyclic heterocyclyl is fully saturated heterocyclyl or partially unsaturated heterocyclyl; or wherein, W₁ is CH, C, or N; W₂ is CH, C, or N; W₃ is CR₁₃, O, S, N, or NH; W₄ is CR₁₃, O, S, N, or NH; W₅ is absent, O, or CR₁₄; 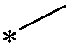 indicates a linking site to the moiety on the left;
W₆ is CR₁₃ or N;
X is 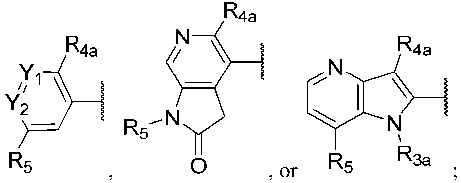
Y₁ is N or CH, wherein N can be oxidized (N⁺-O⁻);
Y₂ is N or CH, wherein N can be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ monocyclic cycloalkyl or substituted or unsubstituted 3- to 8-membered monocyclic heterocyclyl;
R₄₀ is hydrogen, halogen, or substituted or unsubstituted C₁₋₆ alkyl; R₄₁ is hydrogen, halogen, or substituted or unsubstituted C₁₋₆ alkyl; or
R₄₀ and R₄₁ are linked to form -CH₂- or -CH₂CH₂-, wherein the -CH₂- or -CH₂CH₂- is optionally substituted with one or more groups selected from deuterium, halogen, C₁₋₆ alkyl, C₁₋₆ haloalkyl, and deuterated C₁₋₆ alkyl;
when ring A is 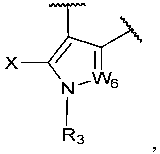 L₁ is -N(R₀₀)C(=O)-, -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ monocyclic cycloalkyl, substituted or unsubstituted 3- to 8-membered monocyclic heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, -L₂-substituted or unsubstituted 8- or 10-membered bicyclic heteroaryl-, or -L₂-substituted or unsubstituted 3- to 8-membered monocyclic heterocyclyl-; L₂ is C₃₋₆ monocyclic cycloalkyl, 3- to 8-membered monocyclic heterocyclyl, -O-, -C₁₋₄ alkyl-, -N(R₀₀)-, -C(=O)-, -CR₀₉=CR₁₀-, - N(R₀₀)C(=O)-, or -N(R₀₀)C(=S)-;
when ring A is 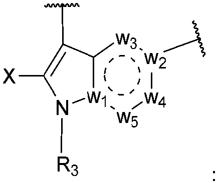
when at least one of W₁, W₂, W₃, W₄, and W₅ is a heteroatom selected from O, S, N, and NH, L₁ is - N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ monocyclic cycloalkyl, substituted or unsubstituted 3- to 8-membered monocyclic heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered monocyclic heterocyclyl-;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
   or,
when W₁ and W₂ are both C, and W₃, W₄, and W₅ are all CH, L₁ is -N(R₀₀)C(=O)-O-, -C₁₋₄ alkyl-N(R₀₀)C(=O)-, -N(R₀₀)C(=O)-C₁₋₄ alkyl-, -C₁₋₄ alkyl-N(R₀₀)C(=S)-, -substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₄ₐ is substituted or unsubstituted C₁₋₆ alkyl, substituted or unsubstituted C₃₋₆ monocyclic cycloalkyl, or substituted or unsubstituted 3- to 8-membered monocyclic heterocyclyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃ R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, -O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂,-CH=CR₀₁R₀₂,-C₂₋₄ alkenyl-NRₒ₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7-to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), - S(=O)(-C₁₋₄ alkyl-NR₀₁R₀₂), -S(=O)₂(-C₁₋₄ alkyl-C(R₀₅)R₀₃R₀₄), -S(=O)(-C₁₋₄ alkyl-C(R₀₅)R₀₃R₀₄), -C₂₋₄ alkynyl-substituted or unsubstituted 5- or 6-membered monocyclic heteroaryl, -C₂₋₄ alkynyl-substituted or unsubstituted 8- to 10-membered bicyclic heteroaryl, -C₂₋₄ alkynyl-substituted or unsubstituted 6- to 12-membered heteroaryl-fused heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, substituted or unsubstituted C₆₋₁₀ aryl-substituted or unsubstituted 3- to 8-membered monocyclic heterocyclyl, substituted or unsubstituted 5- or 6-membered monocyclic heteroaryl-substituted or unsubstituted 3- to 8-membered monocyclic heterocyclyl, substituted or unsubstituted C₆₋₁₀ aryl-C₁₋₄ alkyl-substituted or unsubstituted 3- to 8-membered monocyclic heterocyclyl, substituted or unsubstituted 5- or 6-membered monocyclic heteroaryl-C₁₋₄ alkyl-substituted or unsubstituted 3- to 8-membered monocyclic heterocyclyl, substituted or unsubstituted C₆₋₁₀ aryl-fused substituted or unsubstituted 3- to 8-membered monocyclic heterocyclyl, substituted or unsubstituted 5- or 6-membered monocyclic heteroaryl-fused substituted or unsubstituted 3- to 8-membered monocyclic heterocyclyl, substituted or unsubstituted C₆₋₁₀ aryl-C₁₋₄ alkyl-NR₀₁R₀₂, or substituted or unsubstituted 5- or 6-membered monocyclic heteroaryl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ monocyclic cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein 2 hydrogen atoms on the same carbon atom of the C₁₋₆ alkyl or C₁₋₆ alkoxy are optionally both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7-to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or
Y₃ and R₁₂, together with the nitrogen atom to which they are attached, form substituted or unsubstituted nitrogen-containing 3- to 8-membered monocyclic heterocyclyl; in the nitrogen-containing 3- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ monocyclic cycloalkyl, substituted or unsubstituted 3- to 8-membered monocyclic heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered monocyclic heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6-to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ monocyclic cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ monocyclic cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
Y₅ is O or S;
Y₄ is O or S;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ monocyclic cycloalkyl, substituted or unsubstituted 3- to 8-membered monocyclic heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₃ is hydrogen, halogen, substituted or unsubstituted C₁₋₆ alkoxy, or substituted or unsubstituted C₁₋₆ alkyl; R₁₄ is hydrogen, halogen, or substituted or unsubstituted C₁₋₆ alkyl; or
in W₄ and W₅, R₁₃ and R₁₄, together with the carbon atom to which they are attached, form substituted or unsubstituted C₃₋₆ monocyclic cycloalkyl, substituted or unsubstituted C₆₋₁₀ aryl, substituted or unsubstituted 5- or 6-membered monocyclic heteroaryl, or substituted or unsubstituted 3- to 8-membered monocyclic heterocyclyl; or
in W₅, R₁₄ and R₃, together with the atom to which they are attached, form substituted or unsubstituted nitrogen-containing 4- to 8-membered monocyclic heterocyclyl; in the nitrogen-containing 4- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus; or
in W₆, R₁₃ and R₃, together with the atom to which they are attached, form substituted or unsubstituted nitrogen-containing 3- to 8-membered monocyclic heterocyclyl or substituted or unsubstituted 5- or 6-membered monocyclic heteroaryl; in the nitrogen-containing 3- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus;
in the groups described above, the substitutions each independently refer to that the groups described above are each independently substituted with 1, 2, 3, 4, 5, or 6 groups selected from group S1; the groups of group S1 are each independently selected from the following group consisting of: -SF₅, deuterium, oxo (=O), thio (=S), =CR^{e} R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1} R^{b1}, -C(=O)-NR^{a1} R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1} -S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂; wherein the C₁₋₆ alkyl, the C₁₋₆ alkoxy, the C₂₋₆ alkenyl, and the C₂₋₆ alkynyl are each independently optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl,-C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 6-membered monocyclic heterocyclyl, 3- to 10-membered monocyclic heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

The first aspect of the present disclosure also provides a compound represented by formula (IC), or a stable deuterated derivative thereof, or a stereoisomer thereof, or a pharmaceutically acceptable salt thereof, a solvate thereof, or a prodrug thereof: 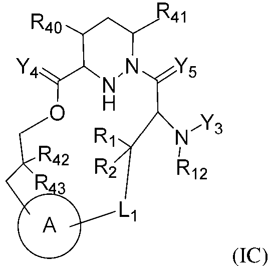 wherein,
ring A is 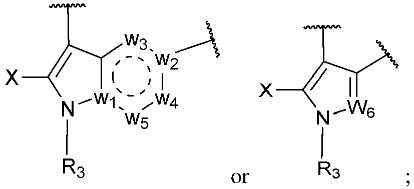 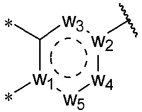 is phenyl, 5- or 6-membered heteroaryl, or 5- or 6-membered heterocyclyl; 1 or 2 ring atoms of the 5- or 6-membered heteroaryl are heteroatoms selected from nitrogen, oxygen, and sulfur; 1 or 2 ring atoms of the 5- or 6-membered heterocyclyl are heteroatoms selected from nitrogen, oxygen, and sulfur;
the 5- or 6-membered heterocyclyl is fully saturated heterocyclyl or partially unsaturated heterocyclyl; 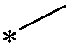
indicates a linking site to the moiety on the left;
wherein W₁ and W₂ are each independently selected from C and N;
W₃ and W₄ are each independently selected from CR₁₃, O, S, N, and NH;
W₆ is selected from CR₁₃ and N;
W₅ is selected from being absent, O, and CR₁₄;
X is 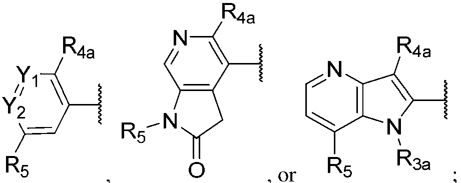
Y₁ is N or CH, wherein N can be oxidized (N⁺-O⁻);
Y₂ is N or CH, wherein N can be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
R₄₀ is hydrogen, halogen, or substituted or unsubstituted C₁₋₆ alkyl; R₄₁ is hydrogen, halogen, or substituted or unsubstituted C₁₋₆ alkyl; or
R₄₀ and R₄₁ are linked to form -CH₂- or -CH₂CH₂-, wherein the -CH₂- or -CH₂CH₂- is optionally substituted with one or more groups selected from deuterium, halogen, C₁₋₆ alkyl, C₁₋₆ haloalkyl, and deuterated C₁₋₆ alkyl;
when ring A is 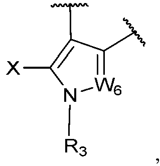 **L₁** is -N(R₀₀)C(=O)-, -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered heterocyclyl-; L₂ is C₃₋₆ cycloalkyl, 3- to 8-membered heterocyclyl, -O-, -C₁₋₄ alkyl-, -N(R₀₀)-, -C(=O)-, -CR₀₉=CR₁₀-, -N(R₀₀)C(=O)-, or - N(R₀₀)C(=S)-;
when ring A is 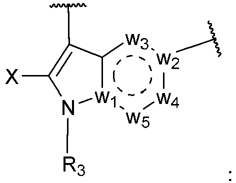
when at least one of W₁, W₂, W₃, W₄, and W₅ is a heteroatom selected from O, S, N, and NH, **L₁** is - N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered heterocyclyl-;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
   or,
when W₁ and W₂ are both C, and W₃, W₄, and W₅ are all CH, L₁ is -N(R₀₀)C(=O)-O-, -C₁₋₄ alkyl-N(R₀₀)C(=O)-, -N(R₀₀)C(=O)-C₁₋₄ alkyl-, -C₁₋₄ alkyl-N(R₀₀)C(=S)-, -substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl; R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃ R₀₄, -O-C₁₋₄ alkyl-NR₀₁R₀₂, -O-C₁₄ alkyl-C(R₀₅)R₀₃R₀₄, -O-C₁₋₄ alkyl-N(R₀₆)C(=O)-R₀₃, -O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂,-C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7-to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), - S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein 2 hydrogen atoms on the same carbon atom of the C₁₋₆ alkyl or C₁₋₆ alkoxy are optionally both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7-to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or
Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6-to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atom to which they are attached, form substituted or unsubstituted C₃₋₆ cycloalkyl or substituted or unsubstituted 3- to 8-membered heterocyclyl;
in the groups described above, the substitutions each independently refer to that the groups described above are each independently substituted with 1, 2, 3, 4, 5, or 6 groups selected from group S1; the groups of group S1 are each independently selected from the following group consisting of: -SF₅, deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C_{1 6} alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O) ₂-NR^{a1}R^{b1}, -NR^{d1}-C(=O)-R^{c1}, -NR^{d1} -C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{e1}, -NR^{d1}-S(=O)₂-NR^{a1}R^{b1}, and -P(=O)-(C₁₋₆ alkyl)₂; wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

The first aspect of the present disclosure also provides a compound represented by formula (IB), or a stable deuterated derivative thereof, or a stereoisomer thereof, or a pharmaceutically acceptable salt thereof, a solvate thereof, or a prodrug thereof: 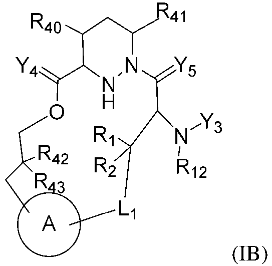 wherein,
ring A is 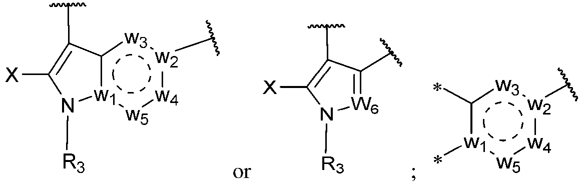 is phenyl, 5- or 6-membered heteroaryl, or 5- or 6-membered heterocyclyl; 1 or 2 ring atoms of the 5- or 6-membered heteroaryl are heteroatoms selected from nitrogen, oxygen, and sulfur; 1 or 2 ring atoms of the 5- or 6-membered heterocyclyl are heteroatoms selected from nitrogen, oxygen, and sulfur; the 5- or 6-membered heterocyclyl is fully saturated heterocyclyl or partially unsaturated heterocyclyl; or
wherein W₁ and W₂ are each independently selected from C and N;
W₃ and W₄ are each independently selected from CR₁₃, O, S, N, and NH;
W₅ is selected from being absent, O, and CR₁₄;
W₆ is selected from CR₁₃ and N;
X is 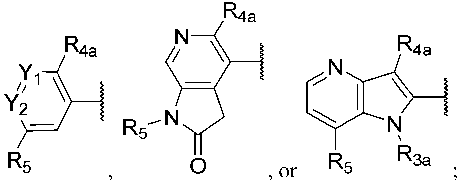
Y₁ is N or CH, wherein N can be oxidized (N⁺-O⁻);
Y₂ is N or CH, wherein N can be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
R₄₀ is hydrogen, halogen, or substituted or unsubstituted C₁₋₆ alkyl; R₄₁ is hydrogen, halogen, or substituted or unsubstituted C₁₋₆ alkyl; or
R₄₀ and R₄₁ are linked to form -CH₂- or -CH₂CH₂ -, wherein -CH₂ - or -CH₂CH₂ - is optionally substituted with halogen;
when ring A is 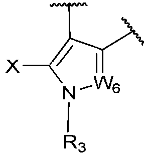 , **L₁** is -N(R₀₀)C(=O)-, -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered heterocyclyl-; L₂ is -O-, -C₁₋₄ alkyl-, - N(R₀₀)-, -C(=O)-, -CR₀₉=CR₁₀-, -N(R₀₀)C(=O)-, or -N(R₀₀)C(=S)-;
when ring A is 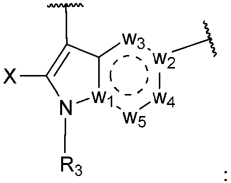
when at least one of W₁, W₂, W₃, W₄, and W₅ is a heteroatom selected from O, S, N, and NH, **L₁** is - N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered heterocyclyl-;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
   or,
when W₁ and W₂ are both C, and W₃, W₄, and W₅ are all CH, **L₁** is -N(R₀₀)C(=O)-O-, -C₁₋₄ alkyl-N(R₀₀)C(=O)-, -N(R₀₀)C(=O)-C₁₋₄ alkyl-, -C₁₋₄ alkyl-N(R₀₀)C(=S)-, -substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl; R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₄ alkyl-C(R₀₅)R₀₃R₀₄, -O-C₁₋₄ alkyl-N(R₀₆)C(=O)-R₀₃, -O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7-to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), - S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and Rₒ₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7-to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or
Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6-to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atom to which they are attached, form substituted or unsubstituted C₃₋₆ cycloalkyl or substituted or unsubstituted 3- to 8-membered heterocyclyl;
in the groups described above, the substitutions each independently refer to that the groups described above are each independently substituted with 1, 2, 3, 4, 5, or 6 groups selected from group S1; the groups of group S1 are each independently selected from the following group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, - O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C ₁₋₆ alkyl)₂, -C₁₋₄ alkyl-P(=O)-(C ₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

The first aspect of the present disclosure also provides a compound represented by formula (IA), or a stable deuterated derivative thereof, or a stereoisomer thereof, or a pharmaceutically acceptable salt thereof, a solvate thereof, or a prodrug thereof: 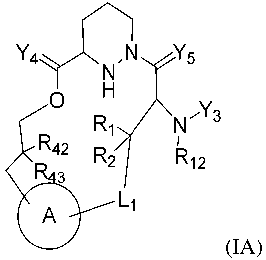 wherein,
ring A is 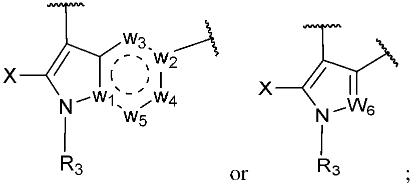 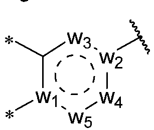 is phenyl, 5- or 6-membered heteroaryl, or 5- or 6-membered heterocyclyl; 1 or 2 ring atoms of the 5- or 6-membered heteroaryl are heteroatoms selected from nitrogen, oxygen, and sulfur; 1 or 2 ring atoms of the 5- or 6-membered heterocyclyl are heteroatoms selected from nitrogen, oxygen, and sulfur; the 5- or 6-membered heterocyclyl is fully saturated heterocyclyl or partially unsaturated heterocyclyl;
wherein W₁ and W₂ are each independently selected from C and N;
W₃ and W₄ are each independently selected from CR₁₃, O, S, N, and NH;
W₅ is selected from being absent, O, and CR₁₄;
W₆ is selected from CR₁₃ and N;
X is 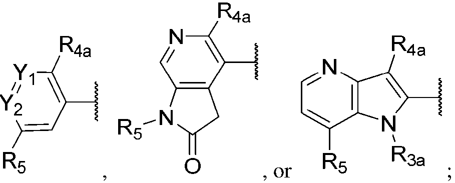
Y₁ is N or CH, wherein N can be oxidized (N⁺-O⁻);
Y₂ is N or CH, wherein N can be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
when ring A is 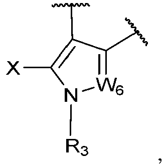 L₁ is -N(R₀₀)C(=O)-, -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl, -L₂-substituted or unsubstituted 5- or 6-membered monocyclic heteroaryl, or -L₂-substituted or unsubstituted 3- to 8-membered heterocyclyl-; L₂ is -O-, -C₁₋₄ alkyl-, - N(R₀₀)-, -C(=O)-, -CR₀₉=CR₁₀-, -N(R₀₀)C(=O)-, or -N(R₀₀)C(=S)-;
when ring A is 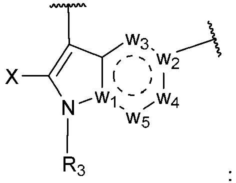
when at least one of W₁, W₂, W₃, W₄, and W₅ is a heteroatom selected from O, S, N, and NH, L₁ is - N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl, -L₂-substituted or unsubstituted 5- or 6-membered monocyclic heteroaryl, or -L₂-substituted or unsubstituted 3- to 8-membered heterocyclyl;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
   or,
when W₁ and W₂ are both C, and W₃, W₄, and W₅ are all CH, L₁ is -N(R₀₀)C(=O)-O-, -C₁₋₄ alkyl-N(R₀₀)C(=O)-, -N(R₀₀)C(=O)-C₁₋₄ alkyl-, -C₁₋₄ alkyl-N(R₀₀)C(=S)-, -substituted or unsubstituted C₁₋₆ alkyl-O-, or -substituted or unsubstituted C₂₋₆ alkenyl-O-; or
when W₁, W₂, W₃, W₄, and W₅ are all C or CH, L₁ is -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, or - substituted or unsubstituted C₂₋₆ alkenyl-O-; R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl; R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl; R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, -O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, - C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), -S(=O)(-C₁₋₄ alkyl-NR₀₁R₀₂), -S(=O)₂(-C₁₋₄ alkyl-C(R₀₅)R₀₃R₀₄), or -S(=O)(-C₁₋₄ alkyl-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7-to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or
Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6-to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
Y₅ is O or S;
Y₄ is O or S;
R₁₃ is hydrogen, halogen, or substituted or unsubstituted C₁₋₆ alkyl; R₁₄ is hydrogen, halogen, or substituted or unsubstituted C₁₋₆ alkyl; or
R₁₃ and R₁₄, together with the carbon atom to which they are attached, form substituted or unsubstituted C₃₋₆ cycloalkyl or substituted or unsubstituted 3- to 8-membered heterocyclyl;
in the groups described above, the substitutions each independently refer to that the groups described above are each independently substituted with 1, 2, 3, 4, 5, or 6 groups selected from group S1; the groups of group S1 are each independently selected from the following group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, - O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C ₁₋₆ alkyl)₂, -C₁₋₄ alkyl-P(=O)-(C ₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, 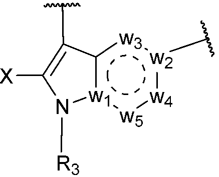 is selected from the following group consisting of: 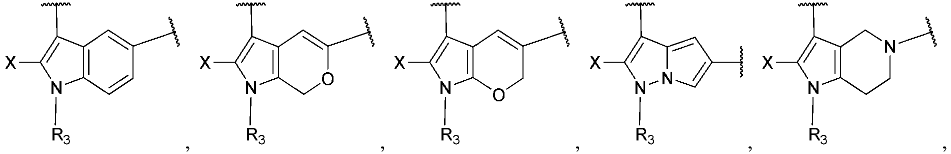 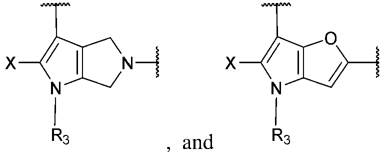 , wherein X and R₃ are each as defined in any one of the embodiments of the present disclosure.

In one embodiment, 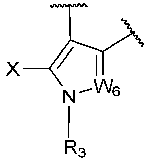 is selected from 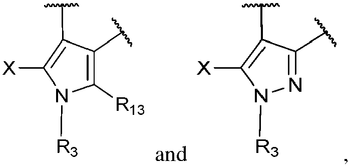 wherein R₁₃, X, and R₃ are each as defined in any one of the embodiments of the present disclosure.

In one embodiment, 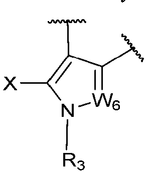 is selected from 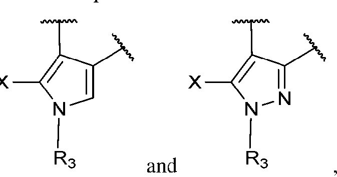 wherein X and R₃ are each as defined in any one of the embodiments of the present disclosure.

The first aspect of the present disclosure also provides a compound represented by formula (A), or a stable deuterated derivative thereof, or a stereoisomer thereof, or a pharmaceutically acceptable salt thereof, a solvate thereof, or a prodrug thereof: 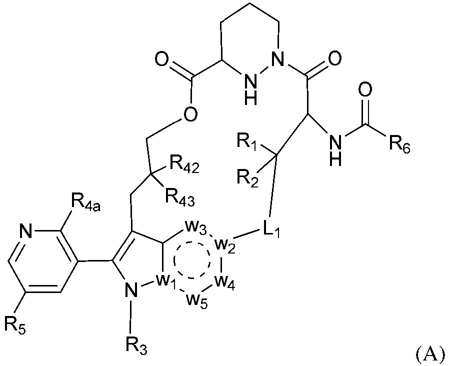
wherein, W₁ is CH, C, or N; W₂ is CH, C, or N; W₃ is CR₁₃, O, S, N, or NH; W₄ is CR₁₃, O, S, N, or NH; W₅ is absent, O, or CR₁₄;
R₁, R₂, R₃, R₄₂, R₄₃, R₄ₐ, R₅, R₆, R₁₃, R₁₄, and L₁ are each as defined in any one of the embodiments of the present disclosure.

The first aspect of the present disclosure also provides a compound represented by formula (B), or a stable deuterated derivative thereof, or a stereoisomer thereof, or a pharmaceutically acceptable salt thereof, a solvate thereof, or a prodrug thereof: 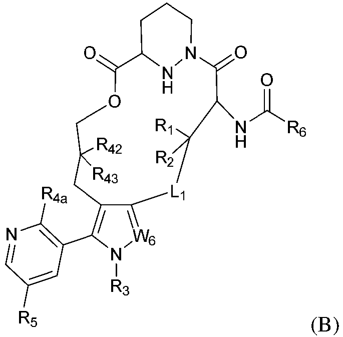
wherein, W₆ is selected from CR₁₃ and N;
R₁, R₂, R₃, R₄₂, R₄₃, R₄ₐ, R₅, R₆, R₁₃, and L₁ are each as defined in any one of the embodiments of the present disclosure.

The first aspect of the present disclosure also provides a compound represented by formula (A1), formula (A2), formula (A3), formula (A4), formula (A5), formula (A6), formula (B1), formula (B2), formula (B3), or formula (B4), or a stable deuterated derivative thereof, or a stereoisomer thereof, or a pharmaceutically acceptable salt thereof, a solvate thereof, or a prodrug thereof: 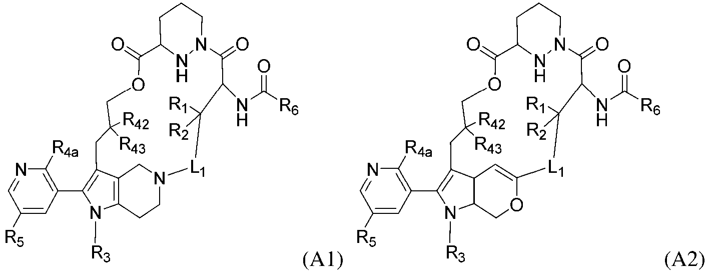 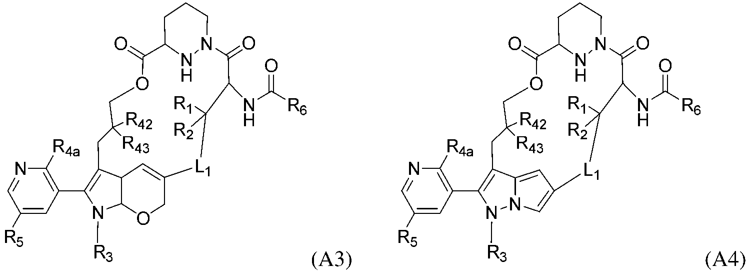 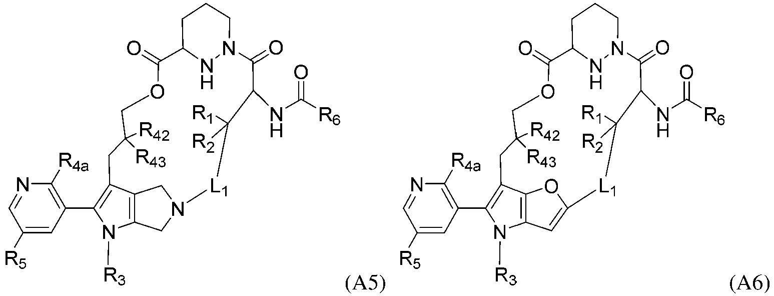 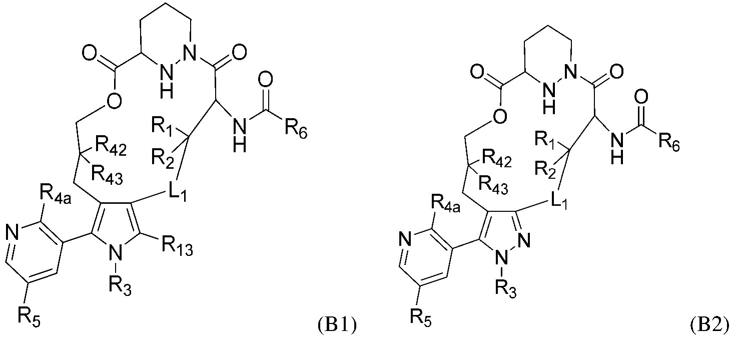 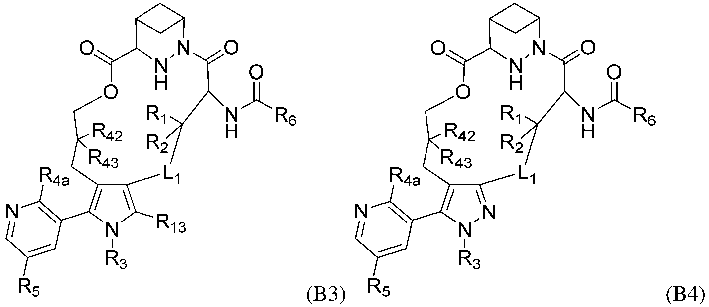 each group is as defined in any one of the embodiments of the present disclosure.

**In** one embodiment, in formula (A1), formula (A2), formula (A3), formula (A4), formula (A5), formula (A6), formula (B1), formula (B2), formula (B3), or formula (B4),
R₁₃ is hydrogen, halogen, or substituted or unsubstituted C₁₋₆ alkyl;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, substituted or unsubstituted C₁₋₆ alkyl, -substituted or unsubstituted C₁₋₆ alkyl-O-, -substituted or unsubstituted C₂₋₆ alkenyl-O-substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl, -L₂-substituted or unsubstituted 5- or 6-membered monocyclic heteroaryl, or -L₂-substituted or unsubstituted 3- to 8-membered heterocyclyl; or
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is -NR₀₁R₀₂ or -C₂₋₄ alkynyl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
in the groups described above, the substitutions each independently refer to that the groups described above are each independently substituted with 1, 2, 3, 4, 5, or 6 groups selected from group S1; the groups of group S1 are each independently selected from the following group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, - O-5- or 6-membered monocyclic heteroaryl, -O-8- to 10-membered bicyclic heteroaryl; -C≡C-C₃₋₂₀ cycloalkyl, -C≡ C-3 to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, and -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1} R^{b1}, -C≡C-C₁₋₄ alkyl-C(=O)-NR^{a1} R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)2-R^{c1}, -C₁₋₄ alkyl-S(=O) ₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O) ₂-NR^{a1}R^{b1}, -NR^{d1}-S(=O) ₂-NR^{a1} R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, the formula (B1) is shown as follows: 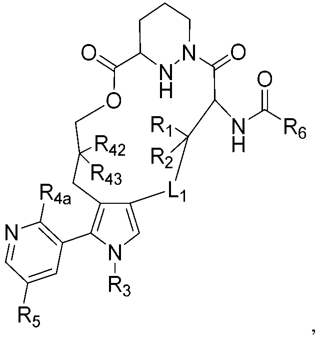 wherein each group is as defined in any one of the embodiments of the present disclosure.

The first aspect of the present disclosure also provides a compound represented by formula (B5), or a stable deuterated derivative thereof, or a stereoisomer thereof, or a pharmaceutically acceptable salt thereof, a solvate thereof, or a prodrug thereof: 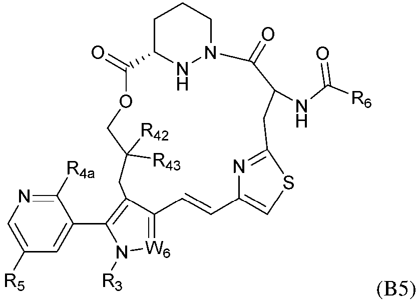
wherein, W₆ is selected from CR₁₃ and N;
R₁, R₂, R₃, R₄₂, R₄₃, R₄ₐ, R₅, R₆, R₁₃, and L₁ are each as defined in any one of the embodiments of the present disclosure.

The first aspect of the present disclosure also provides a compound represented by formula (C1), or a stable deuterated derivative thereof, or a stereoisomer thereof, or a pharmaceutically acceptable salt thereof, a solvate thereof, or a prodrug thereof: 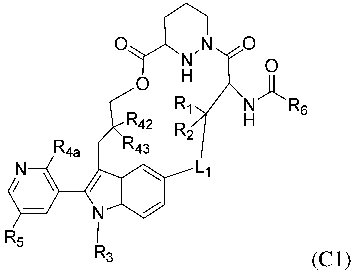 wherein each group is as defined in any one of the embodiments of the present disclosure.

In one embodiment, in formula (C1),
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, - substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl; R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is -NR₀₁R₀₂ or -C₂₋₄ alkynyl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
in the groups described above, the substitutions each independently refer to that the groups described above are each independently substituted with 1, 2, 3, 4, 5, or 6 groups selected from group S1; the groups of group S1 are each independently selected from the following group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, - O-5- or 6-membered monocyclic heteroaryl, -O-8- to 10-membered bicyclic heteroaryl; -C≡C-C₃₋₂₀ cycloalkyl, -C≡ C-3 to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, and -C(=O)-8-to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1} R^{b1}, -C≡C-C₁₋₄ alkyl-C(=O)-NR^{a1} R^{b1}, -C₁₋₄ alkyl-OR^{c1}, -C₁₋₄ alkyl-P(=O)-(C1 ₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O) ₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O) ₂-NR^{a1}R^{b1}, -NR^{d1}-S(=O)₂-NR^{a1} R^{b1}, and -P(=O)-(C₁₋₆ alkyl)₂, wherein the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl, wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C ₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, R₄ₐ is -C₁₋₆ alkyl-C₁₋₆ alkoxy.

In one embodiment, R₄ₐ is -C₁₋₃ alkyl-C₁₋₃ alkoxy.

In one embodiment, R₄ₐ is -CH(CH₃)-O-CH₃.

In one embodiment, R₄ₐ is

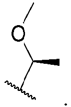

In one embodiment, R₅ is selected from hydrogen, 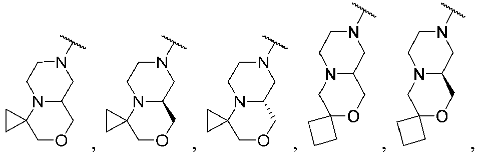 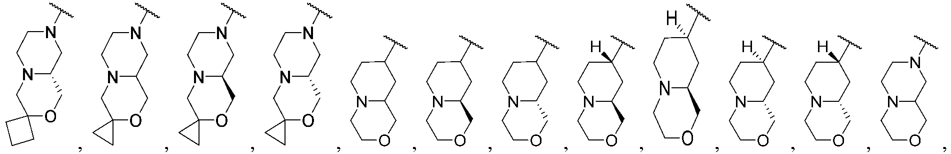 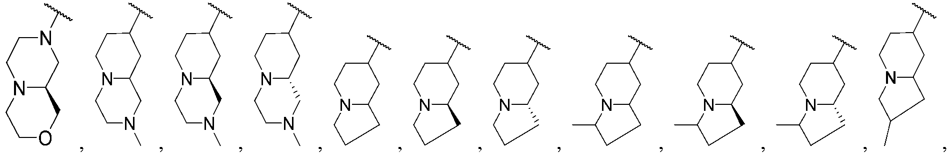 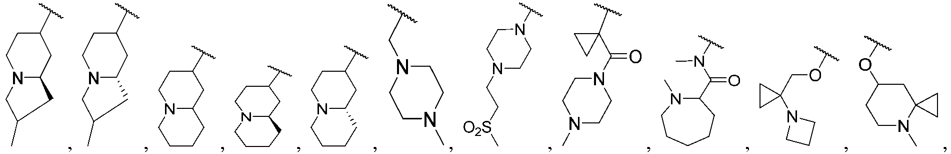 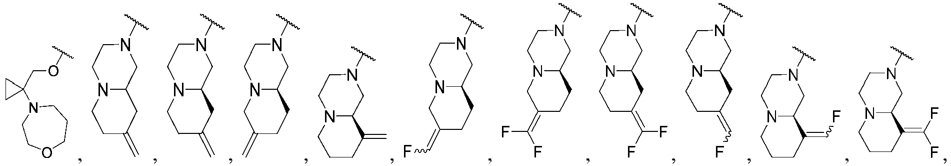 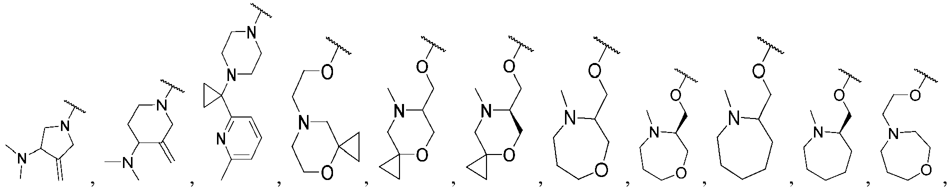 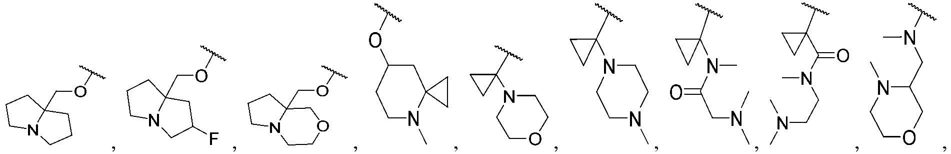 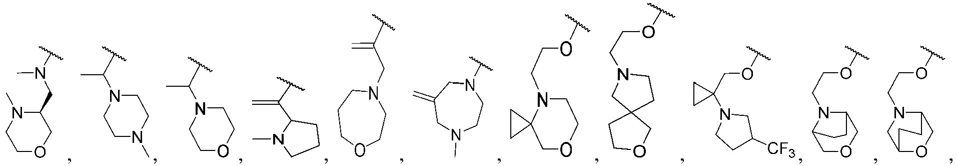 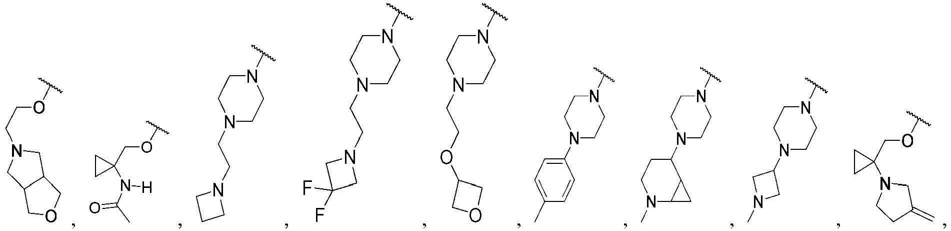 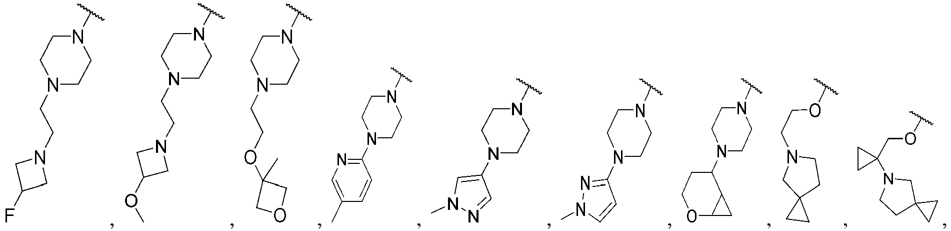 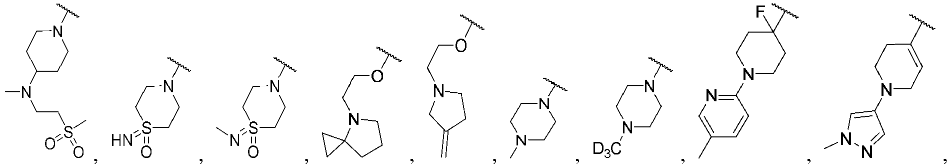 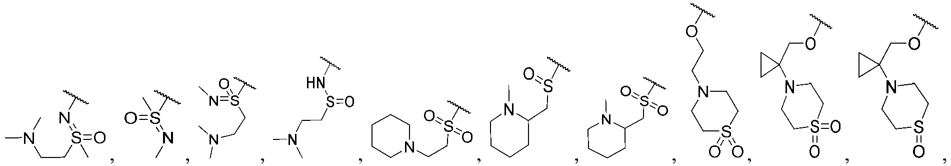 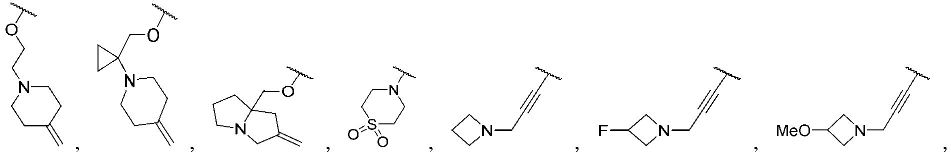 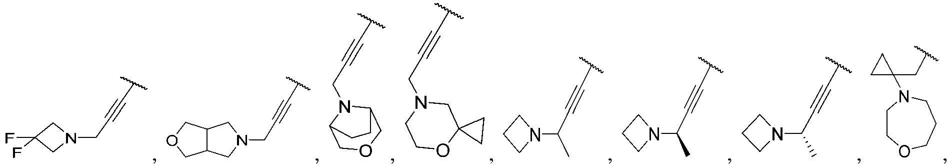 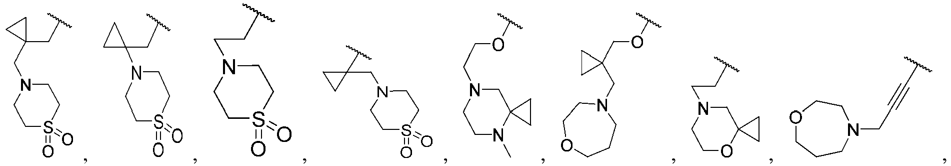 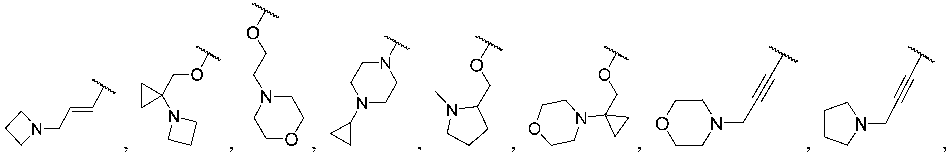 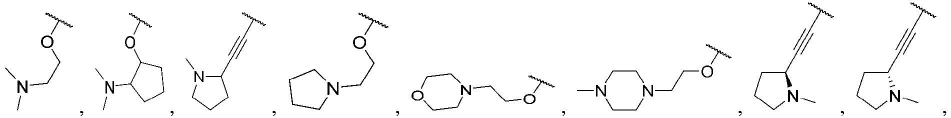 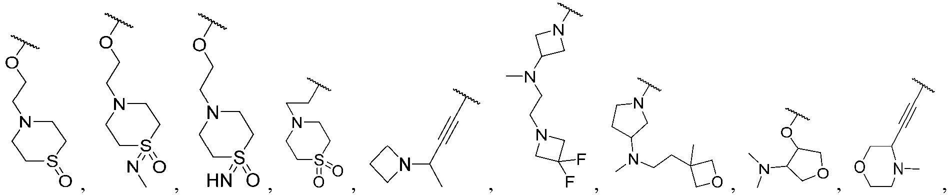 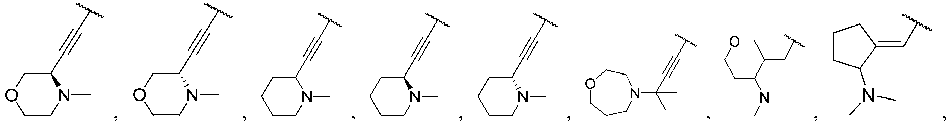 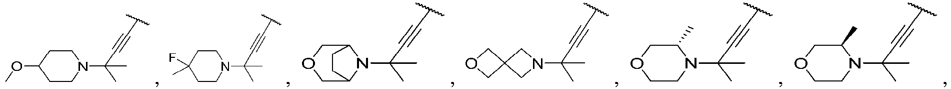 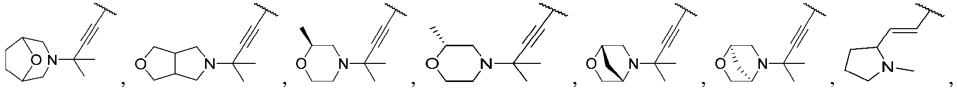 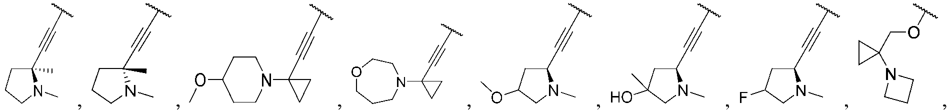 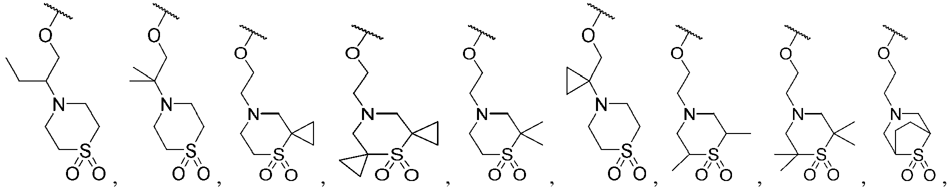 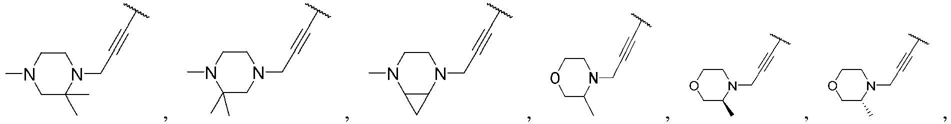 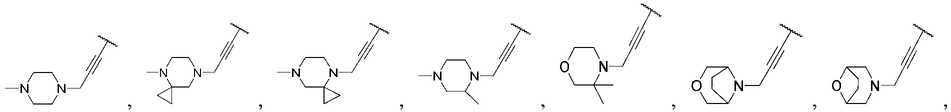 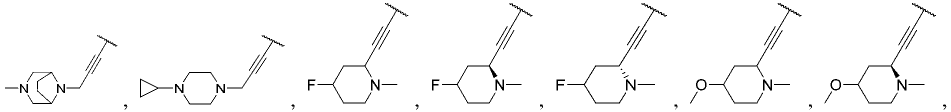 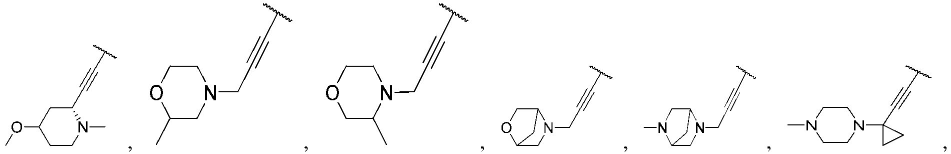 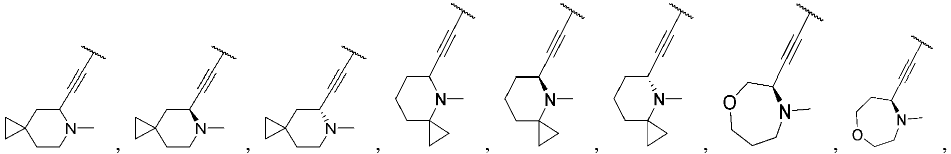 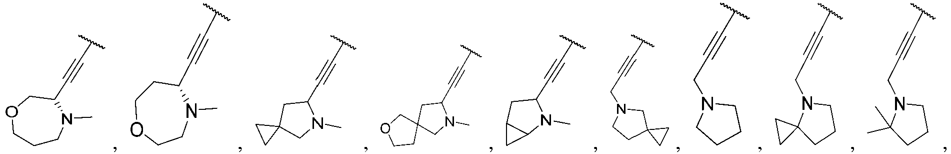 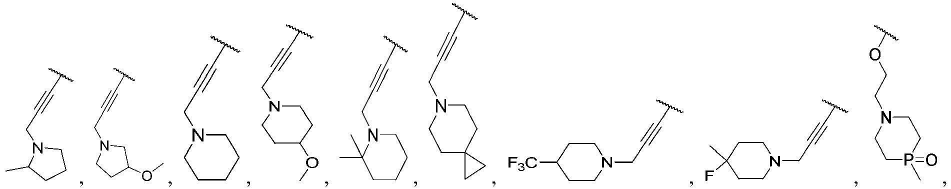 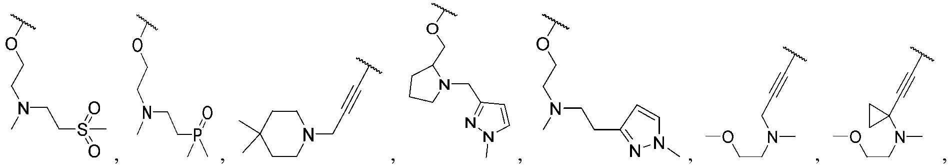 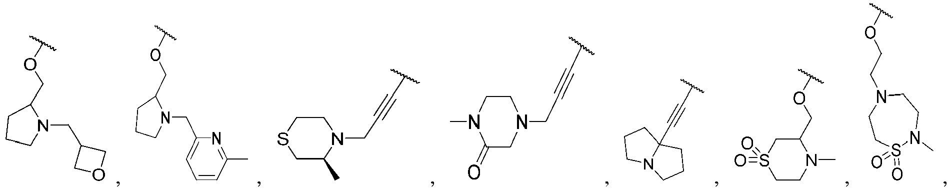 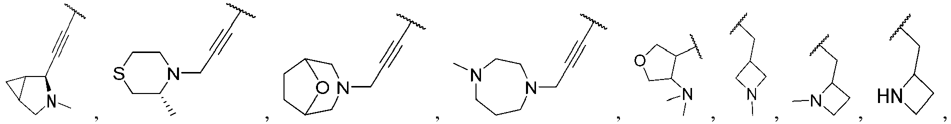 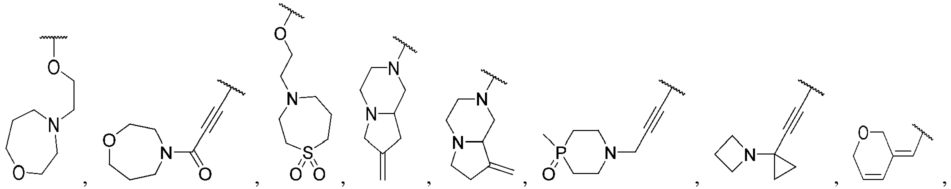 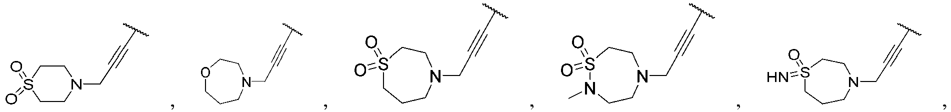 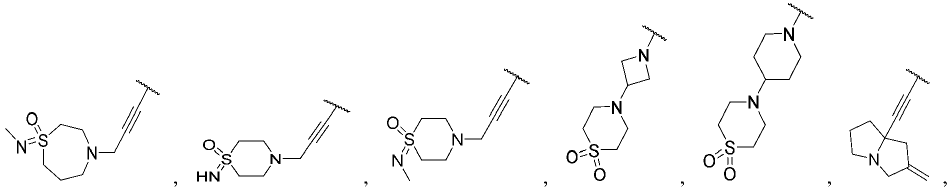 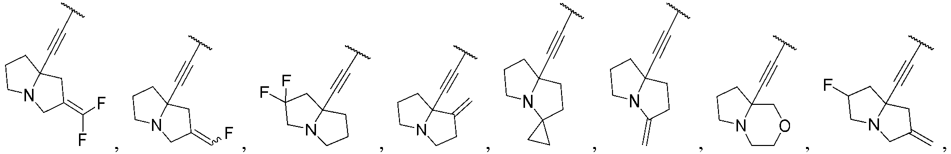 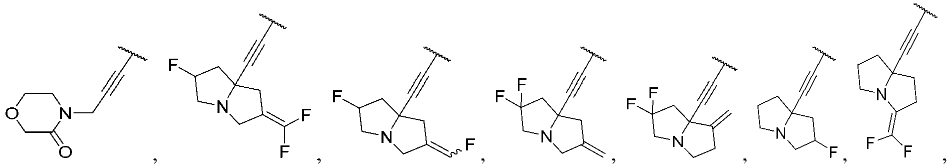 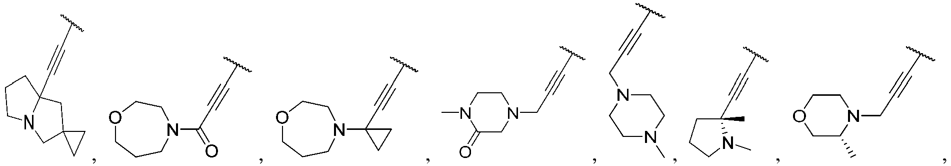 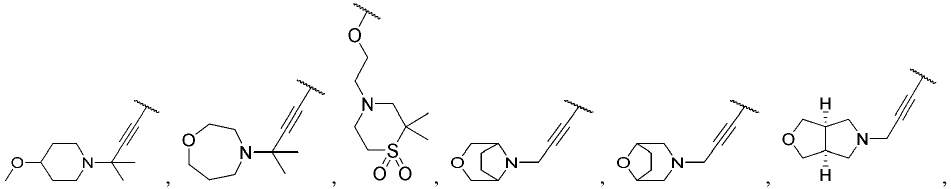 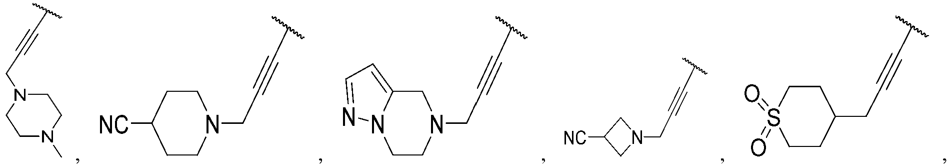 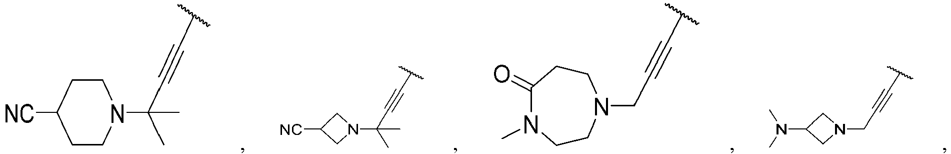 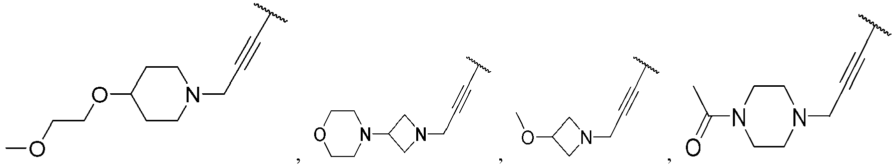 , and

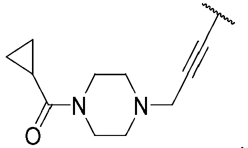

In one embodiment, R₅ is selected from 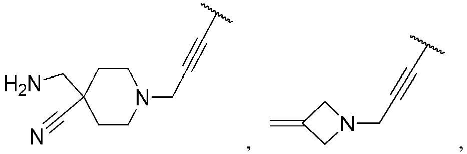 and

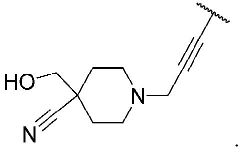

In one embodiment, R₃ is hydrogen, C₁₋₆ alkyl, C₁₋₆ haloalkyl, deuterated C₁₋₆ alkyl, or C₃₋₆ cycloalkyl.

In one embodiment, R₃ is hydrogen, C₁₋₆ alkyl, C₁₋₆ haloalkyl, or deuterated C₁₋₆ alkyl.

In one embodiment, R₃ is hydrogen, C₁₋₃ alkyl, C₁₋₃ haloalkyl, or deuterated C₁₋₃ alkyl.

In one embodiment, R₃ is ethyl, isopropyl, or cyclopentyl.

In one embodiment, R₃ is trifluoroethyl.

In one embodiment, R₁₃ is hydrogen or fluorine.

In one embodiment, R₁₃ is hydrogen or methoxy.

In one embodiment, R₁ is hydrogen; R₂ is hydrogen.

In one embodiment, R₄₂ is methyl; R₄₃ is methyl.

In one embodiment, Y₄ is O.

In one embodiment, Y₅ is O.

In one embodiment, R₁₂ is hydrogen.

In one embodiment, R₅ is -NR₀₁R₀₂; R₀₁ and R₀₂, together with the nitrogen atom to which they are attached, form substituted or unsubstituted 3- to 8-membered monocyclic heterocyclyl.

In one embodiment, R₅ is -C₂₋₄ alkynyl-C₁₋₄ alkyl-NR₀₁R₀₂; R₀₁ and R₀₂, together with the nitrogen atom to which they are attached, form substituted or unsubstituted 3- to 8-membered monocyclic heterocyclyl.

One or more (e.g., 1, 2, 3, or 4) ring atoms of the 3- to 8-membered monocyclic heterocyclyl are heteroatoms selected from nitrogen, oxygen, and sulfur; the substitutions each independently refer to that 1, 2, 3, 4, 5, or 6 hydrogen atoms on the group described above are substituted with a group selected from group S1, wherein when the ring atom is a sulfur atom, the sulfur atom is optionally substituted with one or two groups selected from oxo and =NR^{e}; each R^{e} is independently H, halogen, C₁₋₆ alkyl, C₁₋₆ haloalkyl, or deuterated C₁₋₆ alkyl.

In one embodiment, the 3- to 8-membered monocyclic heterocyclyl is selected from 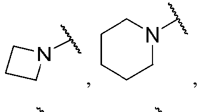 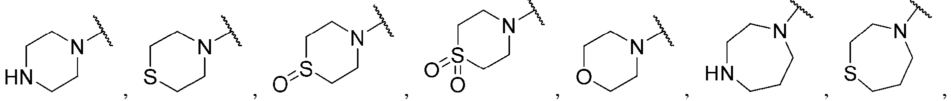 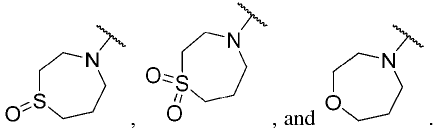 Preferably, the 3-8 membered monocyclic heterocyclyl is unsubstituted or substituted with 1 or 2 groups selected from the following group consisting of: methyl, - CH₂OH, cyano, -C(=O)CH₂OH, and -CH₂NH₂.

In one embodiment, R₅ is selected from 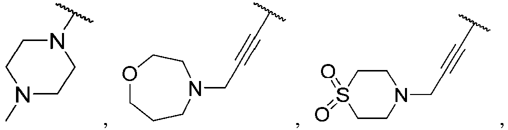 and

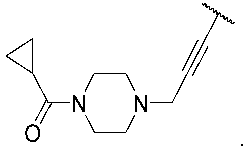

In one embodiment, Y₁ is N; Y₂ is CH.

In one embodiment, Y₁ is CH; Y₂ is N.

In one embodiment, Y₁ is N⁺ -O⁻; Y₂ is CH.

In one embodiment, Y₁ is CH; Y₂ is N⁺-O⁻.

In one embodiment, X is

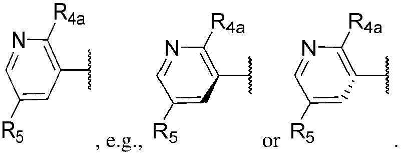

In one embodiment, X is

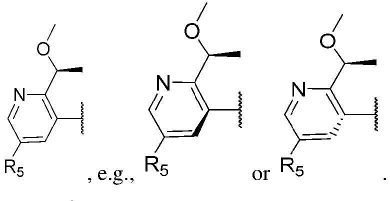

In one embodiment, L₁ is

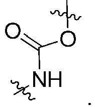

In one embodiment, L₁ is 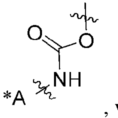 wherein "*A" indicates the linking site to ring A.

In one embodiment, L₁ is

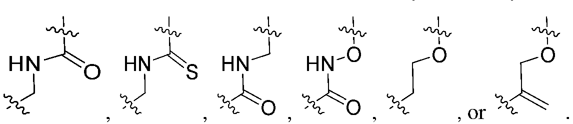

In one embodiment, L₁ is 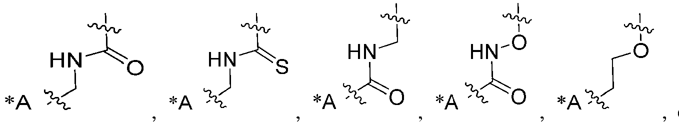 or 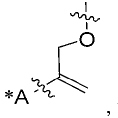 wherein "*A" indicates the linking site to ring A.

In one embodiment, L₁ is

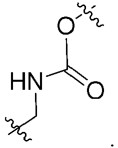

In one embodiment, L₁ is 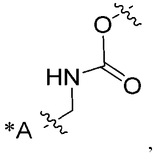 wherein "*A" indicates the linking site to ring A.

In one embodiment, L₁ is -L₂-substituted or unsubstituted C₆₋₁₀ aryl-; L₂ is -O-, -C₁₋₄ alkyl-, -N(R₀₀)-, -C(=O)-, -C=C-, or -N(R₀₀)C(=S)-. In one embodiment, the C₆₋₁₀ aryl is phenyl or naphthyl.

In one embodiment, L₁ is -L₂-substituted or unsubstituted 5- or 6-membered monocyclic heteroaryl-; L₂ is - O-, -C₁₋₄ alkyl-, -N(R₀₀)-, -C(=O)-, -C=C-, or -N(R₀₀)C(=S)-. In one embodiment, the 5- or 6-membered monocyclic heteroaryl is thiazole or oxazole.

In one embodiment, L₁ is -L₂-substituted or unsubstituted 3- to 8-membered heterocyclyl-; L₂ is -O-, -C₁₋₄ alkyl-, -N(R₀₀)-, -C(=O)-, -C=C-, or -N(R₀₀)C(=S)-. In one embodiment, the 3- to 8-membered heterocyclyl is selected from 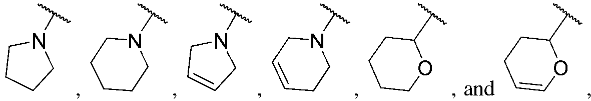 wherein the wavy line indicates that the ring atom is one linking position, and the other linking position may be any position of the remaining ring atoms.

In one embodiment, L₁ is selected from

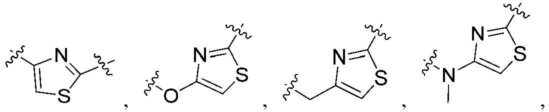

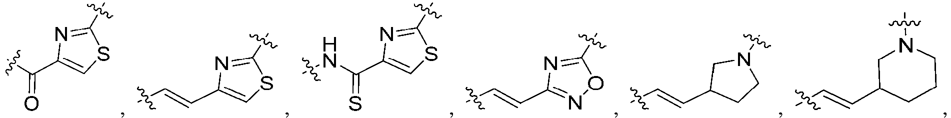

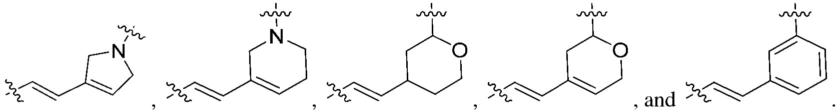

In one embodiment, L₁ is selected from 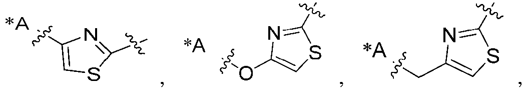 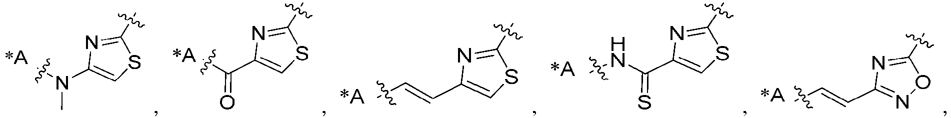 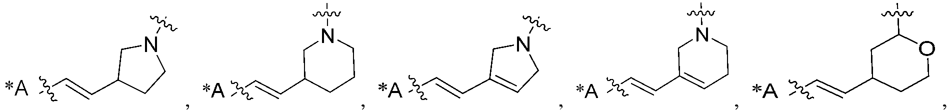 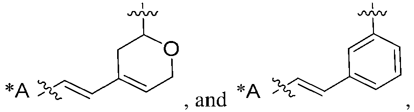 wherein "*A" indicates the linking site to ring A.

In one embodiment, L₁ is selected from 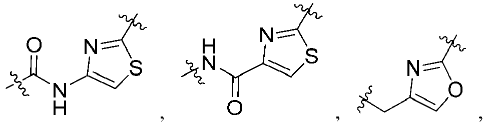 and

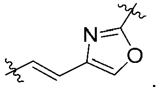

In one embodiment, L₁ is selected from 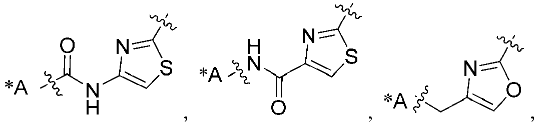 and 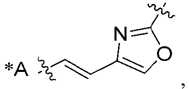 wherein "*A" indicates the linking site to ring A.

In one embodiment, R₆ is substituted or unsubstituted C₁₋₆ alkyl, substituted or unsubstituted C₁₋₆ heteroalkyl, substituted or unsubstituted C₃₋₆ cycloalkyl, or substituted or unsubstituted 3- to 8-membered heterocyclyl; the substitutions are as defined herein.

In one embodiment, the C₁₋₆ alkyl is isopropyl, methyl, sec-butyl, isobutyl, n-propyl, ethyl, pentyl, neopentyl, or tert-pentyl.

In one embodiment, the C₁₋₆ heteroalkyl is

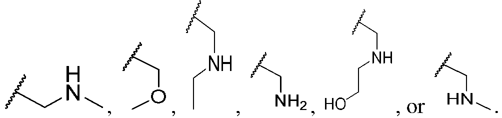

In one embodiment, the C₃₋₆ cycloalkyl is cyclopropyl.

In one embodiment, the 3- to 8-membered heterocyclyl is azetidinyl, thietanyl, tetrahydropyrrolyl, a cyclopropane-fused tetrahydrofuran ring, a cyclopropane-fused tetrahydropyrrole ring, or a cyclopropane-fused tetrahydrothiophene ring.

In one embodiment, the substitutions each independently refer to that the groups described above are each independently substituted with 1, 2, 3, 4, 5, or 6 groups selected from group S1; the groups of group S1 are each independently selected from the following group consisting of: deuterium, oxo (=O), =NR^{e}, halogen, C₁₋₆ alkyl, C₁₋₆ alkoxy, C₁₋₆ haloalkyl, C₃₋₆ cycloalkyl, 3- to 8-membered heterocyclyl, 5- or 6-membered monocyclic heteroaryl, 8- to 10-membered bicyclic heteroaryl, -O-C₃₋₆ cycloalkyl, phenyl, -C₁₋₄ alkyl-C₃₋₆ cycloalkyl, -C₁₋₄ alkyl-phenyl, and -NR^{a1}R^{b1}, wherein the C₁₋₆ alkyl and the C₁₋₆ alkoxy are each independently and optionally substituted with 1, 2, or 3 groups selected from halogen, deuterium, cyano, and hydroxy; the C₃₋₆ cycloalkyl, the 3- to 8-membered heterocyclyl, the phenyl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H or C₁₋₆ alkyl;
in the groups described above, the groups of group S2 are each independently selected from the following group consisting of: halogen, C₁₋₆ alkyl, C₁₋₆ alkoxy, -NH₂, -NHC₁₋₆ alkyl, -(C=O)C₁₋₆ alkyl, -SF₅, and -CH₂-hydroxy;
in the groups described above, each R^{e} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, R₆ is selected from

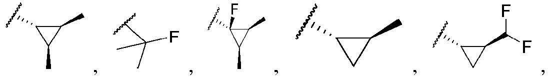

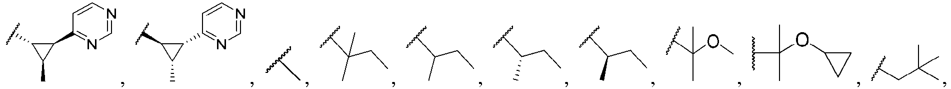

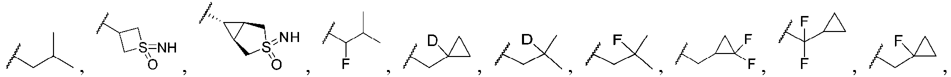

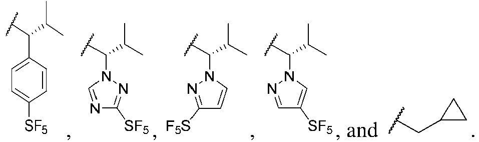

In one embodiment, R₆ is selected from

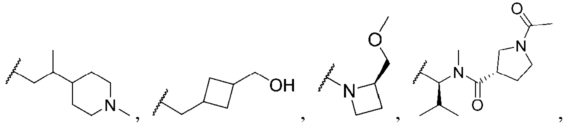

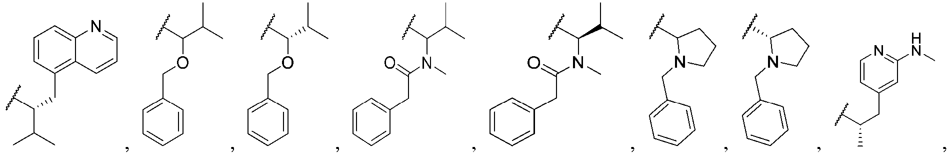

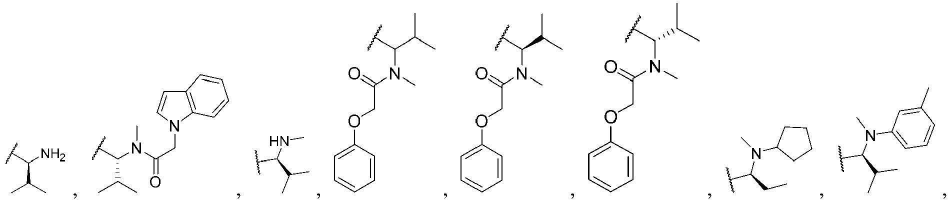

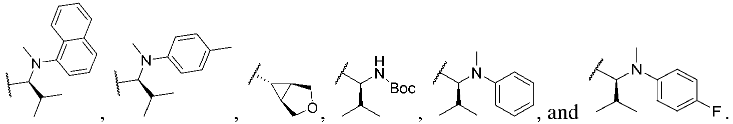

In one embodiment, R₁₁ is selected from

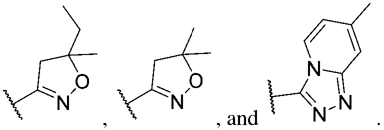

In one embodiment, R₆ is substituted or unsubstituted C₃₋₆ cycloalkyl, wherein the substitution is as defined in any one of the embodiments herein.

In one embodiment, R₆ is cyclopropyl substituted with C₁₋₆ alkyl. Preferably, R₆ is cyclopropyl substituted with methyl. Preferably, R₆ is cyclopropyl substituted with two methyl groups. For example, R₆ is

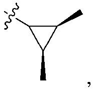

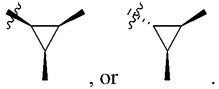

In one embodiment, in the above formulas, 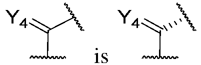 . In one embodiment, when Y₄ is oxygen, it is

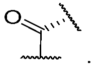

In one embodiment, in the above formulas, 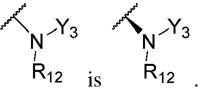 In one embodiment, when Y₃ is C(=O)R₆, it is

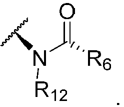

In one embodiment, the compound is selected from Table C:

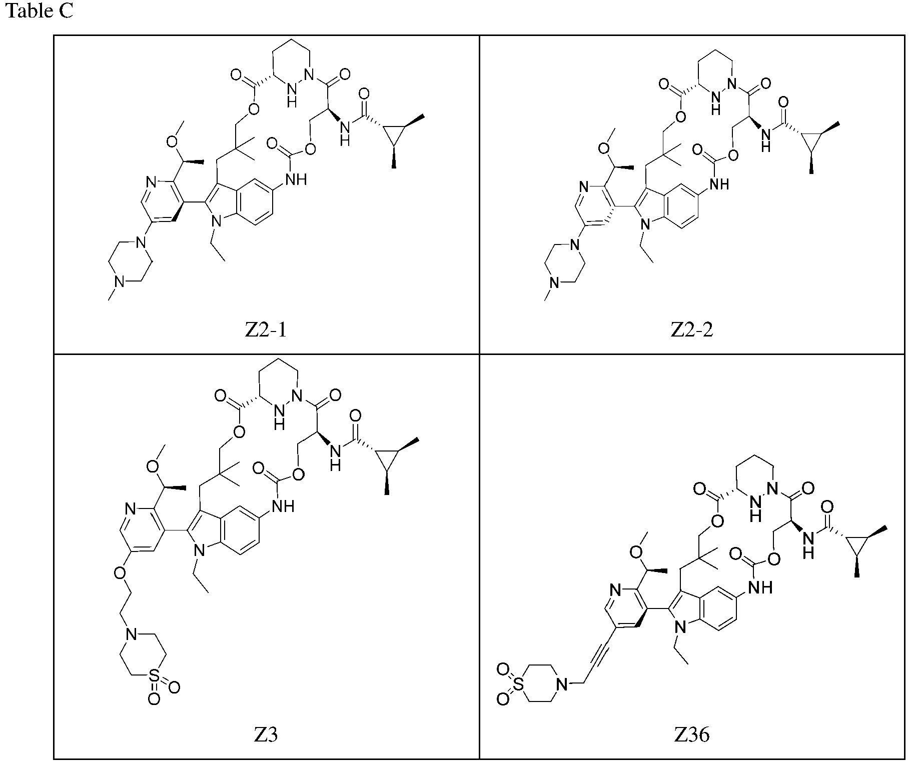

In one embodiment, the compound is selected from Table B:

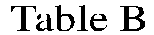

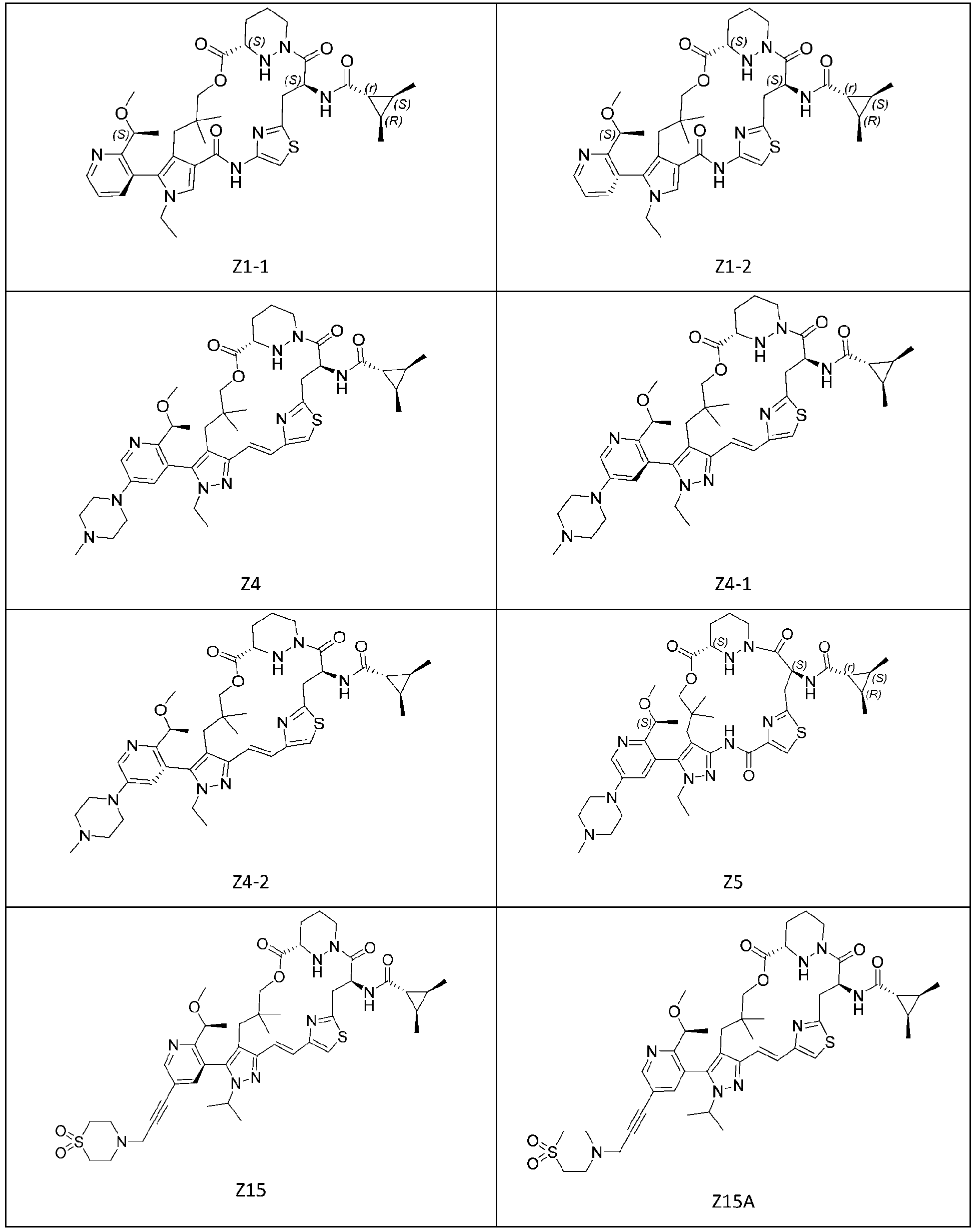

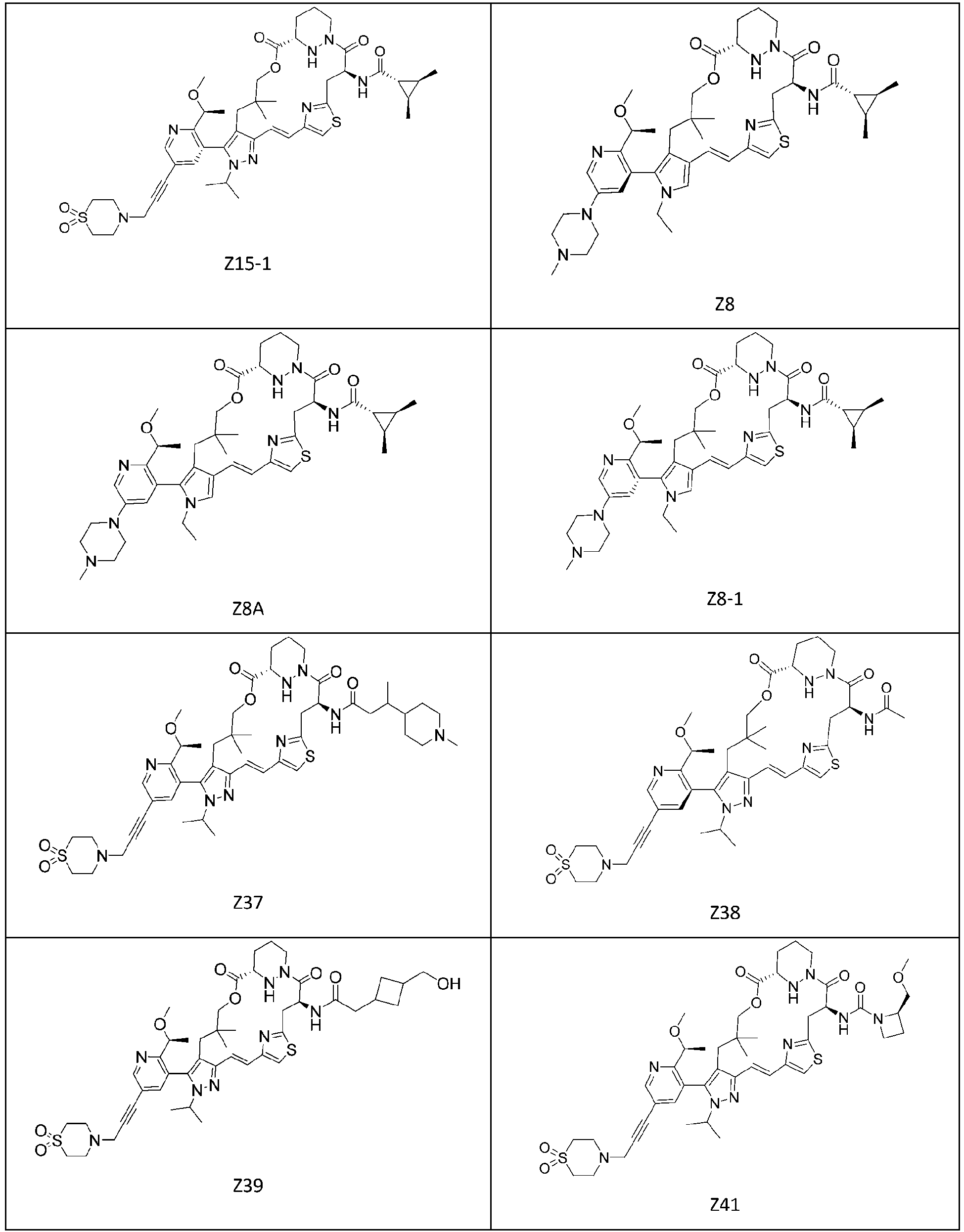

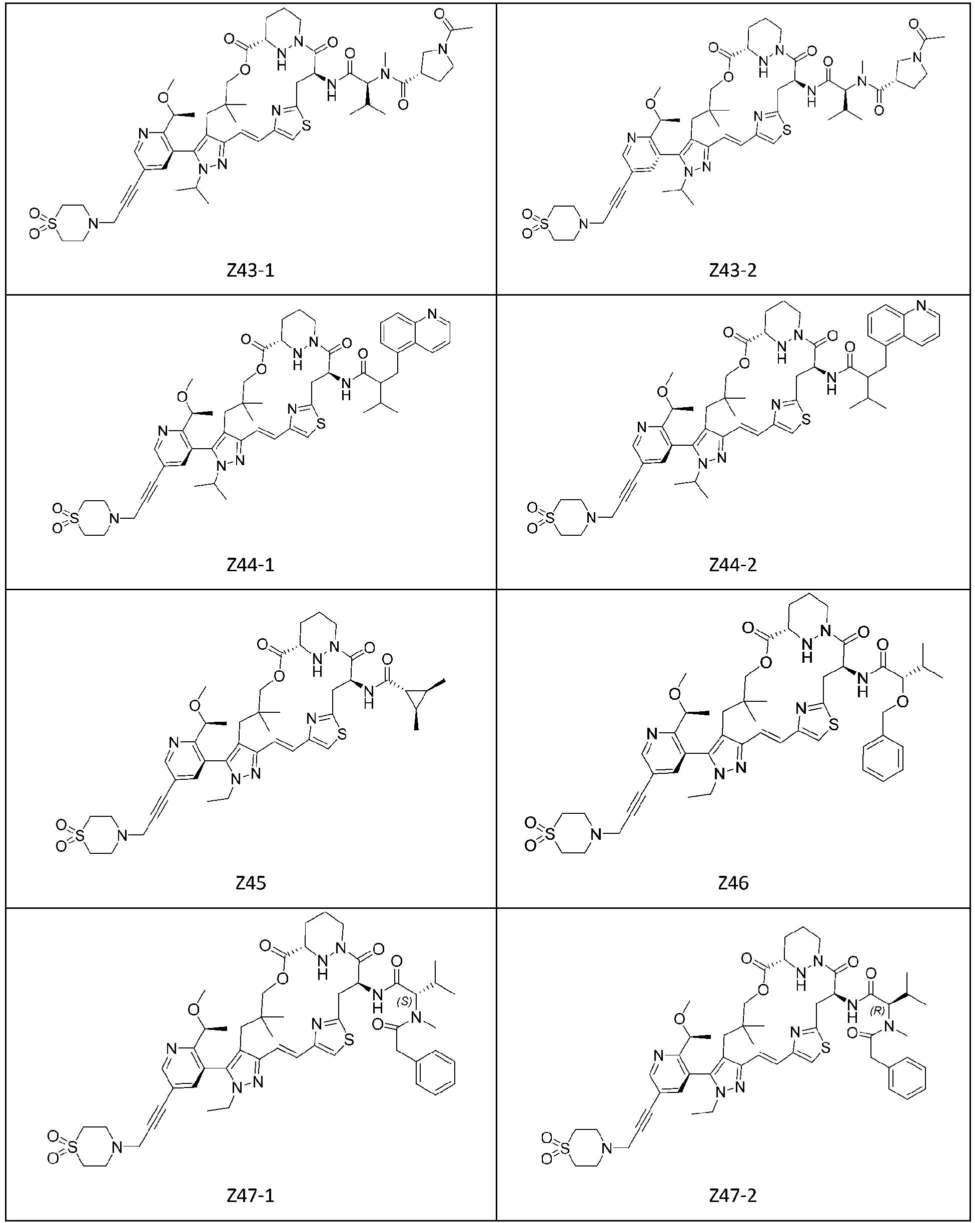

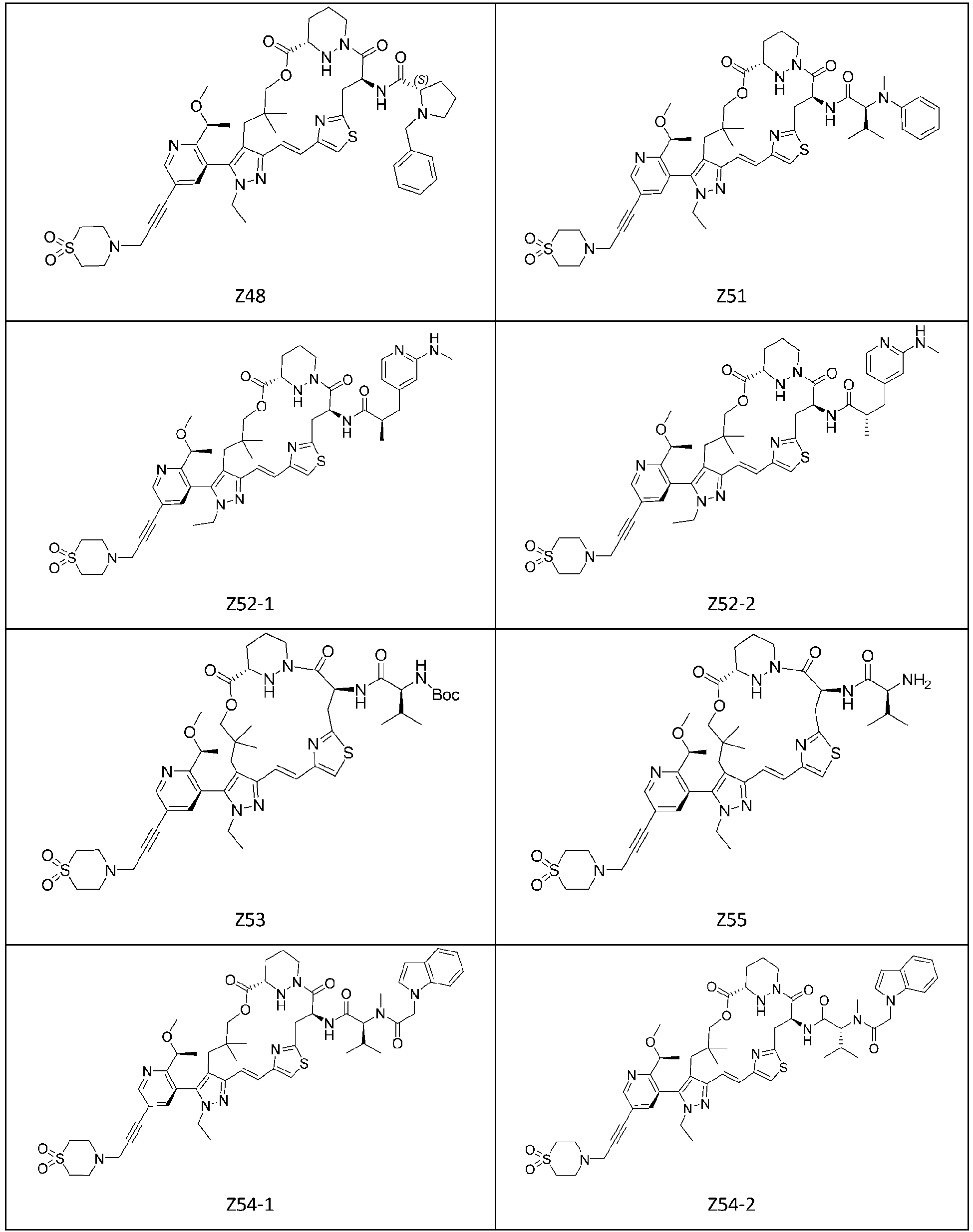

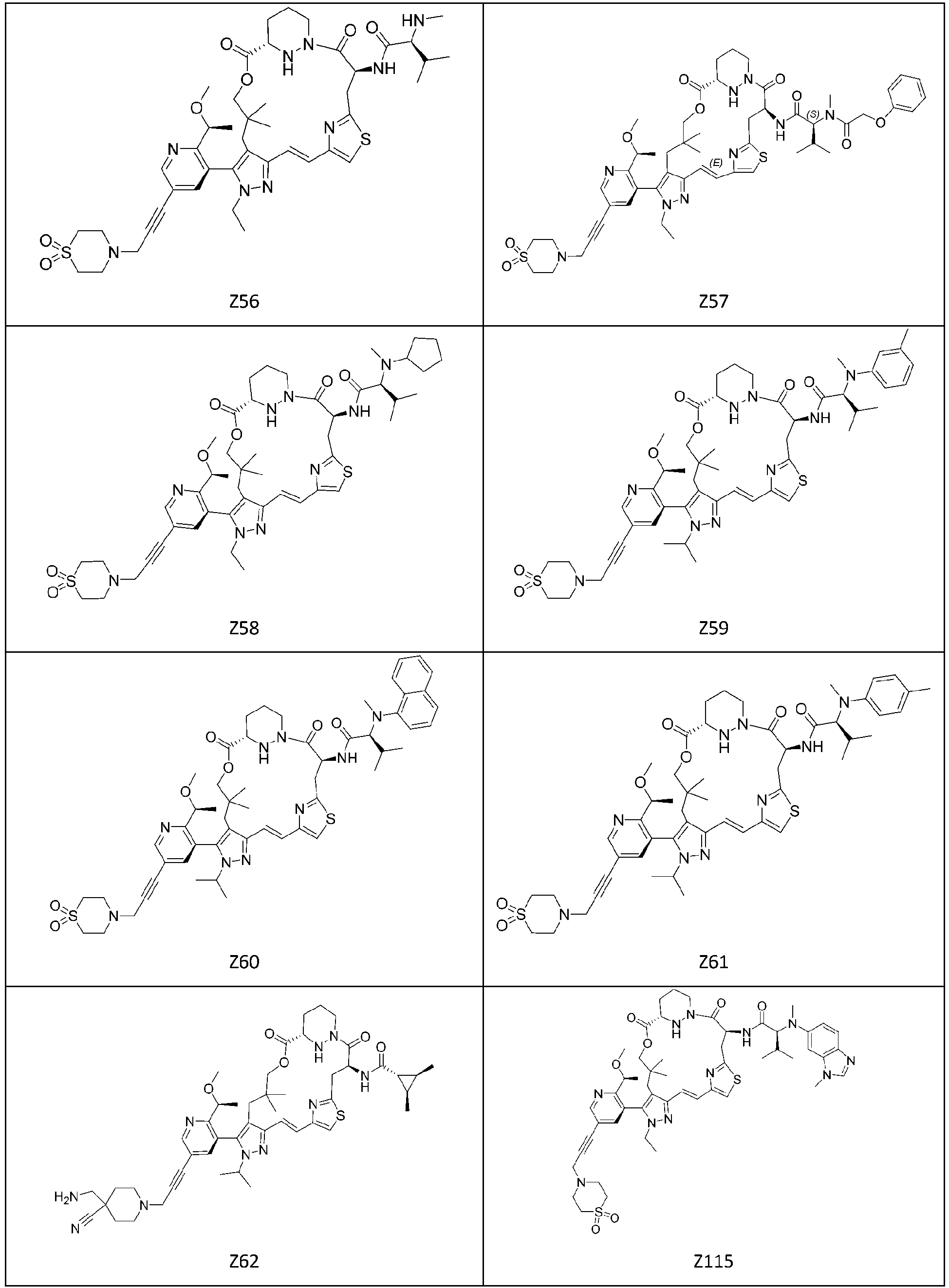

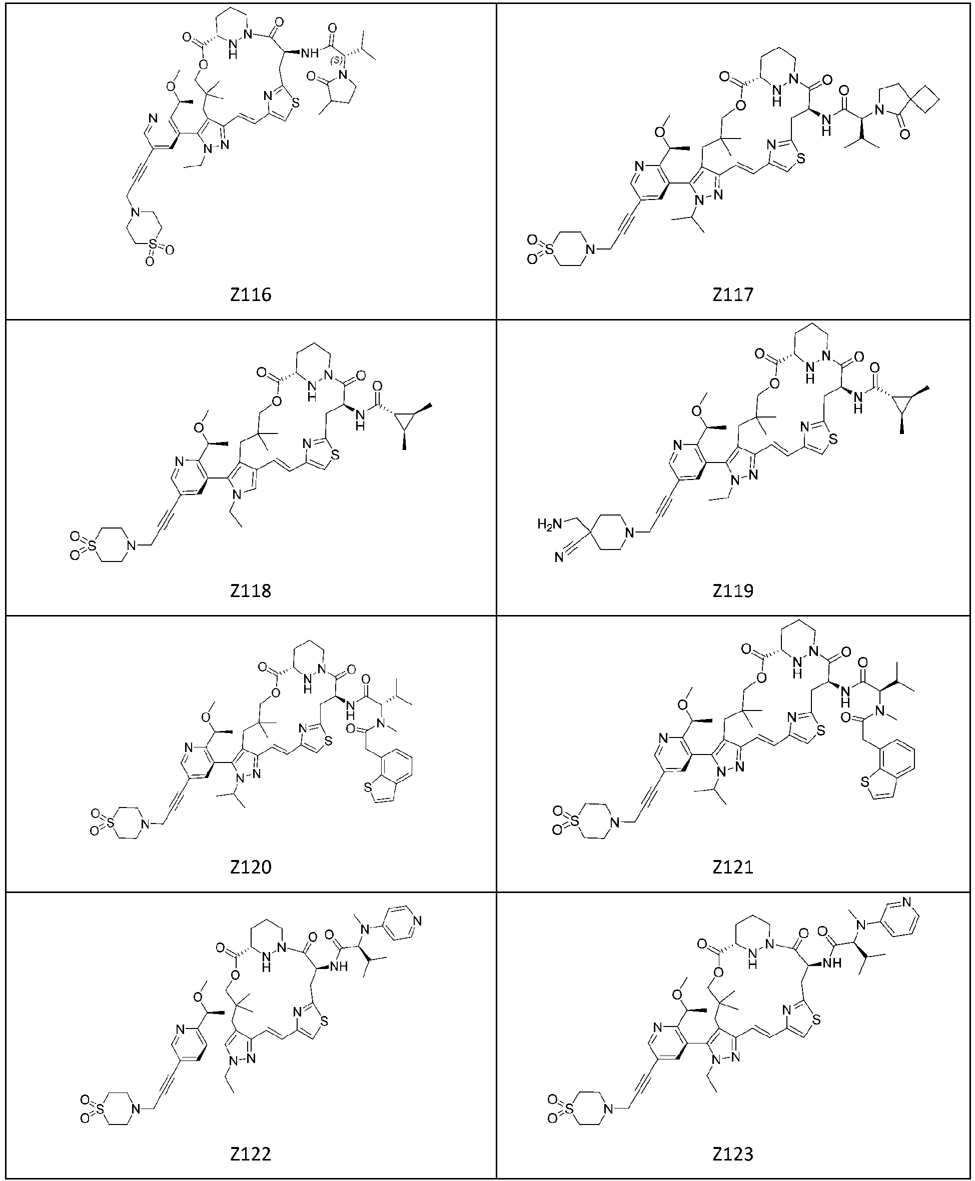

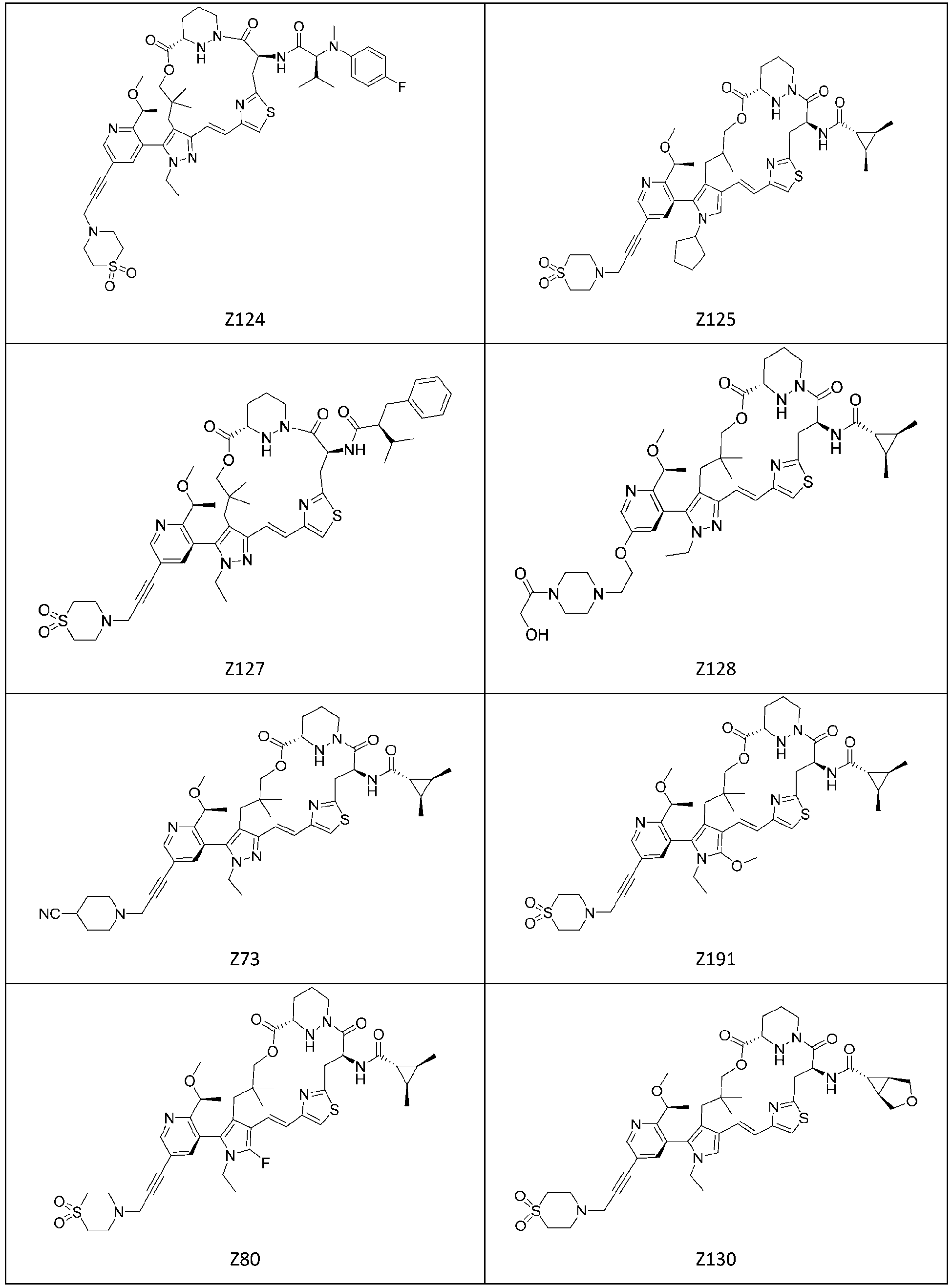

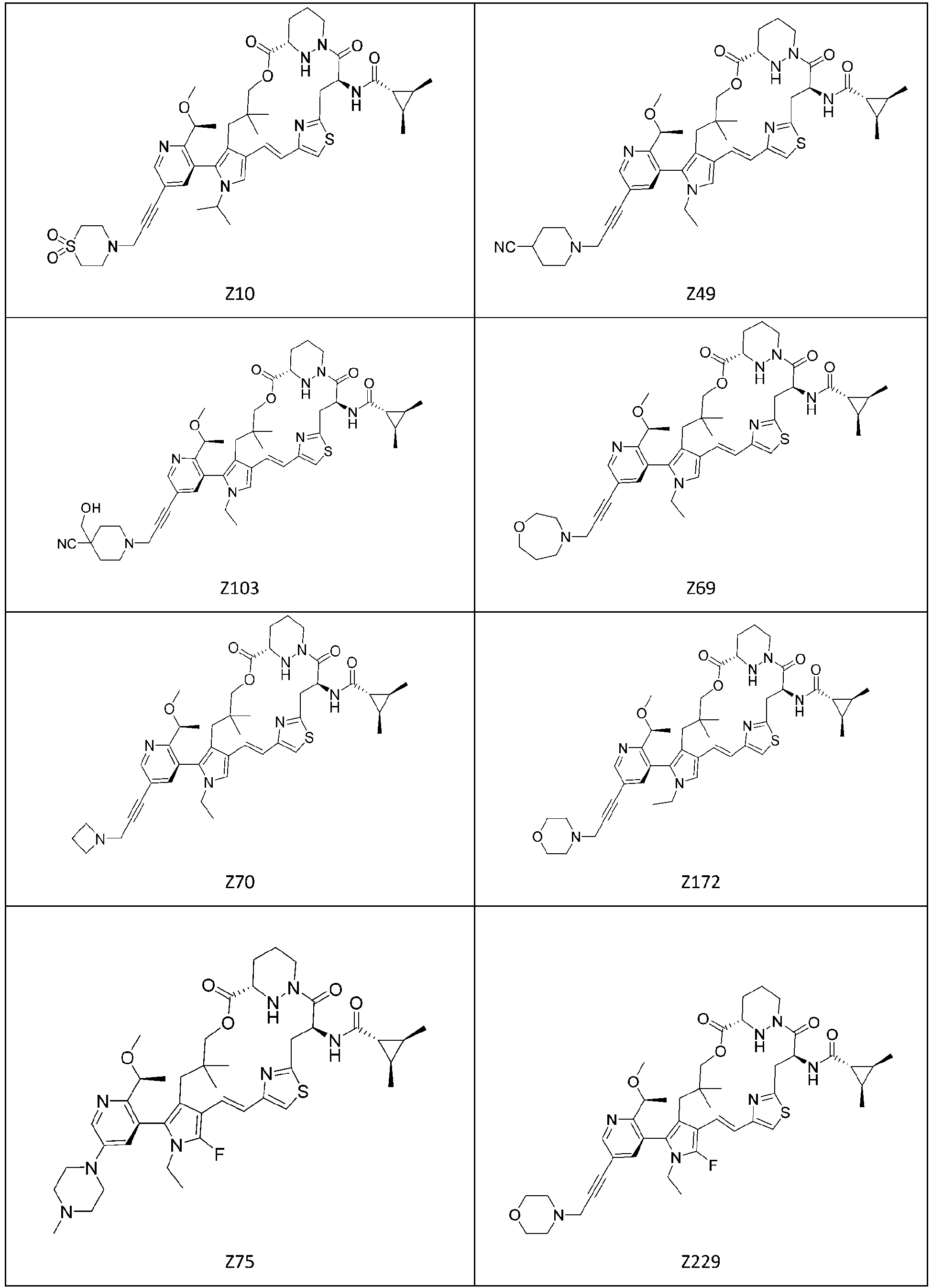

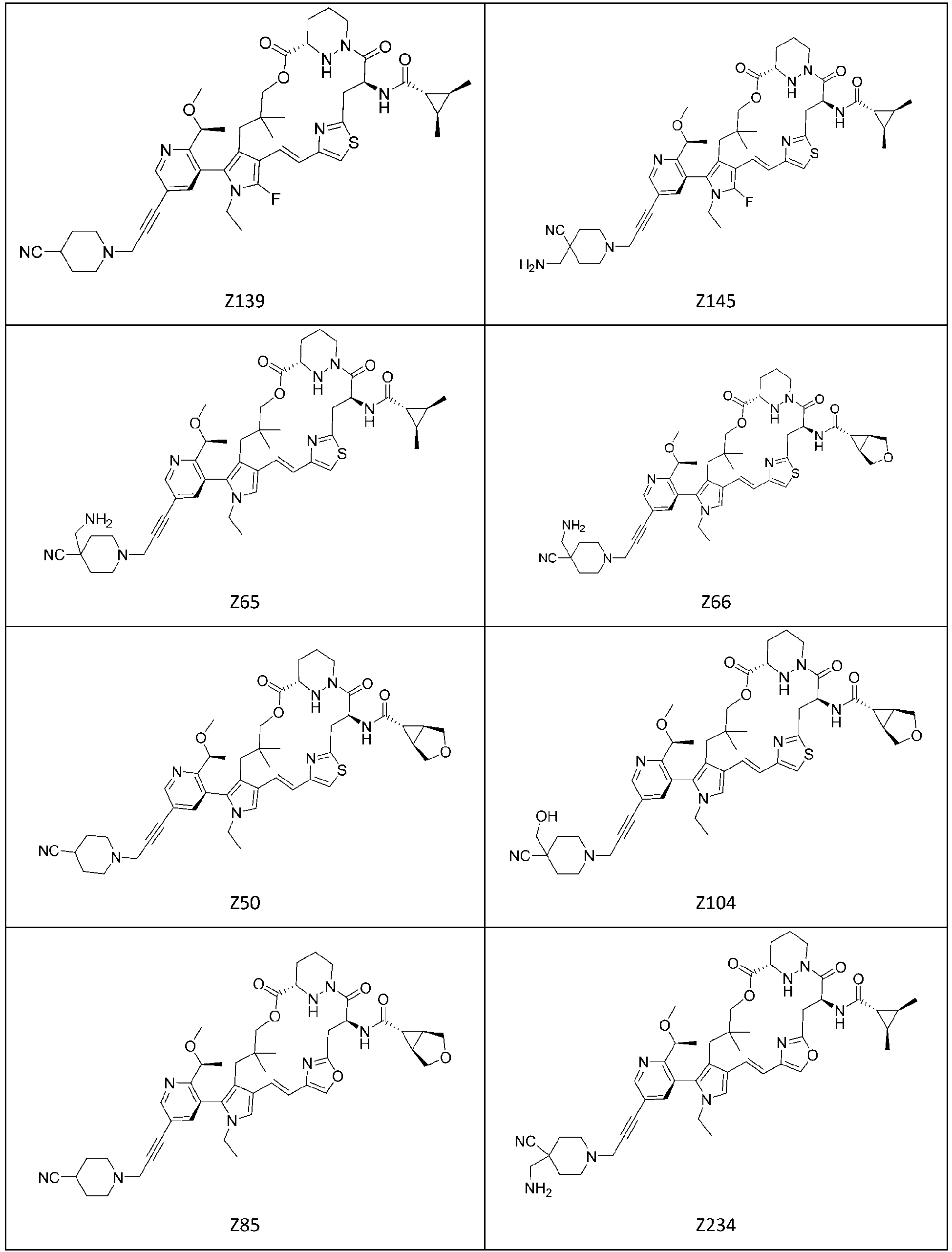

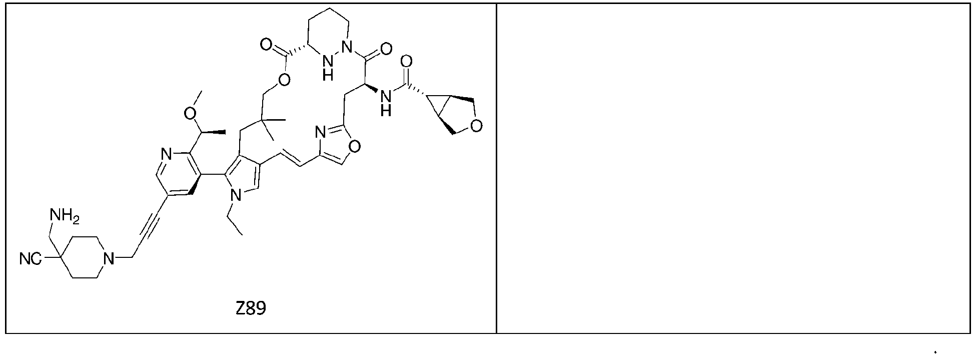

In one embodiment, the compound is selected from Table A.

The present disclosure further provides the following preparation methods, wherein the compounds shown herein can be prepared using synthetic methods known in the art or using methods known in the art in combination with the methods described in the present disclosure.

The present disclosure provides a method for preparing intermediate 1 as follows:

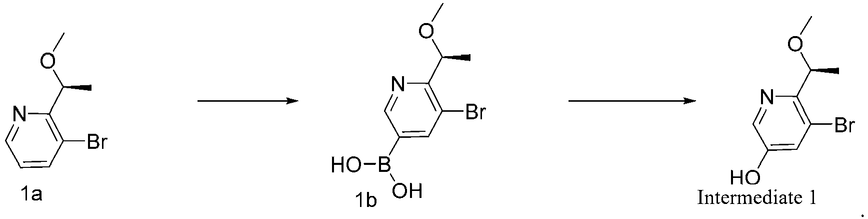

The present disclosure provides a method for preparing intermediate 4-1 as follows:

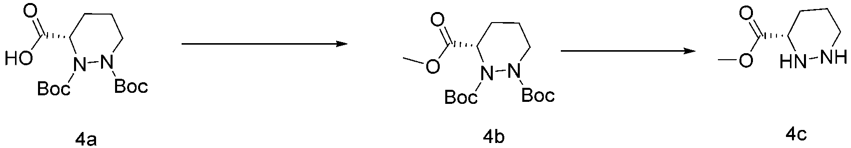

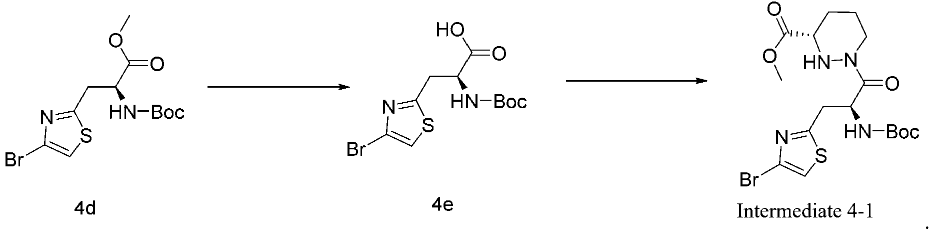

The present disclosure provides a method for preparing intermediate 10-1 as follows:

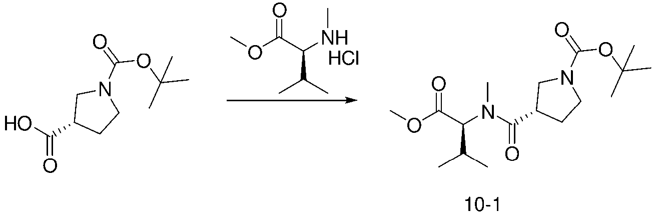

The present disclosure provides a method for preparing intermediate 11 as follows:

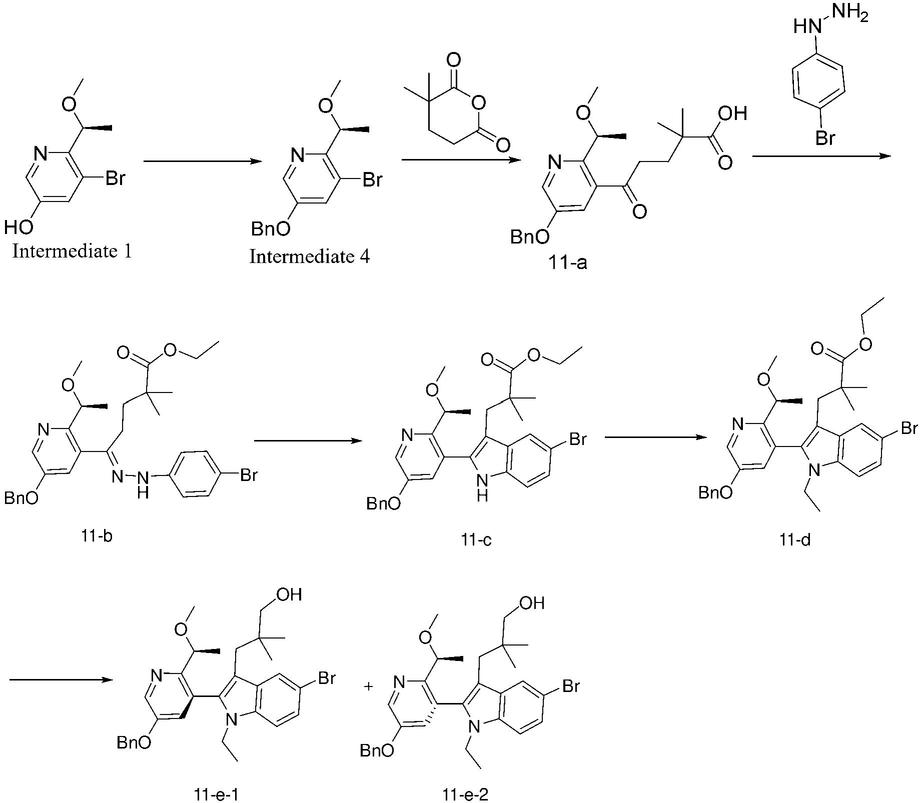

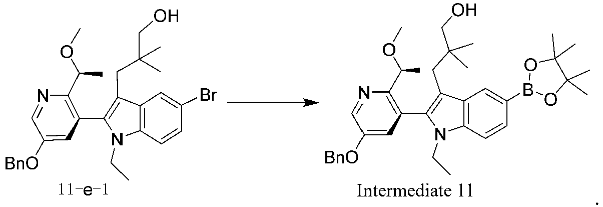

The present disclosure provides a method for preparing intermediate 15-2 as follows:

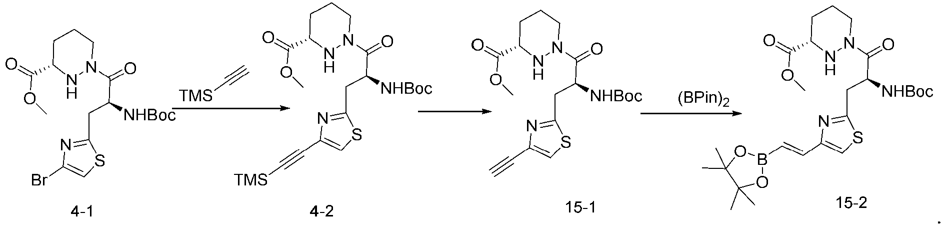

The present disclosure provides a method for preparing intermediate 62-1 as follows:

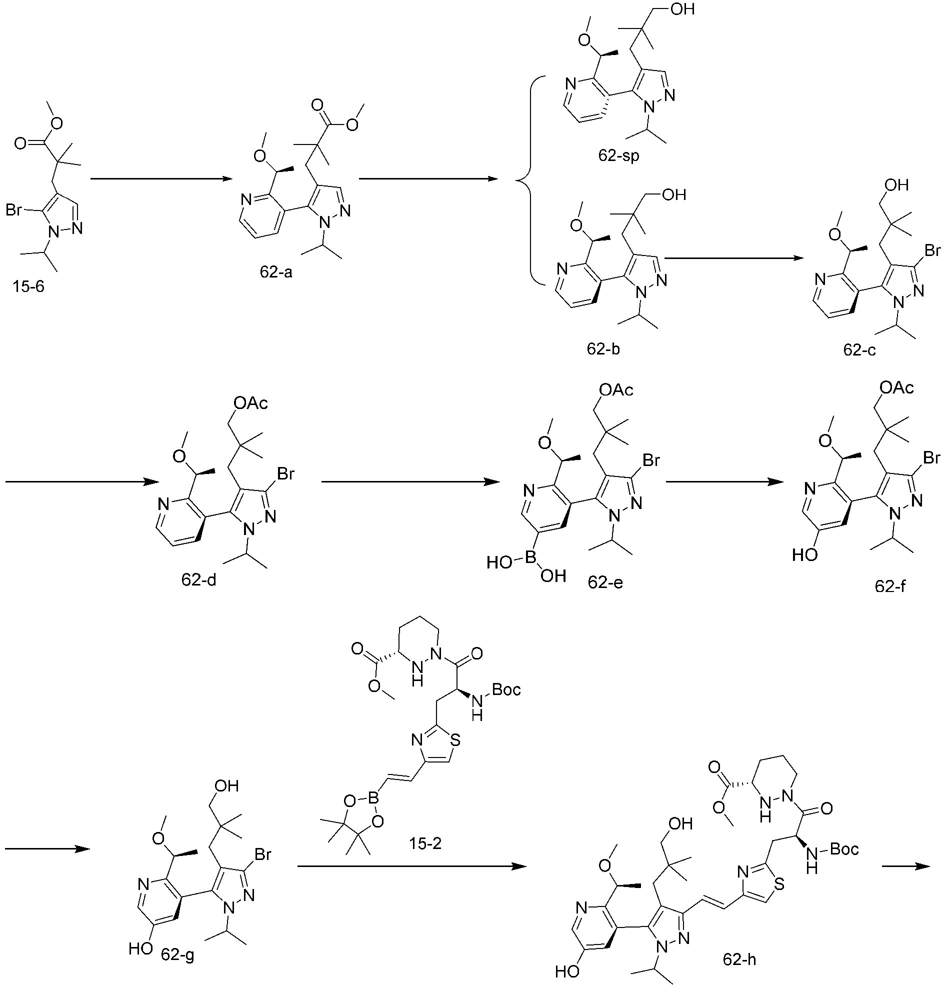

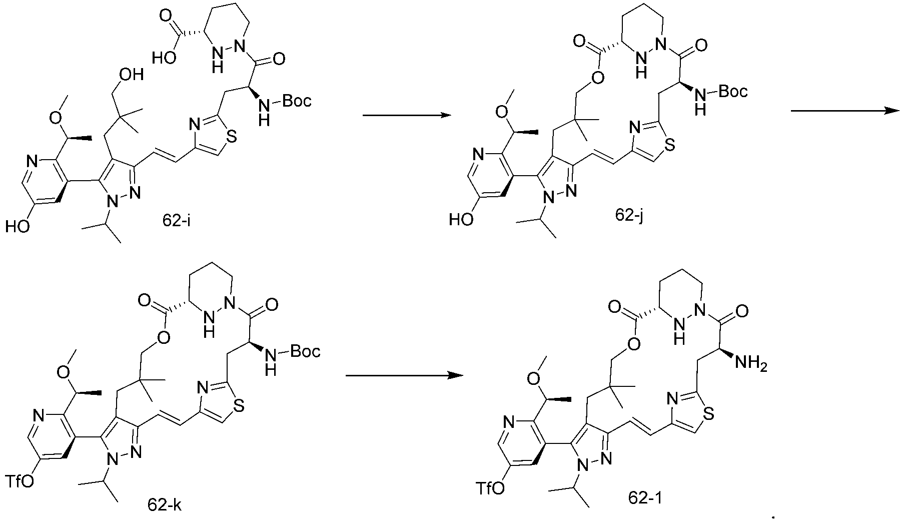

The present disclosure provides a method for preparing intermediate 62-p as follows:

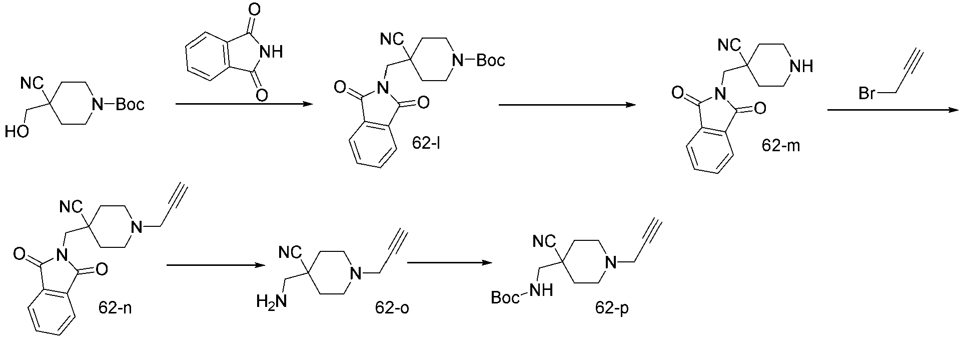

The present disclosure provides a method for preparing intermediate 8-8 as follows:
synthetic route 1: 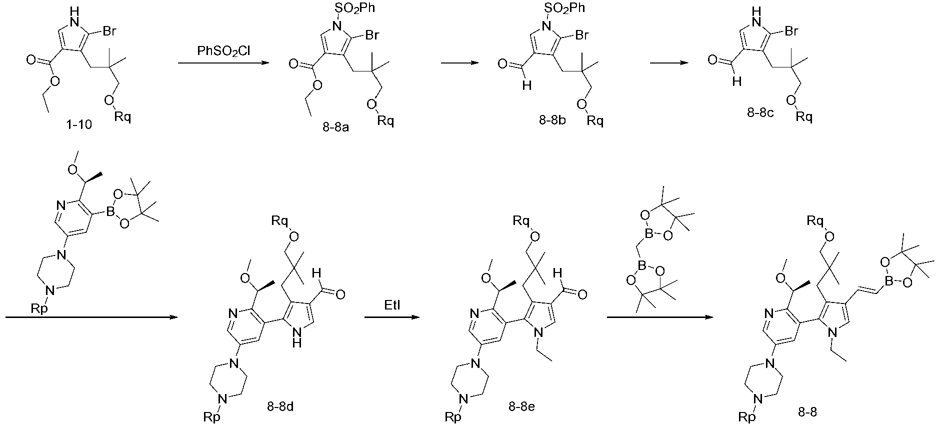 ;
synthetic route 2:

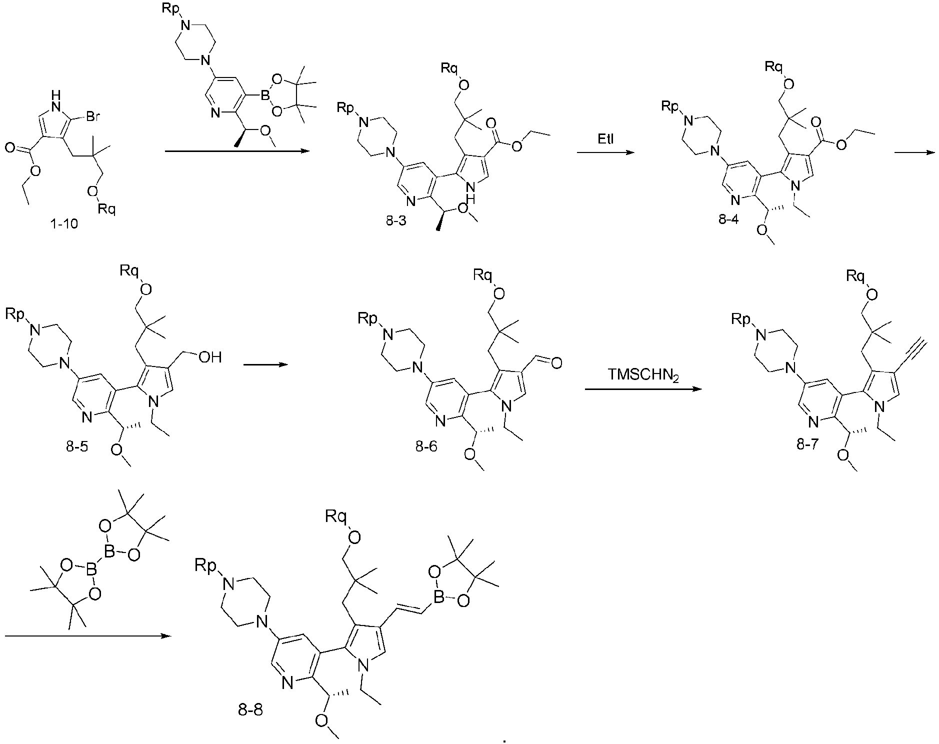

Rₚ is an amino protecting group well known in the art, such as formyl; acyl, such as alkanoyl (such as acetyl, trichloroacetyl, or trifluoroacetyl); alkoxycarbonyl, such as tert-butoxycarbonyl (Boc); arylmethoxycarbonyl, such as carbobenzoxy (Cbz) and 9-fluorenylmethoxycarbonyl (Fmoc); arylmethyl, such as benzyl (Bn), trityl (Tr), or 1,1-di-(4'-methoxyphenyl)methyl; and silyl, such as trimethylsilyl (TMS) and tert-butyldimethylsilyl (TBS).

R_{q} is a hydroxy protecting group well known in the art, such as an ether protecting group (methyl ether (ROMe), tert-butyl ether (ROtBu), benzyl ether (ROBn), p-methoxybenzyl ether (ROPMB), or trityl ether (ROTr)); a silyl ether protecting group (trimethylsilyl (TMS), triethylsilyl (TES), triisopropylsilyl (TIPS), tert-butyldimethylsilyl (TBS), tert-butyl(methoxy)diphenylsilyl (TBDPS), methoxymethyl ether (MOM), tetrahydropyranyl ether (THP), etc.); and an ester protecting group (acetyl (Ac), benzoyl (Bz), pivaloyl (Piv), etc.).

The compounds represented by formula (I), formula (A), and formula (B) of the present disclosure can be prepared using synthetic methods known in the art or using methods known in the art in combination with the methods described in the present disclosure. The solvents, temperatures, and other reaction conditions given in the present disclosure are exemplary and may vary according to methods well known in the art. The compounds in the examples described in the present disclosure can be synthesized, based on their specific structures, using appropriate starting materials according to the methods described in the examples, or can be synthesized using methods similar to those described in the examples. The starting materials for the synthesis of the compounds in the examples of the present disclosure may be prepared by known synthetic methods or similar methods described in the literature, or obtained from commercial sources. The compounds of the examples may be further resolved as needed to obtain stereoisomers thereof by methods well known in the art, such as crystallization and chromatography.

As a further illustration, the compounds of formula (B5-1) and formula (B5-2) of the present disclosure can be synthesized using the following methods, wherein the solvent, temperature, and other reaction conditions in each step may be the same as or similar to those described in the following examples, or reaction conditions known in the art are used;

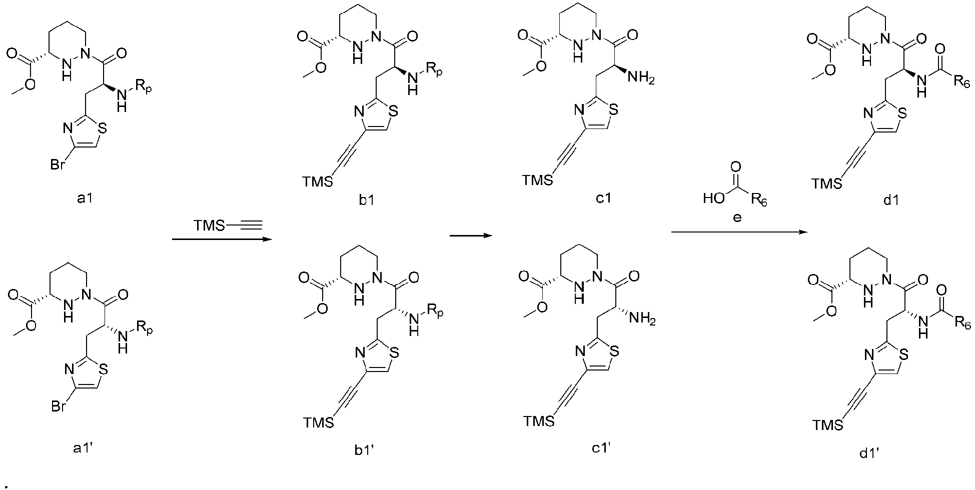

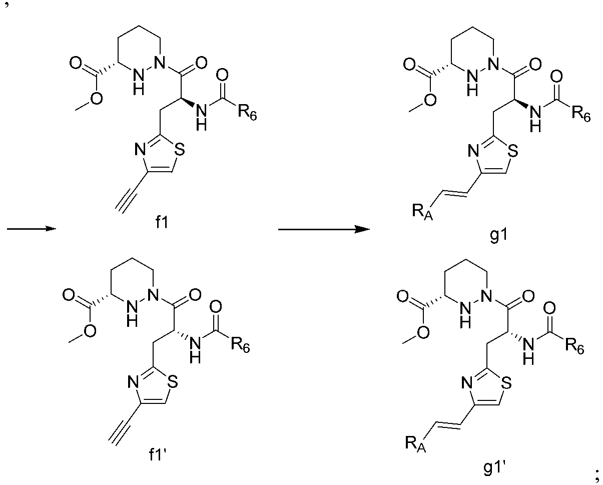

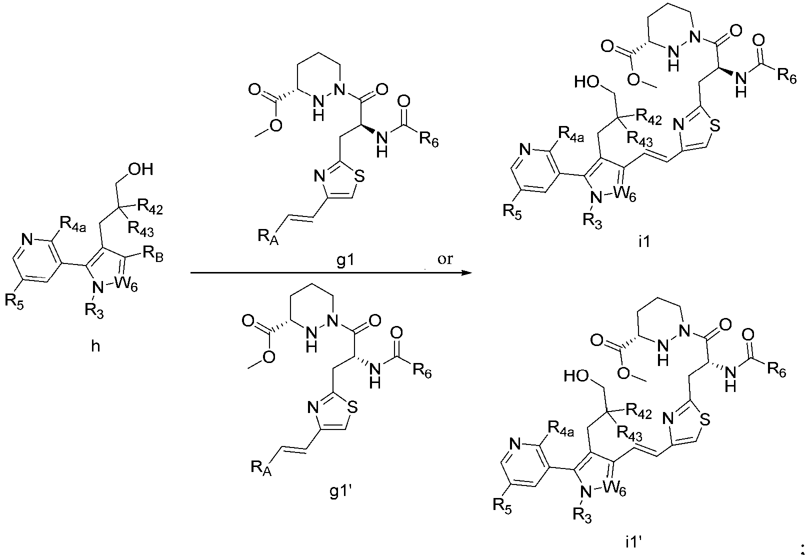

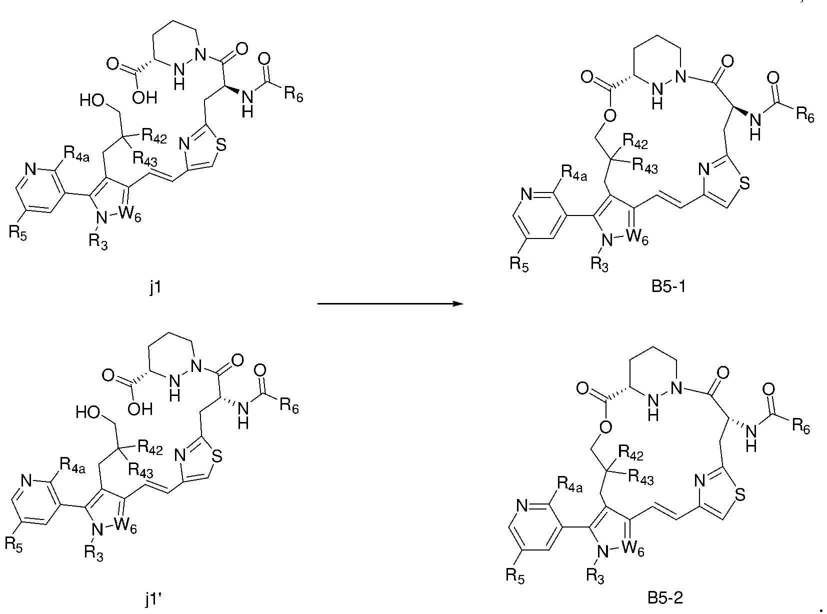

In the preparation routes for the compounds of formulas (B5-1) and formula (B5-2), in each formula, Rₚ is an amino protecting group well known in the art, such as formyl; acyl, such as alkanoyl (such as acetyl, trichloroacetyl, or trifluoroacetyl); alkoxycarbonyl, such as tert-butoxycarbonyl (Boc); arylmethoxycarbonyl, such as carbobenzoxy (Cbz) and 9-fluorenylmethoxycarbonyl (Fmoc); arylmethyl, such as benzyl (Bn), trityl (Tr), or 1,1-di-(4'-methoxyphenyl)methyl; and silyl, such as trimethylsilyl (TMS) and tert-butyldimethylsilyl (TBS).

R_{A} and R_{B} are each independently a halogen atom (e.g., chlorine or bromine), a boric acid group (e.g., - B(OH)₂), or a boronic acid ester group (e.g., 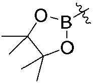 ), provided that when R_{A} is selected from a halogen atom, R_{B} is selected from a boric acid group and a boronic acid ester group, and when R_{B} is selected from a halogen atom, R_{A} is selected from a boric acid group and a boronic acid ester group.

W₆, R₃, R₄₂, R₄₃, R₄ₐ, R₅, and R₆ are each as defined in any one of the embodiments of the present disclosure (e.g., as defined for the corresponding group in formula I or formula B5).

The synthetic route of the compound of formula (B5-1) or formula (B5-2) described above is exemplary, and in the specific preparation process, those skilled in the art can adjust the order of the steps in the reaction route according to the prior art and the structure of the target compound.

A second aspect of the present disclosure provides a pharmaceutical composition comprising the compound, or the stable deuterated derivative thereof, or the stereoisomer thereof, or the pharmaceutically acceptable salt thereof, the solvate thereof, or the prodrug thereof according to the first aspect described above, and a pharmaceutically acceptable carrier.

A third aspect of the present disclosure provides the aforementioned compound as a medicament.

As used herein, the term "pharmaceutically acceptable carrier" refers to any formulation or carrier medium capable of delivering an effective amount of the active substance of the present disclosure without interfering with the biological activity of the active substance and having no toxic and side effects on the host or subject. Representative carriers include water, oils, vegetables and minerals, cream bases, lotion bases, ointment bases, and the like. Such bases include suspending agents, thickeners, transdermal enhancers, and the like. Their formulations are well known to those skilled in the cosmetic or topical pharmaceutical field.

In the embodiments of the present disclosure, the pharmaceutical composition may be administered in any of the following ways: oral administration, spray inhalation, rectal administration, nasal administration, buccal administration, topical administration, parenteral administration (e.g., subcutaneous, intravenous, intramuscular, intraperitoneal, intrathecal, intraventricular, intrasternal, and intracranial injection or infusion), or administration by means of an implantable reservoir. Oral, intraperitoneal, or intravenous administration is preferred. Aqueous suspension formulations are generally used by mixing the active ingredient with suitable emulsifying and suspending agents. If needed, sweetening, flavoring, or coloring agents may be added to the above oral formulations. When being administered topically, especially for treating affected areas or organs that are easily accessible for topical external application, such as eyes, skin, or lower intestinal neurological diseases, the compounds of the present disclosure may be formulated into different formulation forms for topical administration according to different affected areas or organs. When being administered topically to the eye, the compounds of the present disclosure may be formulated into a formulation form of a micronized suspension or solution, and the carrier used is isotonic sterile saline with a certain pH, with or without the addition of a preservative such as chlorinated benzyl alkanolate. For ophthalmic use, the compound may also be formulated into ointments, such as vaseline. When being administered topically to the skin, the compounds of the present disclosure may be formulated into a formulation form of a suitable ointment, lotion, or cream, in which the active ingredient is suspended or dissolved in one or more carriers.

Carriers that can be used in the ointment formulations include, but are not limited to: mineral oil, liquid vaseline, white vaseline, propylene glycol, polyethylene oxide, polypropylene oxide, emulsifying wax, and water; carriers that can be used in the lotions or creams include, but are not limited to: mineral oil, sorbitan monostearate, Tween 60, cetyl ester wax, hexadecene aryl alcohol, 2-octyldodecanol, benzyl alcohol, and water. The compounds of the present disclosure may also be administered in the form of a sterile injectable formulation, including a sterile injectable aqueous or oil suspension or a sterile injectable solution. The carriers and solvents that may be used include water, Ringer's solution, and isotonic sodium chloride solution. In addition, sterilized non-volatile oils may also be used as solvents or suspending media, such as monoglycerides or diglycerides.

Another aspect of the present disclosure provides use of the compound, or the stable deuterated derivative or stereoisomer thereof, or the pharmaceutically acceptable salt, solvate or prodrug thereof according to the first aspect described above, or the pharmaceutical composition according to the second aspect described above in the manufacture of a medicament for preventing and/or treating a disease or disorder, wherein the disease or disorder is a disease or disorder associated with RAS protein activity.

Another aspect of the present disclosure provides a method for preventing and/or treating a disease or disorder associated with RAS protein activity, the method comprising: administering to a subject in need a therapeutically effective amount of the compound, or the stable deuterated derivative or stereoisomer thereof, or the pharmaceutically acceptable salt, solvate or prodrug thereof according to the first aspect of the present disclosure; or administering to a subject in need a therapeutically effective amount of the pharmaceutical composition according to the second aspect of the present disclosure.

Herein, in one embodiment, the disease or disorder associated with RAS protein activity is cancer, including but not limited to pancreatic cancer, colorectal cancer, non-small cell lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer.

In one embodiment, the cancer includes wild-type RAS and an RAS mutation. The RAS includes HRAS, KRAS, and NRAS.

In one embodiment, the RAS mutation is a KRAS G12C, KRAS G12D, KRAS G12V, KRAS G12S, KRAS G13C, KRAS G13D, KRAS Q61L, KRAS Q61H, NRAS G12C, NRAS Q61K, or NRAS Q61R mutation, or other RAS mutations.

Herein, the use or method further comprises administering an additional anti-cancer therapy. In some embodiments, the additional anti-cancer therapy is an HER2 inhibitor, an EGFR inhibitor, a second RAS inhibitor, an SHP2 inhibitor, an SOS1 inhibitor, an RAF inhibitor, an MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, or a combination thereof.

Another aspect of the present disclosure provides a method for treating cancer in a subject in need thereof, the method comprising:
(a) determining that the cancer is associated with RAS protein activity; and
(b) administering to the subject a therapeutically effective amount of the compound, or the stable deuterated derivative or stereoisomer thereof, or the pharmaceutically acceptable salt, solvate or prodrug thereof according to the first aspect of the present disclosure; or administering to the subject a therapeutically effective amount of the pharmaceutical composition according to the second aspect of the present disclosure. In one embodiment, the administration is accomplished by a route selected from parenteral, intraperitoneal, intradermal, intracardiac, intraventricular, intracranial, intracerebrospinal, intracavitary, intrasynovial, intrathecal, intramuscular injection, intravitreal injection, intravenous injection, intraarterial injection, oral, buccal, sublingual, transdermal, topical, intratracheal, rectal, subcutaneous, and topical administrations.

Another aspect of the present disclosure provides a method for inhibiting RAS protein activity in a cell, the method comprising: contacting the cell with the compound, or the stable deuterated derivative or stereoisomer thereof, or the pharmaceutically acceptable salt, solvate or prodrug thereof according to the first aspect of the present disclosure; or contacting the cell with the pharmaceutical composition according to the second aspect of the present disclosure. The cell may be *in vivo* or *in vitro.*

Another aspect of the present disclosure provides use of the compound, or the stable deuterated derivative or stereoisomer thereof, or the pharmaceutically acceptable salt, solvate or prodrug thereof according to the first aspect described above, or the pharmaceutical composition according to the second aspect described above in the manufacture of an RAS protein activity inhibitor.

### Definitions and Terms

As used herein, the term "subject" refers to an animal, particularly a mammal. A human is preferred.

As used herein, the term "effective amount" or "therapeutically effective amount" refers to an amount of a medicament or agent that is non-toxic but can achieve the desired effect. In the embodiments of the present disclosure, when treating a patient according to the present disclosure, the amount of a given medicament depends on a number of factors, such as the specific dosing regimen, the type of disease or disorder and its severity, and the uniqueness of the subject or host in need of treatment (e.g., body weight). However, the dose administered can be routinely determined by methods known in the art according to the specific circumstances, including, for example, the specific medicament that has been used, the route of administration, the disorder being treated, and the subject or host being treated. In general, for dosages used for the treatment of adults, the administration dose is typically in the range of 0.02-5000 mg/day, for example, about 1-1500 mg/day. The required dose may conveniently be represented as a single dose, or as divided doses administered simultaneously (or within a short period of time) or at appropriate intervals, for example, two, three, four, or more doses per day. It can be understood by those skilled in the art that, although the dosage ranges described above are given, the specific effective amount may be appropriately adjusted according to the patient's condition and in conjunction with the physician's diagnosis.

As used herein, the term "pharmaceutically acceptable salt" refers to a salt of the compound of the present disclosure that is pharmaceutically acceptable and has the pharmacological activity of the parent compound. Such salts include acid addition salts formed with inorganic acids (such as nitric acid, phosphoric acid, and carbonic acid) or with organic acids (such as propionic acid, hexanoic acid, cyclopentanepropionic acid, glycolic acid, pyruvic acid, gluconic acid, stearic acid, and muconic acid); or salts formed when an acidic proton present on the parent compound is substituted with a metal ion (e.g., an alkali metal ion or an alkaline earth metal ion); or coordination compounds formed with organic bases (such as ethanolamine). The pharmaceutically acceptable salt of the present disclosure can be synthesized from the parent compound containing an acid radical or a basic group by conventional chemical methods. In general, such salts are prepared by subjecting the compounds in a free acid or base form to a reaction with a stoichiometric amount of appropriate base or acid in water or an organic solvent or a mixture thereof. In general, nonaqueous media such as ethers, ethyl acetate, ethanol, isopropanol, or acetonitrile are preferred. In addition to the salt forms, the compounds provided by the present disclosure also exist in prodrug forms. Prodrugs of the compounds described herein may readily undergo chemical changes under physiological conditions and thus be converted to the compounds of the present disclosure. In addition, prodrugs can be converted to the compounds of the present disclosure in an *in vivo* environment by chemical or biochemical methods.

As used herein, the term "solvate" refers to a substance formed by combining the compound of the present disclosure with a pharmaceutically acceptable solvent. The pharmaceutically acceptable solvent includes acetic acid and the like. The solvate includes both stoichiometric and non-stoichiometric amounts of solvates. Certain compounds of the present disclosure may exist in non-solvated forms or solvated forms. In general, the solvated forms are comparable to the non-solvated forms, and both are encompassed within the scope of the present disclosure.

As used herein, the term "stereoisomer" includes conformational isomers and configurational isomers, wherein the configurational isomers mainly include cis-trans isomers and optical isomers. The compounds described in the present disclosure may exist in the form of stereoisomers and thus encompass all possible stereoisomeric forms, including but not limited to cis-trans isomers, tautomers, enantiomers, diastereomers, atropisomers, and the like. The compounds described in the present disclosure may also exist in the form of any combination or any mixture of the aforementioned stereoisomers, such as equimolar mixtures of mesomers, racemates, and atropisomers. For example, a single enantiomer, a single diastereomer, or a mixture thereof, or a single atropisomer or a mixture thereof. When the compounds described in the present disclosure contain an olefinic double bond, they include cis isomers and trans isomers, as well as any combination thereof, unless otherwise specified. The atropisomers of the present disclosure are stereoisomers with axial or planar chirality that are produced on the basis of restricted rotation within the molecule. As medicaments, stereoisomers with excellent activity are preferred. The compounds of the present disclosure have optical isomers derived from asymmetric carbons and the like. If necessary, single isomers can be obtained by resolution through methods known in the art, such as crystallization or chiral chromatography. As used herein, the =CR^{e}R^{f} substitution refers to the connection of an atom of the molecule to the carbon atom (C) in CR^{e}R^{f} via a double bond. That is, the atom of the molecule is substituted with 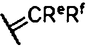 . As used herein, the =NR^{e} substitution refers to the connection of an atom of the molecule to the nitrogen atom (N) in NR^{e} via a double bond. That is, the atom of the molecule is substituted with 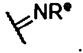 As used herein, the term "oxo" refers to the connection of an atom of the molecule to an oxygen atom (O) via a double bond.

That is, the atom of the molecule is substituted with 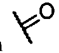 . As used herein, the thio refers to the connection of an atom of the molecule to a sulfur atom (S) via a double bond. That is, the atom of the molecule is substituted with 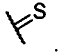 As used herein, when a group such as an alkyl group is located in the middle of a structural formula, the group is a ylene group. For example, an alkyl group corresponds to an alkylene group, etc.

As used herein, the term "alkyl" refers to a linear or branched saturated aliphatic hydrocarbyl group. The term C₁₋₆ alkyl refers to a linear or branched alkyl group having 1, 2, 3, 4, 5, or 6 carbon atoms. C₁₋₄ alkyl is more preferred. C₁₋₃ alkyl is more preferred. Specific examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. The alkyl described herein may be substituted or unsubstituted, and when it is substituted, the substituent is preferably one or more substituent groups described in the present application.

As used herein, the term "heteroalkyl" refers to an alkyl group having at least one carbon atom substituted with a heteroatom (e.g., an O, N, or S atom), wherein the alkyl is as defined above. The term "C₁₋₆ heteroalkyl" refers to a linear or branched heteroalkyl group having 1, 2, 3, 4, 5, or 6 carbon atoms. C₁₋₄ heteroalkyl is preferred. C₁₋₃ heteroalkyl is more preferred. Heteroatoms may be present in the middle or at the end of a group. The heteroalkyl may be substituted or unsubstituted, and when it is substituted, the substituent is preferably one or more substituent groups described in the present application. Exemplary heteroalkyl groups may include alkoxy, alkylthio, etc.

As used herein, the term "alkoxy" refers to a group having a structure of -O-alkyl, wherein the alkyl is as defined above. The term "C₁₋₆ alkoxy" refers to an alkoxy group having 1 to 6 carbon atoms. C₁₋₄ alkoxy is preferred. C₁₋₃ alkoxy is more preferred. Specific examples include, but are not limited to, methoxy, ethoxy, n-propoxy, isopropoxy, n-butoxy, tert-butoxy, isobutoxy, n-pentoxy, etc. The alkoxy may be substituted or unsubstituted, and when it is substituted, the substituent is preferably one or more substituent groups described in the present application.

As used herein, the term "alkylthio" refers to a group having a structure of -S-alkyl, wherein the alkyl is as defined above. The term "C₁₋₆ alkylthio" refers to an alkylthio group having 1 to 6 carbon atoms. C₁₋₄ alkylthio is preferred. C₁₋₃ alkylthio is more preferred. The alkylthio may be substituted or unsubstituted, and when it is substituted, the substituent is preferably one or more substituent groups described in the present application.

As used herein, the term "alkenyl" refers to an alkyl group as defined above having one or more carbon-carbon double bonds at any site on the chain, and the term "C₂₋₆ alkenyl" refers to an alkenyl group having 2 to 6 carbon atoms and at least one (e.g., 1 to 2) carbon-carbon double bond. C₂₋₄ alkenyl (i.e., an alkenyl group having 2 to 4 carbon atoms and 1 to 2 carbon-carbon double bonds) is preferred. Specific examples include, but are not limited to, ethenyl, 1-propenyl, 2-propenyl, 1-, 2-, or 3-butenyl, pentenyl, hexenyl, butadienyl, etc. The alkenyl may be substituted or unsubstituted, and when it is substituted, the substituent is preferably one or more substituent groups described in the present application.

As used herein, the term "alkynyl" refers to an alkyl group as defined above having one or more carbon-carbon triple bonds at any site on the chain, and the term "C₂₋₆ alkynyl" refers to an alkynyl group having 2 to 6 carbon atoms and at least one (e.g., 1 to 2) carbon-carbon triple bond. C₂₋₄ alkynyl (i.e., an alkynyl group having 2 to 4 carbon atoms and 1 to 2 carbon-carbon triple bonds) is preferred. Specific examples include, but are not limited to, ethynyl, 1-propynyl, 2-propynyl, 1-, 2-, or 3-butynyl, etc. The alkynyl may be substituted or unsubstituted, and when it is substituted, the substituent is preferably one or more substituent groups described in the present application.

As used herein, the term "halogen" refers to fluorine, chlorine, bromine, or iodine.

As used herein, the term "halo" refers to fluoro, chloro, bromo, or iodo.

As used herein, the term "haloalkyl" refers to an alkyl group, in which one or more (such as 1, 2, 3, 4, or 5) hydrogen atoms are substituted with halogen atoms, wherein the alkyl is as defined above. The term "C₁₋₆ haloalkyl" refers to a haloalkyl group having 1 to 6 carbon atoms. C₁₋₄ haloalkyl is preferred. C₁₋₃ haloalkyl is more preferred. Specific examples include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, etc. The haloalkyl may be substituted or unsubstituted, and when it is substituted, the substituent is preferably one or more substituent groups described in the present application.

As used herein, the term "haloalkoxy" refers to an alkoxy group, in which one or more (such as 1, 2, 3, 4, or 5) hydrogen atoms are substituted with halogen atoms, wherein the alkoxy is as defined above. The term "C₁₋₆ haloalkoxy" refers to a haloalkoxy group having 1 to 6 carbon atoms. C₁₋₄ haloalkoxy is preferred. C₁₋₃ haloalkoxy is more preferred. Specific examples include, but are not limited to, trifluoromethoxy, trifluoroethoxy, monofluoromethoxy, monofluoroethoxy, difluoromethoxy, difluoroethoxy, etc. The haloalkoxy may be substituted or unsubstituted, and when it is substituted, the substituent is preferably one or more substituent groups described in the present application.

As used herein, the term "deuterated" refers to the substitution of one or more (such as 1, 2, 3, 4, or 5) hydrogen atoms in a group with deuterium atoms.

As used herein, the term "deuterated alkyl" refers to an alkyl group, in which one or more (such as 1, 2, 3, 4, or 5) hydrogen atoms are substituted with deuterium atoms, wherein the alkyl is as defined above. The term "deuterated C₁₋₆ alkyl" refers to a deuterated alkyl group having 1 to 6 carbon atoms. Deuterated C₁₋₄ alkyl is preferred. Deuterated C₁₋₃ alkyl is more preferred. Specific examples include, but are not limited to, monodeuteriomethyl, dideuteriomethyl, trideuteriomethyl, monodeuterioethyl, 1,2-dideuterioethyl, trideuterioethyl, etc. The deuterated alkyl may be substituted or unsubstituted, and when it is substituted, the substituent is preferably one or more substituent groups described in the present application.

As used herein, the term "deuterated alkoxy" refers to an alkoxy group, in which one or more (such as 1, 2, 3, 4, or 5) hydrogen atoms are substituted with deuterium atoms, wherein the alkoxy is as defined above. The term "deuterated C₁₋₆ alkoxy" refers to a deuterated alkoxy group having 1 to 6 carbon atoms. Deuterated C₁₋₄ alkoxy is more preferred. Deuterated C₁₋₃ alkoxy is more preferred. Specific examples include, but are not limited to, trideuteriomethoxy, trideuterioethoxy, monodeuteriomethoxy, monodeuterioethoxy, dideuteriomethoxy, dideuterioethoxy, etc. The deuterated alkoxy may be substituted or unsubstituted, and when it is substituted, the substituent is preferably one or more substituent groups described in the present application.

As used herein, the terms "cycloalkyl" and "cycloalkyl ring" are used interchangeably and refer to a saturated or partially unsaturated monocyclic or polycyclic hydrocarbyl group. The ring carbon atoms of the cycloalkyl may be optionally substituted with one or more (such as 1, 2, 3, 4, or 5) oxo groups to form a cyclic ketone structure. The term "3- to 20-membered cycloalkyl" or "C₃₋₂₀ cycloalkyl" refers to a cycloalkyl group having 3 to 20 ring carbon atoms. The cycloalkyl may include monocyclic cycloalkyl, spirocycloalkyl, fused cycloalkyl, and bridged cycloalkyl. C₃₋₁₂ cycloalkyl (e.g., C₃₋₈ cycloalkyl, C₃₋₆ cycloalkyl, or C₃₋₈ monocyclic cycloalkyl), C₅₋₂₀ spirocycloalkyl, C₅₋₂₀ fused cycloalkyl, C₅₋₂₀ bridged cycloalkyl, etc., are preferred.

As used herein, the term "C₃₋₆ cycloalkyl" refers to a cycloalkyl group having 3 to 6 ring carbon atoms. The cycloalkyl may be monocyclic (specific examples include, but are not limited to, cyclopropyl, cyclobutyl, cyclopentyl, or cyclohexyl), or bicyclic (specific examples include, but are not limited to, cyclopropylcyclopropyl, cyclopropylcyclobutyl, cyclopropylcyclopentyl, etc.).

As used herein, the terms "C₃₋₈ monocyclic cycloalkyl" and "3- to 8-membered monocyclic cycloalkyl" refer to a saturated or partially unsaturated monocyclic cyclic hydrocarbyl group having 3 to 8 ring carbon atoms. C₃₋₆ monocyclic cycloalkyl (i.e., 3- to 6-membered monocyclic cycloalkyl) or C₄₋₆ monocyclic cycloalkyl (i.e., 4- to 6-membered monocyclic cycloalkyl) is preferred. C₃, C₄, C₅, or C₆ monocyclic cycloalkyl is more preferred. Specific examples of monocyclic cycloalkyl include, but are not limited to, cyclopropyl, cyclobutyl, cyclopentyl, cyclohexyl, cycloheptyl, cyclooctyl, etc.

As used herein, the terms "spirocycloalkyl" and "spirocycloalkyl ring" refer to a saturated or partially unsaturated polycyclic cyclic hydrocarbyl group formed by sharing one carbon atom (referred to as a spiro atom) between two or more monocyclic rings. According to the number of spiro atoms shared among rings, the spirocycloalkyl may be classified as monospirocycloalkyl, bispirocycloalkyl, and polyspirocycloalkyl. The term "5- to 20-membered spirocycloalkyl" or "C₅₋₂₀ spirocycloalkyl" refers to a spirocycloalkyl group having 5 to 20 ring carbon atoms. 6- to 14-membered (i.e., C₆₋₁₄) spirocycloalkyl is preferred. 6- to 14-membered monospirocycloalkyl is more preferred. 7- to 11-membered (i.e., C₇₋₁₁) spirocycloalkyl is more preferred. 7- to 11-membered monospirocycloalkyl is more preferred. 7-, 8-, 9-, 10- or 11-membered monospirocycloalkyl is more preferred. Specific examples of spirocycloalkyl include, but are not limited to: 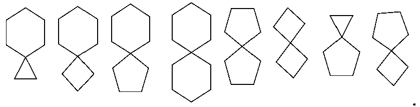 These spirocycloalkyl groups can be attached to the rest of the molecule through any one of the ring atoms.

As used herein, the terms "fused cycloalkyl" and "fused cycloalkyl ring" refer to a saturated or partially unsaturated polycyclic cyclic hydrocarbyl group formed by sharing a pair of adjacent carbon atoms between two or more monocyclic rings. According to the number of the formed rings, the fused cycloalkyl may be classified as bicyclic, tricyclic, tetracyclic, or polycyclic fused cycloalkyl. The term "5- to 20-membered fused cycloalkyl" or "C₅₋₂₀ fused cycloalkyl" refers to a fused cycloalkyl group having 5 to 20 ring carbon atoms. 6- to 14-membered (i.e., C₆₋₁₄) fused cycloalkyl is preferred. 6- to 14-membered bicyclic fused cycloalkyl is more preferred. 7- to 10-membered (i.e., C₇₋₁₀) fused cycloalkyl is more preferred. 7- to 10-membered bicyclic fused cycloalkyl is more preferred. 8-, 9-, or 10-membered bicyclic fused cycloalkyl is more preferred. Specific examples of fused cycloalkyl include, but are not limited to: 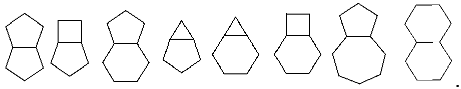 These fused cycloalkyl groups can be attached to the rest of the molecule through any one of the ring atoms.

As used herein, the terms "bridged cycloalkyl" and "bridged cycloalkyl ring" refer to a saturated or partially unsaturated polycyclic cyclic hydrocarbyl group formed by sharing two carbon atoms that are not directly connected between two or more monocyclic rings. According to the number of the formed rings, the bridged cycloalkyl may be classified as bicyclic, tricyclic, tetracyclic, or polycyclic bridged cycloalkyl. The term "5-to 20-membered bridged cycloalkyl" or "C ₅₋₂₀ bridged cycloalkyl" refers to a bridged cycloalkyl group having 5 to 20 ring carbon atoms. 6- to 14-membered (i.e., C₆₋₁₄) bridged cycloalkyl is preferred. 7- to 10-membered (i.e., C₇₋₁₀) bridged cycloalkyl is more preferred. Specific examples of bridged cycloalkyl include, but are not limited to: 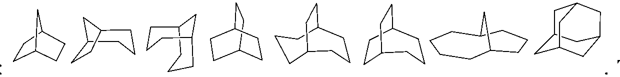 These bridged cycloalkyl groups can be attached to the rest of the molecule through any one of the ring atoms.

The various cycloalkyl groups described above may be substituted or unsubstituted, and when they are substituted, the substituent is preferably one or more substituent groups described in the present application. As used herein, the terms "heterocyclyl" and "heterocyclyl ring" are used interchangeably and refer to a saturated or partially unsaturated monocyclic or polycyclic hydrocarbyl group, wherein one or more (such as 1, 2, 3, 4, 5, 6, 7, or 8) ring atoms are heteroatoms selected from phosphorus, nitrogen, oxygen, and sulfur, excluding a ring moiety of -O-O-, -O-S-, or -S-S-, and the remaining ring atoms are carbon atoms. When the ring atom is a nitrogen atom, the nitrogen atom may be substituted or unsubstituted (i.e., N or NR, wherein R is hydrogen or other substituents already defined herein) and/or the nitrogen atom may form a quaternary ammonium salt. When the heteroatom is a sulfur atom, the sulfur atom may be optionally oxidized (e.g., S(=O)ₘ', wherein m' is an integer of 0 to 2). When the heteroatom is a phosphorus atom, the phosphorus atom may be optionally oxidized (e.g., P(=O) ₘ ", wherein m" is an integer of 0, 1, or 2). For example, the heterocyclyl includes monocyclic heterocyclyl, spiroheterocyclyl, fused heterocyclyl, and bridged heterocyclyl. The ring carbon atoms of the heterocyclyl may be optionally substituted with one or more (such as 1, 2, 3, 4, or 5) oxo groups to form a cyclic ketone, cyclic lactone, or cyclic lactam structure. The term "3-to 20-membered heterocyclyl" refers to a heterocyclyl group having 3 to 20 ring atoms, wherein one or more (such as 1, 2, 3, 4, or 5) ring atoms are heteroatoms selected from phosphorus, nitrogen, oxygen, and sulfur. The 3- to 20-membered heterocyclyl is preferably 3- to 10-membered heterocyclyl, 3- to 12-membered monocyclic heterocyclyl (e.g., 3- to 8-membered monocyclic heterocyclyl), 5- to 20-membered spiroheterocyclyl, 5- to 20-membered fused heterocyclyl, 5- to 20-membered bridged heterocyclyl, etc.

The term "3- to 10-membered heterocyclyl" refers to a saturated or partially unsaturated cyclic hydrocarbyl group having 3 to 10 ring atoms, wherein one or more (such as 1, 2, 3, 4, or 5) ring atoms are heteroatoms selected from phosphorus, nitrogen, oxygen, and sulfur. The term "3- to 8-membered heterocyclyl" refers to a saturated or partially unsaturated cyclic hydrocarbyl group having 3 to 8 ring atoms, wherein one or more (such as 1, 2, 3, 4, or 5) ring atoms are heteroatoms selected from phosphorus, nitrogen, oxygen, and sulfur.

The heterocyclyl may be monocyclic (specific examples are as described in the definition of the 3- to 8-membered monocyclic heterocyclyl) or bicyclic (specific examples include, but are not limited to, cyclopropyl-fused pyrrolidine ring, cyclopropyl-fused tetrahydrofuran ring, etc.).

As used herein, the terms "3- to 8-membered monocyclic heterocyclyl" and "3- to 8-membered monocyclic heterocyclyl ring" refer to a saturated or partially unsaturated monocyclic hydrocarbyl group having 3 to 8 ring atoms, wherein one or more (such as 1, 2, 3, 4, or 5) ring atoms are heteroatoms selected from phosphorus, nitrogen, oxygen, and sulfur. 3- to 6-membered monocyclic heterocyclyl having 3 to 6 ring atoms, wherein one or more (such as 1 or 2) ring atoms are heteroatoms, is preferred. 4- to 6-membered monocyclic heterocyclyl having 4 to 6 ring atoms, wherein one or more (such as 1 or 2) ring atoms are heteroatoms, is more preferred. 5- or 6-membered monocyclic heterocyclyl having 5 or 6 ring atoms, wherein one or more (such as 1 or 2) ring atoms are heteroatoms, is more preferred. Specific examples of monocyclic heterocyclyl include, but are not limited to, aziridine, oxirane, azetidine, azetidin-2-one, oxetane, oxetan-2-one, oxazolidine, pyrrolidin-2-one, pyrrolidine-2,5-dione, 1,3-dioxolane, dihydrofuran-2(3H)-one, dihydrofuran-2,5-dione, piperidin-2-one, piperidine-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholine-3-one, morpholine-2-one, thiomorpholin-3-one 1,1-dioxide, thiomorpholine, thiomorpholin-1,1-dioxide, tetrahydropyran, 1,2-dihydroazete, 1,2-dihydrooxete, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridine-2,6(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine, etc.

As used herein, the term "nitrogen-containing 3- to 8-membered heterocyclyl" refers to a saturated or partially unsaturated cyclic hydrocarbyl group having 3 to 8 ring atoms, wherein 1 ring atom is a nitrogen atom and the other (such as 1, 2, 3, 4, or 5) ring atoms are heteroatoms selected from phosphorus, nitrogen, oxygen, and sulfur. The term "nitrogen-containing 4- to 8-membered heterocyclyl" refers to a saturated or partially unsaturated cyclic hydrocarbyl group having 4 to 8 ring atoms, wherein 1 ring atom is a nitrogen atom and the other (such as 1, 2, 3, 4 or 5) ring atoms are heteroatoms selected from phosphorus, nitrogen, oxygen and sulfur. The heterocyclyl may be monocyclic or bicyclic. Specific examples include, but are not limited to, aziridinyl, azetidinyl, azacyclopentyl (i.e., tetrahydropyrrole), azacyclohexyl (i.e., piperidine), morpholinyl, piperazinyl, and oxazolidine.

The 2 connected ring atoms on the monocyclic heterocyclyl ring described above, including C-C and N-C, may both optionally be fused to the cycloalkyl, heterocyclyl, aryl, or heteroaryl defined in the present disclosure, such as a monocyclic cycloalkyl ring, a monocyclic heterocyclyl ring, a monoaryl ring, or a 5- or 6-membered monocyclic heteroaryl ring, to form a fused polycyclic ring. The 2 connected ring atoms on the monocyclic heterocyclyl that form a fused ring with another ring are preferably C-C.

As used herein, the terms "spiro heterocyclyl" and "spiro heterocyclyl ring" refer to polycyclic heterocyclyl formed from two or more saturated or partially unsaturated monocyclic rings by sharing one carbon atom (referred to as the spiro atom), wherein one or more (such as 1, 2, 3, 4, 5, 6, 7, or 8) ring atoms are heteroatoms selected from phosphorus, nitrogen, oxygen, and sulfur, and the remaining ring atoms are carbon atoms. Each monocyclic ring may contain one or more double bonds, but none of the rings has a fully conjugated π-electron system. According to the number of spiro atoms shared among the rings, the spiroheterocyclyl may be monospiroheterocyclyl, dispiroheterocyclyl, or polyspiroheterocyclyl. The term "5- to 20-membered spiroheterocyclyl" refers to a spiroheterocyclyl group having 5 to 20 ring atoms, wherein one or more (such as 1, 2, 3, 4, or 5) ring atoms are heteroatoms selected from phosphorus, nitrogen, oxygen, and sulfur. 6- to 14-membered spiroheterocyclyl having 6 to 14 ring atoms, wherein one or more (such as 1, 2, 3, 4, or 5) ring atoms are heteroatoms, is preferred. 7- to 11-membered spiroheterocyclyl having 7 to 11 ring atoms, wherein one or more (such as 1, 2, 3, 4, or 5) ring atoms are heteroatoms, is more preferred. 7-membered, 8-membered, or 10-membered monospiroheterocyclyl is the most preferred. Specific examples of spiroheterocyclyl include, but are not limited to:

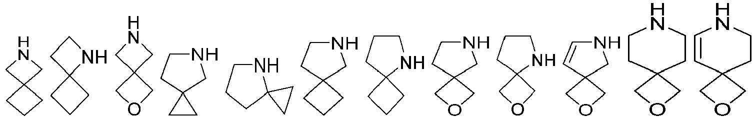

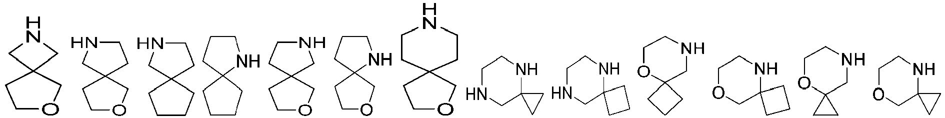

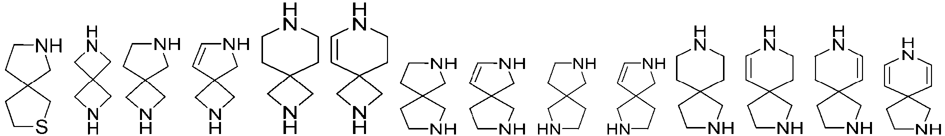

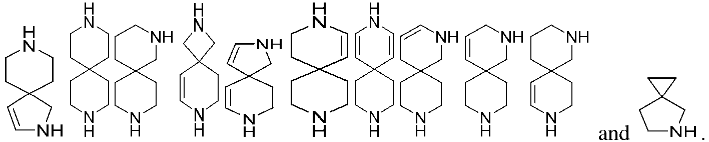

These spiroheterocyclyl groups can be attached to the rest of the molecule through any one of the suitable ring atoms.

As used herein, the terms "fused heterocyclyl" and "fused heterocyclyl ring" refer to polycyclic heterocyclyl formed from two or more saturated or partially unsaturated monocyclic rings by sharing a pair of adjacent ring atoms, wherein one or more ( such as 1, 2, 3, 4, 5, 6, 7, or 8) ring atoms are heteroatoms selected from phosphorus, nitrogen, oxygen, and sulfur, and the remaining ring atoms are carbon atoms. Each monocyclic ring may contain one or more double bonds, but none of the rings has a fully conjugated π-electron system. The shared pair of adjacent ring atoms may be C-C or N-C. According to the number of constituent rings, the fused heterocyclyl may be classified as bicyclic, tricyclic, tetracyclic, or polycyclic fused heterocyclyl. The term "5- to 20-membered fused heterocyclyl" refers to a fused heterocyclyl group having 5 to 20 ring atoms, wherein one or more (such as 1, 2, 3, 4, or 5) ring atoms are heteroatoms selected from phosphorus, nitrogen, oxygen, and sulfur. 6- to 14-membered fused heterocyclyl having 6 to 14 ring atoms, wherein one or more (such as 1, 2, 3, 4, or 5) ring atoms are heteroatoms, is preferred. 6- to 10-membered fused heterocyclyl having 6 to 10 ring atoms, wherein one or more (such as 1, 2, 3, 4, or 5) ring atoms are heteroatoms, is more preferred. 8- to 10-membered fused heterocyclyl having 8 to 10 ring atoms, wherein one or more (such as 1, 2, 3, 4, or 5) ring atoms are heteroatoms, is more preferred. 8-membered, 9-membered, or 10-membered bicyclic fused heterocyclyl is the most preferred. Specific examples of fused heterocyclyl include, but are not limited to:

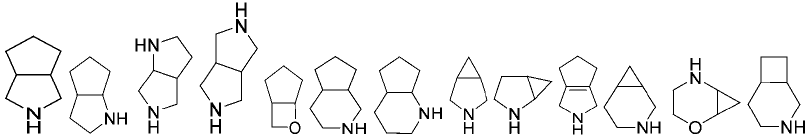

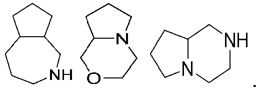

These fused heterocyclyl groups can be attached to the rest of the molecule through any one of the suitable ring atoms.

As used herein, the terms "bridged heterocyclyl" and "bridged heterocyclyl ring" refer to polycyclic heterocyclyl formed from two or more saturated or partially unsaturated monocyclic rings by sharing two ring atoms that are not directly connected, wherein one or more (such as, 1, 2, 3, 4, 5, 6, 7, or 8) ring atoms are heteroatoms selected from phosphorus, nitrogen, oxygen, and sulfur, and the remaining ring atoms are carbon atoms. According to the number of the formed rings, the bridged cycloalkyl may be classified as bicyclic, tricyclic, tetracyclic, or polycyclic bridged cycloalkyl. The term "5- to 20-membered bridged heterocyclyl" refers to a bridged heterocyclyl group having 5 to 20 ring atoms, wherein one or more (such as 1, 2, 3, 4, or 5) ring atoms are heteroatoms selected from phosphorus, nitrogen, oxygen, and sulfur. Each monocyclic ring may contain one or more double bonds, but none of the rings has a fully conjugated π-electron system. 6- to 14-membered bridged heterocyclyl is preferred. 7- to 10-membered bridged heterocyclyl is more preferred. Specific examples of bridged heterocyclyl include, but are not limited to:

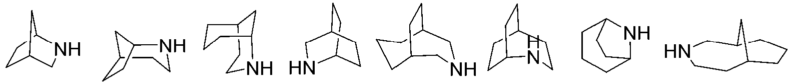

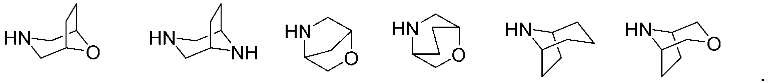

These bridged heterocyclyl groups can be attached to the rest of the molecule through any one of the suitable ring atoms.

The various heterocyclyl groups in the present disclosure may be optionally substituted, and when they are substituted, the substituent is preferably one or more substituent groups described in the present application. The term "5- to 15-membered tricyclic heterocyclyl" refers to a tricyclic heterocyclyl group having 5 to 15 ring atoms, wherein three rings may be connected in one or more ways selected from spiro connection, fused connection, and bridged connection. Specific examples may include, but are not limited to, a first monocyclic ring being a 6-membered monocyclic heterocyclic ring, a second monocyclic ring being a 6-membered monocyclic heterocyclic ring, and a third monocyclic ring being a 3- to 6-membered monocyclic heterocyclic ring; and the first monocyclic ring is fused to the second monocyclic ring, and the third monocyclic ring is spiro-connected to the second monocyclic ring.

The term "7- to 12-membered bicyclic heterocyclyl" refers to a bicyclic heterocyclyl group having 7 to 12 ring atoms, wherein two rings may be connected in one way selected from spiro connection, fused connection, and bridged connection. Specific examples may be selected from the specific examples of the spiroheterocyclyl, the specific examples of the fused heterocyclyl, and the specific examples of the bridged heterocyclyl described above.

As used herein, the terms "aryl", "aryl ring", and "aromatic ring" are used interchangeably and refer to an all-carbon monocyclic, an all-carbon non-fused polycyclic (rings are connected by covalent bonds and not fused), or an all-carbon fused polycyclic (i.e., rings that share a pair of adjacent carbon atoms) group. At least one ring in the group is aromatic, i.e., has a conjugated π-electron system. The term "C₆₋₁₄ aryl" refers to an aryl group having 6 to 14 ring atoms. C₆₋₁₀ aryl is preferred. The C₆₋₁₄ aryl includes monocyclic aryl, non-fused polycyclic aryl, aromatic fused polycyclic ring, etc., wherein examples of monocyclic aryl include phenyl, and examples of non-fused polycyclic aryl include biphenyl, etc.

In the present disclosure, when C₆₋₁₄ aryl is an aromatic fused polycyclic ring, the aromatic fused polycyclic ring may be a polycyclic group formed by fusing a monoaryl ring to one or more monoaryl rings; non-limiting examples include naphthyl, anthryl, etc.

In some embodiments of the present disclosure, when C₆₋₁₄ aryl is an aromatic fused polycyclic ring, the aromatic fused polycyclic ring may also be a polycyclic group formed by fusing a monoaryl ring (e.g., phenyl) to one or more non-aromatic rings, wherein the ring connected to the parent structure is an aromatic ring or a non-aromatic ring. The non-aromatic ring includes, but is not limited to, a 3- to 6-membered monocyclic heterocyclyl ring (preferably a 5- or 6-membered monocyclic heterocyclyl ring, wherein ring carbon atoms of the monocyclic heterocyclyl ring may be substituted with 1 to 2 oxo groups to form a cyclic lactam or cyclic lactone structure) and a 3- to 6-membered monocyclic cycloalkyl ring (preferably a 5- or 6-membered monocyclic cycloalkyl ring, wherein ring carbon atoms of the monocyclic cycloalkyl ring may be substituted with 1 or 2 oxo groups to form a cyclic ketone structure). The polycyclic group formed by fusing the monoaryl ring to one or more non-aromatic rings described above may be connected to another group or the parent structure through a nitrogen atom or carbon atom, and the ring connected to the parent structure is a monoaryl ring or non-aromatic ring.

The various aryl groups in the present disclosure may be substituted or unsubstituted, and when they are substituted, the substituent is preferably one or more substituent groups described in the present application. As used herein, the terms "heteroaryl", "heteroaryl ring", and "heteroaromatic ring" are used interchangeably and refer to a monocyclic or fused polycyclic ring (i.e., sharing a pair of adjacent ring atoms, which may be C-C or N-C) group, in which the ring atoms are substituted with at least one (such as 1, 2, 3, 4, 5, 6, 7, or 8) heteroatom independently selected from phosphorus, nitrogen, oxygen, and sulfur. The heteroaryl has 6, 10, or 14 π electrons shared, and at least one ring in the group is aromatic. When the ring atom is a nitrogen atom, the nitrogen atom may be substituted or unsubstituted (i.e., N or NR, wherein R is hydrogen or other substituents already defined herein) and/or the nitrogen atom may form a quaternary ammonium salt. When the heteroatom is a sulfur atom, the sulfur atom may be optionally oxidized (e.g., S(=O)ₘ', wherein m' is an integer of 0 to 2). When the heteroatom is a phosphorus atom, the phosphorus atom may be optionally oxidized (e.g., P(=O) ₘ ", wherein m" is an integer of 0, 1, or 2). The term "5- to 14-membered heteroaryl" refers to a heteroaryl group having 5 to 14 ring atoms, wherein one or more (such as 1, 2, 3, 4, or 5) ring atoms are heteroatoms selected from phosphorus, nitrogen, oxygen, and sulfur. 5- to 10-membered heteroaryl having 5 to 10 ring atoms, wherein one or more (such as 1, 2, 3, 4, or 5) ring atoms are heteroatoms, is preferred. The 5- to 14-membered heteroaryl may be monocyclic heteroaryl, fused bicyclic heteroaryl, fused tricyclic heteroaryl, etc.

As used herein, the term "5- or 6-membered monocyclic heteroaryl" refers to a monocyclic heteroaryl group having 5 or 6 ring atoms, wherein one or more (such as 1, 2, 3, 4, or 5) ring atoms are heteroatoms selected from phosphorus, nitrogen, oxygen, and sulfur. Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, etc. As used herein, the term "8- to 10-membered bicyclic heteroaryl" refers to a fused bicyclic heteroaryl group having 8 to 10 ring atoms, wherein one or more (such as 1, 2, 3, 4, or 5) ring atoms are heteroatoms selected from phosphorus, nitrogen, oxygen, and sulfur. The fused bicyclic heteroaryl may be a bicyclic group (preferably a 9- or 10-membered bicyclic heteroaryl ring) formed by fusing a monoaryl ring (e.g., phenyl) to a monocyclic heteroaryl ring (preferably a 5- or 6-membered monocyclic heteroaryl ring), or a bicyclic group formed by fusing a monocyclic heteroaryl ring (preferably a 5- or 6-membered monocyclic heteroaryl ring) to a monocyclic heteroaryl ring (preferably a 5- or 6-membered monocyclic heteroaryl ring).

Any 2 connected ring atoms on the monocyclic heteroaryl ring described above, including C-C, N-C, and N-N, may all be fused to the cycloalkyl, heterocyclyl, aryl, or heteroaryl defined in the present disclosure, such as a monocyclic cycloalkyl ring, a monocyclic heterocyclyl ring, a monoaryl ring, or a 5- or 6-membered monocyclic heteroaryl ring, to form a fused polycyclic ring. The 2 connected ring atoms on the monocyclic heteroaryl ring that form a fused ring with another ring are preferably C-C, including but not limited to the following forms:

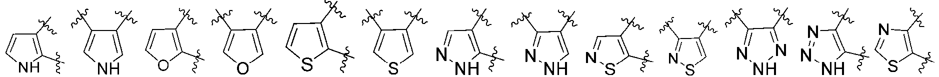

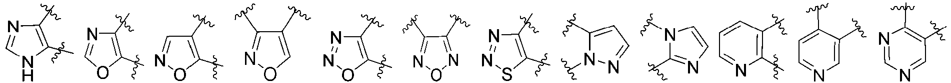

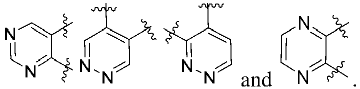

The ring atoms marked by " 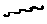 " in the groups described above are connected to other moieties of the molecule.

Non-limiting examples of 8- to 10-membered bicyclic heteroaryl include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, etc.

Specific examples of bicyclic heteroaryl include, but are not limited to: 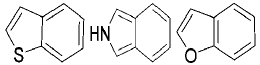 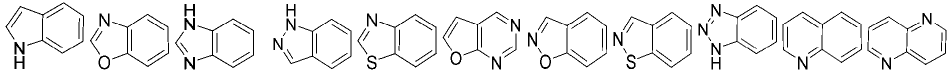 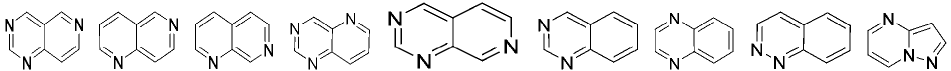 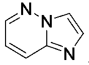 . These groups can be attached to the rest of the molecule through any one of the suitable ring atoms. The ring connected to the parent structure may be a monocyclic heteroaryl ring or a benzene ring.

In some embodiments of the present disclosure, the fused bicyclic heteroaryl or fused tricyclic heteroaryl may be a polycyclic group formed by fusing a monocyclic heteroaryl ring (preferably a 5- or 6-membered monocyclic heteroaryl ring) to one or more non-aromatic rings, wherein the ring connected to the parent structure is a monocyclic heteroaryl ring or a non-aromatic ring. The non-aromatic ring includes, but is not limited to, a 3- to 6-membered monocyclic heterocyclyl ring (preferably a 5- or 6-membered monocyclic heterocyclyl ring, wherein ring carbon atoms of the monocyclic heterocyclyl ring may be substituted with 1 to 2 oxo groups to form a cyclic lactam or cyclic lactone structure), a 3- to 6-membered monocyclic cycloalkyl ring (preferably a 5- or 6-membered monocyclic cycloalkyl ring, wherein ring carbon atoms of the monocyclic cycloalkyl ring may be substituted with 1 or 2 oxo groups to form a cyclic ketone structure), etc. The polycyclic group formed by fusing the monocyclic heteroaryl ring to one or more non-aromatic rings described above may be connected to another group or the parent structure through a nitrogen atom or carbon atom, and the ring connected to the parent structure is a monocyclic heteroaryl ring or non-aromatic ring. The 6- to 12-membered heteroaryl-fused cycloalkyl is formed by fusing monocyclic heteroaryl to cycloalkyl, and the ring connected to the parent structure may be monocyclic heteroaryl or cycloalkyl. For example, 5-membered monocyclic heteroaryl is fused to 3-, 4-, 5-, 6-, or 7-membered cycloalkyl, or 6-membered monocyclic heteroaryl is fused to 3-, 4-, 5-, or 6-membered cycloalkyl.

The 6- to 12-membered heteroaryl-fused heterocyclyl is formed by fusing monocyclic heteroaryl to heterocyclyl, and the ring connected to the parent structure may be monocyclic heteroaryl or heterocyclyl.

For example, 5-membered monocyclic heteroaryl is fused to 3-, 4-, 5-, 6-, or 7-membered heterocyclyl, or 6-membered monocyclic heteroaryl is fused to 3-, 4-, 5-, or 6-membered heterocyclyl.

The various heteroaryl groups in the present disclosure may be substituted or unsubstituted, and when they are substituted, the substituent is preferably one or more substituent groups described in the present application.

As used herein, the term "hydroxy" refers to -OH.

As used herein, the term "cyano" refers to -CN.

As used herein, the term "nitro" refers to -NO₂.

As used herein, the term "benzyl" refers to -CH₂-benzene.

As used herein, the term "oxo" refers to =O.

As used herein, the term "carboxyl" refers to -C(=O)OH.

As used herein, the term "acetyl" refers to -COCH₃.

Abbreviations:
Cbz: carbamoylbenzyl; TBDPS: tert-butyl(methoxy)diphenylsilyl;
NCO: isocyanate group; (Bpin)2: bis(pinacolato)diboron.

The alkyl, heteroalkyl, alkoxy, alkynyl, alkenyl, cycloalkyl, heterocyclyl, aryl, and heteroaryl described above may be substituted or unsubstituted. When they are substituted, the substituent is preferably one or more of the groups described in the present application.

As used herein, the term "substituted" means that any one or more hydrogen atoms on a specific atom are substituted with substituents that may include deuterium and hydrogen variants, as long as the valence of the specific atom is normal and the compound after substitution is stable. When the substituent is oxo (i.e., =O), it means that two hydrogen atoms are substituted. The term "optionally substituted" means that an atom may or may not be substituted with a substituent. Unless otherwise specified, the type and number of the substituent may be arbitrary as long as being chemically achievable. 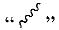 represents the linking position of a chemical bond. 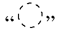 represents that a double bond may be present or absent at any position within the ring, which means that multiple cases such as a saturated ring system, an unsaturated non-aromatic ring system with a double bond and an aromatic ring system are included.

In the present disclosure, 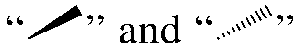 are used to represent the absolute configuration of a stereogenic center. Unless otherwise stated, 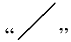 includes the possible use of 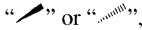 as long as the structure allows.

When 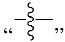 appears in a ring and the linking position is uncertain, it means that the attachment site is limited to any atom on the single ring where the 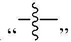 is located, as long as the atomic valence allows.

When any variable (e.g., R) occurs once or more in the constitution or structure of a compound, the definition of the variable in each case is independent. Thus, for example, if a group is substituted with 0-2 R, the group can be optionally substituted with up to two R, and the definition of R in each case is independent.

Furthermore, a combination of a substituent and/or a variant thereof is permissible only if the combination can result in a stable compound.

In any embodiment, any or all hydrogens present in the compound, or hydrogens in a specific group or moiety within the compound, may be replaced by deuterium or tritium. One to the maximum number of hydrogens present in the compound may be replaced by deuterium. One to the maximum number of hydrogens present in any group in the general formula compound or specific compound may be deuterated. For example, when a certain group is described as ethyl, the ethyl may be C₂H₅ or C₂H₅ with x (1 to 5) hydrogens replaced by deuterium, e.g., C₂DₓH₅₋ₓ. When a certain group is described as deuterated ethyl, the deuterated ethyl may be C₂H₅ with x (1 to 5) hydrogens replaced by deuterium, e.g., C₂DₓH₅₋ₓ. The stable deuterated derivatives described in the present disclosure are preferably stable deuterated isotopic derivatives obtained by substituting any hydrogen atom that can be deuterated in each formula with 1 to the maximum number (such as 1 to 2, 1 to 3, 1 to 4, 1 to 5, or 1 to 6, 1 to 7) of deuterium atoms.

Unless otherwise specified, structures depicted herein are also meant to include compounds that differ only by the presence of one or more isotopically enriched atoms. Exemplary isotopes that can be incorporated into the compounds of the present disclosure include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I, and ¹²⁵I. Isotopically labeled compounds (e.g., compounds labeled with ³H and ¹⁴C) can be used in compound or substrate tissue distribution assays. Tritiated (i.e., ³H) and carbon-14 (i.e., ¹⁴C) isotopes are available for ease of preparation and detectability. Additionally, substitution with heavier isotopes such as deuterium (i.e., ²H) may provide certain therapeutic benefits resulting from increased metabolic stability, for example, increased *in vivo* half-life or reduced dosage requirements. In some embodiments, one or more hydrogen atoms are replaced by ²H or ³H, or one or more carbon atoms are replaced by ¹³C- or ¹⁴C-enriched carbon. Positron emitting isotopes, such as ¹⁵O, ¹³N, ¹¹C, and ¹⁸F, can be used in positron emission tomography (PET) studies to determine substrate receptor occupancy. The preparation of isotopically labeled compounds is known to those skilled in the art. For example, isotopically labeled compounds can generally be prepared following procedures analogous to those disclosed for the compounds of the present disclosure described herein by substituting a non-isotopically labeled reagent with an isotopically labeled reagent.

### BRIEF DESCRIPTION OF THE DRAWINGS

FIG. 1 shows a diagram of a three-dimensional molecular structure of intermediate 11-e-1 determined by X-ray single-crystal diffraction.
FIG. 2 shows a diagram of a three-dimensional molecular structure of intermediate 51-7A determined by X-ray single-crystal diffraction.
FIG. 3 shows a diagram of a three-dimensional molecular structure of intermediate 118-12 determined by X-ray single-crystal diffraction.

### DETAILED DESCRIPTION

The compounds of the present disclosure may be prepared by a variety of synthetic methods well known to those skilled in the art, including the specific embodiments listed below, embodiments formed by combinations thereof with other chemical synthetic methods, and equivalents thereof well known to those skilled in the art. Preferred embodiments include, but are not limited to, the examples of the present disclosure. The present disclosure is described in detail below by way of examples. However, this is by no means disadvantageously limiting the scope of the present disclosure. Although the present disclosure has been described in detail herein and specific examples have also been disclosed, it will be apparent to those skilled in the art that various changes and modifications can be made to the specific examples without departing from the spirit and scope of the present disclosure. Experimental procedures without specified conditions in the examples are conducted according to conventional conditions or conditions recommended by the manufacturers. Reagents or instruments without specified manufacturers used herein are commercially available conventional products.

The structures of the compounds were determined by methods such as nuclear magnetic resonance (NMR) and mass spectrometry (MS).

The NMR determination was performed on a Bruker Avance NEO 400 or Bruker Avance NEO 500 nuclear magnetic resonance instrument. The chemical shift (δ) was in parts per million (ppm). The solvents for determination were as shown in each of the examples. The internal standard was tetramethylsilane (TMS). The MS determination was performed on an Agilent 1100 liquid chromatograph.

The high performance liquid chromatography (HPLC) analysis was performed on a Waters 2695 high performance liquid chromatograph.

The preparative high performance liquid chromatography was performed on a Waters 2767 high performance liquid chromatograph.

The chiral HPLC analysis was performed on a Waters 2695 high performance liquid chromatograph or a Waters Investigator SFC system.

The preparative chiral chromatography was performed on a gilson gx-281 chromatograph or a waters SFC-80 chromatograph.

Qingdao GF254 or Yantai Huanghai HSGF254 silica gel plates were used as thin-layer chromatography silica gel plates. 0.15-0.2 mm silica gel plates were used for thin-layer chromatography (TLC). 0.4-0.5 mm silica gel plates were used for thin-layer chromatography product separation and purification.

The ISCO CombiFlash NextGen 300 column chromatography system was generally used for column chromatography, and the Agela Flash Column Silica-Cs series silica gel column was used.

The prep-HPLC used in the following examples, unless otherwise stated, may be performed under the following conditions:
Prep-HPLC (ammonium bicarbonate method 1): column type: Waters XBridge C18, 19 × 250 mm, 5 µm; mobile phase system: A: 5 mmol/L aqueous ammonium bicarbonate solution; B: preparative grade acetonitrile; flow rate: 15 mL/min; B% = 20%-100%; column temperature: room temperature.

Prep-HPLC (ammonium bicarbonate method 2): chromatographic column: Welch Xtimate C18, 21.2 × 150 mm, 5 µm; mobile phase A: 5 mmol/L aqueous ammonium bicarbonate solution, mobile phase B: acetonitrile; flow rate: 15 mL/min; column temperature: room temperature.

Prep-HPLC (formic acid method 1): column type: Waters XBridge C18, 19 × 250 mm, 5 µm; mobile phase system: A: 0.1% aqueous formic acid solution; B: preparative grade acetonitrile; flow rate: 15 mL/min; B% = 20%-100%; column temperature: room temperature.

Prep-HPLC (formic acid method 2): chromatographic column: Welch Xtimate C18, 21.2 × 150 mm, 5 µm; mobile phase A: 0.1% aqueous formic acid solution, mobile phase B: acetonitrile; flow rate: 15 mL/min; column temperature: room temperature.

Prep-HPLC (trifluoroacetic acid method): chromatographic column: Waters XBridge C18, 190 × 250 mm, 5 µm; mobile phase system: A: 0.1% aqueous trifluoroacetic acid solution; B: preparative grade acetonitrile; flow rate: 15 mL/min; B% = 20%-100%; column temperature: room temperature.

Prep-HPLC (hydrochloric acid method): chromatographic column: Waters XBridge C18, 190 × 250 mm, 5 µm; mobile phase system: A: 0.1% aqueous hydrochloric acid solution; B: preparative grade acetonitrile; flow rate: 15 mL/min; B% = 20%-100%; column temperature: room temperature.

Prep-HPLC (ammonia water method): chromatographic column: Waters Xbridge C18, 19 × 150 mm, 5 µm; mobile phase A: 0.1% ammonia water solution; mobile phase B: acetonitrile; flow rate: 15 mL/min; column temperature: room temperature.

3 min LCMS conditions: A: water (0.05% TFA), B: ACN (0.05% TFA); elution gradient: 5%-95% B in 3 min; flow rate: 1.7 mL/min; column type: SunFire C18, 4.6 × 50 mm, 3.5 µm; column temperature: 40 °C. Analytical HPLC conditions: A: water (0.05% TFA), B: ACN (0.05% TFA); elution gradient: 10%-95% B in 15 min; flow rate: 1 mL/min; column type: SunFire C18, 4.6 × 150 mm, 3.5 µm; column temperature: 40 °C. Single crystal diffraction instrument parameters:

| | | | |
|---|---|---|---|
| Light source: | Cu target | X-ray: | Cu-Ka (= 1.54178 Å) |
| Detector: | CMOS area detector | Resolution: | 0.86 Å |
| Current and voltage: | 50 kV, 1.2 mA | Exposure time: | 5 s |
| Area detector-to-sample distance: | 40 mm | Test temperature: | 170(2) K |

### Preparation Example 1. Synthesis of Intermediate 1

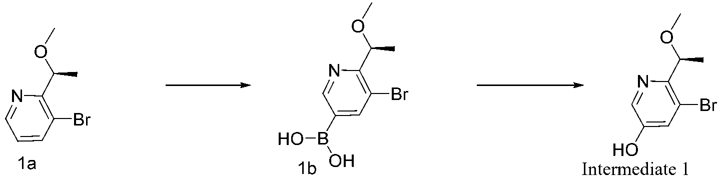

Step 1: Compound 1a (10 g, 46.279 mmol) was weighed out and dissolved in tetrahydrofuran (100 mL), and bis(pinacolato)diboron (17.63 g, 69.419 mmol), chloro(1,5-cyclooctadiene)iridium(I) dimer (0.93 g, 1.388 mmol), and 4,4'-di-tert-butyl-2,2'-dipyridine (1.86 g, 6.942 mmol) were added. The mixture was reacted at 80 °C for 15 h under a nitrogen atmosphere, filtered through celite, and concentrated under reduced pressure to give crude compound 1b, which was directly used in the next step. ES-API: [M+H⁺] = 260.0.

Step 2: The crude compound 1b obtained in the previous step was dissolved in acetonitrile (100 mL), and hydrogen peroxide (1308.25 mg, 11.543 mmol) was added dropwise under an ice-water bath. The mixture was reacted at room temperature for 3 h. A saturated sodium sulfite solution (20 mL) was added to quench the reaction. The reaction mixture was concentrated under reduced pressure and extracted with dichloromethane (25 mL × 3). The organic phase was washed with water (20 mL) and saturated brine (20 mL), dried over anhydrous sodium sulfate, and concentrated under reduced pressure to give a crude product.

The crude product was purified by flash silica gel column chromatography (petroleum ether/ethyl acetate = 1/1) to give intermediate 1 (7000 mg, 30.162 mmol, yield: 87.10%) as a yellow solid, ES-API [M+H]⁺ = 232.1, 234.1.

### Preparation Example 2. Synthesis of Intermediate 4-1

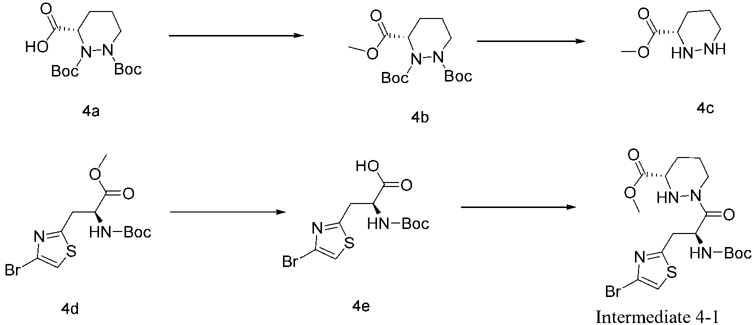

Step 1: Compound 4a (60 g, 181.609 mmol) was dissolved in DMF (200 mL). Sodium bicarbonate (45.77 g, 544.827 mmol) was added, and iodomethane (33.918 mL, 544.827 mmol) was added dropwise under an ice-water bath under nitrogen atmosphere. The mixture was reacted at room temperature overnight. Water (200 mL) was added, and the mixture was extracted with ethyl acetate (200 mL × 3), washed with water (200 mL) and saturated brine (200 mL × 3), dried over anhydrous sodium sulfate, and concentrated under reduced pressure to give crude compound 4b (65 g, 188.729 mmol, yield: 103.92%) as an oily liquid. ES-API [M-156+H]⁺ = 189.1.

Step 2: Crude compound 4b (65 g, 188.729 mmol) was dissolved in dichloromethane (200 mL). Trifluoroacetic acid (200 mL) was added dropwise under an ice-water bath under nitrogen atmosphere. The mixture was reacted at room temperature overnight and concentrated under reduced pressure to give crude compound 4c as a colorless transparent oily liquid, which was directly used in the next step. ES-API [M+H] + = 145.1.

Step 3: Compound 4d (50 g, 136.896 mmol) was dissolved in tetrahydrofuran (300 mL) and water (75 mL), and lithium hydroxide monohydrate (9.38 g, 223.415 mmol) was added. The mixture was reacted at room temperature for 4 h, adjusted to pH 3 with 1 N HCl, extracted with ethyl acetate (200 mL × 3), washed with water (200 mL × 3), washed with saturated brine (200 mL), dried over anhydrous sodium sulfate, and concentrated under reduced pressure to give compound 4e (48 g, 136.670 mmol, yield: 99.83%) as a colorless transparent oily liquid. ES-API [M-56+H]⁺ = 295.0.

Step 4: Compound 4c of step 2 was dissolved in anhydrous dichloromethane (200 mL). Under nitrogen atmosphere, NMM (100 g, 996 mmol) was added dropwise under an ice-water bath, and compound 4e (35 g, 99.655 mmol), EDCI (38.07 g, 199.311 mmol), and HOBt (4.04 g, 29.897 mmol) were added sequentially. The mixture was reacted at room temperature for 4 h, washed sequentially with water (200 mL), 1 N HCl (80 mL), and saturated brine (200 mL), dried over anhydrous sodium sulfate, and concentrated under reduced pressure. The residue was slurried (petroleum ether/ethyl acetate = 8/1) to give compound intermediate 4-1 (33 g, 69.129 mmol). The mother liquor was then purified by flash silica gel column chromatography to give compound intermediate 4-1 (4 g, 8.34 mmol). Compound intermediate 4-1 (in a total amount of 37 g, 77.5 mmol, yield: 78.7%) was obtained as a white solid, ES-API [M-56+H]⁺ = 477.1.

### Preparation Example 3. Synthesis of Intermediate 11

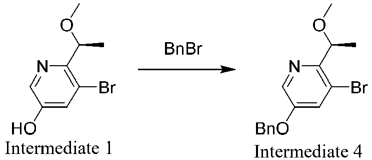

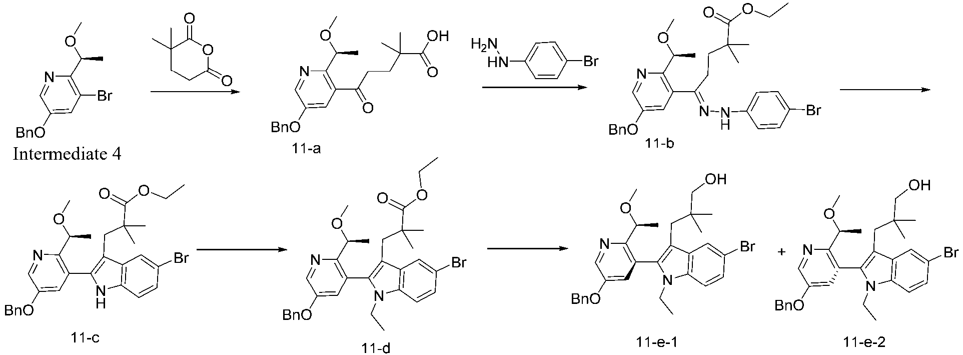

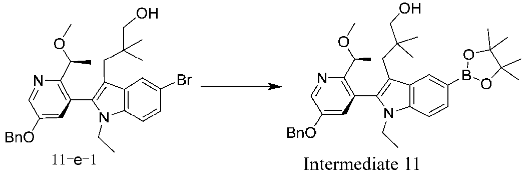

Step 1: Intermediate 1 (500 mg, 2.154 mmol) was dissolved in N,N-dimethylformamide (15 mL), and benzyl bromide (737 mg, 4.308 mmol) and potassium carbonate (893 mg, 6.462 mmol) were added to the above solution. After the addition was completed, the reaction system was stirred at 80 °C for 17 h. After the reaction was completed, water (30 mL) was added to the reaction mixture, and the resulting mixture was extracted with ethyl acetate (20 mL × 2). The organic phase was separated, washed with saturated brine (40 mL × 3), dried over anhydrous sodium sulfate, filtered, and concentrated to give a crude product. The crude product was purified by flash silica gel column chromatography (methanol/dichloromethane: 0%-2%) to give compound intermediate 4 (600 mg, 1.862 mmol, yield: 86.44%) as a white solid. ES-API: [M+H]⁺ = 322.1, 324.1.

Step 2: Isopropylmagnesium chloride-lithium chloride complex (10.027 mL, 13.035 mmol, a 1.3 M solution in tetrahydrofuran) was slowly added dropwise to a solution of intermediate 4 (3 g, 9.311 mmol) in tetrahydrofuran (20 mL) at 0 °C under nitrogen atmosphere, and the mixture was stirred at 0 °C for 0.5 h. The above solution was then added dropwise to a solution of 3,3-dimethyltetrahydropyran-2,6-dione (1.59 g, 11.173 mmol) in tetrahydrofuran (20 mL) at -10 °C, and the mixture was stirred at 0 °C for 2 h. 4 M hydrochloric acid/dioxane (4 mL) was added dropwise to adjust the pH of the solution to 4-5 to quench the reaction. The reaction mixture was extracted with ethyl acetate (20 mL × 3), dried over anhydrous sodium sulfate, filtered, and concentrated. The residue was then purified by silica gel column chromatography (petroleum ether/ethyl acetate = 1/1) to give compound 11-a (2.25 g, 5.837 mmol, yield: 62.69%). ES-API: [M+H]⁺ = 386.2.

Step 3: (4-Bromophenyl)hydrazine (970.36 mg, 5.188 mmol) and a 4 M solution of hydrochloric acid in 1,4-dioxane (3.891 mL) were added to a solution of compound 11-a (1000 mg, 2.594 mmol) in ethanol (20 mL), and the reaction mixture was stirred at 90 °C for 36 h. The mixture was concentrated, and the pH was adjusted to neutrality by adding an aqueous sodium bicarbonate solution. The mixture was diluted with water, extracted with ethyl acetate (20 mL × 3), dried over anhydrous sodium sulfate, filtered, and concentrated. The residue was then purified by silica gel column chromatography (petroleum ether/ethyl acetate = 1/2) to give compound 11-b (1.15 g, 1.974 mmol, yield: 76.09%). ES-API: [M+H]⁺ = 582.2, 584.2.

Step 4: Compound 11-b (1.15 g, 1.974 mmol) was dissolved in trifluoroacetic acid (10 mL). The mixture was heated to 65 °C, stirred for 2 h, concentrated under reduced pressure, and diluted with ethyl acetate. The residue was adjusted to pH 7-8 with saturated ammonium bicarbonate and then extracted with ethyl acetate (20 mL × 3). The organic layers were combined, dried over anhydrous sodium sulfate, and filtered. The filtrate was concentrated under reduced pressure and then purified by silica gel column chromatography (petroleum ether/ethyl acetate = 1/2) to give compound 11-c (780 mg, 1.379 mmol, yield: 69.87%). ES-API: [M+H]⁺ = 565.2, 567.2.

Step 5: Compound 11-c (780 mg, 1.379 mmol) was dissolved in dry N,N-dimethylformamide (5 mL), and cesium carbonate (1347.83 mg, 4.137 mmol) and iodoethane (1075.41 mg, 6.895 mmol) were added sequentially. The mixture was reacted with stirring at room temperature for 2 h under nitrogen atmosphere. The mixture was extracted with ethyl acetate (20 mL × 3). The organic layers were combined, washed sequentially with water (60 mL) and saturated brine (60 mL), dried over anhydrous sodium sulfate, and filtered. The filtrate was concentrated under reduced pressure and then purified by silica gel column chromatography (petroleum ether/ethyl acetate = 1/2) to give compound 11-d (750 mg, 1.264 mmol, yield: 91.61%). ES-API: [M+H]⁺ = 593.2, 595.2.

Step 6: Compound 11-d (750 mg, 1.264 mmol) was dissolved in dry tetrahydrofuran (10 mL). The solution was cooled to 0 °C in an ice-water bath, and lithium aluminum hydride (3.159 mL, 3.159 mmol, 1 M tetrahydrofuran solution) was slowly added dropwise under nitrogen atmosphere. After the dropwise addition was completed, the mixture was further stirred at 0 °C for 1 h. After the reaction was completed as detected by LCMS, water (0.12 mL), an aqueous sodium hydroxide solution (0.12 mL, 15%), and water (0.36 mL) were slowly added sequentially to quench the reaction. The suspension was stirred at room temperature for 30 min, filtered through celite, and washed with ethyl acetate (100 mL). The mother liquor was dried over anhydrous sodium sulfate, filtered, and concentrated. The residue was then purified by flash silica gel column chromatography (ethyl acetate/petroleum ether = 20%-60%) to give 2 intermediate compounds. One of the intermediate compounds has the structure of: 3-((Rₐ)-2-(5-(benzyloxy)-2-((1S)-1-methoxyethyl)pyridin-3-yl)-5-bromo-1-ethylindol-3-yl)-2,2-dimethylpropan-1-ol (11-e-1, 300 mg, 0.544 mmol, yield: 43.05%). 11-e-1: Rt = 1.847 min (3 min LCMS). ¹H NMR (400 MHz, DMSO) δ 8.48 (d, J= 2.8 Hz, 1H), 7.84 (d, *J=* 2.0 Hz, 1H), 7.45 - 7.30 (m, 7H), 7.25 - 7.20 (m, 1H), 5.28 - 5.17 (m, 2H), 4.46 (t, *J* = 5.2 Hz, 1H), 4.00 - 3.90 (m, 2H), 3.82 - 3.72 (m, 1H), 3.02 - 2.90 (m, 2H), 2.79 (s, 3H), 2.55 (d, *J =* 14.0 Hz, 1H), 2.09 (d, *J =* 14.0 Hz, 1H), 1.32 (d, *J* = 6.0 Hz, 3H), 1.00 (t, *J* = 7.2 Hz, 3H), 0.55 - 0.48 (m, 6H). ES-API: [M+H]⁺ = 551.2, 553.2.

The other intermediate compound has the structure of: 3-((Sₐ)-2-(5-(benzyloxy)-2-((1S)-1-methoxyethyl)pyridin-3-yl)-5-bromo-1-ethylindol-3-yl)-2,2-dimethylpropan-1-ol (11-e-2, 210 mg, 0.381 mmol, yield: 30.13%). ES-API: [M+H]⁺ = 551.2, 553.2. 11-e-2: Rt = 1.758 min (3 min LCMS).

Step 7: Compound 11-e-1 (18 g, 32.6 mmol), bis(pinacolato)diboron (16.6 g, 65.2 mmol), potassium acetate (6.41 g, 65.27 mmol), and Pd(dppf)Cl ₂ (2.39 g, 3.26 mmol) were mixed in anhydrous toluene (200 mL) under nitrogen atmosphere, and the system was stirred at 90 °C for 4 h. After the reaction was completed, the mixture was cooled and concentrated to remove toluene, and 300 mL of ethyl acetate and 500 mL of water were poured into the mixture. The resulting mixture was extracted 3 times with ethyl acetate and washed sequentially with 500 mL of water and 500 mL of saturated brine. The organic phase was dried over anhydrous sodium sulfate, filtered, and concentrated, and the residue was purified by flash silica gel column chromatography (0-50% petroleum ether/ethyl acetate) to give compound intermediate 11 (20.8 g, yield: 85%). ES-API [M+H]⁺ = 599.3.

### Single-crystal culture of intermediate 11-e-1

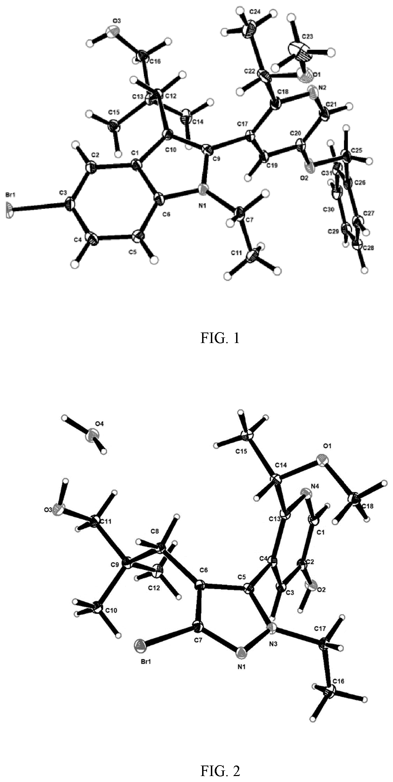

Single-crystal culture: About 10 mg of a powder sample of intermediate 11-e-1 was weighed out, and 1 mL of acetonitrile was added. The mixture was completely dissolved by ultrasonication and placed in a fume hood for slow volatilization. After a period of time, a yellowish-brown needle-like crystal was precipitated, which was the single crystal of intermediate 11-e-1. The X-ray single-crystal diffraction test was performed using a Bruker D8 Venture instrument. The results are shown in Table 1 below and FIG. 1. FIG. 1 shows an ellipsoid plot of the molecular three-dimensional structure.

### Structure analysis and refinement process:

The diffraction data were integrated and reduced using the SAINT program, followed by empirical absorption correction using the SADABS program. The single crystal structure was solved by the direct method using SHELXT2014 and refined by the least square method. For the refinement of hydrogen atoms, isotropic calculation was adopted: hydrogen atoms attached to N and O were located from residual electron density, while those on C-H bonds were added geometrically and refined using a riding model. The Flack constant was 0.035(14). In FIG. 1, C22 is in S configuration, and the axial chirality at positions C9-C17 is in Rₐ configuration.

### Crystal data:

**Table 1**

| | |
|---|---|
| Molecular formula | C₃₀H₃₅BrN₂O₃ |
| Molecular weight | *Mᵣ* = 551.51 |
| Shape | Block, colorless |
| Crystal system | Orthorhombic |
| Space group | *P*2₁2₁2₁ |
| Unit cell parameter | *a* = 7.3026(2) Å; *b* = 17.4557(4) Å; *c* = 20.6871(5) Å |
| Unit cell volume | *V* = 2637.03(11) Å³ |
| Molecule number in unit cell | *Z* = 4 |
| Unit cell dimension | 0.19 × 0.08 × 0.05 mm |
| Electron number in unit cell | *F*(000) = 1152 |
| Calculating density | *D*ₓ = 1.389 Mg m⁻³ |
| Radiation source and wavelength | Cu *K*a radiation, l = 1.54178 Å |
| Determining the number of diffraction points of unit cell | Cell parameters from 9899 reflections |
| Range for data collection | q = 2.5-63.8° |
| Absorption coefficient | m = 2.39 mm⁻¹ |
| Temperature | *T* = 170 K |

### Preparation Example 4. Synthesis of Intermediate 10-1

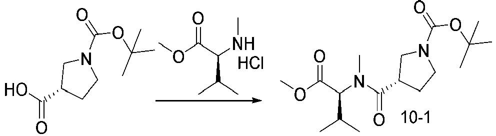

Step 1: (S)-1-Boc-3-carboxypyrrolidine (1.14 g, 5.285 mmol) was dissolved in dichloromethane (40 mL), and methyl N-methyl-L-valinate hydrochloride (800 mg, 4.404 mmol), O-(7-azabenzotriazol-1-yl)-N,N,N',N'-tetramethyluronium hexafluorophosphate (2.51 g, 6.606 mmol), and N,N'-diisopropylethylamine (2.84 g, 22.019 mmol) were added at 0 °C. The reaction system was stirred at room temperature for 1 h. Water (15 mL) was added to the reaction mixture, and the resulting mixture was extracted with dichloromethane (25 mL). The organic phase was washed with saturated brine (15 mL), dried over anhydrous sodium sulfate, and filtered, and the filtrate was concentrated under reduced pressure. The crude product was purified by flash silica gel column chromatography (tetrahydrofuran/petroleum ether: 0-30%) to give compound intermediate 10-1 (900 mg, yield: 59.7%) as a colorless liquid. ES-API: [M+Na]⁺ = 365.2.

### Preparation Example 5. Synthesis of Intermediate 62-1

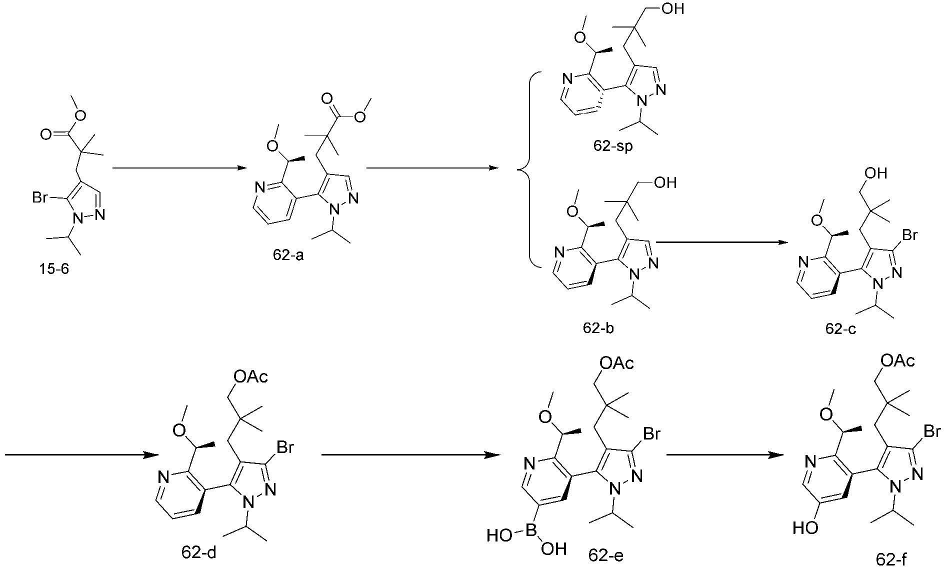

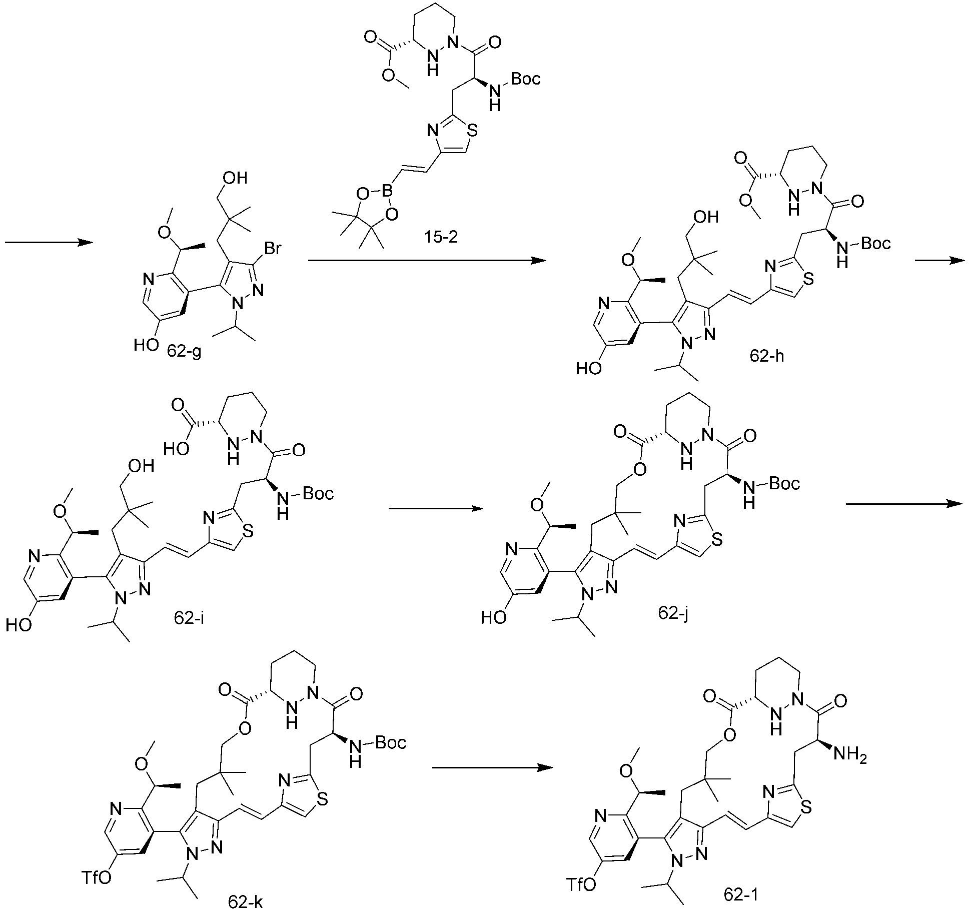

Step 1: Compound 15-6 (10.5 g, 34.63 mmol), rac-(R)-2-(1-methoxyethyl)-3-(4,4,5,5-tetramethyl-1,3,2-dioxaborolan-2-yl)pyridine (16.6 g, 63.084 mmol), CataCXium A Pd G3 (2.52 g, 3.463 mmol), toluene (30 mL), water (30 mL), and potassium phosphate (22.05 g, 103.892 mmol) were mixed in dioxane (90 mL) under nitrogen atmosphere, and the mixture was stirred at 110 °C for 4 h. After the reaction was completed, the reaction mixture was poured into 300 mL of ethyl acetate and washed sequentially with 150 mL of water and 150 mL of saturated brine. The organic phase was dried over anhydrous sodium sulfate, filtered, and concentrated, and the residue was purified by flash silica gel column chromatography (0-80% petroleum ether/ethyl acetate) to give compound 62-a (12 g, 33.382 mmol, yield: 96.40%). ES-API: [M+H]⁺ = 360.3. Step 2: Compound 62-a (9.5 g, 26.428 mmol) was dissolved in tetrahydrofuran (300 mL), and the solution was cooled to 0 °C under nitrogen atmosphere. A lithium aluminum hydride solution (52.856 mL, 52.856 mmol) was slowly added, and the mixture was stirred at room temperature for 1 h, diluted with an appropriate amount of tetrahydrofuran, and then cooled to 0 °C. 2 mL of water was slowly added, 2 mL of a 15% aqueous sodium hydroxide solution was added, followed by 6 mL of water. The mixture was warmed to room temperature and stirred for 15 min. An appropriate amount of anhydrous sodium sulfate was added, and the mixture was stirred for 15 min and then filtered to remove salts. The filter cake was washed with ethyl acetate, concentrated, dried, and purified by silica gel column chromatography (petroleum ether/ethyl acetate = 10:90) to give compound 62-b (2.8 g, 8.447 mmol, yield: 31.96%, retention time: 1.54 min, structure arbitrarily assigned), ES-API [M+H]⁺ = 332.2; and 62-sp (2.5 g, 7.53 mmol, yield: 28.549%, retention time: 1.51 min, structure arbitrarily assigned), ES-API [M+H]⁺ = 332.2.

Step 3: Compound 62-b (2 g, 6.034 mmol) and NBS (2.15 g, 12.068 mmol) were mixed in N,N-dimethylformamide (5 mL) under nitrogen atmosphere, and the mixture was stirred at 50 °C for 2 h. After the reaction was completed, 30 mL of ethyl acetate was poured into the reaction mixture, and the resulting mixture was washed sequentially with 15 mL of water and 15 mL of saturated brine. The organic phase was dried over anhydrous sodium sulfate, filtered, and concentrated, and the residue was purified by flash silica gel column chromatography (0-80% petroleum ether/ethyl acetate) to give compound 62-c (2.2 g, 5.361 mmol, yield: 88.85%), ES-API [M+H]⁺ = 410.1/412.1.

Step 4: Compound 62-c (2 g, 4.874 mmol), DMAP (0.06 g, 0.487 mmol), acetic anhydride (0.915 mL, 9.748 mmol), and DIPEA (1.89 g, 14.621 mmol) were mixed in dichloromethane (50 mL) under nitrogen atmosphere, and the mixture was stirred at 60 °C for 18 h. After the reaction was completed, the reaction mixture was poured into 100 mL of dichloromethane and washed sequentially with 150 mL of water and 150 mL of saturated brine. The organic phase was dried over anhydrous sodium sulfate, filtered, and concentrated to give compound 62-d (2.2 g, 4.87 mmol, yield: 100%), ES-API [M+H]⁺ = 452.1/454.1.

Step 5: Compound 62-d (2.2 g, 4.863 mmol), bis(pinacolato)diboron (2.47 g, 9.726 mmol), chloro(1,5-cyclooctadiene)iridium(I) dimer (0.16 g, 0.243 mmol), and 4,4'-di-tert-butyl-2,2'-bipyridine (0.13 g, 0.486 mmol) were mixed in cyclohexane (50 mL) under nitrogen atmosphere, and the mixture was stirred at 80 °C for 18 h. After the reaction was completed, the reaction mixture was concentrated to give crude compound 62-e (2.38 g).

Step 6: Compound 62-e (2.38 g, 4.8 mmol) was dissolved in acetonitrile (60 mL), and H₂O₂ (1.632 mL, 14.400 mmol) was added at 0 °C. The mixture was stirred at 20 °C for 2 h. After the reaction was completed, the reaction mixture was poured into 100 mL of ethyl acetate and an excess amount of Na₂S₂O₃ solution, and the resulting mixture was washed sequentially with 150 mL of water and 150 mL of saturated brine. The organic phase was dried over anhydrous sodium sulfate, filtered, concentrated, and purified by flash silica gel column chromatography (0-80% petroleum ether/ethyl acetate) to give compound 62-f (1.4 g, 2.989 mmol, two-step yield: 62%), ES-API [M+H]⁺ = 468.1/470.1.

Step 7: Compound 62-f (800 mg, 1.708 mmol) was dissolved in methanol (3 mL), tetrahydrofuran (3 mL), and water (3 mL). Lithium hydroxide (286.67 mg, 6.832 mmol) was added to the above solution. After the addition was completed, the reaction system was stirred at room temperature for 17 h. After the reaction was completed, the reaction mixture was acidified to weak acidity (about pH 6) with diluted hydrochloric acid (1 M). The resulting solution was extracted with ethyl acetate (20 mL × 3). The organic phase was washed with saturated brine (50 mL), dried, and concentrated to give a crude product. The crude product was purified by flash silica gel column chromatography (ethyl acetate/petroleum ether: 0-100%) to give compound 62-g (700 mg, 1.642 mmol, yield: 96.13%) as a light yellow solid. ES-API: [M+H]⁺ = 426.1, 428.1.

Step 8: Compound 62-g (700 mg, 1.642 mmol) was dissolved in toluene (15 mL), dioxane (3 mL), and water (3 mL). Compound 15-2 (1355.67 mg, 2.463 mmol), potassium phosphate (871.22 mg, 4.105 mmol) and [1,1'-bis(di-tert-butylphosphino)ferrocene]dichloropalladium(II) (160.32 mg, 0.246 mmol) were added to the above solution, and the reaction system was purged 3 times with nitrogen and stirred at 80 °C for 3 h. Ethyl acetate (30 mL) and water (20 mL) were added to the reaction mixture. The organic phase was separated, washed with saturated brine (60 mL), dried over anhydrous sodium sulfate, and filtered, and the filtrate was concentrated under reduced pressure. The crude product was purified by flash silica gel column chromatography (tetrahydrofuran/petroleum ether: 0-50%) to give the target compound 62-h (1100 mg, 1.429 mmol, yield: 87.02%) as a gray solid. ES-API: [M+H]⁺ = 770.3.

Step 9: Compound 62-h (1100 mg, 1.429 mmol) was dissolved in tetrahydrofuran (10 mL) and water (10 mL), and lithium hydroxide (85.72 mg, 3.572 mmol) was added at 0 °C. The reaction system was stirred at room temperature for 18 h. The reaction mixture was adjusted to pH 6 with 1 M diluted hydrochloric acid and extracted with ethyl acetate (20 mL × 3). The organic phase was dried over anhydrous sodium sulfate and concentrated to give compound 62-i (1000 mg, 1.323 mmol, yield: 92.60%) as an oily solid. ES-API: [M+H]⁺ = 756.3.

Step 10: Compound 62-i (1100 mg, 1.455 mmol) was dissolved in dichloromethane (40 mL), and 1-(3-dimethylaminopropyl)-3-ethylcarbodiimide hydrochloride (7811 mg, 40.745 mmol), 1-hydroxybenzotriazole (983 mg, 7.276 mmol), and diisopropylethylamine (7.6 mL, 43.655 mmol) were added to the above solution at room temperature. After the addition was completed, the reaction system was stirred at room temperature for 18 h. Dichloromethane (100 mL) was added to the reaction mixture, and the resulting mixture was washed with diluted hydrochloric acid (0.5 M, 20 mL × 2) and saturated brine (100 mL), dried over anhydrous sodium sulfate, and filtered. The filtrate was concentrated under reduced pressure. The crude product was purified by flash silica gel column chromatography (tetrahydrofuran/petroleum ether: 0-60%) to give compound 62-j (150 mg, 0.203 mmol, yield: 13.97%) as a white solid. ES-API: [M+H]⁺ = 738.3.

Step 11: Compound 62-j (290 mg, 0.393 mmol) was dissolved in dichloromethane (10 mL), and triethylamine (0.164 mL, 1.179 mmol) and 1,1,1-trifluoro-N-phenyl-N-[(trifluoromethyl)sulfonyl]methanesulfonamide (238.67 mg, 0.668 mmol) were added to the above solution. After the addition was completed, the reaction system was stirred at room temperature for 2 h. After the reaction was completed, the reaction mixture was concentrated to give a crude product. The crude product was purified by flash silica gel column chromatography (methanol/dichloromethane: 0%-3%) to give compound 62-k (300 mg, 0.345 mmol, yield: 87.75%) as a white solid. ES-API: [M+H]⁺ = 870.1.

Step 12: Compound 62-k (50 mg, 0.057 mmol) was dissolved in methanol (0.5 mL), and 4 M hydrochloric acid/dioxane (3 mL, 0.196 mmol) was added. The mixture was reacted at room temperature for 1 h under nitrogen atmosphere. After the reaction was completed, the reaction mixture was concentrated under reduced pressure to give intermediate compound 62-1 (39 mg, 0.045 mmol, yield: 86.68%) as a yellow solid, ES-API: [M+H]⁺ = 770.2.

### Preparation Example 6. Synthesis of Intermediate 62-p

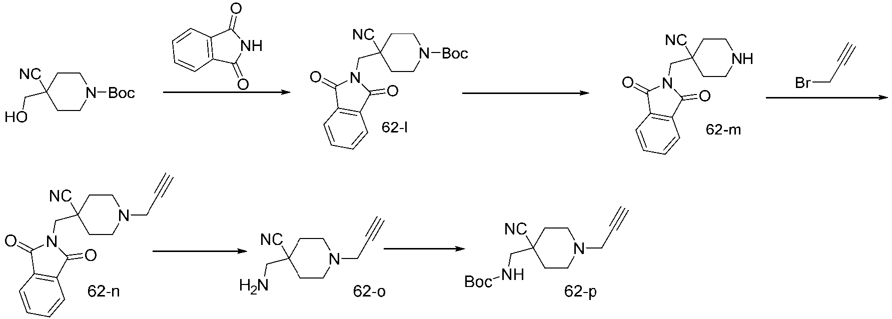

Step 1: 2H-Isoindole-1,3-dione (2.02 g, 13.733 mmol), PPh3 (5.87 g, 22.472 mmol), and DIAD (4.54 g, 22.472 mmol) were added to a solution of tert-butyl 4-cyano-4-(hydroxymethyl)hexahydropyridine-1-carboxylate (3.00 g, 12.4484 mmol) in tetrahydrofuran (150 mL), and the reaction mixture was stirred at 40 °C for 3 h. After the reaction was completed, water (100 mL) and ethyl acetate (100 mL) were added. The organic layer was separated, washed with a saturated sodium chloride solution (100 mL × 3), dried over anhydrous sodium sulfate, filtered, and concentrated. The residue was purified by silica gel column chromatography (ethyl acetate/petroleum ether = 1/2) to give compound 62-l (2500 mg, 6.767 mmol, yield: 54.21%). ES-API: [M+H]⁺ = 370.1.

Step 2: Compound 62-l (2 g, 5.414 mmol) was dissolved in dichloromethane (6 mL), and trifluoroacetic acid (6.219 mL, 81.208 mmol) was added. The mixture was reacted at room temperature for 1 h. The reaction mixture was concentrated in vacuum and dried to give compound 62-m (1.45 g, crude product). ES-API: [M+H]⁺ = 270.1.

Step 3: Compound 62-m (1.45 g, crude product) was dissolved in acetonitrile (15 mL), and potassium carbonate (2.31 g, 16.710 mmol) and 3-bromoprop-1-yne (0.53 g, 4.456 mmol) were added. The mixture was reacted at room temperature for 3 h. After the reaction was completed, water (50 mL) and ethyl acetate (50 mL) were added. The organic layer was separated, washed with a saturated sodium chloride solution (50 mL × 3), dried over anhydrous sodium sulfate, filtered, and concentrated. The residue was purified by silica gel column chromatography to give compound 62-n (800 mg, 2.603 mmol, yield: 46.73%). ES-API: [M+H]⁺ = 308.1.

Step 4: Compound 62-n (800 mg, 2.603 mmol) was dissolved in ethanol (15 mL), and a 30% methylamine/methanol solution (4 g, 39.045 mmol) was added. The mixture was heated to 80 °C and reacted for 6 h. After the reaction was completed, the reaction mixture was concentrated to remove excess methylamine to give compound 62-o (980 mg, crude product). ES-API: [M+H]⁺ = 178.1.

Step 5: Compound 62-o (980 mg, crude product) was dissolved in tetrahydrofuran (15 mL), and a saturated aqueous sodium bicarbonate solution (5 mL) and di-tert-butyl dicarbonate (1.12 mL, 5.2 mmol) were added. The mixture was reacted for 1 h. After the reaction was completed, water (50 mL) and ethyl acetate (50 mL) were added. The organic layer was separated, washed with a saturated sodium chloride solution (50 mL × 3), dried over anhydrous sodium sulfate, filtered, and concentrated. The residue was purified by silica gel column chromatography to give compound 62-p (500 mg, 1.80 mmol, two-step yield: 69.3%). ES-API: [M+H]⁺ = 278.1.

### Preparation Example 7. Synthesis of Intermediate 8-8

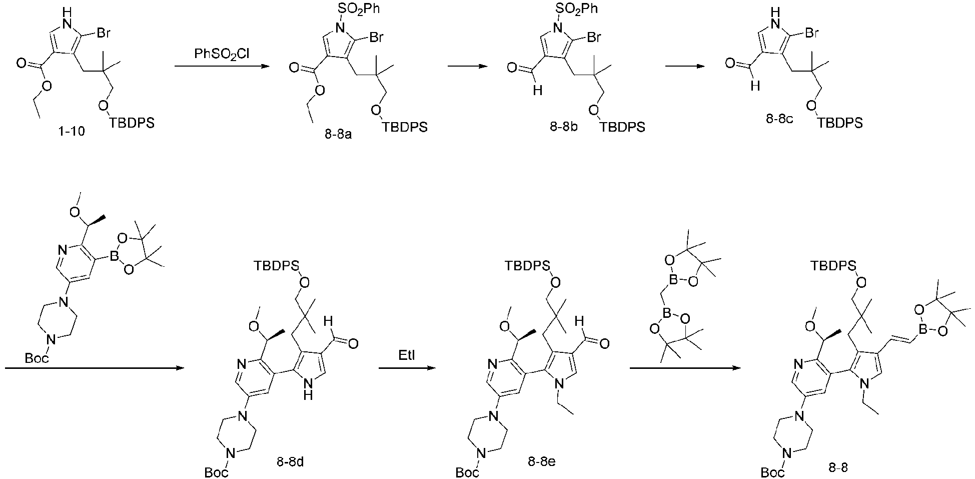

Step 1: Compound 1-10 (5 g, 9.22 mmol) was dissolved in N,N-dimethylformamide (50 mL), and 60% sodium hydride solid (0.55 g, 13.82 mmol) was slowly added under an ice bath. The mixture was stirred for 0.5 h. Subsequently, benzenesulfonyl chloride (1.25 mL, 9.77 mmol) was added, and the mixture was further stirred for 1 h. The reaction was quenched with 50 mL of saturated ammonium chloride solution, and the reaction mixture was extracted three times with 50 mL of ethyl acetate. The organic phases were combined, washed with 50 mL of saturated brine, dried over anhydrous sodium sulfate, filtered, and concentrated. The residue was purified by flash silica gel column chromatography (0-5% tetrahydrofuran/petroleum ether) to give compound 8-8a (6.1 g, yield: 97%) as an oil. ES-API: [M+H]⁺ = 682.0.

Step 2: A 1 M solution of diisobutylaluminum hydride in n-hexane (14.65 mL, 14.65 mmol) was added to a solution of morpholine (1.31 g, 15.01 mmol) in tetrahydrofuran (30 mL) under an ice bath, and the mixture was stirred for 30 min. A solution of compound 8-8a (2.5 g, 3.66 mmol) in tetrahydrofuran (30 mL) was then added to the above reaction mixture, and the resulting mixture was stirred at room temperature for 1 h. After the reaction was completed, the reaction was quenched with 60 mL of 1 M hydrochloric acid solution, and the reaction mixture was extracted three times with 60 mL of ethyl acetate solution. The organic phases were combined, dried over anhydrous sodium sulfate, filtered, and concentrated, and the residue was purified by flash silica gel column chromatography (0-5% tetrahydrofuran/petroleum ether) to give compound 8-8b (2g, yield: 85%) as an oil. ES-API: [M+H]⁺ = 638.0.

Step 3: Compound 8-8b (2 g, 3.13 mmol) was dissolved in tetrahydrofuran (30 mL), and a tetrabutylammonium fluoride solution (5 mL, 5.00 mmol) was added. The mixture was stirred at room temperature for 1 h. The reaction was quenched with 10 mL of saturated ammonium chloride solution, and the reaction mixture was extracted three times with 10 mL of ethyl acetate solution. The organic phases were combined, dried over anhydrous sodium sulfate, filtered, and concentrated, and the residue was purified by flash silica gel column chromatography (0-8% methanol/dichloromethane) to give compound 8-8c (1.5 g, yield: 96%) as a yellow oil. ES-API: [M+H]⁺ = 498.1.

Step 4: A solution of compound 8-8c (1.5 g, 3.01 mmol), tert-butyl (S)-4-(6-(1-methoxyethyl)-5-(4,4,5,5-tetramethyl-1,3,2-dioxaborolan-2-yl)pyridin-3-yl)piperazine-1-carboxylate (2.02 g, 4.51 mmol, prepared by referring to the method in US2021130303A1), [1,1'-bis(diphenylphosphino)ferrocene]palladium(II) dichloride (0.44 g, 0.60 mmol), and potassium carbonate (1.25 g, 9.03 mmol) in water (4 mL) and dioxane (20 mL) was stirred at 100 °C for 2 h under nitrogen atmosphere. The reaction mixture was poured into 50 mL of ethyl acetate, and the resulting mixture was washed sequentially with 15 mL of water and 15 mL of saturated brine. The organic phase was dried over anhydrous sodium sulfate, filtered, and concentrated, and the residue was purified by flash silica gel column chromatography (0-8% methanol/dichloromethane) to give compound 8-8d (1800 mg, yield: 81%). ES-API: [M+H]⁺ = 739.3.

Step 5: Compound 8-8d (1.2 g, 1.62 mmol) was dissolved in tetrahydrofuran (15 mL), and 60% sodium hydride solid (0.19 g, 4.87 mmol) was slowly added under an ice bath. The mixture was stirred for 0.5 h. Subsequently, iodoethane (0.39 mL, 4.87 mmol) was added, and the mixture was further stirred for 1 h. The reaction was quenched with 20 mL of saturated ammonium chloride solution, and the reaction mixture was extracted three times with 20 mL of ethyl acetate. The organic phases were combined, washed with 20 mL of saturated brine, dried over anhydrous sodium sulfate, filtered, and concentrated. The residue was purified by flash silica gel column chromatography (0-2.5% methanol/dichloromethane) to give compound 8-8e (900 mg, yield: 72%) as an oil. ES-API: [M+H]⁺ = 767.3.

Step 6: A 2.5 M solution of n-butyllithium in tetrahydrofuran (0.7 mL, 1.76 mmol) was added to a solution of 2,2,6,6-tetramethylpiperazine (250 mg, 1.76 mmol) in tetrahydrofuran (20 mL) at -60 °C, and the mixture was stirred for 0.5 h. Then the reaction mixture was warmed to 0 °C, and a solution of bis[(pinacolato)boron]methane (472 mg, 1.76 mmol) in tetrahydrofuran (2 mL) was added. The mixture was stirred for 10 min. The reaction mixture was cooled back to -60 °C, and a solution of compound 8-8e (900 mg, 1.17 mmol) in tetrahydrofuran (5 mL) was added. The mixture was stirred for 3 h. The reaction mixture was concentrated and purified by flash silica gel column chromatography (0-6% tetrahydrofuran/petroleum ether ) to give intermediate compound 8-8 (1000 mg, yield: 95.67%). ES-API: [M+H]⁺ = 891.4.

### Preparation Example 8. Synthesis of Intermediate 8-8

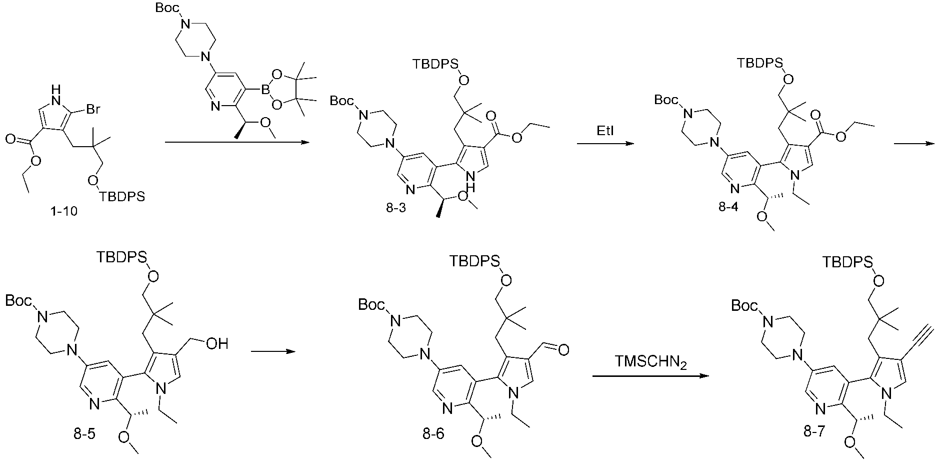

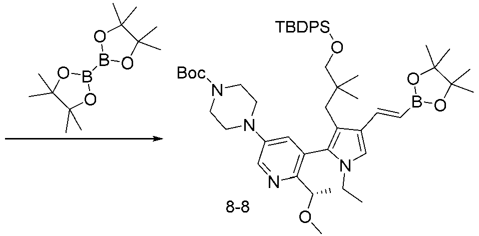

Step 1: A solution of compound 1-10 (4.5 g, 8.29 mmol), 2-[(1S)-1-methoxyethyl]-3-(4,4,5,5-tetramethyl-1,3,2-dioxaborolan-2-yl)pyridine (3.71 g, 8.29 mmol, prepared by referring to the method in US2021130303A1), [1,1'-bis(diphenylphosphino)ferrocene]palladium(II) dichloride (0.61 g, 0.83 mmol), and potassium carbonate (3.44 g, 24.88 mmol) in dioxane/water (10 mL/2 mL) was stirred at 120 °C for 2 h under nitrogen atmosphere. After the reaction was completed, 30 mL of ethyl acetate was added, and the mixture was washed sequentially with 15 mL of water and 15 mL of saturated brine. The organic phase was dried over anhydrous sodium sulfate, filtered, and concentrated, and the residue was purified by flash silica gel column chromatography (0-60% tetrahydrofuran/petroleum ether) to give compound 8-3 (4.3 g, yield: 66%). ES-API: [M+H]⁺ = 783.3.

Step 2: Compound 8-3 (4.3 g, 5.491 mmol) was dissolved in tetrahydrofuran (50 mL). The solution was cooled to 0 °C, and slowly added to sodium hydride (1.10 g, 27.46 mmol), and the mixture was stirred for 0.5 h. Subsequently, iodoethane (1 mL, 12.50 mmol) was added to the reaction mixture, and the resulting mixture was further stirred for 2 h. The reaction was quenched with 10 mL of saturated ammonium chloride solution, and the reaction mixture was extracted three times with 10 mL of ethyl acetate. The organic phase was dried over anhydrous sodium sulfate, filtered, and concentrated, and the residue was purified by flash silica gel column chromatography (0-30% tetrahydrofuran/petroleum ether) to give compound 8-4 (4200 mg, yield: 94.30%) as a yellow oil. ES-API: [M+H]⁺ = 811.3.

Step 3: Compound 8-4 (500 mg, 0.616 mmol) was dissolved in tetrahydrofuran (10 mL) under nitrogen atmosphere, and the solution was cooled to -70 °C. Subsequently, diisobutylaluminum hydride (3.08 mL, 3.08 mmol) was added, and the mixture was stirred for 1 h. After the reaction was completed, the reaction was quenched with sodium sulfate decahydrate. The reaction mixture was purified by flash silica gel column chromatography (0-50% tetrahydrofuran/petroleum ether) to give compound 8-5 (200 mg, yield: 42%) as a yellow oil. ES-API: [M+H]⁺ = 769.3.

Step 4: Dess-Martin periodinane (132 mg, 0.31 mmol) was added to a solution of compound 8-5 (200 mg, 0.260 mmol) in dichloromethane (5 mL), and the mixture was stirred at room temperature for 1 h. After the reaction was completed, 30 mL of dichloromethane was added, and the mixture was washed with 10 mL of water. The organic phase was dried over anhydrous sodium sulfate, filtered, and concentrated, and the residue was purified by flash silica gel column chromatography (0-50% tetrahydrofuran/petroleum ether) to give compound 8-6 (200 mg, yield: 100%). ES-API: [M+H]⁺ = 767.3.

Step 5: A solution of n-butyllithium in tetrahydrofuran (0.28 mL, 0.70 mmol) was slowly added to a solution of (trimethylsilyl)diazomethane (0.35 mL, 0.70 mmol) in tetrahydrofuran (2 mL) at -70 °C, and the mixture was stirred for 0.5 h. A solution of compound 8-6 (180 mg, 0.24 mmol) in tetrahydrofuran (2 mL) was then added to the above solution, and the resulting mixture was stirred for 1 h. The reaction was quenched with 5 mL of saturated ammonium chloride solution, and the reaction mixture was extracted three times with 5 mL of ethyl acetate. The organic phases were combined, dried over anhydrous sodium sulfate, filtered, and concentrated, and the residue was purified by flash silica gel column chromatography (0-80% tetrahydrofuran/petroleum ether) to give compound 8-7 (120 mg, yield: 67.02%). ES-API: [M+H]⁺ = 763.4. Step 6: A solution of copper(I) chloride (8 mg, 0.08 mmol), sodium tert-butoxide (15 mg, 0.16 mmol), and 4,5-bis(diphenylphosphino)-9,9-dimethylxanthene (46 mg, 0.08 mmol) in tetrahydrofuran (1 mL) was stirred at room temperature for 30 min under nitrogen atmosphere. Subsequently, a solution of bis(pinacolato)diboron (48 mg, 0.19 mmol) in tetrahydrofuran (1 mL) was added, and the mixture was further stirred for 10 min. Finally, compound 8-7 (120 mg, 0.16 mmol) and methanol (0.013 mL, 0.32 mmol) were added sequentially, and the mixture was stirred for three hours. The reaction mixture was directly concentrated and purified by flash silica gel column chromatography (0-60% tetrahydrofuran/petroleum ether) to give compound 8-8 (80 mg, yield: 57%) as a white solid. ES-API: [M+H]⁺ = 891.3.

### Preparation Example 9. Synthesis of Intermediate 15-20-1

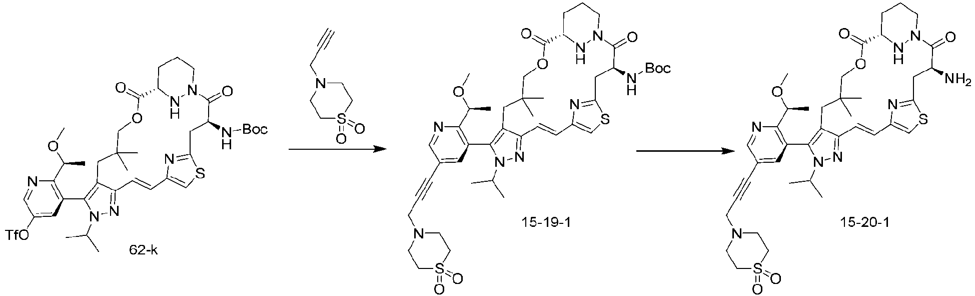

Step 1: 62-k (250 mg, 0.287 mmol) was dissolved in acetonitrile (5 mL). 4-(Prop-2-yn-1-yl)thiomorpholine 1,1-dioxide (149.34 mg, 0.862 mmol), bis(triphenylphosphine)palladium(II) dichloride (40.34 mg, 0.057 mmol), copper(I) iodide (111.43 mg, 0.862 mmol), N,N'-diisopropylethylamine (111.43 mg, 0.862 mmol), and lithium chloride (48.73 mg, 1.149 mmol) were added to the above solution at room temperature. After the addition was completed, the reaction system was stirred at 80°C for 1 h under argon atmosphere. After the reaction was completed, water (30 mL) was added to the reaction mixture, and the resulting mixture was extracted with ethyl acetate (30 mL × 3). The organic phase was washed with saturated brine (30 mL × 2), dried, and concentrated to give a crude product. The crude product was purified by flash silica gel column chromatography (methanol/dichloromethane: 0%-3%) to give intermediate 15-19-1 (150 mg, 0.168 mmol, yield: 58.31%) as a white solid. ES-API: [M+H]⁺ = 893.2.

Step 2: A solution of hydrochloric acid in dioxane (4 M, 4 mL) was added to a solution of compound 15-19-1 (150 mg, 0.168 mmol) in methanol (8 mL) at 0 °C. The reaction mixture was stirred at room temperature for 3 h. After the reaction was completed as detected by LCMS, the pH of the reaction system was adjusted to 6-7. The reaction mixture was concentrated under reduced pressure to obtain a crude product of compound 15-20-1 (125 mg), which was used directly in the next step. ES-API: [M+H]⁺ = 793.2.

### Example 1. Synthesis of Compounds Z1-1 and Z1-2

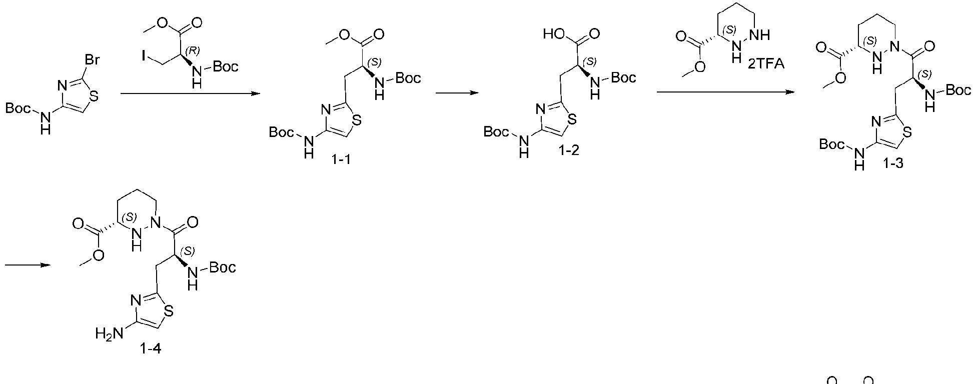

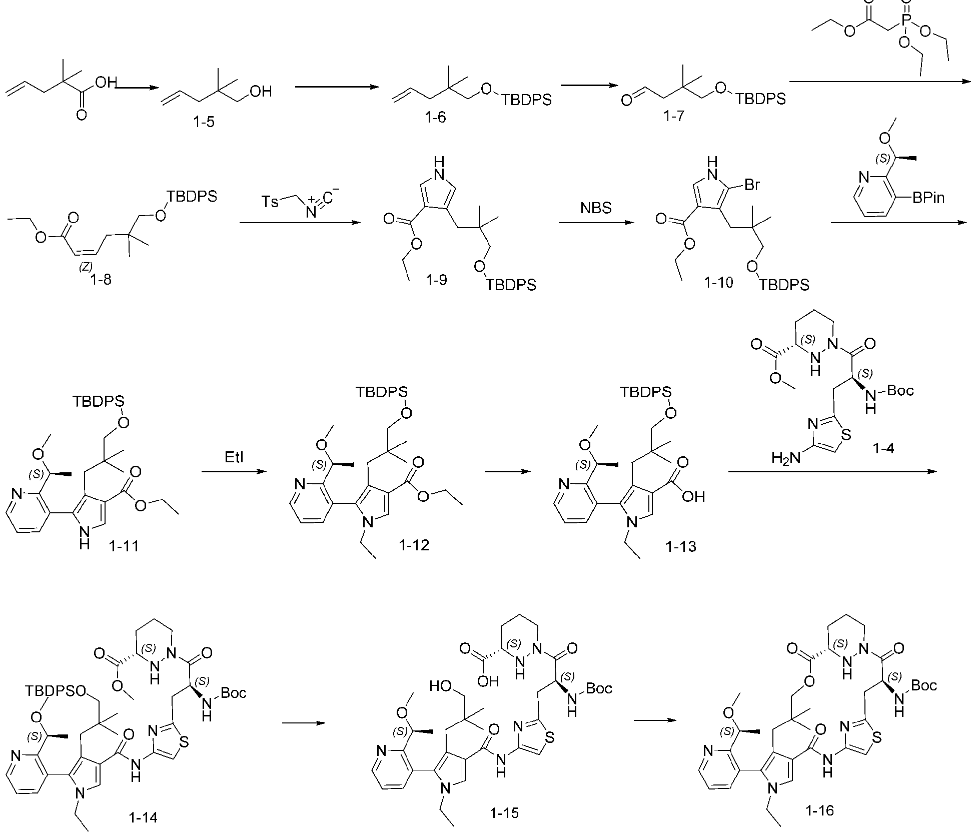

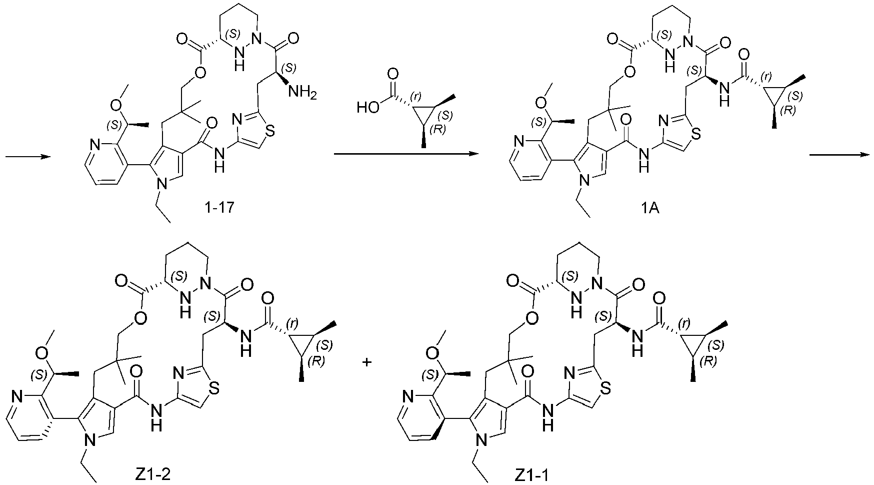

Step 1: Dry N,N-dimethylformamide (50 mL) was added to zinc powder (20 g, 306 mmol) under nitrogen atmosphere. Subsequently, trimethylchlorosilane (4 mL, 31.55 mmol) was added, and the mixture was stirred at 90 °C for half an hour. The reaction mixture was cooled and left to stand, and then most of the N,N-dimethylformamide was removed with a syringe. N,N-dimethylformamide (20 mL) was added again. The mixture was stirred for half a minute and left to stand, and then most of the N,N-dimethylformamide was removed with a syringe. The operation was repeated three times. Dry N,N-dimethylformamide (50 mL) was added to activated zinc powder, and the mixture was cooled to 0 °C. A solution of tert-butyl (R)-2-((tert-butoxycarbonyl)amino)-3-iodopropionate (11 g, 33.42 mmol) in N,N-dimethylformamide (50 mL) was slowly added, and the mixture was stirred for half an hour and left to stand to give a corresponding zinc reagent. The resulting zinc reagent solution (60 mL) was added to tert-butyl (2-bromothiazol-4-yl)carbamate (3 g, 10.75 mmol) and [1,1'-bis(diphenylphosphino)ferrocene]palladium(II) dichloride (0.79 g, 1.08 mmol) in N,N-dimethylformamide (60 mL), and the mixture was reacted at 70 °C for 6 h. After the reaction was completed, 400 mL of ethyl acetate was added, and the mixture was washed sequentially with 100 mL of saturated brine and 100 mL of water. The organic phase was dried over anhydrous sodium sulfate, filtered, and concentrated, and the residue was purified by flash silica gel column chromatography (0-40% ethyl acetate/petroleum ether) to give compound 1-1 (3.6 g, yield: 83%) as a yellow solid. ES-API: [M+H]⁺ = 402.1.

Step 2: Lithium hydroxide (1.32 g, 31.38 mmol) was added to a solution of compound 1-1 (4.2 g, 10.46 mmol) in tetrahydrofuran (40 mL) and water (8 mL), and the mixture was stirred at room temperature for 2 h. After the reaction was completed, the mixture was neutralized to pH = 6 with a 1 M hydrochloric acid solution and extracted three times with 40 mL of ethyl acetate. The organic phases were combined, dried over anhydrous sodium sulfate, filtered, and concentrated to give compound 1-2 (3.9 g) as a yellow solid. ES-API: [M+H]⁺ = 388.1.

Step 3: Methyl (S)-hexahydropyridazine-3-carboxylate bis(trifluoroacetate) (5.62 g, 15 mmol), N-methylmorpholine (11 mL, 101 mmol), 1-(3-dimethylaminopropyl)-3-ethylcarbodiimide hydrochloride (3.86 g, 20 mmol), and 1-hydroxybenzotriazole (0.41 g, 3 mmol) were added sequentially to a solution of compound 1-2 (3.9 g, 10 mmol) in dichloromethane (40 mL) under an ice bath, and the mixture was stirred at room temperature for three hours. After the reaction was completed, the reaction was quenched with 40 mL of 1 M hydrochloric acid solution, and the reaction mixture was extracted three times with 20 mL of dichloromethane. The organic phases were combined, dried over anhydrous sodium sulfate, filtered, and concentrated, and the residue was purified by flash silica gel column chromatography (0-45% tetrahydrofuran/petroleum ether) to give compound 1-3 (4 g, yield: 77%) as a white solid. ES-API: [M+H]⁺ = 514.2.

Step 4: Compound 1-3 (1 g, 1.95 mmol) was dissolved in dichloromethane (3 mL), and trifluoroacetic acid (3 mL) was added. The mixture was stirred at room temperature for 2 h. The reaction mixture was concentrated. The residue was dissolved in dichloromethane (3 mL), and N,N-diisopropylethylamine (3 mL, 17 mmol) and di-tert-butyl carbonate (1 mL, 4.35 mmol) were added. The mixture was stirred for 0.5 h. After the reaction was completed, the reaction mixture was washed with 10 mL of water, concentrated, and purified by flash silica gel column chromatography (0-10% methanol/dichloromethane) to give compound 1-4 (300 mg, yield: 37%). ES-API: [M+H]⁺ = 414.3.

Step 5: A solution of lithium aluminum hydride in tetrahydrofuran (40 mL, 40.000 mmol) was added dropwise to a solution of 2,2-dimethyl-4-pentenoic acid (5 g, 39 mmol) in tetrahydrofuran (100 mL) under an ice bath under nitrogen atmosphere, and the mixture was stirred at room temperature for 4 h. The reaction was sequentially quenched with water (2.6 mL), 10% sodium hydroxide solution (2.6 mL), and water (7.2 mL), and the reaction mixture was dried over anhydrous sodium sulfate, filtered, and concentrated to give compound 1-5 (3.5 g, yield: 78%) as a colorless oil.

Step 6: Imidazole (2.30 g, 33.72 mmol) and tert-butyldiphenylchlorosilane (8 mL, 30.65 mmol) were added to a solution of compound 1-5 (3.5 g, 30.65 mmol) in dichloromethane (50 mL) under an ice bath, and the mixture was stirred at room temperature for 1 h. The reaction mixture was filtered and washed with 50 mL of water. The organic phase was dried and concentrated, and the residue was purified by flash silica gel column chromatography (0-30% ethyl acetate/petroleum ether) to give compound 1-6 (9 g, yield: 83%).

Step 7: Compound 1-6 (6.9 g, 19.57 mmol) was dissolved in tetrahydrofuran (30 mL) and water (30 mL) under an ice bath, and potassium osmate dihydrate (360 mg, 1 mmol), 2,6-dimethylpyridine (4.6 mL, 39 mmol), and sodium periodate (25 g, 117 mmol) were added sequentially. The mixture was stirred at room temperature for 2 h. After the reaction was completed, the reaction mixture was filtered, 30 mL of water was added to the filtrate, and the resulting mixture was extracted three times with 50 mL of ethyl acetate. The organic phases were combined, dried over anhydrous sodium sulfate, filtered, and concentrated, and the residue was purified by flash silica gel column chromatography (0-5% tetrahydrofuran/petroleum ether) to give compound 1-7 (5.3 g, yield: 76%) as a colorless oil. ES-API: [M+H]⁺ = 355.3.

Step 8: Sodium hydride (0.78 g, 19.44 mmol) was added to a solution of triethyl phosphorylacetate (4.36 g, 19.43 mmol) in tetrahydrofuran (20 mL) under an ice bath, and the mixture was stirred for 0.5 h. Subsequently, a solution of compound 1-7 (5.3 g, 14.95 mmol) in tetrahydrofuran was added, and the mixture was further stirred for 2 h. After the reaction was completed, the reaction was quenched with 20 mL of saturated ammonium chloride solution, and the reaction mixture was extracted three times with 20 mL of ethyl acetate. The organic phases were combined, dried over anhydrous sodium sulfate, filtered, and concentrated, and the residue was purified by flash silica gel column chromatography (0-15% tetrahydrofuran/petroleum ether) to give compound 1-8 (4.8 g, yield: 76%) as an oil. ES-API: [M+Na]⁺ = 447.2.

Step 9: A solution of potassium tert-butoxide in tetrahydrofuran (8 mL, 8 mmol) was added to a solution of compound 1-8 (1.14 g, 2.69 mmol) and p-toluenesulfonylmethyl isocyanide (0.68 g, 3.49 mmol) in tetrahydrofuran (20 mL) under an ice bath, and the mixture was stirred at room temperature for 2 h. The reaction was quenched with 20 mL of saturated ammonium chloride solution, and the reaction mixture was extracted three times with 20 mL of ethyl acetate. The organic phases were combined, dried over anhydrous sodium sulfate, filtered, and concentrated, and the residue was purified by flash silica gel column chromatography (0-20% tetrahydrofuran/petroleum ether) to give compound 1-9 (960 mg, yield: 77%) as an oil. ES-API: [M+H]⁺ = 464.2.

Step 10: N-bromosuccinimide (154 mg, 0.86 mmol) was added to a solution of compound 1-9 (400 mL, 0.86 mmol) in tetrahydrofuran (5 mL) under an ice bath, and the mixture was stirred for 1 h. 20 mL of ethyl acetate was poured into the reaction mixture. The resulting mixture was washed with 10 mL of water, dried over anhydrous sodium sulfate, filtered, and concentrated, and the residue was purified by flash silica gel column chromatography (0-20% tetrahydrofuran/petroleum ether) to give compound 1-10 (350 mg, yield: 75%). ES-API: [M+H]⁺ = 542.2, 544.2.

Step 11: A mixture of compound 1-10 (2 g, 3.69 mmol), (S)-2-(1-methoxyethyl)-3-(4,4,5,5-tetramethyl-1,3,2-dioxaborolan-2-yl)pyridine (0.97 g, 3.69 mmol), [1,1'-bis(di-tert-butylphosphino)ferrocene]dichloropalladium(II) (0.27 g, 0.37 mmol), and potassium carbonate (1.53 g, 11.09 mmol) in dioxane (20 mL) and water (4 mL) was stirred at 120 °C for 2 h under nitrogen atmosphere. After the reaction was completed, the reaction mixture was poured into 50 mL of ethyl acetate, and the resulting mixture was washed sequentially with 10 mL of saturated brine and 10 mL of water. The organic phase was dried over anhydrous sodium sulfate, filtered, and concentrated, and the residue was purified by flash silica gel column chromatography (0-100% tetrahydrofuran/petroleum ether) to give compound 1-11 (1.1 g, yield: 50%) as a transparent oil. ES-API: [M+H]⁺ = 599.3.

Step 12: Compound 1-11 (1.1 g, 1.84 mmol) was dissolved in tetrahydrofuran (20 mL) under an ice bath, and sodium hydride (0.37 g, 9.18 mmol) was added. The mixture was stirred for 0.5 h. Subsequently, iodoethane (570 mg, 3.67 mmol) was added, and the mixture was stirred at room temperature for 2 h. The reaction was quenched with 20 mL of saturated ammonium chloride solution, and the reaction mixture was extracted three times with 20 mL of ethyl acetate. The organic phases were combined, dried over anhydrous sodium sulfate, filtered, and concentrated, and the residue was purified by flash silica gel column chromatography (0-30% tetrahydrofuran/petroleum ether) to give compound 1-12 (1 g, yield: 87%) as an oil. ES-API: [M+H]⁺ = 627.4. Step 13: Compound 1-12 (200 mg, 0.32 mmol) was dissolved in methanol (3 mL) and water (0.6 mL), and potassium hydroxide (300 mg, 5.4 mmol) was added. The mixture was stirred at 95 °C overnight. After the reaction was completed, the reaction mixture was concentrated. The residue was dissolved in 10 mL of water, neutralized to pH 6 with a 1 M hydrochloric acid solution, extracted with ethyl acetate, dried, and concentrated, and the residue was purified by flash silica gel column chromatography (0-70% tetrahydrofuran/petroleum ether) to give compound 1-13 (150 mg, yield: 79%). ES-API: [M+H]⁺ = 599.3.

Step 14: Compound 1-13 (210 mg, 0.35 mmol) was dissolved in dichloromethane (5 mL) under an ice bath, and oxalyl chloride (59 µL, 0.70 mmol) and one drop of N,N-dimethylformamide were added. The mixture was stirred for 1 h. The mixture was concentrated. The residue was redissolved in 5 mL of dichloromethane, and pyridine (0.5 mL) was added, followed by the addition of compound 1-4 (174 mg, 0.42 mmol). The mixture was stirred for 1 h. After the reaction was completed, the reaction mixture was washed with water (2 mL), dried over anhydrous sodium sulfate, filtered, and concentrated, and the residue was purified by flash silica gel column chromatography (methanol/dichloromethane = 0-5%) to give compound 1-14 (220 mg, yield: 63%). ES-API: [M+H]⁺ = 994.2.

Step 15: Compound 1-14 (210 mg, 0.211 mmol) was dissolved in a solution of tetrabutylammonium fluoride in tetrahydrofuran (2 mL, 2 mmol), and the mixture was stirred at 60 °C for 5 h. The reaction mixture was concentrated, and the residue was purified by flash silica gel column chromatography (0-10% methanol/dichloromethane) to give compound 1-15 (150 mg, yield: 96%). ES-API: [M+H]⁺ = 742.1.

Step 16: A solution of compound 1-15 (200 mg, 0.27 mmol) in dichloroethane (30 mL) was slowly added dropwise to a solution of N,N'-dicyclohexylcarbodiimide (556 mg, 2.70 mmol), pyridine (3.43 g, 27 mmol), and 4-methylbenzenesulfonate (677 mg, 2.70 mmol) in dichloroethane (30 mL) at 95 °C, and the mixture was stirred for 3 h. After the reaction was completed, the reaction mixture was cooled, washed with 30 mL of 1 M hydrochloric acid, and concentrated, and the residue was purified by flash silica gel column chromatography (0-100% tetrahydrofuran/petroleum ether) to give compound 1-16 (80 mg, yield: 41%). ES-API: [M+H]⁺ = 724.2.

Step 17: Compound 1-16 (70 mg, 0.1 mmol) was dissolved in methanol (0.2 mL), and a 4 M solution of hydrochloric acid in dioxane (1 mL, 4 mmol) was added. The mixture was stirred for 1 h. The reaction mixture was concentrated to give compound 1-17 (60 mg). ES-API: [M+H]⁺ = 624.0.

Step 18: N,N-Diisopropylethylamine (0.124 mg, 0.96 mmol) and O-(7-azabenzotriazol-1-yl)-N,N,N',N'-tetramethyluronium hexafluorophosphate (73 mg, 0.19 mmol) were added sequentially to a solution of (1r,2R,3S)-2,3-dimethylcyclopropane-1-carboxylic acid (11 mg, 0.1 mmol) and compound 1-17 (60 mg, 0.1 mmol) in dichloromethane (2 mL), and the mixture was stirred at room temperature for 2 h. After the reaction was completed, the mixture was washed with 2 mL of water and concentrated to give crude compound 1A. The crude compound was purified by preparative HPLC (ammonium bicarbonate method 1) to give 2 isomeric compounds. The structure of one of the isomeric compounds was arbitrarily designated as (1r,2R,3S)-N-((8³S,6S,Z)-(Sₐ)-1¹-ethyl-1⁵-(2-((S)-1-methoxyethyl)pyridin-3-yl)-12,12-dimethyl-2,7,9-trioxo-8¹,8¹,8¹,8⁴,8⁵,8⁶-hexahydro-1¹H-10-oxa-3-aza-4(4,2)-thiazola-8(1,3)-pyridazina-1(3,4)-pyrrolacyclotridecaphan-6-yl)-2,3-dimethylcyclopropane-1-carboxamide(Z1-2, 1.73 mg, yield: 2.5%, purity: 98%, retention time = 6.68 min).

The structure of the other isomeric compound was arbitrarily designated as (1r,2R,3S)-N-((8³S,6S,Z)-(Rₐ)-1¹-ethyl-1³-(2-((S)-1-methoxyethyl)pyridin-3-yl)-12,12-dimethyl-2,7,9-trioxo-8¹,8²,8³,8⁴,8⁵,8⁶-hexahydro-1¹H-10-oxa-3-aza-4(4,2)-thiazola-8(1,3)-pyridazina-1(3,4)-pyrrolacyclotridecaphan-6-yl)-2,3-dimethylcyclopropane-1-carboxamide (Z1-1, 3.73 mg, yield: 5.4%, purity: 98%, retention time = 6.93 min).

### Example 2. Synthesis of Compounds Z2-1 and Z2-2

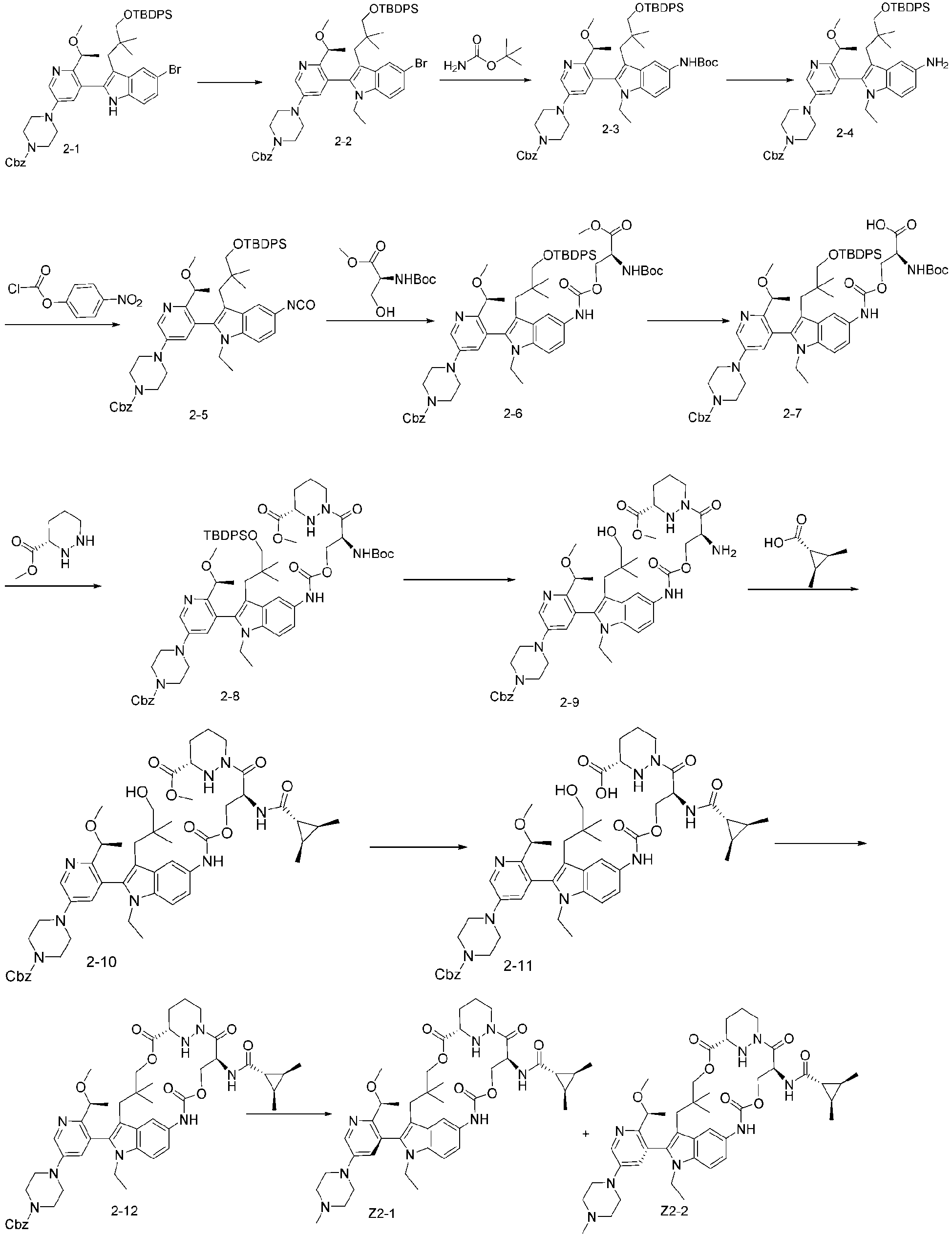

Step 1: Cesium carbonate (18.64 g, 57.210 mmol) and iodoethane (8.92 g, 57.210 mmol) were added sequentially to a solution of compound 2-1 (5 g, 5.721 mmol, refer to Intermediate 5 in WO2022060836A1 for the preparation method) in N,N-dimethylformamide (50 mL). The reaction mixture was stirred at room temperature overnight. After the reaction was completed as detected by LCMS, the reaction mixture was filtered to remove insoluble substances and washed with ethyl acetate. The filtrate was diluted with 100 mL of ethyl acetate, and then washed with water (200 mL) and saturated brine (50 mL × 3). The organic phase was dried over anhydrous sodium sulfate, filtered, and concentrated to dryness to give a crude product, which was purified by silica gel column chromatography to give compound 2-2 (4.450 g, yield: 86%). ES-API: [M+H]⁺ = 901.3.

Step 2: Compound 2-2 (4450 mg, 4.933 mmol), tert-butyl carbamate (1155.84 mg, 9.866 mmol), cesium carbonate (4018 mg, 12.333 mmol), palladium acetate (111 mg, 0.493 mmol), and 2-dicyclohexylphosphino-2',4',6'-triisopropylbiphenyl (470 mg, 0.987 mmol) were dissolved in dioxane (100 mL) under nitrogen atmosphere, and the reaction mixture was heated to 100 °C under an oil bath and stirred overnight. After the reaction was completed as detected by LCMS, the reaction mixture was cooled to room temperature and concentrated to dryness under reduced pressure. The residue was purified by automated flash chromatography on silica gel (ethyl acetate:petroleum ether = 0-70%) to give compound 2-3 (2.915 g, yield: 63%). ES-API: [M+H]⁺ = 938.5.

Step 3: Trifluoroacetic acid (6.0 mL) was slowly added to a solution of compound 2-3 (1.85 g, 1.972 mmol) in dichloromethane (20 mL) under an ice-water bath. After the dropwise addition was completed, the reaction mixture was slowly warmed to room temperature and stirred for 3 h. After the reaction was completed as detected by LCMS, the reaction mixture was concentrated under reduced pressure. The crude product was dissolved in dichloromethane (20 mL), and an ammonia-methanol solution was added to adjust the pH of the solution to about 8.0. Then the mixture was concentrated under reduced pressure, and the residue was purified by automated flash chromatography on silica gel (methanol:dichloromethane = 0-10%) to give compound 2-4 (1.85 g, yield: 100%). ES-API: [M+H]⁺ = 838.4.

Step 4: N,N-Diisopropylethylamine (2.18 mL, 12.527 mmol) and p-nitrophenyl chloroformate (631 mg, 3.132 mmol) were added sequentially to a solution of compound 2-4 (1750 mg, 2.088 mmol) in dichloromethane (25 mL) under an ice-water bath, and the reaction mixture was stirred at 0 °C for 10 min under nitrogen atmosphere. After the starting material was consumed completely and the reaction was completed as detected by LCMS, methyl (tert-butoxycarbonyl)-D-serinate (1831 mg, 8.350 mmol) and N,N-diisopropylethylamine (1.46 mL, 8.352 mmol) were added sequentially to the reaction mixture, and the reaction mixture was stirred at room temperature overnight. After the reaction was completed as detected by LCMS, the reaction mixture was diluted with dichloromethane (50 mL), and the resulting mixture was washed with saturated brine (20 mL × 3). The organic phase was dried over anhydrous sodium sulfate, filtered, and concentrated to dryness. The residue was purified by automated flash chromatography on silica gel (methanol/dichloromethane = 0-7%) to give compound 2-6 (1550 mg, yield: 69%). ES-API: [M+H]⁺ = 1083.5.

Step 5: Lithium hydroxide monohydrate (78 mg, 1.869 mmol) was added to a solution of compound 2-6 (1350 mg, 1.246 mmol) in tetrahydrofuran (12 mL) and water (3 mL) under an ice-water bath, and the reaction mixture was stirred for 1 h. After the reaction was completed as detected by LCMS, the reaction mixture was concentrated under reduced pressure to remove the solvent. The residue was adjusted to about pH 6.0 with diluted hydrochloric acid and extracted with ethyl acetate (20 mL × 3). The organic phase was dried over anhydrous sodium sulfate, filtered, and concentrated to dryness. The crude product was purified by automated flash chromatography on silica gel (methanol:dichloromethane = 0-5%) to give compound 2-7 (900 mg, yield: 68%). ES-API: [M+H]⁺ = 1069.5.

Step 6: Methyl (R)-hexahydropyridazine-3-carboxylate (557 mg, 1.496 mmol), N-methylmorpholine (1.11 mL, 10.099 mmol), 1-(3-dimethylaminopropyl)-3-ethylcarbodiimide hydrochloride (387 mg, 2.020 mmol), and 1-hydroxybenzotriazole (40 mg, 0.299 mmol) were added sequentially to a solution of compound 2-7 (800 mg, 0.748 mmol) in dichloromethane (10 mL) under an ice-water bath. The reaction mixture was stirred at room temperature for 3 h. After the reaction was completed as detected by LCMS, 30 mL of water was added to the reaction mixture to quench the reaction, and the resulting mixture was then extracted with dichloromethane (15 mL × 3). The organic phase was washed with saturated brine (10 mL × 3), dried over anhydrous sodium sulfate, filtered, and concentrated to dryness. The crude product was purified by automated flash chromatography on silica gel (tetrahydrofuran/petroleum ether = 0-40%) to give compound 2-8 (407 mg, yield: 46%). ES-API: [M+H]⁺ = 1195.6.

Step 7: A solution of hydrochloric acid in dioxane (5.0 mL) was added to a solution of compound 2-8 (150 mg, 0.125 mmol) in methanol (1 mL). The reaction mixture was stirred at room temperature for 7 h. After the reaction was completed as detected by LCMS, the reaction mixture was concentrated under reduced pressure to give crude compound 2-9 (107 mg, yield: 99%). ES-API: [M+H]⁺ = 857.3.

Step 8: (1r,2R,3S)-2,3-Dimethylcyclopropane-1-carboxylic acid (17 mg, 0.150 mmol), N,N-diisopropylethylamine (0.13 mL, 0.750 mmol), and O-(7-azabenzotriazol-1-yl)-N,N,N',N'-tetramethyluronium hexafluorophosphate (71 mg, 0.188 mmol) were added sequentially to a solution of compound 2-9 (107 mg, 0.125 mmol) in N,N-dimethylformamide (5 mL) under an ice-water bath. The reaction mixture was warmed to room temperature and stirred for 10 min. After the reaction was completed as detected by LCMS, the reaction mixture was diluted with 20 mL of ethyl acetate, and then washed with water (10 mL) and saturated brine (10 mL × 3). The organic phase was dried over anhydrous sodium sulfate, filtered, and concentrated to dryness. The crude product was purified by automated flash chromatography on silica gel (tetrahydrofuran/petroleum ether = 0-80%) to give compound 2-10 (109 mg, yield: 92 %). ES-API: [M+H]⁺ = 953.3.

Step 9: Lithium hydroxide monohydrate (5.4 mg, 0.129 mmol) was added to a solution of compound 2-10 (82 mg, 0.086 mmol) in tetrahydrofuran (4 mL) and water (1 mL) under an ice-water bath, and the reaction mixture was stirred for 10 min. After the reaction was completed as detected by LCMS, the reaction mixture was concentrated under reduced pressure to remove the solvent. The residue was adjusted to about pH 6.0 with diluted hydrochloric acid and extracted with ethyl acetate (5 mL × 4). The organic phase was dried over anhydrous sodium sulfate, filtered, and concentrated to give crude compound 2-11 (91 mg). ES-API: [M+H]⁺ = 939.4.

Step 10: 1-(3-Dimethylaminopropyl)-3-ethylcarbodiimide hydrochloride (518 mg, 2.702 mmol) was added to a solution of compound 2-11 (91 mg, 0.096 mmol), N,N-diisopropylethylamine (0.5 mL, 2.895 mmol) and 1-hydroxybenzotriazole (65 mg, 0.482 mmol) in dichloromethane (8 mL) under an ice-water bath, and the reaction mixture was stirred at room temperature for 48 h. After the reaction was completed as detected by LCMS, the reaction mixture was washed sequentially with diluted hydrochloric acid (10 mL × 2, 0.5 N) and saturated brine (15 mL × 3). The organic phase was dried over anhydrous sodium sulfate, filtered, and concentrated to dryness. The crude product was purified by automated flash chromatography on silica gel (tetrahydrofuran/petroleum ether = 0-60%) to give compound 2-12 (15 mg, yield: 17 %). ES-API: [M+H]⁺ = 921.5.

Step 11: Compound 2-12 (15 mg, 0.016 mmol) and paraformaldehyde (7.33 mg, 0.081 mmol) were dissolved in methanol (5 mL), and palladium hydroxide (15 mg, 0.107 mmol) was added to the reaction mixture under hydrogen atmosphere. The reaction mixture was stirred at room temperature for 24 h. After the reaction was completed as detected by LCMS, the reaction mixture was filtered through celite to remove insoluble substances. The filtrate was concentrated under reduced pressure, and the residue was purified by prep-HPLC (formic acid method 1) to give 2 isomeric compounds. The structure of one of the isomeric compounds was arbitrarily designated as: (1r,2R,3S)-N-((7³S,9S)-1¹-ethyl-1²-(2-((S)-1-methoxyethyl)-(Rₐ)-5-(4-methylpiperazin-1-yl)pyridin-3-yl)-3,3-dimethyl-6,8-12-trioxo-7¹,7²,7³,7⁴,7⁵,7⁶-hexahydro-1¹H-5,11-dioxa-13-aza-1(3,5)-indola-7(3,1)-pyridazinacyclotridecaphane-9-yl)-2,3-dimethylcyclopropane-1-carboxamide (Z2-1, 1.05 mg, yield: 8%, retention time = 1.73 min), formate, ES-API [M+H]⁺ = 801.3.

The structure of the other isomeric compound was arbitrarily designated as: (1r,2R,3S)-N-((7³S,9S)-1¹-ethyl-1²-(2-((S)-1-methoxyethyl)-(Sₐ)-5-(4-methylpiperazin-1-yl)pyridin-3-yl)-3,3-dimethyl-6,8-12-trioxo-7¹,7¹,7¹,7⁴,7⁵,7⁶-hexahydro-1¹H-5,11-dioxa-13-aza-1(3,5)-indola-7(3,1)-pyridazinacyclotridecaphane-9-yl)-2,3-dimethylcyclopropane-1-carboxamide (Z2-2, 1.05 mg, yield: 8%, retention time = 1.75 min), formate, ES-API [M+H]⁺ = 801.3.

### Example 3. Synthesis of Compound Z3

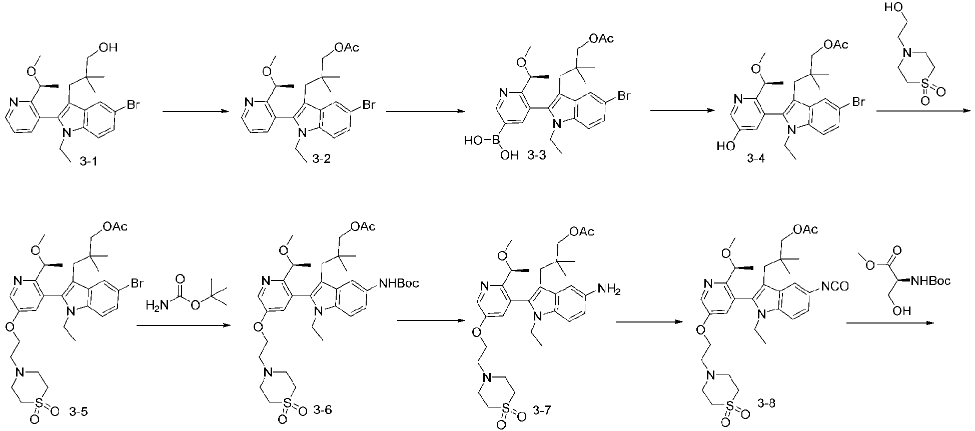

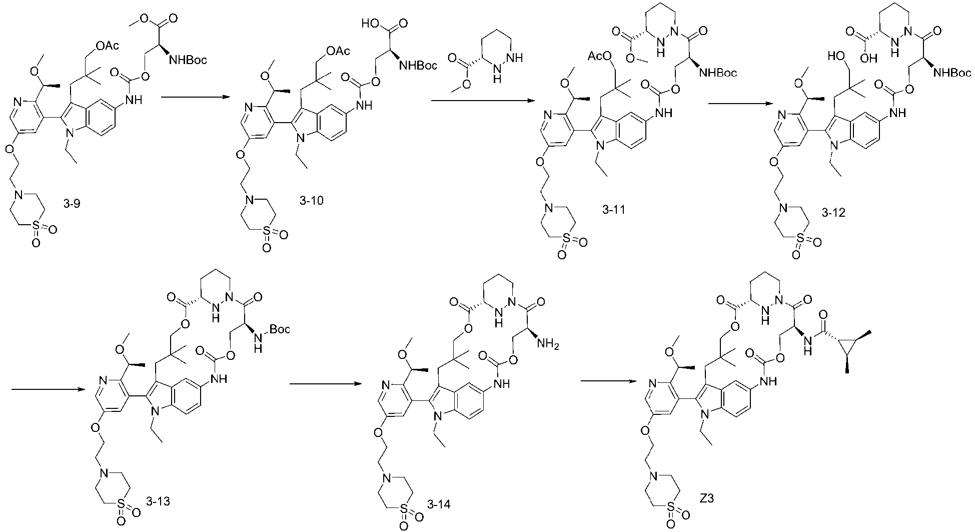

Step 1: Acetic anhydride (1.05 mL, 11.226 mmol), triethylamine (2.34 mL, 16.839 mmol), and 4-dimethylaminopyridine (137 mg, 1.123 mmol) were added to a solution of compound 3-1 (5 g, 11.226 mmol) in dichloromethane (25 mL). The reaction mixture was stirred at room temperature for 1 h. After the reaction was completed as detected by LCMS, the reaction mixture was concentrated under reduced pressure to remove the solvent. 50 mL of dichloromethane was added to the residue, and then the mixture was washed with water (20 mL) and saturated brine (30 mL × 3). The organic phase was dried over anhydrous sodium sulfate, filtered, and concentrated to dryness to give a crude product. The residue was purified by automated flash chromatography on silica gel (ethyl acetate:petroleum ether = 0-100%) to give compound 3-2 (4.922 g, yield: 90%). ES-API: [M+H]⁺ = 487.1.

Step 2: Compound 3-2 (4922 mg, 10.098 mmol), bis(pinacolato)diboron (3846.3 mg, 15.146 mmol), chloro(1,5-cyclooctadiene)iridium(I) dimer (203.48 mg, 0.303 mmol), and 4,4'-di-tert-butyl-2,2'-bipyridine (406.53 mg, 1.515 mmol) were dissolved in cyclohexane (50 mL) under nitrogen atmosphere, and the reaction mixture was heated to 85 °C under an oil bath and stirred overnight. After the reaction was completed as detected by LCMS, the reaction mixture was cooled to room temperature, and concentrated under reduced pressure to give crude compound 3-3 (5.364 g, yield: 99%). ES-API: [M+H]⁺ = 531.2.

Step 3: Hydrogen peroxide (3.5 mL, 30.291 mmol) was added to a solution of compound 3-3 (5.364 g, 10.097 mmol) in acetonitrile (30 mL), and the reaction mixture was stirred at room temperature for 2 h. After the reaction was completed as detected by LCMS, the reaction mixture was concentrated under reduced pressure to remove the solvent. 50 mL of dichloromethane was added to the residue, and then the mixture was washed with water (20 mL) and saturated brine (30 mL × 3). The organic phase was dried over anhydrous sodium sulfate, filtered, and concentrated to dryness to give a crude product. The residue was purified by automated flash chromatography on silica gel (ethyl acetate:petroleum ether = 0-50%) to give compound 3-4 (3.7 g, yield: 73%). ES-API: [M+H]⁺ = 503.2.

Step 4: 4-(2-Hydroxyethyl)thiomorpholine 1,1-dioxide (2332 mg, 2.687 mmol), triphenylphosphine (3413.56 mg, 13.014 mmol), and diisopropyl azodicarboxylate (3.2 mL, 16.268 mmol) were added sequentially to a solution of compound 3-4 (3276 mg, 6.507 mmol) in dichloromethane (10 mL) under an ice-water bath. The reaction mixture was stirred at 0 °C for 1 h. After the reaction was completed as detected by LCMS, the reaction mixture was concentrated under reduced pressure to remove the solvent. The residue was purified by automated flash chromatography on silica gel (ethyl acetate:petroleum ether = 0-25%) to give compound 3-5 (4.461 g, yield: 99 %). ES-API: [M+H]⁺ = 664.2.

Step 5: Compound 3-5 (4400 mg, 6.620 mmol), tert-butyl carbamate (1151 mg, 13.240 mmol), cesium carbonate (5392 mg, 16.550 mmol), palladium acetate (149 mg, 0.662 mmol), and 2-dicyclohexylphosphino-2',4',6'-triisopropylbiphenyl (631 mg, 1.324 mmol) were dissolved in dioxane (10 mL) under nitrogen atmosphere, and the reaction mixture was heated to 100 °C under an oil bath and stirred for 3 h. After the reaction was completed as detected by LCMS, the reaction mixture was cooled to room temperature and concentrated to dryness under reduced pressure. The residue was purified by automated flash chromatography on silica gel (methanol/dichloromethane = 0-2.5%) to give compound 3-6 (453 mg, yield: 10 %). ES-API: [M+H]⁺ = 701.3.

Step 6: Trifluoroacetic acid (3.0 mL) was slowly added to a solution of compound 3-6 (453 mg, 0.646 mmol) in dichloromethane (3 mL) under an ice-water bath, and the reaction mixture was stirred at 0 °C for 1 h. After the reaction was completed as detected by LCMS, the reaction mixture was concentrated under reduced pressure. The crude product was dissolved in a saturated sodium bicarbonate solution (10 mL), and the solution was extracted with dichloromethane (15 mL × 3). The organic phases were combined and washed with saturated brine (15 mL × 3). The organic phase was dried over anhydrous sodium sulfate, filtered, and concentrated to give crude compound 3-7 (296 mg, yield: 76%). ES-API: [M+H]⁺ = 601.3.

Step 7: N,N-Diisopropylethylamine (0.34 mL, 1.971 mmol) and p-nitrophenyl chloroformate (149 mg, 0.739 mmol) were added sequentially to a solution of compound 3-7 (149 mg, 0.739 mmol) in dichloromethane (15 mL) under an ice-water bath, and the reaction mixture was stirred at 0 °C for 10 min under nitrogen atmosphere. After the starting material was consumed completely and the reaction was completed as detected by LCMS, methyl (tert-butoxycarbonyl)-D-serinate (431 mg, 1.966 mmol) and N,N-diisopropylethylamine (0.34 mL, 1.964 mmol) were added sequentially to the reaction mixture under an ice-water bath, and the reaction mixture was stirred at room temperature overnight. After the reaction was completed as detected by LCMS, the reaction mixture was diluted with dichloromethane (20 mL), and the resulting mixture was washed with saturated brine (15 mL × 3). The organic phase was dried over anhydrous sodium sulfate, filtered, and concentrated to dryness. The residue was purified by automated flash chromatography on silica gel (methanol/dichloromethane = 0-2%) to give compound 3-9 (178 mg, yield: 43 %). ES-API: [M+H]⁺ = 846.3. Step 8: Lithium hydroxide monohydrate (8.8 mg, 0.210 mmol) was added to a solution of compound 3-9 (178 mg, 0.210 mmol) in tetrahydrofuran (5 mL) and water (1 mL) under an ice-water bath, and the reaction mixture was stirred at 0 °C for 1.5 h. After the reaction was completed as detected by LCMS, the reaction mixture was concentrated under reduced pressure to remove the solvent. The residue was adjusted to about pH 6.0 with diluted hydrochloric acid and extracted with ethyl acetate (20 mL × 3). The organic phase was dried over anhydrous sodium sulfate, filtered, and concentrated to give crude compound 3-10 (102 mg, yield: 58%). ES-API: [M+H]⁺ = 832.3.

Step 9: Methyl (R)-hexahydropyridazine-3-carboxylate (91 mg, 0.245 mmol), N-methylmorpholine (0.19 mL, 1.716 mmol), 1-(3-dimethylaminopropyl)-3-ethylcarbodiimide hydrochloride (59 mg, 0.306 mmol), and 1-hydroxybenzotriazole (8.3 mg, 0.061 mmol) were added sequentially to a solution of compound 3-10 (102 mg, 0.123 mmol) in dichloromethane (10 mL) under an ice-water bath. The reaction mixture was stirred at room temperature overnight. After the reaction was completed as detected by LCMS, 15 mL of water was added to the reaction mixture to quench the reaction, and the resulting mixture was then extracted with dichloromethane (10 mL × 3). The organic phase was washed with saturated brine (10 mL), dried over anhydrous sodium sulfate, filtered, and concentrated to dryness. The crude product was purified by automated flash chromatography on silica gel (methanol:dichloromethane = 0-3%) to give compound 3-11 (69 mg, yield: 59 %). ES-API: [M+H]⁺ = 958.3.

Step 10: Lithium hydroxide monohydrate (12 mg, 0.288 mmol) was added to a solution of compound 3-11 (69 mg, 0.072 mmol) in tetrahydrofuran (5 mL) and water (1 mL) under an ice-water bath, and the reaction mixture was stirred at room temperature overnight. After the reaction was completed as detected by LCMS, the reaction mixture was concentrated under reduced pressure to remove the solvent. The residue was adjusted to about pH 6.0 with diluted hydrochloric acid and extracted with ethyl acetate (5 mL × 4). The organic phase was dried over anhydrous sodium sulfate, filtered, and concentrated to give crude compound 3-12 (64 mg, yield: 99%g). ES-API: [M+H]⁺ = 902.3.

Step 11: 1-(3-Dimethylaminopropyl)-3-ethylcarbodiimide hydrochloride (380.8 mg, 1.987 mmol) was added to a solution of compound 3-12 (64 mg, 0.071 mmol), N,N-diisopropylethylamine (0.37 mL, 2.128 mmol) and 1-hydroxybenzotriazole (48 mg, 0.355 mmol) in dichloromethane (5 mL) under an ice-water bath, and the reaction mixture was stirred at room temperature for 18 h. After the reaction was completed as detected by LCMS, the reaction mixture was washed sequentially with diluted hydrochloric acid (10 mL × 2, 1.0 N) and saturated brine (15 mL × 3). The organic phase was dried over anhydrous sodium sulfate, filtered, and concentrated to dryness. The crude product was purified by automated flash chromatography on silica gel (tetrahydrofuran/petroleum ether = 0-95%) to give compound 3-13 (28 mg, yield: 45%). ES-API: [M+H]⁺ = 884.3.

Step 12: A solution of hydrochloric acid in dioxane (1.5 mL) was added to a solution of compound 3-13 (28 mg, 0.032 mmol) in methanol (0.5 mL). The reaction mixture was stirred at room temperature for 0.5 h. After the reaction was completed as detected by LCMS, the reaction mixture was concentrated under reduced pressure to give crude compound 3-14 (24 mg, yield: 97 %). ES-API: [M+H]⁺ = 784.3.

Step 13: (1r,2R,3S)-2,3-Dimethylcyclopropane-1-carboxylic acid (4 mg, 0.037 mmol), N,N-diisopropylethylamine (0.03 mL, 0.184 mmol), and O-(7-azabenzotriazol-1-yl)-N,N,N',N'-tetramethyluronium hexafluorophosphate (17 mg, 0.046 mmol) were added sequentially to a solution of compound 3-14 (24 mg, 0.031 mmol) in N,N-dimethylformamide (2 mL) under an ice-water bath. The reaction mixture was warmed to room temperature and stirred for 10 min. After the reaction was completed as detected by LCMS, the reaction mixture was diluted with 20 mL of ethyl acetate, and then washed with water (10 mL) and saturated brine (10 mL × 3). The organic phase was dried over anhydrous sodium sulfate, filtered, and concentrated to dryness. The residue was purified by prep-HPLC (formic acid method 1) to give (1r,2R,3S)-N-((7³S,9S)-12-(5-(2-(1,1-dioxidomorpholinyl)ethoxy)-2-(((S)-1-methoxyethyl)pyridin-3-yl)-1¹-ethyl-3,3-dimethyl-6,8-12-trioxy-7¹,7²,7³,7⁴,7⁵,7⁶_hexahydro-11H-5,11-dioxa-13-aza-1(3,5)-indola-7(3,1)-pyridazinacyclotridecaphane-9-yl)-2,3-dimethylcyclopropane-1-carboxamide (Z3, 1.11 mg, yield: 4%), formate. ES-API: [M+H]⁺= 880.3.

### Example 4. Synthesis of Compounds Z4, Z4-1, and Z4-2

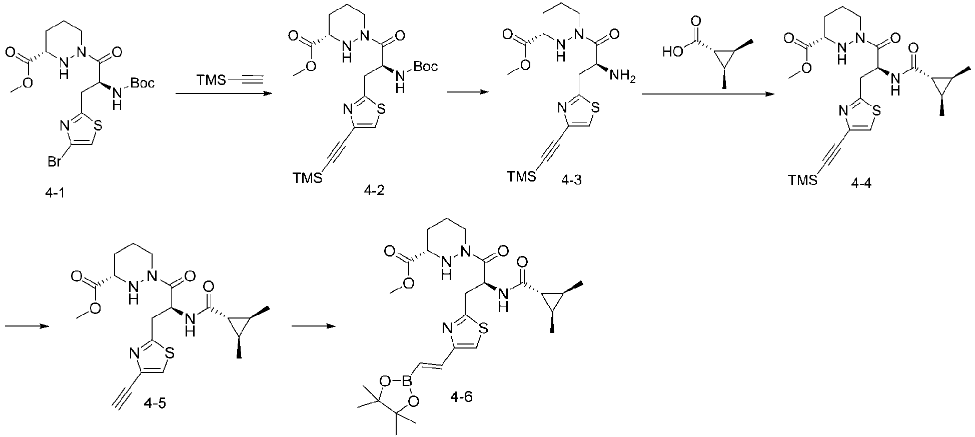

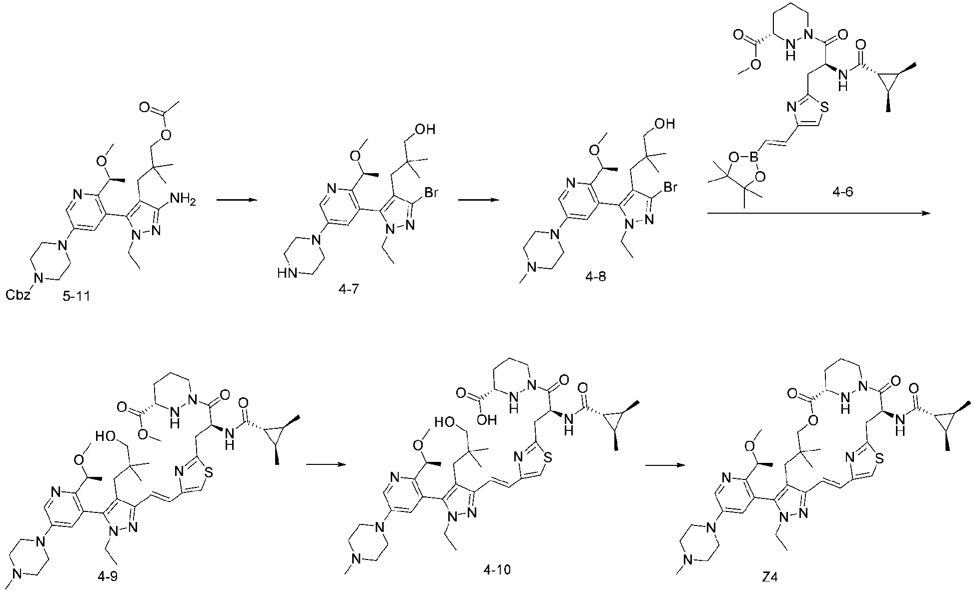

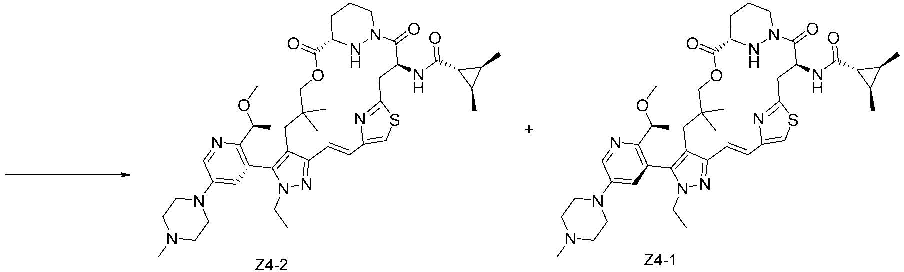

Step 1: A solution of intermediate compound 4-1 (1 g, 2.095 mmol), copper(I) iodide (0.04 g, 0.21 mmol), bis(triphenylphosphine)palladium(II) chloride (0.15 g, 0.21 mmol), triethylamine (2.91 mL, 21 mmol), and trimethylsilylalkyne (0.89 mL, 6.28 mmol) in N,N-dimethylformamide (10 mL) was reacted under microwave irradiation at 120 °C for 1.5 h under nitrogen atmosphere. Then additional trimethylsilyl alkyne (0.89 mL, 6.28 mmol) was added to the mixture, and the resulting mixture was further reacted under microwave irradiation for 1.5 h. The reaction mixture was poured into 60 mL of ethyl acetate and filtered. The filtrate was washed sequentially with 30 mL of water and 30 mL of saturated brine, dried over anhydrous sodium sulfate, filtered, and concentrated, and the residue was purified by flash silica gel column chromatography to give compound 4-2 (600 mg, yield: 60%). ES-API: [M+H]⁺ = 495.3.

Step 2: A 4 M solution of hydrochloric acid in dioxane (5 mL, 20 mmol) was added to a solution of compound 4-2 (500 mg, 1.01 mmol) in methanol (1 mL) under an ice bath, and the mixture was stirred at room temperature for 1 h. The reaction mixture was concentrated to give compound 4-3 (400 mg). ES-API: [M+H]⁺ = 395.2.

Step 3: N,N-Diisopropylethylamine (1.57 g, 12 mmol) and O-(7-azabenzotriazol-1-yl)-N,N,N',N'-tetramethyluronium hexafluorophosphate (925 mg, 2.43 mmol) were added sequentially to a solution of (1r,2R,3S)-2,3-dimethylcyclopropane-1-carboxylic acid (167 mg, 1.46 mmol) and compound 4-3 (480 mg, 1.22 mmol) in dichloromethane (10 mL), and the mixture was stirred at room temperature for 2 h. After the reaction was completed, the reaction mixture was washed with 2 mL of water and 2 mL of brine and concentrated, and the residue was purified by flash silica gel column chromatography (0-70% ethyl acetate/petroleum ether) to give compound 4-4 (450 mg, yield: 75%). ES-API: [M+H]⁺ = 491.2.

Step 4: Potassium carbonate (350 mg, 2.53 mmol) was added to compound 4-4 (400 mg, 0.82 mmol) in methanol (5 mL). The mixture was stirred at room temperature for 1 h. The reaction mixture was dissolved in 20 mL of dichloromethane, washed with 5 mL of water, dried over anhydrous sodium sulfate, filtered, and concentrated, and the residue was purified by flash silica gel column chromatography (0-100% ethyl acetate/petroleum ether) to give compound 4-5 (260 mg, yield: 68%) as a white solid. ES-API: [M+H]⁺ = 419.2.

Step 5: A mixture of copper(I) chloride (4 mg, 0.04 mmol), sodium tert-butoxide (8 mg, 0.08 mmol), and 4,5-bis(diphenylphosphino)-9,9-dimethylxanthene (22 mg, 0.04 mmol) in tetrahydrofuran (1 mL) was stirred at room temperature for half an hour under nitrogen atmosphere. Subsequently, a solution of bis(pinacolato)diboron (116 mg, 0.46 mmol) in tetrahydrofuran (1 mL) was added, and the mixture was stirred for 10 min. Finally, compound 4-5 (160 mg, 0.38 mmol) and methanol (31 µL, 0.77 mmol) were added, and the mixture was further stirred for 1 h. The reaction mixture was filtered and concentrated, and the residue was purified by flash silica gel column chromatography (0-60% tetrahydrofuran/petroleum ether) to give compound 4-6 (140 mg, yield: 67%) as a white solid. ES-API: [M+H]⁺ = 547.3

Step 6: A solution of sodium nitrite (700 mg, 10 mmol) in water (10 mL) and a solution of copper(I) bromide (1.5 g, 10 mmol) in hydrobromic acid (20 mL) were added sequentially to a solution of compound 5-11 (700 mg, 1.18 mmol) in hydrobromic acid (20 mL) under an ice bath, and the mixture was warmed to 70 °C and stirred for 1 h. The reaction mixture was cooled to room temperature, neutralized to pH 8 with a 1 M sodium hydroxide solution, filtered, extracted with 30 mL of dichloromethane, and concentrated to give compound 4-7 (600 mg). ES-API: [M+H]⁺ = 480.3, 482.2.

Step 7: Compound 4-7 (600 mg, 1.25 mmol) was dissolved in methanol (10 mL), and an aqueous formaldehyde solution (0.2 mL, 2.69 mmol) and acetic acid (0.07 mL, 1.25 mmol) were added. The mixture was stirred at room temperature for 0.5 h. Subsequently, sodium triacetoxyborohydride (790 mg, 3.75 mmol) was added, and the mixture was further stirred for 1 h. The reaction was quenched with 20 mL of saturated sodium bicarbonate solution, and the mixture was extracted three times with 20 mL of dichloromethane. The organic phases were combined, dried over anhydrous sodium sulfate, filtered, and concentrated, and the residue was purified by flash silica gel column chromatography (0-20% methanol (1% amine)/dichloromethane) to give compound 4-81 (220 mg, yield: 36%). ES-API: [M+H]⁺ = 494.1, 496.1.

Step 8: A mixed solution of compound 4-8 (120 mg, 0.24 mmol), compound 4-6 (133 mg, 0.24 mmol), Pd(dppf)Cl2 (18 mg, 0.02 mmol), and potassium phosphate (155 mg, 0.73 mmol) in dioxane (2 mL), toluene (0.5 mL), and water (0.5 mL) was stirred under microwave irradiation at 90 °C for 0.5 h under nitrogen atmosphere. 30 mL of ethyl acetate was poured into the reaction mixture, and the resulting mixture was washed with 15 mL of water and 15 mL of saturated brine. The organic phase was dried over anhydrous sodium sulfate and concentrated, and the residue was purified by flash silica gel column chromatography (0-10% methanol (1% amine)/dichloromethane) to give compound 4-9 (64 mg, yield: 32%). ES-API: [M+H]⁺ = 834.1.

Step 9: Lithium hydroxide (13 mg, 0.31 mmol) was added to a solution of compound 4-9 (64 mg, 0.08 mmol) in tetrahydrofuran (2 mL) and water (0.5 mL), and the mixture was stirred at room temperature for 1 h. After the reaction was completed, the reaction mixture was adjusted to pH 6 with 1 M hydrochloric acid, then adjusted to pH 8 with a saturated sodium bicarbonate solution, extracted with 20 mL of dichloromethane, dried, and concentrated to give compound 4-10 (60 mg). ES-API: [M+H]⁺ = 820.1.

Step 10: 1-(3-Dimethylaminopropyl)-3-ethylcarbodiimide hydrochloride (842 mg, 4.39 mmol), 1-(3-dimethylaminopropyl)-3-ethylcarbodiimide hydrochloride (158 mg, 1.17 mmol), and N,N-diisopropylethylamine (662 mg, 5.12 mmol) were added to a solution of compound 4-10 (120 mg, 0.15 mmol) in dichloromethane (30 mL), and the mixture was stirred at room temperature for 5 h. After the reaction was completed, the mixture was washed with 10 mL of water, dried, and concentrated, and the residue was purified by prep-HPLC (ammonium bicarbonate method 1) to give (1r,2R,3S)-N-((4²Z,8³S,2E,6S)-1¹-ethyl-15-(2-((S)-1-methoxyethyl)-5-(4-methylpiperazin-1-yl))pyridin-3-yl)-12,12-dimethyl-7,9-dioxo-8¹,8²,8³,8⁴,8⁵,3⁶-hexahydro-11H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z4, 36.5 mg, yield: 31%, purity: 98%). ES-API: [M+H]⁺ = 802.2.

Step 11: Compound Z4 (35 mg) was subjected to chiral resolution (column type: Chiralpak IB: 10 µm, 20 × 250 mm, mobile phase: n-hexane:isopropanol:diethylamine = 70:30:0.2, flow rate: 15 mL/min, column temperature: room temperature) to give 2 isomeric compounds. The structure of one of the isomeric compounds was arbitrarily designated as (1r,2R,3S)-N-((4²Z,8³S,2E,6S)-(Sₐ)-1¹-ethyl-1⁵-(2-((S)-1-methoxyethyl)-5-(4-methylpiperazin-1-yl))pyridin-3-yl)-12,12-dimethyl-7,9-dioxo-8¹,8²,8³,8⁴,8⁵ 3⁶-hexahydro-11H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z4-2, 12.5 mg, yield: 35.7%, retention time = 6.66 min, purity: 100%, de value: 100%). ES-API: [M+H]⁺ = 802.2. ¹H NMR (400 MHz, DMSO-*d₆*) δ 8.44 (d, *J =* 2.8 Hz, 1H), 8.38 (d, *J* = 8.8 Hz, 1H), 7.43 (s, 1H), 7.37 (d, *J =* 15.6 Hz, 1H), 7.30 (d, *J =* 2.8 Hz, 1H), 7.24 (d, *J =* 15.6 Hz, 1H), 5.48 (t, *J =* 8.8 Hz, 1H), 5.00 (d, *J =* 12.0 Hz, 1H), 4.21 (d, *J =* 12.0 Hz, 1H), 4.13 - 4.00 (m, 1H), 3.94 - 3.84 (m, *J* = 6.3 Hz, 1H), 3.84 - 3.74 (m, 2H), 3.66 (d, *J =* 10.8 Hz, 1H), 3.55 (d, *J =* 10.8 Hz, 1H), 3.28 - 3.18 (m, 5H), 3.18 - 3.11 (m, 1H), 3.09 (s, 3H), 2.81 - 2.68 (m, 2H), 2.47 (s, 1H), 2.23 (s, 3H), 2.19 (d, *J =* 14.0 Hz, 1H), 2.08 - 1.98 (m, 1H), 1.82 - 1.66 (m, 2H), 1.54 - 1.41 (m, 1H), 1.32 - 1.13 (m, 12H), 1.11 - 1.03 (m, 6H), 0.84 (s, 3H), 0.46 (s, 3H).

The structure of the other isomeric compound was arbitrarily designated as (1r,2R,3S)-N-((4²Z,8³S,2E,6S)-(Rₐ)-1¹-ethyl-1⁵-(2-((S)-1-methoxyethyl)-5-(4-methylpiperazin-1-yl))pyridin-3-yl)-12,12-dimethyl-7,9-dioxo-8¹,8²,8³,8⁴,8⁵,8⁶_hexahydro-11H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z4-1, 12.5 mg, yield: 35.7%, retention time = 7.66 min, purity: 100%, de value: 100%). ES-API: [M+H]⁺ = 802.2. ¹H NMR (400 MHz, DMSO-d₆) δ 8.43 (d, *J* = 2.8 Hz, 1H), 8.35 (d, *J =* 8.8 Hz, 1H), 7.43 (s, 1H), 7.36 (d, *J =* 15.6 Hz, 1H), 7.24 (d, *J=* 15.6 Hz, 1H), 7.14 (d, *J =* 2.8 Hz, 1H), 5.53 (t, *J =* 8.4 Hz, 1H), 5.03 (d, *J =* 12.0 Hz, 1H), 4.21 (d, *J* = 10.4 Hz, 1H), 4.17 - 4.08 (m, 1H), 4.08 - 3.95 (m, 3H), 3.66 - 3.52 (m, 2H), 3.29 - 3.24 (m, 4H), 3.22 (s, 1H), 3.19 (s, 3H), 3.15 - 3.08 (m, 1H), 2.77 - 2.65 (m, 1H), 2.65 - 2.56 (m, 1H), 2.28 - 2.19 (m, 4H), 2.02 - 1.93 (m, 1H), 1.81 - 1.73 (m, 1H), 1.71 - 1.59 (m, 1H), 1.54 - 1.41 (m, 1H), 1.34 (d, *J =* 6.0 Hz, 3H), 1.30 (s, 1H), 1.25 (d, *J* = 9.2 Hz, 3H), 1.18 - 1.12 (m, 6H), 1.08 - 1.03 (m, 6H), 0.77 (s, 3H), 0.33 (s, 3H).

### Example 5. Synthesis of Compound Z5

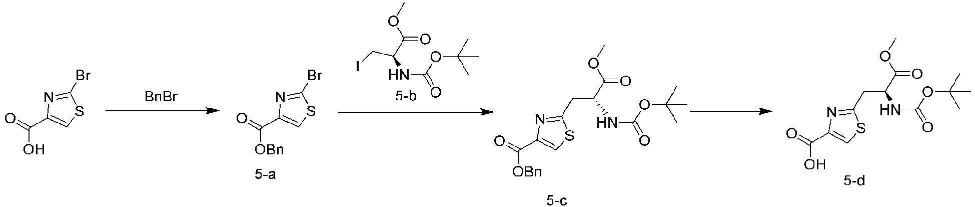

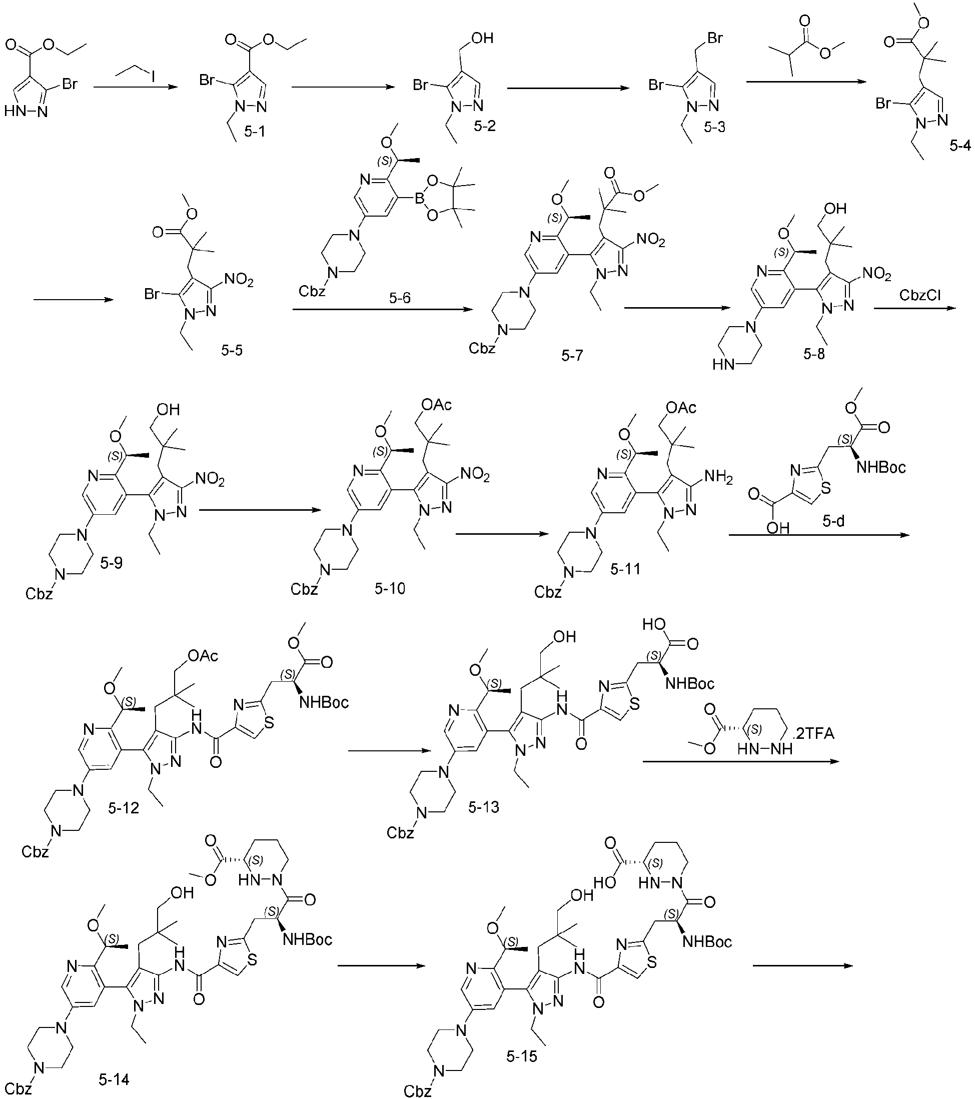

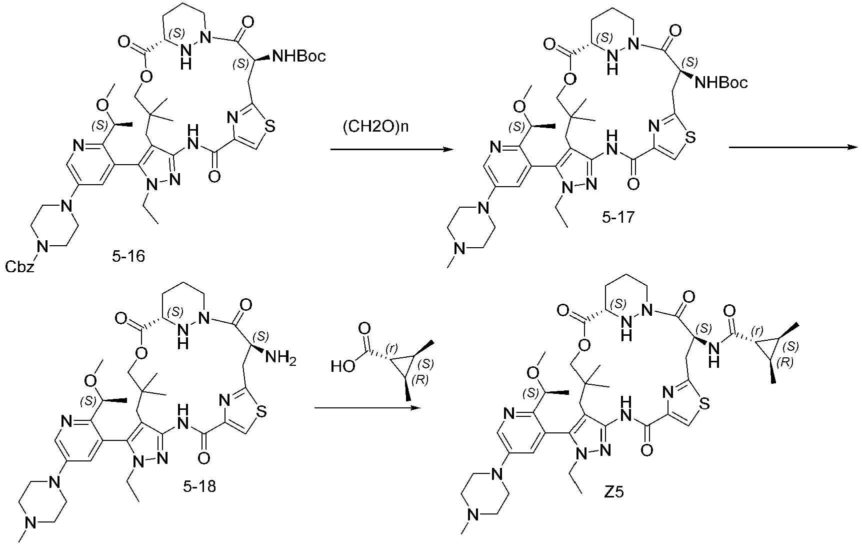

Step 1: 2-Bromothiazole-4-carboxylic acid (3 g, 14.421 mmol) was dissolved in N,N-dimethylformamide (50 mL), and benzyl bromide (2.96 g, 17.305 mmol) and potassium carbonate (3.99 g, 28.842 mmol) were added to the above solution. After the addition was completed, the reaction system was stirred at room temperature for 17 h. After the reaction was completed, water (100 mL) was added to the reaction mixture, and the resulting mixture was extracted with ethyl acetate (50 mL). The organic phase was washed with saturated brine (100 mL × 4), dried, and concentrated to give a crude product. The crude product was purified by flash silica gel column chromatography (ethyl acetate/petroleum ether: 0%-20%) to give compound 5-a (3 g, 10.062 mmol, yield: 69.77%) as a colorless transparent liquid. ES-API: [M+H]⁺ = 298.0, 300.0.

Step 2: Zinc powder (0.60 g, 9.115 mmol) and dry N,N-dimethylformamide (10 mL) were placed in a three-necked flask and purged three times with nitrogen. To the above three-necked flask was slowly added trimethylchlorosilane (0.116 mL, 0.911 mmol) at room temperature. After the addition was completed, the reaction mixture was stirred at 90 °C for 30 min. The stirring was stopped. After the reaction mixture was cooled to room temperature, zinc powder was precipitated. N,N-Dimethylformamide was removed from the three-necked flask with a double-ended needle. After removal, N,N-dimethylformamide (10 mL) was added to the three-necked flask, and the mixture was stirred for 30 s to wash the zinc powder. Subsequently, zinc powder was precipitated again, and N,N-dimethylformamide (10 mL) was removed again. After the above washing operation was repeated twice, the zinc powder in the three-necked flask was dried using a heat gun under nitrogen atmosphere. Compound 5-b (1 g, 3.038 mmol) was dissolved in N,N-dimethylformamide (10 mL). The above solution was slowly added dropwise to the activated zinc powder under an ice bath under nitrogen atmosphere. After the addition was completed, the reaction system was further stirred for 30 min. The stirring was stopped. Zinc powder was precipitated, and the supernatant was introduced into a mixed reaction mixture containing bis(triphenylphosphine)palladium(II) dichloride (0.12 g, 0.152 mmol), compound 5-a (0.91 g, 3.038 mmol), and N,N-dimethylformamide (20 mL) using a double-ended needle. After the introduction was completed, the reaction system was stirred at 70 °C for 3 h. After the reaction was completed, water (40 mL) was added to the reaction mixture, and the resulting mixture was extracted with ethyl acetate (50 mL). The organic phase was washed with saturated brine (100 mL × 3), dried over anhydrous sodium sulfate, filtered, and concentrated to give a crude product. The crude product was purified by flash silica gel column chromatography (ethyl acetate/petroleum ether: 0%-20%) to give compound 5-c (900 mg, 2.140 mmol, yield: 70.44%) as a white solid. ES-API: [M+H]⁺ = 421.1.

Step 3: Compound 5-c (900 mg, 2.140 mmol) was dissolved in methanol (20 mL). Wet palladium/carbon (10 wt%, 455.48 mg, 0.430 mmol) was added to the above solution. After the addition was completed, the reaction system was stirred at 50 °C for 72 h under hydrogen atmosphere (15 psi). The reaction mixture was filtered through celite. The filter cake was washed with methanol (50 mL), and the filtrate was concentrated to give a crude product. The crude product was purified by flash silica gel column chromatography (methanol/dichloromethane: 0%-4%) to give compound 5-d (130 mg, 0.394 mmol, yield: 18.38%) as a white solid. ES-API: [M+H]⁺ = 331.1.

Step 4: Ethyl 3-bromo-1H-pyrazole-4-carboxylate (5.0 g, 22.83 mmol) was dissolved in acetonitrile (50 mL), and potassium carbonate (9.46 g, 68.48 mmol) and iodoethane (7.12 g, 45.65 mmol) were added at room temperature. The reaction system was stirred at 85 °C for 18 h. The reaction mixture was concentrated. Ethyl acetate (100 mL) was added, and the mixture was washed sequentially with water (25 mL) and saturated brine (25 mL). The organic phase was dried over anhydrous sodium sulfate and filtered, and the filtrate was concentrated under reduced pressure. The crude product was purified by flash silica gel column chromatography (ethyl acetate/petroleum ether: 0-30%) to give compound 5-1 (1.6 g, yield: 28.4%) as a colorless liquid. ¹H NMR (400 MHz, CDCl3) δ 7.85 (s, 1H), 4.29 (q, J = 7.2 Hz, 2H), 4.14 (q, J = 7.2 Hz, 2H), 1.50 (t, J = 7.2 Hz, 3H), 1.34 (t, J = 7.2 Hz, 3H). ES-API: [M+H]⁺ = 247.1, 249.1.

Step 5: Compound 5-1 (1.35 g, 5.46 mmol) was dissolved in tetrahydrofuran (25 mL), and a 1.0 M solution of DIBAL-H in n-hexane (13.66 mL, 13.66 mmol) was added dropwise at 0 °C. The reaction system was stirred at room temperature for 5 h. A saturated potassium sodium tartrate solution (30 mL) was added to the reaction mixture to quench the reaction, and the reaction mixture was stirred at room temperature for 1 h, and extracted with ethyl acetate (60 mL × 2). The organic phase was dried over anhydrous sodium sulfate and filtered, and the filtrate was concentrated under reduced pressure. The crude product was purified by flash silica gel column chromatography (ethyl acetate/petroleum ether: 0-30%) to give compound 5-2 (1.0 g, yield: 89.3%) as a white solid. ES-API: [M+H]⁺ = 205.1, 207.1. ¹H NMR (400 MHz, DMSO) δ 7.53 (s, 1H), 4.92 (t, *J =* 5.4 Hz, 1H), 4.26 (d, *J = 5.4* Hz, 2H), 4.13 (q, *J =* 7.2 Hz, 2H), 1.30 (t, *J =* 7.2 Hz, 3H).

Step 6: Compound 5-2 (200 mg, 0.97 mmol) and triphenylphosphine (384 mg, 1.46 mmol) were dissolved in dichloromethane (5 mL), and a solution of carbon tetrabromide (582 mg, 1.76 mmol) in dichloromethane (1 mL) was added at 0 °C. The reaction system was stirred at room temperature for 18 h. The reaction mixture was concentrated, and the crude product was purified by flash silica gel column chromatography (ethyl acetate/petroleum ether: 0-10%) to give the desired product, compound 5-3 (175 mg, yield: 67.0%) as a white solid. ¹H NMR (400 MHz, CDCl3) δ 7.59 (s, 1H), 4.38 (s, 2H), 4.21 (q, J = 7.2 Hz, 2H), 1.43 (t, J = 7.2 Hz, 3H).

Step 7: Methyl isobutyrate (457 mg, 4.48 mmol) was dissolved in tetrahydrofuran (15 mL), and a 2.0 M solution of lithium diisopropylamide in tetrahydrofuran/n-hexane (2.24 mL, 4.48 mmol) was added dropwise at -70 °C. The reaction system was stirred at -70 °C for 1 h. A solution of compound 5-3 (800 mg, 2.99 mmol) in tetrahydrofuran (8 mL) was added dropwise. The reaction system was stirred at -70 °C for 1 h, and then slowly warmed to room temperature and stirred for 3 h. The reaction was quenched with a saturated ammonium chloride solution (20 mL), and the reaction mixture was extracted with ethyl acetate (60 mL). The organic phase was washed with saturated brine (25 mL), dried over anhydrous sodium sulfate, and filtered, and the filtrate was concentrated under reduced pressure. The crude product was purified by flash silica gel column chromatography (ethyl acetate/petroleum ether: 0-20%) to give compound 5-4 (730 mg, yield: 84.5%) as a light yellow liquid. ¹H NMR (400 MHz, CD3OD) 7.32 (s, 1H), 4.21 (q, J = 7.2 Hz, 2H), 3.68 (s, 3H), 2.68 (s, 2H), 1.36 (t, J = 7.2 Hz, 3H), 1.18 (s, 6H).ES-API: [M+H]+= 289.1,291.1.

Step 8: Compound 5-4 (680 mg, 2.35 mmol) was dissolved in acetic anhydride (6 mL), and concentrated nitric acid (0.77 mL, 7.05 mmol) was added dropwise at 0 °C. The reaction system was stirred at 0 °C for 2 h, and then stirred at room temperature for 1 h. The reaction mixture was poured into ice water, and a saturated sodium bicarbonate solution (25 mL) was slowly added. The mixture was extracted with ethyl acetate (25 mL × 2). The organic phase was washed with water (15 mL × 2) and saturated brine (25 mL), dried over anhydrous sodium sulfate, and filtered, and the filtrate was concentrated under reduced pressure. The crude product was purified by flash silica gel column chromatography (ethyl acetate/petroleum ether: 0-30%) to give the desired product, which was concentrated. The crude product was purified by flash silica gel column chromatography (tetrahydrofuran/petroleum ether: 0-20%) to give compound 5-5 (620 mg, yield: 78.9%) as a white solid. ¹H NMR (400 MHz, CDCl3) δ 4.31 (q, J = 7.2 Hz, 2H), 3.64 (s, 3H), 3.09 (s, 2H), 1.49 (t, J = 7.2 Hz, 3H), 1.20 (s, 6H). ES-API: [M+H]⁺ = 334.0, 336.1.

Step 9: Compound 5-5 (200 mg, 0.60 mmol) and compound 5-6 (519 mg, 1.08 mmol) were dissolved in toluene (2 mL), and dioxane (0.5 mL), water (0.5 mL), potassium phosphate (318 mg, 1.50 mmol), and methanesulfonyloxy(diadamantyl-n-butylphosphino)-2'-amino-1,1'-biphenyl-2-yl)palladium(II) (65 mg, 0.090 mmol) were added. The reaction system was purged with nitrogen for 30 s and stirred at 110 °C for 1 h in a microwave reactor. Ethyl acetate (40 mL) and water (5 mL) were added to the reaction mixture. The separated organic phase was washed with saturated brine (10 mL), dried over anhydrous sodium sulfate, and filtered, and the filtrate was concentrated under reduced pressure. The crude product was purified by flash silica gel column chromatography (tetrahydrofuran/petroleum ether: 0-40%) to give compound 5-7 (200 mg, yield: 54.9%) as a brown solid. ES-API: [M+H]⁺ = 609.3.

Step 10: Compound 5-7 (200 mg, 0.33 mmol) was dissolved in tetrahydrofuran (2 mL), and a 1.0 M solution of DIBAL-H in n-hexane (0.82 mL, 0.82 mmol) was added dropwise at -70 °C. The reaction system was stirred at -70 °C for 30 min, and then slowly heated to -20 °C and stirred for 1 h. Water (0.1 mL), 15% sodium hydroxide solution (0.1 mL), and water (0.3 mL) were added sequentially to the reaction mixture. The reaction system was stirred at room temperature for 15 min. Anhydrous magnesium sulfate was added, and the reaction system was further stirred for 15 min, and filtered. The filter cake was washed with ethyl acetate, and the filtrate was concentrated under reduced pressure to give compound 5-8 (180 mg, crude product). ES-API: [M+H]⁺ = 447.3.

Step 11: Compound 5-8 (180 mg, crude product) was dissolved in tetrahydrofuran (5 mL) and water (5 mL), and sodium bicarbonate (135 mg, 1.61 mmol) was added. The reaction system was cooled to 0 °C. Benzyl chloroformate (69 mg, 0.40 mmol) was added, and the reaction system was stirred at 0 °C for 1 h. The reaction mixture was extracted with ethyl acetate (40 mL). The organic phase was washed with saturated brine (20 mL), dried over anhydrous sodium sulfate, and filtered, and the filtrate was concentrated under reduced pressure. The crude product was purified by flash silica gel column chromatography (ethyl acetate/petroleum ether: 0-100%) to give compound 5-9 (90 mg, two-step yield: 38.4%) as a light yellow solid. ES-API: [M+H]⁺ = 581.2.

Step 12: Compound 5-9 (90 mg, 0.155 mmol), diisopropylethylamine (60.10 mg, 0.465 mmol), and 4-dimethylaminopyridine (2 mg, 0.016 mmol) were dissolved in dichloromethane (4 mL), and acetic anhydride (24 mg, 0.24 mmol) was added at 0 °C. The reaction system was stirred at room temperature for 1 h. The reaction mixture was concentrated, and the crude product was purified by flash silica gel column chromatography (ethyl acetate/petroleum ether: 0-100%) to give compound 5-10 (90 mg, yield: 93.2%) as a light yellow viscous liquid. ES-API: [M+H]⁺ = 623.2.

Step 13: Compound 5-10 (300 mg, 0.48 mmol) was dissolved in ethanol (7.5 mL) and water (3 mL), and iron powder (134 mg, 2.41 mmol) and ammonium chloride (129 mg, 2.41 mmol) were added at room temperature. The reaction system was stirred at 80 °C for 4 h. The reaction mixture was filtered. The filter cake was washed with methanol, and the filtrate was concentrated under reduced pressure. Dichloromethane (30 mL) was added, and the mixture was washed with saturated brine (20 mL), dried over anhydrous sodium sulfate, and filtered. The filtrate was concentrated under reduced pressure. The crude product was purified by flash silica gel column chromatography (methanol/dichloromethane: 0-3%) to give compound 5-11 (280 mg, two-step yield: 98.0%) as a light yellow viscous liquid. ES-API: [M+H]⁺ = 593.3.

Step 14: Compound 5-11 (301 mg, 0.51 mmol) and compound 5-d (140 mg, 0.42 mmol) were dissolved in dichloromethane (8 mL), and triethylamine (0.35 mL, 2.54 mmol) and a 50% solution of 1-propylphosphoric cyclic anhydride in ethyl acetate (809 mg, 1.27 mmol) were added at room temperature. The reaction system was stirred at room temperature for 18 h. Dichloromethane (30 mL) was added to the reaction mixture, and the resulting mixture was washed sequentially with water (10 mL) and saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-50%) to give compound 5-12 (300 mg, yield: 78.2%) as a white solid. ES-API: [M+H]⁺ = 905.3.

Step 15: Compound 5-12 (270 mg, 0.30 mmol) was dissolved in tetrahydrofuran (9 mL) and water (3 mL), and lithium hydroxide monohydrate (50 mg, 1.19 mmol) was added at 0 °C. The reaction system was stirred at room temperature for 18 h. The reaction mixture was adjusted to pH 6 with 1 M diluted hydrochloric acid and extracted with ethyl acetate (30 mL × 2). The organic phase was dried over anhydrous sodium sulfate and concentrated to give compound 5-13 (250 mg, yield: 98.7%) as a light yellow solid. ES-API: [M+H]⁺ = 849.3.

Step 16: Compound 5-13 (265 mg, 0.31 mmol) was dissolved in dichloromethane (10 mL), and (S)-hexahydropyridazine-3-carboxylate trifluoroacetate (232 mg, 0.62 mmol), N-methylmorpholine (0.34 mL, 3.12 mmol), 1-(3-dimethylaminopropyl)-3-ethylcarbodiimide hydrochloride (120 mg, 0.62 mmol), and 1-hydroxybenzotriazole (13 mg, 0.094 mmol) were added at 0 °C. The reaction system was stirred at room temperature for 18 h. Water (15 mL) was added to the reaction mixture, and the resulting mixture was extracted with ethyl acetate (50 mL). The organic phase was washed with saturated brine (15 mL), dried over anhydrous sodium sulfate, and filtered, and the filtrate was concentrated under reduced pressure. The crude product was purified by flash silica gel column chromatography (methanol/dichloromethane: 0-4%) to give compound 5-14 (210 mg, yield: 69.0%) as a white solid. ES-API: [M+H]⁺ = 975.3.

Step 17: Compound 5-14 (200 mg, 0.21 mmol) was dissolved in tetrahydrofuran (6 mL) and water (1.5 mL), and lithium hydroxide monohydrate (17 mg, 0.41 mmol) was added at 0 °C. The reaction system was stirred at room temperature for 2 h. The reaction mixture was adjusted to pH 6 with 1 M diluted hydrochloric acid and extracted with ethyl acetate (30 mL × 2). The organic phase was dried over anhydrous sodium sulfate and concentrated to give compound 5-15 (195 mg, yield: 99.0%) as a white solid. ES-API: [M+H]⁺ = 961.3. Step 18: Compound 5-15 (275 mg, 0.37 mmol) was dissolved in dichloromethane (25 mL), and 1-(3-dimethylaminopropyl)-3-ethylcarbodiimide hydrochloride (1.0 g, 5.24 mmol), 1-hydroxybenzotriazole (126 mg, 0.94 mmol), and diisopropylethylamine (726 mg, 5.62 mmol) were added at room temperature. The reaction system was stirred at room temperature for 18 h. Dichloromethane (25 mL) was added to the reaction mixture, and the resulting mixture was washed sequentially with water (15 mL × 2) and saturated brine (15 mL), dried over anhydrous sodium sulfate, and filtered. The filtrate was concentrated under reduced pressure. The crude product was purified by flash silica gel column chromatography (methanol/dichloromethane: 0-4%) to give compound 5-16 (130 mg, yield: 73.6%) as a white solid. ES-API: [M+H]⁺ = 943.3.

Step 19: Compound 5-16 (130 mg, 0.14 mmol) and paraformaldehyde (86 mg, 2.85 mmol) were dissolved in absolute methanol (18 mL), and 10% palladium hydroxide on carbon (150 mg) was added at room temperature. The reaction system was stirred at 30 °C for 24 h under hydrogen atmosphere. The reaction mixture was filtered through celite, and the filtrate was concentrated. The crude product was purified by flash silica gel column chromatography (methanol/dichloromethane: 0-5%) to give compound 5-17 (100 mg, yield: 88.1%) as a white solid. ES-API: [M+H]⁺ = 823.3.

Step 20: Compound 5-17 (85 mg, 0.103 mmol) was dissolved in absolute methanol (1 mL), and a 4.0 M hydrogen chloride/dioxane solution (4.0 mL) was added at 0 °C. The reaction system was stirred for 1 h under an ice bath. The reaction mixture was concentrated to give compound 5-18 (74 mg, yield: 100%) as a white solid. ES-API: [M+H]⁺ = 723.3.

Step 21: Compound 5-18 (74 mg, 0.10 mmol) was dissolved in dichloromethane (10 mL), and (1r,2R,3S)-2,3-dimethylcyclopropane-1-carboxylic acid (18 mg, 0.15 mmol), O-(7-azabenzotriazol-1-yl)-N,N,N',N'-tetramethyluronium hexafluorophosphate (78 mg, 0.20 mmol), and N,N'-diisopropylethylamine (79 mg, 0.61 mmol) were added. The reaction system was stirred at room temperature for 1 h. Water (10 mL) was added to the reaction mixture, and the resulting mixture was extracted with ethyl acetate (40 mL). The organic phase was washed with saturated brine (10 mL), dried over anhydrous sodium sulfate, and filtered, and the filtrate was concentrated under reduced pressure. The crude product was subjected to prep-HPLC (ammonium bicarbonate method 1) to give the desired product (1r,2R,3S)-N-((8³S,6S,Z)-1¹-ethyl-1³-(2-((S)-1-methoxyethyl)-5-(4-methylpiperazin-1-yl)pyridin-3-yl)-12,12-dimethyl-3,7,9-trioxo-8¹,8²,8¹,8⁴,8⁵,8⁶-hexahydro-11H-10-oxa-2-aza-4(4,2)-thiazola-8(1,3)-pyridazina-1(3,4)-pyrrolacyclotridecaphan-6-yl)-2,3-dimethylcyclopropane-1-carboxamide (Z5, 60 mg, yield: 71.6%) as a white solid. ES-API: [M+H]⁺ = 819.4.

### Example 6. Synthesis of Compound Z36

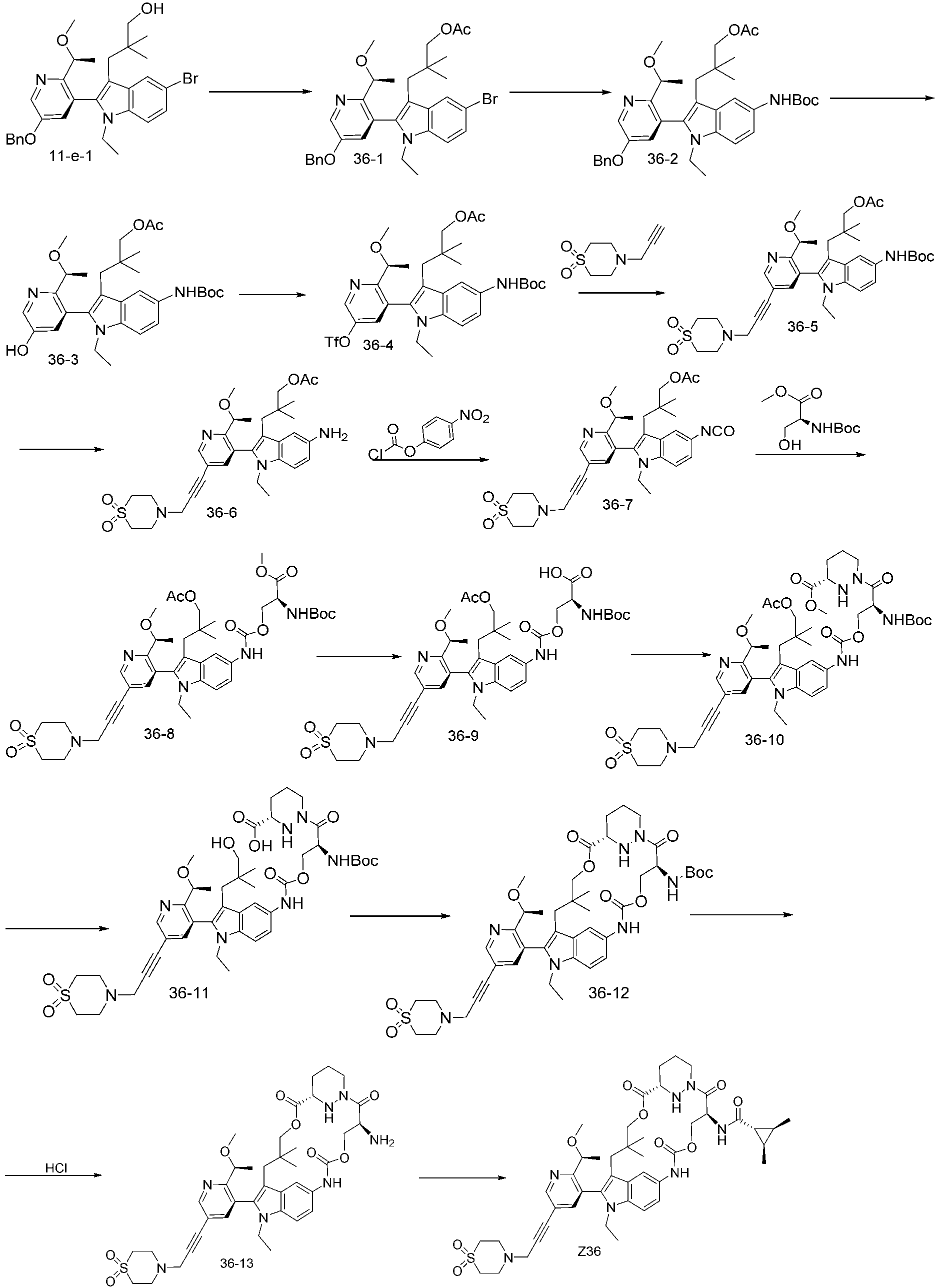

Step 1: Acetic anhydride (1.05 mL, 11.226 mmol), triethylamine (2.34 mL, 16.839 mmol), and 4-dimethylaminopyridine (137 mg, 1.123 mmol) were added to a solution of compound 11-e-1 (7.0 g, 12.69 mmol) in dichloromethane (25 mL). The reaction mixture was stirred at room temperature for 1 h. After the reaction was completed as detected by LCMS, the reaction mixture was concentrated under reduced pressure to remove the solvent. 100 mL of dichloromethane was added to the residue, and then the mixture was washed with water (150 mL) and saturated brine (60 mL × 3). The organic phase was dried over anhydrous sodium sulfate, filtered, and concentrated to dryness to give a crude product. The residue was purified by automated flash chromatography on silica gel (ethyl acetate:petroleum ether = 0-100%) to give compound 36-1 (6.1 g, yield: 94.47 %). ES-API: [M+H]⁺ = 593.2.

Step 2: Compound 36-1 (4.0 g, 6.739 mmol), tert-butyl carbamate (1.58 g, 13.478 mmol), cesium carbonate (5.5 g, 16.84 mmol), palladium acetate (149 mg, 0.662 mmol), and 2-dicyclohexylphosphino-2',4',6'-triisopropylbiphenyl (631 mg, 1.324 mmol) were dissolved in dioxane (10 mL) under nitrogen atmosphere. The reaction mixture was heated to 100 °C under an oil bath and stirred for 3 h. After the reaction was completed as detected by LCMS, the reaction mixture was cooled to room temperature and concentrated to dryness under reduced pressure. The residue was purified by automated flash chromatography on silica gel (methanol/dichloromethane = 0-2.5%) to give compound 36-2 (3.6 g, yield: 84.82%). ES-API: [M+H]⁺ = 630.3.

Step 3: 10% (by mass) Pd/C (6.08 g, 57.161 mmol) was added to a solution of compound 36-2 (3.6 g, 5.716 mmol) in methanol (50 mL), and the reaction mixture was stirred at room temperature for 1 h. The reaction system was filtered, concentrated in vacuum, and dried to give compound 36-3 (2.90 g, 5.374 mmol, yield: 94.16%). ES-API: [M+H]⁺ = 540.3.

Step 4: Triethylamine (3.3 mL, 23.74 mmol) and N-[dioxo(trifluoromethyl)-16-sulfanyl]-1,1,1-trifluoro-N-phenylmethanesulfonamide (3.85 g, 10.777 mmol) were added to a solution of compound 36-3 (2.90 g, 5.374 mmol) in dichloromethane (100.0 mL) at room temperature. The reaction system was stirred at room temperature for 1 h under nitrogen atmosphere. The reaction mixture was concentrated and purified by automated flash chromatography on silica gel (eluting with 0% to 2% methanol in dichloromethane) to give compound 36-4 (3.3 g, 4.913 mmol, yield: 91.42%) as a white solid. ES-API: [M+H]⁺ = 672.2.

Step 5: Bis(triphenylphosphine)palladium(II) dichloride (0.69 g, 0.983 mmol), copper(I) iodide (0.37 g, 1.965 mmol), N,N-diisopropylethylenediamine (3.3 mL, 18.945 mmol), and lithium chloride (0.83 g, 19.651 mmol) were added to a solution of compound 36-4 (3.3 g, 4.913 mmol) and 4-(prop-2-yn-1-yl)thiomorpholine 1,1-dioxide (1.80 g, 10.391 mmol) in N,N-dimethylformamide (70.0 mL). The reaction mixture was stirred at 80 °C for 6 h under nitrogen atmosphere. After the reaction was completed, the reaction mixture was diluted with water (80.0 mL) and extracted with ethyl acetate (100 mL × 3). The organic layers were combined, washed with brine, dried over anhydrous sodium sulfate, filtered, and concentrated. The residue was purified by automated flash chromatography on silica gel (0% to 5% methanol/dichloromethane) to give compound 36-5 (2.40 g, 3.454 mmol, yield: 70.30%) as a white solid. ES-API: [M+H]⁺ = 695.3.

Step 6: Trifluoroacetic acid (3 mL) was added to a solution of compound 36-5 (1.40 g, 2.015 mmol) in dichloromethane (10.0 mL) at 0 °C, and the mixture was stirred at 0 °C for 1 h. After the reaction was completed as detected by LCMS, the solvent was removed under reduced pressure, and the pH of the crude product was adjusted with 10 mL of saturated sodium bicarbonate solution. Then 50 mL of saturated sodium bicarbonate solution was added, and the mixture was extracted with dichloromethane (10 mL × 3). The organic phases were combined, washed with saturated brine (10 mL), dried, filtered, and concentrated to dryness to give compound 36-6 (1.10 g, 1.849 mmol, yield: 91.80%). ES-API: [M+H]⁺ = 595.4.

Step 7: N,N-Diisopropylethylamine (1.289 mL, 7.398 mmol) was added to a solution of compound 36-6 (1.10 g, 1.849 mmol) in dichloromethane (15 mL) at 0 °C, and then 4-nitrophenyl chloroformate (0.56 g, 2.774 mmol) was added. The reaction system was stirred at 0 °C for 10 min. After the reaction was completed as detected by LC-MS, compound 36-7 was obtained, which was used in the next step without further purification.

Step 8: Methyl (tert-butoxycarbonyl)-L-serinate (431 mg, 1.966 mmol) and N,N-diisopropylethylamine (0.34 mL, 1.964 mmol) were added to a solution of compound 36-7 (1.14 g, 1.836 mmol) in dichloromethane (15 mL) at 0 °C. The reaction mixture was stirred at room temperature overnight. The reaction mixture was diluted with dichloromethane (20 mL) and washed with saturated brine (15 mL × 2). The organic layer was dried over anhydrous sodium sulfate, filtered, and concentrated in vacuum. The residue was purified by automated flash chromatography on silica gel (0-2% methanol/dichloromethane) to give compound 36-8 (0.60 g, 0.714 mmol, yield: 38.89%).

Step 9: Lithium hydroxide (29.97 mg, 0.714 mmol) was added to a solution of compound 36-8 (600.0 mg, 0.714 mmol) in tetrahydrofuran (15.0 mL) and water (3.0 mL) at 0 °C, and the reaction mixture was stirred at 0 °C for 1.5 h. After the reaction was completed as monitored by LCMS, the mixture was adjusted to pH 3-5 with 1 M aqueous hydrochloric acid solution, and extracted with ethyl acetate (5 mL × 4). The organic layer was dried over anhydrous sodium sulfate, filtered, and concentrated to dryness in vacuum to give crude compound 36-9, which was used in the next step without further purification. ES-API [M+H]⁺ = 826.4.

Step 10: Methyl (3S)-1,2-diazine-3-carboxylate (450.64 mg, 1.211 mmol), NMM (0.632 mL, 8.475 mmol), 1-(3-dimethylaminopropyl)-3-ethylcarbodiimide hydrochloride (290.1 mg, 1.513 mmol), and 1-hydroxybenzotriazole (41.0 mg, 0.303 mmol) were added to a solution of compound 36-9 (500.0 mg, 0.605 mmol) in dichloromethane (10 mL) at 0 °C. The solution was warmed to room temperature and reacted overnight. After the reaction was completed, the reaction was quenched with water (15 mL), and the reaction mixture was extracted with dichloromethane (10 mL × 3). The organic layer was washed with brine (10 mL), dried over anhydrous sodium sulfate, filtered, and concentrated under reduced pressure. The crude product was purified by automated flash chromatography on silica gel (0-3% methanol/dichloromethane) to give compound 36-10 (0.30 g, 0.315 mmol, yield: 52.05%). ES-API [M+H]⁺ = 952.4.

Step 11: Lithium hydroxide (13.22 mg, 0.315 mmol) was added to a solution of compound 36-10 (300.0 mg, 0.315 mmol) in tetrahydrofuran (15.0 mL) and water (3.0 mL) at 0 °C, and the reaction mixture was stirred at room temperature for 18 h. After the reaction was completed, the mixture was adjusted to pH 3-5 with 1 M aqueous hydrochloric acid solution and extracted with ethyl acetate (25 mL × 4). The organic layer was dried over anhydrous sodium sulfate, filtered, and concentrated to dryness in vacuum to give compound 36-11 (0.28 g, 0.312 mmol, yield: 99.17%). ES-API [M+H]⁺ = 896.3.

Step 12: 1-(3-Dimethylaminopropyl)-3-ethylcarbodiimide hydrochloride (1797.05 mg, 9.374 mmol) was added to a solution of compound 36-11 (280 mg, 0.312 mmol), N,N-diisopropylethylamine (2019.37 mg, 15.624 mmol) and 1-hydroxybenzotriazole (0.42 g, 3.125 mmol) in dichloromethane (5 mL) under an ice-water bath, and the reaction mixture was stirred at room temperature for 18 h. After the reaction was completed as detected by LCMS, the reaction mixture was washed with 1 M diluted hydrochloric acid (100 mL × 2) and saturated brine (150 mL × 3). The organic phase was dried over anhydrous sodium sulfate, filtered, and concentrated to dryness. The crude product was purified by automated flash chromatography on silica gel (tetrahydrofuran/petroleum ether = 0-95%) to give compound 36-12 (80.0 mg, 0.091mmol, yield: 29.0 %). ES-API: [M+H]⁺ = 878.3.

Step 13: A solution of hydrochloric acid in dioxane (1.5 mL, 4.0 M, 6.0 mmol) was added to a solution of compound 36-12 (80.0 mg, 0.091 mmol) in methanol (0.5 mL), and the reaction mixture was stirred at room temperature for 0.5 h. After the reaction was completed as detected by LCMS, the reaction mixture was concentrated under reduced pressure to give compound 36-13 (88.0 mg, crude product). ES-API: [M+H]⁺ = 778.3.

Step 14: (1r,2R,3S)-2,3-Dimethylcyclopropane-1-carboxylic acid (15.5 mg, 0.137 mmol), N,N-diisopropylethylamine (60.0 mL, 0.455 mmol), and O-(7-azabenzotriazol-1-yl)-N,N,N',N'-tetramethyluronium hexafluorophosphate (52.0 mg, 0.137 mmol) were added sequentially to a solution of compound 36-13 (88.0 mg, crude product) in N,N-dimethylformamide (2 mL) under an ice-water bath. The reaction mixture was warmed to room temperature and stirred for 10 min. After the reaction was completed as detected by LCMS, the reaction mixture was diluted with 50.0 mL of ethyl acetate, and then washed with water (50.0 mL) and saturated brine (20.0 mL × 3). The organic phase was dried over anhydrous sodium sulfate, filtered, and concentrated to dryness. The residue was purified by prep-HPLC (ammonium bicarbonate method 1) to give (1r,2R,3S)-N-((7³S,9S)-1²-(5-(3-(1,1-dioxidothiomorpholino)prop-1-yn-1-yl)-2-((S)-1-methoxyethyl)pyridin-3-yl)-1¹-ethyl-(Rₐ)-3,3-dimethyl-6,8,12-trioxo-7¹,7²,7³,7⁴,7⁵,7⁶-hexahydro-1¹H-5,11-dioxa-13-aza-1(3,5)-indola-7(3,1)-pyridazinaocycloundecan-9-yl)-2,3-dimethylcyclopropane-1-carboxamide (Z36, 13.0 mg, 0.015 mmol, yield: 16.34%). ES-API: [M+H]⁺ = 874.3.

### Example 7. Synthesis of Compounds Z15A, Z15, and Z15-1

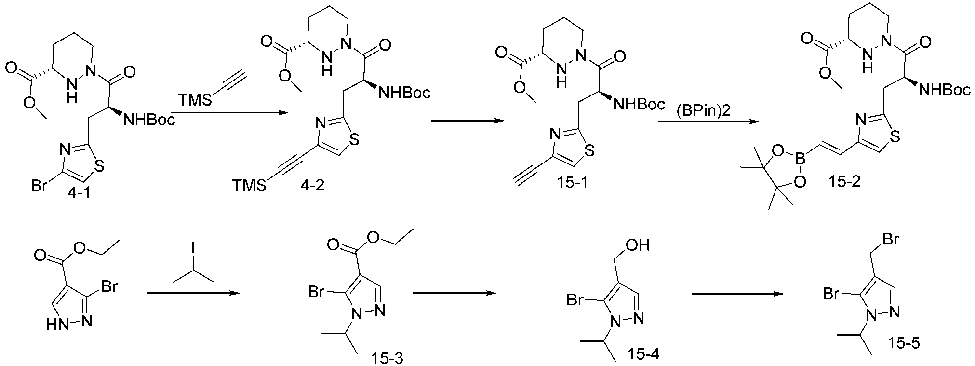

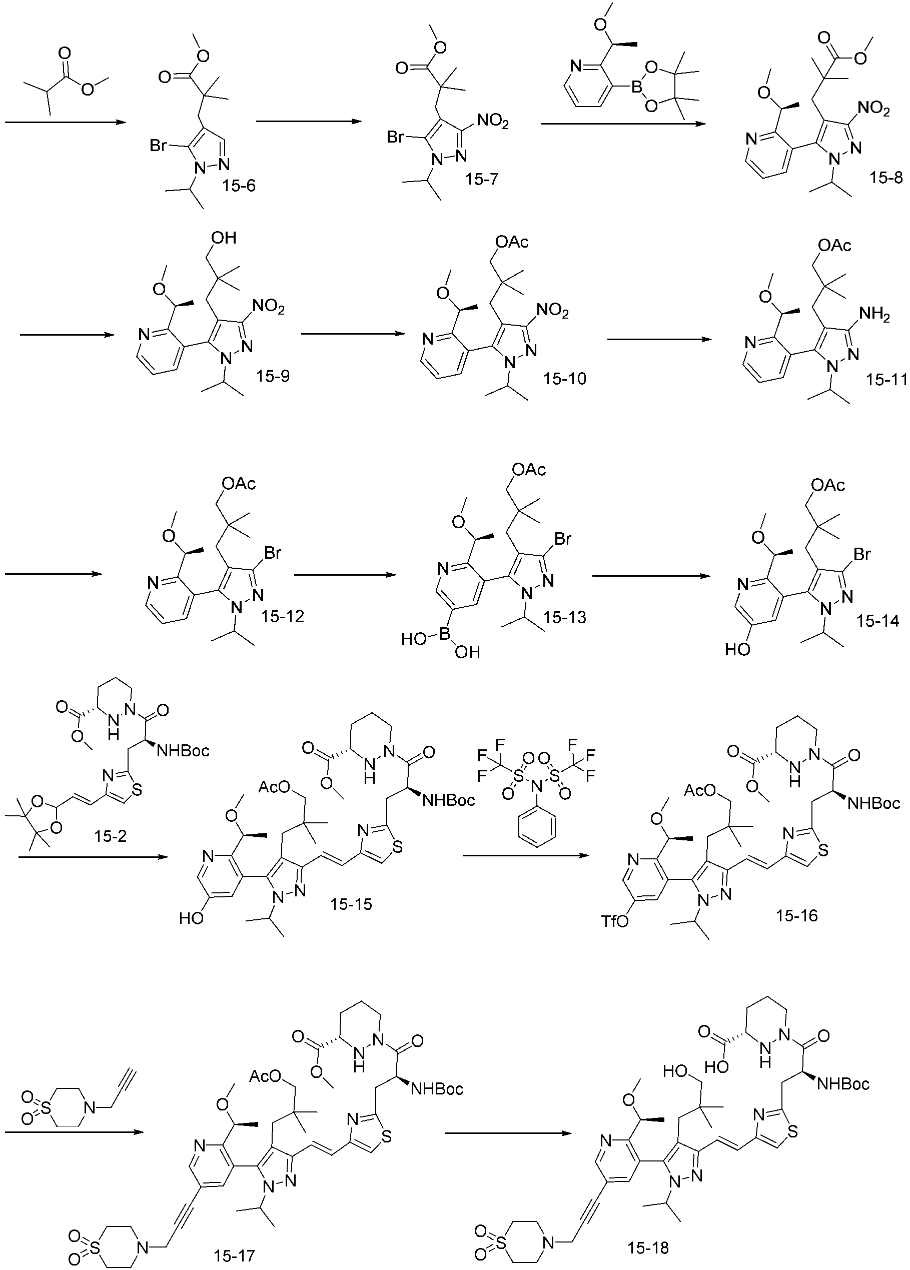

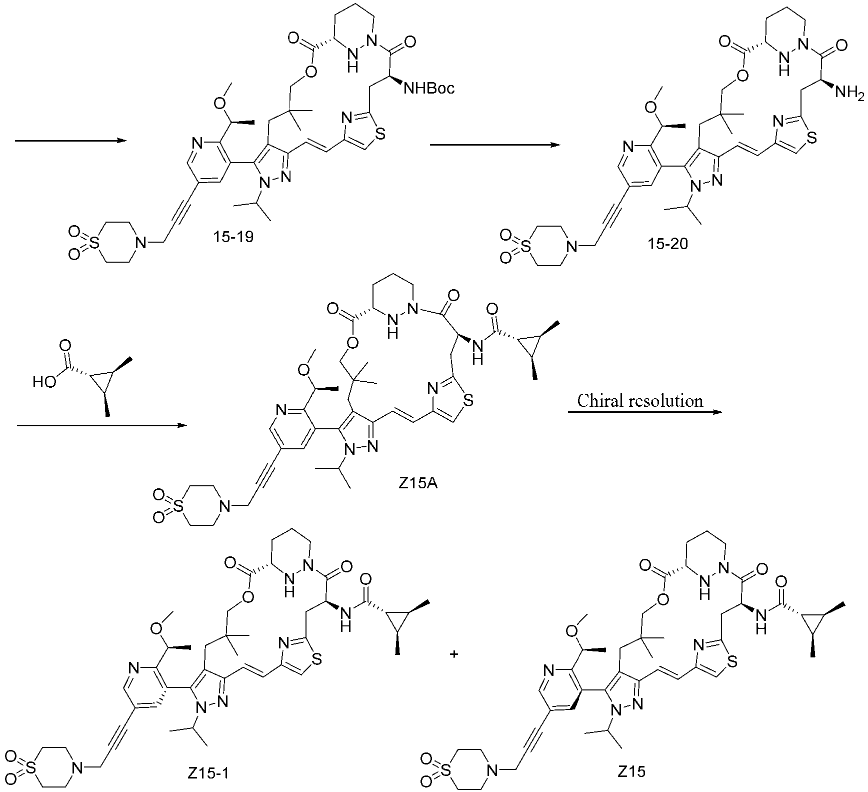

Step 1: Compound 4-1 (1.0 g, 2.10 mmol), bis(triphenylphosphine)palladium(II) dichloride (150 mg, 0.21 mmol), copper(I) iodide (40 mg, 0.21 mmol), and triethylamine (4.4 mL, 31.42 mmol) were dissolved in N,N-dimethylformamide (5 mL) under nitrogen atmosphere, and trimethylsilylacetylene (1.48 mL, 10.47 mmol) was added. The reaction system was stirred at 80 °C for one and a half hours in a microwave reactor. Ethyl acetate (60 mL) was added to the reaction mixture, and the resulting mixture was washed sequentially with water (30 mL × 2) and saturated brine (30 mL × 2). The organic phase was dried over anhydrous sodium sulfate and filtered, and the filtrate was concentrated under reduced pressure. The crude product was purified by flash silica gel column chromatography (ethyl acetate/petroleum ether: 0-60%) to give compound 4-2 (850 mg, yield: 82.0%) as a light brown solid. ES-API: [M+H]⁺ = 495.1.

Step 2: Compound 4-2 (850 mg, 1.718 mmol) was dissolved in methanol (12 mL), and potassium carbonate (712 mg, 5.15 mmol) was added under an ice bath. The reaction system was stirred at room temperature for 1 h. The reaction mixture was filtered. The filtrate was poured into ice water, and the resulting mixture was extracted with ethyl acetate (100 mL). The organic phase was washed with saturated brine (30 mL), dried over anhydrous sodium sulfate, and filtered, and the filtrate was concentrated under reduced pressure. The crude product was purified by flash silica gel column chromatography (ethyl acetate/petroleum ether: 0-80%) to give compound 15-1 (425 mg, yield: 58.5%) as a light yellow solid. ES-API: [M+H]⁺ = 423.1.

Step 3: Copper(I) chloride (9 mg, 0.09 mmol), sodium tert-butoxide (17 mg, 0.18 mmol), and 4,5-bis(diphenylphosphino)-9,9-dimethylxanthene (51 mg, 0.09 mmol) were dissolved in anhydrous tetrahydrofuran (5 mL) under nitrogen atmosphere, and the reaction mixture was stirred at room temperature for 30 min. A solution of bis(pinacolato)diboron (271 mg, 1.06 mmol) in tetrahydrofuran (2 mL) was added, and the reaction system was stirred at room temperature for 10 min. Then a solution of compound 15-1 (375 mg, 0.89 mmol) and absolute methanol (57 mg, 1.78 mmol) in tetrahydrofuran (2 mL) was added, and the reaction system was further stirred at room temperature for 4 h. The reaction mixture was filtered, and the filtrate was concentrated. The crude product was purified by flash silica gel column chromatography (tetrahydrofuran/petroleum ether: 0-60%) to give compound 15-2 (350 mg, yield: 71.6%) as a white solid. ES-API: [M+H]⁺= 551.2.

Step 4: Ethyl 3-bromo-1-H-pyrazole-4-carboxylate (11.0 g, 50.22 mmol) was dissolved in acetonitrile (150 mL), and cesium carbonate (32.72 g, 100.44 mmol) and 2-iodopropane (12.81 g, 75.33 mmol) were added at room temperature. The reaction system was stirred at 85 °C for 18 h. The reaction mixture was concentrated. Ethyl acetate (300 mL) was added, and the mixture was washed sequentially with water (100 mL) and saturated brine (100 mL). The organic phase was dried over anhydrous sodium sulfate and filtered, and the filtrate was concentrated under reduced pressure. The crude product was purified by flash silica gel column chromatography (ethyl acetate/petroleum ether: 0-20%) to give compound 15-3 (4 g, yield: 30.5%) as a colorless liquid. ¹H NMR (400 MHz, CDCl₃) δ 7.98 (s, 1H), 4.79 (hept, J = 6.8 Hz, 1H), 4.31 (q, *J =* 7.2 Hz, 2H), 1.49 (d, *J =* 6.8 Hz, 6H), 1.36 (t, *J =* 7.2 Hz, 3H). ES-API: [M+H]⁺= 261.1, 263.1.

Step 5: Compound 15-3 (500 mg, 1.92 mmol) was dissolved in tetrahydrofuran (10 mL), and DIBAL-H (4.21 mL, 4.21 mmol, 1.0 M in n-hexane) was added dropwise at 0 °C. The reaction system was stirred at room temperature for 1 h. The reaction mixture was cooled to 0 °C. A saturated potassium sodium tartrate solution (20 mL) was slowly added to quench the reaction, and the reaction mixture was stirred at room temperature for 1 h, and extracted with ethyl acetate (30 mL × 2). The organic phase was dried over anhydrous sodium sulfate and filtered, and the filtrate was concentrated under reduced pressure. The crude product was purified by flash silica gel column chromatography (methanol/dichloromethane: 0-5%) to give compound 15-4 (400 mg, yield: 95.3%) as a colorless solid. ES-API: [M+H]⁺= 219.1, 221.1.

Step 6: Compound 15-4 (3.25 g, 14.83 mmol) and triphenylphosphine (5.84 mg, 22.25 mmol) were dissolved in dichloromethane (70 mL), and a solution of carbon tetrabromide (8.36 g, 25.22 mmol) in dichloromethane (15 mL) was added at 0 °C. The reaction system was stirred at room temperature for 18 h. The reaction mixture was concentrated, and the crude product was purified by flash silica gel column chromatography (ethyl acetate/petroleum ether: 0-15%) to give compound 15-5 (2.53 g, yield: 60.5%) as a colorless liquid. Step 7: Methyl isobutyrate (1.09 g, 10.64 mmol) was dissolved in tetrahydrofuran (40 mL), and a 2.0 M solution of lithium diisopropylamide in tetrahydrofuran/n-hexane (5.32 mL, 10.64 mmol) was added dropwise at -70°C. The reaction system was stirred at -70 °C for 1 h. A solution of compound 15-5 (2 g, 7.09 mmol) in tetrahydrofuran (3 mL) was added dropwise. The reaction system was stirred at -70 °C for 1 h, and then slowly warmed to room temperature and stirred for 1 h. The reaction was quenched with a saturated ammonium chloride solution (25 mL), and the reaction mixture was extracted with ethyl acetate (100 mL). The organic phase was washed with saturated brine (50 mL), dried over anhydrous sodium sulfate, and filtered, and the filtrate was concentrated under reduced pressure. The crude product was purified by flash silica gel column chromatography (ethyl acetate/petroleum ether: 0-20%) to give compound 15-6 (2.0 g, yield: 93.0%) as a light yellow liquid. ES-API: [M+H]⁺ = 303.1,305.1.

Step 8: Compound 15-6 (2 g, 6.60 mmol) was dissolved in acetic anhydride (15 mL), and concentrated nitric acid (2.18 mL, 19.79 mmol) was added dropwise at 0 °C. Then the mixture was stirred at room temperature for 1 h. The reaction mixture was poured into ice water, and a saturated sodium bicarbonate solution (50 mL) was slowly added. The mixture was extracted with ethyl acetate (80 mL × 2). The organic phase was washed with water (50 mL × 2) and saturated brine (50 mL), dried over anhydrous sodium sulfate, and filtered, and the filtrate was concentrated under reduced pressure. The crude product was purified by flash silica gel column chromatography (ethyl acetate/petroleum ether: 0-30%) to give a crude product, which was purified by flash silica gel column chromatography (ethyl acetate/petroleum ether: 0-20%) to give compound 15-7 (2.0 g, yield: 87.1%) as a light yellow solid. ES-API: [M+H]⁺ = 348.1,350.1.

Step 9: Compound 15-7 (150 mg, 0.43 mmol) and 2-[(1S)-1-methoxyethyl]-3-(4,4,5,5-tetramethyl-1,3,2-dioxaborolan-2-yl)pyridine (170 mg, 0.65 mmol) were dissolved in toluene (2.5 mL) and water (0.5 mL), and potassium phosphate (274 mg, 1.29 mmol) and methanesulfonyloxy(diadamantyl-n-butylphosphino)-2'-amino-1,1'-biphenyl-2-yl)palladium(II) (47 mg, 0.065 mmol) were added. The reaction system was purged with nitrogen for 30 s and stirred at 110 °C for 1 h in a microwave reactor. Ethyl acetate (40 mL) and water (5 mL) were added to the reaction mixture. The separated organic phase was washed with saturated brine (10 mL), dried over anhydrous sodium sulfate, and filtered, and the filtrate was concentrated under reduced pressure. The crude product was purified by flash silica gel column chromatography (ethyl acetate/petroleum ether: 0-60%) to give compound 15-8 (125 mg, yield: 71.7%) as a light brown liquid. ES-API: [M+H]⁺ = 405.3.

Step 10: Compound 15-8 (625 mg, 1.55 mmol) was dissolved in dichloromethane (15 mL), and DIBAL-H (3.40 mL, 3.40 mmol, 1 M n-hexane solution) was added dropwise at -70 °C. The reaction system was stirred at -70 °C for 1 h. Water (0.1 mL), 15% sodium hydroxide solution (0.1 mL), and water (0.25 mL) were added sequentially to the reaction mixture. The reaction system was stirred at room temperature for 15 min. Anhydrous magnesium sulfate was added, and the reaction system was further stirred for 15 min, and filtered. The filter cake was washed with ethyl acetate, and the filtrate was concentrated under reduced pressure. The crude product was purified by flash silica gel column chromatography (methanol/dichloromethane: 0-5%) to give compound 15-9 (450 mg, yield: 77.4%) as a light brown liquid. ES-API: [M+H]⁺= 377.2.

Step 11: Compound 15-9 (450 mg, 1.20 mmol), diisopropylethylamine (463 mg, 3.59 mmol), and 4-dimethylaminopyridine (15 mg, 0.12 mmol) were dissolved in dichloromethane (10 mL), and acetic anhydride (183 mg, 0.79 mmol) was added at 0 °C. The reaction system was stirred at 0 °C for 30 min. The reaction mixture was concentrated, and the crude product was purified by flash silica gel column chromatography (ethyl acetate/petroleum ether: 0-60%) to give compound 15-10 (485 mg, yield: 97.0%) as a light yellow viscous liquid. ES-API: [M+H]⁺ = 419.2.

Step 12: Compound 15-10 (485 mg, 1.16 mmol) was dissolved in ethanol (12 mL) and water (4.8 mL), and iron powder (323 mg, 5.80 mmol) and ammonium chloride (310 mg, 5.80 mmol) were added at room temperature. The reaction system was stirred at 80 °C for 4 h. The reaction mixture was filtered. The filter cake was washed with methanol, and the filtrate was concentrated under reduced pressure. Dichloromethane (30 mL) was added, and the mixture was washed with saturated brine (20 mL), dried over anhydrous sodium sulfate, and filtered. The filtrate was concentrated under reduced pressure. The resulting crude product was purified by flash silica gel column chromatography (methanol/dichloromethane: 0-5%) to give compound 15-11 (360 mg, yield: 80.0%) as a light yellow viscous liquid. ES-API: [M+H]⁺ = 389.3.

Step 13: Compound 15-11 (260 mg, 0.67 mmol) was dissolved in acetonitrile (15 mL), copper(I) bromide (192 mg, 1.34 mmol) and copper bromide (15 mg, 0.07 mmol) were added under an ice bath, and then a solution of tert-butyl nitrite (103 mg, 1.00 mmol) in acetonitrile (0.5 mL) was added dropwise. The reaction system was stirred at 50 °C for 3 h. Water (15 mL) was added to the reaction mixture, and the resulting mixture was extracted with ethyl acetate (40 mL × 2). The organic phases were combined, washed with saturated brine (30 mL), dried over anhydrous sodium sulfate, and filtered. The filtrate was concentrated under reduced pressure. The resulting crude product was purified by flash silica gel column chromatography (ethyl acetate/petroleum ether: 0-60%) to give compound 15-12 (220 mg, yield: 72.7%) as a light yellow viscous liquid. ES-API: [M+H]⁺ = 452.1,454.1.

Step 14: Compound 15-12 (170 mg, 0.38 mmol), bis(pinacolato)diboron (162 mg, 0.64 mmol), chloro(1,5-cyclooctadiene)iridium(I) dimer (25 mg, 0.038 mmol), and 4,4'-di-tert-butyl-2,2'-bipyridine (20 mg, 0.075 mmol) were dissolved in cyclohexane (15 mL). The reaction system was stirred at 80 °C for 17 h under nitrogen atmosphere. The reaction mixture was concentrated to give compound 15-13 (372 mg, crude product) as a black oily liquid. ES-API: [M+H]⁺ = 496.1,498.1.

Step 15: Compound 15-13 (372 mg, crude product) was dissolved in acetonitrile (6 mL), and 30% hydrogen peroxide (128 mg, 0.33 mmol) was added under an ice bath. The reaction system was warmed to room temperature and stirred for 2 h. After the reaction was completed, the reaction was quenched with saturated sodium sulfite (30 mL). The reaction mixture was extracted with ethyl acetate (50 mL). The organic phase was washed with saturated brine (30 mL), dried, and concentrated to give a crude product. The crude product was purified by flash silica gel column chromatography (tetrahydrofuran/petroleum ether: 0-50%) to give the desired product, compound 15-14 (150 mg, two-step yield: 85.4%) as a yellow liquid. ES-API: [M+H]⁺ = 468.1,470.1.

Step 16: Compound 15-14 (125 mg, 0.27 mmol) and compound 15-2 (220 mg, 0.40 mmol) were dissolved in toluene (0.5 mL), dioxane (1.5 mL), and water (0.5 mL), and potassium phosphate (142 mg, 0.67 mmol) and [1,1'-bis(di-tert-butylphosphino)ferrocene]dichloropalladium(II) (26 mg, 0.040 mmol) were added. The reaction system was purged 3 times with nitrogen and stirred at 80 °C for 3 h. Ethyl acetate (50 mL) and water (15 mL) were added to the reaction mixture. The separated organic phase was washed with saturated brine (15 mL), dried over anhydrous sodium sulfate, and filtered, and the filtrate was concentrated under reduced pressure. The crude product was purified by flash silica gel column chromatography (tetrahydrofuran/petroleum ether: 0-60%) to give compound 15-15 (160 mg, yield: 73.8%) as a white solid. ES-API: [M+H]⁺ = 812.3.

Step 17: Compound 15-15 (110 mg, 0.135 mmol) was dissolved in dichloromethane (5 mL), and diisopropylethylamine (52 mg, 0.41 mmol) and 1,1,1-trifluoro-N-phenyl-N-[(trifluoromethyl)sulfonyl]methanesulfonamide (72 mg, 0.20 mmol) were added. The reaction system was stirred at room temperature for 2 h. The reaction mixture was concentrated. The crude product was purified by flash silica gel column chromatography (tetrahydrofuran/petroleum ether: 0-60%) to give compound 15-16 (120 mg, yield: 93.8%) as a white solid. ES-API: [M+H]⁺ = 944.2.

Step 18: Compound 15-16 (120 mg, 0.13 mmol) was dissolved in N,N-dimethylformamide (5 mL), and 4-prop-2-ynyl-thiomorpholine-1,1-dioxide (88 mg, 0.51 mmol), bis(triphenylphosphine)palladium(II) dichloride (4.8 mg, 0.007 mmol), copper(I) iodide (2.6 mg, 0.014 mmol), N,N'-diisopropylethylamine (27 mg, 0.038 mmol), and lithium chloride (22 mg, 0.51 mmol) were added. The reaction system was stirred at 80 °C for 18 h under argon atmosphere. Water (20 mL) was added to the reaction mixture, and the resulting mixture was extracted with ethyl acetate (30 mL × 2). The organic phase was washed with saturated brine (20 mL × 2), dried, and concentrated to give a crude product. The crude product was purified by flash silica gel column chromatography (tetrahydrofuran/petroleum ether: 0-60%) to give compound 15-17 (100 mg, yield: 81.3%) as a light brown solid. ES-API: [M+H]⁺ = 967.3.

Step 19: Compound 15-17 (130 mg, 0.134 mmol) was dissolved in tetrahydrofuran (3 mL) and water (0.5 mL), and lithium hydroxide monohydrate (17 mg, 0.40 mmol) was added at 0 °C. The reaction system was stirred at room temperature for 18 h. The reaction mixture was adjusted to pH 6 with 1 M diluted hydrochloric acid and extracted with ethyl acetate (30 mL × 2). The organic phase was dried over anhydrous sodium sulfate and concentrated to give compound 15-18 (120 mg, yield: 98.0%) as a light yellow solid. ES-API: [M+H]⁺ = 911.3.

Step 20: Compound 15-18 (95 mg, 0.10 mmol) was dissolved in dichloromethane (10 mL), and 1-(3-dimethylaminopropyl)-3-ethylcarbodiimide hydrochloride (560 mg, 2.92 mmol), 1-hydroxybenzotriazole (70 mg, 0.52 mmol), and diisopropylethylamine (404 mg, 3.13 mmol) were added at room temperature. The reaction system was stirred at room temperature for 18 h. Dichloromethane (25 mL) was added to the reaction mixture, and the resulting mixture was washed sequentially with water (15 mL × 2) and saturated brine (15 mL), dried over anhydrous sodium sulfate, and filtered. The filtrate was concentrated under reduced pressure. The resulting crude product was purified by flash silica gel column chromatography (methanol/dichloromethane = 0-5%) to give compound 15-19 (35 mg, yield: 37.6%) as a white solid. ES-API: [M+H]⁺ = 893.3.

Step 21: Compound 15-19 (30 mg, 0.034 mmol) was dissolved in absolute methanol (0.2 mL), and a 4.0 M hydrogen chloride/dioxane solution (2.0 mL) was added at 0 °C. The reaction system was stirred at room temperature for 1 h. The reaction mixture was concentrated to give compound 15-20 (26 mg, yield: 97.6%) as a white solid. ES-API: [M+H]⁺ = 793.3.

Step 22: Compound 15-20 (26 mg, 0.033 mmol) was dissolved in dichloromethane (5 mL), and (1r,2R,3S)-2,3-dimethylcyclopropane-1-carboxylic acid (6 mg, 0.049 mmol), O-(7-azabenzotriazol-1-yl)-N,N,N',N'-tetramethyluronium hexafluorophosphate (25 mg, 0.066 mmol), and N,N'-diisopropylethylamine (26 mg, 0.20 mmol) were added. The reaction system was stirred at room temperature for 1 h. Water (10 mL) was added to the reaction mixture, and the resulting mixture was extracted with ethyl acetate (40 mL). The organic phase was washed with saturated brine (10 mL), dried over anhydrous sodium sulfate, and filtered, and the filtrate was concentrated under reduced pressure. The crude product was purified by prep-HPLC (ammonium bicarbonate method 1) to give the desired product (1r,2R,3S)-N-((4²Z,8³S,2E,6S)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵ 3⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z15A, 19 mg, yield: 65.2%) as a white solid. ES-API: [M+H]⁺ = 889.3.

Step 23: Compound Z15A (18 mg, 0.020 mmol) was subjected to preparative chiral resolution (separation column: IB, 250 mm × 4.6 mm × 5 µm, mobile phase: n-hexane:ethanol:diethylamine = 50:50:2, flow rate: 1 mL/min, column temperature: 30 °C) to give 2 isomeric compounds. The structure of one of the isomeric compounds was arbitrarily designated as (1r,2R,3S)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵ 3⁶-hexahydro-1¹H-10-oxa-4(4,2)-thiazola-8(1,3)-pyridazina-1(3,4)-pyrazolocyclotridec-2-en-6-yl)-2,3-dimethylcyclopropane-1-carboxamide (Z15, 5 mg, retention time: 8.536 min, yield: 27.8%) as a white solid. ES-API :[M+H]⁺ = 889.3.

The structure of the other isomeric compound was arbitrarily designated as (1r,2R,3S)-N-((4²Z,8³S,2E,6S)-1⁵-(Sₐ)-(5-(3-(1,1-dioxidothiomorpholino)prop-1-yn-1-yl)-2-((S)-1-methoxyethyl)pyridin-3-yl)-1'-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-2,3-dimethylcyclopropane-1-carboxamide (Z15-1, 8 mg, retention time: 9.778 min, yield: 44.4%) as a white solid. ES-API: [M+H]⁺ = 889.3. ¹H NMR (400 MHz, DMSO-d₆) δ 8.80 (d, *J =* 1.6 Hz, 1H), 8.39 (d, *J =* 8.8 Hz, 1H), 7.89 (d, *J =* 1.6 Hz, 1H), 7.48 (s, 1H), 7.37 (d, *J=* 15.6 Hz, 1H), 7.25 (d, *J =* 15.6 Hz, 1H), 5.47 (t, *J =* 8.8 Hz, 1H), 5.01 (d, *J =* 12.0 Hz, 1H), 4.21 (d, *J* = 11.6 Hz, 1H), 4.11 - 3.89 (m, 3H), 3.76 (s, 2H), 3.65 (d, *J =* 10.8 Hz, 1H), 3.55 (d, *J =* 11.2 Hz, 1H), 3.26 - 3.08 (m, 9H), 3.07 - 2.99 (m, 4H), 2.80 - 2.70 (m, 2H), 2.13 - 2.00 (m, 2H), 1.77 - 1.66 (m, 2H), 1.50 - 1.40 (m, 4H), 1.28 - 1.23 (m, 6H), 1.19 - 1.12 (m, 3H), 1.09 - 1.02 (m, 6H), 0.83 (s, 3H), 0.43 (s, 3H).

### Example 8. Synthesis of Compounds Z8A, Z8, and Z8-1

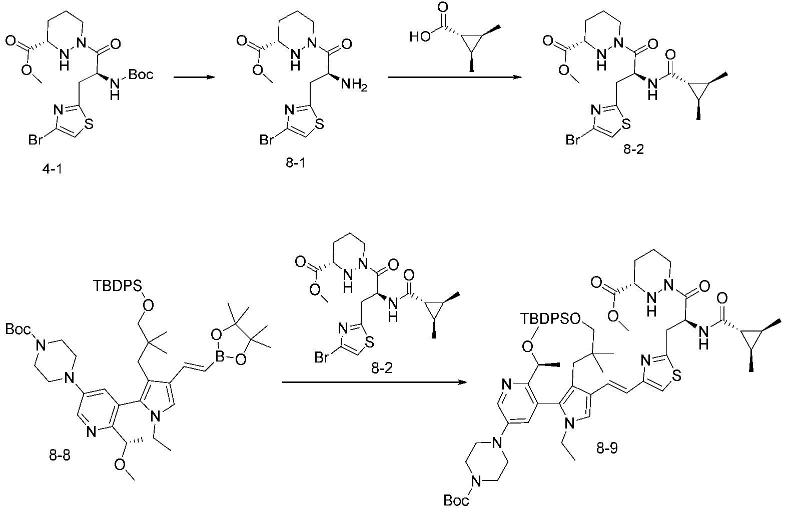

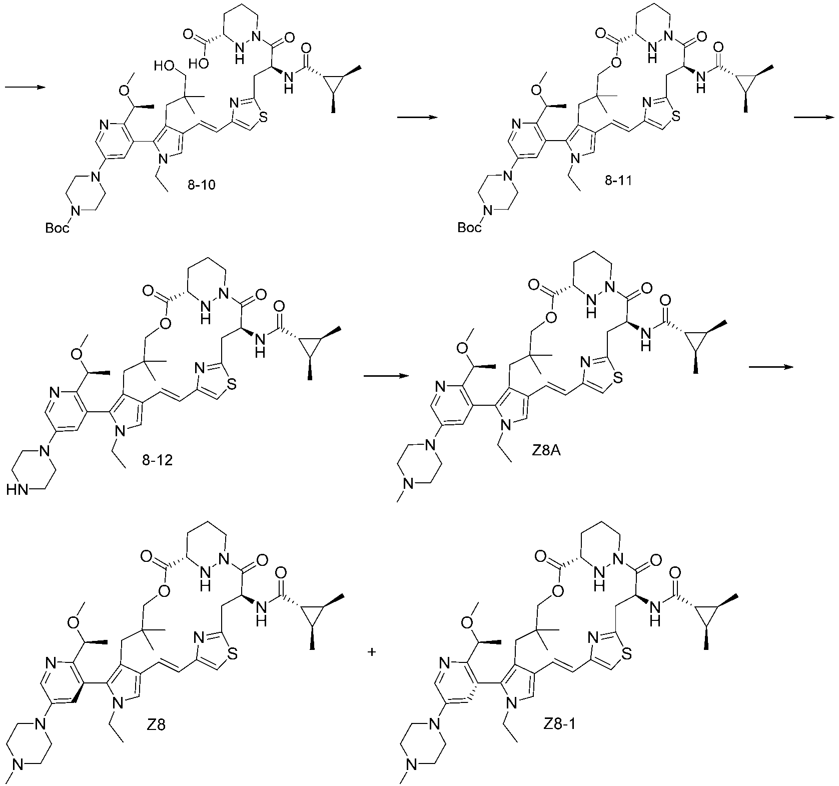

Step 1: A 4 M hydrogen chloride/dioxane solution (5 mL) was added to a solution of compound 4-1 (1 g, 2.1 mmol) in methanol (1 mL), and the mixture was stirred at room temperature for 1 h. After the reaction was completed, the reaction mixture was concentrated to give compound 8-1 (867 mg) as a white solid. ES-API: [M+H]⁺ = 377.0.

Step 2: Compound 8-1 (867 mg, 2.10 mmol) and N,N-diisopropylethylamine (1.83 mL, 10.45 mmol) were dissolved in dichloromethane (20 mL), and (1r,2R,3S)-2,3-dimethylcyclopropane-1-carboxylic acid (263 mg, 2.31 mmol) and O-(7-azabenzotriazol-1-yl)-N,N,N',N'-tetramethyluronium hexafluorophosphate (1200 mg, 3.15 mmol) were added sequentially. The mixture was stirred at room temperature for 1 h. The reaction mixture was washed sequentially with 5 mL of water and 5 mL of saturated brine, dried over anhydrous sodium sulfate, filtered, and concentrated, and the residue was purified by flash silica gel column chromatography (0-50% tetrahydrofuran/petroleum ether) to give compound 8-2 (900 mg, yield: 91%) as a white solid. ES-API: [M+H]⁺ = 473.1, 475.0.

Step 3: A mixed solution of compound 8-8 (80 mg, 0.09 mmol), compound 8-2 (430 mg, 0.09 mmol), [1,1'-bis(di-tert-butylphosphino)ferrocene]dichloropalladium(II) (6 mg, 0.01 mmol), and potassium phosphate (57 mg, 0.27 mmol) in toluene (0.9 mL), dioxane (0.3 mL), and water (0.3 mL) was reacted under microwave irradiation at 75 °C for 0.5 h under nitrogen atmosphere. The reaction mixture was washed with 1 mL of saturated brine and 1 mL of water, and extracted with 2 mL of ethyl acetate. The organic phases were combined, dried over anhydrous sodium sulfate, filtered, and concentrated, and the residue was purified by flash silica gel column chromatography (0-30% tetrahydrofuran/petroleum ether) to give compound 8-9 (90 mg, yield: 86.60%). ES-API: [M+H]⁺ = 1157.2.

Step 4: Tetrabutylammonium fluoride (1 mL, 1.00 mmol) was added to a solution of compound 8-9 (70 mg, 0.060 mmol) in tetrahydrofuran (2 mL), and the mixture was stirred at 70 °C for 5 h. The reaction mixture was dissolved in 5 mL of saturated ammonium chloride solution, and the solution was extracted three times with 5 mL of ethyl acetate. The organic phases were combined, dried over anhydrous sodium sulfate, filtered, and concentrated by rotary evaporation to give compound 8-10 (40 mg). ES-API: [M+H]⁺ = 905.2.

Step 5: Compound 8-10 (120 mg, 0.133 mmol) was dissolved in dichloromethane (20 mL), and N,N-diisopropylethylamine (0.81 mL, 4.64 mmol), 1-hydroxybenzotriazole (143 mg, 1.06 mmol), and 1-(3-dimethylaminopropyl)-3-ethylcarbodiimide hydrochloride (762 mg, 3.98 mmol) were added. The mixture was stirred at room temperature overnight. The reaction mixture was washed with water, concentrated, and purified by preparative thin-layer chromatography (dichloromethane:methanol = 20:1) to give compound 8-11 (60 mg, yield: 51%) as a white solid. ES-API: [M+H]⁺ = 887.2.

Step 6: A hydrogen chloride/dioxane solution (1 mL, 4 mmol) was added dropwise to a solution of compound 8-11 (30 mg, 0.034 mmol) in methanol (0.2 mL), and the mixture was stirred at room temperature for 1 h. The reaction mixture was concentrated to give compound 8-12 (27 mg, 0.034 mmol). ES-API: [M+H]⁺ = 787.3.

Step 7: Compound 8-12 (27 mg, 0.034 mmol) was dissolved in methanol (1 mL), and one drop of 37% aqueous formaldehyde solution was added under an ice bath, followed by the addition of sodium borohydride acetate (11 mg, 0.051 mmol). The mixture was stirred at room temperature for 1 h. After the reaction was completed, the reaction was quenched with 3 mL of saturated sodium bicarbonate solution, and the reaction mixture was extracted three times with 5 mL of ethyl acetate. The organic phases were combined, dried over anhydrous sodium sulfate, filtered, and concentrated by rotary evaporation, and the residue was purified by preparative thin-layer chromatography (dichloromethane:methanol = 10:1) to give (1r,2S,3R)-N-((4²Z,8³R,2E,6R)-1¹-ethyl-1⁵-(2-((S)-1-methoxyethyl)-5-(4-methylpiperazin-1-yl)pyridin-3-yl)-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrrolacyclotridecaphan-2-en-6-yl)-2,3-dimethylcyclopropane-1-carboxamide (Z8A, 10 mg, yield: 36%). ES-API: [M+H]⁺ = 801.3.

Step 8: Compound Z8A (8 mg) was purified by prep-HPLC (ammonium bicarbonate method 1) to give 2 isomeric compounds. The structure of one of the isomeric compounds was arbitrarily designated as (1r,2R,3S)-*N*-((4²Z,8³R,2E,6R)-1¹-ethyl-(Rₐ)-1⁵-(2-((S)-1-methoxyethyl)-5-(4-methylpiperazin-1-yl)pyridin-3-yl)-12,12-dime^{t}hyl-7,9-dioxo-8¹,8²,8³,8⁴,8⁵-8⁶-hexahydro-1¹*H*-10-oxa-4(4,2)-thiazola-8(1,3)-pyridazina-1(3,4)-pyrrolacyclotridecaphan-2-en-6-yl)-2,3-dimethylcyclopropane-1-carboxamide (Z8, 2.77 mg, yield: 35%, retention time: 7.48 min). ES-API: [M+H]⁺ = 801.3. ¹H NMR (400 MHz, DMSO) δ 8.37 (d, *J=* 2.8 Hz, 1H), 8.33 (d, *J* = 8.8 Hz, 1H), 7.41 - 7.29 (m, 2H), 7.11 (s, 1H), 7.08 (d, *J =* 2.8 Hz, 1H), 6.79 (d, *J =* 15.7 Hz, 1H), 5.53 (t, *J =* 8.2 Hz, 1H), 5.00 (d, *J =* 12.2 Hz, 1H), 4.21 (d, *J =* 11.0 Hz, 1H), 4.12 (q, *J =* 6.1 Hz, 1H), 3.95 - 3.76 (m, 3H), 3.67 (d, *J =* 10.7 Hz, 1H), 3.53 (d, *J =* 10.8 Hz, 1H), 3.27 - 3.21 (m, 4H), 3.21 - 3.18 (m, 1H), 3.17 (s, 3H), 3.14 - 3.05 (m, 1H), 2.78 - 2.66 (m, 1H), 2.62 - 2.54 (m, 1H), 2.49 - 2.42 (m, 4H), 2.35 - 2.25 (m, 1H), 2.22 (s, 3H), 1.99 - 1.90 (m, 1H), 1.79 - 1.71 (m, 1H), 1.69 - 1.55 (m, 1H), 1.53 - 1.39 (m, 1H), 1.32 (d, *J =* 6.0 Hz, 3H), 1.23 - 1.10 (m, 3H), 1.10 - 1.02 (m, 9H), 0.70 (s, 3H), 0.31 (s, 3H).

The structure of the other isomeric compound was arbitrarily designated as (1r,2R,3S)-*N*-((4²Z,8³R,2E,6R)-1¹-ethyl-(Sₐ)-1⁵-(2-((S)-1-methoxyethyl)-5-(4-methylpiperazin-1-yl)pyridin-3-yl)-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrrolacyclotridecaphan-2-en-6-yl)-2,3-dimethylcyclopropane-1-carboxamide (Z8-1, 1.30 mg, yield: 16%, retention time: 7.36 min). ES-API: [M+H]⁺ = 801.3. ¹H NMR (400 MHz, DMSO) δ 8.38 (d, *J=* 2.8 Hz, 1H), 8.34 (d, *J=* 8.8 Hz, 1H), 7.38 (s, 1H), 7.34 (d, *J =* 15.6 Hz, 1H), 7.17 (d, *J=* 2.8 Hz, 1H), 7.12 (s, 1H), 6.78 (d, *J =* 15.6 Hz, 1H), 5.46 (t, *J =* 9.2 Hz, 1H), 4.96 (d, *J =* 12.0 Hz, 1H), 4.21 (d, *J =* 12.4 Hz, 1H), 4.02 - 3.88 (m, 2H), 3.69 - 3.60 (m, 2H), 3.57 - 3.45 (m, 2H), 3.27 - 3.11 (m, 6H), 3.09 (s, 3H), 2.71 (s, 2H), 2.48 - 2.39 (m, 4H), 2.22 (s, 3H), 2.16 (d, *J =* 14.0 Hz, 1H), 2.05 - 1.97 (m, 1H), 1.84 - 1.64 (m, 2H), 1.54 - 1.39 (m, 1H), 1.21 - 1.10 (m, 9H), 1.09 - 1.03 (m, 6H), 0.78 (s, 3H), 0.47 (s, 3H).

### Example 9. Synthesis of Compound Z37

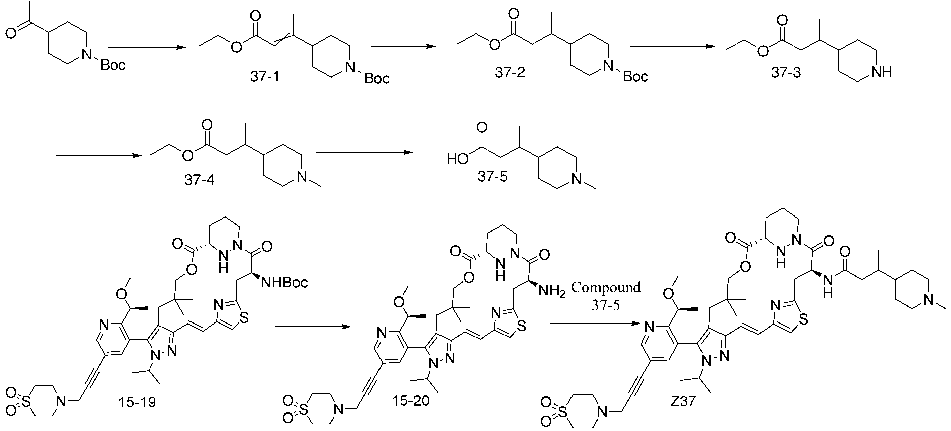

Step 1: Sodium hydride (1.27 g, 31.67 mmol) was added to 30.0 mL of anhydrous tetrahydrofuran. A solution of triethyl phosphonoacetate (7.10 g, 31.676 mmol) in tetrahydrofuran (10 mL) was added dropwise at 0 °C. After 1 h of reaction, a solution of tert-butyl 4-acetylpiperidine-1-carboxylate (6.0 g, 26.39 mmol) in tetrahydrofuran (30 mL) was added. After the addition was completed, the reaction mixture was warmed to 20 °C and reacted for 3 h. After the reaction was completed, the reaction was quenched with saturated ammonium chloride (60 mL). The reaction mixture was extracted with ethyl acetate (200 mL). The organic phase was dried over anhydrous sodium sulfate and concentrated by rotary evaporation under reduced pressure to remove the solvent to give compound 37-1 (5.40 g, yield: 68.79%). ES-API: [M-56+H]⁺ = 225.3. Step 2: Pd/C (0.6 g) was added to a solution of compound 37-1 (5.4 g, 18.158 mmol) in ethanol (20.0 mL), and the mixture was stirred at 25 °C for 3-12 h under hydrogen atmosphere. After the reaction was completed as monitored by LCMS, the solution was filtered, and the filtrate was dried under reduced pressure to give compound 37-2 (4.0 g, yield: 73.57%). ES-API: [M-Boc+H]⁺ = 200.1.

Step 3: Trifluoroacetic acid (5.0 mL, 65.0 mmol) was added to a solution of compound 37-2 (1.11 g, 3.707 mmol) in dichloromethane (5.0 mL) at 0 °C, and the mixture was stirred for 1 h. After the reaction was completed as detected by LCMS, the reaction mixture was concentrated by rotary evaporation under reduced pressure to remove the solvent to give compound 37-3 (1.50 g, crude product), which was directly used in the next step. ES-API: [M+H]⁺ = 200.2.

Step 4: Pd/C (0.3 g) and paraformaldehyde (1.0 mL, 12.7 mmol) were added to a solution of compound 37-3 (0.74 g, 3.707 mmol) in methanol (20.0 mL), and the mixture was stirred at 25 °C for 18 h under hydrogen atmosphere. After the reaction was completed, the reaction mixture was filtered, and the filtrate was concentrated to give compound 37-4 (0.50 g, yield: 63.23%). ES-API: [M-56+H]⁺ = 214.3.

Step 5: Lithium hydroxide (118.0 mg, 2.813 mmol) was added to a solution of compound 37-4 (100 mg, 0.469 mmol) in tetrahydrofuran/water (6.0 mL/1.0 mL), and the mixture was stirred at 60 °C for 12 h. After the reaction was completed as monitored by LC-MS, the reaction mixture was concentrated by rotary evaporation under reduced pressure to remove the solvent to give compound 37-5 (1.50 g, crude product), which was directly used in the next step. ES-API: [M-56+H]⁺ = 186.3.

Step 6: A 4.0 M solution of hydrochloric acid in dioxane (1 mL, 4.000 mmol) was added to Compound 15-19 (60.0 mg, 0.067 mmol), and the mixture was stirred at room temperature for 1 h. After the reaction was completed, the mixture was concentrated in vacuum to give compound 15-20 (70.0 mg, crude product) as a white solid. ES-API: [M-56+H]⁺ = 793.3.

Step 7: Compound 37-5 (22.23 mg, 0.120 mmol), O-(7-azabenzotriazol-1-yl)-N,N,N',N'-tetramethyluronium hexafluorophosphate (57.04 mg, 0.150 mmol), and N,N-diisopropyldiamine (0.030 mL, 0.180 mmol) were added to a solution of compound 15-20 (23.79 mg, 0.03 mmol) in N,N-dimethylformamide (2 mL), and the reaction mixture was stirred at room temperature for 1 h. After the reaction was completed as monitored by LCMS, the reaction mixture was purified by prep-HPLC (formic acid method 2) to give N-((4²Z,8³S,2E,6S)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,3⁶-hexahydro-1¹H-10-oxa-4(4,2)-thiazola-8(1,3)-pyridazina-1(3,4)-pyrrolacyclotridecaphan-2-en-6-yl)-3-(1-methylpiperidin-4-yl)butanamide (Z37, 10.0 mg, yield: 37.49%). ES-API: [M+H]⁺ = 960.4.

### Example 10. Synthesis of Compound Z38

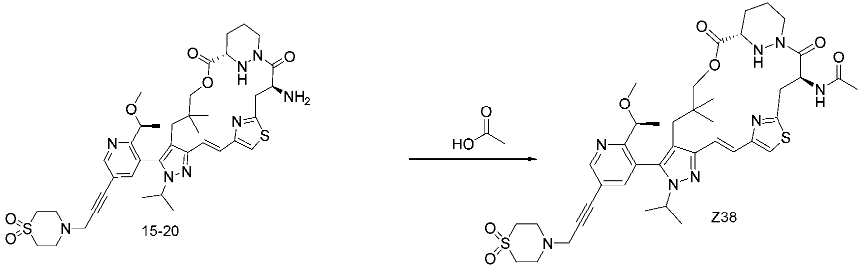

Acetic acid (7.20 mg, 0.120 mmol), O-(7-azabenzotriazol-1-yl)-N,N,N',N'-tetramethyluronium hexafluorophosphate (57.04 mg, 0.150 mmol), and N,N-diisopropyldiamine (0.030 mL, 0.180 mmol) were added to a solution of compound 15-20 (23.79 mg, 0.03 mmol) in N,N-dimethylformamide (2 mL), and the reaction mixture was stirred at room temperature for 1 h. After the reaction was completed as monitored by LCMS, the reaction mixture was purified by prep-HPLC (formic acid method 2) to give N-((4²Z,8³S,2E,6S)-1⁵-(5-(3-(1,1-dioxidothiomorpholino)prop-1-yn-1-yl)-2-((S)-1-methoxyethyl)pyridin-3-yl)-1¹-isopropyl-(Rₐ)-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)acetamide formate (Z38, 4.46 mg, yield: 17.84%). ES-API: [M+H]⁺= 835.3.

### Example 11. Synthesis of Compound Z39

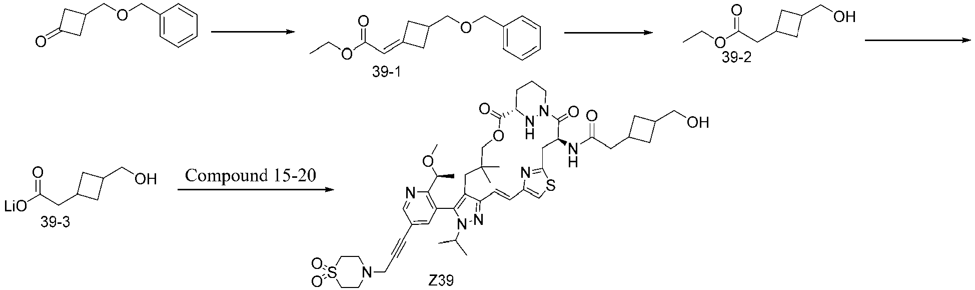

Step 1: Sodium hydride (0.50 g, 12.61mmol) was added to 30.0 mL of anhydrous tetrahydrofuran. A solution of triethyl phosphonoacetate (2.83 g, 12.61 mmol) in tetrahydrofuran (10 mL) was added dropwise at 0 °C. After 1 h of reaction, a solution of 3-(benzyloxy)methyl)cyclobutan-1-one (2.0 g, 10.513 mmol) in tetrahydrofuran (30 mL) was added. After the addition was completed, the reaction mixture was warmed to 20 °C and reacted for 3 h. After the reaction was completed, the reaction was quenched with saturated ammonium chloride (30 mL). The reaction mixture was extracted with ethyl acetate (100 mL). The organic phase was dried over anhydrous sodium sulfate and concentrated by rotary evaporation under reduced pressure to remove the solvent to give compound 39-1 (2.26 g, yield: 82.58%).

Step 2: Pd/C (0.6 g) was added to a solution of compound 39-1 (2.26 g, 8.681 mmol) in ethanol (20.0 mL), and the mixture was stirred at 25 °C for 3-12 h under hydrogen atmosphere. After the reaction was completed as monitored by LCMS, the solution was filtered, and the filtrate was dried under reduced pressure to give compound 39-2 (1.53 g, crude product).

Step 3: Lithium hydroxide (250.0 mg, 10.42 mmol) was added to a solution of compound 39-2 (300 mg, 0.1742 mmol) in tetrahydrofuran/water (6.0 mL/1.0 mL), and the mixture was stirred at 60 °C for 12 h. After the reaction was completed as monitored by LC-MS, the reaction mixture was concentrated by rotary evaporation under reduced pressure to remove the solvent to give compound 39-3 (0.45 g, crude product), which was directly used in the next step. ES-API: [M-18+H]⁺ = 127.1.

Step 4: Compound 39-3 (17.3 mg, 0.120 mmol), O-(7-azabenzotriazol-1-yl)-N,N,N',N'-tetramethyluronium hexafluorophosphate (57.04 mg, 0.150 mmol), and N,N-diisopropyldiamine (0.030 mL, 0.180 mmol) were added to a solution of compound 15-20 (23.79 mg, 0.03 mmol) in N,N-dimethylformamide (2 mL), and the reaction mixture was stirred at room temperature for 1 h. After the reaction was completed as monitored by LCMS, the reaction mixture was purified by prep-HPLC (ammonium bicarbonate method 1) to give N-((4²Z,8³S,2E,6S)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2-(3-(hydroxymethyl)cyclobutyl)acetamide (Z39, 10.41 mg, yield: 37.74%). ES-API: [M+H]⁺ = 919.4.

### Example 12. Synthesis of compound Z41

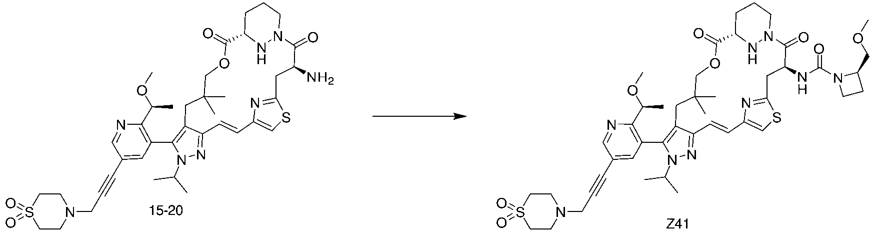

Step 1: Compound 15-20 (22 mg, 0.028 mmol) and DIPEA (17.93 mg, 0.139 mmol) were dissolved in dichloromethane (3 mL), and nitrophenyl chloroformate (8.39 mg, 0.042 mmol) was added at 0 °C. The reaction system was stirred under an ice bath for 0.5 h. Then (2R)-2-(methoxymethyl)-azetidine hydrochloride (7.64 mg, 0.055 mmol) was added, and the reaction system was stirred at 0 °C for 2 h. After the reaction was completed, the reaction mixture was concentrated to remove dichloromethane. Water (10 mL) was added to the residue, and the resulting mixture was extracted with ethyl acetate (15 mL × 2). The organic phase was washed with saturated brine (10 mL × 3), dried, and concentrated to give a crude product. The crude product was purified by prep-HPLC (ammonium bicarbonate method 1) to give *(2R)-N-*((4²*Z*,8³*S,*2*E*,6*S*)-1⁵-(5-(3-(1,1-dioxidothiomorpholino)prop-1-yn-1-yl)-2-((*S*)-1-methoxyethyl)pyridin-3-yl)-1¹-isopropyl-12,12-dimethyl-7,9-dioxo-8¹,8²,8³,8⁴,8⁵,,8⁶-hexahydro-1¹H-10-oxa-4(4,2)-thiazola-8(1,3)-pyridazina-1(3,4)-pyrrolacyclotridecaphan-2-en-6-yl)-2-(methoxymethyl)azetidine-1-carboxamide (Z41, 5 mg, 0.005 mmol, yield: 17.99%) as a white solid. ES-API: [M+H]⁺ = 920.3.

### Example 13. Synthesis of Compounds Z43-1 and Z43-2

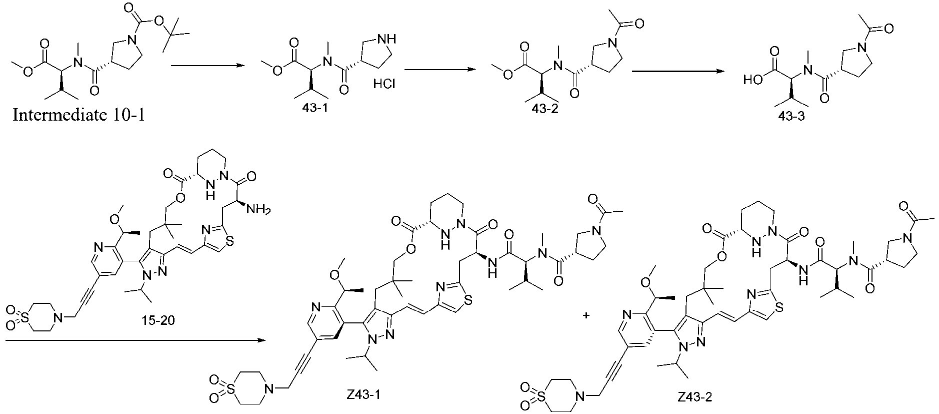

Step 1: Compound intermediate 10-1 (500 mg, 1.460 mmol) was dissolved in methanol (5 mL), and a 4.0 M hydrogen chloride/dioxane solution (10 mL, 40.000 mmol) was added at 0 °C. The reaction system was stirred at room temperature for 2 h. The reaction mixture was concentrated under reduced pressure to give compound 43-1 (407 mg, yield: 100%), hydrochloride, as a white solid. ES-API: [M+H]⁺ = 243.2. (Free base) Step 2: Compound 43-1 (407 mg, 1.460 mmol) and N,N'-diisopropylethylamine (566 mg, 4.380 mmol) were dissolved in dichloromethane (15 mL), and acetic anhydride (224 mg, 2.190 mmol) was added at 0 °C. The reaction system was stirred at room temperature for 2 h. Dichloromethane (25 mL) was added to the reaction mixture, and the resulting mixture was washed sequentially with 1 M diluted hydrochloric acid (20 mL), a saturated sodium bicarbonate solution (25 mL), and saturated brine (25 mL). The organic phase was dried over anhydrous sodium sulfate, filtered, and concentrated. The crude product was purified by flash silica gel column chromatography (methanol/dichloromethane: 0-10%) to give compound 43-2 (300 mg, yield: 72.3%) as a white solid. ES-API: [M+H]⁺ = 285.2.

Step 3: Compound 43-2 (300 mg, 1.055 mmol) was dissolved in tetrahydrofuran (10 mL) and water (5 mL), and lithium hydroxide monohydrate (88 mg, 2.110 mmol) was added at 0 °C. The reaction system was stirred at room temperature for 2 h. The reaction mixture was adjusted to pH 6 with 1 M diluted hydrochloric acid and extracted with dichloromethane (40 mL × 3). The organic phase was dried over anhydrous sodium sulfate and concentrated to give compound 43-3 (220 mg, yield: 77.1%) as a colorless liquid. ES-API: [M+H]⁺ = 271.1.

Step 4: Compound 15-20 (20 mg, 0.025 mmol) was dissolved in dichloromethane (3 mL), and compound 43-3 (14 mg, 0.050 mmol), O-(7-azabenzotriazol-1-yl)-N,N,N',N'-tetramethyluronium hexafluorophosphate (14 mg, 0.038 mmol), and N,N'-diisopropylethylamine (16 mg, 0.126 mmol) were added. The reaction system was stirred at room temperature for 2 h. Water (10 mL) was added to the reaction mixture, and the resulting mixture was extracted with dichloromethane (20 mL). The organic phase was washed with saturated brine (10 mL), dried over anhydrous sodium sulfate, and filtered, and the filtrate was concentrated under reduced pressure to give a crude product. The crude product was purified by prep-HPLC (ammonium bicarbonate method 1) to give 2 isomeric compounds. The structure of one of the isomeric compounds was arbitrarily designated as (3S)-1-acetyl-N-((2S)-1-(((4²Z,8³S,2E,6S)-(Rₐ)-1⁵-(5-(3-(1,1-dioxothiomorpholinyl)prop-1-yn-1-yl)-2-((S)-1-methoxyethyl)pyridin-3-yl)-1¹-isopropyl-12,12-dimethyl-7,9-dioxo-8¹,8²,8⁴,8⁵,8⁶-hexahydro-1¹H-10-oxa-4(4,2)-thiazola-8(1,3)-pyridazina-1(3,4)-pyrrolacyclotridecaphan-2-en-6-yl)amino)-3-methyl-1-oxobutan-2-yl)-N-methylpyrrolidine-3-carboxamide (Z43-1, 6 mg, retention time: 6.51 min, yield: 22.8%) as a white solid. ES-API: [M+H]⁺ = 1045.3.

The structure of the other isomeric compound was arbitrarily designated as (3S)-1-acetyl-N-((2S)-1-(((4²Z,8³S,2E,6S)-(Sₐ)-1⁵-(5-(3-(1,1-dioxothiomorpholinyl)prop-1-yn-1-yl)-2-((S)-1-methoxyethyl)pyridin-3-yl)-1¹-isopropyl-12,12-dimethyl-7,9-dioxo-8¹,8²,8⁴,8⁵,3⁶-hexahydro-1¹H-10-oxa-4(4,2)-thiazola-8(1,3)-pyridazina-1(3,4)-pyrrolacyclotridecaphan-2-en-6-yl)amino)-3-methyl-1-oxobutan-2-yl)-N-methylpyrrolidine-3-carboxamide (Z43-2, 3 mg, retention time: 6.73 min, yield: 11.5%) as a white solid. ES-API: [M+H]⁺ = 1045.3.

### Example 14. Synthesis of Compounds Z44-1 and Z44-2

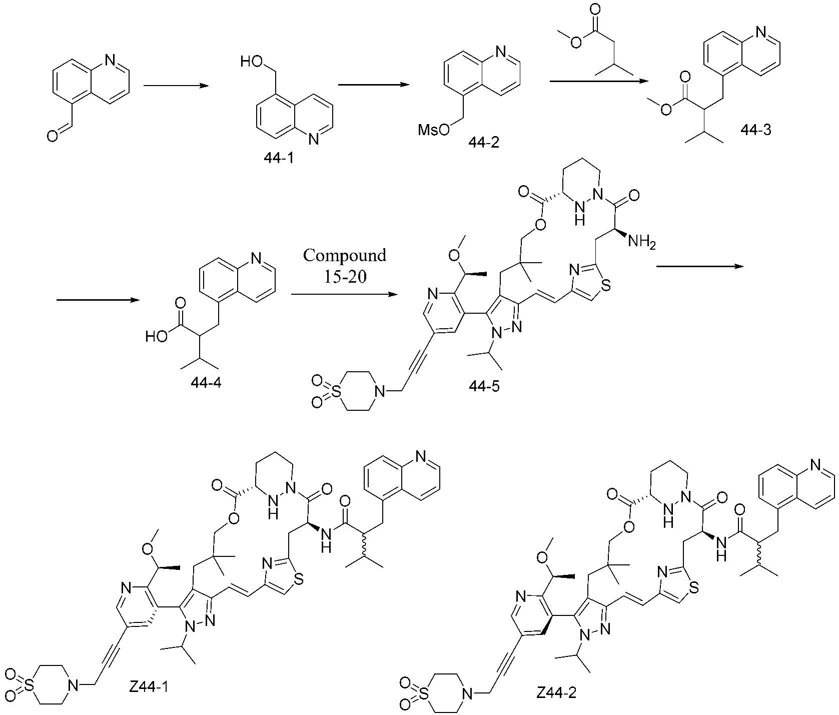

Step 1: Sodium borohydride (0.66 g, 17.561 mmol) was added to a solution of quinoline-5-carbaldehyde (2.30 g, 14.63 mmol) in methanol (50.0 mL) under an ice-water bath at 0-5 °C, and the mixture was stirred for 2 h. After the reaction was completed, the reaction mixture was poured into ethyl acetate (300 mL) and washed with saturated brine (200 mL). The organic phase was dried over anhydrous sodium sulfate and concentrated under reduced pressure to give compound 44-1 (2.0 g, yield: 85.85%). ES-API: [M+H]⁺= 160.1. Step 2: Methanesulfonyl chloride (1.51 g, 13.19 mmol) and triethylamine (2.67 g, 26.38 mmol) were added dropwise to a solution of compound 44-1 (1.40 g, 8.795 mmol) in dichloromethane (30.0 mL) under stirring, and the mixture was stirred at room temperature for 16 h. After the reaction was completed, the reaction mixture was poured into water (50.0 mL). The two phases were separated, and the aqueous phase was extracted with dichloromethane (15 mL × 2). The collected organic layer was washed with saturated brine (200 mL), dried over anhydrous sodium sulfate, and concentrated under reduced pressure. The resulting crude product was purified by flash silica gel column chromatography (99:1-95:5, dichloromethane/methanol, v/v) to give compound 44-2 (600 mg, yield: 28.75%). ES-API: [M-OMs +MeCN+H]⁺ = 178.3.

Step 3: Lithium diisopropylamide (0.95 mL, 1.897 mmol) was added dropwise to a solution of methyl 3-methylbutanoate (0.22 g, 1.897 mmol) in tetrahydrofuran (5.0 mL) at -70 °C, and the mixture was stirred at -70 °C for 1 h. A solution of compound 44-2 (0.30 g, 1.264 mmol) in tetrahydrofuran (3 mL) was then added dropwise. The solution was further stirred at -70 °C for 1 h. After the reaction was completed, the reaction was quenched with saturated sodium bicarbonate solution (30 mL). Saturated ammonium chloride (25 mL) and water (10 mL) were added, and the mixture was extracted with ethyl acetate (100 mL). The organic layer was washed with saturated brine (50 mL), dried over anhydrous sodium sulfate, filtered, and concentrated. The crude product was purified by flash silica gel column chromatography (0-20% petroleum ether/ethyl acetate) to give compound 44-3 (70.0 mg, 0.272 mmol, yield: 21.51%) as a light yellow oil. ES-API: [M+H]⁺ = 258.2.

Step 4: Sodium hydroxide (360.0 mg, 9.0 mmol) was added to a solution of compound 44-3 (70.0 mg, 0.291 mmol) in water (10.0 mL) at 0 °C, and the mixture was stirred at 60 °C for 12 h. The solution was adjusted to pH 5-6 under an ice-water bath and extracted with dichloromethane (30 mL), and the dichloromethane phase was evaporated under reduced pressure to give compound 44-4 (40.0 mg, yield: 56.41%). ES-API: [M+H]⁺ = 244.2.

Step 5: Compound 44-4 (10.71 mg, 0.044 mmol), O-(7-azabenzotriazol-1-yl)-N,N,N',N'-tetramethyluronium hexafluorophosphate (15.0 mg, 0.040 mmol), and N,N-diisopropyldiamine (0.030 mL, 0.180 mmol) were added to a solution of compound 15-20 (17.45 mg, 0.022 mmol) in N,N-dimethylformamide (2 mL), and the reaction mixture was stirred at room temperature for 1 h. After the reaction was completed as monitored by LCMS, the reaction mixture was purified by prep-HPLC (formic acid method 2) to give 2 isomeric compounds. The structure of one of the isomeric compounds was arbitrarily designated as N-((4²Z,8³S,2E,6S)-1⁵-(5-(3-(1,1-dioxidothiomorpholino)prop-1-yn-1-yl)-2-((S)-1-methoxyethyl)pyridin-3-yl)-1¹-isopropyl-(Rₐ)-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methyl-2-(quinolin-5-ylmethyl)butanamide formate (Z44-1, 4.14 mg, yield: 18.48%). ES-API: [M+H]⁺ = 1018.3.

The structure of the other isomeric compound was arbitrarily designated as (2R)-N-((4²Z,8³S,2E,6S)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-3-methyl-2-(quinolin-5-ylmethyl)butanamide formate (Z44-2, 3.21 mg, yield: 14.33%). ES-API: [M+H]⁺ = 1018.3.

### Example 15. Synthesis of Compound Z45

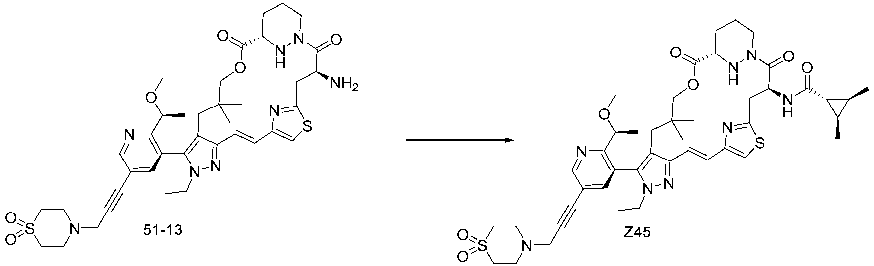

Step 1: Compound 51-13 (26 mg, 0.033 mmol) was dissolved in dichloromethane (1.0 mL), and (1R,2R,3S)-2,3-dimethylcyclopropane-1-carboxylic acid (7.6 mg, 0.067 mmol), O-(7-azabenzotriazol-1-yl)-N,N,N',N'-tetramethyluronium hexafluorophosphate (24.41 mg, 0.064 mmol), and N,N'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with dichloromethane (8.0 mL × 2). The organic phase was washed with saturated brine (5.0 mL × 3), dried, and concentrated, and the crude product was purified by prep-HPLC (ammonium bicarbonate method 2) to give (1r,2R,3S)-N-((4²Z,8³S,2E,6S)-*(Rₐ)-*1⁵-(5-(3-(1,1-dioxothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵8⁶-hexahydro-1'H-10-oxo-4(4,2)-thiazola-8(1,3)-pyridazina-1(3,4)-pyrazolocyclotridec-2-en-6-yl)-2,3-dimethylcyclopropane-1-carboxamide (Z45, 8 mg, 0.009 mmol, yield: 27%). ES-API: [M+H]⁺ = 875.3. ¹H NMR (400 MHz, DMSO-d₆) δ 8.80 (d, J = 4.0 Hz, 1H), 8.36 (d, J = 12.0 Hz, 1H), 7.82 (s, 1H), 7.44 (s, 1H), 7.37 (d, J = 12.0 Hz, 1H), 7.26 (d, J = 16.0 Hz, 1H), 5.54 (t, J = 8.0 Hz, 1H), 5.04 (d, J = 12.0 Hz, 1H), 4.30-4.21 (m, 3H), 4.07-3.94 (m, 3H), 3.75 (s, 2H), 3.63-3.55 (m, 3H), 3.22 (s, 3H), 3.19-3.12 (m, 4H), 3.07-2.99 (m, 4H), 2.76-2.70 (m, 1H), 2.66-2.60 (m, 1H), 2.19 (d, J = 12.0 Hz, 1H), 1.98 (d, J = 12.0 Hz, 1H), 1.80-1.72 (m, 1H), 1.68-1.60(m, 1H), 1.54-1.43 (m, 1H), 1.36 (d, J = 4.0 Hz, 3H), 1.20-1.12 (m, 6H), 1.11-0.98 (m, 6H), 0.78 (s, 3H), 0.33 (s, 3H).

### Example 16. Synthesis of Compound Z46

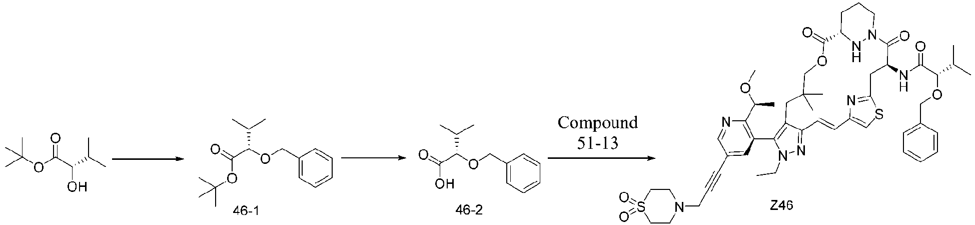

Step 1: tert-Butyl (S)-2-hydroxy-3-methylbutyrate (400 mg, 2.296 mmol) was dissolved in dry tetrahydrofuran (2.0 mL), and sodium hydride (275.48 mg, 6.887 mmol, purity: 60%) was slowly added under an ice bath. The mixture was stirred for 10 min under an ice bath. Then a solution of benzyl bromide (0.6 mL, 4.592 mmol) in tetrahydrofuran (2.0 mL) was added dropwise to the above mixture, and the resulting mixture was stirred at room temperature for 2 h. After the reaction was completed, water (4.0 mL) was added dropwise under an ice bath to quench the reaction, and the reaction mixture was extracted with ethyl acetate (8.0 mL × 2). The organic phase was washed with saturated brine (5.0 mL × 3), dried, and concentrated, and the residue was purified by flash silica gel column chromatography (tetrahydrofuran/petroleum ether: 0-30%) to give compound 46-1 (289 mg, 1.093 mmol, yield: 47.62%). ES-API: [M+23]⁺ = 287.2.

Step 2: Compound 46-1 (243 mg, 0.919 mmol) was dissolved in dichloromethane (10.0 mL). Trifluoroacetic acid (2.0 mL) was slowly added to the above solution under an ice bath, and the reaction system was stirred at room temperature for 3 h. After the reaction was completed, the reaction mixture was concentrated, and the residue was repeatedly concentrated with dichloromethane to remove excess trifluoroacetic acid to give compound 46-2 (175 mg, 0.84 mmol, yield: 91.42%). ES-API: [M+23]⁺ = 231.1.

Step 3: Compound 46-2 (15.51 mg, 0.074 mmol) was dissolved in dichloromethane (1.0 mL), and compound 51-13 (29.0 mg, 0.030 mmol), O-(7-azabenzotriazol-1-yl)-N,N,N',N'-tetramethyluronium hexafluorophosphate (28.31 mg, 0.074 mmol), and N,N'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated, and the residue was purified by prep-HPLC (ammonium bicarbonate method 1) to give (2S)-2-(benzyloxy)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z46, 10.63 mg, 0.011 mmol, yield: 26.57%). ES-API: [M+H]⁺ = 969.3. ¹H NMR (400 MHz, DMSO-d₆) *δ* 8.80 (d, *J=* 2.0 Hz, 1H), 8.27 (d, *J=* 9.2 Hz, 1H), 7.81 (d, *J=* 2.0 Hz, 1H), 7.47 (s, 1H), 7.44 - 7.32 (m, 6H), 7.27 (d, *J=* 15.6 Hz, 1H), 5.54 (t, *J= 8.8* Hz, 1H), 5.12 (d, *J=* 12.0 Hz, 1H), 4.69 (d, *J=* 11.6 Hz, 1H), 4.34 *(d, J= 11.6* Hz, 1H), 4.29 - 4.20 (m, 2H), 4.17 - 4.08 (m, 1H), 4.07 - 3.95 (m, 2H), 3.75 (s, 2H), 3.63 - 3.53 (m, 2H), 3.50 (d, *J=* 6.4 Hz, 1H), 3.45 - 3.37 (m, 1H), 3.30 - 3.20 (m, 4H), 3.18 - 3.15 (m, 4H), 3.07 - 3.00 (m, 4H), 2.82 - 2.72 (m, 1H), 2.70 - 2.62 (m, 1H), 2.17 - 2.10 (m, 1H), 2.06 - 1.97 (m, 2H), 1.90 - 1.70 (m, 2H), 1.55 - 1.45 (m, 1H), 1.36 (d, *J =* 6.0 Hz, 3H), 1.15 (t, *J =* 7.2 Hz, 3H), 0.98 (d, *J =* 6.8 Hz, 3H), 0.91 (d, *J* = 6.8 Hz, 3H), 0.84 (s, 3H), 0.35 - 0.26 (m, 3H).

### Example 17. Synthesis of Compounds Z47-1 and Z47-2

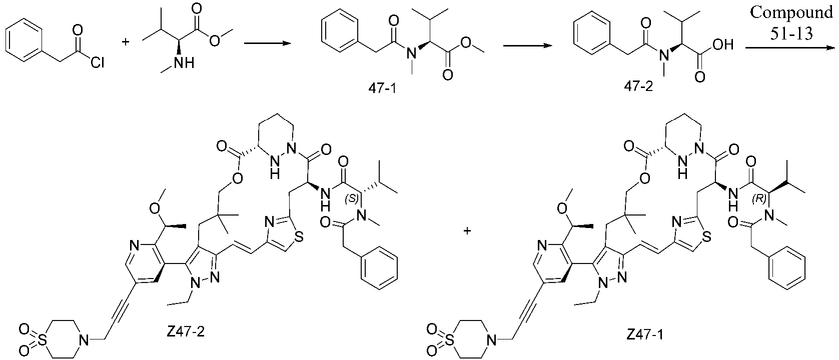

Step 1: Methyl N-methyl-L-valinate (676.27 mg, 4.657 mmol) was dissolved in dichloromethane (5 mL), and N,N'-diisopropylethylamine (1504.95 mg, 11.644 mmol) and 2-phenylacetyl chloride (600 mg, 3.881 mmol) were added. The mixture was reacted at room temperature for 30 min. After the reaction was completed, water (10.0 mL) was added to the reaction system, and the resulting mixture was extracted with dichloromethane (20.0 mL × 2). The organic phase was washed with saturated brine (5.0 mL × 3), dried, and concentrated, and the crude product was purified by flash silica gel column chromatography (ethyl acetate/petroleum ether: 0-20%) to give compound 47-1 (0.82 g, 3.105 mmol, yield: 80%). ES-API: [M+H]⁺ = 264.1.

Step 2: Compound 47-1 (0.2 g, 0.759 mmol) was dissolved in tetrahydrofuran (5 mL) and water (1 mL), and lithium hydroxide (0.16 g, 3.797 mmol) was added. The mixture was reacted at room temperature for 3 h. Hydrochloric acid (1 M) was slowly added to adjust the pH of the reaction system to 3 under an ice-water bath. Water (10.0 mL) was added to the reaction system, and the resulting mixture was extracted with dichloromethane (20.0 mL × 2). The organic phase was washed with saturated brine (5.0 mL × 3), dried, and concentrated to give compound 47-2 (189 mg, crude product). ES-API: [M+H]⁺ = 250.1.

Step 3: Compound 51-13 (26 mg, 0.033 mmol) was dissolved in dichloromethane (1.0 mL), and compound 47-2 (24.00 mg, 0.096 mmol), O-(7-azabenzotriazol-1-yl)-*N*,*N*,*N*',*N*'-tetramethyluronium hexafluorophosphate (24.41 mg, 0.064 mmol), and *N*,*N*'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with dichloromethane (8.0 mL × 2). The organic phase was washed with saturated brine (5.0 mL × 3), dried, and concentrated, and the crude product was purified by prep-HPLC (ammonium bicarbonate method 1) to give 2 isomeric compounds. The structure of one of the isomeric compounds was arbitrarily designated as (2S)-N-((4²Z,8³S,2E,6S)-(R*ₐ*)-1⁵-(5-(3-(1,1-dioxothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxo-4(4,2)-thiazola-8(1,3)-pyridazina-1(3,4)-pyrrolacyclotridecaphan-2-en-6-yl)-3-methyl-2-(N-methyl-2-phenylacetamido)butanamide (Z47-1, 2.5 mg, yield: 7.5%, retention time: 7.02 min).

The structure of the other isomeric compound was arbitrarily designated as (2R)-N-((4²Z,8³S,2E,6S)-*(*R*ₐ)-*1⁵-(5-(3-(1,1-dioxothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxo-4(4,2)-thiazola-8(1,3)-pyridazina-1(3,4)-pyrrolacyclotridecaphan-2-en-6-yl)-3-methyl-2-(N-methyl-2-phenylacetamido)butanamide (Z47-2, 3.2 mg, yield: 9.6%, retention time: 7.10 min). ES-API: [M+H]⁺ = 1010.2.

### Example 18. Synthesis of Compound Z48

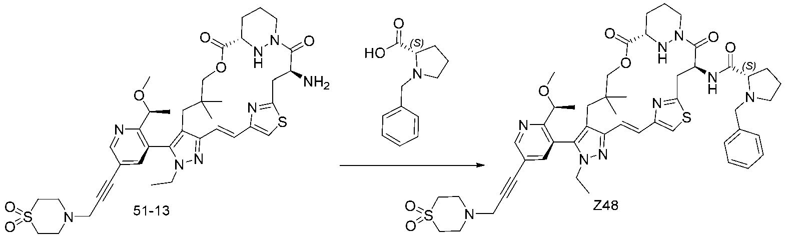

Step 1: Compound 51-13 (26 mg, 0.033 mmol) was dissolved in dichloromethane (1.0 mL), and (1R,2R,3S)-2,3-dimethylcyclopropane-1-carboxylic acid (7.6 mg, 0.067 mmol), O-(7-azabenzotriazol-1-yl)-*N,N,N',N*'-tetramethyluronium hexafluorophosphate (24.41 mg, 0.064 mmol), and *N*,*N*'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with dichloromethane (8.0 mL × 2). The organic phase was washed with saturated brine (5.0 mL × 3), dried, and concentrated, and the crude product was purified by prep-HPLC (ammonium bicarbonate method 2) to give (2S)-1-benzyl-N-((4²Z,8³S,2E,6S)-*(Rₐ)*-1⁵-(5-(3-(1,1-dioxothiomorpholine)-1-prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)pyrrolidine-2-carboxamide (Z48, 10 mg, yield: 32.3%). ES-API: [M+H]⁺ = 966.2. ¹H NMR (400 MHz, DMSO-d₆) δ 8.80 (d, J = 4.0 Hz, 1H), 8.25 (d, J = 12.0 Hz, 1H), 7.82 (d, J = 4.0 Hz, 1H), 7.50 (d, J = 4.0 Hz, 2H), 7.44 (s, 1H), 7.35 (m, 3H), 7.27 (m, 2H), 5.60-5.52 (m, 1H), 5.14 (d, J = 12.0 Hz, 1H), 4.32-4.20 (m, 2H), 4.16-4.08 (m, 1H), 4.03-3.98 (m, 2H), 3.92 (d, J = 12.0 Hz, 1H), 3.75 (s, 2H), 3.65-3.57 (m, 2H), 3.34-3.30 (m, 1H), 3.25 (s, 3H), 3.20-3.14 (m, 4H), 3.08-2.98 (m, 5H), 2.90-2.80 (m, 1H), 2.77-2.70 (m, 1H), 2.67-2.60 (m, 1H), 2.27 (d, J = 8.0 Hz, 1H), 2.20-2.10 (m, 2H), 2.03 (d, J = 12.0 Hz, 1H), 1.90-1.70 (m, 6H), 1.55-1.45 (m, 1H), 1.37 (d, J = 4.0 Hz, 3H), 1.23 (s, 1H), 1.16 (t, J = 8.0 Hz, 3H), 0.84 (s, 3H), 0.31 (s, 3H).

### Example 19. Synthesis of Compound Z51

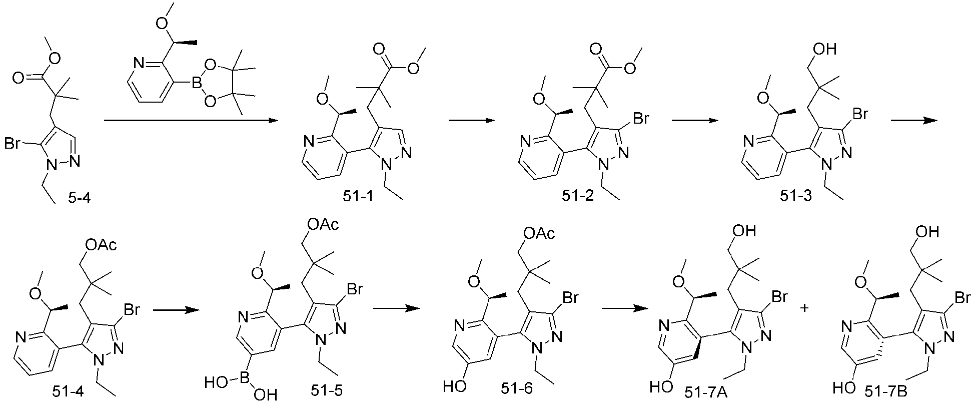

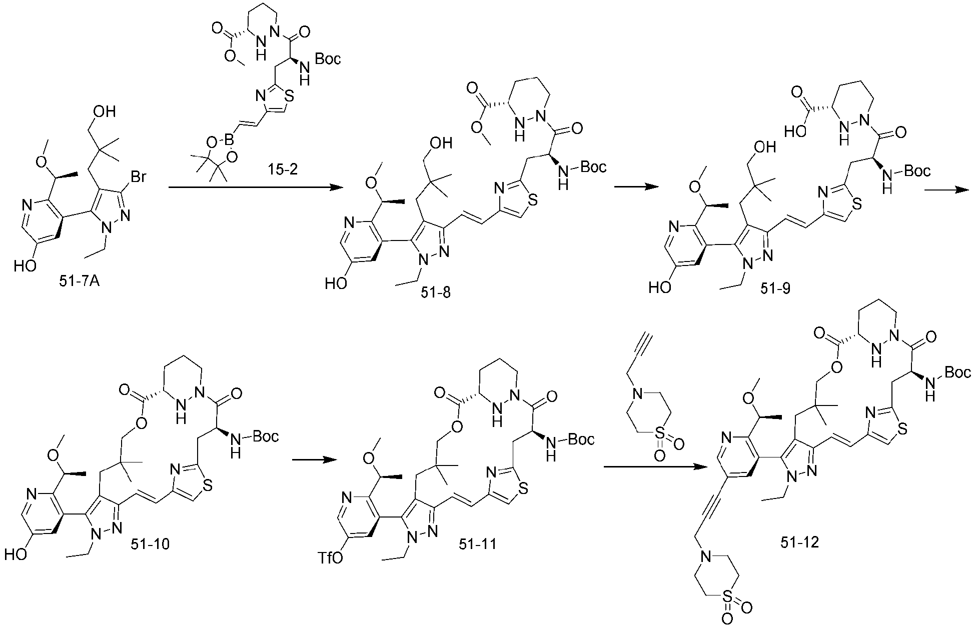

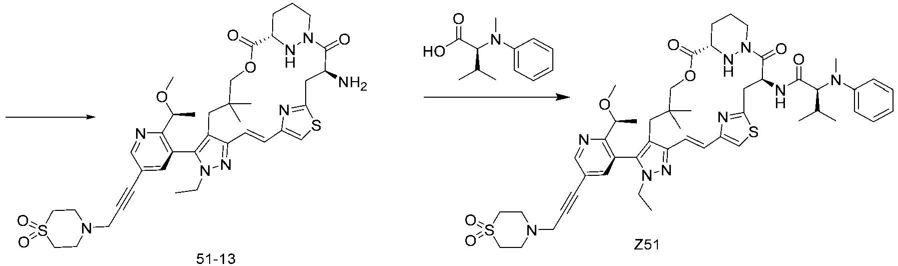

Step 1: Compound 5-4 (7500 mg, 25.936 mmol) was dissolved in toluene (90 mL), dioxane (30 mL), and water (30 mL). Potassium phosphate (16515.72 mg, 77.809 mmol) and methanesulfonyloxy(diadamantyl-n-butylphosphino)-2'-amino-1,1'-biphenyl-2-yl)palladium(II) (1888.86 mg, 2.594 mmol) were added to the above solution. After the addition was completed, the reaction system was stirred at 110 °C for 5 h in a microwave reactor. Water (100 mL) was added to the reaction mixture, and the resulting mixture was extracted with water (100 mL × 2). The separated organic phase was washed with saturated brine (100 × 2 mL), dried over anhydrous sodium sulfate, and filtered, and the filtrate was concentrated under reduced pressure. The crude product was purified by flash silica gel column chromatography (ethyl acetate/petroleum ether: 0-60%) to give compound 51-1 (8700 mg, 25.185 mmol, yield: 97.10%) as a light brown liquid. ES-API: [M+H]⁺ = 346.1.

Step 2: Compound 51-1 (9000 mg, 26.054 mmol) was dissolved in N,N-dimethylformamide, and bromosuccinimide (6955.95 mg, 39.081 mmol) was added to the above solution. After the addition was completed, the reaction system was stirred at 80°C for 1 h. After the reaction was completed, water (300 mL) was added to the reaction mixture, and the resulting mixture was extracted with ethyl acetate (300 mL). The organic phase was washed with saturated brine (400 mL × 3), dried over anhydrous sodium sulfate, and filtered, and the filtrate was concentrated under reduced pressure. The crude product was purified by flash silica gel column chromatography (ethyl acetate/petroleum ether: 0-40%) to give compound 51-2 (8200 mg, 19.233 mmol, yield: 73.82%). ES-API: [M+H]⁺ = 424.1, 426.1.

Step 3: Compound 51-2 (8000 mg, 18.853 mmol) was dissolved in dichloromethane (200 mL), and the solution was cooled to 0 °C. Diisobutylaluminum hydride (80 mL, 80.000 mmol, 1 M n-hexane solution) was slowly added to the above solution under nitrogen atmosphere. After the addition was completed, the reaction mixture was warmed to room temperature and stirred for 1 h. After the reaction was completed, the reaction mixture was diluted with an appropriate amount of tetrahydrofuran (200 mL) and cooled to 0 °C. Water (3.2 mL) was slowly added, and a 15% aqueous sodium hydroxide solution (3.2 mL) was added, followed by the addition of water (8 mL). The mixture was warmed to room temperature and stirred for 15 min, and anhydrous sodium sulfate (50 g) was added. The mixture was stirred for 15 min and then filtered to remove salts. The filter cake was washed with ethyl acetate (1000 mL), concentrated, and dried to give a crude product. The crude product was purified by flash silica gel column chromatography (methanol/dichloromethane = 0-3%) to give compound 51-3 (5500 mg, 13.877 mmol, yield: 73.61%) as a white solid. ES-API: [M+H]⁺ = 396.1, 398.1.

Step 4: Compound 51-3 (5500 mg, 13.403 mmol), N,N-diisopropylethylamine (5370.52 mg, 41.632 mmol), and 4-dimethylaminopyridine (163.74 mg, 1.340 mmol) were dissolved in dichloromethane (100 mL). Acetic anhydride (1.955 mL, 20.816 mmol) was slowly added to the above solution at 0 °C. After the addition was completed, the reaction system was stirred at 0 °C for half an hour. After the reaction was completed, the reaction was quenched with a saturated aqueous sodium bicarbonate solution (100 mL), and the reaction mixture was extracted with dichloromethane (100 mL × 2). The organic phase was washed with saturated brine (100 mL × 2), dried over anhydrous sodium sulfate, and filtered, and the filtrate was concentrated under reduced pressure. The crude product was purified by flash silica gel column chromatography (ethyl acetate/petroleum ether: 0-60%) to give compound 51-4 (5200 mg, 11.495 mmol, yield: 85.76%) as a light yellow solid. ES-API: [M+H]⁺ = 438.1, 440.1.

Step 5: Compound 51-4 (4200 mg, 9.581 mmol), bis(pinacolato)diboron (4136.08 mg, 16.288 mmol), chloro(1,5-cyclooctadiene)iridium(I) dimer (321.78 mg, 0.479 mmol), and 4,4'-di-tert-butyl-2,2'-bipyridine (257.15 mg, 0.958 mmol) were dissolved in cyclohexane (100 mL). The reaction system was stirred at 80 °C for 17 h under nitrogen atmosphere. The reaction mixture was concentrated to give compound 51-5 (4620 mg, crude product) as a black oily liquid. ES-API: [M+H]⁺ = 482.1, 484.1.

Step 6: Compound 51-5 (4620 mg, crude product) was dissolved in acetonitrile (100 mL), and 30% hydrogen peroxide (3.257 mL, 28.738 mmol) was added under an ice bath. The reaction system was warmed to room temperature and stirred for 2 h. After the reaction was completed, the reaction was quenched with saturated sodium sulfite (100 mL). The reaction mixture was extracted with ethyl acetate (100 mL). The organic phase was washed with saturated brine (100 mL), dried, and concentrated to give a crude product. The crude product was purified by flash silica gel column chromatography (tetrahydrofuran/petroleum ether: 0-50%) to give compound 51-6 (3800 mg, 8.363 mmol, two-step yield: 87.29%) as a yellow oily liquid. ES-API: [M+H]⁺ = 454.1, 456.1.

Step 7: Compound 51-6 (3500 mg, 7.703 mmol) was dissolved in methanol (10 mL), tetrahydrofuran (10 mL), and water (10 mL). Lithium hydroxide (1292.87 mg, 30.812 mmol) was added to the above solution. After the addition was completed, the reaction system was stirred at room temperature for 17 h. After the reaction was completed, the reaction mixture was acidified to weak acidity (about pH 6) with diluted hydrochloric acid (1 M). The resulting solution was extracted with ethyl acetate (50 mL × 3). The organic phase was washed with saturated brine (100 mL), dried, and concentrated to give a crude product. The crude product was purified by flash silica gel column chromatography (ethyl acetate/petroleum ether: 0-100%) to give 2 isomeric compounds. The structure of one of the isomeric compounds was (Rₐ)-5-(3-bromo-1-ethyl-4-(3-hydroxy-2,2-dimethylpropyl)-1H-pyrazol-5-yl)-6-((S)-1-methoxyethyl)pyridin-3-ol (51-7A, 1800 mg, 4.365 mmol, yield: 56.67%) (ethyl acetate, R_{f} = 0.5) as a light yellow solid. ES-API: [M+H]⁺ = 412.1, 414.1. ¹H NMR (400 MHz, MeOD) δ 8.29 (d, *J =* 2.8 Hz, 1H), 7.16 (d, *J =* 2.8 Hz, 1H), 4.13 - 4.06 (m, 1H), 3.98 - 3.83 (m, 2H), 3.19 (d, *J =* 10.8 Hz, 1H), 3.11 (d, *J =* 10.8 Hz, 1H), 3.06 (s, 3H), 2.43 (d, *J =* 14.4 Hz, 1H), 2.17 (d, *J=* 14.4 Hz, 1H), 1.49 (d, *J =* 6.4 Hz, 3H), 1.35 (t, *J =* 7.2 Hz, 3H), 0.78 (s, 3H), 0.65 (s, 3H).

The structure of the other isomeric compound was (Sₐ)-5-(3-bromo-1-ethyl-4-(3-hydroxy-2,2-dimethylpropyl)-1H-pyrazol-5-yl)-6-((S)-1-methoxyethyl)pyridin-3-ol (51-7B, 1000 mg, 2.425 mmol, yield: 31.48%) (ethyl acetate, R_{f} = 0.3) as a light yellow solid. ES-API: [M+H]⁺ = 412.1, 414.1. ¹H NMR (400 MHz, MeOD) δ 8.30 (d, *J =* 2.8 Hz, 1H), 7.17 (d, *J =* 2.8 Hz, 1H), 4.10 - 4.03 (m, 1H), 3.94 - 3.84 (m, 1H), 3.82 - 3.73 (m, 1H), 3.27 (s, 3H), 3.21 (d, *J =* 10.8 Hz, 1H), 3.11 (d, *J =* 10.8 Hz, 1H), 2.46 (d, *J =* 14.4 Hz, 1H), 2.32 (d, *J =* 14.4 Hz, 1H), 1.34 (t, *J =* 7.2 Hz, 3H), 1.27 (d, *J =* 6.4 Hz, 3H), 0.83 (s, 3H), 0.67 (s, 3H). Step 8: Compound 51-7A (1800 mg, 4.365 mmol) was dissolved in toluene (30 mL), dioxane (10 mL), and water (10 mL), and potassium phosphate (2316.52 mg, 10.914 mmol), [1,1'-bis(di-tert-butylphosphino)ferrocene]dichloropalladium(II) (426.28 mg, 0.655 mmol), and methyl (S)-1-((S)-2-((tert-butoxycarbonyl)amino)-3-(4-((E)-2-(4,4,5,5-tetramethyl-1,3,2-dioxaborolan-2-yl)vinyl)thiazol-2-yl)propanoyl)hexahydropyridazine-3-carboxylate (3604.63 mg, 6.548 mmol) were added. The reaction system was purged 3 times with nitrogen and stirred at 80 °C for 3 h. Ethyl acetate (100 mL) and water (50 mL) were added to the reaction mixture. The organic phase was separated, washed with saturated brine (60 mL), dried over anhydrous sodium sulfate, and filtered, and the filtrate was concentrated under reduced pressure. The crude product was purified by flash silica gel column chromatography (tetrahydrofuran/petroleum ether: 0-60%) to give compound 51-8 (2800 mg, 3.704 mmol, yield: 84.85%) as a white solid. ES-API: [M+H]⁺ = 756.3.

Step 9: Compound 51-8 (2800 mg, 3.704 mmol) was dissolved in tetrahydrofuran (20 mL) and water (20 mL), and lithium hydroxide monohydrate (248.67 mg, 5.926 mmol) was added at 0 °C. The reaction system was stirred at room temperature for 18 h. The reaction mixture was adjusted to pH 6 with 1 M diluted hydrochloric acid and extracted with ethyl acetate (30 mL × 2). The organic phase was dried over anhydrous sodium sulfate and concentrated to give compound 51-9 (2700 mg, crude product) as a light yellow solid. ES-API: [M+H]⁺ = 742.3.

Step 10: compound 51-9 (2700 mg, crude product) was dissolved in dichloromethane (100 mL), and 1-(3-dimethylaminopropyl)-3-ethylcarbodiimide hydrochloride (19534 mg, 101.90 mmol), 1-hydroxybenzotriazole (2459 mg, 18.20 mmol), and diisopropylethylamine (19 mL, 109 mmol) were added at room temperature. The reaction system was stirred at room temperature for 18 h. Dichloromethane (100 mL) was added to the reaction mixture, and the resulting mixture was washed with diluted hydrochloric acid (0.5 M, 20 mL × 2) and saturated brine (100 mL), dried over anhydrous sodium sulfate, and filtered. The filtrate was concentrated under reduced pressure. The crude product was purified by flash silica gel column chromatography (methanol/dichloromethane: 0-5%) to give compound 51-10 (1000 mg, 1.381 mmol, two-step yield: 37.3%) as a white solid. ES-API: [M+H]⁺ = 724.3.

Step 11: Compound 51-10 (700 mg, 0.967 mmol) was dissolved in dichloromethane (20 mL), and triethylamine (0.403 mL, 2.901 mmol) and 1,1,1-trifluoro-N-phenyl-N-[(trifluoromethyl)sulfonyl]methanesulfonamide (518.18 mg, 1.450 mmol) were added to the above solution. After the addition was completed, the reaction system was stirred at room temperature for 2 h. After the reaction was completed, the reaction mixture was concentrated to give a crude product. The crude product was purified by flash silica gel column chromatography (methanol/dichloromethane: 0%-3%) to give compound 51-11 (800 mg, 0.935 mmol, yield: 96.65%) as a white solid. ES-API: [M+H]⁺ = 856.3.

Step 12: Compound 51-11 (750 mg, 0.876 mmol) was dissolved in acetonitrile (15 mL). 4-(Prop-2-yn-1-yl)thiomorpholine 1,1-dioxide (455.36 mg, 2.629 mmol), bis(triphenylphosphine)palladium(II) dichloride (123.00 mg, 0.175 mmol), copper(I) iodide (66.75 mg, 0.350 mmol), N,N'-diisopropylethylamine (339.75 mg, 2.629 mmol), and lithium chloride (148.57 mg, 3.505 mmol) were added to the above solution at room temperature. After the addition was completed, the reaction system was stirred at 80°C for 1 h under argon atmosphere. After the reaction was completed, water (30 mL) was added to the reaction mixture, and the resulting mixture was extracted with ethyl acetate (30 mL × 3). The organic phase was washed with saturated brine (30 mL × 2), dried, and concentrated to give a crude product. The crude product was purified by flash silica gel column chromatography (methanol/dichloromethane: 0%-3%) to give compound 51-12 (700 mg, 0.794 mmol, yield: 90.69%) as a white solid. ES-API: [M+H]⁺ = 879.3.

Step 13: Compound 51-12 (33 mg, 0.038 mmol) was dissolved in methanol (0.5 mL). A 4 M solution of hydrogen chloride in dioxane (1.0 mL) was slowly added dropwise to the above solution under an ice bath, and the reaction system was stirred at room temperature for 3 h. After the reaction was completed, the reaction mixture was concentrated to give hydrochloride. A saturated aqueous sodium bicarbonate solution (5 mL) and ethyl acetate (10 mL × 2) were slowly added dropwise to the above hydrochloride for extraction. The organic phase was separated, washed with saturated brine (5 mL × 2), dried, and concentrated to give compound 51-13 (29 mg, 0.037 mmol, yield: 96.7%). ES-API: [M+H]⁺ = 779.2.

Step 14: Compound 51-13 (25 mg, 0.032 mmol) was dissolved in dichloromethane (4 mL). N-methyl-N-phenyl-L-valine (10 mg, 0.05 mmol; prepared by referring to the method in Journal of the American Chemical Society, 1998, vol. 120, # 48, p. 12459-12467), O-(7-azabenzotriazol-1-yl)-N,N,N',N'-tetramethyluronium hexafluorophosphate (16 mg, 0.042 mmol) and N,N'-diisopropylethylamine (16 mg, 0.124 mmol) were added to the above solution. After the addition was completed, the reaction system was stirred at room temperature for 24 h. After the reaction was completed, the reaction mixture was concentrated to remove dichloromethane. Water (10 mL) was added to the residue, and the resulting mixture was extracted with dichloromethane (15 mL × 2). The organic phase was washed with saturated brine (10 mL × 3), dried, and concentrated to give a crude product. The crude product was purified by prep-HPLC (trifluoroacetic acid method) to give *(2S)-N-*((4²*Z*,8³*S*,2*E*,6*S*)-(Rₐ)-1⁵-(5-(3-(1,1-dioxidothiomorpholine)prop-1-yn-1-yl)-2-((*S*)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolecyclotridec-2-en-6-yl)-3-methyl-2-(methyl(phenyl)amino)butanamide (Z51, 8 mg, 0.007 mmol, yield: 23.10%), trifluoroacetate, as a white solid. ES-API: [M+H]⁺ = 968.3.

### Single-crystal culture of intermediate 51-7A

### 5-(3-Bromo-1-ethyl-4-(3-hydroxy-2,2-dimethylpropyl)-1H-pyrazol-5-yl)-6-((S)-1-methoxyethyl)pyridin-3-ol

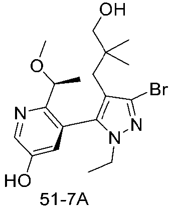

5 mg of intermediate 51-7A was dissolved in 1 mL of acetonitrile solution, and the solution was slowly volatilized at room temperature for 24 h to give a columnar crystal. The X-ray single-crystal diffraction test was performed using a Bruker D8 Venture instrument. The results are shown in Table 2 below and FIG. 2. FIG. 2 shows an ellipsoid plot of the three-dimensional molecular structure, which contains one molecule of water of crystallization.

### Structure analysis and refinement process:

The diffraction data were integrated and reduced using the SAINT program, followed by empirical absorption correction using the SADABS program. The single crystal structure was solved by the direct method using SHELXT2014 and refined by the least square method. For the refinement of hydrogen atoms, isotropic calculation was adopted: hydrogen atoms attached to N were located from residual electron density, while those on C-H bonds were added geometrically and refined using a riding model. The Flack constant was 0.069(16). C14 in FIG. 2 is in S configuration. The axial chirality at positions C4-C5 is in Rₐ configuration.

**Table 2**

| | |
|---|---|
| Molecular formula | C₁₈H₂₆BN₃O₃·H₂O |
| Molecular weight | *Mᵣ* = 430.34 |
| Shape | Block, colorless |
| Crystal system | Monoclinic |
| Space group | *P*2₁ |
| Unit cell parameter | *a* = 10.8029 (3) Å; *b* = 8.1107 (2) Å; *c* = 12.3214 (3) Å |
| Unit cell volume | *V* = 983.73 (4) Å³ |
| Molecule number in unit cell | Z = 2 |
| Unit cell dimension | 0.12 × 0.06 × 0.05 mm |
| Electron number in unit cell | *F*(000) = 448 |
| Calculating density | *Dₓ* = 1.453 Mg m⁻³ |
| Radiation source and wavelength | Cu *K*a radiation, l = 1.54178 Å |
| Determining the number of diffraction points of unit cell | Cell parameters from 7110 reflections |
| Range for data collection | θ= 5.5-63.6° |
| Absorption coefficient | µ= 3.08 mm⁻¹ |
| Temperature | *T* = 170 K |

### Example 20. Synthesis of Compounds Z52-1 and Z52-2

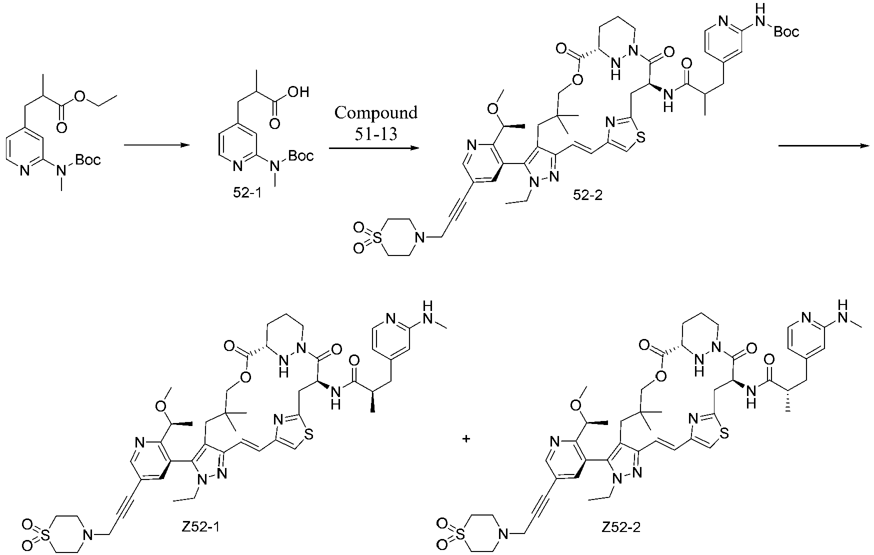

Step 1: Ethyl 3-(2-((tert-butoxycarbonyl)(methyl)amino)pyridin-4-yl)-2-methylpropionate (80 mg, 0.248 mmol) was dissolved in tetrahydrofuran (5 mL), methanol (1 mL), and water (1 mL), and lithium hydroxide (31.24 mg, 0.744 mmol) was added. The mixture was reacted at room temperature for 3 h. Hydrochloric acid (1 M) was slowly added to adjust the pH of the reaction system to 3 under an ice-water bath. Water (10.0 mL) was added to the reaction system, and the resulting mixture was extracted with dichloromethane (20.0 mL × 2). The organic phase was washed with saturated brine (5.0 mL × 3), dried, and concentrated to give compound 52-1 (73 mg, crude product). ES-API: [M+H]⁺ = 295.1.

Step 2: Compound 51-13 (26 mg, 0.033 mmol) was dissolved in dichloromethane (1.0 mL), and compound 52-1 (28.0 mg, 0.096 mmol), O-(7-azabenzotriazol-1-yl)-*N*,*N*,*N*',*N'*-tetramethyluronium hexafluorophosphate (26.9 mg, 0.064 mmol), and *N*,*N*'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with dichloromethane (8.0 mL × 2). The organic phase was washed with saturated brine (5.0 mL × 3), dried, and concentrated to give compound 52-2 (40.64 mg, crude product). ES-API: [M+H]⁺ = 1037.3.

Step 3: Compound 52-2 (40.64 mg, crude product) was dissolved in methanol (0.5 mL). A 4 M solution of hydrogen chloride in dioxane (1.0 mL) was slowly added dropwise to the above solution under an ice bath, and the reaction system was stirred at room temperature for 3 h. After the reaction was completed, the reaction mixture was concentrated to give hydrochloride. A saturated aqueous sodium bicarbonate solution (5 mL) and ethyl acetate (10 mL × 2) were slowly added dropwise to the above hydrochloride for extraction. The organic phase was separated and washed with saturated brine (5 mL × 2), and the crude product was purified by prep-HPLC (ammonium bicarbonate method 2) to give 2 isomeric compounds. The structure of one of the isomeric compounds was arbitrarily designated as (2R)-N-((4²Z,8³S,2E,6S)-*(Rₐ)*-1⁵-(5-(3-(1,1-dioxidothiomorpholine)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxo-4(4,2)-thiazola-8(1,3)-pyridazina-1(3,4)-pyrrolacyclotridecaphan-2-en-6-yl)-2-methyl-3-(2-(methylamino)pyridin-4-yl)propanamide) (Z52-1, 4 mg, yield: 11.05%, retention time: 6.85 min). ES-API: [M+H]⁺ = 956.2. ¹H NMR (400 MHz, DMSO-d₆) δ 8.80 (d, J = 4.0 Hz, 1H), 8.32 (d, J = 8.0 Hz, 1H), 7.87 (d, J = 4.0 Hz, 1H), 7.81 (d, J = 4.0 Hz, 1H), 7.36-7.31 (m, 2H), 7.26 (s, 1H), 6.37 (d, J = 4.0 Hz, 1H), 6.35-6.29 (m, 1H), 6.21 (s, 1H), 5.42 (t, J = 8.0 Hz, 1H), 5.05 (d, J = 12.0 Hz, 1H), 4.26 (q, J = 8.0 Hz, 1H), 4.22-4.15 (m, 1H), 4.10-3.98 (m, 3H), 3.75 (s, 2H), 3.60-3.50 (m, 2H), 3.27-3.22 (m, 4H), 3.16-3.05 (m, 6H), 3.00-2.95 (m, 6H), 2.85-2.76 (m, 4H), 2.72-2.68 (m, 4H), 2.40-2.30 (m, 2H), 2.13 (d, J = 12.0 Hz, 1H), 2.00 (d, J = 8.0 Hz, 1H), 1.80-1.71 (m, 3H), 1.55-1.47 (m, 1H), 1.36 (d, J = 8.0 Hz, 3H), 1.24-1.20 (m, 1H), 1.15 (t, J = 8.0 Hz, 3H), 1.00 (d, J = 8.0 Hz, 3H), 0.81 (s, 3H), 0.30 (s, 3H). The structure of the other isomeric compound was arbitrarily designated as (2S)-N-((4²Z,8³S,2E,6S)-*(*R*ₐ)*-1⁵-(5-(3-(1,1-dioxidothiomorpholine)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxo-4(4,2)-thiazola-8(1,3)-pyridazina-1 (3,4)-pyrazolocyclotridec-2-en-6-yl)-2-methyl-3-(2-(methylamino)pyridin-4-yl)propanamide)(Z52-2, 3 mg, yield: 8.29%, retention time: 6.92 min). ES-API: [M+H]⁺ = 955.2.

### Example 21. Synthesis of Compounds Z53 and Z55

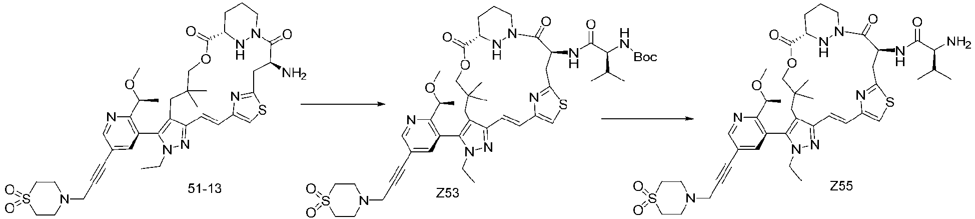

Step 1: Compound 51-13 (29 mg, 0.037 mmol) was dissolved in dichloromethane (1.0 mL), and (S)-2-((tert-butoxycarbonyl)amino)-3-methylbutanoic acid (8.04 mg, 0.037 mmol), O-(7-azabenzotriazol-1-yl)-N,N,N',N'-tetramethyluronium hexafluorophosphate (28.31 mg, 0.074 mmol), and *N,N'-*diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated to obtain half of a crude product (10 mg), which was directly used in the next step. The other half of the crude product was purified by prep-HPLC (ammonium bicarbonate method 1) to give tert-butyl (2S)-1-(((4²Z,8³S,2E,6S)-1⁵-(Rₐ)-(5-(3-(1,1-dioxidothiomorpholino)-1-propyn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)amino)-3-methyl-1-oxobutan-2-yl)carbamate (Z53, 5.42 mg, 0.006 mmol, yield: 14.98%). ES-API: [M+H]⁺ = 979.3.

Step 2: Compound Z53 (10 mg, 0.010 mmol) was dissolved in methanol (1.0 mL), and a 4 M solution of hydrogen chloride in dioxane (1.0 mL) was slowly added dropwise under an ice bath. The mixture was stirred at room temperature for 1.5 h. After the reaction was completed, the reaction mixture was concentrated by rotary evaporation, and the residue was purified by prep-HPLC (ammonium bicarbonate method 1) to give (2S)-2-amino-N-((4²Z,8³S,2E,6S)-1⁵-(Rₐ)-(5-(3-(1,1-dioxidothiomorpholino)-1-propyn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z55, 5.73 mg, 0.007 mmol, yield: 63.81%). ES-API: [M+H]⁺ = 878.2.

### Example 22. Synthesis of Compounds Z54-1 and Z54-2

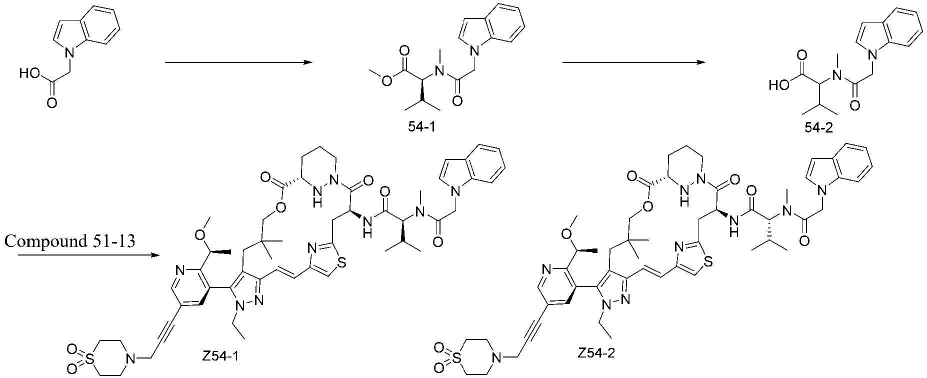

Step 1: 2-(1H-Indol-1-yl)acetic acid (900 mg, 5.137 mmol) was dissolved in N,N-dimethylformamide (10.0 mL), and N-methyl-L-valinate (1.4 g, 7.706 mmol), O-(7-azabenzotriazol-1-yl)-N,N,N',N'-tetramethyluronium hexafluorophosphate (2.34 g, 6.165 mmol), and N,N'-diisopropylethylamine (1.99 g, 15.412 mmol) were added. The system was stirred at room temperature for 2 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, concentrated, and purified by flash silica gel column chromatography (tetrahydrofuran/petroleum ether: 0-45%) to give compound 54-1 (1.17 g, 3.869 mmol, yield: 75.32%). ES-API: [M+H]⁺ = 303.3.

Step 2: Compound 54-1 (1.12 g, 3.704 mmol) was dissolved in methanol (4.0 mL) and water (1.0 mL). Anhydrous lithium hydroxide (0.78 g, 18.520 mmol) was slowly added to the above solution under an ice bath, and the reaction system was stirred at room temperature for 3 h. After the reaction was completed, 1 M hydrochloric acid was slowly added dropwise under an ice bath until the pH was about 6, and the resulting mixture was extracted with water (3 mL) and ethyl acetate (5 mL × 2). The organic phase was separated, washed with saturated brine (5 mL × 2), dried, and concentrated to give compound 54-2 (1.07 g, 3.201 mmol, yield: 86.26%). ES-API: [M+H]⁺ = 289.1.

Step 3: Compound 54-2 (8.65 mg, 0.30 mmol) was dissolved in dichloromethane (1.0 mL), and compound 51-13 (23.0 mg, 0.030 mmol), O-(7-azabenzotriazol-1-yl)-*N*,*N*,*N*',*N*'-tetramethyluronium hexafluorophosphate (22.45 mg, 0.059 mmol), and *N*,*N*'-diisopropylethylamine (11.45 mg, 0.089 mmol) were added. The reaction system was stirred at room temperature for 2 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated to give a crude product. The crude product was purified by prep-HPLC (ammonium bicarbonate method 1) to give 2 isomeric compounds. The structure of one of the isomeric compounds was arbitrarily designated as (2S)-2-(2-(1H-indol-1-yl)-N-methylacetamido)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z54-1, 3.98 mg, 0.004 mmol, yield: 12.64%, retention time: 7.734 min).

The structure of the other isomeric compound was arbitrarily designated as (2R)-2-(2-(1H-indol-1-yl)-N-methylacetamido)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z54-2, 6.20 mg, 0.006 mmol, yield: 19.70%, retention time: 7.897 min). ES-API: [M+H]⁺ = 1049.3. ¹H NMR (400 MHz, DMSO-d₆) δ 8.80 (s, 1H), 7.82 (d, *J =* 1.6 Hz, 1H), 7.57 - 7.44 (m, 1H), 7.40 - 7.25 (m, 2H), 7.10 (t, *J =* 7.2 Hz, 1H), 7.01 (t, *J =* 7.2 Hz, 1H), 6.46 - 6.40 (m, 1H), 5.65 - 5.55 (m, 1H), 5.30 - 5.09 (m, 3H), 4.32 - 4.20 (m, 2H), 4.10 - 3.98 (m, 3H), 3.75 (s, 2H), 3.65 - 3.52 (m, 2H), 3.26 - 3.21 (m, 4H), 3.20 - 3.13 (m, 4H), 3.09 (s, 2H), 3.06 - 3.00 (m, 4H), 2.87 - 2.77 (m, 2H), 2.70 - 2.55 (m, 1H), 2.35 -2.26 (m, 1H), 2.24 - 2.07 (m, 2H), 2.05 - 1.95 (m, 1H), 1.85 - 1.70 (m, 2H), 1.55 - 1.43 (m, 1H), 1.37 (d, *J =* 6.0 Hz, 3H), 1.30 - 1.21 (m, 1H), 1.16 (t, *J =* 7.2 Hz, 3H), 1.07 (d, *J =* 6.4 Hz, 1H), 0.99 (d, *J =* 6.4 Hz, 1H), 0.92 (s, 1H), 0.86 - 0.76 (m, 5H), 0.36 -0.27 (m, 3H).

### Example 23. Synthesis of Compound Z56

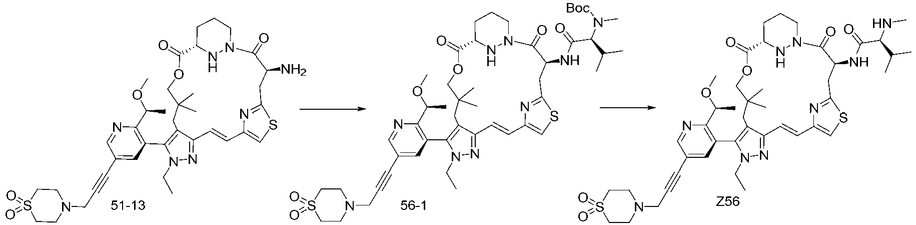

Step 1: Compound 51-13 (29 mg, 0.037 mmol) was dissolved in dichloromethane (1.0 mL), and N-(tert-butoxycarbonyl)-N-methyl-L-valine (17.22 mg, 0.074 mmol), O-(7-azabenzotriazol-1-yl)-N,N,N',N'-tetramethyluronium hexafluorophosphate (28.31 mg, 0.074 mmol), and N,N'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated to give compound 56-1 (32 mg, 0.032 mmol, yield: 87.16%). ES-API: [M+H]⁺ = 992.3.

Step 2: Compound 56-1 (32 mg, 0.032 mmol) was dissolved in methanol (4.0 mL), and a 4 M solution of hydrogen chloride in dioxane (1.0 mL) was slowly added dropwise under an ice bath. The mixture was stirred at room temperature for 2.0 h. After the reaction was completed, the reaction mixture was concentrated by rotary evaporation to give a crude product. The crude product was purified by prep-HPLC (ammonium bicarbonate method 1) to give (2S)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methyl-2-(methylamino)butanamide (Z56, 16.5 mg, 0.018 mmol, yield: 57.35%). ES-API: [M+H]⁺ = 892.3.

### Example 24. Synthesis of Compound Z57

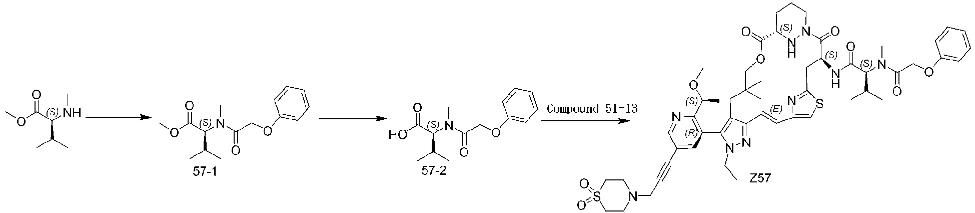

Step 1: Methyl N-methyl-L-valinate (300 mg, 2.066 mmol) and DIPEA (1599.17 mg, 12.397 mmol) were mixed in dichloromethane (10 mL), and (phenoxy)acetyl chloride (704.92 mg, 4.132 mmol) was slowly added at 0 °C. The reaction mixture was stirred at 20 °C for 2 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by flash silica gel column chromatography (0-80% petroleum ether/ethyl acetate) to give compound 57-1 (300 mg, yield: 51.98%), ES-API [M+H]⁺ = 280.1.

Step 2: Compound 57-1 (50 mg, 0.179 mmol), lithium hydroxide (15.02 mg, 0.358 mmol), and water (2 mL) were mixed in methanol (5 mL), and the mixture was stirred at 20 °C for 3 h. After the reaction was completed, an appropriate amount of diluted hydrochloric acid (1 M) was added to adjust the pH to weak acidity, and the mixture was concentrated to obtain crude compound 57-2, ES-API [M+H]⁺ = 266.2.

Step 3: Compound 51-13 (20 mg, 0.026 mmol), crude compound 57-2, and DIPEA (9.96 mg, 0.077 mmol) were mixed in dichloromethane (3 mL), and HATU (19.52 mg, 0.051 mmol) was slowly added to the solution. The mixture was stirred at 20 °C for 2 h. After the reaction was completed, 30 mL of ethyl acetate was poured into the reaction mixture, and the resulting mixture was washed sequentially with 15 mL of water and 15 mL of saturated brine. The organic phase was dried over anhydrous sodium sulfate, filtered, and concentrated, and the residue was purified by prep-HPLC (ammonium bicarbonate 1) to give (2S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8¹,8⁴,8¹,8⁶-hexahydro-1¹H-10-oxa-4(4,2)-thiazola-8(1,3)-pyridazina-1(3,4)-pyrazolocyclotridec-2-en-6-yl)-3-methyl-2-(N-methyl-2-phenoxyacetamido)butanamide (Z57, 5 mg, yield: 18.70%, purity: 98%). ES-API [M+H]⁺ = 1026.4.

### Example 25. Synthesis of Compound Z58

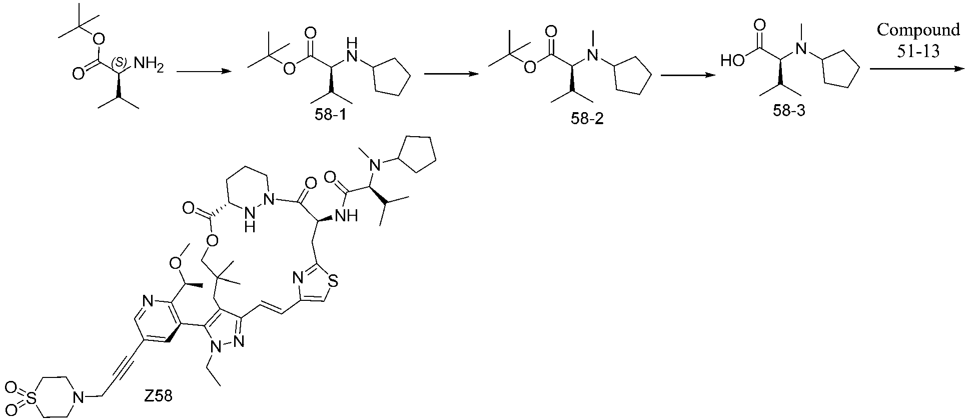

Step 1: Dichloromethane (20 mL) was added to a flask containing tert-butyl L-valinate (1.5 g, 8.658 mmol), and then cyclopentanone (1.46 g, 17.315 mmol), sodium acetate (1.07 g, 12.986 mmol), and sodium triacetoxyborohydride (4.57 g, 21.644 mmol) were added. The mixture was stirred at room temperature for 6 h. The reaction mixture was washed with sodium bicarbonate, extracted with dichloromethane, and purified by flash silica gel column chromatography (ethyl acetate/petroleum ether: 0-20%) to give compound 58-1 (700 mg, yield: 33.50%).

Step 2: Acetonitrile (10 mL) was added to a flask containing compound 58-1 (700 mg, 2.898 mmol), and then potassium carbonate (800 mg, 5.8 mmol) and iodomethane (1.23 g, 8.7 mmol) were added. The mixture was stirred at room temperature overnight. The reaction mixture was diluted with ethyl acetate and washed with water. The organic layer was dried over anhydrous sodium sulfate, filtered, and concentrated to give compound 58-2 (739 mg, crude product). ES-API: [M+H]⁺ = 256.3.

Step 3: Dichloromethane (2 mL) was added to a flask containing compound 58-2 (739 mg, 2.898 mmol), and then trifluoroacetic acid (3 mL, 0.315 mmol) was added under an ice bath. The mixture was stirred at room temperature for 1.5 h. The resulting yellow oily compound was concentrated, and the residue was purified by flash silica gel column chromatography (ethyl acetate/petroleum ether: 0-50%) to give compound 58-3 (461 mg, yield: 80%). ES-API: [M+H]⁺ = 200.1.

Step 4: Compound 51-13 (20 mg, 0.026 mmol) was dissolved in dichloromethane (1.0 mL), and compound 58-3 (19.0 mg, 0.096 mmol), O-(7-azabenzotriazol-1-yl)-*N*,*N*,*N*',*N*'-tetramethyluronium hexafluorophosphate (20.0 mg, 0.051 mmol), and *N*,*N*'-diisopropylethylamine (9.96 mg, 0.077 mmol) were added. The reaction system was stirred at room temperature for 2 h. After the reaction was completed, the reaction mixture was concentrated. Water (5.0 mL) was added to the residue, and the resulting mixture was extracted with dichloromethane (10.0 mL × 2). The organic phase was washed with saturated brine (5.0 mL × 3), dried, and concentrated, and the crude product was purified by prep-HPLC (ammonium bicarbonate method 2) to give (2S)-2-(cyclopentyl(methyl)amino)-N-((4²Z,8³S,2E,6S)-*(Rₐ)*-1⁵-(5-(3-(1,1-dioxidothiomorpholine)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolecyclotridec-2-en-6-yl)-3-methylbutanamide (Z58, 4.6 mg, yield: 18.42%). ES-API: [M+H]⁺= 960.2. ¹H NMR (400 MHz, DMSO-d₆) δ = 8.80 (d, *J*=4.0*,* 1H), 8.27 (d, *J*=8.0, 1H), 7.81 (d, *J=*4.0*,* 1H), 7.50 (s, 1H), 7.31 (q, *J*=12.0, 2H), 5.45-5.35 (m, 1H), 5.02 (d, *J*=12.0*,* 1H), 4.30-4.20 (m, 2H), 4.05-3.95 (m, 3H), 3.75 (s, 2H), 3.57 (q, *J*=12.0, 2H), 3.27-3.20 (m, 5H), 3.16-3.10 (m, 5H), 3.03-2.95 (m, 4H), 2.85-2.79 (m, 3H), 2.70-2.60 (m, 1H), 2.25-2.16 (m, 3H), 2.05-2.02 (m, 1H), 1.95-1.89 (m, 1H), 1.80-1.75 (m, 1H), 1.69-1.60 (m, 1H), 1.55-1.45 (m, 3H), 1.36 (d, *J*=8.0, 1H), 1.24-1.20 (m, 1H), 1.15 (t, *J*=8.0, 3H), 0.90 (d, *J*=4.0, 3H), 0.85-0.75 (m, 6H), 0.30 (s, 3H).

### Example 26. Synthesis of Compound Z59

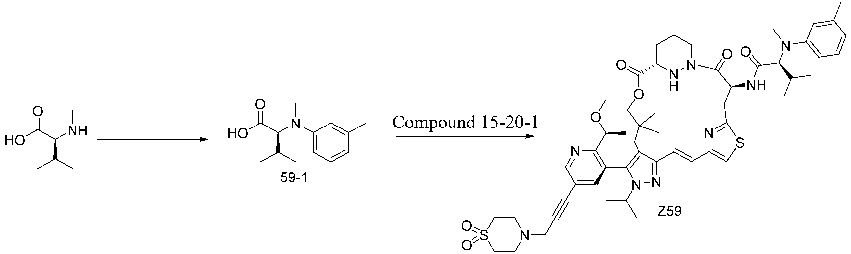

Step 1: Methyl-L-valine (1.0 g, 7.623 mmol) was dissolved in dry N,N-dimethylacetamide (9.0 mL), and then copper(I) iodide (217.77 mg, 1.143 mmol), 1-iodo-3-methylbenzene (1.994 g, 9.148 mmol), and potassium carbonate (2.107 g, 15.246 mmol) were added. The mixture was stirred at 100 °C for 18 h under nitrogen atmosphere. After the reaction was completed, the reaction mixture was filtered, and the filter cake was washed with ethyl acetate. The filter cake was then added to water (4.0 mL), adjusted to about pH 6 with 1 M hydrochloric acid under an ice bath, and extracted with a mixture of dichloromethane and methanol (10:1) (8.0 mL × 2). The organic phase was washed with saturated brine (5.0 mL × 3), dried, concentrated, and purified by flash silica gel column chromatography (tetrahydrofuran/petroleum ether: 0-65%) to give compound 59-1 (272 mg, 1.229 mmol, yield: 16.12%). ES-API: [M+H]⁺ = 222.3.

Step 2: Compound 59-1 (8.37 mg, 0.038 mmol) was dissolved in dichloromethane (1.0 mL), and compound 15-20-1 (15.0 mg, 0.019 mmol), O-(7-azabenzotriazol-1-yl)-*N*,*N*,*N*',*N*'-tetramethyluronium hexafluorophosphate (14.38 mg, 0.038 mmol), and *N*,*N*'-diisopropylethylamine (7.33 mg, 0.057 mmol) were added. The reaction system was stirred at room temperature for 3 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated to give a crude product. The crude product was purified by prep-HPLC (formic acid method 1) to give (2S)-N-((4²Z,8³S,2E,6S)-1⁵-(Rₐ)-(5-(3-(1,1-dioxidothiomorpholino)prop-1-yn-1-y1)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-3-methyl-2-(methyl(m-tolyl)amino)butanamide (Z59, 3.88 mg, 0.004 mmol, yield: 20.50%), formate. ES-API: [M+H]⁺ = 996.3. ¹H NMR (400 MHz, DMSO-d₆) δ 8.79 (s, 1H), 8.60 (d, *J =* 8.0 Hz, 1H), 7.77 (s, 1H), 7.39 - 7.28 (m, 2H), 7.24 -7.19 (m, 1H), 7.05 (t*, J=* 8.0 Hz, 1H), 6.80 (s, 1H), 6.74 (d, *J*= 7.6 Hz, 1H), 6.46 (d, *J*= 7.2 Hz, 1H), 5.46 - 5.37 (m, 1H), 5.06 - 5.00 (m, 1H), 4.40 - 4.30 (m, 2H), 4.27 -4.17 (m, 1H), 4.09 - 4.02 (m, 1H), 3.93 (d, *J=* 10.8 Hz, 1H), 3.74 (s, 2H), 3.60 - 3.50 (m, 2H), 3.26 (s, 2H), 3.18 - 3.06 (m, 6H), 3.05 - 2.98 (m, 4H), 2.85 (s, 3H), 2.79 - 2.73 (m, 1H), 2.67 - 2.61 (m, 1H), 2.27 (s, 3H), 2.19 - 2.09 (m, 2H), 2.05 - 1.90 (m, 1H), 1.81 - 1.67 (m, 2H), 1.56 (d, *J =* 6.0 Hz, 3H), 1.50 - 1.45 (m, 1H), 1.35 (d, *J =* 6.0 Hz, 3H), 1.25 - 1.20 (m, 1H), 1.01 (d, *J =* 6.4 Hz, 3H), 0.96 (d, *J =* 6.4 Hz, 3H), 0.85 - 0.75 (m, 6H), 0.35 - 0.25 (m, 3H).

### Example 27. Synthesis of Compound Z60

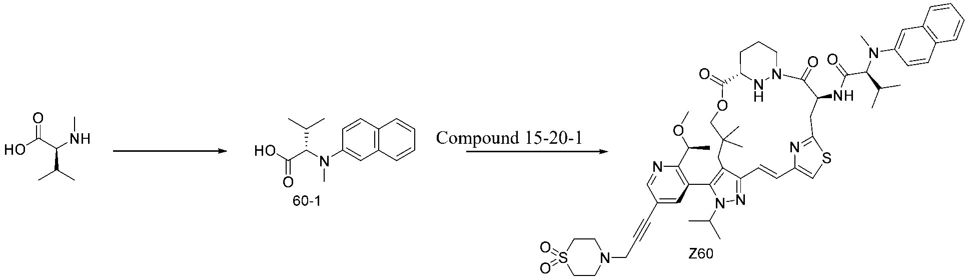

Step 1: Methyl-L-valine (1.0 g, 7.623 mmol) was dissolved in dry N,N-dimethylacetamide (10.0 mL), and then copper(I) iodide (217.77 mg, 1.143 mmol), 2-bromonaphthalene (3.157 g, 15.246 mmol), and potassium carbonate (2.107 g, 15.246 mmol) were added. The mixture was stirred at 100 °C for 18 h under nitrogen atmosphere. After the reaction was completed, the reaction mixture was filtered, and the filter cake was washed with ethyl acetate. The filter cake was then added to water (4.0 mL), adjusted to about pH 6 with 1 M hydrochloric acid under an ice bath, and extracted with a mixture of dichloromethane and methanol (10:1) (8.0 mL × 2). The organic phase was washed with saturated brine (5.0 mL × 3), dried, concentrated, and purified by flash silica gel column chromatography (tetrahydrofuran/petroleum ether: 0-65%) to give compound 60-1 (19 mg, 0.074 mmol, yield: 0.97%). ES-API: [M+H]⁺ = 258.1.

Step 2: Compound 60-1 (4.87 mg, 0.019 mmol) was dissolved in dichloromethane (2.0 mL), and compound 15-20-1 (15.0 mg, 0.019 mmol), O-(7-azabenzotriazol-1-yl)-*N*,*N*,*N*',*N*'-tetramethyluronium hexafluorophosphate (14.38 mg, 0.038 mmol), and *N*,*N'*-diisopropylethylamine (7.33 mg, 0.057 mmol) were added. The reaction system was stirred at room temperature for 3 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated to give a crude product. The crude product was purified by prep-HPLC (formic acid method 1) to give (2S)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-3-methyl-2-(methyl(naphthalen-1-yl)amino)butanamide (Z60, 5.15 mg, 0.005 mmol, yield: 26.37%), formate. ES-API: [M+H]⁺ = 1032.3. ¹H NMR (400 MHz, DMSO-d₆) δ 8.79 (d, *J =* 2.0 Hz, 1H), 8.69 (d, *J =* 8.4 Hz, 1H), 7.77 - 7.65 (m, 4H), 7.55 - 7.47 (m, 1H), 7.38 - 7.28 (m, 2H), 7.20 - 7.10 (m, 4H), 5.43 (t, *J =* 8.8 Hz, 1H), 5.04 (d, *J =* 12.4 Hz, 1H), 4.40 - 4.31 (m, 2H), 4.27 - 4.17 (m, 1H), 4.13 - 4.02 (m, 2H), 3.74 (s, 2H), 3.60 -3.50 (m, 2H), 3.26 (s, 2H), 3.20 - 3.13 (m, 4H), 3.06 -3.01 (m, 4H), 2.99 (s, 3H), 2.81 - 2.73 (m, 1H), 2.70 - 2.60 (m, 1H), 2.28 - 2.20 (m, 1H), 2.13 - 2.06 (m, 1H), 2.05 -1.93 (m, 2H), 1.82 - 1.65 (m, 2H), 1.55 (d, *J=* 6.4 Hz, 3H), 1.51 - 1.47 (m, 1H), 1.35 (d, *J =* 6.0 Hz, 3H), 1.30 - 1.20 (m, 4H), 1.05 - 0.95 (m, 6H), 0.87- 0.72 (m, 7H), 0.35 - 0.23 (m, 3H).

### Example 28. Synthesis of Compound Z61

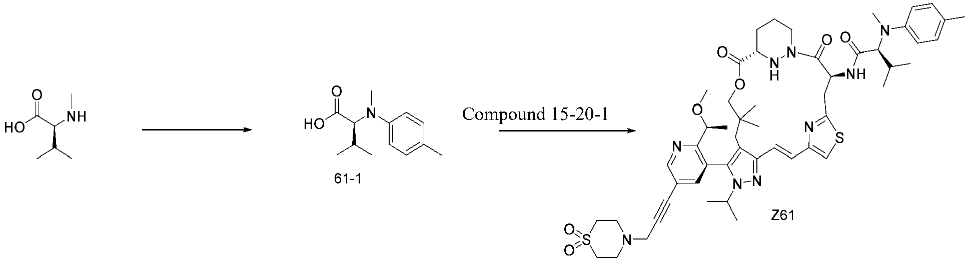

Step 1: Methyl-L-valine (1.0 g, 7.623 mmol) was dissolved in dry N,N-dimethylacetamide (8.0 mL), and then copper(I) iodide (217.77 mg, 1.143 mmol), 4-iodomethylbenzene (1.828 g, 8.385 mmol), and potassium carbonate (2.107 g, 15.246 mmol) were added. The mixture was stirred at 100 °C for 48 h under nitrogen atmosphere. After the reaction was completed, the reaction mixture was filtered, and the filter cake was washed with ethyl acetate. The filter cake was then added to water (4.0 mL), adjusted to about pH 6 with 1 M hydrochloric acid under an ice bath, and extracted with a mixture of dichloromethane and methanol (10:1) (8.0 mL × 2). The organic phase was washed with saturated brine (5.0 mL × 3), dried, concentrated, and purified by flash silica gel column chromatography (tetrahydrofuran/petroleum ether: 0-65%) to give compound 61-1 (23 mg, 0.104 mmol, yield: 1.36%). ES-API: [M+H]⁺ = 222.3.

Step 2: Compound 61-1 (8.37 mg, 0.038 mmol) was dissolved in dichloromethane (2.0 mL), and compound 15-20-1 (15.0 mg, 0.019 mmol), O-(7-azabenzotriazol-1-yl)-*N*,*N*,*N*',*N*'-tetramethyluronium hexafluorophosphate (14.38 mg, 0.038 mmol), and *N*,*N*'-diisopropylethylamine (7.33 mg, 0.057 mmol) were added. The reaction system was stirred at room temperature for 3 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated to give a crude product. The crude product was purified by prep-HPLC (formic acid method 1) to give (2S)-N-((4²Z,8³S,2E,6S)-1⁵-(Rₐ)-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-3-methyl-2-(methyl(p-tolyl)amino)butanamide (Z61, 5.02 mg, 0.005 mmol, yield: 26.64%), formate. ES-API: [M+H]⁺ = 996.3. ¹H NMR (400 MHz, DMSO-d₆) δ 8.79 (d, *J* = 1.6 Hz, 1H), 8.53 (d, *J =* 8.4 Hz, 1H), 7.78 (s, 1H), 7.38 (s, 1H), 7.36 - 7.29 (m, 1H), 7.25 - 7.19 (m, 1H), 7.01 - 6.96 (m, 2H), 6.89 - 6.83 (m, 2H), 5.41 (t, *J* = 8.8 Hz, 1H), 5.06 - 4.99 (m, 1H), 4.42 - 4.30 (m, 2H), 4.25 - 4.17 (m, 1H), 4.10 - 4.00 (m, 1H), 3.93 - 3.85 (m, 1H), 3.75 (s, 2H), 3.61 - 3.51 (m, 2H), 3.28 - 3.23 (m, 3H), 3.17 - 3.13 (m, 4H), 3.04 (s, 4H), 2.83 (s, 3H), 2.79 -2.73 (m, 1H), 2.67 - 2.60 (m, 1H), 2.22 - 2.13 (m, 5H), 2.05 - 1.95 (m, 1H), 1.81 - 1.65 (m, 2H), 1.56 (d, *J =* 6.4 Hz, 3H), 1.35 (d, *J =* 6.0 Hz, 3H), 1.02 (d, *J =* 6.4 Hz, 3H), 0.95 (d, *J =* 6.4 Hz, 3H), 0.85 - 0.75 (m, 6H), 0.32 - 0.26 (m, 3H).

### Example 29. Synthesis of Compound Z62

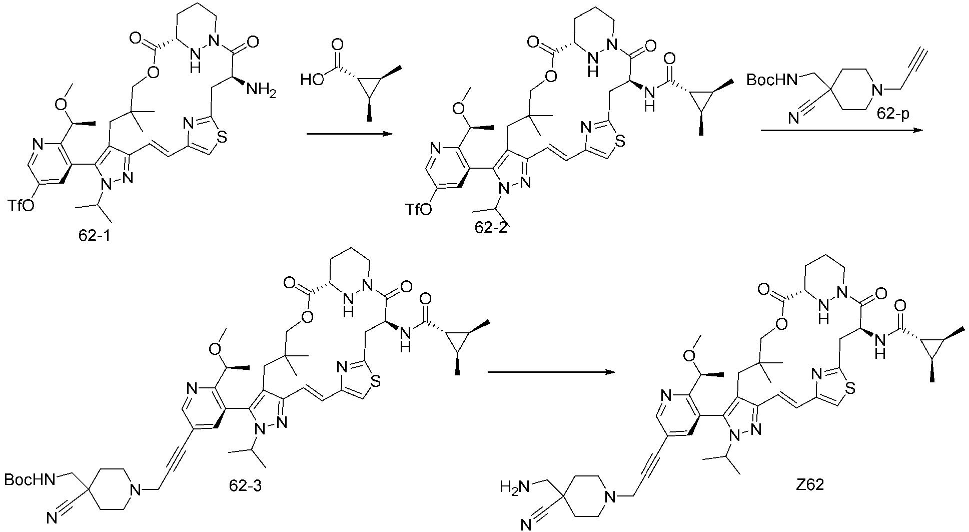

The starting materials in this example were used for synthesis by referring to the synthetic method of Z51, with ethyl replaced by isopropyl, or by referring to Preparation Example 5.

Step 1: Compound 62-1 (40 mg, 0.052 mmol) was dissolved in N,N-dimethylformamide (2 mL). (1r,2S,3R)-2,3-Dimethylcyclopropane-1-carboxylic acid (7.71 mg, 0.068 mmol), O-(7-azabenzotriazol-1-yl)-N,N,N',N'-tetramethyluronium hexafluorophosphate (27.66 mg, 0.073 mmol), and N,N'-diisopropylethylamine (20.15 mg, 0.156 mmol) were added to the above solution. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by flash silica gel column chromatography (methanol/dichloromethane: 0%-10%) to give compound 62-2 (39 mg, 0.045 mmol, yield: 86.68%) as a yellow solid, ES-API: [M+H]⁺ = 866.2.

Step 2: Compound 62-2 (38 mg, 0.044 mmol) and compound 62-p (24.34 mg, 0.088 mmol) were dissolved in acetonitrile (3 mL), and bis(triphenylphosphine)palladium(II) dichloride (12.82 mg, 0.018 mmol), copper(I) iodide (5.27 mg, 0.027 mmol), N,N-diisopropylethylamine (23.59 mg, 0.183 mmol), and anhydrous lithium chloride (7.68 mg, 0.183 mmol) were added. The mixture was reacted at 80 °C for 5 h under a nitrogen atmosphere. After the reaction was completed, the mixture was filtered under vacuum and concentrated under reduced pressure to give a crude product. The crude product was purified by flash silica gel column chromatography (methanol/dichloromethane: 0%-10%) to give compound 62-3 (35 mg, 0.035 mmol, yield: 80.30%) as a white solid, ES-API: [M+H]⁺ = 993.3.

Step 3: Compound 62-3 (35 mg, 0.035 mmol) was dissolved in dichloromethane (2 mL), and trifluoroacetic acid (2 mL, 2.301 mmol) was added. The mixture was reacted for 1 h under nitrogen atmosphere, and concentrated to dryness under reduced pressure. Water (10 mL) was added, sodium bicarbonate solution was added to adjust the pH to 8, and the resulting mixture was extracted with ethyl acetate (15 mL × 2). The organic phase was dried over anhydrous sodium sulfate and concentrated to dryness under reduced pressure to give a crude product, which was purified by prep-HPLC (ammonium bicarbonate method 1) to give (1*r*,2*R*,3*S*)-N-((4²*Z*,8³*S*,2*E*,6*S*)-(*Rₐ*)-1⁵-(5-(3-(4-(aminomethyl)-4-cyanopiperidin-1-yl)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹*H-*10-oxa-4(4,2)-thiazola-8(1,3)-pyridazina-1(3,4)-pyrrolacyclotridecaphan-2-en-6-yl)-2,3-dimethylcyclopropane-1-carboxamide (Z62, 15 mg, 0.017 mmol, yield: 55.60%) as a white solid. ES-API: [M+H] ⁺ =893.3. ¹H NMR (400 MHz, DMSO-d₆) δ 8.78 (d, *J =* 2.0 Hz, 1H), 8.34 (d, *J =* 8.8 Hz, 1H), 7.75 (d, *J* = 2.0 Hz, 1H), 7.46 (s, 1H), 7.37 (d, *J =* 15.6 Hz, 1H), 7.25 (d, *J =* 15.6 Hz, 1H), 5.55 (t, *J =* 8.8 Hz, 1H), 5.02 (d, *J* = 12.0 Hz, 1H), 4.40 - 4.30 (m, 2H), 4.21 (d, *J =* 10.4 Hz, 1H), 4.01-3.99 (m, 1H), 3.65 - 3.53 (m, 4H), 3.23-3.21 (m, 5H), 3.15 - 3.04 (m, 2H), 2.88 (d, *J =* 9.8 Hz, 2H), 2.77 - 2.69 (m, 3H), 2.60 (d, *J =* 14.4 Hz, 1H), 2.45-2.43 (m, 2H), 2.18 (d, *J =* 14.4 Hz, 1H), 1.97 (d, *J =* 11.6 Hz, 1H), 1.88 (d, *J =* 12.6 Hz, 2H), 1.75-1.68 (m, 2H), 1.55 (d, *J =* 6.6 Hz, 3H), 1.51 - 1.45 (m, 2H), 1.37 (d, *J =* 6.0 Hz, 3H), 1.23 (s, 1H), 1.19 - 1.12 (m, 3H), 1.10-1.05 (m, 9H), 0.76 (s, 3H), 0.34 (s, 3H).

### Example 30. Synthesis of Compound Z115

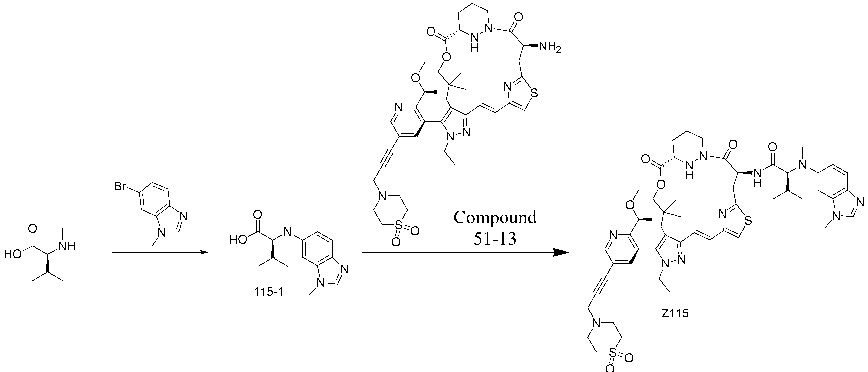

Step 1: Methyl-*L*-valine (372.92 mg, 2.843 mmol) and 6-bromo-1-methyl-1*H*-benzo[*d*]imidazole (300 mg, 1.421 mmol) were dissolved in *N*,*N*-dimethylacetamide (10 mL). Copper(I) iodide (54.14 mg, 0.284 mmol) and potassium carbonate (392.87 mg, 2.843 mmol) were added to the above solution. After the addition was completed, the reaction system was stirred at 120 °C for 18 h under nitrogen atmosphere. The reaction mixture was filtered through a sand core funnel to give a mother liquor. The mother liquor was purified by reversed-phase column chromatography (acetonitrile/water: 0%-40%) to give compound 115-1 (120 mg, 0.459 mmol, yield: 32.31%) as a colorless oily liquid. ES-API: [M+H]⁺ = 262.2.

Step 2: Compound 51-13 (20 mg, 0.026 mmol) was dissolved in dichloromethane (2 mL). Compound 115-1 (20.38 mg, 0.078 mmol), O-(7-azabenzotriazol-1-yl)-*N*,*N*,*N*',*N*'-tetramethyluronium hexafluorophosphate (20 mg, 0.052 mmol), and *N*,*N*'-diisopropylethylamine (13 mg, 0.1 mmol) were added to the above solution. After the addition was completed, the reaction system was stirred at room temperature for 2 h. After the reaction was completed, the reaction mixture was concentrated to remove dichloromethane. Water (10 mL) was added to the residue, and the resulting mixture was extracted with ethyl acetate (15 mL × 2). The organic phase was washed with saturated brine (10 mL × 3), dried, and concentrated to give a crude product. The crude product was purified by prep-HPLC (formic acid method 1) to give (2*S*)-*N*-((4²*Z*,8³*S*,2*E*,6*S*)-1⁵-(*Rₐ*)-(5-(3-(1,1-dioxidothiomorpholino)prop-1-yn-1-yl)-2-((*S*)-1-methoxyethyl)pyridin-3-yl)-1¹-ethyl-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrazolocyclotridec-2-en-6-yl)-3-methyl-2-(methyl(1-methyl-1H-benzo[d]imidazol-6-yl)amino)butanamide (Z115, 3.89 mg, 0.003 mmol, yield: 12.60%), formate, as a white solid. ES-API: [M+H]⁺ = 1022.3.

### Example 31. Synthesis of Compound Z116

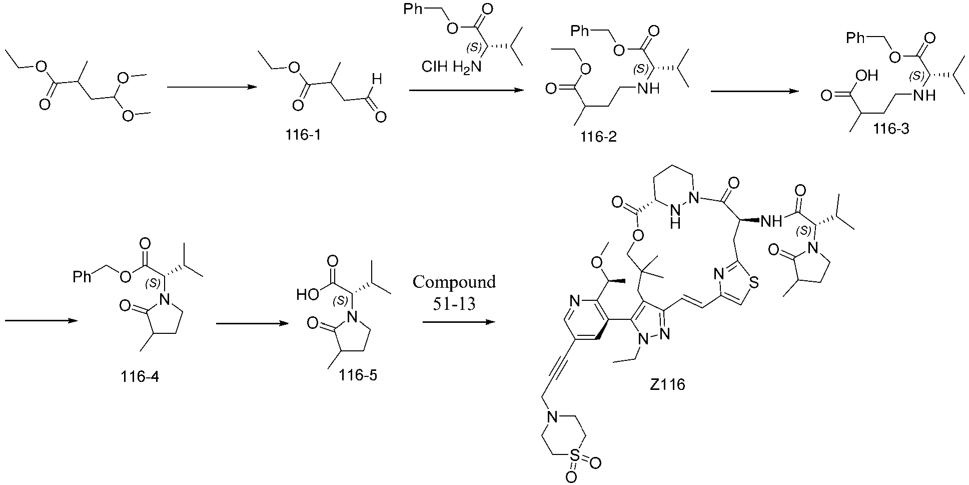

Step 1: Chloroform (40 mL) and water (10 mL) were added to a flask containing ethyl 4,4-dimethoxy-2-methylbutanoate (4.6 g, 24.180 mmol), and then trifluoroacetic acid (27.57 g, 241.800 mmol) was added. The mixture was stirred at room temperature for 2 h. A saturated potassium carbonate solution was added to the reaction mixture until the reaction pH was 7.5, and the resulting mixture was diluted with dichloromethane and washed with water. The organic phase was dried over anhydrous sodium sulfate, filtered, and concentrated. The crude product was purified by flash silica gel column chromatography (ethyl acetate/petroleum ether: 0-20%) to give compound 116-1 (3.2 g, yield: 91.79%). ES-API: [M+1]⁺ = 145.1. Step 2: Methanol (20 mL) was added to a flask containing compound 116-1 (3.2 g, 22.196 mmol) and benzyl L-valinate hydrochloride (6.29 g, 25.803 mmol), and then sodium cyanoborohydride (6.67 g, 107.510 mmol) was added. The mixture was stirred at room temperature for 2 h. The reaction mixture was diluted with ethyl acetate and washed with water. The organic phase was dried over anhydrous sodium sulfate, filtered, and concentrated. The resulting crude product was purified by flash silica gel column chromatography (ethyl acetate/petroleum ether: 0-20%) to give compound 116-2 (3.6 g, yield: 49.91%). ES-API: [M+1]⁺ = 336.3. Step 3: Tetrahydrofuran (10 mL), methanol (2 mL), and water (2 mL) were added to a flask containing compound 116-2 (3.6 g, 10.732 mmol), and then lithium hydroxide (0.90 g, 21.464 mmol) was added. The mixture was stirred at room temperature overnight. The reaction mixture was poured into an added 2 M hydrochloric acid suspension until pH = 3. The organic phase was separated, and the aqueous layer was extracted with another portion of ethyl acetate. The combined organic extracts were washed with water and brine, and then dried over anhydrous sodium sulfate, filtered, and concentrated to give compound 116-3 (2.5 g, 8.133 mmol, yield: 75.78%). ES-API: [M+1]⁺ = 308.2.

Step 4: Dichloromethane (20 mL) was added to a flask containing compound 116-3 (2.4 g, 7.808 mmol), and then O-(7-azabenzotriazol-1-yl)-N,N,N',N'-tetramethyluronium hexafluorophosphate (5.93 g, 15.615 mmol) and N,N'-diisopropylethylamine (3.03 g, 23.423 mmol) were added. The mixture was stirred at room temperature for 2 h. The reaction mixture was diluted with dichloromethane and washed with water. The organic phase was dried over anhydrous sodium sulfate, filtered, and concentrated. The residue was purified by flash silica gel column chromatography (ethyl acetate/petroleum ether: 0-50%) to give compound 116-4 (600 mg, yield: 26.56%). ES-API: [M+1]⁺ = 290.2.

Step 5: Methanol (5 mL) was added to a flask containing compound 116-4 (400 mg, 1.382 mmol), and then palladium on carbon (29.42 mg, 0.276 mmol, 10%) was added. The mixture was stirred at room temperature overnight under hydrogen atmosphere. The reaction mixture was filtered to give a filtrate, and the filtrate was concentrated. The resulting crude product was purified by flash silica gel column chromatography (ethyl acetate/petroleum ether: 0-50%) to give compound 116-5 (180 mg, yield: 65.35%). ES-API: [M+1]⁺ = 262.2. Step 6: Compound 51-13 (10 mg, 0.013 mmol), compound 116-5 (7.67 mg, 0.039 mmol), O-(7-azabenzotriazol-1-yl)-N,N,N',N'-tetramethyluronium hexafluorophosphate (9.76 mg, 0.026 mmol), and N,N'-diisopropylethylamine (4.98 mg, 0.039 mmol) were stirred at room temperature for 2 h. After the reaction was completed, the reaction mixture was concentrated. Water (5.0 mL) was added to the residue, and the resulting mixture was extracted with dichloromethane (10.0 mL × 2). The organic phase was washed with saturated brine (5.0 mL × 3), dried over anhydrous sodium sulfate, filtered, and concentrated. The crude product was purified by prep-HPLC (ammonium bicarbonate method 2) to give (2S)-N-((4²Z,8³S,2E,6S)-(Rₐ*)*-1⁵-(5-(3-(1,1-dioxothiomorpholinyl)prop-1-yn-1-yl)-2-((S)-1-methoxyethyl)pyridin-3-yl)-1¹-ethyl-12,12-dimethyl-7,9-dioxo-8¹,8²,8¹,8⁴,8⁵,8⁶-hexahydro-1¹H-10-oxo-4 (4,2)-thiazola-8(1,3)-pyridazina-1(3,4)-pyrrolacyclotridecaphan-2-en-6-yl)-3-methyl-2-(3-methyl-2-oxopyrrolidin-1-yl)butanamide (Z116, 2.5 mg, yield: 20.28%). ES-API: [M+1]⁺ = 960.2.

### Example 32. Synthesis of Compound Z117

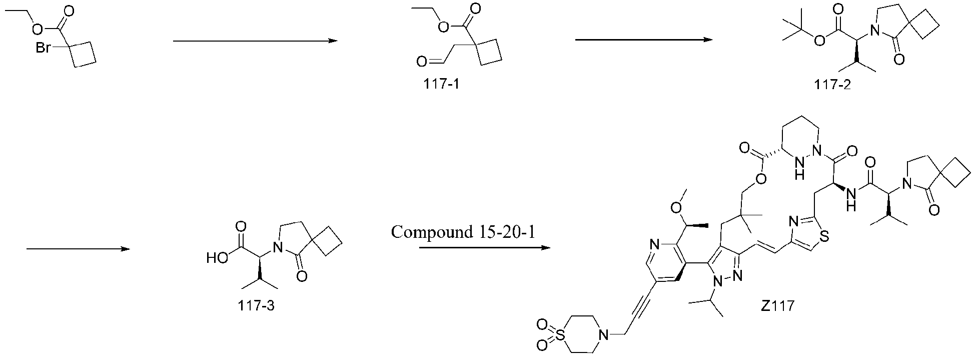

Step 1: Ethyl 1-bromocyclobutane-1-carboxylate (1.0 g, 4.829 mmol) was dissolved in tetrahydrofuran (8.0 mL) and water (2.0 mL), and N-methyl-N-vinylacetamide (0.96 g, 9.659 mmol), copper bromide (0.54 g, 2.414 mmol), N,N,N',N",N"'-pentamethyldiethylenetriamine (0.42 g, 2.414 mmol), and DIPEA (1.87 g, 14.487 mmol) were added. The mixture was stirred at 60 °C for 24 h under nitrogen atmosphere. After the reaction was completed, the reaction mixture was diluted with 10.0 mL of ethyl acetate and 4.0 mL of water. The organic layer was further washed with 5 mL of a saturated sodium chloride solution, and concentrated in vacuum, and the residue was purified by silica gel column chromatography (tetrahydrofuran/petroleum ether = 0%-15%) to give the target compound 117-1 (624.0 mg, 3.666 mmol, yield: 75.91%). ES-API: [M+H]⁺ = 171.2. ¹H NMR (400 MHz, DMSO-D₆) *δ* 9.60 (s, 1H), 4.06 (q, *J =* 7.2 Hz, 2H), 2.98 (s, 2H), 2.43 - 2.33 (m, 2H), 2.01 - 1.85 (m, 4H), 1.16 (t, *J =* 7.2 Hz, 3H).

Step 2: Compound 117-1 (544 mg, 3.196 mmol) was dissolved in 1,2-dichloroethane (5.0 mL), and tert-butyl L-valinate (664.50 mg, 3.835 mmol) was added. The mixture was stirred at room temperature for 1 h. Sodium triacetoxyborohydride (1.685 g, 7.990 mmol) was then added in portions under an ice bath, and the mixture was stirred at room temperature for 18 h. After the reaction was completed, the reaction mixture was diluted with ethyl acetate (10.0 mL) and water (5.0 mL). The organic layer was washed with saturated sodium chloride solution (10.0 mL × 2), concentrated in vacuum, and purified by silica gel column chromatography (tetrahydrofuran/petroleum ether = 0%-15%) to give the target compound 117-2 (368.0 mg, 1.308 mmol, yield: 40.92%).

Step 3: Compound 117-2 (65.0 mg, 0.231 mmol) was dissolved in dichloromethane (1.0 mL). Trifluoroacetic acid (0.5 mL) was slowly added to the above solution under an ice bath, and the reaction system was stirred at room temperature for 3 h. After the reaction was completed, the reaction mixture was concentrated, and the residue was repeatedly concentrated with dichloromethane to remove excess trifluoroacetic acid to give crude compound 117-3 (48.0 mg, 0.213 mmol, yield: 92.24%). ES-API: [M+23]⁺ = 226.1.

Step 4: Compound 117-3 (7.95 mg, 0.035 mmol) was dissolved in dichloromethane (1.0 mL), and compound 15-20-1 (14.0 mg, 0.018 mmol), O-(7-azabenzotriazol-1-yl)-N,N,N',N'-tetramethyluronium hexafluorophosphate (13.43 mg, 0.035 mmol), and N,N'-diisopropylethylamine (6.85 mg, 0.053 mmol) were added. The reaction system was stirred at room temperature for 3 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated, and the residue was purified by prep-HPLC (ammonium bicarbonate method 1) to give (2S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methyl-2-(5-oxo-6-azaspiro[3.4]octan-6-yl)butanamide (Z117, 4.4 mg, 0.004 mmol, yield: 24.92%). ES-API: [M+H]⁺ = 1000.4.

### Example 33. Synthesis of Compound Z118

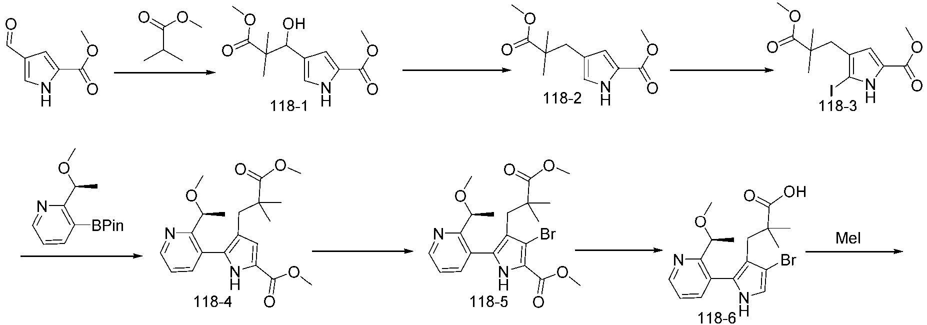

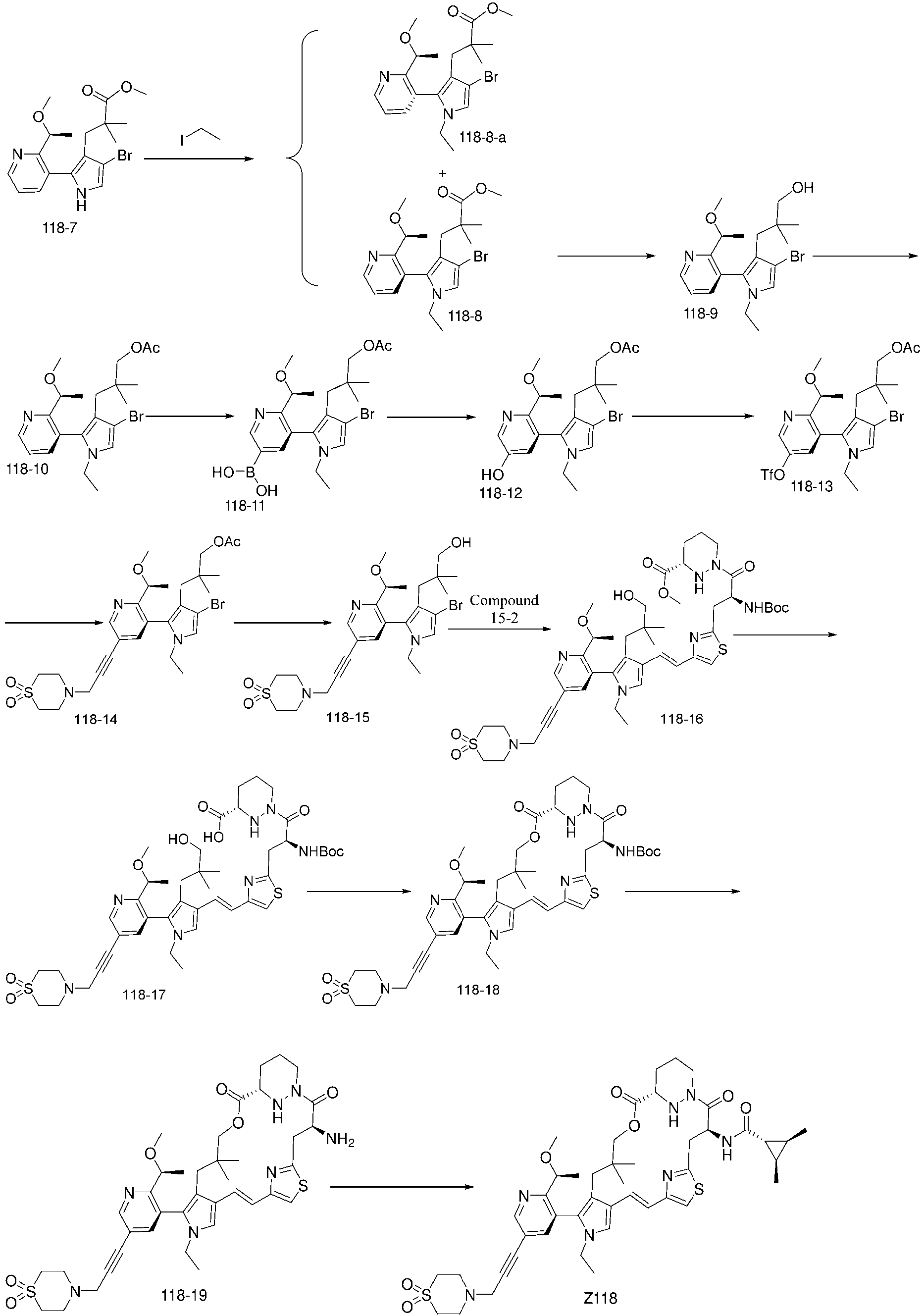

Step 1: Methyl isobutyrate (14.67 g, 143.66 mmol) was dissolved in tetrahydrofuran (150 mL), and a 2.0 M solution of lithium diisopropylamide in tetrahydrofuran/n-hexane (71.83 mL, 143.66 mmol) was added dropwise at -70 °C. The reaction system was stirred at -70 °C for 1 h. A solution of methyl 4-formyl-1H-pyrrole-2-carboxylate (10 g, 65.30 mmol) in tetrahydrofuran (15 mL) was added dropwise. The reaction system was stirred at -70 °C for 1 h, and then slowly warmed to 0 °C and stirred for 2 h. The reaction was quenched with a saturated ammonium chloride solution (50 mL) and water (30 mL), and the reaction mixture was extracted with ethyl acetate (200 mL). The organic phase was washed with saturated brine (100 mL), dried over anhydrous sodium sulfate, and filtered, and the filtrate was concentrated under reduced pressure. The crude product was purified by flash silica gel column chromatography (ethyl acetate/petroleum ether: 0-50%) to give compound 118-1 (16.0 g, yield: 96.0%) as a yellow liquid. ES-API: [M-18+H]⁺ = 238.1.

Step 2: Compound 118-1 (9 g, 35.26 mmol) was dissolved in dichloromethane (120 mL). Trifluoroacetic acid (13.50 mL, 176.28 mmol) was added at 0 °C, and then triethylsilane (33.70 mL, 211.54 mmol) was slowly added dropwise. The reaction system was stirred at room temperature for 18 h. The reaction mixture was concentrated under reduced pressure. A saturated sodium bicarbonate solution (50 mL) was slowly added, and the resulting mixture was extracted with ethyl acetate (150 mL). The organic phase was dried over anhydrous sodium sulfate and filtered, and the filtrate was concentrated under reduced pressure. The crude product was purified by flash silica gel column chromatography (ethyl acetate/petroleum ether: 0-20%) to give compound 118-2 (5.7 g, yield: 67.6%) as a yellow liquid. ES-API: [M+H]⁺ = 240.2.

Step 3: Compound 118-2 (2.9 g, 12.12 mmol) was dissolved in acetonitrile (60 mL), and N-iodosuccinimide (3.55 g, 15.76 mmol) was added at 0 °C. The reaction system was stirred at 10 °C overnight. Ethyl acetate (150 mL) was added to the reaction mixture, and the resulting mixture was washed sequentially with a saturated sodium bicarbonate solution (80 mL) and saturated brine (80 mL). The organic phase was dried over anhydrous sodium sulfate and filtered, and the filtrate was concentrated under reduced pressure. The crude product was purified by flash silica gel column chromatography (ethyl acetate/petroleum ether: 0-15%) to give compound 118-3 (2.9 g, yield: 65.5%) as a white solid. ES-API: [M+H]⁺ = 366.0.

Step 4: Compound 118-3 (2.7 g, 7.39 mmol) and 2-[(1S)-1-methoxyethyl]-3-(4,4,5,5-tetramethyl-1,3,2-dioxaborolan-2-yl)pyridine (2.92 g, 11.09 mmol) were dissolved in 1,4-dioxane (50 mL) and water (25 mL), and potassium carbonate (2.25 g, 16.27 mmol) and 1,1-bis(diphenylphosphino)ferrocene dichloropalladium (0.54 g, 0.739 mmol) were added. The reaction system was purged with nitrogen for 30 s and stirred at 90 °C for 8 h in a microwave reactor. Ethyl acetate (100 mL) was added to the reaction mixture, and the resulting mixture was washed sequentially with water (30 mL × 2) and saturated brine (30 mL), dried over anhydrous sodium sulfate, and filtered, and the filtrate was concentrated under reduced pressure. The crude product was purified by flash silica gel column chromatography (ethyl acetate/petroleum ether: 0-50%) to give compound 118-4 (2.7 g, yield: 97.5%) as a light brown liquid. ES-API: [M+H]⁺ = 375.1.

Step 5: Compound 118-4 (2.7 g, 6.95 mmol) was dissolved in acetonitrile (40 mL), and N-bromosuccinimide (1.67 g, 9.37 mmol) was added at room temperature. The reaction system was stirred at 40 °C for 1 h. The reaction mixture was cooled to room temperature. Water (5 mL) and concentrated ammonia water (1.5 mL) were added, and the resulting mixture was further stirred for 15 min. Ethyl acetate (100 mL) was added to the reaction mixture, and the resulting mixture was washed sequentially with saturated brine (50 mL × 2). The organic phase was dried over anhydrous sodium sulfate and filtered, and the filtrate was concentrated under reduced pressure. The crude product was purified by flash silica gel column chromatography (ethyl acetate/petroleum ether: 0-50%) to give compound 118-5 (3.2 g, yield: 97.9%) as a yellow liquid. ES-API: [M+H]⁺ = 453.1, 455.1. ¹H NMR (400 MHz, CDCl₃) δ 10.60 (s, 1H), 8.61 (dd, *J* = 4.8, 1.6 Hz, 1H), 7.77 (dd, J= 8.0, 1.6 Hz, 1H), 7.34 (dd, *J =* 8.0, 4.8 Hz, 1H), 4.54 (q, *J =* 6.4 Hz, 1H), 3.89 (s, 3H), 3.42 (s, 3H), 3.13 (s, 3H), 3.05 (d, *J* = 14.4 Hz, 1H), 2.96 (d, *J =* 14.4 Hz, 1H), 1.54 (d, *J =* 6.5 Hz, 3H), 1.02 (s, 3H), 0.96 (s, 3H).

Step 6: Compound 118-5 (2.6 g, 5.74 mmol) was dissolved in absolute ethanol (50 mL) and water (25 mL), and lithium hydroxide monohydrate (1.93 g, 45.88 mmol) was added at room temperature. The reaction system was stirred at 100 °C for 64 h. The reaction mixture was cooled to room temperature and concentrated under reduced pressure to remove the ethanol solvent, and the pH of the reaction mixture was adjusted to 5 with 1 M diluted hydrochloric acid. The precipitated solid was filtered and dried in vacuum under reduced pressure. The organic phase was dried over anhydrous sodium sulfate. The resulting crude product was purified by flash silica gel column chromatography (methanol/dichloromethane: 0-5%) to give compound 118-6 (1.3 g, yield: 59.5%) as a white solid. ES-API: [M+H]⁺ = 381.0, 383.1.

Step 7: Compound 118-6 (600 mg, 1.57 mmol) was dissolved in N,N-dimethylformamide (10 mL), and potassium carbonate (283 mg, 2.05 mmol) and iodomethane (268 mg, 1.89 mmol) were added at room temperature. The reaction system was stirred at room temperature for 2 h. Ethyl acetate (100 mL) was added to the reaction mixture, and the resulting mixture was washed with saturated brine (30 mL × 3). The organic phase was dried over anhydrous sodium sulfate and filtered, and the filtrate was concentrated under reduced pressure to give compound 118-7 (622 mg, yield: 100%) as a light yellow liquid. ES-API: [M+H]⁺ = 395.0, 397.0.

Step 8: Compound 118-7 (622 mg, 1.57 mmol) was dissolved in N,N-dimethylformamide (8 mL), and cesium carbonate (1.02 g, 3.15 mmol) and iodoethane (491 mg, 3.15 mmol) were added at room temperature. The reaction system was stirred at 60 °C for 1 h. Ethyl acetate (100 mL) was added to the reaction mixture, and the resulting mixture was washed with saturated brine (30 mL × 3). The organic phase was dried over anhydrous sodium sulfate and filtered, and the filtrate was concentrated under reduced pressure. The crude product was purified by flash silica gel column chromatography (tetrahydrofuran/petroleum ether: 0-30%) to give compound 118-8 (350 mg, 3 min, LCMS retention time: 1.96 min, yield: 52.5%, structure arbitrarily designated) as a colorless liquid, ES-API: [M+H]⁺ = 423.1, 425.1, and isomer 118-8-a (240 mg, 3 min, LCMS retention time: 1.82 min, yield: 36.0%, structure arbitrarily designated) as a colorless liquid, ES-API: [M+H]⁺ = 423.1, 425.1.

Step 9: Compound 118-8 (350 mg, 0.83 mmol) was dissolved in dichloromethane (15 mL), and a 1.0 M solution of DIBAL-H in n-hexane (2.07 mL, 2.07 mmol) was added dropwise at -70 °C. The reaction system was stirred at -70 °C for 1 h, and then warmed to room temperature and stirred for 2 h. The reaction mixture was cooled to 0 °C. Water (0.10 mL), 15% sodium hydroxide solution (0.10 mL), and water (0.25 mL) were slowly added dropwise sequentially to quench the reaction. The reaction mixture was stirred at room temperature for 15 min, dried over anhydrous magnesium sulfate, and filtered, and the filtrate was concentrated under reduced pressure to give compound 118-9 (326 mg, yield: 99.7%) as a light yellow viscous liquid. ES-API: [M+H]⁺ = 395.1, 397.1.

Step 10: Compound 118-9 (326 mg, 0.83 mmol), diisopropylethylamine (320 mg, 2.47 mmol), and 4-dimethylaminopyridine (10 mg, 0.08 mmol) were dissolved in dichloromethane (8 mL), and acetic anhydride (168 mg, 1.65 mmol) was added at 0 °C. The reaction system was stirred at 0 °C for 30 min. The reaction mixture was concentrated, and the crude product was purified by flash silica gel column chromatography (tetrahydrofuran/petroleum ether: 0-20%) to give compound 118-10 (320 mg, yield: 88.7%) as a light yellow viscous liquid. ES-API: [M+H]⁺= 437.1, 439.1.

Step 11: Compound 118-10 (320 mg, 0.73 mmol), bis(pinacolato)diboron (334 mg, 1.32 mmol), chloro(1,5-cyclooctadiene)iridium(I) dimer (59 mg, 0.088 mmol), and 4,4'-di-tert-butyl-2,2'-bipyridine (39 mg, 0.146 mmol) were dissolved in cyclohexane (20 mL). The reaction system was stirred at 85°C for 18 h under nitrogen atmosphere. The reaction mixture was concentrated to give compound 118-11 (352 mg, crude product). ES-API: [M+H]⁺ = 481.1, 483.1.

Step 12: Compound 118-11 (352 mg, crude product) was dissolved in acetonitrile (6 mL), and 30% hydrogen peroxide (248 mg, 2.19 mmol) was added under an ice bath. The reaction system was warmed to room temperature and stirred for 3 h. After the reaction was completed, the reaction was quenched with saturated sodium sulfite (4 mL), and the reaction mixture was extracted with ethyl acetate (25 mL × 2). The organic phases were combined, washed with saturated brine (30 mL), dried over anhydrous sodium sulfate, filtered, and concentrated to give a crude product. The crude product was purified by flash silica gel column chromatography (tetrahydrofuran/petroleum ether: 0-40%) to give compound 118-12 (200 mg, two-step yield: 60.3%) as a yellow liquid. ES-API: [M+H]⁺ = 453.1, 455.0. ¹H NMR (400 MHz, DMSO) δ 10.09 (s, 1H), 8.22 (d, *J=* 2.8 Hz, 1H), 7.13 (s, 1H), 7.00 (d, *J =* 2.8 Hz, 1H), 4.03 (dd, *J =* 12.2, 6.0 Hz, 1H), 3.62-3.55 (m, 3H), 3.43 (d, *J* = 10.8 Hz, 1H), 2.86 (s, 3H), 2.31 (d, *J* = 14.4 Hz, 1H), 2.11 (d, *J* = 14.4 Hz, 1H), 1.82 (s, 3H), 1.38 (d, *J =* 6.4 Hz, 3H), 1.17 (t, *J =* 7.2 Hz, 3H), 0.71 (s, 3H), 0.62 (s, 3H).

Step 13: Compound 118-12 (170 mg, 0.38 mmol) was dissolved in dichloromethane (5 mL), and diisopropylethylamine (145 mg, 1.13 mmol) and 1,1,1-trifluoro-N-phenyl-N-[(trifluoromethyl)sulfonyl]methanesulfonamide (201 mg, 0.56 mmol) were added. The reaction system was stirred at room temperature for 2 h. The reaction mixture was concentrated, and the crude product was purified by flash silica gel column chromatography (tetrahydrofuran/petroleum ether: 0-20%) to give compound 118-13 (165 mg, yield: 75.2%) as a colorless liquid. ES-API: [M+H]⁺ = 585.0, 587.0.

Step 14: Compound 118-13 (165 mg, 0.28 mmol) and 4-prop-2-ynyl-thiomorpholine-1,1-dioxide (98 mg, 0.56 mmol) were dissolved in acetonitrile (6 mL), and bis(triphenylphosphine)palladium(II) dichloride (49 mg, 0.07 mmol), copper(I) iodide (27 mg, 0.14 mmol), N,N'-diisopropylethylamine (182 mg, 1.41 mmol) and lithium chloride (48 mg, 1.13 mmol) were added. The reaction system was stirred at 85 °C for 1 h under argon atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by flash silica gel column chromatography (tetrahydrofuran/petroleum ether: 0-60%) to give compound 118-14 (160 mg, yield: 93.3%) as a light brown solid. ES-API: [M+H]⁺ = 608.1, 610.1.

Step 15: Compound 118-14 (160 mg, 0.26 mmol) was dissolved in tetrahydrofuran (5 mL) and water (2.5 mL), and lithium hydroxide monohydrate (33 mg, 0.79 mmol) was added at 0 °C. The reaction system was stirred at room temperature for 16 h. Methanol (2 mL) was then added, and the reaction system was further stirred at room temperature for 4 h. The reaction mixture was adjusted to pH 9 with 1 M diluted hydrochloric acid and extracted with ethyl acetate (15 mL × 2). The organic phase was dried over anhydrous sodium sulfate, filtered, and concentrated to give compound 118-15 (145 mg, yield: 97.3%) as a light brown solid. ES-API: [M+H]⁺ = 566.0, 568.1.

Step 16: Compound 118-15 (125 mg, 0.22 mmol) and compound 15-2 (182 mg, 0.33 mmol) were dissolved in toluene (3 mL), dioxane (3 mL), and water (1.5 mL), and potassium phosphate (117 mg, 0.55 mmol) and [1,1'-bis(di-tert-butylphosphino)ferrocene]dichloropalladium(II) (14 mg, 0.022 mmol) were added. The reaction system was purged 3 times with nitrogen and stirred at 85 °C for 2 h. Ethyl acetate (50 mL) and water (10 mL) were added to the reaction mixture. The separated organic phase was washed with saturated brine (15 mL), dried over anhydrous sodium sulfate, and filtered, and the filtrate was concentrated under reduced pressure. The crude product was purified by flash silica gel column chromatography (tetrahydrofuran/petroleum ether: 0-70%) to give compound 118-16 (120 mg, yield: 59.8%) as a light yellow solid. ES-API: [M+H]⁺ = 910.3.

Step 17: Compound 118-16 (120 mg, 0.132 mmol) was dissolved in tetrahydrofuran (2.5 mL) and water (0.6 mL), and lithium hydroxide (5 mg, 0.24 mmol) was added at 0 °C. The reaction system was stirred at room temperature for 1 h. The reaction mixture was adjusted to pH 6 with 1 M diluted hydrochloric acid and extracted with ethyl acetate (15 mL × 2). The organic phase was dried over anhydrous sodium sulfate and concentrated to give compound 118-17 (118 mg, yield: 99.9%) as a light yellow solid. ES-API: [M+H]⁺ = 896.2.

Step 18: Compound 118-17 (118 mg, 0.13 mmol) was dissolved in dichloromethane (12 mL), and 1-(3-dimethylaminopropyl)-3-ethylcarbodiimide hydrochloride (631 mg, 3.29 mmol), 1-hydroxybenzotriazole (89 mg, 0.66 mmol), and diisopropylethylamine (510 mg, 3.95 mmol) were added at room temperature. The reaction system was stirred at room temperature for 18 h. Dichloromethane (15 mL) was added to the reaction mixture, and the resulting mixture was washed with 0.5 M diluted hydrochloric acid (2 mL × 2) and saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-60%) to give compound 118-18 (70 mg, yield: 60.5%) as a white solid. ES-API: [M+H]⁺ = 878.2.

Step 19: Compound 118-18 (20 mg, 0.023 mmol) was dissolved in absolute methanol (0.2 mL), and a 4.0 M hydrogen chloride/dioxane solution (1.2 mL) was added at 0 °C. The reaction system was stirred at room temperature for 1 h. The reaction mixture was concentrated to give compound 118-19 (17 mg, yield: 95.9%) as a yellow solid. ES-API: [M+H]⁺ = 778.2.

Step 20: Compound 118-19 (17 mg, 0.022 mmol) was dissolved in dichloromethane (5 mL), and (1r,2R,3S)-2,3-dimethylcyclopropane-1-carboxylic acid (4 mg, 0.033 mmol), O-(7-azabenzotriazol-1-yl)-N,N,N',N'-tetramethyluronium hexafluorophosphate (13 mg, 0.033 mmol), and N,N'-diisopropylethylamine (14 mg, 0.109 mmol) were added. The reaction system was stirred at room temperature for 1 h. Water (10 mL) was added to the reaction mixture, and the resulting mixture was extracted with ethyl acetate (40 mL). The organic phase was washed with saturated brine (10 mL), dried over anhydrous sodium sulfate, and filtered, and the filtrate was concentrated under reduced pressure. The crude product was purified by prep-HPLC (ammonium bicarbonate method 1) to give (1r,2R,3S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z118, 6.3 mg, yield: 33.0%) as a white solid. ES-API: [M+H]⁺ = 874.3. ¹H NMR (400 MHz, DMSO-d₆) δ 8.72 (d, *J =* 1.6 Hz, 1H), 8.34 (d, *J=* 8.8 Hz, 1H), 7.72 (d, *J =* 1.6 Hz, 1H), 7.44 (s, 1H), 7.35 (d, *J =* 15.6 Hz, 1H), 7.13 (s, 1H), 6.81 (d, *J* = 15.6 Hz, 1H), 5.53 (t, *J =* 8.0 Hz, 1H), 5.01 (d, *J =* 12.0 Hz, 1H), 4.29 - 4.15 (m, 2H), 3.95 - 3.72 (m, 5H), 3.67 (d, *J =* 10.8 Hz, 1H), 3.53 (d, *J =* 10.8 Hz, 1H), 3.25 - 3.14 (m, 8H), 3.13 - 3.00 (m, 5H), 2.78 - 2.67 (m, 1H), 2.59 (d, *J =* 13.6 Hz, 1H), 2.23 (d, *J =* 13.6 Hz, 1H), 1.95 (d, *J =* 10.4 Hz, 1H), 1.80 - 1.57 (m, 2H), 1.52 - 1.39 (m, 1H), 1.35 (d, *J =* 6.0 Hz, 3H), 1.24 - 1.11 (m, 3H), 1.10 - 1.00 (m, 9H), 0.71 (s, 3H), 0.32 (s, 3H).

### Single-crystal culture of compound intermediate 118-12

### 3-(4-Bromo-1-ethyl-(Rₐ)-2-(5-hydroxy-2-((S)-1-methoxyethyl)pyridin-3-yl)-1H-pyrrol-3-yl)-2,2-dimethylpropyl acetate

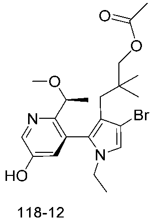

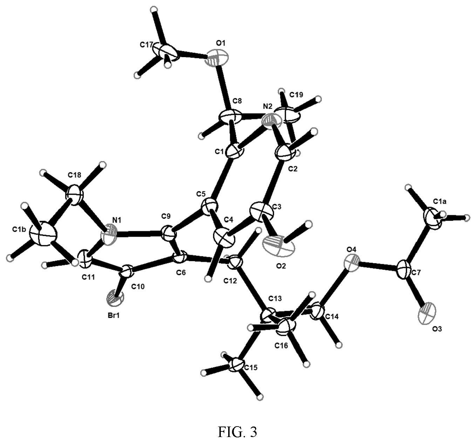

5 mg of intermediate 118-12 was dissolved in 1 mL of methanol solution, and the solution was slowly volatilized at room temperature for 24 h to give a columnar crystal. The X-ray single-crystal diffraction test was performed using a Bruker D8 Venture instrument. The results are shown in Table 3 below and FIG. 3. FIG. 3 shows an ellipsoid plot of the molecular three-dimensional structure.

### Structure analysis and refinement process:

The diffraction data were integrated and reduced using the SAINT program, followed by empirical absorption correction using the SADABS program. The single crystal structure was solved by the direct method using SHELXT2014 and refined by the least square method. For the refinement of hydrogen atoms, isotropic calculation was adopted: hydrogen atoms attached to N were located from residual electron density, while those on C-H bonds were added geometrically and refined using a riding model. The Flack constant was 0.032(19). C8 in FIG. 3 is in S configuration. The axial chirality at positions C5-C9 is in Rₐ configuration. Crystal data:

**Table 3**

| | |
|---|---|
| Molecular formula | C₂₁H₂₉BrN₂O₄ |
| Molecular weight | *Mᵣ* = 453.37 |
| Shape | Block, colorless |
| Crystal system | Hexagonal |
| Space group | *P*6₅ |
| Unit cell parameter | *a* = 22.7525 (7) Å; *c* = 7.7655 (3) Å |
| Unit cell volume | *V* = 3481.4 (3) Å³ |
| Molecule number in unit cell | *Z* = 6 |
| Unit cell dimension | 0.12 × 0.08 × 0.06 mm |
| Electron number in unit cell | *F*(000) = 1416 |
| Calculating density | *D*ₓ = 1.297 Mg m⁻³ |
| Radiation source and wavelength | Cu *K*a radiation, l = 1.54178 Å |
| Determining the number of diffraction points of unit cell | Cell parameters from 6965 reflections |
| Range for data collection | θ= 2.2-63.7° |
| Absorption coefficient | µ= 2.63 mm⁻¹ |
| Temperature | *T* = 170 K |

### Example 34. Synthesis of Compound Z119

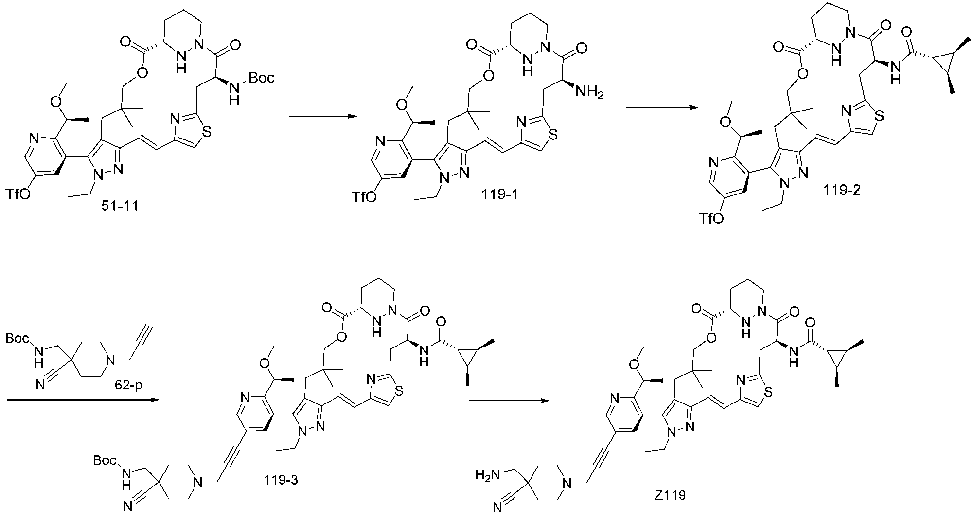

Step 1: A solution of hydrochloric acid in dioxane (2 mL, 4 M, 8.0 mmol) was added to a solution of compound 51-11 (130.0 mg, 0.152 mmol) in methanol (2 mL), and the reaction mixture was stirred at room temperature for 1.0 h. After the reaction was completed as monitored by LC-MS, the reaction system was concentrated to give crude compound 119-1 (150.0 mg, crude product). ES-API: [M+H]⁺ = 756.1.

Step 2: N,N-diisopropylethylamine (0.18 mL, 1.026 mmol) was added to a solution of compound 119-1 (150.0 mg, crude product) and (1r,2R,3S)-2,3-dimethylcyclopropane-1-carboxylic acid (34.7 mg, 0.304 mmol) in dry N,N-dimethylformamide (2.0 mL) at 0 °C, and then O-(7-azabenzotriazol-1-yl)-N,N,N',N'-tetramethyluronium hexafluorophosphate (174.0 mg, 0.456 mmol) was added. Then the reaction system was warmed to room temperature and reacted for 10 min. After the reaction was completed as monitored by LCMS, the reaction mixture was diluted with 100 mL of ethyl acetate and washed with saturated brine (30 mL × 5). The organic phase was concentrated. The crude product was purified by flash silica gel chromatography (methanol/dichloromethane = 0-2%) to give compound 119-2 (180.0 mg, crude product). ES-API: [M+H]⁺ = 852.2.

Step 3: Intermediate 62-p (305 mg, 1.10 mmol) was added to a solution of compound 119-2 (180.0 mg, crude product) in dry N,N-dimethylformamide (2.0 mL), and then bis(triphenylphosphine)palladium(II) dichloride (50.0 mg, 0.071 mmol), copper(I) iodide (30.0 mg, 0.158 mmol), N,N-diisopropylethylamine (90.0 mg, 0.696 mmol), and lithium chloride (50.0 mg, 1.180 mmol) were added sequentially. The reaction mixture was stirred at 80 °C for 1.5 h under nitrogen atmosphere. After the reaction was completed as detected by LCMS, the reaction mixture was diluted with ethyl acetate (15 mL) and washed with water (15 mL) and saturated brine (15 mL × 2). The organic phase was dried over anhydrous sodium sulfate and filtered, and the filtrate was concentrated to dryness. The crude product was purified by flash silica gel column chromatography [petroleum ether/tetrahydrofuran = 100:0-20:80 (v/v)] to give the target compound 119-3 (230.0 mg, crude product). ES-API: [M+H]⁺ = 979.3.

Step 4: Trifluoroacetic acid (2.0 mL, 26.12 mmol) was added to a solution of compound 119-3 (230.0 mg, crude product) in dichloromethane (5.0 mL) at room temperature. After 1 h of reaction, the reaction mixture was concentrated by rotary evaporation under reduced pressure to remove the solvent to give a crude product, which was purified by prep-HPLC (formic acid method 1) to give (1r,2R,3S)-N-((4²Z,8³S,2E,6S)-1⁵-(5-(3-(4-(aminomethyl)-4-cyanopiperidin-1-yl)prop-1-yn-1-yl)-2-((S)-1-methoxyethyl)pyridin-3-yl)-1¹-ethyl-12,12-dimethyl-7,9-oxo-8¹,8²,8¹,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide formate (Z119, 36.6 mg, yield: 26.03%). ES-API: [M+H]⁺ = 879.4.

### Example 35. Synthesis of Compounds Z120 and Z121

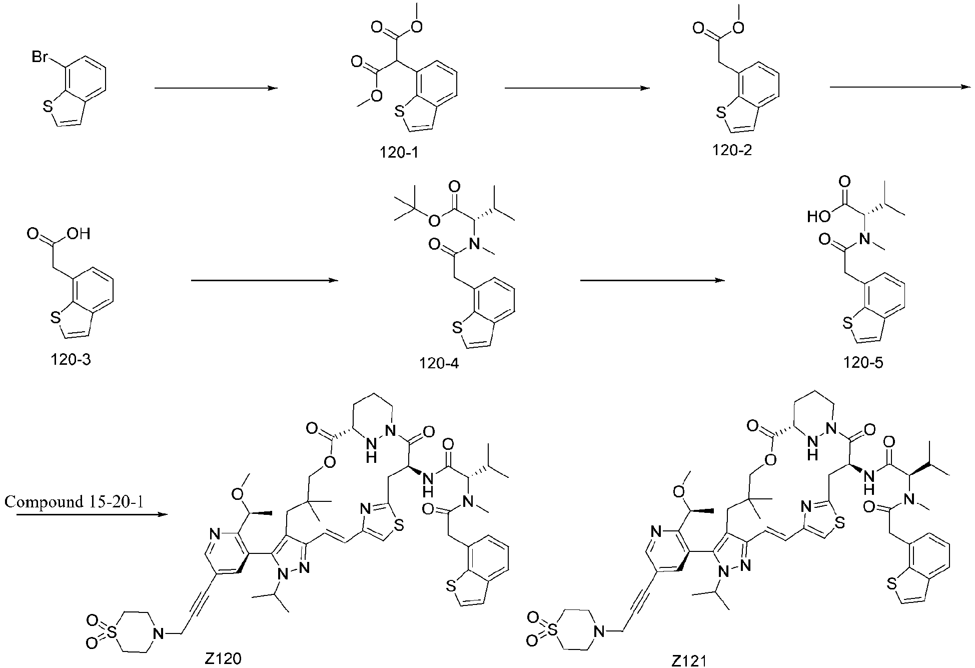

Step 1: 7-Bromobenzo[B]thiophene (4.0 g, 18.771 mmol) was dissolved in toluene (10.0 mL), and dimethyl malonate (4.96 g, 37.542 mmol), tris(dibenzylideneacetone)palladium (1.72 g, 1.877 mmol), cesium carbonate (12.23 g, 37.543 mmol), and tri-tert-butylphosphine (0.76 g, 3.754 mmol) were added. The reaction system was stirred at 90 °C for 18 h under nitrogen atmosphere. After the reaction was completed, the reaction mixture was diluted with ethyl acetate (10.0 mL) and water (5.0 mL). The organic phase was washed with saturated sodium chloride solution (10.0 mL), concentrated in vacuum, and purified by silica gel column chromatography (eluent: ethyl acetate:petroleum ether = 5%-25%) to give compound 120-1 (2.31 g, 8.740 mmol, yield: 46.56%). ES-API: [M+H]⁺ = 265.1.

Step 2: Compound 120-1 (1.786 g, 6.757 mmol) was dissolved in 2.0 mL of dimethyl sulfoxide and 8.0 mL of water, and lithium chloride (0.86 g, 20.272 mmol) was added. The system was stirred at 100 °C for 24 h. After the reaction was completed, the reaction mixture was diluted with 20.0 mL of ethyl acetate and 5.0 mL of water. The organic layer was washed with 10.0 mL of saturated sodium chloride solution, concentrated in vacuum, and purified by silica gel column chromatography (eluent: ethyl acetate:petroleum ether = 1%-32%) to give compound 120-2 (576 mg, 2.793 mmol, yield: 41.33%). ES-API: [M+H]⁺ = 207.1.

Step 3: Compound 120-2 (511 mg, 2.477 mmol) was dissolved in 6.0 mL of methanol and 0.5 mL of water, and lithium hydroxide (415.82 mg, 9.910 mmol) was added under an ice bath. The mixture was stirred at room temperature for 18 h. After the reaction was completed, the reaction mixture was adjusted to pH 6-7 with 1 M diluted hydrochloric acid and concentrated in vacuum to give crude compound 120-3 (432 mg, 2.247 mmol, yield: 90.71%). ES-API: [M+H]⁺ = 192.3.

Step 4: Compound 120-3 (401 mg, 2.086 mmol) was dissolved in 5.0 mL of dichloromethane, and tert-butyl methyl-L-valinate (468.81 mg, 2.503 mmol), O-(7-azabenzotriazol-1-yl)-N,N,N',N'-tetramethyluronium hexafluorophosphate (1586.39 mg, 4.172 mmol), and N,N-diisopropylethylamine (1.090 mL, 6.258 mmol) were added. The mixture was stirred at room temperature for 3 h. After the reaction was completed, the reaction mixture was diluted with ethyl acetate (10.0 mL × 2) and water (5.0 mL). The organic layer was washed with saturated sodium chloride solution (10.0 mL × 2), concentrated in vacuum, and purified by silica gel column chromatography (tetrahydrofuran/petroleum ether = 1%-15%) to give compound 120-4 (645 mg, 1.784 mmol, yield: 85.53%). ES-API: [M+H]⁺ = 362.2.

Step 5: Compound 120-4 (600 mg, 1.660 mmol) was dissolved in dichloromethane (10.0 mL), and trifluoroacetic acid (3.0 mL) was slowly added under an ice bath. The mixture was stirred at room temperature for 3 h. After the reaction was completed, the reaction mixture was concentrated, and the residue was repeatedly concentrated with dichloromethane to remove excess trifluoroacetic acid to give crude compound 120-5 (235 mg, 0.770 mmol, yield: 46.36%). ES-API: [M+23]⁺ = 306.1.

Step 6: Compound 120-5 (11.55 mg, 0.038 mmol) was dissolved in dichloromethane (2.0 mL), and compound 15-20-1 (15 mg, 0.019 mmol), O-(7-azabenzotriazol-1-yl)-*N*,*N*,*N*',*N*'-tetramethyluronium hexafluorophosphate (14.38 mg, 0.038 mmol), and *N*,*N*'-diisopropylethylamine (7.33 mg, 0.057 mmol) were added. The mixture was stirred at room temperature for 3 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated, and the residue was purified by prep-HPLC (ammonium bicarbonate method 1) to give 2 isomeric compounds. The structure of one of the isomeric compounds was arbitrarily designated as (2S)-2-(2-(benzo[b]thiophen-7-yl)-N-methylacetamido)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z120, 4.29 mg, 0.004 mmol, yield: 20.99%, retention time: 8.30 min).

The structure of the other isomeric compound was arbitrarily designated as (2R)-2-(2-(benzo[b]thiophen-7-yl)-N-methylacetamido)-N-((4 ²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z121, 0.67 mg, 0.001 mmol, yield: 3.26%). ES-API: [M+H]⁺ = 1080.3.

### Example 36. Synthesis of Compound Z122

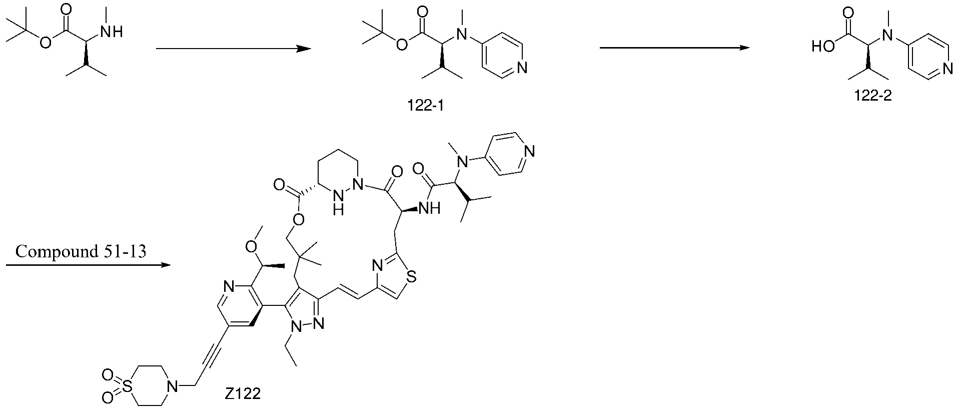

Step 1: 2-Methylpropan-2-yl N-methyl-L-valinate (100 mg, 0.534 mmol) was dissolved in dry ethylene glycol dimethyl ether (1.0 mL), and then 4-bromopyridine (168.73 mg, 1.068 mmol), dichloro[1,3-bis(2,6-di-3-pentylphenyl)imidazol-2-ylidene](3-chloropyridyl)palladium(II) (42.27 mg, 0.053 mmol), and cesium carbonate (260.96 mg, 0.801 mmol) were added. The mixture was stirred at 100 °C for 48 h under nitrogen atmosphere. After the reaction was completed, the reaction mixture was diluted with 10.0 mL and 5.0 mL of water. The organic phase was washed with 5.0 mL of a saturated sodium chloride solution, concentrated in vacuum, and purified and eluted by silica gel column chromatography (tetrahydrofuran:petroleum ether = 10%-60%) to give the target compound 122-1 (135 mg, 0.511 mmol, yield: 95.64%). ES-API: [M+H]⁺ = 265.3.

Step 2: Compound 122-1 (120 mg, 0.454 mmol) was dissolved in dichloromethane (6.0 mL), and trifluoroacetic acid (3.0 mL) was slowly added under an ice bath. The mixture was stirred at room temperature for 1 h. After the reaction was completed, the reaction mixture was concentrated, and the residue was repeatedly concentrated with dichloromethane to remove excess trifluoroacetic acid to give crude compound 122-2 (75 mg, 0.360 mmol, yield: 79.34%). ES-API: [M+23]⁺ = 209.2.

Step 3: Compound 122-2 (11.25 mg, 0.054 mmol) was dissolved in dichloromethane (2.0 mL), and compound 51-13 (21 mg, 0.027 mmol), O-(7-azabenzotriazol-1-yl)-*N*,*N*,*N*',*N*'-tetramethyluronium hexafluorophosphate (20.53 mg, 0.054 mmol), and *N*,*N*'-diisopropylethylamine (10.47 mg, 0.081 mmol) were added. The reaction system was stirred at room temperature for 2 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated, and the residue was purified by prep-HPLC (ammonium bicarbonate method 1) to give (2S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-3-methyl-2-(methyl(pyridin-4-yl)amino)butanamide (Z122, 9.29 mg, 0.008 mmol, yield: 28.44%). ES-API: [M+H]⁺ = 969.3.

### Example 37. Synthesis of Compound Z123

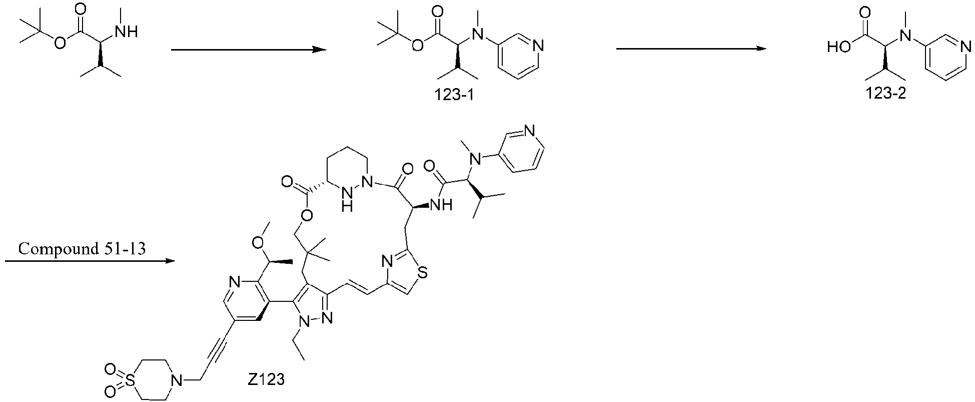

Step 1: 2-Methylpropan-2-yl N-methyl-L-valinate (200 mg, 1.068 mmol) was dissolved in dry ethylene glycol dimethyl ether (1.0 mL), and then 3-bromopyridine (337.46 mg, 2.136 mmol), dichloro[1,3-bis(2,6-di-3-pentylphenyl)imidazol-2-ylidene](3-chloropyridyl)palladium(II) (84.55 mg, 0.107 mmol), and cesium carbonate (521.92 mg, 1.602 mmol) were added. The mixture was stirred at 100 °C for 48 h under nitrogen atmosphere. After the reaction was completed, the reaction mixture was diluted with 10.0 mL and 5.0 mL of water. The organic phase was washed with 5.0 mL of a saturated sodium chloride solution, concentrated in vacuum, and purified by silica gel column chromatography (tetrahydrofuran:petroleum ether = 10%-60%) to give the target compound 123-1 (30 mg, 0.113 mmol, yield: 10.63%). ES-API: [M+H]⁺ = 265.1.

Step 2: Compound 123-1 (30 mg, 0.113 mmol) was dissolved in dichloromethane (2.0 mL), and trifluoroacetic acid (1.0 mL) was slowly added under an ice bath. The mixture was stirred at room temperature for 1 h. After the reaction was completed, the reaction mixture was concentrated, and the residue was repeatedly concentrated with dichloromethane to remove excess trifluoroacetic acid to give crude compound 123-2 (17 mg, 0.082 mmol, yield: 71.94%). ES-API: [M+23]⁺ = 209.2.

Step 3: Compound 123-2 (10.69 mg, 0.051 mmol) was dissolved in dichloromethane (2.0 mL), and compound 51-13 (20 mg, 0.026 mmol), O-(7-azabenzotriazol-1-yl)-*N,N,N',N*'-tetramethyluronium hexafluorophosphate (19.52 mg, 0.051 mmol), and *N*,*N*'-diisopropylethylamine (9.96 mg, 0.077 mmol) were added. The reaction system was stirred at room temperature for 0.5 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated, and the residue was purified by prep-HPLC (ammonium bicarbonate method 1) to give (2S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrazolocyclotridec-2-en-6-yl)-3-methyl-2-(methyl(pyridin-3-yl)amino)butanamide (Z123, 5.94 mg, 0.006 mmol, yield: 23.87%). ES-API: [M+H]⁺ = 969.2. ¹H NMR (400 MHz, DMSO-d₆) δ 8.79 (d, *J =* 1.6 Hz, 1H), 8.68 (d, *J =* 8.4 Hz, 1H), 8.35 (d, *J* = 2.8 Hz, 1H), 7.89 (d, *J =* 4.4 Hz, 1H), 7.80 (d, *J* = 2.0 Hz, 1H), 7.39 - 7.28 (m, 3H), 7.24 - 7.16 (m, 2H), 5.43 (t, *J =* 8.4 Hz, 1H), 5.06 (d, *J =* 12.0 Hz, 1H), 4.27 - 4.19 (m, 2H), 4.10 - 3.95 (m, 4H), 3.75 (s, 2H), 3.62 - 3.50 (m, 2H), 3.26 -3.21 (m, 4H), 3.20 - 3.13 (m, 4H), 3.06 - 3.00 (m, 4H), 2.89 (s, 3H), 2.81 - 2.70 (m, 1H), 2.67 - 2.60 (m, 1H), 2.26 - 2.19 (m, 1H), 2.15 - 2.07 (m, 1H), 2.05 - 1.95 (m, 1H), 1.80 - 1.65 (m, 2H), 1.55 -1.42 (m, 1H), 1.35 (d, *J* = 6.0 Hz, 3H), 1.14 (t, *J =* 7.2 Hz, 3H), 0.96 (d, *J =* 6.4 Hz, 3H), 0.85 -0.75 (m, 6H), 0.29 (s, 3H).

### Example 38. Synthesis of Compound Z124

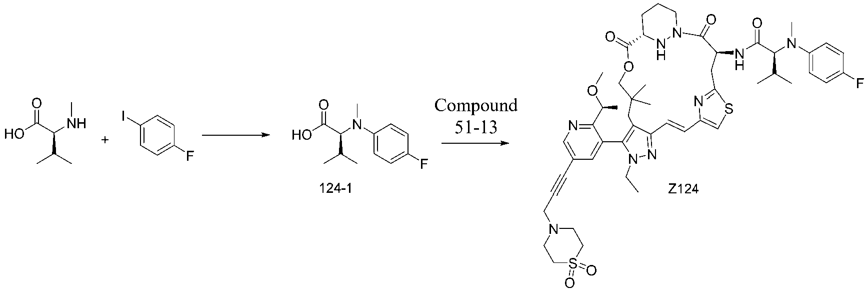

Step 1: Methyl-L-valine (1 g, 7.623 mmol) was dissolved in N,N-dimethylacetamide (20 mL), and p-fluoroiodobenzene (2.03 g, 9.148 mmol), copper(I) iodide (0.22 g, 1.143 mmol), and potassium carbonate (2.10 g, 15.246 mmol) were added. The mixture was reacted at 100 °C for 6 h under nitrogen atmosphere. After the reaction was completed, water (30.0 mL) was added to the reaction system, and the resulting mixture was extracted with ethyl acetate (50.0 mL × 2). The organic phase was washed with saturated brine (5.0 mL × 3), dried, and concentrated, and the crude product was purified by flash silica gel column chromatography (methanol/dichloromethane: 0-20%) to give compound 124-1 (0.8 g, yield: 46.59%). ES-API: [M+1]⁺= 226.1.

Step 2: Compound 51-13 (26 mg, 0.033 mmol) was dissolved in dichloromethane (1.0 mL), and compound 124-1 (12.15 mg, 0.054 mmol), O-(7-azabenzotriazol-1-yl)-*N*,*N*,*N*',*N*'-tetramethyluronium hexafluorophosphate (26.9 mg, 0.064 mmol), and *N*,*N*'-diisopropylethylamine (14.44 mg, 0.112 mmol) were added. The reaction system was stirred at room temperature for 2 h. After the reaction was completed, the reaction mixture was concentrated. Water (4.0 mL) was added to the residue, and the resulting mixture was extracted with dichloromethane (8.0 mL × 2). The organic phase was washed with saturated brine (5.0 mL × 3), dried, and concentrated, and the crude product was purified by prep-HPLC (ammonium bicarbonate method 2) to give (2S)-N-((4²Z,8³S,2E,6S)-(Rₐ)-1⁵-(5-(3-(1,1-dioxidomorpholinyl)prop-1-yn-1-yl)-2-((S)-1-methoxyethyl)pyridin-3-yl)-1¹-ethyl-12,12-dimethyl-7,9-dioxo-8¹,8²,8⁴,8⁵,8⁶ -hexahydro-1¹H-10-oxa-4(4,2)-thiazola-8(1,3)-pyridazina-1(3,4)-pyrrolacyclotridecaphan-2-en-6-yl)-2-[(4-fluorophenyl)(methyl)amino]-3-methylbutanamide (Z124, 5 mg, yield: 18.79%). ES-API: [M+H]⁺ = 986.4. ¹H NMR (400 MHz, DMSO-d₆) δ = 8.79 (d, J=4.0, 1H), 8.58 (d, J=8.0, 1H), 7.80 (d, J=4.0, 1H), 7.36 (s, 1H), 7.30 (s, 1H), 7.25-7.18 (m, 1H), 7.05-7.01 (m, 2H), 6.98-6.90 (m, 2H), 5.42 (t, J=8.0, 1H), 5.04 (d, J=12.0, 1H), 4.30-4.20 (m, 2H), 4.10-4.02 (m, 3H), 3.87 (d, J=8.0, 1H), 3.75 (s, 2H), 3.60-3.55 (m, 2H), 3.28-3.23 (m, 4H), 3.20-3.10 (m, 4H), 3.08-2.98 (m, 4H), 2.84 (s, 3H), 2.80-2.70 (m, 1H), 2.68-2.60 (m, 1H), 2.17-2.10 (m, 2H), 2.05-1.95 (m, 1H), 1.80-1.72 (m, 2H), 1.55-1.47 (m, 1H), 1.35 (d, J=8.0, 3H), 1.27-1.22 (m, 1H), 1.14 (t, J=8.0, 3H), 0.95 (d, J=8.0, 3H), 0.85-0.78 (m, 6H), 0.29 (s, 3H).

### Example 39. Synthesis of Compound Z125

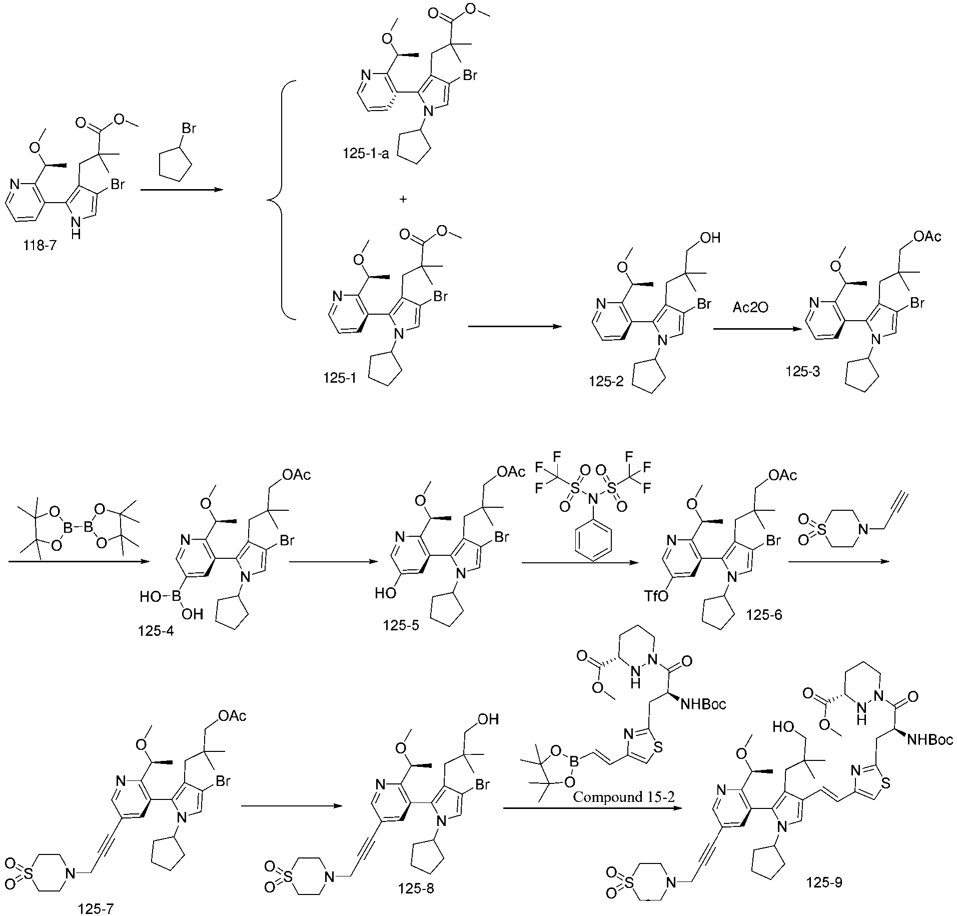

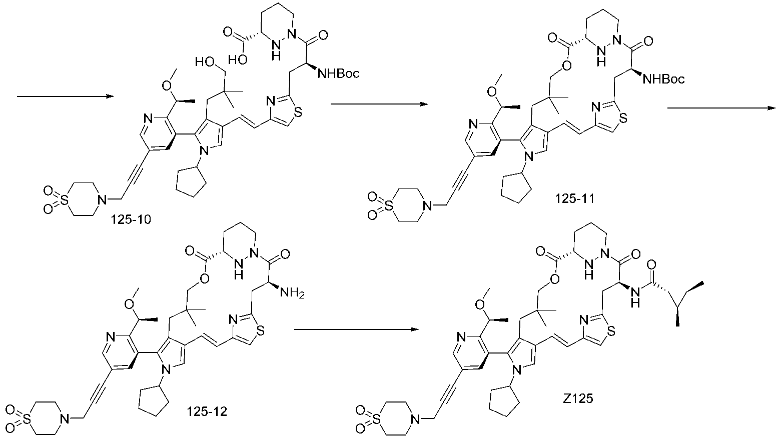

Step 1: Compound 118-7 (500 mg, 1.27 mmol) was dissolved in N,N-dimethylformamide (15 mL), and cesium carbonate (1.24 g, 3.80 mmol) and bromocyclopentane (471 mg, 3.16 mmol) were added at room temperature. The reaction system was stirred at 85 °C for 48 h. Ethyl acetate (60 mL) was added to the reaction mixture, and the resulting mixture was washed with saturated brine (30 mL × 3). The organic phase was dried over anhydrous sodium sulfate and filtered, and the filtrate was concentrated under reduced pressure. The crude product was purified by flash silica gel column chromatography (tetrahydrofuran/petroleum ether: 0-30%) to give the target compound 125-1 (150 mg, 3 min, LCMS retention time: 2.05 min, yield: 25.6%, structure arbitrarily designated) as a yellow liquid, ES-API: [M+H]⁺ = 463.1, 465.1, and isomer 125-1-a (120 mg, 3 min, LCMS retention time 1.92 min, yield: 20.5%, structure arbitrarily designated) as a yellow liquid. ES-API: [M+H]⁺ = 463.1, 465.1.

Step 2: Compound 125-1 (150 mg, 0.32 mmol) was dissolved in dichloromethane (8 mL), and a 1.0 M solution of DIBAL-H in n-hexane (0.65 mL, 0.65 mmol) was added dropwise at -70 °C. The reaction system was stirred at -70 °C for 1 h, and then warmed to room temperature and stirred for 1 h. The reaction mixture was cooled to 0 °C, and water (0.10 mL), a 15% sodium hydroxide solution (0.10 mL), and water (0.25 mL) were slowly added dropwise sequentially to quench the reaction. The reaction mixture was stirred at room temperature for 15 min, dried over anhydrous magnesium sulfate, and filtered, and the filtrate was concentrated under reduced pressure. The crude product was purified by flash silica gel column chromatography (tetrahydrofuran/petroleum ether: 0-35%) to give compound 125-2 (90 mg, yield: 63.9%) as a white solid. ES-API: [M+H]⁺ = 435.1, 437.1.

Step 3: Compound 125-2 (150 mg, 0.35 mmol), diisopropylethylamine (134 mg, 1.03 mmol), and 4-dimethylaminopyridine (5 mg, 0.04 mmol) were dissolved in dichloromethane (8 mL), and acetic anhydride (70 mg, 0.69 mmol) was added at 0 °C. The reaction system was stirred at 0 °C for 30 min. The reaction mixture was concentrated, and the crude product was purified by flash silica gel column chromatography (tetrahydrofuran/petroleum ether: 0-20%) to give the target compound 125-3 (140 mg, yield: 85.1%) as a light yellow viscous liquid. ES-API: [M+H]⁺= 477.1, 479.1.

Step 4: Compound 125-3 (140 mg, 0.29 mmol), bis(pinacolato)diboron (149 mg, 0.59 mmol), chloro(1,5-cyclooctadiene)iridium(I) dimer (24 mg, 0.035 mmol), and 4,4'-di-tert-butyl-2,2'-bipyridine (16 mg, 0.059 mmol) were dissolved in cyclohexane (10 mL). The reaction system was stirred at 85°C for 18 h under nitrogen atmosphere. The reaction mixture was concentrated under reduced pressure to give crude compound 125-4 (152 mg, crude product). ES-API: [M+H]⁺ = 521.1, 523.1.

Step 5: Compound 125-4 (152 mg, crude product) was dissolved in acetonitrile (5 mL), and 30% hydrogen peroxide (100 mg, 0.88 mmol) was added under an ice bath. The reaction system was warmed to room temperature and stirred for 3 h. After the reaction was completed, the reaction was quenched with saturated sodium sulfite (4 mL). The reaction mixture was extracted with ethyl acetate (25 mL × 2). The organic phases were combined, washed with saturated brine (30 mL), dried, and concentrated to give a crude product. The crude product was purified by flash silica gel column chromatography (tetrahydrofuran/petroleum ether: 0-40%) to give compound 125-5 (75 mg, two-step yield: 52.1%) as a yellow solid. ES-API: [M+H]⁺ = 493.1,495.1.

Step 6: Compound 125-5 (75 mg, 0.15 mmol) was dissolved in dichloromethane (5 mL), and diisopropylethylamine (59 mg, 0.46 mmol) and 1,1,1-trifluoro-N-phenyl-N-[(trifluoromethyl)sulfonyl]methanesulfonamide (81 mg, 0.23 mmol) were added. The reaction system was stirred at room temperature for 2 h. The reaction mixture was concentrated. The crude product was purified by flash silica gel column chromatography (tetrahydrofuran/petroleum ether: 0-15%) to give the desired product, compound 125-6 (70 mg, yield: 73.6%) as a light yellow liquid. ES-API: [M+H]⁺ = 625.0,627.0. Step 7: Compound 125-6 (70 mg, 0.11 mmol) and 4-prop-2-ynyl-thiomorpholine-1,1-dioxide (39 mg, 0.22 mmol) were dissolved in acetonitrile (3 mL), and bis(triphenylphosphine)palladium(II) dichloride (20 mg, 0.028 mmol), copper(I) iodide (11 mg, 0.056 mmol), N,N'-diisopropylethylamine (72 mg, 0.56 mmol), and lithium chloride (19 mg, 0.45 mmol) were added. The reaction system was stirred at 85 °C for 1 h under argon atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by flash silica gel column chromatography (tetrahydrofuran/petroleum ether: 0-60%) to give the target compound 125-7 (70 mg, yield: 96.4%) as a light brown solid. ES-API: [M+H]⁺ = 648.1, 650.1.

Step 8: Compound 125-7 (70 mg, 0.108 mmol) was dissolved in tetrahydrofuran (3 mL), methanol (1 mL), and water (1.5 mL), and lithium hydroxide monohydrate (14 mg, 0.324 mmol) was added at 0 °C. The reaction system was stirred at room temperature for 16 h. The reaction mixture was adjusted to pH 9 with 1 M diluted hydrochloric acid and extracted with ethyl acetate (15 mL × 2). The organic phase was dried over anhydrous sodium sulfate and concentrated to give the target compound 125-8 (65 mg, yield: 99.3%) as a light brown solid. ES-API: [M+H]⁺ = 606.0, 608.1.

Step 9: Compound 125-8 (65 mg, 0.107 mmol) and compound 15-2 (88 mg, 0.161 mmol) were dissolved in toluene (2 mL), dioxane (2 mL), and water (1 mL), and potassium phosphate (57 mg, 0.267 mmol) and [1,1'-bis(di-tert-butylphosphino)ferrocene]dichloropalladium(II) (7 mg, 0.011 mmol) were added. The reaction system was purged 3 times with nitrogen and stirred at 85°C for 2 h. Ethyl acetate (50 mL) and water (10 mL) were added to the reaction mixture. The separated organic phase was washed with saturated brine (15 mL), dried over anhydrous sodium sulfate, and filtered, and the filtrate was concentrated under reduced pressure. The crude product was purified by flash silica gel column chromatography (tetrahydrofuran/petroleum ether: 0-70%) to give the target compound 125-9 (60 mg, yield: 58.9%) as a light brown solid. ES-API: [M+H]⁺ = 950.3.

Step 10: Compound 125-9 (60 mg, 0.063 mmol) was dissolved in tetrahydrofuran (2 mL) and water (0.5 mL), and lithium hydroxide (3 mg, 0.126 mmol) was added at 0 °C. The reaction system was stirred at room temperature for 1 h. The reaction mixture was adjusted to pH 6 with 1 M diluted hydrochloric acid and extracted with ethyl acetate (15 mL × 2). The organic phase was dried over anhydrous sodium sulfate and concentrated to give the target compound 125-10 (59 mg, yield: 99.8%) as a light yellow solid. ES-API: [M+H]⁺ = 936.3.

Step 11: Compound 125-10 (59 mg, 0.063 mmol) was dissolved in dichloromethane (12 mL), and 1-(3-dimethylaminopropyl)-3-ethylcarbodiimide hydrochloride (302 mg, 1.576 mmol), 1-hydroxybenzotriazole (43 mg, 0.315 mmol), and diisopropylethylamine (244 mg, 1.891 mmol) were added at room temperature. The reaction system was stirred at room temperature for 18 h. Dichloromethane (15 mL) was added to the reaction mixture, and the resulting mixture was washed with 0.5 M diluted hydrochloric acid (2 mL × 2) and saturated brine (10 mL), dried over anhydrous sodium sulfate, and filtered, and the filtrate was concentrated under reduced pressure. The crude product was purified by flash silica gel column chromatography (tetrahydrofuran/petroleum ether: 0-70%) to give the target compound 125-11 (35 mg, yield: 60.5%) as a light yellow solid. ES-API: [M+H]⁺ = 918.3.

Step 12: Compound 125-11 (35 mg, 0.038 mmol) was dissolved in absolute methanol (0.3 mL), and a 4.0 M hydrogen chloride/dioxane solution (1.5 mL) was added at 0 °C. The reaction system was stirred at room temperature for 1 h. The reaction mixture was concentrated to give the target compound 125-12 (31 mg, yield: 99.1%) as a yellow solid. ES-API: [M+H]⁺ = 818.2.

Step 13: Compound 125-12 (31 mg, 0.038 mmol) was dissolved in dichloromethane (6 mL), and (1r,2R,3S)-2,3-dimethylcyclopropane-1-carboxylic acid (6 mg, 0.052 mmol), O-(7-azabenzotriazol-1-yl)-N,N,N',N'-tetramethyluronium hexafluorophosphate (17 mg, 0.045 mmol), and N,N'-diisopropylethylamine (24 mg, 0.189 mmol) were added. The reaction system was stirred at room temperature for 1 h. Dichloromethane (20 mL) was added to the reaction mixture. The resulting mixture was washed sequentially with water (5 mL) and saturated brine (10 mL), dried over anhydrous sodium sulfate, and filtered. The filtrate was concentrated under reduced pressure. The crude product was purified by prep-HPLC (ammonium bicarbonate method 1) to give the desired product (1r,2R,3S)-N-((4²Z,8³S,2E,6S)-1¹-cyclopentyl-(Rₐ)-1⁵-(5-(3-(1,1-dioxidothiomorpholino)prop-1-yn-1-yl)-2-((S)-1-methoxyethyl)pyridin-3-yl)-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolycyclotridec-2-en-6-yl)-2,3-dimethylcyclopropane-1-carboxamide (Z125, 17 mg, yield: 49.1%) as a white solid. ES-API: [M+H]⁺ = 914.3. ¹H NMR (400 MHz, DMSO-d₆) δ 8.72 (d, *J =* 1.6 Hz, 1H), 8.33 (d, *J =* 8.8 Hz, 1H), 7.66 (d, *J =* 1.6 Hz, 1H), 7.48 (s, 1H), 7.34 (d, *J =* 15.6 Hz, 1H), 7.10 (s, 1H), 6.89 (d, *J =* 15.6 Hz, 1H), 5.54 (t, *J =* 8.4 Hz, 1H), 5.01 (d, *J =* 12.0 Hz, 1H), 4.37 - 4.16 (m, 3H), 3.90 (t, *J =* 11.2 Hz, 1H), 3.74 (s, 2H), 3.67 (d, *J =* 10.8 Hz, 1H), 3.53 (d, *J =* 10.8 Hz, 1H), 3.24 - 2.98 (m, 13H), 2.72 (t, *J =* 11.2 Hz, 1H), 2.57 (d, *J =* 14.0 Hz, 1H), 2.27 - 1.99 (m, 3H), 1.98 - 1.80 (m, 2H), 1.74 (m, 1H), 1.70 - 1.32 (m, 9H), 1.27 - 1.09 (m, 4H), 1.08 - 0.98 (m, 6H), 0.70 (s, 3H), 0.32 (s, 3H).

### Example 40. Synthesis of Compound Z127

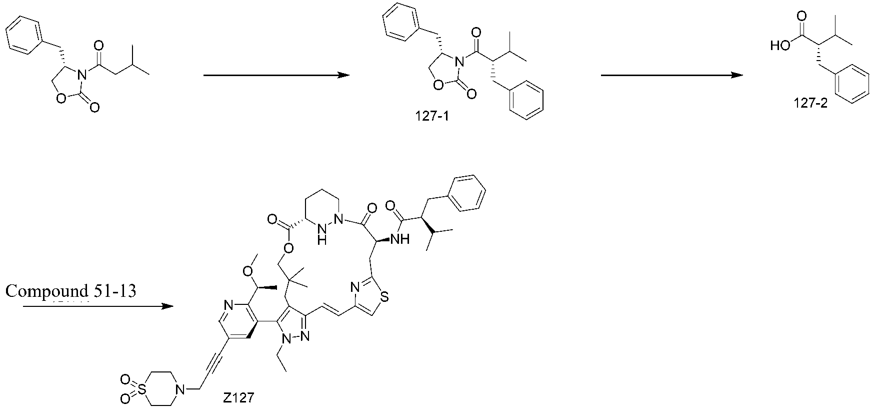

Step 1: (S)-4-Benzyl-3-(3-methylbutanoyl)oxazolidin-2-one (2.0 g, 7.653 mmol) was dissolved in tetrahydrofuran (8.0 mL), and the solution was cooled to -78 °C. Lithium hexamethyldisilazane (9.184 mL, 9.184 mmol) was slowly added dropwise, and the resulting mixture was stirred at -78 °C for 45 min and then stirred at room temperature for 15 min. Subsequently, a solution of benzyl bromide (1.273 mL, 10.715 mmol) in tetrahydrofuran (8.0 mL) was added dropwise at -78 °C, and the mixture was stirred at 0 °C for 3 h. After the reaction was completed, the reaction was quenched with 5.0 mL of saturated aqueous ammonium chloride solution, and the resulting mixture was extracted with ethyl acetate (10.0 mL × 3). The organic layer was washed with saturated brine, dried over sodium sulfate, filtered, and concentrated, and the residue was purified by flash silica gel column chromatography (tetrahydrofuran:petroleum ether = 0-70%) to give compound 127-1 (2.1 g, 5.975 mmol, yield: 78.07%). ES-API: [M+H]⁺ = 352.2.

Step 2: Compound 127-1 (200 mg, 0.569 mmol) was dissolved in a mixture of 4.0 mL of tetrahydrofuran and 0.5 mL of water, and the solution was cooled to 0-5 °C. Hydrogen peroxide (0.970 mL, 0.854 mmol) was slowly added dropwise, and then a 1 mol/L aqueous lithium hydroxide solution (0.854 mL, 0.854 mmol) was slowly added dropwise. The mixture was stirred at 20-25 °C for 18 h. After the reaction was completed, 1.0 mL of saturated sodium sulfite solution was slowly added dropwise. The aqueous layer was extracted twice with 10.0 mL of ethyl acetate. The combined organic layers were dried and evaporated under reduced pressure to give a residue. The residue was dissolved in dichloromethane, and the desired product was extracted with an aqueous lithium hydroxide monohydrate solution. The pH of the aqueous lithium hydroxide solution was adjusted to 2.5-3.0 with diluted hydrochloric acid. The aqueous layer was extracted with dichloromethane, dried over sodium sulfate, and concentrated in vacuum, and the residue was purified by silica gel column chromatography (tetrahydrofuran:petroleum ether = 20%-60%) to give the target compound 127-2 (71 mg, 0.369 mmol, yield: 64.89%). ES-API: [M+23]⁺ = 192.3.

Step 3: Compound 127-2 (10.37 mg, 0.054 mmol) was dissolved in dichloromethane (2.0 mL), and compound 51-13 (21 mg, 0.027 mmol), O-(7-azabenzotriazol-1-yl)-*N,N,N',N'*-tetramethyluronium hexafluorophosphate (20.50 mg, 0.054 mmol), and *N,N'*-diisopropylethylamine (10.45 mg, 0.081 mmol) were added. The reaction system was stirred at room temperature for 1.0 h. After the reaction was completed, the reaction mixture was concentrated. Water (4.0 mL) was added to the residue, and the resulting mixture was extracted with ethyl acetate (8.0 mL × 2). The organic phase was washed with saturated brine (5.0 mL × 3), dried, and concentrated, and the residue was purified by prep-HPLC (formic acid method 1) to give (2S)-2-benzyl-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-methylbutanamide (Z127, 7.35 mg, 0.008 mmol, yield: 28.60%). ES-API: [M+H]⁺ = 953.3. ¹H NMR (400 MHz, DMSO-d₆) δ 8.80 (d, *J* = 1.2 Hz, 1H), 8.13 (d, *J =* 8.4 Hz, 1H), 7.80 (d, *J =* 1.6 Hz, 1H), 7.35 - 7.27 (m, 2H), 7.25 - 7.14 (m, 6H), 5.31 (t, *J* = 8.4 Hz, 1H), 5.02 (d, *J* = 12.4 Hz, 1H), 4.27 (q, *J =* 6.0 Hz, 1H), 4.21 - 4.17 (m, 1H), 4.09 - 3.96 (m, 3H), 3.76 (s, 2H), 3.61 - 3.50 (m, 2H), 3.26 (s, 3H), 3.18 - 3.14 (m, 4H), 3.06 - 3.01 (m, 4H), 2.88 - 2.64 (m, 4H), 2.15 - 2.07 (m, 1H), 2.01 - 1.97 (m, 1H), 1.81 - 1.74 (m, 2H), 1.70 -1.60 (m, 1H), 1.52 -1.40 (m, 1H), 1.36 (d, *J =* 6.0 Hz, 3H), 1.15 (t, *J =* 7.2 Hz, 3H), 1.01 - 0.93 (m, 6H), 0.81 (s, 3H), 0.29 (s, 3H).

### Example 41. Synthesis of Compound Z128

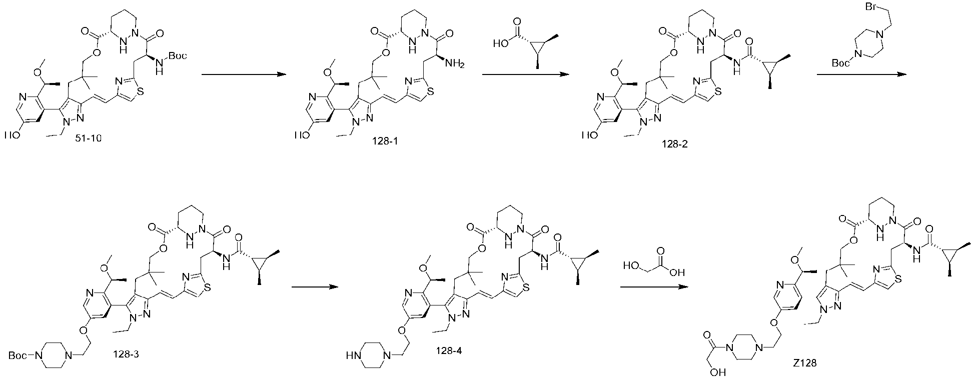

Step 1: Compound 51-10 (100 mg, 0.138 mmol) was dissolved in methanol (0.5 mL), and a solution of hydrogen chloride in dioxane (4 *M,* 2 mL) was added to the above solution. After the addition was completed, the reaction system was stirred at room temperature for 1 h. The reaction mixture was concentrated to give compound 128-1 (86 mg, crude product) as a light yellow solid, ES-API: [M+H]⁺ = 624.1.

Step 2: Compound 128-1 (86 mg, crude product) was dissolved in dichloromethane (4 mL). (1*r*,2*R*,3*S*)-2,3-Dimethylcyclopropane-1-carboxylic acid (17.31 mg, 0.152 mmol), O-(7-azabenzotriazol-1-yl)-*N,N,N',N'-*tetramethyluronium hexafluorophosphate (68.15 mg, 0.179 mmol), and *N,N'*-diisopropylethylamine (53.46 mg, 0.414 mmol) were added to the above solution. After the addition was completed, the reaction system was stirred at room temperature for 24 h. After the reaction was completed, the reaction mixture was concentrated to remove dichloromethane. Water (20 mL) was added to the residue, and the resulting mixture was extracted with dichloromethane (15 mL × 2). The organic phase was washed with saturated brine (20 mL × 2), dried, and concentrated to give a crude product. The crude product was purified by flash silica gel column chromatography (methanol/dichloromethane: 0%-3%) to give compound 128-2 (70 mg, 0.097 mmol, two-step yield: 70.53%) as a white solid. ES-API: [M+H]⁺ = 720.3.

Step 3: Compound 128-2 (60 mg, 0.083 mmol) was dissolved in acetonitrile (3 mL), and potassium carbonate (34.55 mg, 0.250 mmol), potassium iodide (13.84 mg, 0.083 mmol), and tert-butyl 4-(2-bromoethyl)piperazine-1-carboxylate (73.31 mg, 0.250 mmol) were added to the above solution. After the addition was completed, the reaction system was stirred at 70 °C for 17 h under nitrogen atmosphere. After the reaction was completed, water (10 mL) was added to the reaction mixture, and the resulting mixture was extracted with ethyl acetate (10 mL × 3). The organic phase was washed with saturated brine (20 mL × 2), dried, and concentrated to give a crude product. The crude product was purified by flash silica gel column chromatography (ethyl acetate/petroleum ether: 0%-40%) to give compound 128-3 (60 mg, 0.064 mmol, yield: 77.55%) as a light yellow solid. ES-API: [M+H]⁺ = 932.3.

Step 4: Compound 128-3 (30 mg, 0.032 mmol) was dissolved in methanol (0.5 mL), and a solution of hydrogen chloride in dioxane (4 *M,* 1 mL) was added to the above solution. After the addition was completed, the reaction system was stirred at room temperature for 1 h. The reaction mixture was concentrated to give compound 128-4 (27 mg, crude product) as a light yellow solid, ES-API: [M+H]⁺ = 832.2.

Step 5: Compound 128-4 (27 mg, crude product) was dissolved in dichloromethane (2 mL). 2-Hydroxyacetic acid (3.7 mg, 0.049 mmol), O-(7-azabenzotriazol-1-yl)-*N,N,N',N'*-tetramethyluronium hexafluorophosphate (15 mg, 0.04 mmol), and *N,N'*-diisopropylethylamine (13 mg, 0.1 mmol) were added to the above solution. After the addition was completed, the reaction system was stirred at room temperature for 2 h. After the reaction was completed, the reaction mixture was concentrated to remove dichloromethane. Water (10 mL) was added to the residue, and the resulting mixture was extracted with ethyl acetate (15 mL × 2). The organic phase was washed with saturated brine (10 mL × 3), dried, and concentrated to give a crude product. The crude product was purified by prep-HPLC (ammonium bicarbonate method 1) to give (1*r,*2*R*,3*S*)*-N-*((4⁷*Z*,8³*S*,2*E*,6*S*)-1'-ethyl-1⁵-(Rₐ)-(5-(2-(4-(2-hydroxyacetyl)piperazin-1-yl)ethoxy)-2-((*S*)-1-methoxyethyl)pyridin-3-yl)-12,12-dimethyl-7,9-dioxo-8¹,8²,8³,8⁴,8⁵,8⁶-hexahydro-1¹ *H*-10-oxa-4(4,2)-thiazola-8(1,3)-pyridazina-1(3,4)-pyrrolacyclotridecaphan-2-en-6-yl)-2,3-dimethylcyclopropane-1-carboxamide (Z128, 6 mg, 0.007 mmol, two-step yield: 21%) as a white solid. ES-API: [M+H]⁺ = 890.5.

### Example 42. Synthesis of Compound Z73

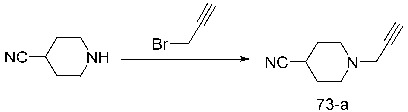

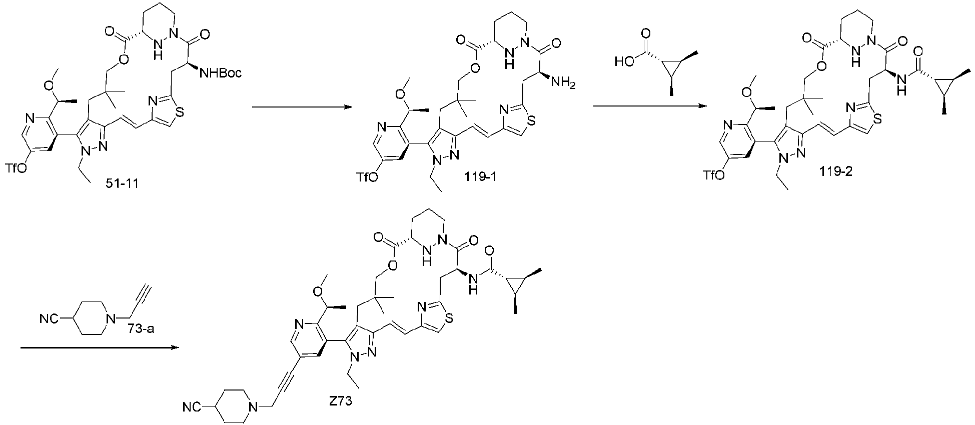

Step 1: 4-Piperidinecarbonitrile (1000 mg, 9.078 mmol) was dissolved in acetonitrile (15 mL), and potassium carbonate (2509.16 mg, 18.156 mmol) and 3-bromoprop-1-yne (1079.88 mg, 9.078 mmol) were added. The mixture was reacted at room temperature for 3 h. After the reaction was completed, water (50 mL) and ethyl acetate (50 mL) were added. The organic layer was separated, washed with a saturated sodium chloride solution (50 mL × 3), dried over anhydrous sodium sulfate, filtered, and concentrated. The residue was purified by silica gel column chromatography to give compound 73-a (1000 mg, 6.747 mmol, yield: 74.33%). ES-API: [M+H]⁺ = 149.1.

Step 2: A hydrogen chloride/dioxane solution (7.5 mL, 4 M) was added to a solution of compound 51-11 (250 mg, 0.292 mmol) in methanol (1.5 mL). The reaction mixture was stirred at room temperature for 1 h. After the reaction was completed as detected by LCMS, the reaction mixture was concentrated under reduced pressure to give crude compound 119-1 (220 mg, yield: 99%). ES-API: [M+H]⁺ = 756.1.

Step 3: (1r,2R,3S)-2,3-Dimethylcyclopropane-1-carboxylic acid (43.19 mg, 0.378 mmol), N,N-diisopropylethylamine (0.3 mL, 1.746 mmol), and O-(7-azabenzotriazol-1-yl)-N,N,N',N'-tetramethyluronium hexafluorophosphate (144 mg, 0.378 mmol) were added sequentially to a solution of compound 119-1 (220 mg, 0.291 mmol) in dichloromethane (5 mL) under an ice-water bath. The reaction mixture was warmed to room temperature and stirred for 30 min. After the reaction was completed as detected by LCMS, the reaction mixture was diluted with dichloromethane (20 mL), and then the resulting mixture was washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-40%) to give compound 119-2 (170 mg, yield: 69 %). ES-API: [M+H]⁺ = 852.2.

Step 4: Bis(triphenylphosphine)palladium(II) dichloride (11.5 mg, 0.016 mmol), copper(I) iodide (6 mg, 0.033 mmol), N,N-diisopropylethylamine (0.06 mL, 0.329 mmol), and lithium chloride (21 mg, 0.493 mmol) were added to a solution of compounds 119-2 (70 mg, 0.082 mmol) and 73-a (37 mg, 0.246 mmol) in acetonitrile (10 mL) under nitrogen atmosphere. The reaction mixture was purged 3 times with nitrogen, heated to 85 °C, and stirred for 2 h. After the reaction was completed as detected by LCMS, the reaction mixture was cooled to room temperature, and 20 mL of water was added to the reaction mixture. The reaction mixture was then extracted with ethyl acetate (10 mL × 3). The organic phases were combined, washed with saturated brine (10 mL × 3), dried over anhydrous sodium sulfate, filtered, and concentrated to dryness. The residue was purified by prep-HPLC (ammonium bicarbonate method 2) to give (1r,2R,3S)-N-((4²Z,8³S,2E,6S)-(Rₐ)-1⁵-(5-(3-(4-cyanopiperidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z73, 32.05 mg, yield: 46%). ES-API: [M+H]⁺ = 850.3. ¹H NMR (400 MHz, DMSO-d₆) δ 8.79 (d, J= 2.0 Hz, 1 H), 8.36 (d, J= 8.8 Hz, 1 H), 7.79 (d, J= 2.0 Hz, 1 H), 7.44 (s, 1 H), 7.36 (d, J= 15.6 Hz, 1 H), 7.25 (d, J= 15.2 Hz, 1 H), 5.54 (t, J= 8.8 Hz, 1 H), 5.03 (d, J= 12.0 Hz, 1 H), 4.27- 4.20 (m, 2 H), 4.04- 3.96 (m, 3 H), 3.63-3.54 (m, 4 H), 3.25- 3.22 (m, 4 H), 3.14- 3.08 (m, 1 H), 2.91- 2.85 (m, 1 H), 2.76- 2.59 (m, 4 H), 2.47- 2.45 (m, 2 H), 2.20- 2.17 (m, 1 H), 1.99- 1.96 (m, 1 H), 1.92- 1.87 (m, 2 H), 1.78- 1.64 (m, 4 H), 1.51- 1.45 (m, 1 H), 1.36 (d, J= 6.4 Hz, 3 H), 1.18- 1.12 (m, 6 H), 1.07- 1.04 (m, 6 H), 0.77 (s, 3 H), 0.33 (s, 3 H).

### Example 43. Synthesis of Compounds Z80 and Z191

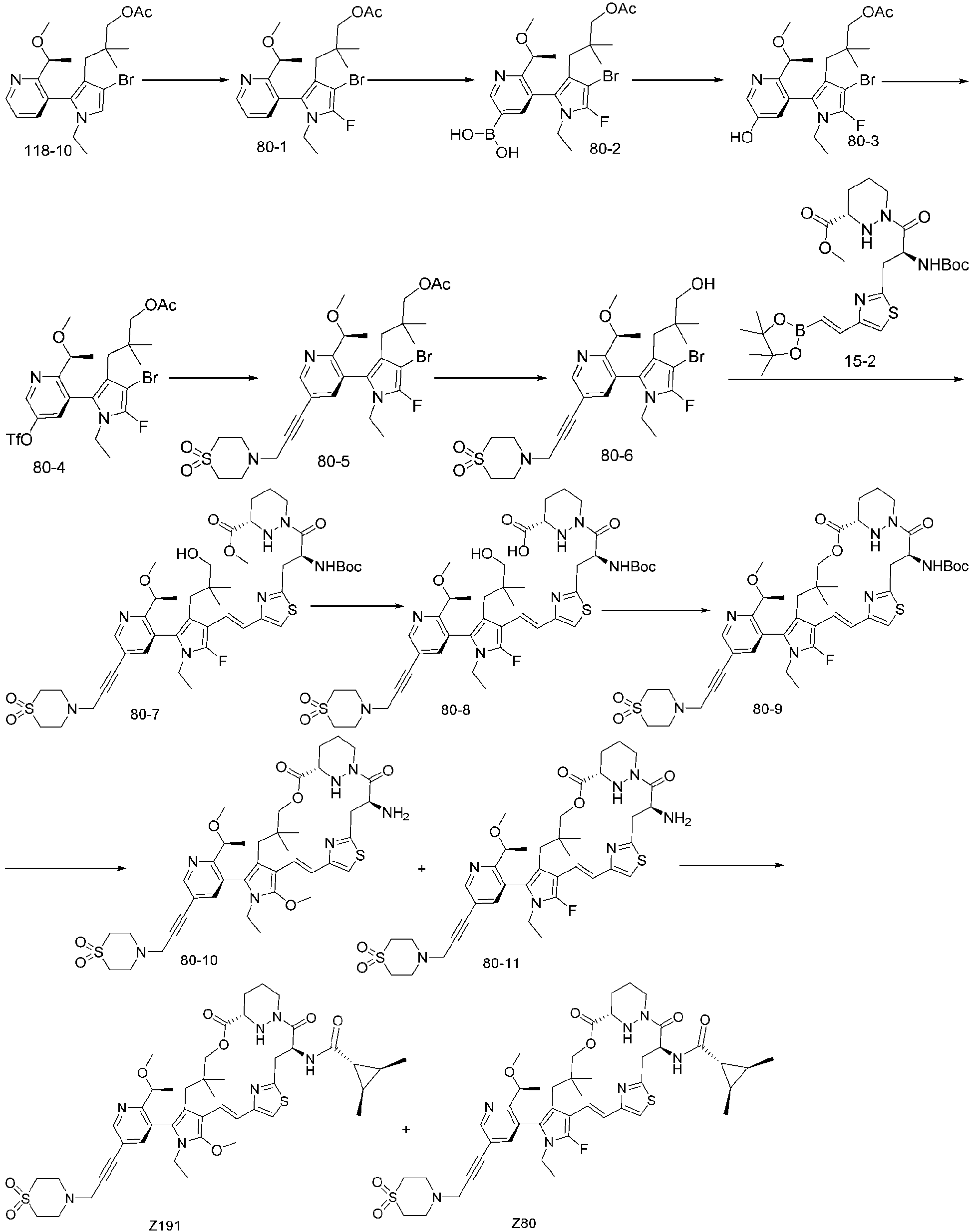

Step 1: Compound 118-10 (160 mg, 0.37 mmol) was dissolved in acetonitrile (12 mL), and 1-chloromethyl-4-fluoro-1,4-diazabicyclo[2.2.2]octane bis(tetrafluoroborate) (142 mg, 0.40 mmol) was added at 0 °C. The reaction system was stirred at 0 °C for 1 h. A saturated sodium bicarbonate solution (4 mL) was added to the reaction mixture, and the resulting mixture was extracted with ethyl acetate (40 mL). The organic phase was washed with saturated brine (15 mL), dried over anhydrous sodium sulfate, and filtered, and the filtrate was concentrated under reduced pressure. The crude product was purified by flash silica gel column chromatography (tetrahydrofuran/petroleum ether: 0-20%) to give compound 80-1 (75 mg, yield: 45.02%) as a light yellow liquid. ES-API: [M+H]⁺= 455.1, 457.1.

Step 2: Compound 80-1 (75 mg, 0.165 mmol), bis(pinacolato)diboron (84 mg, 0.329 mmol), chloro(1,5-cyclooctadiene)iridium(I) dimer (13 mg, 0.020 mmol), and 4,4'-di-tert-butyl-2,2'-bipyridine (9 mg, 0.033 mmol) were dissolved in cyclohexane (8 mL). The reaction system was stirred at 85°C for 18 h under nitrogen atmosphere. The reaction mixture was concentrated to give compound 80-2 (82 mg, crude product). ES-API: [M+H]⁺ = 499.1, 501.1.

Step 3: Compound 80-2 (82 mg, crude product) was dissolved in acetonitrile (5 mL), and 30% hydrogen peroxide (99 mg, 0.875 mmol) was added under an ice bath. The reaction system was warmed to room temperature and stirred for 3 h. After the reaction was completed, the reaction was quenched with saturated sodium sulfite (4 mL). The reaction mixture was extracted with ethyl acetate (25 mL × 2). The organic phases were combined, washed with saturated brine (30 mL), dried over anhydrous sodium sulfate, filtered, and concentrated to give a crude product. The crude product was purified by flash silica gel column chromatography (tetrahydrofuran/petroleum ether: 0-40%) to give compound 80-3 (35 mg, two-step yield: 45.2%) as a yellow liquid. ES-API: [M+H]⁺ = 471.1,473.1.

Step 4: Compound 80-3 (35 mg, 0.75 mmol) was dissolved in dichloromethane (2 mL), and diisopropylethylamine (29 mg, 0.223 mmol) and 1,1,1-trifluoro-N-phenyl-N-[(trifluoromethyl)sulfonyl]methanesulfonamide (53 mg, 0.149 mmol) were added. The reaction system was stirred at room temperature for 2 h. The reaction mixture was concentrated. The crude product was purified by flash silica gel column chromatography (tetrahydrofuran/petroleum ether: 0-10%) to give compound 80-4 (30 mg, yield: 67.0%) as a colorless liquid. ES-API: [M+H]⁺ = 603.0, 605.0.

Step 5: Compound 80-4 (30 mg, 0.050 mmol) and 4-prop-2-ynyl-thiomorpholine-1,1-dioxide (16 mg, 0.089 mmol) were dissolved in acetonitrile (2 mL), and bis(triphenylphosphine)palladium(II) dichloride (9 mg, 0.012 mmol), copper(I) iodide (5 mg, 0.025 mmol), N,N'-diisopropylethylamine (32 mg, 0.249 mmol), and lithium chloride (8 mg, 0.199 mmol) were added. The reaction system was stirred at 85 °C for 1 h under argon atmosphere. Water (20 mL) was added to the reaction mixture, and the resulting mixture was extracted with ethyl acetate (30 mL × 3). The organic phase was washed with saturated brine (20 mL × 3), dried over anhydrous sodium sulfate, filtered, and concentrated to give a crude product. The crude product was purified by flash silica gel column chromatography (tetrahydrofuran/petroleum ether: 0-60%) to give compound 80-5 (30 mg, yield: 96.3%) as a light brown solid. ES-API: [M+H]⁺ = 626.1, 628.1.

Step 6: Compound 80-5 (30 mg, 0.048 mmol) was dissolved in tetrahydrofuran (1.5 mL), methanol (0.5 mL), and water (0.5 mL), and lithium hydroxide monohydrate (5 mg, 0.120 mmol) was added at 0 °C. The reaction system was stirred at room temperature for 24 h. The reaction mixture was adjusted to pH 9 with 1 M diluted hydrochloric acid and extracted with ethyl acetate (15 mL × 2). The organic phase was dried over anhydrous sodium sulfate and concentrated. The crude product was purified by flash silica gel column chromatography (methanol/dichloromethane: 0-3%) to give compound 80-6 (25 mg, yield: 89.3%) as a white solid. ES-API: [M+H]⁺ = 584.2, 586.2.

Step 7: Compound 80-6 (25 mg, 0.043 mmol) and compound 15-2 (35 mg, 0.064 mmol) were dissolved in toluene (1.5 mL), dioxane (1.5 mL), and water (0.8 mL), and potassium phosphate (23 mg, 0.107 mmol) and [1,1'-bis(di-tert-butylphosphino)ferrocene]dichloropalladium(II) (4 mg, 0.005 mmol) were added. The reaction system was purged 3 times with nitrogen and stirred at 85°C for 2 h. Ethyl acetate (50 mL) and water (10 mL) were added to the reaction mixture. The separated organic phase was washed with saturated brine (15 mL), dried over anhydrous sodium sulfate, and filtered, and the filtrate was concentrated under reduced pressure. The crude product was purified by flash silica gel column chromatography (methanol/dichloromethane: 0-5%) to give compound 80-7 (28 mg, yield: 70.5%) as a light brown solid. ES-API: [M+H]⁺ = 928.3.

Step 8: Compound 80-7 (28 mg, 0.030 mmol) was dissolved in tetrahydrofuran (1.5 mL) and water (0.5 mL), and lithium hydroxide monohydrate (2 mg, 0.045 mmol) was added at 0 °C. The reaction system was stirred at room temperature for 1 h. The reaction mixture was adjusted to pH 6 with 1 M diluted hydrochloric acid and extracted with ethyl acetate (15 mL × 2). The organic phase was dried over anhydrous sodium sulfate and concentrated to give compound 80-8 (27 mg, yield: 97.9%) as a light yellow solid. ES-API: [M+H]⁺ = 914.2.

Step 9: Compound 80-8 (27 mg, 0.030 mmol) was dissolved in dichloromethane (8 mL), and 1-(3-dimethylaminopropyl)-3-ethylcarbodiimide hydrochloride (141 mg, 0.738 mmol), 1-hydroxybenzotriazole (20 mg, 0.148 mmol), and diisopropylethylamine (114 mg, 0.886 mmol) were added at room temperature. The reaction system was stirred at room temperature for 18 h. Dichloromethane (15 mL) was added to the reaction mixture, and the resulting mixture was washed with 0.5 M diluted hydrochloric acid (2 mL × 2) and saturated brine (10 mL), dried over anhydrous sodium sulfate, and filtered, and the filtrate was concentrated under reduced pressure. The crude product was purified by flash silica gel column chromatography (methanol/dichloromethane: 0-5%) to give compound 80-9 (15 mg, yield: 56.7%) as a white solid. ES-API: [M+H]⁺ = 896.3.

Step 10: Compound 80-9 (15 mg, 0.017 mmol) was dissolved in absolute methanol (0.2 mL), and a 4.0 M hydrogen chloride/dioxane solution (1.0 mL) was added at 0 °C. The reaction system was stirred at room temperature for 1 h. The reaction mixture was concentrated to give a mixture (13 mg, crude product) of compounds 80-10 and 80-11 as a yellow solid. Compound 80-10: ES-API: [M+H]⁺ = 808.3, and compound 80-11: ES-API: [M+H]⁺ = 796.3.

Step 11: The mixture (13 mg, crude product) of compound 80-10 and compound 80-11 was dissolved in dichloromethane (3 mL), and (1r,2R,3S)-2,3-dimethylcyclopropane-1-carboxylic acid (3 mg, 0.026 mmol), O-(7-azabenzotriazol-1-yl)-N,N,N',N'-tetramethyluronium hexafluorophosphate (8 mg, 0.021 mmol), and N,N'-diisopropylethylamine (11 mg, 0.082 mmol) were added. The reaction system was stirred at room temperature for 1 h. Dichloromethane (20 mL) was added to the reaction mixture. The resulting mixture was washed sequentially with water (5 mL) and saturated brine (10 mL), dried over anhydrous sodium sulfate, and filtered. The filtrate was concentrated under reduced pressure. The crude product was purified by prep-HPLC (trifluoroacetic acid method) to obtain 2 compounds. One of the compounds was (1r,2R,3S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-1²-methoxy-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z191, 3.5 mg, yield: 21.5%) as a white solid. ES-API: [M+H]⁺ = 904.3.

The other compound was (1r,2R,3S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-ethyl-1²-fluoro-12,12-dimethy^{l}-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z80, 1.7 mg, yield: 10.6%) as a white solid. ES-API: [M+H]⁺ = 892.3.

### Example 44. Synthesis of Compound Z130

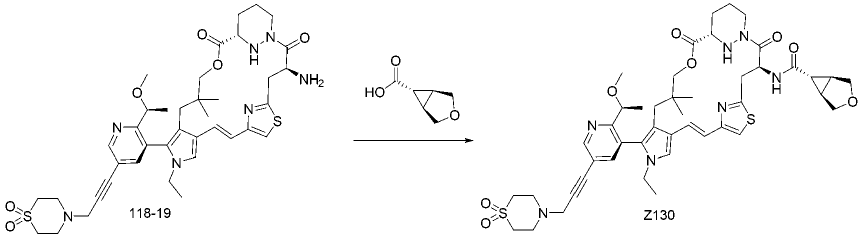

Step 1: Compound 118-19 (97 mg, 0.125 mmol) was dissolved in dichloromethane (5 mL), and (1*R*,5*S*,6*r*)-3-oxabicyclo[3.1.0]hexane-6-carboxylic acid (16.02 mg, 0.125 mmol), O-(7-azabenzotriazol-1-yl)-*N,N,N',N'*-tetramethyluronium hexafluorophosphate (94.82 mg, 0.249 mmol), and *N,N'-*diisopropylethylamine (48.34 mg, 0.374 mmol) were added to the above solution. After the addition was completed, the reaction system was stirred at room temperature for 1 h. After the reaction was completed, the reaction mixture was concentrated to remove dichloromethane. Water (10 mL) was added to the residue, and the resulting mixture was extracted with ethyl acetate (15 mL × 2). The organic phase was washed with saturated brine (10 mL × 3), dried over anhydrous sodium sulfate, filtered, and concentrated to give a crude product. The crude product was purified by flash silica gel column chromatography (methanol/dichloromethane: 0%-4%) to give (1*R*,5*S*,6*r*)*-N-*((4²*Z*,8³*S*,2*E*,6*S*)*-*(*Rₐ*)-1⁵-(5-(3-(1,1-dioxidothiomorpholino)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹*H* -10-oxa-4(4,2)-thiazola-8(1,3)-pyridazina-1(3,4)-pyrrolacyclotridecaphan-2-en-6-yl)-3-oxabicyclo[3.1.0]hexane-6-carboxamide (Z130, 80 mg, 0.090 mmol, yield: 72.25%) as a light yellow solid. ES-API: [M +H]⁺ = 888.3. ¹H NMR (400 MHz, DMSO-d₆) δ 8.72 (d, *J* = 2.0 Hz, 1H), 8.52 (d, *J =* 8.8 Hz, 1H), 7.72 (d, *J =* 2.0 Hz, 1H), 7.44 (s, 1H), 7.35 (d, *J =* 15.6 Hz, 1H), 7.13 (s, 1H), 6.81 (d, *J =* 15.6 Hz, 1H), 5.54 (t, *J =* 8.0 Hz, 1H), 5.03 (d, *J =* 12.0 Hz, 1H), 4.30 - 4.15 (m, 2H), 3.96 - 3.85 (m, 1H), 3.84 - 3.77 (m, 4H), 3.75 (s, 2H), 3.70 - 3.59 (m, 3H), 3.56 - 3.50 (m, 1H), 3.21 (s, 3H), 3.19 - 3.14 (m, 4H), 3.10 - 3.00 (m, 5H), 2.78 - 2.67 (m, 1H), 2.63 - 2.56 (m, 1H), 2.23 (d, *J =* 14.4 Hz, 1H), 2.00 - 1.85 (m, 3H), 1.79 - 1.72 (m, 1H), 1.69 - 1.57 (m, 2H), 1.51 - 1.42 (m, 1H), 1.35 (d, *J =* 6.0 Hz, 3H), 1.29 - 1.22 (m, 1H), 1.06 (t, *J =* 7.2 Hz, 3H), 0.71 (s, 3H), 0.31 (s, 3H).

### Example 45. Synthesis of Compound Z10

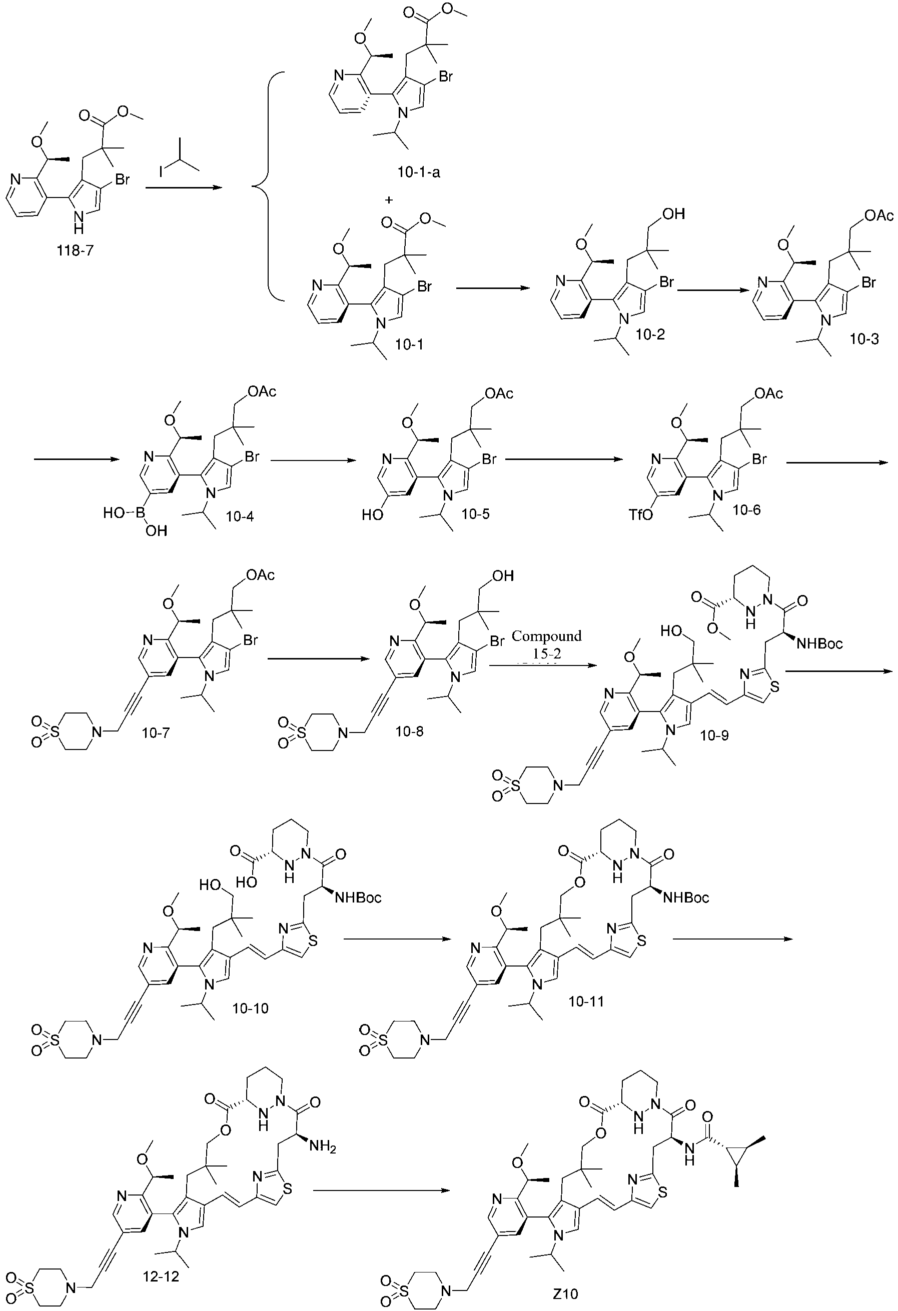

Step 1: Compound 118-7 (1.25 g, 3.16 mmol) was dissolved in N,N-dimethylformamide (25 mL), and cesium carbonate (3.09 g, 9.49 mmol) and 2-iodopropane (1.61 g, 9.49 mmol) were added at room temperature. The reaction system was stirred at 60 °C for 8 h. Ethyl acetate (100 mL) was added to the reaction mixture, and the resulting mixture was washed with saturated brine (30 mL × 3). The organic phase was dried over anhydrous sodium sulfate and filtered, and the filtrate was concentrated under reduced pressure. The crude product was purified by flash silica gel column chromatography (tetrahydrofuran/petroleum ether: 0-30%) to give compound 10-1 (700 mg, 3 min, LCMS retention time: 1.97 min, yield: 50.6%, structure arbitrarily designated) as a colorless liquid, ES-API: [M+H]⁺ = 437.1, 439.1. and isomer 10-1-a (600 mg, 3 min, LCMS retention time 1.85 min, yield: 43.4%, structure arbitrarily designated) as a yellow liquid. ES-API: [M+H]⁺ = 437.1, 439.1.

Step 2: Compound 10-1 (700 mg, 1.60 mmol) was dissolved in dichloromethane (25 mL), and a 1.0 M solution of DIBAL-H in n-hexane (4.80 mL, 4.80 mmol) was added dropwise at -70 °C. The reaction system was stirred at -70 °C for 1 h, and then warmed to room temperature and stirred for 1 h. The reaction mixture was cooled to 0 °C. Water (0.50 mL), 15% sodium hydroxide solution (0.50 mL), and water (1.25 mL) were slowly added dropwise sequentially to quench the reaction. The reaction mixture was stirred at room temperature for 15 min, dried over anhydrous magnesium sulfate, and filtered, and the filtrate was concentrated under reduced pressure. The crude product was purified by flash silica gel column chromatography (tetrahydrofuran/petroleum ether: 0-35%) to give compound 10-2 (390 mg, yield: 59.5%) as a light yellow viscous liquid. ES-API: [M+H]⁺ = 409.1, 411.1.

Step 3: Compound 10-2 (390 mg, 0.95 mmol), diisopropylethylamine (369 mg, 2.86 mmol), and 4-dimethylaminopyridine (12 mg, 0.095 mmol) were dissolved in dichloromethane (10 mL), and acetic anhydride (146 mg, 1.43 mmol) was added at 0 °C. The reaction system was stirred at 0 °C for 30 min. The reaction mixture was concentrated. The crude product was purified by flash silica gel column chromatography (tetrahydrofuran/petroleum ether: 0-20%) to give compound 10-3 (345 mg, yield: 80.2%) as a light yellow viscous liquid. ES-API: [M+H]⁺ = 451.1, 453.1.

Step 4: Compound 10-3 (345 mg, 0.76 mmol), bis(pinacolato)diboron (349 mg, 1.38 mmol), chloro(1,5-cyclooctadiene)iridium(I) dimer (62 mg, 0.092 mmol), and 4,4'-di-tert-butyl-2,2'-bipyridine (41 mg, 0.153 mmol) were dissolved in cyclohexane (20 mL). The reaction system was stirred at 85 °C for 18 h under nitrogen atmosphere. The reaction mixture was concentrated to give compound 10-4 (378 mg, crude product). ES-API: [M+H]⁺ = 495.2, 497.2.

Step 5: Compound 10-4 (378 mg, crude product) was dissolved in acetonitrile (5 mL), and 30% hydrogen peroxide (259 mg, 2.29 mmol) was added under an ice bath. The reaction system was warmed to room temperature and stirred for 3 h. After the reaction was completed, the reaction was quenched with saturated sodium sulfite (4 mL). The reaction mixture was extracted with ethyl acetate (25 mL × 2). The organic phases were combined, washed with saturated brine (30 mL), dried over anhydrous sodium sulfate, filtered, and concentrated to give a crude product. The crude product was purified by flash silica gel column chromatography (tetrahydrofuran/petroleum ether: 0-40%) to give compound 10-5 (220 mg, two-step yield: 61.7%) as a yellow solid. ES-API: [M+H]⁺ = 467.1,469.1.

Step 6: Compound 10-5 (220 mg, 0.47 mmol) was dissolved in dichloromethane (6 mL), and diisopropylethylamine (182 mg, 1.41 mmol) and 1,1,1-trifluoro-N-phenyl-N-[(trifluoromethyl)sulfonyl]methanesulfonamide (252 mg, 0.71 mmol) were added. The reaction system was stirred at room temperature for 2 h. The reaction mixture was concentrated. The crude product was purified by flash silica gel column chromatography (tetrahydrofuran/petroleum ether: 0-20%) to give compound 10-6 (240 mg, yield: 85.1%) as a colorless liquid. ES-API: [M+H]⁺ = 599.0, 601.0.

Step 7: Compound 10-6 (240 mg, 0.400 mmol) and 4-prop-2-ynyl-thiomorpholine-1,1-dioxide (125 mg, 0.72 mmol) were dissolved in acetonitrile (8 mL), and bis(triphenylphosphine)palladium(II) dichloride (70 mg, 0.10 mmol), copper(I) iodide (38 mg, 0.20 mmol), N,N'-diisopropylethylamine (259 mg, 2.0 mmol), and lithium chloride (68 mg, 1.60 mmol) were added. The reaction system was stirred at 85 °C for 1 h under argon atmosphere. Water (20 mL) was added to the reaction mixture, and the resulting mixture was extracted with ethyl acetate (30 mL × 3). The organic phase was washed with saturated brine (20 mL × 3), dried over anhydrous sodium sulfate, filtered, and concentrated to give a crude product. The crude product was purified by flash silica gel column chromatography (tetrahydrofuran/petroleum ether: 0-50%) to give compound 10-7 (245 mg, yield: 98.3%) as a light brown solid. ES-API: [M+H]⁺ = 622.2, 624.2.

Step 8: Compound 10-7 (245 mg, 0.39 mmol) was dissolved in tetrahydrofuran (5 mL), methanol (2.5 mL), and water (2.5 mL), and lithium hydroxide monohydrate (41 mg, 0.98 mmol) was added at 0 °C. The reaction system was stirred at room temperature for 16 h. The reaction mixture was adjusted to pH 9 with 1 M diluted hydrochloric acid and extracted with ethyl acetate (30 mL × 2). The organic phase was dried over anhydrous sodium sulfate, filtered, and concentrated. The crude product was purified by flash silica gel column chromatography (methanol/dichloromethane: 0-3%) to give compound 10-8 (200 mg, yield: 87.5%) as a white solid. ES-API: [M+H]⁺ = 580.2, 582.1.

Step 9: Compound 10-8 (200 mg, 0.34 mmol) and compound 15-2 (284 mg, 0.52 mmol) were dissolved in toluene (2 mL), dioxane (6 mL), and water (2 mL), and potassium phosphate (183 mg, 0.86 mmol) and [1,1'bis(di-tert-butylphosphino)ferrocene]dichloropalladium(II) (22 mg, 0.034 mmol) were added. The reaction system was purged 3 times with nitrogen and stirred at 85 °C for 3 h. Ethyl acetate (50 mL) and water (10 mL) were added to the reaction mixture. The separated organic phase was washed with saturated brine (15 mL), dried over anhydrous sodium sulfate, and filtered, and the filtrate was concentrated under reduced pressure. The crude product was purified by flash silica gel column chromatography (methanol/dichloromethane: 0-8%) to give compound 10-9 (245 mg, yield: 77.1%) as a light yellow solid. ES-API: [M+H]⁺ = 924.2.

Step 10: Compound 10-9 (245 mg, 0.265 mmol) was dissolved in tetrahydrofuran (6 mL) and water (1.5 mL), and lithium hydroxide monohydrate (17 mg, 0.40 mmol) was added at 0 °C. The reaction system was stirred at room temperature for 1 h. The reaction mixture was adjusted to pH 6 with 1 M diluted hydrochloric acid and extracted with ethyl acetate (15 mL × 2). The organic phase was dried over anhydrous sodium sulfate, filtered, and concentrated to give compound 10-10 (240 mg, yield: 99.5%) as a light yellow solid. ES-API: [M+H]⁺ = 910.3.

Step 11: Compound 10-10 (240 mg, 0.26 mmol) was dissolved in dichloromethane (25 mL), and 1-(3-dimethylaminopropyl)-3-ethylcarbodiimide hydrochloride (1.26 g, 6.59 mmol), 1-hydroxybenzotriazole (178 mg, 1.32 mmol), and diisopropylethylamine (1.02 g, 7.91 mmol) were added at room temperature. The reaction system was stirred at room temperature for 18 h. Dichloromethane (15 mL) was added to the reaction mixture, and the resulting mixture was washed with 0.5 M diluted hydrochloric acid (5 mL × 2) and saturated sodium bicarbonate (15 mL), dried over anhydrous sodium sulfate, and filtered. The filtrate was concentrated under reduced pressure. The crude product was purified by flash silica gel column chromatography (tetrahydrofuran/petroleum ether: 0-70%) to give compound 10-11 (130 mg, yield: 55.3%) as a light pink solid. ES-API: [M+H]⁺ = 892.2.

Step 12: Compound 10-11 (130 mg, 0.146 mmol) was dissolved in absolute methanol (0.75 mL), and a 4.0 M hydrogen chloride/dioxane solution (3 mL) was added at 0 °C. The reaction system was stirred at room temperature for 1 h. The reaction mixture was concentrated to give compound 10-12 (115 mg, yield: 99.6%) as a yellow solid. ES-API: [M+H]⁺ = 792.3.

Step 13: Compound 10-12 (115 mg, 0.145 mmol) was dissolved in dichloromethane (15 mL), and (1r,2R,3S)-2,3-dimethylcyclopropane-1-carboxylic acid (22 mg, 0.189 mmol), O-(7-azabenzotriazol-1-yl)-N,N,N',N'-tetramethyluronium hexafluorophosphate (66 mg, 0.174 mmol), and N,N'-diisopropylethylamine (150 mg, 1.162 mmol) were added. The reaction system was stirred at room temperature for 1 h. Dichloromethane (15 mL) was added to the reaction mixture. The resulting mixture was washed sequentially with water (5 mL) and saturated brine (10 mL), dried over anhydrous sodium sulfate, and filtered. The filtrate was concentrated under reduced pressure. The crude product was purified by prep-HPLC (ammonium bicarbonate method 1) to give the desired product (1r,2R,3S)-N-((4²Z,8³S,2E,6S)-(Rₐ)-1⁵-(5-(3-(1,1-dioxidothiomorpholino)prop-1-yn-1-yl)-2-((S)-1-methoxyethyl)pyridin-3-yl)-1¹-isopropyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z10, 80 mg, yield: 61.0%) as a white solid. ES-API: [M+H]⁺ = 888.3. ¹H NMR (400 MHz, DMSO-d₆) δ 8.72 (d, *J=* 1.6 Hz, 1H), 8.34 (d, *J* = 8.8 Hz, 1H), 7.69 (d, *J* = 1.6 Hz, 1H), 7.53 (s, 1H), 7.35 (d, *J* = 15.6 Hz, 1H), 7.12 (s, 1H), 6.86 (d, *J* = 15.6 Hz, 1H), 5.53 (t, *J* = 9.2 Hz, 1H), 5.00 (d, *J =* 12.4 Hz, 1H), 4.35 (q, *J =* 6.4 Hz, 1H), 4.27 - 4.07 (m, 2H), 3.91 (t, *J =* 12.0 Hz, 1H), 3.74 (s, 2H), 3.66 (d, *J=* 10.4 Hz, 1H), 3.53 (d, *J=* 10.4 Hz, 1H), 3.23 (s, 3H), 3.20 - 2.99 (m, 10H), 2.73 (t, *J=* 11.6 Hz, 1H), 2.59 (d, *J =* 14.0 Hz, 1H), 2.20 (d, *J=* 15.2 Hz, 1H), 1.95 (d, *J =* 12.0 Hz, 1H), 1.82 - 1.57 (m, 2H), 1.52 - 1.31 (m, 7H), 1.21 - 1.11 (m, 3H), 1.11 - 1.01 (m, 6H), 0.97 (d, *J* = 6.0 Hz, 3H), 0.71 (s, 3H), 0.31 (s, 3H).

### Example 46. Synthesis of Compound Z49

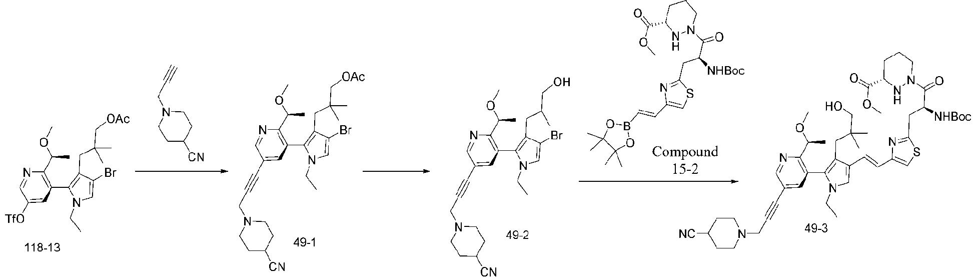

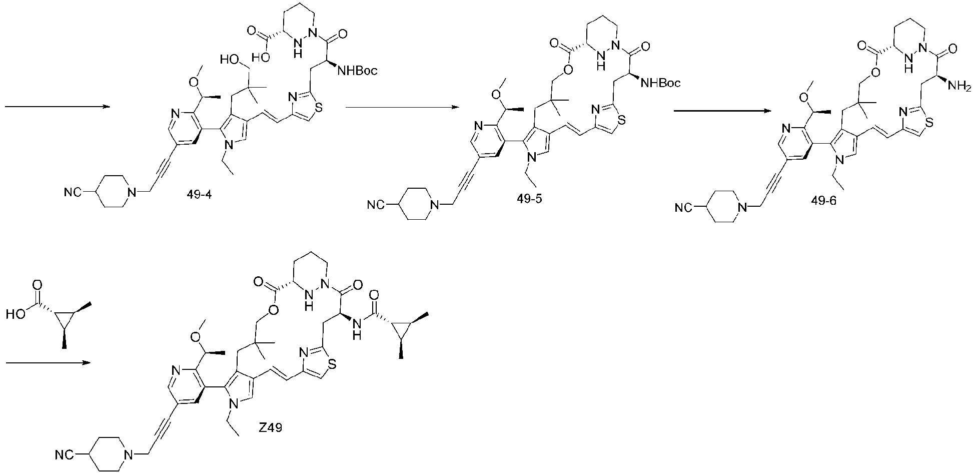

Step 1: Compound 118-13 (180 mg, 0.307 mmol) was dissolved in acetonitrile (8 mL). 1-(Prop-2-yn-1-yl)piperidine-4-carbonitrile (91.14 mg, 0.615 mmol), bis(triphenylphosphine)palladium(II) dichloride (32.37 mg, 0.046 mmol), copper(I) iodide (17.57 mg, 0.092 mmol), N,N-diisopropylethylamine (198.70 mg, 1.537 mmol), and lithium chloride (52.13 mg, 1.230 mmol) were added to the above solution at room temperature. After the addition was completed, the reaction system was stirred at 85°C for 4 h under argon atmosphere. After the reaction was completed, water (20 mL) was added to the reaction mixture, and the resulting mixture was extracted with ethyl acetate (20 mL × 3). The organic phase was washed with saturated brine (30 mL), dried over anhydrous sodium sulfate, filtered, and concentrated to give a crude product. The crude product was purified by flash silica gel column chromatography (tetrahydrofuran/petroleum ether: 0%-40%) to give compound 49-1 (150 mg, 0.257 mmol, yield: 83.60%) as a light yellow solid. ES-API: [M +H]⁺ = 583.1, 585.1.

Step 2: Compound 49-1 (150 mg, 0.257 mmol) was dissolved in methanol (0.3 mL), tetrahydrofuran (3 mL), and water (3 mL). Lithium hydroxide (15.39 mg, 0.643 mmol) was added to the above solution. After the addition was completed, the reaction system was stirred at room temperature for 10 h. After the reaction was completed, the reaction mixture was acidified to weak acidity (about pH 6) with diluted hydrochloric acid (1 *M*). The resulting solution was extracted with ethyl acetate (20 mL × 2). The organic phase was washed with saturated brine (50 mL), dried over anhydrous sodium sulfate, filtered, and concentrated to give a crude product. The crude product was purified by flash silica gel column chromatography (ethyl acetate/petroleum ether: 0-4%) to give compound 49-2 (140 mg, 0.259 mmol, yield: 100%) as a light yellow solid. ES-API: [M+H]⁺ = 541.2, 543.2.

Step 3: Compound 49-2 (120 mg, 0.222 mmol) was dissolved in toluene (1.5 mL), dioxane (4.5 mL), and water (1.5 mL). Compound 15-2 (243.97 mg, 0.443 mmol), potassium phosphate (117.59 mg, 0.554 mmol), and [1,1'-bis(di-tert-butylphosphino)ferrocene]dichloropalladium(II) (14.43 mg, 0.022 mmol) were added to the above solution, and the reaction system was purged 3 times with nitrogen and stirred at 80 °C for 3 h. Ethyl acetate (30 mL) and water (30 mL) were added to the reaction mixture. The organic phase was separated, washed with saturated brine (20 mL), dried over anhydrous sodium sulfate, and filtered, and the filtrate was concentrated under reduced pressure. The crude product was purified by flash silica gel column chromatography (tetrahydrofuran/petroleum ether: 0-30%) to give compound 49-3 (170 mg, 0.192 mmol, yield: 86.67%) as a gray solid. ES-API: [M+H]⁺ = 885.4.

Step 4: Compound 49-3 (170 mg, 0.192 mmol) was dissolved in tetrahydrofuran (3 mL) and water (1 mL), and lithium hydroxide (9.20 mg, 0.384 mmol) was added at 0 °C. The reaction system was stirred at room temperature for 18 h. The reaction mixture was adjusted to pH 6 with 1 M diluted hydrochloric acid and extracted with ethyl acetate (30 mL × 2). The organic phase was dried over anhydrous sodium sulfate and concentrated to give compound 49-4 (165 mg, 0.189 mmol, yield: 98.62%) as a light yellow solid. ES-API: [M+H]⁺ = 871.3.

Step 5: Compound 49-4 (165 mg, 0.189 mmol) was dissolved in dichloromethane (40 mL), and 1-(3-dimethylaminopropyl)-3-ethylcarbodiimide hydrochloride (1072.05 mg, 5.592 mmol), 1-hydroxybenzotriazole (251.90 mg, 1.864 mmol), and diisopropylethylamine (1.136 mL, 6.524 mmol) were added at room temperature. The reaction system was stirred at room temperature for 18 h. Dichloromethane (50 mL) was added to the reaction mixture, and the resulting mixture was washed with diluted hydrochloric acid (0.5 *M,* 20 mL × 2) and saturated brine (100 mL), dried over anhydrous sodium sulfate, and filtered. The filtrate was concentrated under reduced pressure. The crude product was purified by flash silica gel column chromatography (ethyl acetate/petroleum ether: 0-70%) to give compound 49-5 (100 mg, 0.117 mmol, yield: 62.88%) as a light yellow solid. ES-API: [M+H]⁺ = 853.3.

Step 6: Compound 49-5 (100 mg, 0.117 mmol) was dissolved in dichloromethane (1 mL) and trifluoroacetic acid (1 mL). The reaction system was stirred at room temperature for 2 h. After the reaction was completed, the reaction mixture was concentrated to remove the solvent. The residue was re-dissolved in dichloromethane (30 mL) and washed with saturated sodium bicarbonate solution (20 mL × 2). The separated organic phase was washed with saturated brine (20 mL × 2), dried over anhydrous sodium sulfate, and filtered, and the filtrate was concentrated under reduced pressure to give compound 49-6 (90 mg, crude product) as a light yellow solid. ES-API: [M+H]⁺ = 753.2.

Step 7: Compound 49-6 (90 mg, crude product) was dissolved in dichloromethane (3 mL). (1*r*,2*R*,3*S*)-2,3-Dimethylcyclopropane-1-carboxylic acid (20.01 mg, 0.175 mmol), O-(7-azabenzotriazol-1-yl)-*N,N,N',N'-*tetramethyluronium hexafluorophosphate (57.77 mg, 0.152 mmol), and *N,N'*-diisopropylethylamine (0.061 mL, 0.351 mmol) were added to the above solution. After the addition was completed, the reaction system was stirred at room temperature for 24 h. After the reaction was completed, the reaction mixture was concentrated to remove dichloromethane. Water (10 mL) was added to the residue, and the resulting mixture was extracted with dichloromethane (15 mL × 2). The organic phase was washed with saturated brine (10 mL × 3), dried over anhydrous sodium sulfate, filtered, and concentrated to give a crude product. The crude product was purified by flash silica gel column chromatography (methanol/dichloromethane: 0-4%) to give the desired product (*1r,*2*R*,3*S*)*-N-*((4 ²*Z*,8³*S*,2*E*,6*S*)*-*(*Rₐ*)*-*1⁵-(5-(3-(4-cyanopiperidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z49, 70 mg, 0.082 mmol, two-step yield: 70.54%) as a white solid. ES-API: [M +H]⁺ = 849.3. ¹H NMR (400 MHz, DMSO-d₆) δ 8.72 (d, *J =* 2.0 Hz, 1H), 8.34 (d, *J =* 9.2 Hz, 1H), 7.69 (d, *J* = 1.6 Hz, 1H), 7.44 (s, 1H), 7.35 (d, *J =* 15.6 Hz, 1H), 7.13 (s, 1H), 6.81 (d, *J =* 15.6 Hz, 1H), 5.53 (t, *J* = 8.0 Hz, 1H), 5.01 (d, *J =* 12.4 Hz, 1H), 4.25 - 4.19 (m, 2H), 3.81 - 3.75 (m, 3H), 3.67 (d, *J =* 10.8 Hz, 1H), 3.57 (s, 2H), 3.53 (d, *J =* 10.8 Hz, 1H), 3.22 - 3.21 (m, 4H), 3.11 - 3.06 (m, 1H), 2.90 - 2.89 (m, 1H), 2.75 - 2.67 (m, 4H), 2.45 - 2.41 (m, 1H),2.23 (d, *J =* 14.8 Hz, 1H), 1.94 - 1.87 (m, 3H), 1.75 - 1.65 (m, 5H), 1.53 - 1.41 (m, 1H), 1.35 (d, *J =* 6.0 Hz, 3H), 1.19 - 1.14 (m, 3H), 1.07 - 1.04 (m, 9H), 0.70 (s, 3H), 0.31 (s, 3H).

### Example 47. Synthesis of Compound Z103

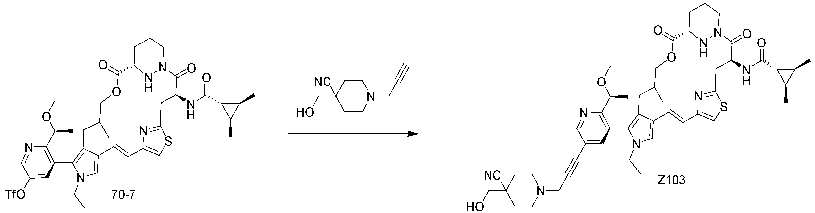

Step 1: Compound 70-7 (100 mg, 0.118 mmol) was dissolved in acetonitrile (6 mL). 4-(Hydroxymethyl)-1-(prop-2-yn-1-yl)piperidine-4-carbonitrile (62.84 mg, 0.353 mmol), bis(triphenylphosphine)palladium(II) dichloride (16.50 mg, 0.024 mmol), copper(I) iodide (4.28 mg, 0.022 mmol), *N*,*N*'-diisopropylethylamine (0.082 mL, 0.470 mmol), and lithium chloride (29.89 mg, 0.705 mmol) were added to the above solution at room temperature. After the addition was completed, the reaction system was stirred at 80 °C for 1.5 h under nitrogen atmosphere. After the reaction was completed, water (20 mL) was added to the reaction mixture, and the resulting mixture was extracted with ethyl acetate (20 mL × 2). The organic phase was washed with saturated brine (20 mL × 2), dried over anhydrous sodium sulfate, filtered, and concentrated to give a crude product. The crude product was purified by flash silica gel column chromatography (methanol/dichloromethane: 0%-4%) to give the desired product (1*r*,2*R*,3*S*)*-N-*((4²*Z*,8³*S*,2*E*,6*S*)-(*Rₐ*)-1⁵-(5-(3-(4-cyano-4-(hydroxymethyl)piperidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z103, 80 mg, 0.091 mmol, yield: 77.44%) as a light yellow solid. ES-API: [M+H]⁺ = 879.3. ¹H NMR (400 MHz, DMSO-d₆) δ 8.72 (d, *J* = 1.6 Hz, 1H), 8.34 (d, *J =* 8.8 Hz, 1H), 7.69 (d, *J =* 1.6 Hz, 1H), 7.43 (s, 1H), 7.35 (d, *J =* 15.6 Hz, 1H), 7.13 (s, 1H), 6.81 (d, *J =* 15.6 Hz, 1H), 5.54 (t, *J =* 8.0 Hz, 1H), 5.43 (t, *J =* 5.6 Hz, 1H), 5.01 (d, *J* = 12.0 Hz, 1H), 4.28 - 4.17 (m, 2H), 3.90 (t, *J =* 12.4 Hz, 1H), 3.85 - 3.74 (m, 2H), 3.75 - 3.65 (d, *J =* 10.8 Hz, 1H), 3.61 (s, 2H), 3.53 (d, *J =* 10.8 Hz, 1H), 3.46 (d, *J =* 5.6 Hz, 2H), 3.25 - 3.17 (m, 4H), 3.13 - 3.05 (m, 1H), 2.94 - 2.85 (m, 2H), 2.78 - 2.67 (m, 1H), 2.63 - 2.54 (m, 1H), 2.50 - 2.42 (m, 2H), 2.30 - 2.20 (m, 1H), 1.95 (d, *J =* 10.8 Hz, 1H), 1.87 (d, *J =* 12.8 Hz, 2H), 1.79 - 1.71 (m, 1H), 1.69 - 1.61 (m, 1H), 1.58 - 1.45 (m, 3H), 1.35 (d, *J =* 6.0 Hz, 3H), 1.27 - 1.13 (m, 3H), 1.08 - 1.05 (m, 9H), 0.70 (s, 3H), 0.31 (s, 3H).

### Example 48. Synthesis of Compound Z69

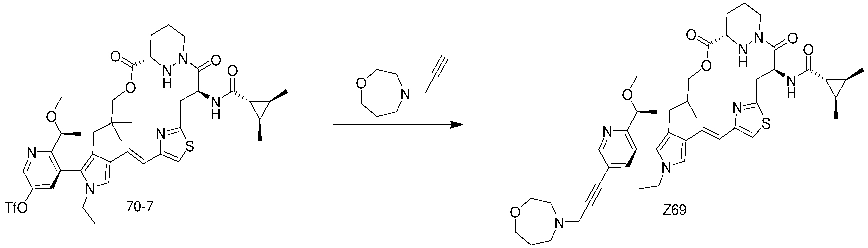

Step 1: Compound 70-7 (120 mg, 0.141 mmol) was dissolved in acetonitrile (5 mL). 4-(Prop-2-yn-1-yl)-1,4-oxazepane (58.89 mg, 0.423 mmol), bis(triphenylphosphine)palladium(II) dichloride (19.80 mg, 0.028 mmol), copper(I) iodide (10.74 mg, 0.056 mmol), *N*,*N'*-diisopropylethylamine (0.098 mL, 0.564 mmol), and lithium chloride (35.87 mg, 0.846 mmol) were added to the above solution at room temperature. After the addition was completed, the reaction system was stirred at 80 °C for 1.5 h under nitrogen atmosphere. After the reaction was completed, water (20 mL) was added to the reaction mixture, and the resulting mixture was extracted with ethyl acetate (20 mL × 2). The organic phase was washed with saturated brine (20 mL × 2), dried over anhydrous sodium sulfate, filtered, and concentrated to give a crude product. The crude product was purified by flash silica gel column chromatography (methanol/dichloromethane: 0%-4%) to give (1*r*,2*R*,3*S*)-*N*-((4²*Z*,8³*S*,2*E*,6*S*)-(*Rₐ*)-1⁵-(5-(3-(1,4-oxazepan-4-yl)prop-1-yn-1-yl)-2-((*S*)-1-methoxyethyl)pyridin-3-yl)-1¹-ethyl-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrrolacyclotridecaphan-2-en-6-yl)-2,3-dimethylcyclopropane-1-carboxamide (Z69, 90 mg, 0.107 mmol, yield: 75.97%) as a white solid. ES-API: [M +H]⁺ = 840.3. ¹H NMR (400 MHz, DMSO-d₆) δ 8.71 (d, *J =* 1.6 Hz, 1H), 8.34 (d, *J =* 8.8 Hz, 1H), 7.69 (d, *J=* 1.6 Hz, 1H), 7.44 (s, 1H), 7.35 (d, *J* = 15.6 Hz, 1H), 7.13 (s, 1H), 6.81 (d, *J* = 15.6 Hz, 1H), 5.54 (t, *J =* 8.0 Hz, 1H), 5.01 (d, *J* = 12.0 Hz, 1H), 4.27 - 4.16 (m, 2H), 3.96 - 3.87 (m, 1H), 3.85 - 3.75 (m, 2H), 3.64 - 3.58 (m, 2H), 3.56 - 3.51 (m, 2H), 3.25 - 3.18 (m, 4H), 3.14 - 3.07 (m, 1H), 2.79 - 2.71 (m, 5H), 2.68 - 2.59 (m, 3H), 2.30 - 2.18 (m, 1H), 2.01 - 1.92 (m, 1H), 1.90 - 1.72 (m, 5H), 1.69 - 1.58 (m, 1H), 1.53 - 1.42 (m, 1H), 1.35 (d, *J* = 6.0 Hz, 3H), 1.21 - 1.12 (m, 3H), 1.09 - 1.02 (m, 9H), 0.71 (s, 3H), 0.32 (s, 3H).

### Example 49. Synthesis of Compound Z70

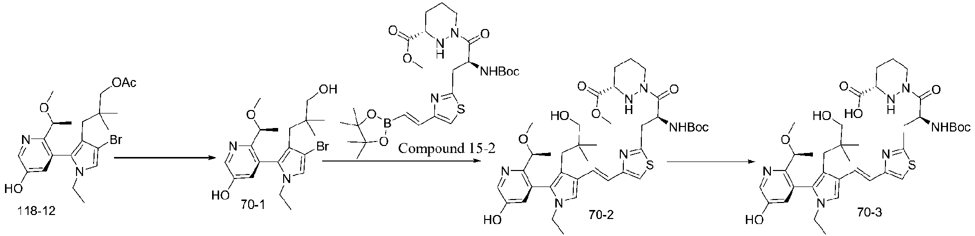

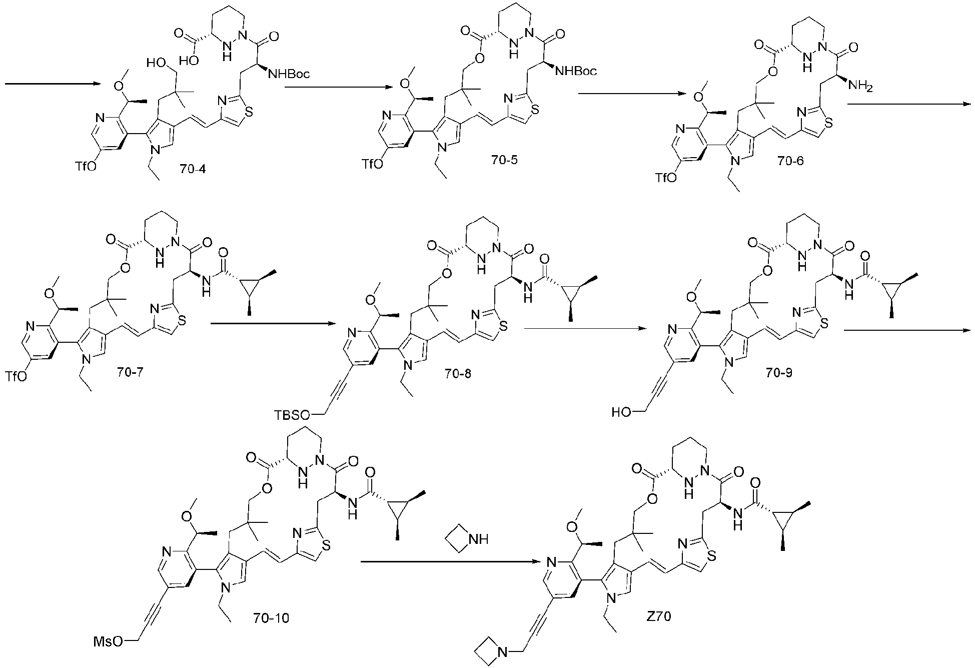

Step 1: Compound 118-12 (150 mg, 0.33 mmol) was dissolved in tetrahydrofuran (4 mL), methanol (2 mL), and water (2 mL), and lithium hydroxide monohydrate (41 mg, 0.98 mmol) was added at 0 °C. The reaction system was stirred at room temperature for 5 h. The reaction mixture was adjusted to pH 9 with 1 M diluted hydrochloric acid and extracted with ethyl acetate (30 mL × 2). The organic phase was dried over anhydrous sodium sulfate and concentrated. The crude product was purified by flash silica gel column chromatography (methanol/dichloromethane: 0-5%) to give compound 70-1 (95 mg, yield: 69.8%) as a white solid. ES-API: [M+H]⁺ = 411.2,413.2. ¹H NMR (400 MHz, DMSO-d₆) δ 10.07 (s, 1H), 8.22 (d, *J =* 2.8 Hz, 1H), 7.10 (s, 1H), 6.99 (d, *J =* 2.8 Hz, 1H), 4.33 (t, *J =* 5.6 Hz, 1H), 4.01 (q, *J =* 6.4 Hz, 1H), 3.62-3.58 (m, 2H), 2.95-2.89 (m, 2H), 2.87 (s, 3H), 2.26 (d, *J =* 14.0 Hz, 1H), 1.99 (d, *J =* 14.0 Hz, 1H), 1.36 (d, *J =* 6.4 Hz, 3H), 1.16 (t, *J* = 7.2 Hz, 3H), 0.62 (s, 3H), 0.48 (s, 3H).

Step 2: Compound 70-1 (95 mg, 0.23 mmol) and compound 15-2 (191 mg, 0.35 mmol) were dissolved in toluene (1 mL), dioxane (3 mL), and water (1 mL), and potassium phosphate (122 mg, 0.58 mmol) and [1,1'-bis(di-tert-butylphosphino)ferrocene]dichloropalladium(II) (15 mg, 0.023 mmol) were added. The reaction system was purged 3 times with nitrogen and stirred at 85 °C for 3 h. Ethyl acetate (50 mL) and water (10 mL) were added to the reaction mixture. The separated organic phase was washed with saturated brine (15 mL), dried over anhydrous sodium sulfate, and filtered, and the filtrate was concentrated under reduced pressure. The crude product was purified by flash silica gel column chromatography (tetrahydrofuran/petroleum ether: 0-50%) to give compound 70-2 (150 mg, yield: 86.0%) as a light yellow solid. ES-API: [M+H]⁺ = 755.3.

Step 3: Compound 70-2 (80 mg, 0.106 mmol) was dissolved in tetrahydrofuran (2.5 mL) and water (0.8 mL), and lithium hydroxide monohydrate (7 mg, 0.16 mmol) was added at 0 °C. The reaction system was stirred at room temperature for 1 h. The reaction mixture was adjusted to pH 6 with 1 M diluted hydrochloric acid and extracted with ethyl acetate (10 mL × 2). The organic phase was dried over anhydrous sodium sulfate, filtered, and concentrated to give compound 70-3 (74 mg, yield: 94.3%) as a light yellow solid. ES-API: [M+H]⁺ = 741.3.

Step 4: Compound 70-3 (74 mg, 0.10 mmol) was dissolved in dichloromethane (5 mL), and diisopropylethylamine (39 mg, 3.0 mmol) and 1,1,1-trifluoro-N-phenyl-N-[(trifluoromethyl)sulfonyl]methanesulfonamide (53 mg, 0.15 mmol) were added. The reaction system was stirred at room temperature for 18 h. Dichloromethane (15 mL) was added to the reaction mixture, and the resulting mixture was washed with 0.5 M diluted hydrochloric acid (2 mL × 2) and saturated brine (5 mL), dried over anhydrous sodium sulfate, and filtered, and the filtrate was concentrated under reduced pressure. The crude product was purified by flash silica gel column chromatography (methanol/dichloromethane: 0-30%) to give compound 70-4 (70 mg, yield: 80.3%) as a light yellow solid. ES-API: [M+H]⁺ = 873.3.

Step 5: Compound 70-4 (130 mg, 0.149 mmol) was dissolved in dichloromethane (15 mL), and 1-(3-dimethylaminopropyl)-3-ethylcarbodiimide hydrochloride (714 mg, 3.72 mmol), 1-hydroxybenzotriazole (101 mg, 0.745 mmol), and diisopropylethylamine (577 mg, 4.47 mmol) were added at room temperature. The reaction system was stirred at room temperature for 64 h. Dichloromethane (15 mL) was added to the reaction mixture, and the resulting mixture was washed with 0.5 M diluted hydrochloric acid (4 mL × 2) and saturated sodium bicarbonate (15 mL), dried over anhydrous sodium sulfate, and filtered. The filtrate was concentrated under reduced pressure. The crude product was purified by flash silica gel column chromatography (tetrahydrofuran/petroleum ether: 0-35%) to give compound 70-5 (55 mg, yield: 43.2%) as a light pink solid. ES-API: [M+H]⁺ = 855.3. ¹H NMR (400 MHz, DMSO) δ 8.90 (d, *J =* 2.8 Hz, 1H), 8.07 (d, *J* = 2.4 Hz, 1H), 7.49 (s, 1H), 7.34 (d, *J =* 15.6 Hz, 1H), 7.25 (d, *J* = 9.2 Hz, 1H), 7.15 (s, 1H), 6.84 (d, *J* = 15.6 Hz, 1H), 5.18 (t, *J =* 4.8 Hz, 1H), 5.01 (d, *J =* 12.2 Hz, 1H), 4.31-4.19 (m, 2H), 3.99 (t, *J =* 10.8 Hz, 1H), 3.94 - 3.81 (m, 2H), 3.85 - 3.63 (m, 1H), 3.52 (d, *J* = 10.8 Hz, 1H), 3.26 (s, 3H), 3.13-3.12 (m, 2H), 2.82 - 2.59 (m, 2H), 2.28-2.24 (m, 1H), 1.99 (d, *J* = 10.2 Hz, 1H), 1.78-1.71 (m, 2H), 1.46-1.43 (m, 1H), 1.40 - 1.34 (m, 12H), 1.03 (t, *J =* 7.2 Hz, 3H), 0.73 (s, 3H), 0.24 (s, 3H).

Step 6: Compound 70-5 (55 mg, 0.064 mmol) was dissolved in absolute methanol (0.3 mL), and a 4.0 M hydrogen chloride/dioxane solution (1.5 mL) was added at 0 °C. The reaction system was stirred at room temperature for 1 h. The reaction mixture was concentrated to give compound 70-6 (48 mg, yield: 98.9%) as a yellow solid. ES-API: [M+H]⁺ = 755.1.

Step 7: Compound 70-6 (48 mg, 0.064 mmol) was dissolved in dichloromethane (5 mL), and (1r,2R,3S)-2,3-dimethylcyclopropane-1-carboxylic acid (9 mg, 0.083 mmol), O-(7-azabenzotriazol-1-yl)-N,N,N',N'-tetramethyluronium hexafluorophosphate (31 mg, 0.083 mmol), and N,N'-diisopropylethylamine (66 mg, 0.509 mmol) were added. The reaction system was stirred at room temperature for 1 h. Dichloromethane (15 mL) was added to the reaction mixture. The resulting mixture was washed sequentially with water (5 mL) and saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-40%) to give compound 70-7 (52 mg, yield: 96.1%) as a white solid. ES-API: [M+H]⁺ = 851.2.

Step 8: Compound 70-7 (52 mg, 0.061 mmol) and 4-tert-butyldimethyl(2-propynyloxy)silane (31 mg, 0.183 mmol) were dissolved in acetonitrile (4 mL), and bis(triphenylphosphine)palladium(II) dichloride (9 mg, 0.012 mmol), copper(I) iodide (4 mg, 0.018 mmol), N,N'-diisopropylethylamine (31 mg, 0.244 mmol), and lithium chloride (10 mg, 0.244 mmol) were added. The reaction system was stirred at 85 °C for 3 h under argon atmosphere. Water (20 mL) was added to the reaction mixture, and the resulting mixture was extracted with ethyl acetate (30 mL × 3). The organic phase was washed with saturated brine (20 mL × 3), dried over anhydrous sodium sulfate, filtered, and concentrated to give a crude product. The crude product was purified by flash silica gel column chromatography (tetrahydrofuran/petroleum ether: 0-50%) to give compound 70-8 (32 mg, yield: 60.1%) as a light brown solid. ES-API: [M+H]⁺ = 871.3.

Step 9: Compound 70-8 (167 mg, 0.192 mmol) was dissolved in tetrahydrofuran (3 mL), and a 1.0 M solution of tetrabutylammonium fluoride in tetrahydrofuran (0.3 mL, 0.30 mmol) was added at 0 °C. The reaction system was stirred at room temperature for 2 h. Ethyl acetate (30 mL) was added to the reaction mixture, and the resulting mixture was washed sequentially with water (5 mL) and saturated brine (5 mL), dried over anhydrous sodium sulfate, and concentrated. The crude product was purified by flash silica gel column chromatography (methanol/dichloromethane: 0-3%) to give compound 70-9 (125 mg, yield: 86.1%) as a light pink solid. ES-API: [M+H]⁺ = 757.3.

Step 10: Compound 70-9 (125 mg, 0.165 mmol) was dissolved in dichloromethane (4 mL), and diisopropylethylamine (64 mg, 0.495 mmol) and methanesulfonic anhydride (43 mg, 0.247 mmol) were added. The reaction system was stirred at room temperature for 6 h. The reaction mixture was concentrated under reduced pressure, and the crude product was purified by flash silica gel column chromatography (tetrahydrofuran/petroleum ether: 0-60%) to give compound 70-10 (130 mg, yield: 94.3%) as a light yellow solid. ES-API: [M+H]⁺ = 835.3.

Step 11: Compound 70-10 (115 mg, 0.138 mmol) and diisopropylethylamine (36 mg, 0.275 mmol) were dissolved in dichloromethane (5 mL), and azetidine (39 mg, 0.689 mmol) was added at 0 °C. The reaction system was stirred at room temperature for 2 h. The reaction mixture was concentrated, and the crude product was purified by flash silica gel column chromatography (methanol/dichloromethane: 0-4%) to give the desired product (1r,2R,3S)-N-((4²Z, 8³S,2E,6S)-(Rₐ)-1⁵-(5-(3-(azetidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z70, 70 mg, yield: 63.8%) as a yellow solid. ES-API: [M+H]⁺ = 796.3. ¹H NMR (400 MHz, DMSO-d₆) δ 8.73 (d, *J =* 2.0 Hz, 1H), 8.34 (d, *J* = 9.0 Hz, 1H), 7.70 (d, *J =* 2.0 Hz, 1H), 7.44 (s, 1H), 7.35 (d, *J* = 15.6 Hz, 1H), 7.13 (s, 1H), 6.80 (d, *J =* 15.6 Hz, 1H), 5.53 (t, *J =* 8.8 Hz, 1H), 5.01 (d, *J =* 12.0 Hz, 1H), 4.29 - 4.18 (m, 2H), 3.95 - 3.75 (m, 3H), 3.67 (d, *J =* 10.8 Hz, 1H), 3.53 (d, *J =* 10.8 Hz, 1H), 3.44 (s, 2H), 3.27 - 3.16 (m, 8H), 3.09 (dd, *J =* 14.8, 8.8 Hz, 1H), 2.78 - 2.69 (m, 1H), 2.58 (d, *J =* 12.8 Hz, 1H), 2.23 (d, *J =* 13.2 Hz, 1H), 2.03 - 1.91 (m, 3H), 1.80 - 1.55 (m, 2H), 1.53 - 1.42 (m, 1H), 1.35 (d, *J =* 6.0 Hz, 3H), 1.20 - 1.11 (m, 3H), 1.09 - 0.99 (m, 9H), 0.70 (s, 3H), 0.31 (s, 3H).

### Example 50. Synthesis of Compound Z172

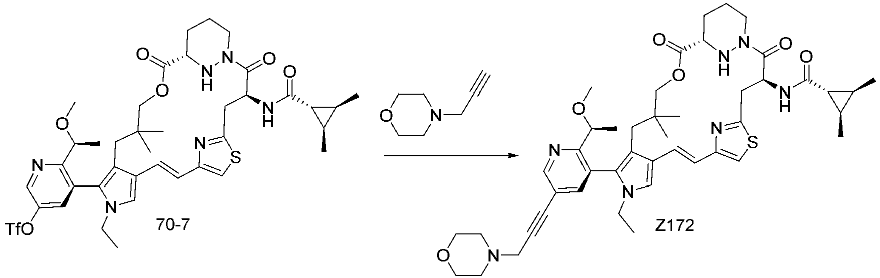

Step 1: Compound 70-7 (120 mg, 0.141 mmol) and 1-(morpholin-4-yl)-2-propyne (71 mg, 0.564 mmol) were dissolved in acetonitrile (6 mL), and bis(triphenylphosphine)palladium(II) dichloride (20 mg, 0.028 mmol), copper(I) iodide (8 mg, 0.042 mmol), N,N'-diisopropylethylamine (73 mg, 0.564 mmol), and lithium chloride (24 mg, 0.564 mmol) were added. The reaction system was stirred at 85 °C for 2 h under argon atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by prep-HPLC(ammonium bicarbonate method 1) to give the desired product (1r,2R,3S)-N-((4²Z, 8¹S, 2E, 6S)-1¹-ethyl-(Rₐ)-1⁵-(2-((S)-1-methoxyethyl)-5-(3-morpholinoprop-1-yn-1-yl)pyridin-3-yl)-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z172, 80 mg, yield: 68.7%) as a white solid. ES-API: [M+H]⁺ = 826.3. ¹H NMR (400 MHz, DMSO-d₆) δ 8.72 (d, *J =* 2.0 Hz, 1H), 8.34 (d, *J* = 9.2 Hz, 1H), 7.71 (d, *J =* 2.0 Hz, 1H), 7.43 (s, 1H), 7.35 (d, *J =* 16.0 Hz, 1H), 7.13 (s, 1H), 6.80 (d, *J =* 16.0 Hz, 1H), 5.53 (t, *J =* 8.0 Hz, 1H), 5.01-4.96 (m, 1H), 4.25 - 4.19 (m, 2H), 3.93 - 3.86 (m, 3H), 3.62 - 3.52 (m, 8H), 3.22-3.21 (m, 4H), 3.12-3.06 (m, 1H), 2.75-2.69 (m, 1H), 2.60-2.53 (m, 5H), 2.22 (d, *J* = 14.4 Hz, 1H), 1.95 (d, *J =* 10.0 Hz, 1H), 1.76-1.73 (m, 1H), 1.64-1.61 (m, 1H), 1.44-1.43 (m, 1H), 1.34 (d, *J =* 6.0 Hz, 3H), 1.22-1.13 (m, 3H), 1.07 - 1.04 (m, 9H), 0.70 (s, 3H), 0.31 (s, 3H).

### Example 51. Synthesis of Compound Z75

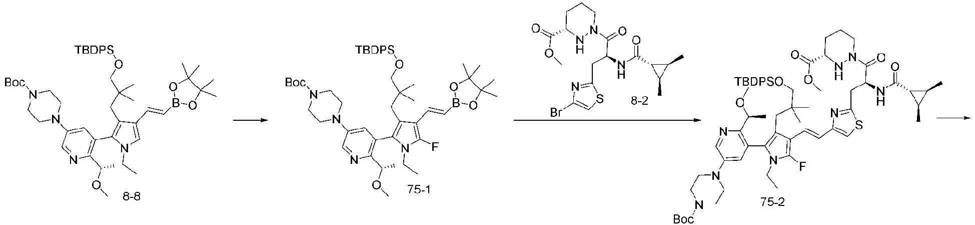

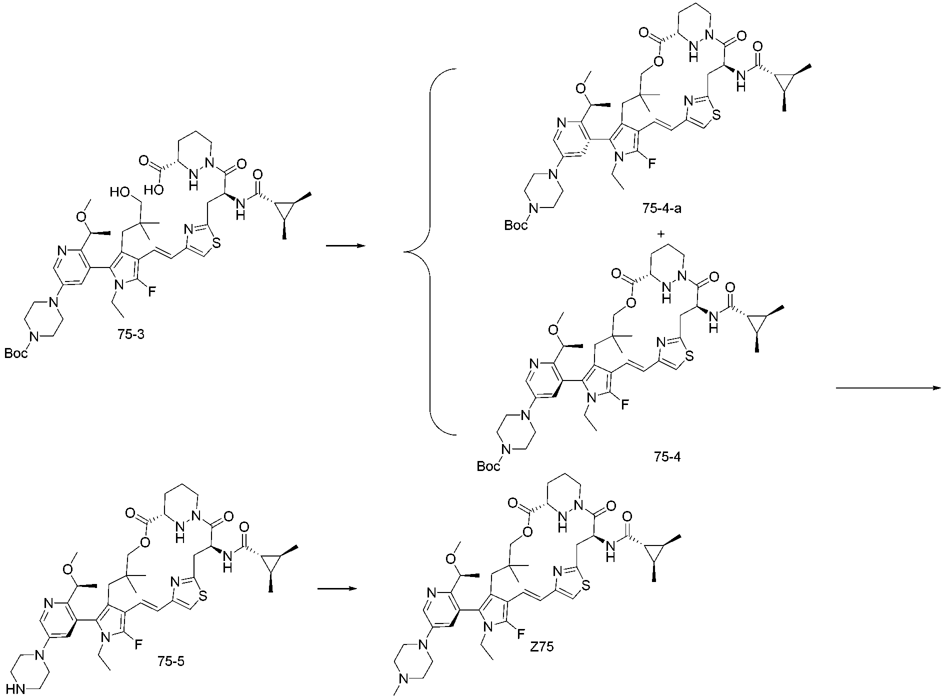

Step 1: Compound 8-8 (450 mg, 0.51 mmol) was dissolved in acetonitrile (10 mL) under an ice bath, and 1-chloromethyl-4-fluoro-1,4-diazabicyclo[2.2.2]octane bis(tetrafluoroborate) (179 mg, 0.51 mmol) was added. The reaction system was stirred for 10 min. The reaction mixture was directly purified by flash silica gel column chromatography (0-60% tetrahydrofuran/petroleum ether) to give compound 75-1 (250 mg) as a yellow oil. ES-API: [M+H]⁺ = 909.3.

Step 2: A mixed solution of compound 75-1 (250 mg, 0.28 mmol), compound 8-2 (169 mg, 0.36 mmol), [1,1'-bis(di-tert-butylphosphino)ferrocene]dichloropalladium(II) (18 mg, 0.03 mmol), and potassium phosphate (175 mg, 0.83 mmol) in toluene (4 mL), dioxane (4 mL), and water (2 mL) was reacted at 85 °C for 2 h under nitrogen atmosphere. Ethyl acetate (20 mL) was added to the reaction mixture, and the resulting mixture was washed with saturated brine (10 mL) and water (10 mL). The organic phase was dried over anhydrous sodium sulfate, filtered, and concentrated, and the residue was purified by flash silica gel column chromatography (0-55% (tetrahydrofuran:dichloromethane = 5:4)/petroleum ether) to give compound 75-2 (150 mg). ES-API: [M+H]⁺ = 1175.2.

Step 3: Compound 75-2 (120 mg, 0.1 mmol) in a 1 M solution of tetrabutylammonium fluoride in tetrahydrofuran (2 mL, 2.00 mmol) was stirred at 70 °C for 5 h. The reaction mixture was dissolved in a saturated ammonium chloride solution (5 mL) and extracted three times with ethyl acetate (5 mL). The organic phases were combined, dried over anhydrous sodium sulfate, filtered, and concentrated by rotary evaporation to give compound 75-3 (90 mg). ES-API: [M+H]⁺ = 923.3.

Step 4: Compound 75-3 (90 mg, 0.1 mmol) was dissolved in dichloromethane (20 mL), and N,N-diisopropylethylamine (0.51 mL, 2.93 mmol), 1-hydroxybenzotriazole (105 mg, 0.78 mmol), and 1-(3-dimethylaminopropyl)-3-ethylcarbodiimide hydrochloride (467 mg, 2.44 mmol) were added. The mixture was stirred at room temperature overnight. The reaction mixture was washed with water, concentrated, and purified by preparative chromatography (tetrahydrofuran:petroleum ether = 1:1) to give compound 75-4 (29 mg, 3 min, LCMS retention time 1.98 min, yield: 33%, structure arbitrarily designated) as a white solid, ES-API: [M+H]⁺ = 905.3, and isomer 75-4-a (20 mg, 3 min, LCMS retention time: 1.94 min, yield: 23%, structure arbitrarily designated) as a white solid. ES-API: [M+H]⁺ = 905.3.

Step 5: Trifluoroacetic acid (0.1 mL) was added dropwise to a solution of compound 75-4 (29 mg, 0.032 mmol) in dichloromethane (0.2 mL). The mixture was stirred at room temperature for 1 h. The reaction was quenched with a saturated sodium bicarbonate solution (1 mL), and the reaction mixture was extracted with ethyl acetate (1 mL) and concentrated to give compound 75-5 (25 mg). ES-API: [M+H]⁺ = 805.2.

Step 6: Compound 75-5 (25 mg, 0.031 mmol) was dissolved in acetonitrile (1 mL), and one drop of 37% aqueous formaldehyde solution and acetic acid (2 mg) were added under an ice bath, followed by the addition of sodium borohydride acetate (20 mg, 0.093 mmol). The mixture was stirred at room temperature for 1 h. After the reaction was completed, the reaction was quenched with 3 mL of saturated sodium bicarbonate solution, and the reaction mixture was extracted three times with 5 mL of ethyl acetate. The organic phases were combined, dried over anhydrous sodium sulfate, and filtered, and concentrated by rotary evaporation, and the residue was purified by prep-HPLC(ammonium bicarbonate method 1) to give (1r,2S,3R)-*N-*((4²Z,8³R,2E,6R)-1¹-ethyl-1²-fluoro-1⁵-(2-((S)-1-methoxyethyl)-5-(4-methylpiperazin-1-yl)pyridin-3-yl)-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrrolacyclotridecaphan-2-en-6-yl)-2,3-dimethylcyclopropane-1-carboxamide (Z75, 3.7 mg, yield: 13%). ES-API: [M+H]⁺ = 820.3. ¹H NMR (400 MHz, DMSO-d₆) δ 8.43 - 8.30 (m, 2H), 7.30 - 7.21 (m, 2H), 7.16 (d, *J* = 2.8 Hz, 1H), 6.77 (d, *J =* 16.0 Hz, 1H), 5.53 (t, *J =* 8.4 Hz, 1H), 5.02 (d, *J =* 12.0 Hz, 1H), 4.27 - 4.11 (m, 2H), 3.96 - 3.78 (m, 3H), 3.70 (d, *J =* 10.8 Hz, 1H), 3.54 (d, *J* = 10.9 Hz, 1H), 3.30 - 3.21 (m, 5H), 3.19 (s, 3H), 3.13 - 3.05 (m, 1H), 2.78 - 2.64 (m, 1H), 2.57 - 2.53 (m, 1H), 2.48 - 2.42 (m, 4H), 2.31 (d, *J =* 14.0 Hz, 1H), 2.22 (s, 3H), 2.01 - 1.87 (m, 1H), 1.81 - 1.55 (m, 2H), 1.51 - 1.39 (m, 1H), 1.35 (d, *J* = 6.0 Hz, 3H), 1.21 - 1.11 (m, 3H), 1.09 - 1.01 (m, 6H), 0.97 (t, *J* = 7.2 Hz, 3H), 0.70 (s, 3H), 0.34 (s, 3H).ES-API: [M+H]⁺= 819.3.

### Example 52. Synthesis of Compound Z229

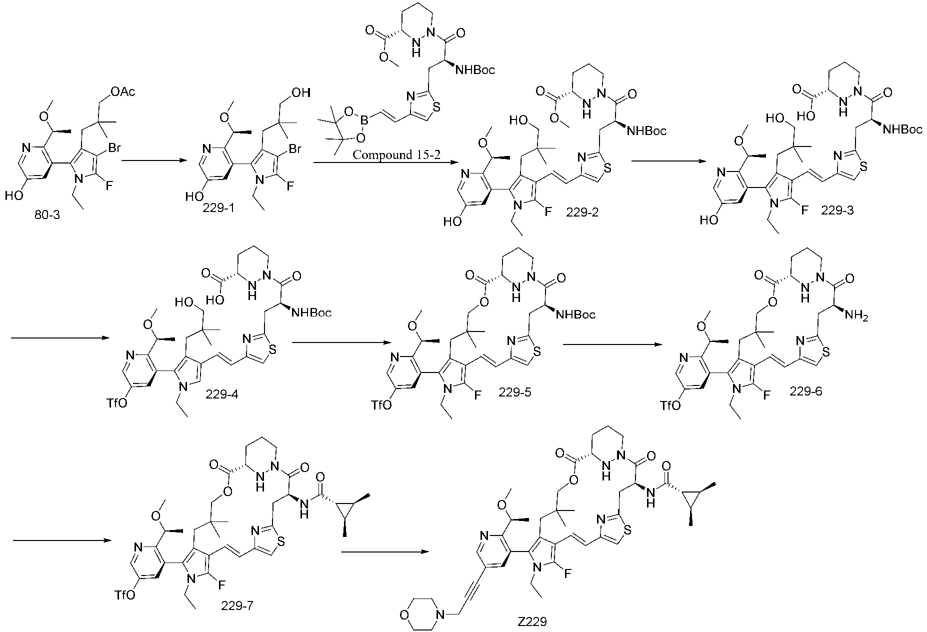

Step 1: Compound 80-3 (2.3 g, 4.89 mmol) was dissolved in tetrahydrofuran (50 mL), methanol (10 mL), and water (20 mL), and lithium hydroxide monohydrate (0.51 g, 12.20 mmol) was added at 0 °C. The reaction system was stirred at room temperature for 18 h. The reaction mixture was adjusted to pH 7 with 1 M diluted hydrochloric acid and extracted with ethyl acetate (50 mL × 2). The organic phase was dried over anhydrous sodium sulfate and concentrated. The crude product was purified by flash silica gel column chromatography (methanol/dichloromethane: 0-5%) to give compound 229-1 (1.69 g, yield: 80.7%) as a white solid. ES-API: [M+H]⁺ = 429.1, 431.1.

Step 2: Compound 229-1 (1.64 g, 3.82 mmol) and compound 15-2 (3.36 g, 6.11 mmol) were dissolved in toluene (17 mL), dioxane (50 mL), and water (17 mL), and potassium phosphate (2.03 g, 9.55 mmol) and [1,1'-bis(di-tert-butylphosphino)ferrocene]dichloropalladium(II) (0.25 g, 0.38 mmol) were added. The reaction system was purged 3 times with nitrogen and stirred at 85 °C for 23 h. Ethyl acetate (150 mL) and water (10 mL) were added to the reaction mixture. The separated organic phase was washed with saturated brine (60 mL), dried over anhydrous sodium sulfate, and filtered, and the filtrate was concentrated under reduced pressure. The crude product was purified by flash silica gel column chromatography (tetrahydrofuran/petroleum ether: 0-70%) to give compound 229-2 (2.45 g, yield: 83.0%) as a light yellow solid. ES-API: [M+H]⁺ = 773.2.

Step 3: Compound 229-2 (2.45 g, 3.17 mmol) was dissolved in tetrahydrofuran (25 mL) and water (10 mL), and lithium hydroxide monohydrate (0.27 g, 6.34 mmol) was added at 0 °C. The reaction system was stirred at room temperature for 1 h. The reaction mixture was adjusted to pH 6 with 1 M diluted hydrochloric acid and extracted with ethyl acetate (80 mL × 2). The organic phase was dried over anhydrous sodium sulfate and concentrated to give compound 229-3 (2.40 g, yield: 99.8%) as a yellow solid. ES-API: [M+H]⁺ = 759.3. Step 4: Compound 229-3 (2.2 g, 2.90 mmol) was dissolved in dichloromethane (40 mL), and diisopropylethylamine (1.50 g, 11.60 mmol) and 1,1,1-trifluoro-N-phenyl-N-[(trifluoromethyl)sulfonyl]methanesulfonamide (1.55 g, 4.35 mmol) were added. The reaction system was stirred at room temperature for 4 h. Dichloromethane (50 mL) was added to the reaction mixture, and the resulting mixture was washed with 0.5 M diluted hydrochloric acid (15 mL × 2) and saturated brine (30 mL), dried over anhydrous sodium sulfate, and filtered, and the filtrate was concentrated under reduced pressure. The crude product was purified by flash silica gel column chromatography (methanol/dichloromethane: 0-30%) to give compound 229-4 (2.37 g, yield: 91.8%) as a light brown solid. ES-API: [M+H]⁺ = 891.2.

Step 5: Compound 229-4 (2.37 g, 2.66 mmol) was dissolved in dichloromethane (150 mL), and 1-(3-dimethylaminopropyl)-3-ethylcarbodiimide hydrochloride (7.65 g, 39.90 mmol), 1-hydroxybenzotriazole (1.80 g, 13.30 mmol), and diisopropylethylamine (6.88 g, 53.20 mmol) were added at room temperature. The reaction system was stirred at room temperature for 18 h. The reaction mixture was washed with 0.5 M diluted hydrochloric acid (50 mL × 2) and saturated sodium bicarbonate (50 mL), dried over anhydrous sodium sulfate, and filtered. The filtrate was concentrated under reduced pressure. The crude product was purified by flash silica gel column chromatography (tetrahydrofuran/petroleum ether: 0-35%) to give compound 229-5 (1.55 g, yield: 66.8%) as a white solid. ES-API: [M+H]⁺ = 873.1.

Step 6: Compound 229-5 (100 mg, 0.115 mmol) was dissolved in dichloromethane (2.5 mL), and trifluoroacetic acid (0.5 mL) was added at 0 °C. The reaction system was stirred at room temperature for 1 h. The reaction mixture was concentrated, and a saturated sodium bicarbonate solution (5 mL) was added. The mixture was extracted with dichloromethane (15 mL × 2). The organic phases were combined, washed with saturated brine (15 mL), dried over anhydrous sodium sulfate, and filtered. The filtrate was concentrated under reduced pressure to give compound 229-6 (88 mg, yield: 99.4%) as a light yellow solid. ES-API: [M+H]⁺ = 773.2.

Step 7: Compound 229-6 (88 mg, 0.114 mmol) was dissolved in dichloromethane (5 mL), and (1r,2R,3S)-2,3-dimethylcyclopropane-1-carboxylic acid (17 mg, 0.148 mmol), O-(7-azabenzotriazol-1-yl)-N,N,N',N'-tetramethyluronium hexafluorophosphate (56 mg, 0.148 mmol), and N,N'-diisopropylethylamine (44 mg, 0.342 mmol) were added. The reaction system was stirred at room temperature for 1 h. Dichloromethane (20 mL) was added to the reaction mixture. The resulting mixture was washed sequentially with water (5 mL) and saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-40%) to give compound 229-7 (80 mg, yield: 80.8%) as a white solid. ES-API: [M+H]⁺ = 869.2.

Step 8: Compound 229-7 (200 mg, 0.23 mmol) and 1-(morpholin-4-yl)-2-propyne (86 mg, 0.69 mmol) were dissolved in acetonitrile (10 mL), and bis(triphenylphosphine)palladium(II) dichloride (24 mg, 0.035 mmol), copper(I) iodide (10 mg, 0.053 mmol), N,N'-diisopropylethylamine (119 mg, 0.92 mmol), and lithium chloride (39 mg, 0.92 mmol) were added. The reaction system was stirred at 85 °C for 2 h under nitrogen atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by prep-HPLC(ammonium bicarbonate method 1) to give (1r, 2R, 3S)-N-((4²Z, 8³S, 2E,6S)-1¹-ethyl-1²-fluoro-(Rₐ)-1⁵-(2-((S)-1-methoxyethyl)-5-(3-morpholinoprop-yn-1-yl)pyridin-3-yl)-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z229, 105 mg, yield: 54.0%) as a white solid. ¹H NMR (400 MHz, DMSO-d₆) δ 8.74 (d, *J* = 1.6 Hz, 1H), 8.34 (d, *J =* 8.8 Hz, 1H), 7.80 (d, *J =* 1.6 Hz, 1H), 7.34 - 7.16 (m, 2H), 6.78 (d, *J* = 16.0 Hz, 1H), 5.53 (t, *J* = 8.8 Hz, 1H), 5.02 (d, *J* = 12.0 Hz, 1H), 4.33 - 4.14 (m, 2H), 3.96 - 3.66 (m, 4H), 3.64 - 3.48 (m, 7H), 3.27 - 3.17 (m, 4H), 3.14 - 3.04 (m, 1H), 2.77 - 2.66 (m, 1H), 2.60 - 2.51 (m, 5H), 2.26 (d, *J =* 14.0 Hz, 1H), 1.94 (d, *J =* 10.8 Hz, 1H), 1.82 - 1.55 (m, 2H), 1.51 - 1.30 (m, 4H), 1.22 - 1.11 (m, 3H), 1.10 - 1.00 (m, 6H), 0.95 (t, *J* = 6.8 Hz, 3H), 0.70 (s, 3H), 0.34 (s, 3H).ES-API: [M+H]⁺= 844.3.

### Example 53. Synthesis of Compound Z139

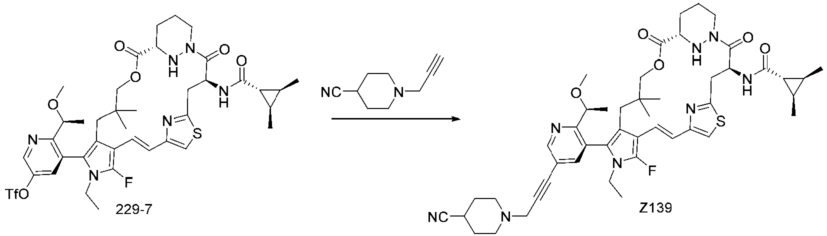

Step 1: Compound 229-7 (200 mg, 0.23 mmol) and 1-(prop-2-yn-1-yl)piperidine-4-carbonitrile (68 mg, 0.46 mmol) were dissolved in acetonitrile (10 mL), and bis(triphenylphosphine)palladium(II) dichloride (24 mg, 0.035 mmol), copper(I) iodide (10 mg, 0.053 mmol), N,N'-diisopropylethylamine (119 mg, 0.92 mmol), and lithium chloride (39 mg, 0.92 mmol) were added. The reaction system was stirred at 85 °C for 2 h under nitrogen atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by prep-HPLC (ammonium bicarbonate method 1) to give the desired product (1r, 2R, 3S)-N-((4²Z, 8³S,2E,6S)-(Rₐ)-1⁵-(5-(3-(4-cyanopiperidin-1-yl)prop-1-yn-1-yl)-2-((S)-1-methoxyethyl)pyridin-3-yl)-1¹-ethyl-1²-fluoro-12,12-dimethyl-7,9-dioxo-8¹,8¹,8¹,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z139, 120 mg, yield: 60.2%) as a white solid. ¹H NMR (400 MHz, DMSO-d₆) δ 8.74 (d, *J =* 1.6 Hz, 1H), 8.34 (d, *J =* 8.8 Hz, 1H), 7.79 (d, *J* = 1.6 Hz, 1H), 7.32 - 7.16 (m, 2H), 6.78 (d, *J =* 16.0 Hz, 1H), 5.53 (t, *J =* 8.4 Hz, 1H), 5.02 (d, *J =* 12.0 Hz, 1H), 4.38 - 4.16 (m, 2H), 3.99 - 3.65 (m, 4H), 3.62 - 3.48 (m, 3H), 3.27 - 3.16 (m, 4H), 3.14 - 3.04 (m, 1H), 2.96 - 2.85 (m, 1H), 2.80 - 2.64 (m, 3H), 2.62 - 2.53 (m, 1H), 2.48 - 2.40 (m, 2H), 2.26 (d, *J =* 13.6 Hz, 1H), 2.01 - 1.83 (m, 3H), 1.80 - 1.56 (m, 4H), 1.53 - 1.30 (m, 4H), 1.21 - 1.10 (m, 3H), 1.10 - 1.01 (m, 6H), 0.95 (t, *J =* 6.8 Hz, 3H), 0.70 (s, 3H), 0.34 (s, 3H).ES-API: [M+H]⁺= 867.3.

### Example 54. Synthesis of Compound Z145

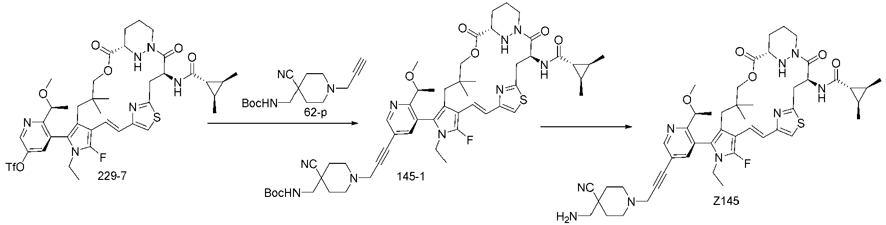

Step 1: Compound 229-7 (180 mg, 0.21 mmol) and intermediate 62-p (115 mg, 0.41 mmol) were dissolved in acetonitrile (10 mL), and bis(triphenylphosphine)palladium(II) dichloride (22 mg, 0.031 mmol), copper(I) iodide (9 mg, 0.048 mmol), N,N'-diisopropylethylamine (107 mg, 0.83 mmol), and lithium chloride (35 mg, 0.83 mmol) were added. The reaction system was stirred at 85 °C for 2 h under nitrogen atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by flash silica gel column chromatography (tetrahydrofuran/petroleum ether: 20-80%) to give compound 145-1 (200 mg, yield: 96.9%) as a light brown solid. ES-API: [M+H]⁺ = 996.3.

Step 2: Compound 145-1 (200 mg, 0.20 mmol) was dissolved in dichloromethane (4 mL), and trifluoroacetic acid (1 mL) was added at 0 °C. The reaction system was stirred at room temperature for 1 h. The reaction mixture was concentrated, and a saturated sodium bicarbonate solution (10 mL) was added. The mixture was extracted with dichloromethane (40 mL × 2). The organic phases were combined, washed with saturated brine (15 mL), dried over anhydrous sodium sulfate, and filtered, and the filtrate was concentrated under reduced pressure. The crude product was purified by flash silica gel column chromatography (7 M ammoniamethanol solution/dichloromethane: 0-8%) to give the desired product (1r,2R,3S)-N-((4²Z, 8³S, 2E, 6S)-(Rₐ)-1⁵-(5-(3-(4-(aminomethyl)-4-cyanopiperidin-1-yl)prop-1-yn-1-yl)-2-((S)-1-methoxyethy)pyridin-3-yl)-1¹-ethyl-1²-fluoro-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-2,3-dimethylcyclopropane-1-carboxamide (Z145, 150 mg, yield: 83.4%) as a light yellow solid. ES-API: [M+H]⁺ = 896.3. ¹H NMR (400 MHz, DMSO-d₆) δ 8.73 (d, *J =* 1.6 Hz, 1H), 8.34 (d, *J =* 8.8 Hz, 1H), 7.78 (d, *J =* 1.6 Hz, 1H), 7.29 - 7.19 (m, 2H), 6.78 (d, *J =* 16.0 Hz, 1H), 5.53 (t, *J =* 8.0 Hz, 1H), 5.02 (d, *J =* 12.0 Hz, 1H), 4.29 (q, *J =* 6.0 Hz, 1H), 4.21 (d, *J =* 11.2 Hz, 1H), 3.96 - 3.67 (m, 4H), 3.61 (s, 2H), 3.54 (d, *J =* 11.2 Hz, 1H), 3.25 - 3.18 (m, 4H), 3.13 - 3.06 (m, 1H), 2.88 (d, *J* = 11.6 Hz, 2H), 2.77 - 2.65 (m, 3H), 2.57 - 2.52 (m, 1H), 2.48 - 2.39 (m, 2H), 2.26 (d, *J =* 14.4 Hz, 1H), 2.10 - 1.80 (m, 5H), 1.74 (d, *J =* 12.0 Hz, 1H), 1.69 - 1.57 (m, 1H), 1.55 - 1.40 (m, 3H), 1.38 - 1.32 (m, 3H), 1.20 - 1.11 (m, 3H), 1.09 - 1.02 (m, 6H), 0.95 (t, *J =* 7.2 Hz, 3H), 0.69 (s, 3H), 0.34 (s, 3H).

### Example 55. Synthesis of Compound Z65

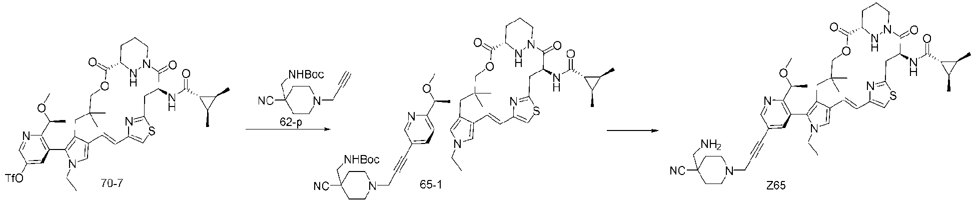

Step 1: Compound 70-7 (350 mg, 0.411 mmol) and intermediate 62-p (228 mg, 0.82 mmol) were dissolved in acetonitrile (10 mL), and bis(triphenylphosphine)palladium(II) dichloride (43 mg, 0.06 mmol), copper(I) iodide (18.2 mg, 0.095 mmol), N,N'-diisopropylethylamine (212 mg, 1.65 mmol), and lithium chloride (69 mg, 1.64 mmol) were added. The reaction system was stirred at 85 °C for 2 h under nitrogen atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product. The crude product was purified by flash silica gel column chromatography (tetrahydrofuran/petroleum ether: 0-60%) to give compound 65-1 (340 mg, 0.348 mmol, yield: 84.50%) as a light yellow solid. ES-API: [M+H]⁺ = 978.4.

Step 2: Compound 65-1 (340 mg, 0.348 mmol) was dissolved in dichloromethane (5 mL), and trifluoroacetic acid (2 mL) was added under an ice-water bath. The reaction system was stirred at 85 °C for 2 h under nitrogen atmosphere and concentrated under reduced pressure. Dichloromethane (15 mL) and a saturated sodium bicarbonate solution (10 mL) were added, and the organic phase was washed with water (15 mL) and saturated brine (15 mL), dried over anhydrous sodium sulfate, and concentrated to give a crude product. The crude product was purified by flash silica gel column chromatography (methanol/dichloromethane: 0-10%) to give (1*r*,2*R*,3*S*)-N-((4²*Z*,8³*S*,2*E*,6*S*)-(Rₐ)-1⁵-(5-(3-(4-(aminomethyl)-4-cyanopiperidin-1-yl)prop-1-yn-1-yl)-2-((S)-1-methoxyethyl)pyridin-3-yl-1¹-ethyl-12,12-dimethyl-7,9-dioxo-8¹,8²,8³,8⁴,8⁵,8⁶-hexahydro-1¹*H-*10-oxa-4(4,2)-thiazola-8(1,3)-pyridazina-1(3,4)-pyrrolacyclotridecaphan-2-en-6-yl)-2,3-dimethylcyclopropane-1-carboxamide (Z65, 250 mg, 0.285 mmol, yield: 81.91%) as a white solid. ES-API: [M+H]⁺ = 878.3. ES-API: [M+H]⁺ = 878.3. ¹H NMR (400 MHz, DMSO-d₆) δ 8.71 (d, *J =* 2.0 Hz, 1H), 8.33 (d, *J* = 9.2 Hz, 1H), 7.68 (d, *J* = 2.0 Hz, 1H), 7.43 (s, 1H), 7.34 (d, *J =* 15.6 Hz, 1H), 7.13 (s, 1H), 6.80 (d, *J* = 15.6 Hz, 1H), 5.53 (t, *J =* 8.8 Hz, 1H), 5.01 (d, *J =* 12.0 Hz, 1H), 4.25-4.22 (m, 2H), 3.92-3.78 (m, 3H), 3.69-3.51 (m,4H), 3.23-3.21 (m, 4H), 3.13-3.09 (m, 1H), 2.88 (d, *J =* 12.0 Hz, 2H), 2.71-2.66 (m, 4H), 2.57-2.54 (m, 1H), 2.45-2.41 (m, 2H), 2.25-2.22 (m, 1H), 1.93-1.91 (m, 3H), 1.73-1.70 (m, 1H), 1.51-1.48 (m, 4H), 1.34 (d, *J =* 6.0 Hz, 3H), 1.25-1.23 (m, 1H), 1.19-1.14 (m, 3H), 1.06-1.03 (m, 9H), 0.70 (s, 3H), 0.31 (s, 3H).

### Example 56. Synthesis of Compound Z66

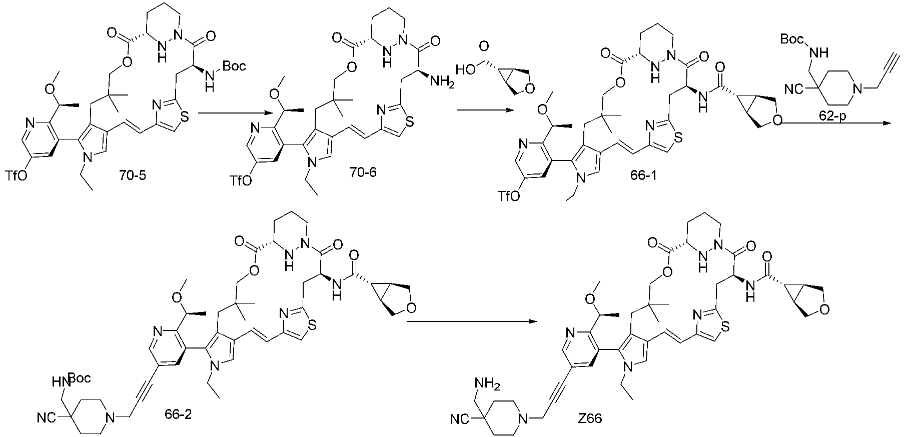

Step 1: A solution of hydrochloric acid in dioxane (2 mL, 4 M, 8.0 mmol) was added to a solution of compound 70-5 (300.0 mg, 0.351 mmol) in methanol (2 mL), and the reaction mixture was stirred at room temperature for 1.0 h. After the reaction was completed as monitored by LC-MS, the reaction system was concentrated to give crude compound 70-6 (310.0 mg, crude product). ES-API: [M+H]⁺ = 755.2.

Step 2: N,N-diisopropylethylamine (453.67 mg, 3.510 mmol) was added to a solution of compound 70-6 (310.0 mg, crude product) and (1R,5S,6r)-3-oxabicyclo[3.1.0]hexane-6-carboxylic acid (67.46 mg, 0.526 mmol) in dry N,N-dimethylformamide (2.0 mL) at 0 °C, and then O-(7-azabenzotriazol-1-yl)-N,N,N',N'-tetramethyluronium hexafluorophosphate (400.0 mg, 1.053 mmol) was added. The reaction system was warmed to room temperature and reacted for 10 min. After the reaction was completed as monitored by LCMS, the reaction mixture was diluted with ethyl acetate (100 mL) and washed with saturated brine (30 mL × 5). The organic phase was concentrated, and the crude product was purified by flash silica gel column chromatography (methanol/dichloromethane = 0-2%) to give compound 66-1 (240.0 mg, 0.277 mmol, yield: 79.05%). ES-API: [M+H]⁺ = 865.2.

Step 3: Intermediate 62-p (60.0 mg, 0.216 mmol) was added to a solution of compound 66-1 (63.0 mg, 0.073 mmol) in dry N,N-dimethylformamide (2.0 mL), and then bis(triphenylphosphine)palladium(II) dichloride (15.0 mg, 0.021 mmol), copper(I) iodide (10.0 mg, 0.052 mmol), N,N-diisopropylethylamine (40.0 mg, 0.310 mmol), and lithium chloride (15.0 mg, 0.357 mmol) were added. The reaction mixture was stirred at 85 °C for 1.5 h under nitrogen atmosphere. After the reaction was completed as detected by LCMS, the reaction mixture was diluted with ethyl acetate (15 mL) and washed with water (15 mL) and saturated brine (15 mL × 2). The organic phase was dried over anhydrous sodium sulfate and filtered, and the filtrate was concentrated to dryness. The crude product was purified by flash silica gel column chromatography (petroleum ether/tetrahydrofuran = 100:0 to 20:80, v/v) to give compound 66-2 (60.0 mg, 0.060 mmol, yield: 83.02%). ES-API: [M+H]⁺ = 992.3.

Step 4: Trifluoroacetic acid (0.5 mL, 6.530 mmol) was added to a solution of compound 66-2 (60.0 mg, 0.060 mmol) in dichloromethane (2.0 mL) at room temperature. The mixture was reacted for 1 h, and then concentrated by rotary evaporation under reduced pressure to remove the solvent to give a crude product, which was purified by prep-HPLC(ammonium bicarbonate method 1) to give (1*R*,5*S*,6*r*)-N-((4²*Z*,8³*S*,2*E*,6*S*)-(Rₐ)-1⁵-(5-(3-(4-(aminomethyl)-4-cyanopiperidin-1-yl)prop-1-yn-1-yl)-2-((S)-1-methoxyethyl)pyridin-3-yl-1¹-ethyl-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrrolacyclotridecaphan-2-en-6-yl)-3-oxabicyclo[3.1.0]hexane-6-carboxamide (Z66, 25.0 mg, yield: 46.34%). ES-API: [M+H]⁺ = 892.3. ¹H NMR (400 MHz, DMSO-d₆) δ 8.47-8.40 (m, 1H), 8.28-8.26 (m, 1H), 7.45~7.42 (m, 1H), 7.19 (s, 1H), 7.12~7.08(m, 1H), 6.89 (s, 1H), 6.58~6.54(m, 1H), 5.33-5.28 (m, 1H), 4.79∼4.75 (m, 1H), 3.99∼3.96 (m, 2H), 3.68∼3.60 (m, 1H), 3.57∼3.55 (m, 4H), 3.42∼3.35 (m, 1H), 3.29∼3.26 (m, 1H), 3.09 (s, 10H), 2.96 (s, 3H), 2.88 - 2.79 (m, 1H), 2.65∼2.62 (m, 2H), 2.47∼2.43(m, 3H), 2.22∼2.18(m, 2H), 2.01∼1.95 (m, 1H), 1.67∼1.64 (m, 7H), 1.49 (s, 1H), 1.38 (s, 2H), 1.32∼1.18 (m, 3H), 1.12∼1.09 (m, 3H), 0.83∼0.79 (m, 3H), 0.46 (s, 3H).

### Example 57. Synthesis of Compound Z50

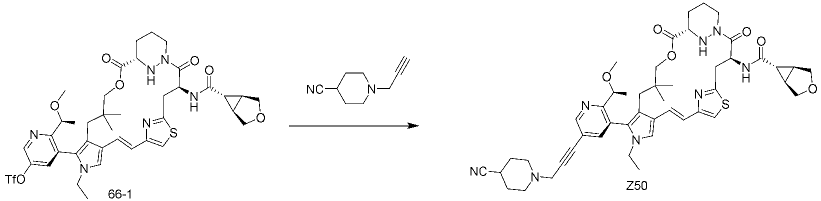

Step 1: Compound 66-1 (35 mg, 0.040 mmol) was dissolved in acetonitrile (2 mL). 1-(Prop-2-yn-1-yl)piperidine-4-carbonitrile (17.99 mg, 0.121 mmol), bis(triphenylphosphine)palladium(II) dichloride (4.26 mg, 0.006 mmol), copper(I) iodide (2.31 mg, 0.012 mmol), N,N-diisopropylethylamine (15.69 mg, 0.121 mmol), and lithium chloride (6.86 mg, 0.162 mmol) were added to the above solution at room temperature. After the addition was completed, the reaction system was stirred at 80 °C for 1.5 h under nitrogen atmosphere. After the reaction was completed, water (20 mL) was added to the reaction mixture, and the resulting mixture was extracted with ethyl acetate (20 mL × 2). The organic phase was washed with saturated brine (20 mL × 2), dried, and concentrated to give a crude product. The crude product was purified by flash silica gel column chromatography (methanol/dichloromethane: 0%-7%) to give (1*R*,5*S*,6*r*)-*N-*((4²*Z*,8³*S*,2*E*,6*S*)-(*R*ₐ)-1⁵-(5-(3-(4-cyanopiperidin-1-yl)prop-1-yn-1-yl)-2-((*S*)-1-methoxyethyl)pyridin-3-yl)-1¹-ethyl-12,12-dimethyl-7,9-dioxo-8¹,8²,8³,8⁴,8⁵,8⁶-hexahydro-1¹*H*-10-oxa-4(4,2)-thiazola-8(1,3)-pyridazina-1(3,4)-pyrrolacyclotridecaphan-2-en-6-yl)-3-oxabicyclo[3.1.0]hexane-6-carboxamide (Z50, 25 mg, 0.029 mmol, yield: 71.58%) as a light yellow solid. ES-API: [M +H]⁺ = 863.2. ¹H NMR (400 MHz, DMSO-d₆) δ 8.47 (d, *J =* 1.6 Hz, 1H), 8.27 (d, *J =* 8.8 Hz, 1H), 7.45 (s, 1H), 7.19 (s, 1H), 7.10 (d, *J =* 15.6 Hz, 1H), 6.88 (s, 1H), 6.56 (d, *J =* 15.6 Hz, 1H), 5.29 (t, *J =* 8.4 Hz, 1H), 4.78 (d, *J =* 12.0 Hz, 1H), 4.03 - 3.91 (m, 2H), 3.67 (t, *J =* 12.0 Hz, 1H), 3.60 - 3.49 (m, 4H), 3.46 - 3.24 (m, 6H), 2.96 (s, 3H), 2.87 - 2.78 (m, 1H), 2.70 - 2.60 (s, 1H), 2.52 - 2.31 (m, 4H), 2.03 - 1.92 (m, 1H), 1.75 - 1.60 (m, 5H), 1.54 - 1.39 (m, 4H), 1.29 - 1.19 (m, 1H), 1.10 (d, *J =* 6.0 Hz, 3H), 1.04 - 0.89 (m, 4H), 0.81 (t, *J =* 7.2 Hz, 3H), 0.47 (s, 3H), 0.07 (s, 3H).

### Example 58. Synthesis of Compound Z104

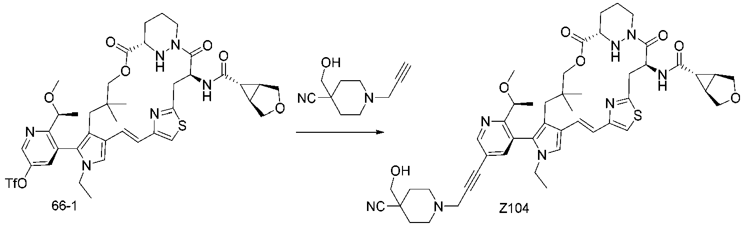

Step 1: Compound 66-1 (20 mg, 0.023 mmol) and 4-(hydroxymethyl)-1-(prop-2-ynyl)hexahydropyridine-4-carbonitrile (12.36 mg, 0.069 mmol) were dissolved in acetonitrile (2 mL), and bis(triphenylphosphine)palladium(II) dichloride (6.48 mg, 0.009 mmol), copper(I) iodide (2.66 mg, 0.014 mmol), N,N'-diisopropylethylamine (0.016 mL, 0.092 mmol), and lithium chloride (3.88 mg, 0.092 mmol) were added. The reaction system was stirred at 85 °C for 2 h under nitrogen atmosphere. Water (20 mL) was added to the reaction mixture, and the resulting mixture was extracted with ethyl acetate (30 mL × 3). The organic phase was washed with saturated brine (20 mL × 3), dried, and concentrated to give a crude product.

The crude product was purified by flash silica gel column chromatography (methanol/dichloromethane: 0-10%) to give (1R,5S,6r)-*N*-((4²Z,8³*S*,2*E*,6*S*)-(*Rₐ*)-1⁵-(5-(3-(4-cyano-4-(hydroxymethyl)piperidin-1-yl)prop-1-yn-1-yl)-2-((S)-1-methoxyethyl)pyridin-3-yl)-1¹ethyl-12,12-dimethyl-7,9-dioxo-8¹,8²,8³,8⁴,8⁵,8⁶-hexahydro-1¹H-10-oxa-4(4,2)-thiazola-8(1,3)-pyridazina-1(3,4)-pyrrolacyclotridecaphan-2-en-6-yl)-3-oxabicyclo[3.1.0]hexane-6-carboxamide (Z104, 10 mg, 0.011 mmol, yield: 48.43%) as a white solid. ES-API: [M+H]⁺ = 893.2. ¹H NMR (400 MHz, DMSO-d₆) δ 8.72 (d, *J =* 2.0 Hz, 1H), 8.52 (d, *J =* 8.8 Hz, 1H), 7.69 (d, *J* = 2.0Hz, 1H), 7.44 (s, 1H), 7.35 (d, *J =* 15.6 Hz, 1H), 7.13 (s, 1H), 6.81 (d, *J* = 15.6 Hz, 1H), 5.55 (t, *J* = 7.6 Hz, 1H), 5.43 (t, *J =* 5.6 Hz, 1H), 5.03 (d, *J =* 12.0 Hz, 1H), 4.27 - 4.17 (m, 2H), 3.94-3.88 (m, 1H), 3.85 - 3.71 (m, 4H), 3.71 - 3.60 (m, 5H), 3.53 (d, *J =* 10.6 Hz, 1H), 3.47 (d, *J =* 5.2 Hz, 2H), 3.28-3.23 (m, 1H), 3.21 (s, 3H), 3.10-3.04 (m, 1H), 2.90-2.87 (m, 2H), 2.76-2.67 (m, 1H), 2.59-2.56 (m, 1H), 2.45-2.42 (m, 2H), 2.24 (d, *J =* 14.0 Hz, 1H), 1.95-1.85 (m, 5H), 1.76-1.74 (m, 1H), 1.63-1.62 (m, 2H), 1.57 - 1.43 (m, 3H), 1.35 (d, *J =* 6.0 Hz, 3H), 1.06 (t, *J =* 7.2 Hz, 3H), 0.71 (s, 3H), 0.32 (s, 3H).

### Example 59. Synthesis of Compound Z85

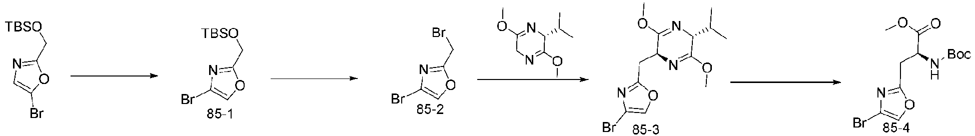

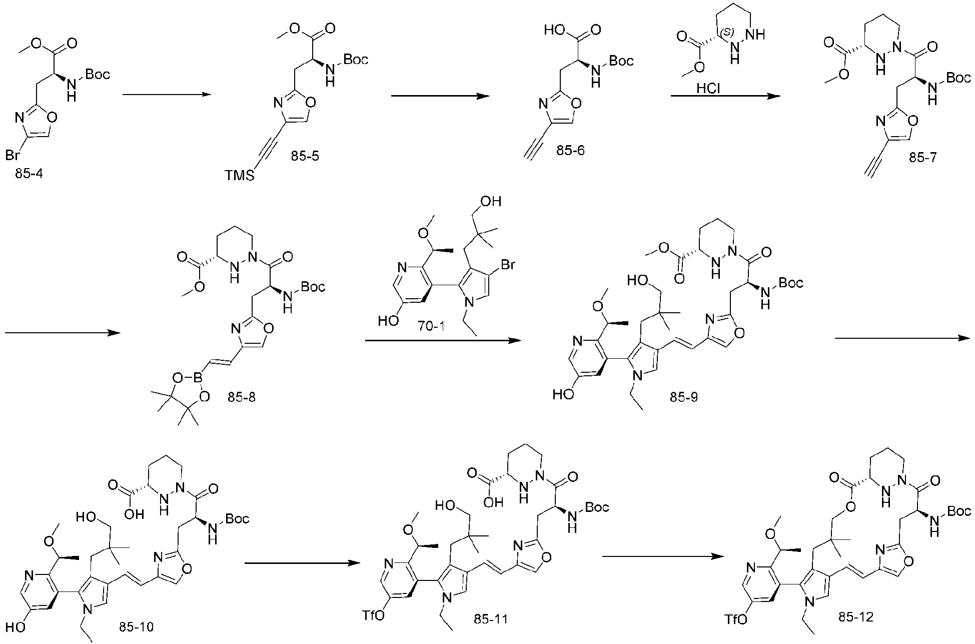

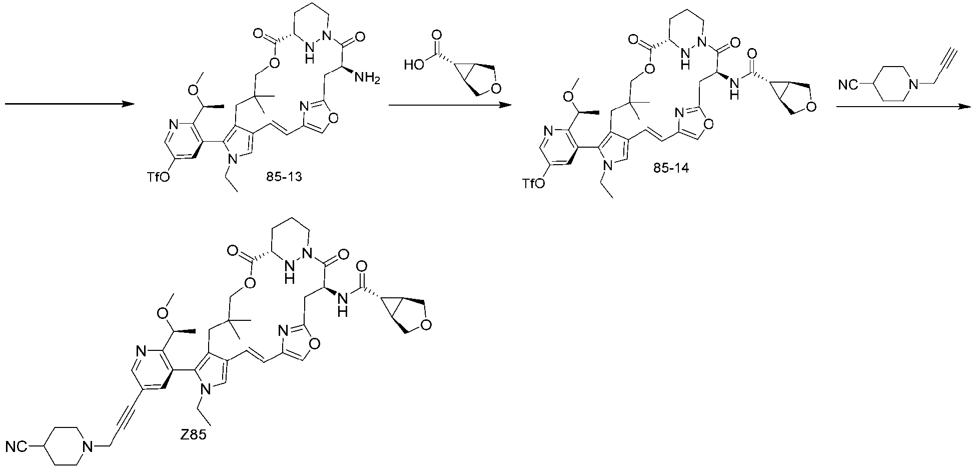

Step 1: A solution of lithium diisopropylamide (12.318 mL, 24.636 mmol) in tetrahydrofuran (2 M) was added dropwise to a solution of 5-bromo-2-(3,3,4,4-tetramethyl-2-oxa-3-silapentan-1-yl)-1,3-oxazole (4.8 g, 16.424 mmol) in tetrahydrofuran (100 mL) under a dry ice bath at -78 °C. After 30 min, wet tetrahydrofuran was added, and the reaction mixture was warmed to room temperature. The reaction mixture was diluted with ethyl acetate (50 mL), washed with a saturated aqueous sodium bicarbonate solution (20 mL × 1) and a saturated aqueous sodium chloride solution (20 mL × 1), dried over anhydrous sodium sulfate, filtered, and concentrated. The crude product was purified by silica gel column chromatography (0-5% ethyl acetate/petroleum ether) to give compound 85-1 (2 g, 6.843 mmol, yield: 41.67%). ES-API: [M+H]⁺ = 292.0,294.0.

Step 2: Phosphorus tribromide (1.042 mL, 15.398 mmol) was added dropwise to a solution of compound 85-1 (1500 mg, 5.133 mmol) in dichloromethane (5 mL) under an ice-water bath. The reaction system was reacted at room temperature for 4 h under nitrogen atmosphere, and then an aqueous sodium bicarbonate solution (50 mL) was slowly added. The mixture was extracted with ethyl acetate (40 mL × 3), washed with an aqueous sodium chloride solution (100 mL × 1), dried over Na₂SO₄, filtered, and concentrated. The crude product was purified by silica gel column chromatography (0-5% ethyl acetate/petroleum ether) to give compound 85-2 (433 mg, 1.8 mmol, yield: 35%). ES-API: [M+H]⁺ = 239.9, 241.9.

Step 3: n-BuLi (0.651 mL, 2.5 M n-hexane) was added dropwise to a solution of (2R)-3,6-dimethyl-2-(prop-2-yl)-2,5-dihydropyrazine (300 mg, 1.628 mmol) in tetrahydrofuran (5 mL) under a dry ice bath at -78 °C, and the mixture was reacted for 30 min under this condition. Then, a solution of compound 85-2 (392.15 mg, 1.628 mmol) in tetrahydrofuran (2 mL) was slowly added dropwise using a syringe. The reaction was continued under this condition for 30 min and quenched with an aqueous ammonium chloride solution (10 mL). The reaction mixture was extracted with ethyl acetate (40 mL × 3), washed with an aqueous NaCl solution (100 mL × 1), dried over Na₂SO₄, filtered, and concentrated. The crude product was purified by silica gel column chromatography (30% ethyl acetate/petroleum ether) to give compound 85-3 (360 mg, 1.05 mmol, yield: 64.4%). ES-API: [M+H]⁺ = 344.1, 346.1.

Step 4: 0.5 M HCl (8.4 mL) was added to a solution of compound 85-3 (360 mg, 1.05 mmol) in acetonitrile (8 mL). The mixture was reacted at room temperature for 3 h. The reaction mixture was cooled to 0 °C, and an aqueous sodium bicarbonate solution (8 mL) and (Boc)₂O (0.719 mL, 3.132 mmol) were added. The reaction mixture was reacted at room temperature for 1 h. The reaction mixture was extracted with ethyl acetate (20 mL × 3), washed with an aqueous NaCl solution (100 mL × 1), dried over Na₂SO₄, filtered, and concentrated. The crude product was purified by silica gel column chromatography (30% ethyl acetate/petroleum ether) to give compound 85-4 (260.52 mg, 1.046 mmol, yield: 100%). ES-API: [M+H]⁺ = 349.0, 351.0.

Step 5: Compound 85-4 (650 mg, 1.592 mmol) was dissolved in acetonitrile (5 mL), and ethynyltrimethylsilane (675.09 mg, 6.873 mmol), copper(I) iodide (32.73 mg, 0.172 mmol), PdCl₂(pph₃)₂ (120.63 mg, 0.172 mmol), and DIPEA (1.197 mL, 6.873 mmol) were added. The mixture was stirred at 80 °C for 5 h. The reaction mixture was diluted with ethyl acetate (50 mL), washed with saturated brine (20 mL × 1), dried over Na₂SO₄, filtered, and concentrated. The crude product was purified by silica gel column chromatography (0-15% tetrahydrofuran/petroleum ether) to give compound 85-5 (450 mg, 1.228 mmol, yield: 71.46%). ES-API: [M+H]⁺ = 367.1.

Step 6: Compound 85-5 (450 mg, 1.228 mmol) was dissolved in tetrahydrofuran (5 mL), methanol (1 mL), and water (1 mL), and then lithium hydroxide monohydrate (51.57 mg, 1.228 mmol) was added. The mixture was stirred at room temperature for 2 h. After the reaction was completed as detected by LC-MS, 1 M HCl was added until pH = 6, and ethyl acetate (10 mL) was added. The organic phase obtained by liquid separation was dried over anhydrous sodium sulfate, filtered, and concentrated to give compound 85-6 (360 mg, crude product). ES-API: [M+H]⁺ = 281.0.

Step 7: Compound 85-6 (360 mg, crude product) was dissolved in dry dichloromethane (5 mL), and NMM (372.60 mg, 3.684 mmol), HOBt (33.18 mg, 0.246 mmol), EDCI (353.08 mg, 1.842 mmol), and methyl (S)-hexahydropyridazine-3-carboxylate hydrochloride (288.33 mg, 1.596 mmol) were added sequentially. The mixture was reacted at room temperature for 1 h. Dichloromethane (30 mL) was added. The mixture was washed with water (20 mL × 1) and saturated brine (20 mL × 1) sequentially, dried over anhydrous sodium sulfate, and concentrated, and the residue was purified by silica gel column chromatography (tetrahydrofuran/petroleum ether = 1/3) to give compound 85-7 (350 mg, 0.861 mmol, yield: 70.13%). ES-API: [M+H]⁺ = 407.2.

Step 8: CuCl (14.61 mg, 0.148 mmol), t-BuONa (14.17 mg, 0.148 mmol), and Xantphos (85.42 mg, 0.148 mmol) were added to a three-necked flask that was dried under nitrogen atmosphere, and then dry tetrahydrofuran (9 mL) was added. The reaction mixture was stirred at 35 °C for 30 min, and then a solution of 4,4,5,5-tetramethyl-2-(4,4,5,5-tetramethyl-1,3,2-dioxaborolan-2-yl)-1,3,2-dioxaborolane (337.39 mg, 1.329 mmol) in tetrahydrofuran (2 mL) was added. After the reaction mixture was stirred for 10 min, a solution of compound 85-7 (300 mg, 0.738 mmol) and methanol (0.060 mL, 1.476 mmol) in tetrahydrofuran (2 mL) was added. The mixture was reacted at 35 °C for 3 h. After the reaction was completed, the reaction mixture was filtered through celite and concentrated, and the residue was purified by silica gel column chromatography (petroleum ether/ethyl acetate = 1/5) to give compound 85-8 (250 mg, 0.468 mmol, yield: 63.38%), ES-API: [M+H]⁺ = 535.3.

Step 9: Compound 85-8 (250 mg, 0.468 mmol) and compound 70-1 (250.15 mg, 0.608 mmol) were dissolved in 1,4-dioxane (6 mL), toluene (2 mL), and water (2 mL), and potassium phosphate (248.04 mg, 1.170 mmol) and Pd(dtbpf)Cl₂ (30.47 mg, 0.047 mmol) were added. The mixture was stirred at 85 °C for 3 h under nitrogen atmosphere. Then, water (20 mL) was added to quench the reaction, and the resulting mixture was extracted with ethyl acetate (20 mL × 2). The organic layer was washed with brine (30 mL × 2), dried over Na₂SO₄, filtered, and concentrated under reduced pressure. The residue was purified by silica gel column chromatography (0-8% MeOH/DCM for elution) to give compound 85-9 (230 mg, 0.311 mmol, yield: 66.54%), ES-API: [M+H]⁺ = 739.3.

Step 10: Compound 85-9 (230 mg, 0.311 mmol) was dissolved in tetrahydrofuran (3 mL), methanol (1 mL), and water (1 mL), and lithium hydroxide monohydrate (14.37 mg, 0.342 mmol) was added. The mixture was stirred at room temperature for 2 h under nitrogen atmosphere. Then, a 1 M aqueous HCl solution was added until pH = 5-6, and the resulting mixture was extracted with EtOAc (20 mL × 3). The organic layer was washed with brine (10 mL × 1), dried over Na₂SO ₄, filtered, and concentrated under reduced pressure to give compound 85-10 (205 mg, crude product), ES-API: [M+H]⁺ = 725.3.

Step 11: DIPEA (0.159 mL, 0.911 mmol) and N-phenyl-bis(trifluoromethanesulfonimide) (184.62 mg, 0.517 mmol) were added to a solution of compound 85-10 (205 mg, crude product) in dichloromethane (10 mL), and the mixture was stirred at room temperature for 5 h under a balloon of N₂. The reaction mixture was diluted with dichloromethane (30 mL), washed with a 0.5 M aqueous HCl solution (5 mL × 2) and brine (20 mL), dried over Na₂SO₄, filtered, and concentrated, and the residue was purified by silica gel column chromatography (0-30% MeOH/DCM for elution) to give compound 85-11 (130 mg, 0.149 mmol, two-step yield: 47.9%). ES-API: [M+H]⁺ = 857.2.

Step 12: Compound 85-11 (130 mg, 0.149 mmol) was dissolved in dry dichloromethane (30 mL), and HOBt (205.00 mg, 1.517 mmol), DIPEA (686.29 mg, 5.310 mmol), and EDCI (872.47 mg, 4.551 mmol) were added sequentially. The mixture was reacted overnight at room temperature, and water (10 mL) was added. The resulting mixture was washed sequentially with 0.5 M HCl (20 mL × 2) and saturated brine (20 mL × 1), dried over Na₂SO₄, filtered, and concentrated. The crude product was purified by silica gel column chromatography (70% ethyl acetate/petroleum ether) to give compound 85-12 (69 mg, 0.082 mmol, yield: 54.22%). ES-API: [M+H]⁺ = 839.2.

Step 13: Compound 85-12 (69 mg, 0.082 mmol) was dissolved in dichloromethane (1 mL), and TFA (0.5 mL) was added. The mixture was reacted at room temperature for 1 h. The reaction mixture was concentrated to give compound 85-13 (70 mg, crude product), which was directly used in the next step. ES-API: [M+H]⁺ = 739.3.

Step 14: trans-3-Oxabicyclo[3.1.0]hexane-6-carboxylic acid (9.09 mg, 0.071 mmol), DIPEA (18.3 mg, 0.142 mmol), and HATU (27 mg, 0.071 mmol) were added sequentially to a solution of compound 85-13 (35 mg, crude product) in dichloromethane (5 mL), and the mixture was reacted at room temperature for 1 h. The reaction mixture was extracted with dichloromethane (20 mL × 3), washed with an aqueous sodium chloride solution (30 mL × 1), dried over anhydrous sodium sulfate, filtered, and concentrated. The residue was purified by silica gel column chromatography (5% MeOH/DCM) to give compound 85-14 (18 mg, 0.0212 mmol, two-step yield: 51.7%). ES-API: [M+H]⁺ = 849.3.

Step 15: Pd(PPh₃)₂Cl₂ (8.41 mg, 0.012 mmol), CuI (2.30 mg, 0.012 mmol), DIPEA (12.16 mg, 0.094 mmol), and lithium chloride (3.96 mg, 0.094 mmol) were added sequentially to a solution of compound 85-14 (18 mg, 0.0212 mmol) and 1-(prop-2-yn-1-yl)piperidine-4-carbonitrile (14.23 mg, 0.096 mmol) in acetonitrile (1 mL). The mixture was heated to 85 °C and reacted for 1 h under nitrogen atmosphere. The reaction mixture was extracted with ethyl acetate (5 mL × 2), washed sequentially with a saturated aqueous ammonium chloride solution (5 mL × 1) and an aqueous sodium chloride solution (5 mL × 1), dried over Na₂SO₄, filtered, and concentrated. The residue was purified by prep-HPLC (ammonium bicarbonate method 1) to give (1R,5S,6r)-N-((4²Z,8³S,2E,6S)-(*Rₐ*)-1⁵-(5-(3-(4-cyanopiperidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-oxazola-8(1,3)-pyridazina-1(3,4)-pyrrolacyclotridecaphan-2-en-6-yl)-3-oxabicyclo[3.1.0]hexane-6-carboxamide (Z85, 5.6 mg, yield: 31.2%). ES-API: [M+H]⁺ = 847.4. ¹H NMR (400 MHz, DMSO-*d₆*) δ 8.65 (d, J = 2.1 Hz, 1H), 8.45 (d, J = 8.7 Hz, 1H), 7.75 (s, 1H), 7.62 (d, J = 2.1 Hz, 1H), 7.31 (s, 1H), 7.08 (d, J = 15.9 Hz, 1H), 6.54 (d, J = 15.8 Hz, 1H), 5.54 - 5.43 (m, 1H), 4.91 (d, J = 12.0 Hz, 1H), 4.14 (dd, J = 10.8, 7.6 Hz, 2H), 3.96 (td, J = 12.0, 3.1 Hz, 1H), 3.72 (q, J = 9.8, 8.8 Hz, 4H), 3.55 (dt, J = 8.5, 2.9 Hz, 3H), 3.50 (s, 2H), 3.37 (d, J = 10.7 Hz, 1H), 3.13 (s, 3H), 2.99 - 2.96 (m, 1H), 2.85 - 2.80 (m, 2H), 2.64 - 2.60 (m, 3H), 2.51 (d, J = 14.5 Hz, 1H), 2.38 (d, J = 10.7 Hz, 2H), 2.14 (d, J = 14.5 Hz, 1H), 1.93 - 1.77 (m, 6H), 1.71 - 1.65 (m, 3H), 1.53 (t, J = 3.2 Hz, 2H), 1.27 (d, J = 6.1 Hz, 3H), 0.97 (t, J = 7.2 Hz, 3H), 0.66 (s, 3H), 0.20 (s, 3H).

### Example 60. Synthesis of Compound Z234

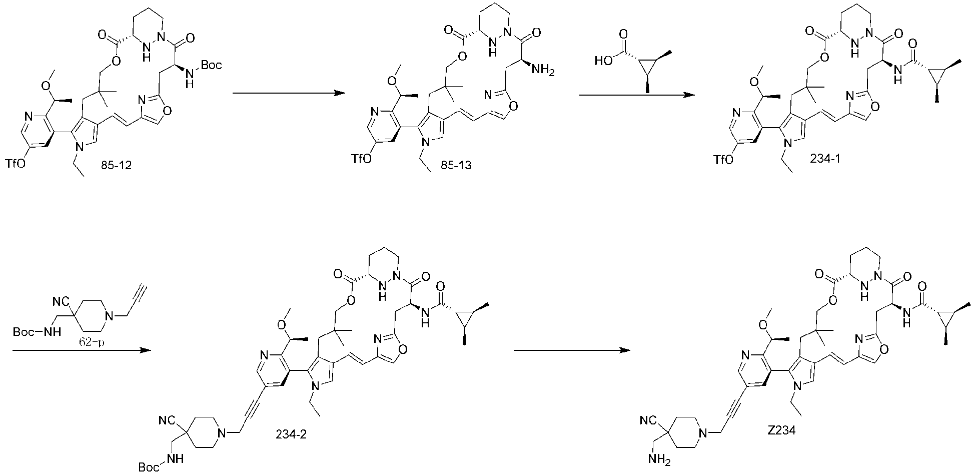

Step 1: Compound 85-12 (69 mg, 0.082 mmol) was dissolved in dichloromethane (1 mL), and trifluoroacetic acid (0.5 mL) was added. The mixture was reacted at room temperature for 1 h. The reaction mixture was concentrated to give compound 85-13 (70 mg, crude product), which was directly used in the next step. ES-API: [M+H]⁺ = 739.3.

Step 2: (1r,2R,3S)-2,3-dimethylcyclopropane-1-carboxylic acid (8.09 mg, 0.071 mmol), DIPEA (18.3 mg, 0.142 mmol), and HATU (27 mg, 0.071 mmol) were added sequentially to a solution of compound 85-13 (35 mg, crude product) in dichloromethane (5 mL), and the mixture was reacted at room temperature for 1 h. The reaction mixture was extracted with dichloromethane (20 mL × 3), washed with an aqueous sodium chloride solution (30 mL × 1), dried over anhydrous sodium sulfate, filtered, and concentrated. The residue was purified by silica gel column chromatography (ethyl acetate/petroleum ether = 1/1) to give compound 234-1 (15 mg, 0.018 mmol, two-step yield: 44%). ES-API: [M+H]⁺ = 835.3.

Step 3: Pd(PPh₃)₂Cl₂ (4.2 mg, 0.006 mmol), copper(I) iodide (2.30 mg, 0.012 mmol), DIPEA (12.16 mg, 0.094 mmol), and lithium chloride (3.96 mg, 0.094 mmol) were added sequentially to a solution of compound 234-1 (15 mg, 0.018 mmol) and compound intermediate 62-p (23.3 mg, 0.084 mmol) in acetonitrile (1.5 mL). The mixture was heated to 85 ° C and reacted for 1 h. The reaction mixture was extracted with ethyl acetate (5 mL × 2), washed sequentially with a saturated aqueous ammonium chloride solution (5 mL) and a saturated aqueous NaCl solution (30 mL × 1), dried over Na₂SO₄, filtered, and concentrated. The crude product was purified by silica gel column chromatography (5% MeOH/DCM) to give compound 234-2 (15 mg, 0.0156 mmol, yield: 86.6%). ES-API: [M+H]⁺ = 962.4.

Step 4: Trifluoroacetic acid (0.5 mL) was added to a solution of compound 234-2 (15 mg, 0.0156 mmol) in dichloromethane (1 mL), and the mixture was reacted at room temperature for 1 h. After concentration, the residue was purified by prep-HPLC (ammonium bicarbonate method 1) to give (1r,2R,3S)-N-((4²Z,8³ S,2E,6S)-(Rₐ)-1⁵-(5-(3-(4-(aminomethyl)-4-cyanopiperidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹H-10-oxa-4(4,2)-oxazola-8(1,3)-pyridazina-1(3,4)-pyrrolacyclotridecaphan-2-en-6-yl)-2,3-dimethylcyclopropane-1-carboxamide (Z234, 5.4 mg, yield: 40.1%). ES-API: [M+H]⁺ = 862.5. ¹H NMR (400 MHz, DMSO-*d*₆) δ 8.64 (d, *J* = 2.1 Hz, 1H), 8.27 (d, *J =* 8.6 Hz, 1H), 7.75 (s, 1H), 7.61 (d, *J =* 2.2 Hz, 1H), 7.30 (s, 1H), 7.08 (d, *J* = 15.8 Hz, 1H), 6.53 (d, *J =* 15.8 Hz, 1H), 5.51 - 5.46 (m, 1H), 4.89 (d, *J =* 12.0 Hz, 1H), 4.17- 4.12 (m, 2H), 3.94 (td, *J =* 12.0, 3.2 Hz, 1H), 3.77 - 3.66 (m, 2H), 3.57-3.54 (m, 3H), 3.37 (d, *J =* 10.9 Hz, 2H), 3.13 (s, 3H), 2.98-2.93 (m, 1H), 2.88 - 2.76 (m, 2H), 2.64 - 2.60 (m, 3H), 2.38 - 2.35 (m, 2H), 2.15 (d, *J =* 14.5 Hz, 1H), 1.97 - 1.75 (m, 4H), 1.66 (d, *J* = 12.5 Hz, 1H), 1.54 - 1.32 (m, 4H), 1.27 (d, *J* = 6.1 Hz, 3H), 1.21 - 1.10 (m, 3H), 1.08 - 1.03 (m, 2H), 0.99 - 0.96 (m, 9H), 0.65 (s, 3H), 0.20 (s, 3H).

### Example 61. Synthesis of Compound Z89

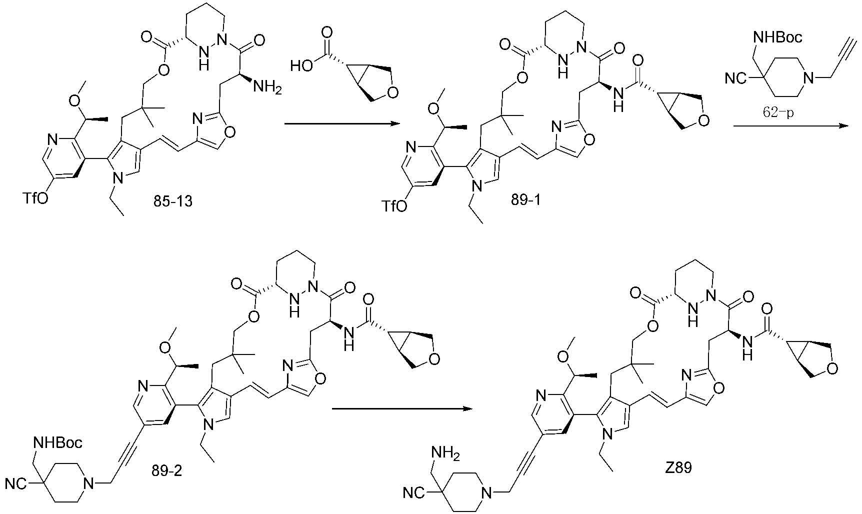

Step 1: compound 85-13 (35 mg, 0.047 mmol) was dissolved in dichloromethane (4 mL), and trans-3-oxabicyclo[3.1.0]hexane-6-carboxylic acid (9 mg, 0.071 mmol), O-(7-azabenzotriazol-1-yl)-N,N,N',N'-tetramethyluronium hexafluorophosphate (23 mg, 0.062 mmol) and N,N'-diisopropylethylamine (37 mg, 0.284 mmol) were added. The reaction system was stirred at room temperature for 1 h. Dichloromethane (15 mL) was added to the reaction mixture. The resulting mixture was washed sequentially with water (5 mL) and saturated brine (5 mL), dried over anhydrous sodium sulfate, and filtered. The filtrate was concentrated under reduced pressure. The crude product was purified by flash silica gel column chromatography (tetrahydrofuran/petroleum ether: 0-75%) to give compound 89-1 (35 mg, yield: 87.0%) as a light brown solid. ES-API: [M+H]⁺ = 849.3.

Step 2: Compound 89-1 (20 mg, 0.024 mmol) and compound intermediate 62-p (20 mg, 0.071 mmol) were dissolved in acetonitrile (2 mL), and bis(triphenylphosphine)palladium(II) dichloride (3 mg, 0.004 mmol), copper(I) iodide (1 mg, 0.005 mmol), N,N'-diisopropylethylamine (12 mg, 0.094 mmol), and lithium chloride (4 mg, 0.094 mmol) were added. The reaction system was stirred at 85 °C for 2 h under nitrogen atmosphere. Water (5 mL) was added to the reaction mixture, and the resulting mixture was extracted with ethyl acetate (20 mL). The organic phase was washed with saturated brine (5 mL), dried, and concentrated to give a crude product. The crude product was purified by flash silica gel column chromatography (methanol/dichloromethane: 0-8%) to give compound 89-2 (23 mg, yield: 100%) as a light brown solid. ES-API: [M+H]⁺ = 976.3.

Step 3: Compound 89-2 (23 mg, 0.024 mmol) was dissolved in dichloromethane (1 mL), and trifluoroacetic acid (0.3 mL) was added at 0 °C. The reaction system was stirred at room temperature for 1 h. The reaction mixture was concentrated, and a saturated sodium bicarbonate solution (2 mL) was added. The mixture was extracted with dichloromethane (10 mL × 2). The organic phases were combined, dried over anhydrous sodium sulfate, and filtered, and the filtrate was concentrated under reduced pressure. The crude product was purified by prep-HPLC (ammonium bicarbonate method 1) to give the desired product (1R,5S,6r)-N-((4²Z,8³S,2E, 6S)-(Rₐ)-1⁵-(5-(3-(4-(aminomethyl)-4-cyanopiperidin-1-yl)prop-1-yn-1-yl)-2-((S)-1-methoxyethyl)pyridin-3-yl)-1¹-ethyl-12,12-dimethyl-7,9-dioxo-8¹,8²,8³,8⁴,8⁵,8⁶-hexahydro-1¹ H-10-oxa-4(4,2)-oxazola-8(1,3)-pyridazina-1(3,4)-pyrrolacyclotridecaphan-2-en-6-yl)-3-oxabicyclo[3.1.0]hexane-6-carboxamide (Z89, 6 mg, yield: 29.1%) as a white solid. ¹H NMR (400 MHz, DMSO-d₆) δ 8.71 (d, *J =* 2.0 Hz, 1H), 8.52 (d, *J =* 8.8 Hz, 1H), 7.82 (s, 1H), 7.67 (d, *J =* 2.0 Hz, 1H), 7.38 (s, 1H), 7.15 (d, *J =* 16.0 Hz, 1H), 6.60 (d, *J =* 16.0 Hz, 1H), 5.61 - 5.45 (m, 1H), 4.98 (d, *J =* 12.0 Hz, 1H), 4.31 - 4.13 (m, 2H), 4.08 - 3.96 (m, 1H), 3.89 - 3.69 (m, 4H), 3.67 - 3.53 (m, 5H), 3.44 (d, *J =* 10.8 Hz, 1H), 3.20 (s, 3H), 3.05 (dd, *J* = 15.2, 4.8 Hz, 1H), 2.96 - 2.82 (m, 3H), 2.75 - 2.62 (m, 3H), 2.57 (d, *J =* 15.2 Hz, 1H), 2.47 - 2.40 (m, 2H), 2.21 (d, *J* = 14.4 Hz, 1H), 1.99 - 1.68 (m, 8H), 1.64 - 1.38 (m, 5H), 1.34 (d, *J* = 6.0 Hz, 3H), 1.04 (t, *J* = 7.2 Hz, 3H), 0.72 (s, 3H), 0.27 (s, 3H).ES-API: [M+H]⁺= 876.3.

Based on the methods described in the above examples and by modifying some of the starting materials and/or referring to known synthetic methods, the compounds listed in Table A below were synthesized:

**Table A**

| Structure and Number | MS [M+H]⁺ | Structure and Number | MS [M+H]⁺ |
|---|---|---|---|
| 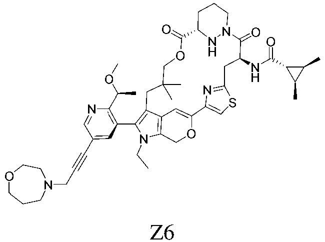 | 868.4 | 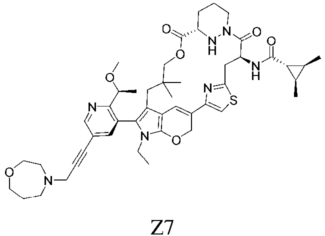 | 868.4 |
| 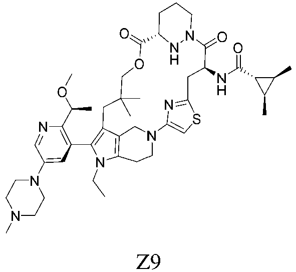 | 830.5 | 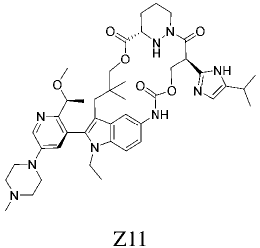 | 798.5 |
| 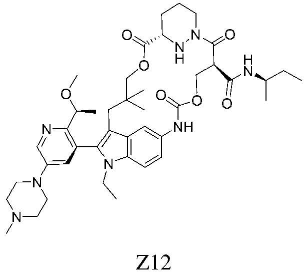 | 789.5 | 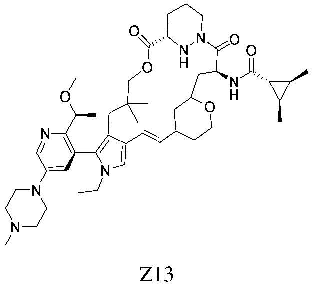 | 802.5 |
| 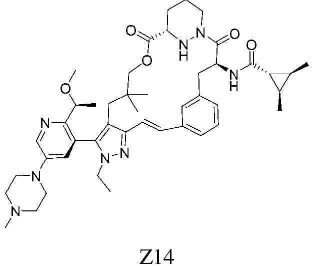 | 795.5 | 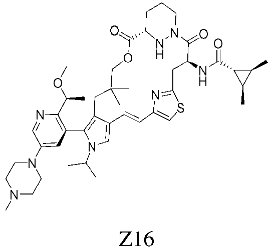 | 815.5 |
| 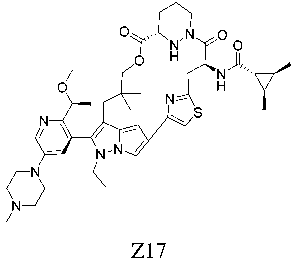 | 814.4 | 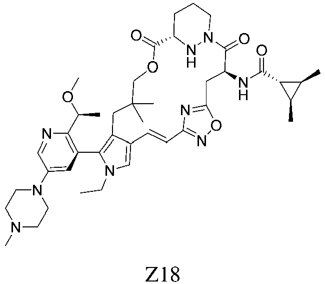 | 786.5 |
| 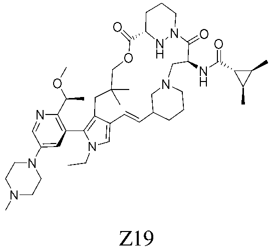 | 801.5 | 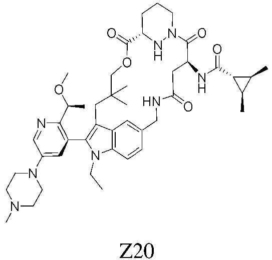 | 799.5 |
| 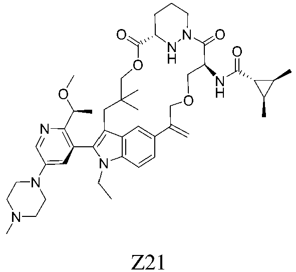 | 798.5 | 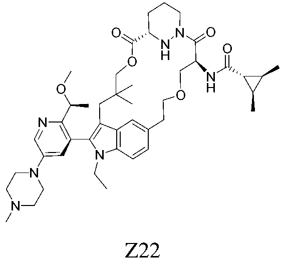 | 786.5 |
| 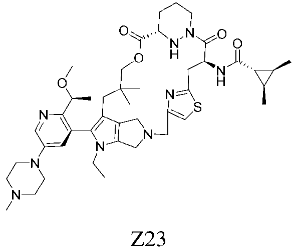 | 830.5 | 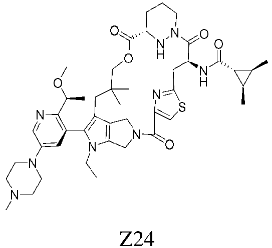 | 844.4 |
| 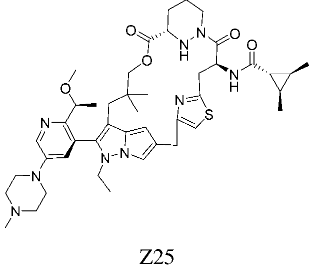 | 828.5 | 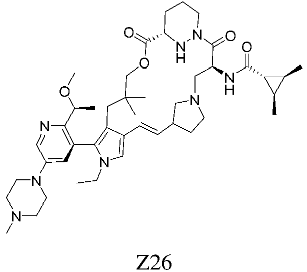 | 787.5 |
| 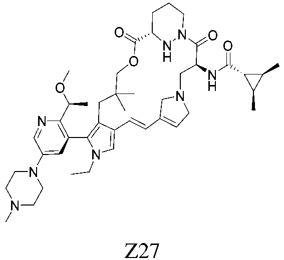 | 785.5 | 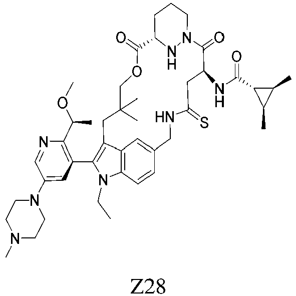 | 815.5 |
| 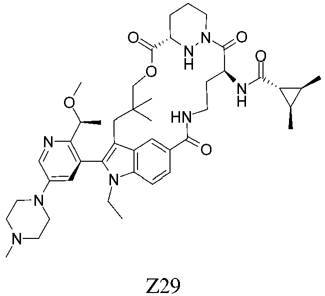 | 799.5 | 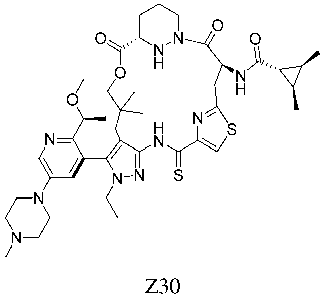 | 835.4 |
| 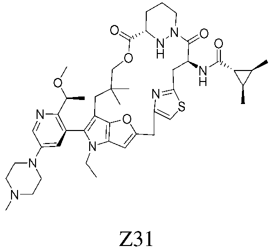 | 829.4 | 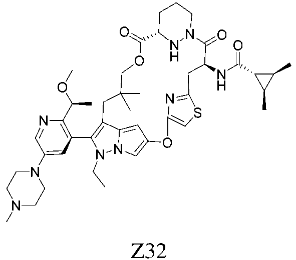 | 829.4 |
| 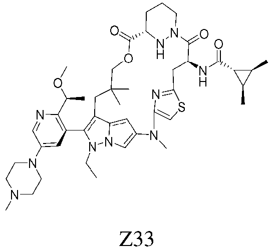 | 843.5 | 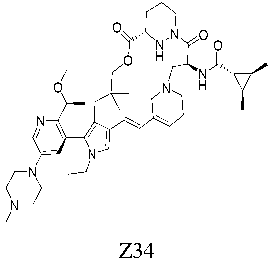 | 799.5 |
| 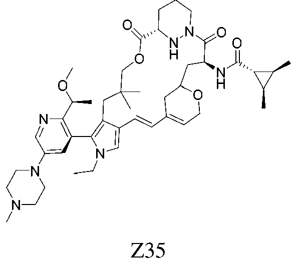 | 800.5 | 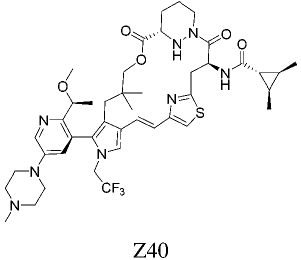 | 855.4 |
| 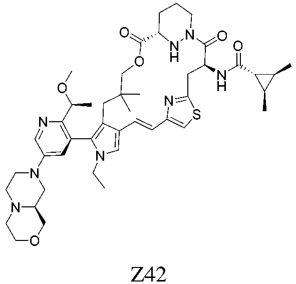 | 843.5 | 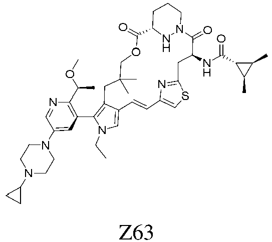 | 827.5 |
| 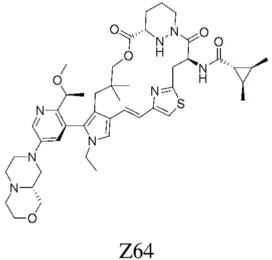 | 843.5 | 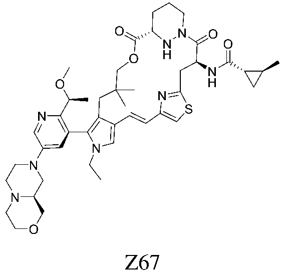 | 829.4 |
| 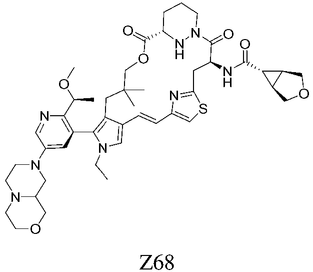 | 857.4 | 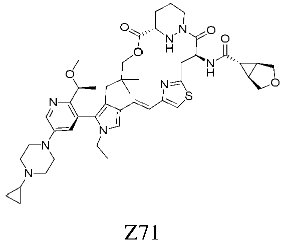 | 841.4 |
| 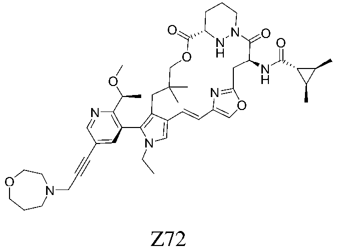 | 824.5 | 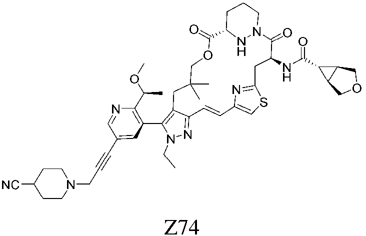 | 864.4 |
| 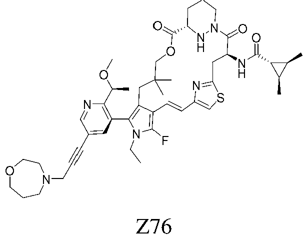 | 858.4 | 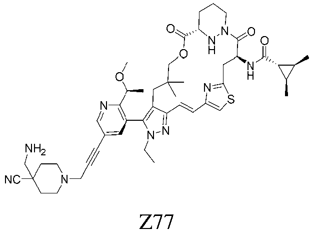 | 879.5 |
| 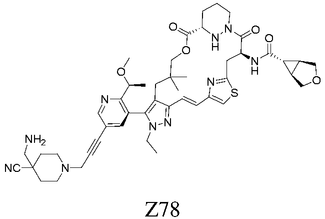 | 893.4 | 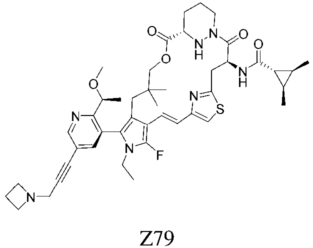 | 814.4 |
| 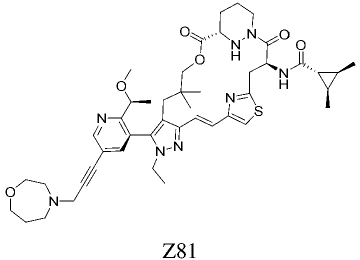 | 841.4 | 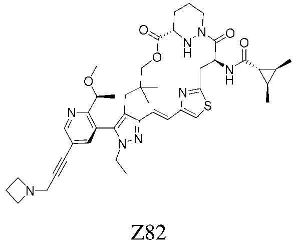 | 797.4 |
| 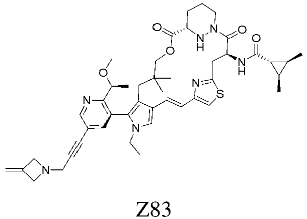 | 808.4 | 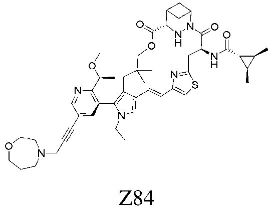 | 852.4 |
| 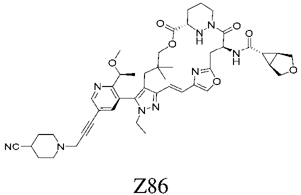 | 848.4 | 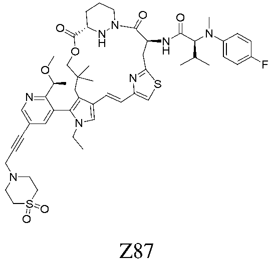 | 985.4 |
| 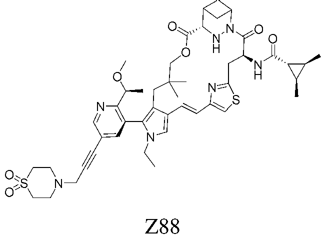 | 886.4 | 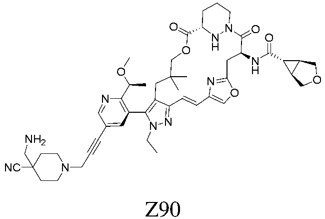 | 877.5 |
| 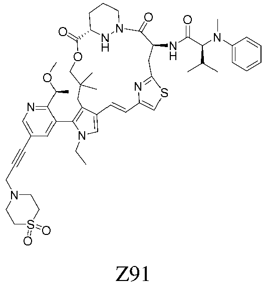 | 967.4 | 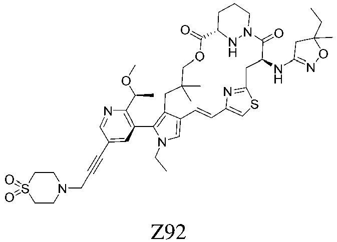 | 889.4 |
| 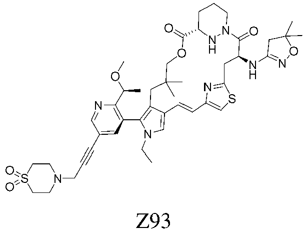 | 875.5 | 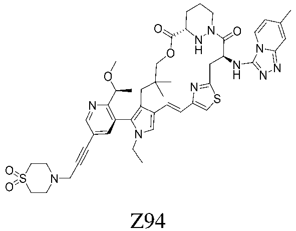 | 909.4 |
| 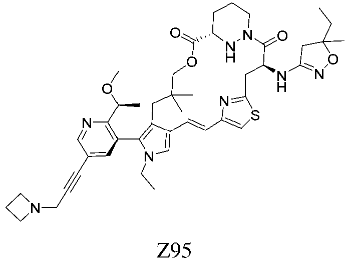 | 811.4 | 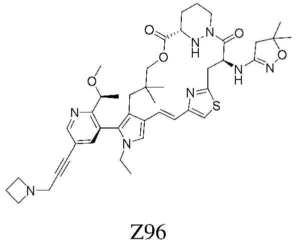 | 797.4 |
| 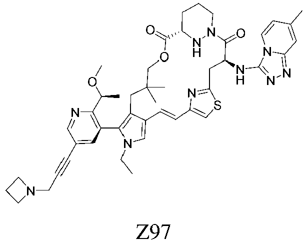 | 831.4 | 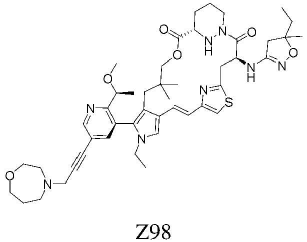 | 855.5 |
| 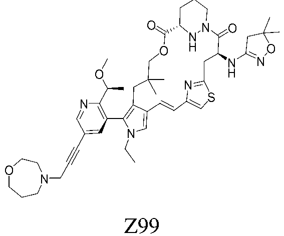 | 841.4 | 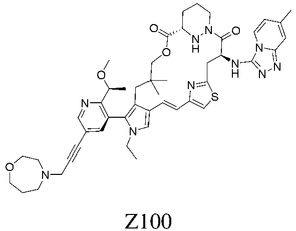 | 875.4 |
| 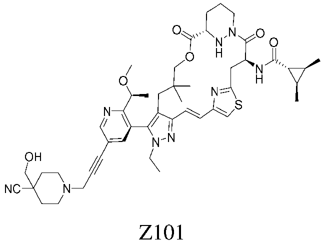 | 880.4 | 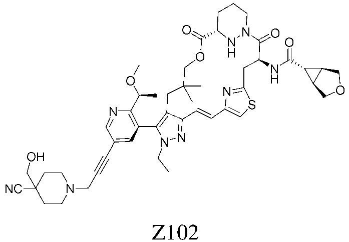 | 894.4 |
| 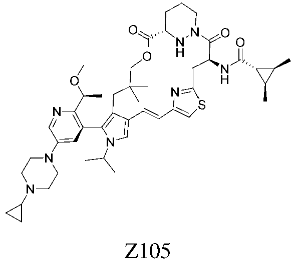 | 841.5 | 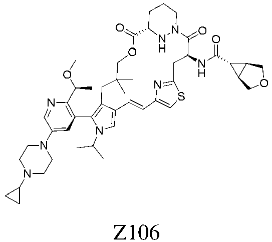 | 855.5 |
| 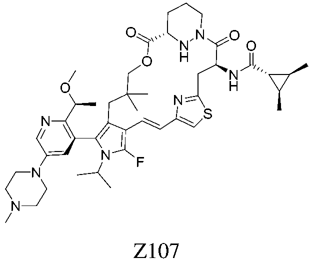 | 833.4 | 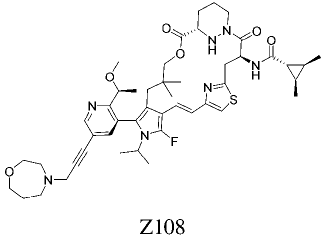 | 872.4 |
| 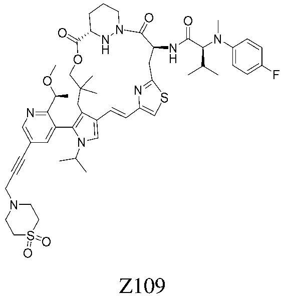 | 999.5 | 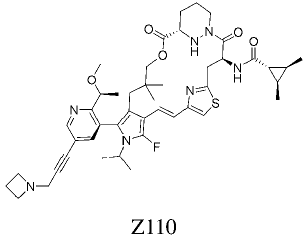 | 828.4 |
| 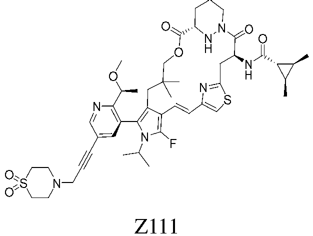 | 906.4 | 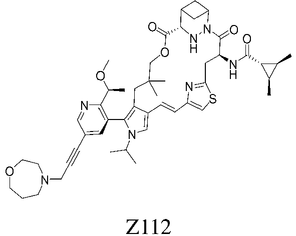 | 866.5 |
| 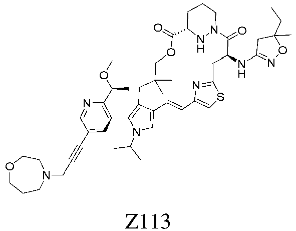 | 869.5 | 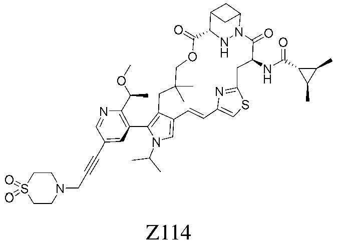 | 900.4 |
| 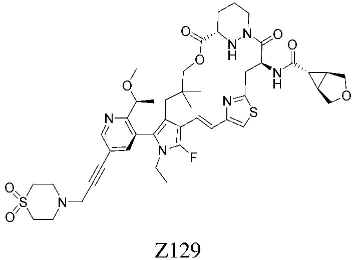 | 906.4 | 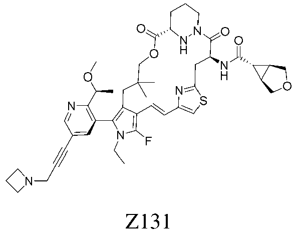 | 828.4 |
| 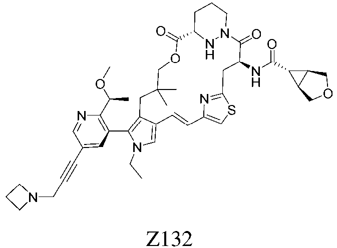 | 810.4 | 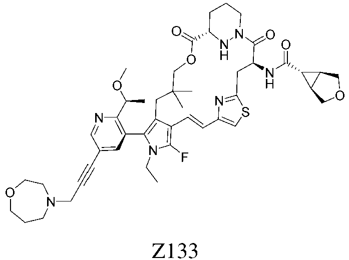 | 872.4 |
| 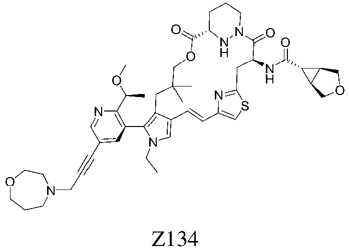 | 854.4 | 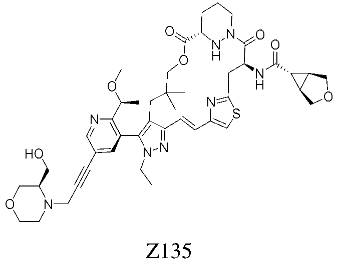 | 871.4 |
| 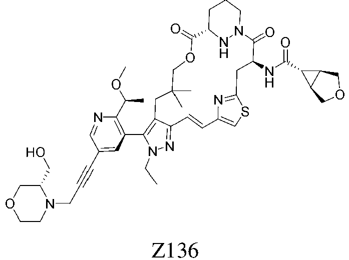 | 871.4 | 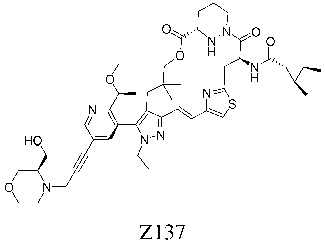 | 857.4 |
| 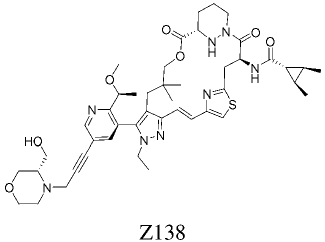 | 857.4 | 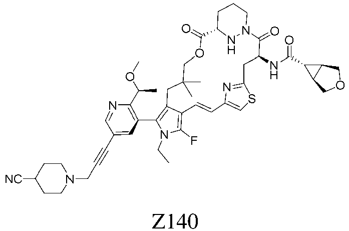 | 881.4 |
| 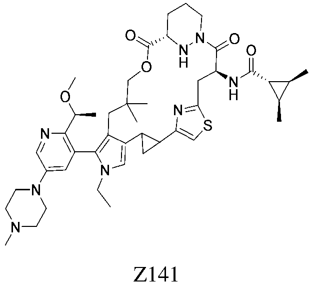 | 815.5 | 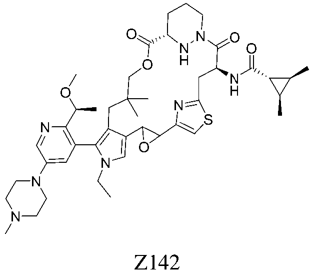 | 817.4 |
| 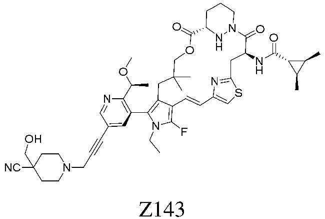 | 897.4 | 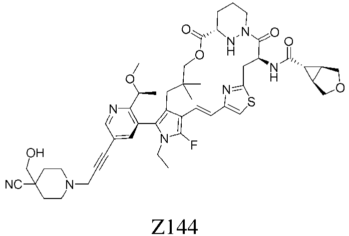 | 911.4 |
| 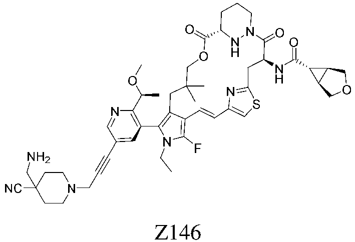 | 910.4 | 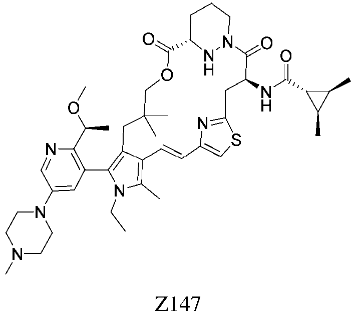 | 815.5 |
| 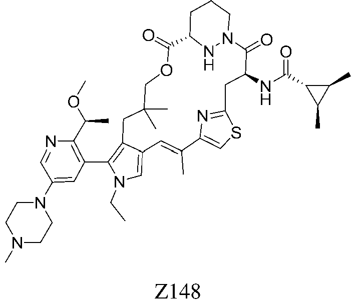 | 815.5 | 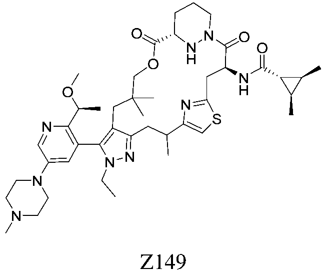 | 818.5 |
| 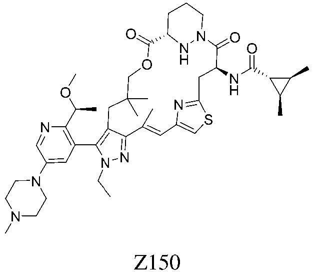 | 816.5 | 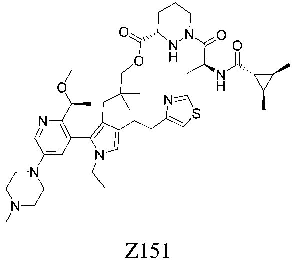 | 803.5 |
| 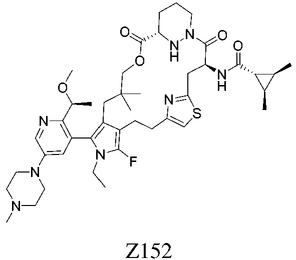 | 821.4 | 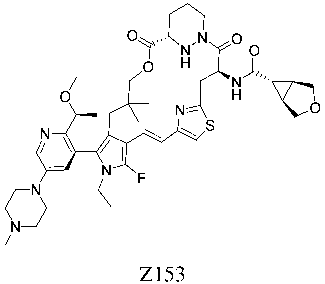 | 833.4 |
| 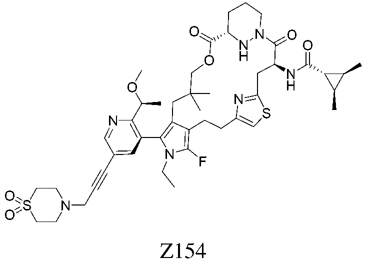 | 894.4 | 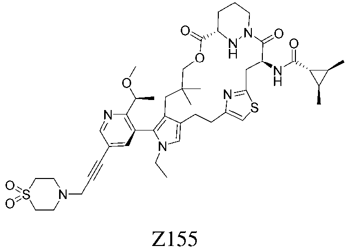 | 876.4 |
| 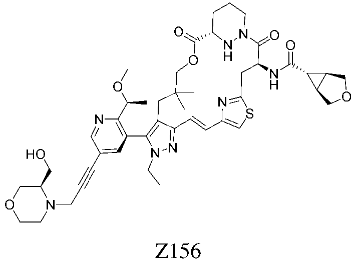 | 871.4 | 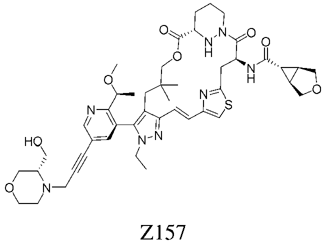 | 871.4 |
| 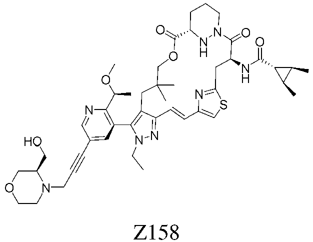 | 857.4 | 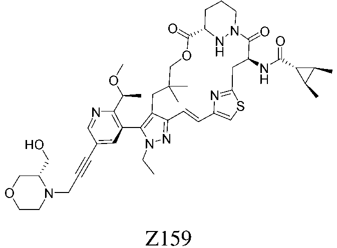 | 857.4 |
| 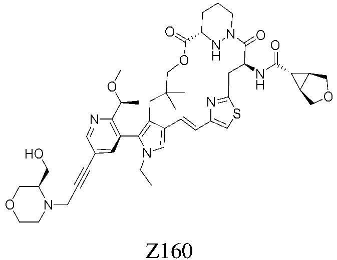 | 570.4 | 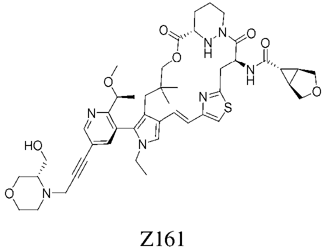 | 870.4 |
| 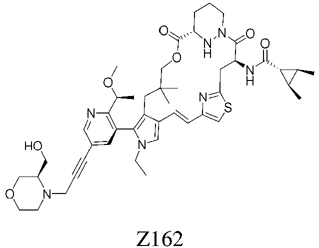 | 856.4 | 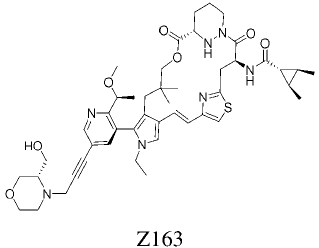 | 856.4 |
| 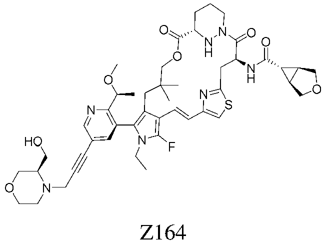 | 888.4 | 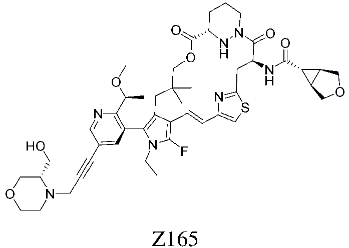 | 888.4 |
| 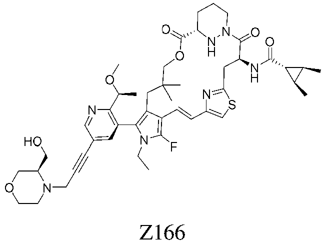 | 874.4 | 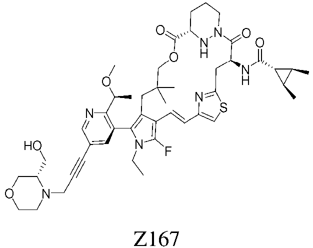 | 874.4 |
| 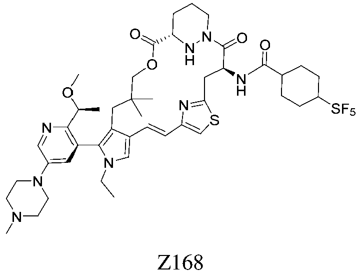 | 941.4 | 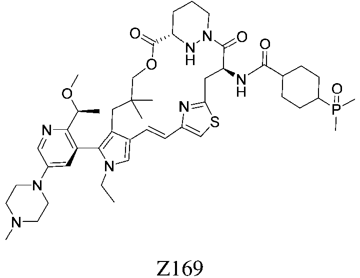 | 891.5 |
| 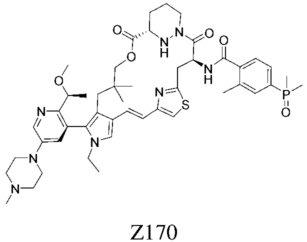 | 899.4 | 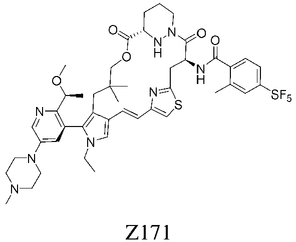 | 949.4 |
| 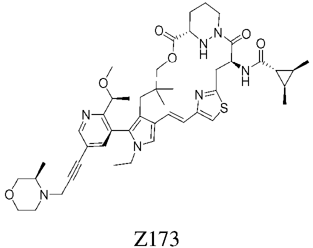 | 840.4 | 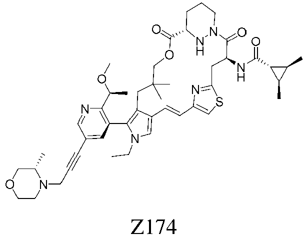 | 840.4 |
| 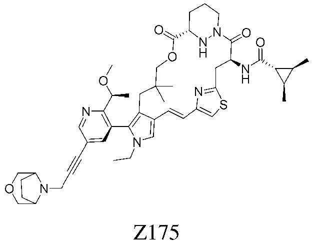 | 852.4 | 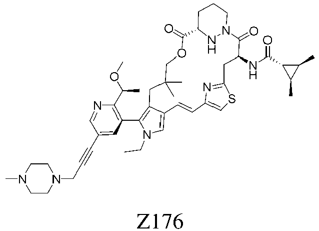 | 839.5 |
| 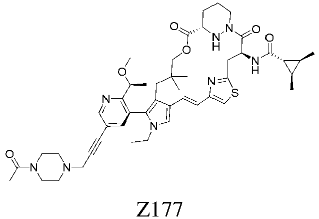 | 867.5 | 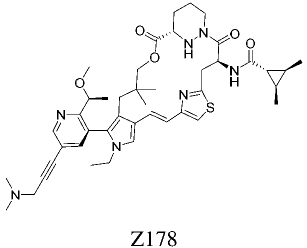 | 784.4 |
| 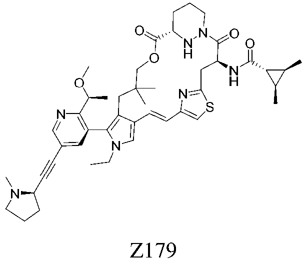 | 810.4 | 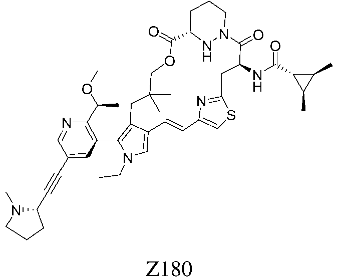 | 810.4 |
| 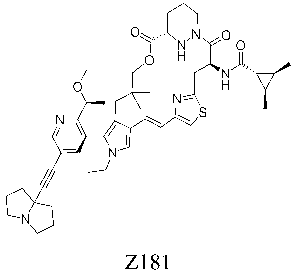 | 836.4 | 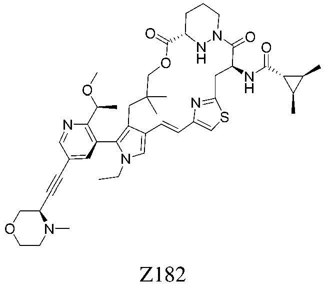 | 826.4 |
| 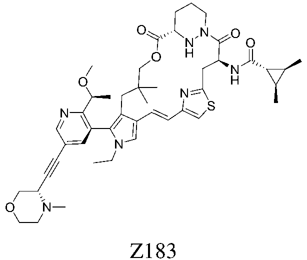 | 826.4 | 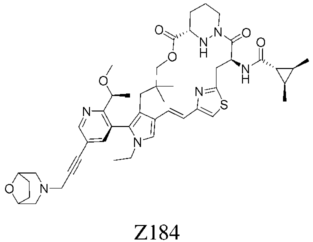 | 852.4 |
| 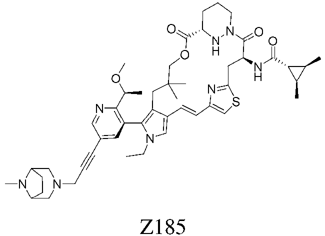 | 865.5 | 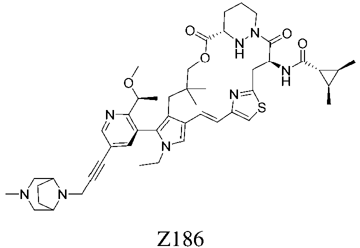 | 865.5 |
| 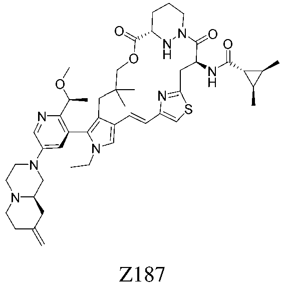 | 853.5 | 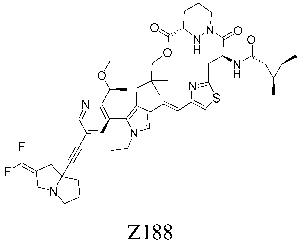 | 884.4 |
| 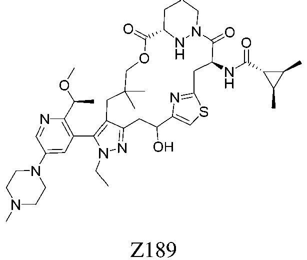 | 820.4 | 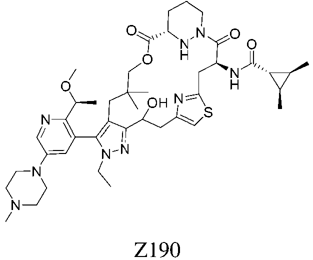 | 820.4 |
| 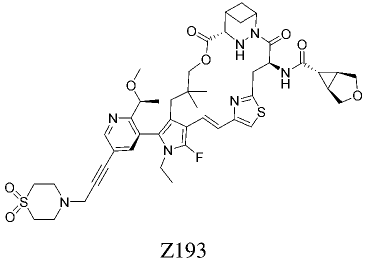 | 918.4 | 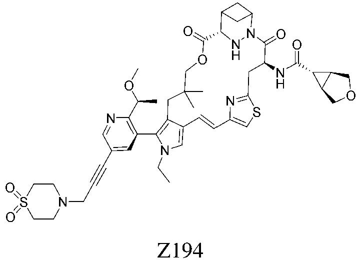 | 900.4 |
| 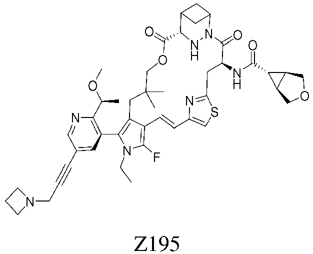 | 840.4 | 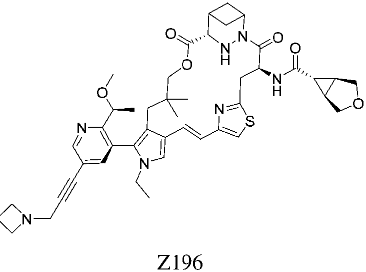 | 822.4 |
| 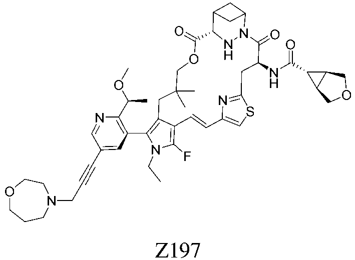 | 884.4 | 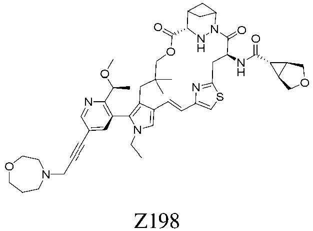 | 866.4 |
| 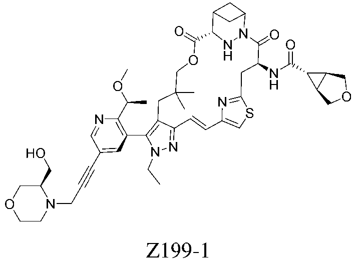 | 883.4 | 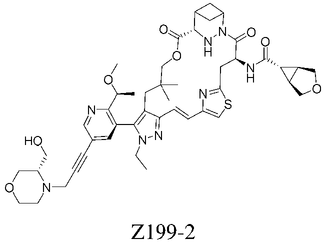 | 883.4 |
| 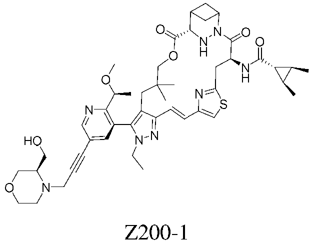 | 869.4 | 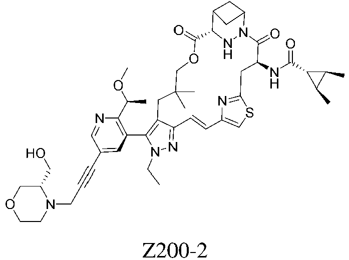 | 869.4 |
| 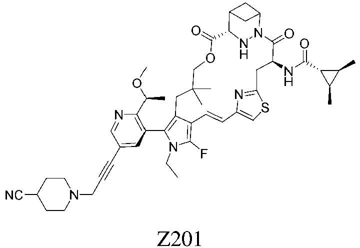 | 879.4 | 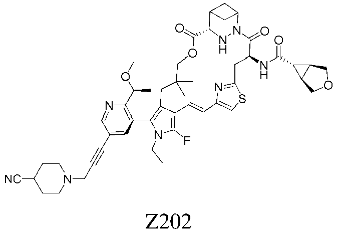 | 893.4 |
| 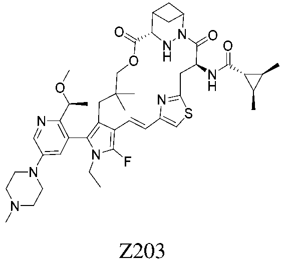 | 831.4 | 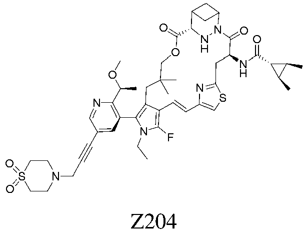 | 904.4 |
| 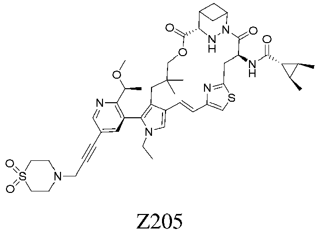 | 886.4 | 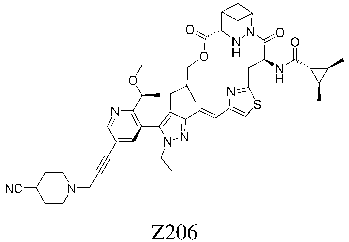 | 862.4 |
| 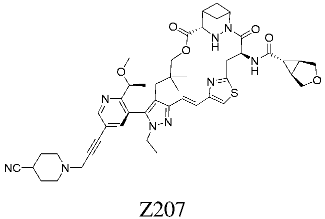 | 876.4 | 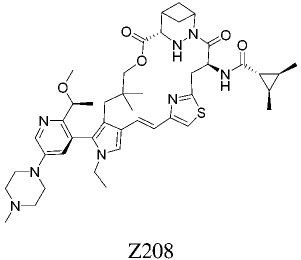 | 813.4 |
| 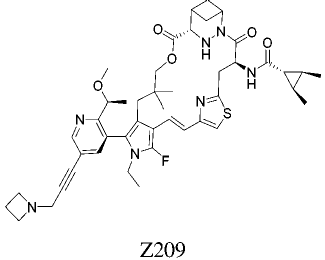 | 826.4 | 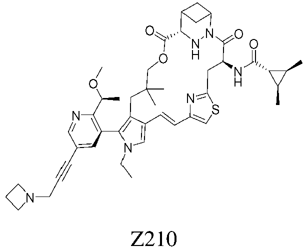 | 808.4 |
| 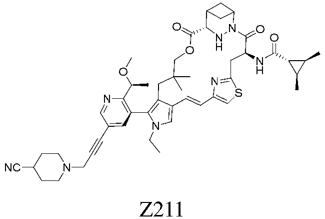 | 861.4 | 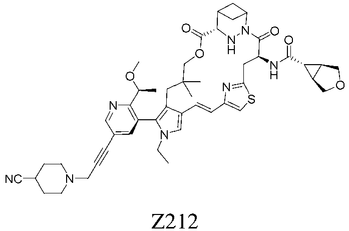 | 875.4 |
| 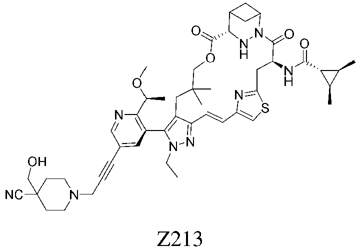 | 892.4 | 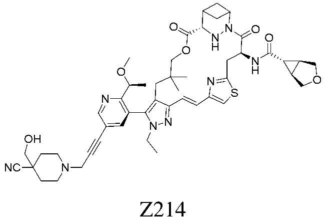 | 906.4 |
| 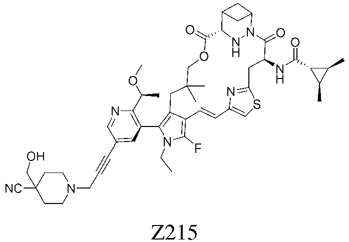 | 909.4 | 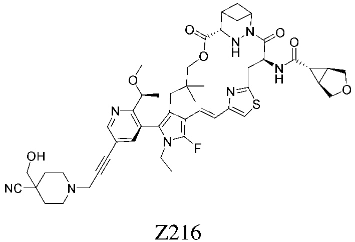 | 923.4 |
| 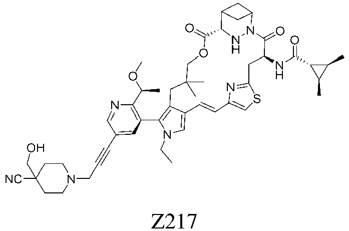 | 891.5 | 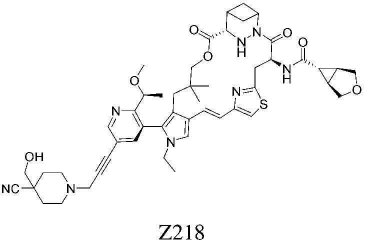 | 905.4 |
| 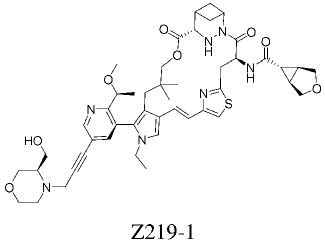 | 882.4 | 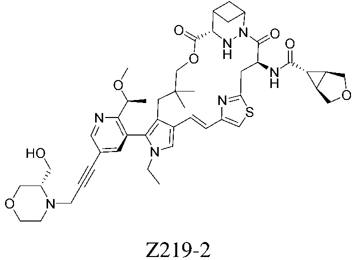 | 882.4 |
| 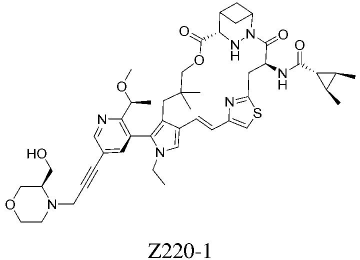 | 868.4 | 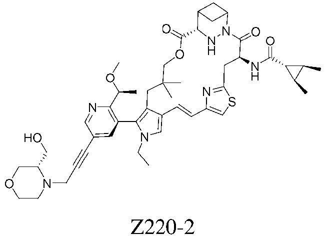 | 868.4 |
| 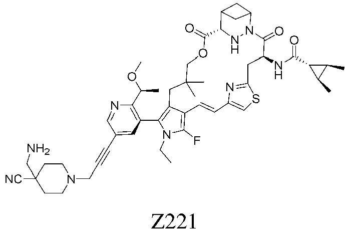 | 908.5 | 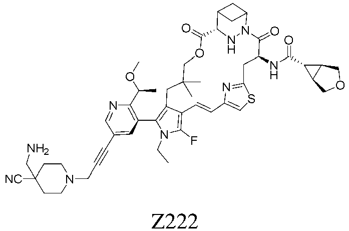 | 922.4 |
| 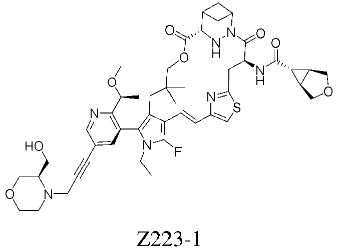 | 900.4 | 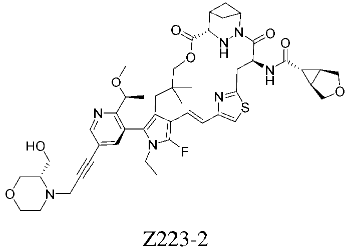 | 900.4 |
| 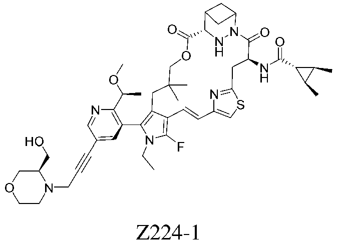 | 886.4 | 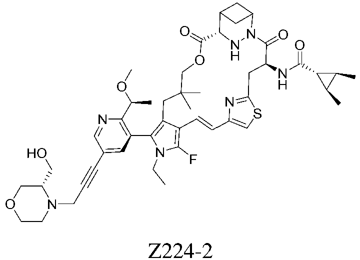 | 886.4 |
| 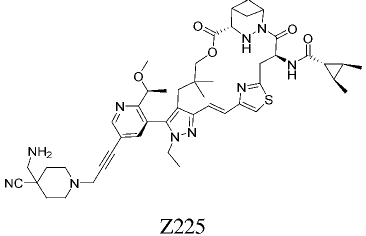 | 891.5 | 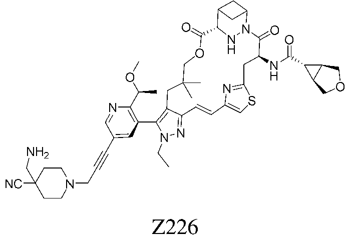 | 905.4 |
| 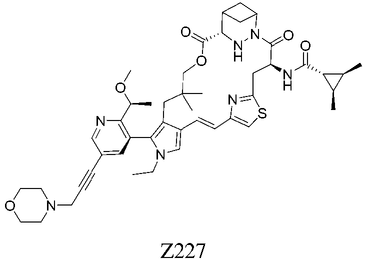 | 838.4 | 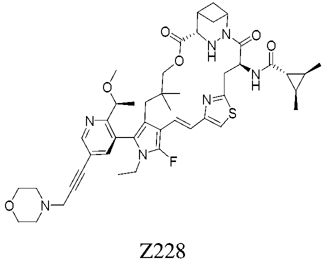 | 856.4 |
| 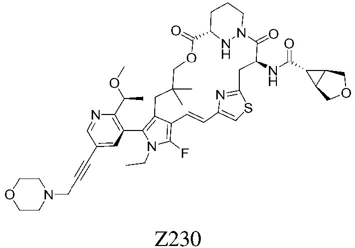 | 858.4 | 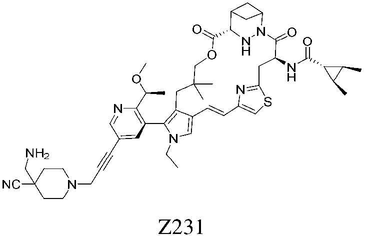 | 890.5 |
| 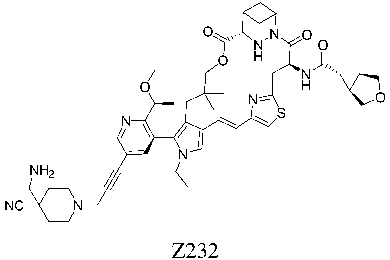 | 894.4 | 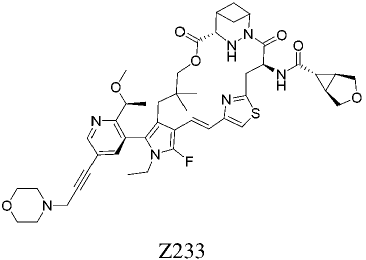 | 844.4 |
| 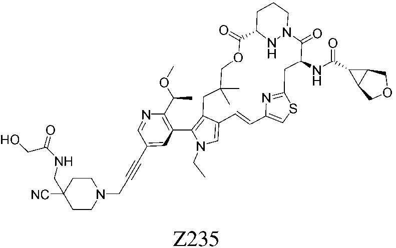 | 950.4 | 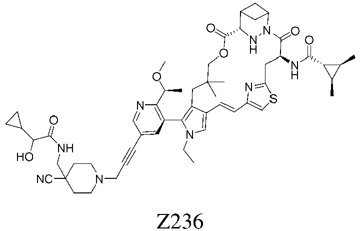 | 988.5 |
| 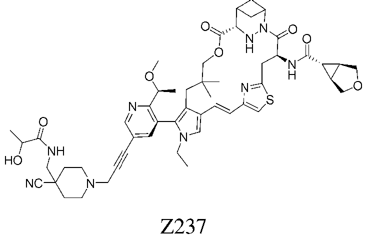 | 976.4 | 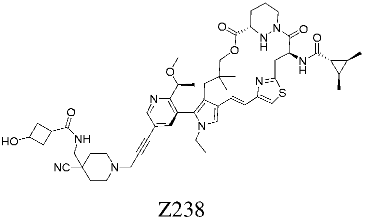 | 976.5 |
| 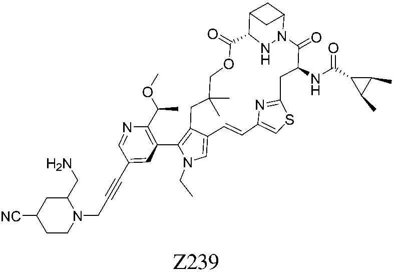 | 890.5 | 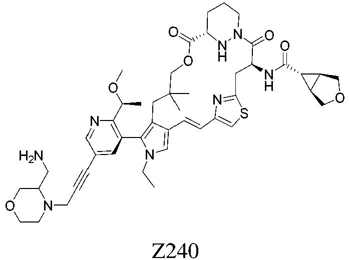 | 869.5 |
| 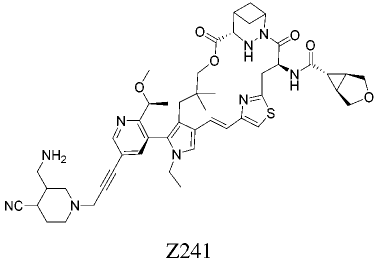 | 904.4 | 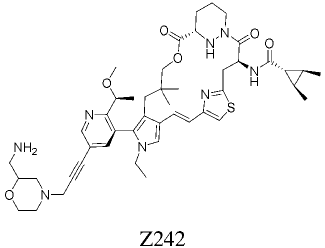 | 855.4 |

### Test Example 1: Cell p-ERK Assay

AsPC-1 cells were purchased from ATCC, Cat. No. CRL-1682; NCI-H441 cells were purchased from ATCC, Cat. No. HTB-174; RPMI-1640 medium was purchased from Gibco, Cat. No. 11875-093; FBS was purchased from Gibco, Cat. No. 10099-141C; penicillin/streptomycin double-antibiotic solution (PS) was purchased from Gibco, Cat. No. 15140-122; trypsin (containing EDTA) was purchased from Gibco, Cat. No. 25200-072; DMSO was purchased from VWRAMRESCO, Cat. No. 0231-500ML; 96-well cell culture plates were purchased from Corning, Cat. No. 3599; white-bottomed, opaque 96-well plates were purchased from Cisbio, Cat. No. 66PL96025; Advance ERK phospho-T202/Y204 kit was purchased from Cisbio, Cat. No. 64AERPEH; cell counter was purchased from CHEMOMETEC, model: NC-200; microplate reader was purchased from PerkinElmer, model: EnVision.

AsPC-1, a human pancreatic cancer cell strain endogenously containing a KRAS^{G12D} mutation, was cultured in a complete medium containing RPMI-1640 + 10% FBS + 1% PS. NCI-H441, a human non-small cell lung cancer cell strain endogenously containing a KRAS^{G12V} mutation, was cultured in a complete medium containing RPMI-1640 + 10% FBS + 1% PS.

The cells were digested with trypsin (containing EDTA), collected in their corresponding complete media, and counted. The density of the cell suspensions was adjusted with their corresponding complete media, and AsPC-1 cells (10000 cells/well) or NCI-H441 cells (20000 cells/well) were seeded into a 96-well cell culture plate, with the volume of the medium in each well being 80 µL. The cell culture plate was incubated in an incubator at 37 °C with 5% CO₂ overnight. A series of 3.16-fold gradient dilutions of a 1000× compound stock solution were prepared with DMSO, and then diluted 200-fold with the complete medium described above to give 5× compound stock solutions. On the day after cell seeding, 20 µL of the 5× compound stock solution was added to each cell culture well. As a result, the final concentration of the compound in the cell culture well became 1×, and the DMSO content in the well was 0.1%. In addition to the compound-treated wells described above, cell-containing DMSO control wells were also set. A complete medium containing 0.5% DMSO was added at 20 µL/well. After the mixture was well mixed, the culture plate was incubated in the incubator at 37 °C with 5% CO₂ for another 3 h. The medium was then removed from the culture wells, and 50 µL of cell lysis buffer (from Advance ERK phospho-T202/Y204 kit) was added to each well. The mixture was well mixed, incubated for 30 min, and then transferred to a white-bottomed, opaque 96-well assay plate at 16 µL/well, and separately, 16 µL of cell lysis buffer was added to additional wells (serving as blank wells) in the assay plate. After the transfer was completed, 4 µL of p-ERK HTRF antibody mixture (from Advance ERK phospho-T202/Y204 kit) was added to each well of the assay plate, followed by a 4-hour incubation. The HTRF signal values (RLU) were then measured using a microplate reader, and the inhibition rate of the compound against p-ERK levels was calculated as follows: IR(%) = (RLU_{DMSO} - RLU_{compound})/(RLU_{DMSO} - RLU_{blank}) × 100%, where RLU_{DMSO} is the average HTRF signal value from the DMSO control well samples, RLU is the average HTRF signal value from the blank well samples, and RLU_{compound} is the HTRF signal value from the compound-treated well samples. Using an XLfit software, the IR(%) values were plotted against the logarithm of the compound concentrations, and the dose-response curves were fitted using the four-parameter method to calculate the IC₅₀ values.

The compounds of the present disclosure exhibited relatively high inhibitory activity against the p-ERK in the cancer cells described above. The IC₅₀ values of some of the compounds were less than 1 µM, and the IC₅₀ values of some of the compounds were less than 500 nM or 100 nM. The results for some of the exemplary compounds are shown in Tables 4 and 5 below.

**Table 4**

| Number | H441 p-ERK IC₅₀(nM) | Number | H441 p-ERK IC₅₀(nM) |
|---|---|---|---|
| Z2-1 | 239.34 | Z60 | 260.54 |
| Z4 | 157.12 | Z61 | 69.88 |
| Z4-1 | 71.77 | Z62 | 3.5 |
| Z36 | 40.39 | Z115 | 3.97 |
| Z15A | 7.99 | Z116 | 115.46 |
| Z8A | 8.08 | Z118 | 0.47 |
| Z15 | 6.74 | Z119 | 1.81 |
| Z8-1 | 98.6 | Z120 | 513.03 |
| Z8 | 4.74 | Z122 | 181.18 |
| Z41 | 356.06 | Z123 | 4.63 |
| Z44-2 | 15.38 | Z124 | 2.05 |
| Z45 | 11.06 | Z125 | 0.53 |
| Z46 | 167.81 | Z126 | 403.29 |
| Z47-2 | 17.27 | Z127 | 104.23 |
| Z48 | 78.62 | Z73 | 0.88 |
| Z51 | 3.02 | Z191 | 3.92 |
| Z52-1 | 52.31 | Z80 | 0.2 |
| Z52-2 | 32.03 | Z130 | 0.54 |
| Z53 | 182.44 | Z10 | 0.83 |
| Z54-1 | 150.06 | Z49 | 0.17 |
| Z56 | 125.76 | Z103 | 0.32 |
| Z57 | 87.96 | Z69 | 0.9 |
| Z58 | 55.72 | Z70 | 2.03 |
| Z59 | 49.19 | Z172 | 0.75 |
| Z39 | 177.27 | Z75 | 1.3 |
| Z229 | 0.6 | Z139 | 0.42 |
| Z145 | 0.29 | Z65 | 0.58 |
| Z66 | 0.38 | Z50 | 0.29 |
| Z104 | 0.47 | Z89 | 0.23 |
| Z85 | 0.20 | Z234 | 0.36 |

**Table 5**

| Number | AsPC-1 p-ERK IC₅₀(nM) | Number | AsPC-1 p-ERK IC₅₀(nM) |
|---|---|---|---|
| Z4 | 366.77 | Z125 | 0.51 |
| Z4-1 | 190.88 | Z73 | 1.73 |
| Z36 | 230.37 | Z80 | 0.24 |
| Z15A | 30.02 | Z130 | 0.94 |
| Z8A | 8.19 | Z10 | 1.2 |
| Z15 | 20.29 | Z49 | 0.13 |
| Z8-1 | 102.73 | Z103 | 0.08 |
| Z8 | 4.98 | Z69 | 1.54 |
| Z39 | 506.34 | Z70 | 3.6 |
| Z229 | 1.04 | Z172 | 0.74 |
| Z145 | 0.15 | Z75 | 1.46 |
| Z66 | 0.16 | Z139 | 0.4 |
| Z104 | 0.17 | Z65 | 0.14 |
| Z62 | 4.9 | Z50 | 0.11 |
| Z118 | 0.2 | Z89 | 0.13 |
| Z119 | 2.29 | Z234 | 0.07 |
| Z85 | 0.04 | | |

### Test Example 2: 3D Cell Proliferation Assay

MIA PaCa-2 cells were purchased from ATCC, Cat. No. CRL-1420; AsPC-1 cells were purchased from ATCC, Cat. No. CRL-1682; NCI-H441 cells were purchased from ATCC, Cat. No. HTB-174; NCI-H1975 cells were purchased from ATCC, Cat. No. CRL-5908; DMEM medium was purchased from Gibco, Cat. No. 11995-065; RPMI-1640 medium was purchased from Gibco, Cat. No. 11875-093; FBS was purchased from Gibco, Cat. No. 10099-141C; penicillin/streptomycin double-antibiotic solution (PS) was purchased from Gibco, Cat. No. 15140-122; Horse serum was purchased from Gibco, Cat. No. 16050130; trypsin (containing EDTA) was purchased from Gibco, Cat. No. 25200-072; DMSO was purchased from VWRAMRESCO, Cat. No. 0231-500ML; 384-well cell culture plates were purchased from Corning, Cat. No. 3830; white-bottomed, opaque 384-well assay plates were purchased from Corning, Cat. No. 3570; CellTiter Glo-3D kit was purchased from Promega, Cat. No. G9683; cell counter was purchased from CHEMOMETEC, model: NC-200; microplate reader was purchased from PerkinElmer, model: EnVision.

MIA PaCa-2, a human pancreatic cancer cell strain endogenously containing a KRAS^{G12C}mutation, was cultured in a complete medium containing DMEM + 10% FBS + 2.5% Horse serum + 1% PS. AsPC-1, a human pancreatic cancer cell strain endogenously containing a KRAS^{G12D} mutation, was cultured in a complete medium containing RPMI-1640 + 10% FBS + 1% PS. NCI-H441 cells, a human lung cancer cell strain endogenously containing a KRAS^{G12V} mutation, were cultured in a complete medium containing RPMI-1640 + 10% FBS + 1% PS. NCI-H1975, a KRAS wild-type human lung cancer cell strain whose cell proliferation depends on KRAS activity, was cultured in a complete medium containing RPMI-1640 + 10% FBS + 1% PS.

The cells were digested with trypsin (containing EDTA), collected in their respective low-serum complete media containing 2% FBS (for MIA Paca-2 cell culture: DMEM + 2% FBS + 0.5% Horse serum + 1% PS), and counted. After the cell density was adjusted as required, MIA PaCa-2 cells (200 cells/well), AsPC-1 cells (800 cells/well), NCI-H441 cells (800 cells/well), or NCI-H1975 cells (400 cells/well) were seeded into a 384-well cell culture plate, with the volume of the medium in each well being 45 µL. The cell culture plate was incubated in an incubator at 37 °C with 5% CO₂ overnight. A series of 3.16-fold gradient dilutions of a 1000× compound stock solution were prepared with DMSO, and then diluted 100-fold with the complete medium containing 2% FBS described above to give 10× compound stock solutions. On the day after cell seeding, 5 µL of the 10× compound stock solution was added to each cell culture well. As a result, the final concentration of the compound in the cell culture well became 1×, and the DMSO content in the well was 0.1%. In addition to the compound-treated wells described above, cell-containing DMSO control wells (with 5 µL of the complete medium containing 1% DMSO added to each well) and cell-free medium control wells (containing 50 µL of the complete medium with 2% FBS) were also set. The culture plate was incubated in an incubator at 37 °C with 5% CO₂ for another 5 days, and then 30 µL of CellTiter-Glo working solution was added to each well. The mixture was well mixed, incubated for 30 min, left to stand at room temperature for 30 min, and then transferred to a white-bottomed, opaque 384-well assay plate at 40 µL/well. The chemiluminescence values (RLU) were measured using a microplate reader, and the cell proliferation inhibition rate for each well was calculated: IR(%) = (RLU_{DMSO} - RLU_{compound})/(RLU_{DMSO} - RLU_{blank}) × 100%, where RLU_{DMSO} is the average RLU signal value from the DMSO control well samples, RLU_{blank} is the average RLU signal value from the medium control well samples, and RLU_{compound} is the RLU signal value from the compound-treated well samples. Using an XLfit software, the IR(%) values were plotted against the logarithm of the compound concentrations, and the dose-response curves were fitted using the four-parameter method to calculate the IC₅₀ values.

The compounds of the present disclosure exhibited relatively high inhibitory activity against the proliferation of KRAS mutant cancer cells, where the IC₅₀ values of some of the compounds were lower than 1000 nM, and the IC₅₀ values of some of the compounds were lower than 500 nM, or even lower than 100 nM. The results for some of the exemplary compounds are shown in Tables 6 and 7 below.

**Table 6**

| Number | H441 AP IC₅₀(nM) | Number | H441 AP IC₅₀(nM) |
|---|---|---|---|
| Z2-1 | 373.66 | Z56 | 195.56 |
| Z36 | 40.61 | Z57 | 244.78 |
| Z15A | 7.63 | Z62 | 4.56 |
| Z8A | 3.98 | Z115 | 18.87 |
| Z15 | 4.31 | Z116 | 385.63 |
| Z8-1 | 62.66 | Z118 | 0.28 |
| Z8 | 2.39 | Z119 | 1.45 |
| Z44-2 | 76.28 | Z125 | 0.36 |
| Z45 | 4.74 | Z73 | 0.91 |
| Z46 | 176.11 | Z191 | 7.19 |
| Z47-2 | 37.56 | Z80 | 0.18 |
| Z48 | 122.72 | Z130 | 0.41 |
| Z51 | 3.46 | Z10 | 0.42 |
| Z52-1 | 76.46 | Z49 | 0.14 |
| Z52-2 | 25 | Z103 | 0.11 |
| Z53 | 115.3 | Z69 | 0.58 |
| Z54-1 | 239.67 | Z70 | 1.12 |
| Z75 | 1.85 | Z172 | 0.41 |
| Z229 | 0.56 | Z139 | 0.12 |
| Z145 | 0.2 | Z65 | 0.17 |
| Z66 | 0.18 | Z50 | 0.14 |
| Z104 | 0.14 | Z89 | 0.15 |

**Table 7**

| Number | AsPC-1 AP IC₅₀(nM) | Number | AsPC-1 AP IC₅₀(nM) |
|---|---|---|---|
| Z36 | 373.59 | Z125 | 0.35 |
| Z15A | 22.72 | Z73 | 1.34 |
| Z8A | 3.71 | Z80 | 0.16 |
| Z15 | 10.58 | Z130 | 0.36 |
| Z8-1 | 64.26 | Z10 | 0.56 |
| Z8 | 2.37 | Z49 | 0.08 |
| Z75 | 2.17 | Z103 | 0.05 |
| Z229 | 0.56 | Z69 | 0.67 |
| Z145 | 0.15 | Z70 | 1.54 |
| Z66 | 0.24 | Z172 | 0.4 |
| Z104 | 0.19 | Z139 | 0.1 |
| Z62 | 5.12 | Z65 | 0.1 |
| Z118 | 0.24 | Z50 | 0.19 |
| Z119 | 2.39 | Z89 | 0.11 |

Although specific embodiments of the present disclosure have been described in detail, various modifications and substitutions can be made to the details of the technical solutions of the present disclosure by those skilled in the art according to all the teachings that have been disclosed, and these changes are all encompassed within the claimed scope of the present disclosure. The entire scope of the present disclosure is given by the appended claims and any equivalents thereof.

## Claims

1. A compound represented by formula (I), or a stable deuterated derivative thereof, or a stereoisomer thereof, or a pharmaceutically acceptable salt thereof, a solvate thereof, or a prodrug thereof: 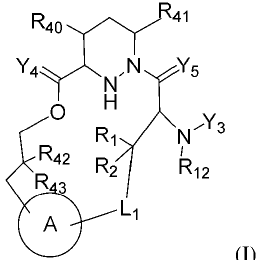 wherein,
ring A is 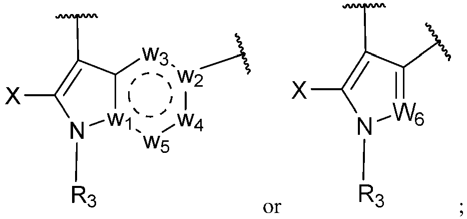 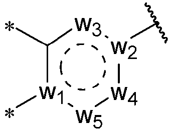 is phenyl, 5- or 6-membered heteroaryl, or 5- or 6-membered heterocyclyl; 1 or 2 ring atoms of the 5- or 6-membered heteroaryl are heteroatoms selected from nitrogen, oxygen, and sulfur; 1 or 2 ring atoms of the 5- or 6-membered heterocyclyl are heteroatoms selected from nitrogen, oxygen, and sulfur; the 5- or 6-membered heterocyclyl is fully saturated heterocyclyl or partially unsaturated heterocyclyl; or wherein, W₁ is CH, C, or N; W₂ is CH, C, or N; W₃ is CR₁₃, O, S, N, or NH; W₄ is CR₁₃, O, S, N, or NH; W₅ is absent, O, or CR₁₄; 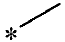 indicates a linking site to the moiety on the left;
W₆ is selected from CR₁₃ and N;
X is 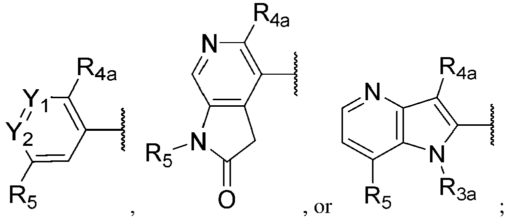
Y₁ is N or CH, wherein N can be oxidized (N⁺-O⁻);
Y₂ is N or CH, wherein N can be oxidized (N⁺-O⁻);
R₄₂ is hydrogen, halogen, or substituted or unsubstituted C₁₋₆ alkyl; R₄₃ is hydrogen, halogen, or substituted
or unsubstituted C₁₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
R₄₀ is hydrogen, halogen, or substituted or unsubstituted C₁₋₆ alkyl; R₄₁ is hydrogen, halogen, or substituted or unsubstituted C₁₆ alkyl; or
R₄₀ and R₄₁ are linked to form -CH₂- or -CH₂CH₂-, wherein the -CH₂- or -CH₂CH₂- is optionally substituted with one or more groups selected from deuterium, halogen, C₁₋₆ alkyl, C₁₋₆ haloalkyl, and deuterated C₁₋₆ alkyl;
when ring A is 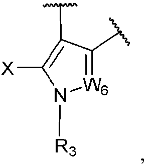
L₁ is -N(R₀₀)C(=O)-, -N(R₀₀)C(=O)-O-, -C₁₋₄ alkyl-N(R₀₀)C(=O)-, - C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, -L₂-substituted or unsubstituted 8- or 10-membered bicyclic heteroaryl-, or -L₂-substituted or unsubstituted 3- to 8-membered heterocyclyl-;
L₂ is C₃₋₆ cycloalkyl, 3- to 8-membered heterocyclyl, -O-, -C₁₋₄ alkyl-, -N(R₀₀)-, -C(=O)-, -CR₀₉=CR₁₀-, - N(R₀₀)C(=O)-, or -N(R₀₀)C(=S)-;
when ring A is 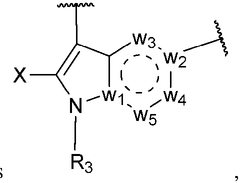 when at least one of W₁, W₂, W₃, W₄, and W₅ is a heteroatom selected from O, S, N, and NH, L₁ is - N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, -substituted or unsubstituted C₁₋₆ alkyl-O-, -substituted or unsubstituted C₂₋₆ alkenyl-O-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl-, -L₂-substituted or unsubstituted 5- or 6-membered monocyclic heteroaryl-, or -L₂-substituted or unsubstituted 3- to 8-membered heterocyclyl-;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
or,
when W₁ and W₂ are both C, and W₃, W₄, and W₅ are all CH, L₁ is -N(R₀₀)C(=O)-O-, -C₁₋₄ alkyl-N(R₀₀)C(=O)-, -N(R₀₀)C(=O)-C₁₋₄ alkyl-, -C₁₋₄ alkyl-N(R₀₀)C(=S)-, -substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkyl-NR₀₁R₀₂, -C₁₋₄ alkyl-C(R₀₅)R₀₃R₀₄, -C₁₋₄ alkyl-C(=O)-NR₀₁R₀₂, -C₁₋₄ alkyl-C(=O)-C(R₀₅)R₀₃R₀₄, -C₁₋₄ alkyl-C(=O)-C₁₋₄ alkyl-NR₀₁R₀₂, -C₁₋₄ alkyl-C(=O)-C₁₋₄ alkyl-C(R₀₅)R₀₃R₀₄, -C₁₋₄ alkyl-N(R₀₆)C(=O)-NR₀₁R₀₂, -C₁₋₄ alkyl-N(R₀₆)C(=O)-C(R₀₅)R₀₃R₀₄, -C₁₋₄ alkyl-N(R₀₆)C(=O)-C₁₋₄ alkyl-NR₀₁R₀₂, -C₁₋₄ alkyl-N(R₀₆)C(=O)-C₁₋₄ alkyl-C(R₀₅)R₀₃R₀₄, -O-C₁₋₄ alkyl-NR₀₁R₀₂, -O-C₁₋₄ alkyl-C(R₀₅)R₀₃R₀₄, -O-C₁₋₄ alkyl-N(R₀₆)C(=O)-R₀₃, -O-C(R₀₅)R₀₃R₀₄, -C₁₋₄ alkyl-NR₀₁R₀₂, -C₁₋₄ alkyl-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynyl-NR₀₁R₀₂, -C₂₋₄ alkynyl-C(=O)-NR₀₁R₀₂, -C₂₋₄ alkynyl-C(R₀₅)R₀₃R₀₄, -C₁₋₄ alkyl-C₂₋₄ alkynyl-NR₀₁R₀₂, -C₁₋₄ alkyl-C₂₋₄ alkynyl-C(R₀₅)R₀₃R₀₄, -C₂₋₄ alkynyl-C₁₋₄ alkyl-NR₀₁R₀₂, -C₂₋₄ alkynyl-C₁₋₄ alkyl-C(R₀₅)R₀₃R₀₄, -C₁₋₄ alkyl-C₂₋₄ alkynyl-C₁₋₄ alkyl-NR₀₁R₀₂, -CH=CR₀₁R₀₂, -C₂₋₄ alkenyl-NR₀₁R₀₂, -C₂₋₄ alkenyl-C(R₀₅)R₀₃R₀₄, -C₁₋₄ alkyl-C₂₋₄ alkenyl-NR₀₁R₀₂, -C₁₋₄ alkyl-C₂₋₄ alkenyl-C(R₀₅)R₀₃R₀₄, -C₂₋₄ alkenyl-C₁₋₄ alkyl-NR₀₁R₀₂, -C₂₋₄ alkenyl-C₁₋₄ alkyl-C(R₀₅)R₀₃R₀₄, -C₁₋₄ alkyl-C₂₋₄ alkenyl-C₁₋₄ alkyl-NR₀₁R₀₂, -C=CR₀₃R₀₄, -O-C₁₋₄ alkyl-substituted or unsubstituted 3- to 8-membered monocyclic heterocyclyl, -O-C₁₋₄ alkyl-substituted or unsubstituted 5- to 15-membered tricyclic heterocyclyl, -O-C₁₋₄ alkyl-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7-to 12-membered bicyclic heterocyclyl, -N=S(=O)(Rₒ₆)(-C₁₋₄ alkyl-NR₀₁R₀₂), -N(R₀₆)-S(=O)(-C₁₋₄ alkyl-NR₀₁R₀₂), -S(=O)(R₀₆)(=N-R₀₆), -S(=O)(-C₁₋₄ alkyl-NR₀₁R₀₂)(=N-R₀₆), -S(=O)₂(-C₁₋₄ alkyl-NR₀₁R₀₂), - S(=O)(-C₁₋₄ alkyl-NR₀₁R₀₂), -S(=O)₂(-C₁₋₄ alkyl-C(R₀₅)R₀₃R₀₄), -S(=O)(-C₁₋₄ alkyl-C(R₀₅)R₀₃R₀₄), -C₂₋₄ alkynyl-substituted or unsubstituted 5- or 6-membered monocyclic heteroaryl, -C₂₋₄ alkynyl-substituted or unsubstituted 8- to 10-membered bicyclic heteroaryl, -C₂₋₄ alkynyl-substituted or unsubstituted 6- to 12-membered heteroaryl-fused heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, substituted or unsubstituted C₆₋₁₀ aryl-substituted or unsubstituted 3- to 8-membered monocyclic heterocyclyl, substituted or unsubstituted 5- or 6-membered monocyclic heteroaryl-substituted or unsubstituted 3- to 8-membered monocyclic heterocyclyl, substituted or unsubstituted C₆₋₁₀ aryl-C₁₋₄ alkyl-substituted or unsubstituted 3- to 8-membered monocyclic heterocyclyl, substituted or unsubstituted 5- or 6-membered monocyclic heteroaryl-C₁₋₄ alkyl-substituted or unsubstituted 3- to 8-membered monocyclic heterocyclyl, substituted or unsubstituted C₆₋₁₀ aryl-fused substituted or unsubstituted 3- to 8-membered monocyclic heterocyclyl, substituted or unsubstituted 5- or 6-membered monocyclic heteroaryl-fused substituted or unsubstituted 3- to 8-membered monocyclic heterocyclyl, substituted or unsubstituted C₆₋₁₀ aryl-C₁₋₄ alkyl-NR₀₁R₀₂, or substituted or unsubstituted 5- or 6-membered monocyclic heteroaryl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein 2 hydrogen atoms on the same carbon atom of the C₁₋₆ alkyl or C₁₋₆ alkoxy are optionally both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted substituted or unsubstituted 5- to 15-membered tricyclic heterocyclyl, 7- to 12-membered bicyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₇), -P(=O)(R₀₇)(R₀₈), - C(=O)-C₁₋₄ alkyl-NR₆-S(=O)(R₀₇)(=N-R₀₈), -C(=O)-C₁₋₄ alkyl-N=S(=O)(R₀₇)(R₀₈), or R₁₁; R₁₂ is hydrogen, C₁₋₆ alkyl, or deuterated C₁₋₆ alkyl; or,
Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6-to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the following group consisting of: deuterium, C₁₋₆ alkyl, C₁₋₆ heteroalkyl, and 5- or 6-membered monocyclic heteroaryl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
Y₅ is O or S;
Y₄ is O or S;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₃ is hydrogen, halogen, substituted or unsubstituted C₁₋₆ alkyl, or substituted or unsubstituted C₁₋₆ alkoxy; R₁₄ is hydrogen, halogen, or substituted or unsubstituted C₁₋₆ alkyl; or
in W₄ and W₅, R₁₃ and R₁₄, together with the carbon atom to which they are attached, form substituted or unsubstituted C₃₋₆ cycloalkyl, substituted or unsubstituted C₆₋₁₀ aryl, substituted or unsubstituted 5- or 6-membered monocyclic heteroaryl, or substituted or unsubstituted 3- to 8-membered heterocyclyl; or
in W₅, R₁₄ and R₃, together with the atom to which they are attached, form substituted or unsubstituted nitrogen-containing 4- to 8-membered monocyclic heterocyclyl; in the nitrogen-containing 4- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus; or,
in W₆, R₁₃ and R₃, together with the atom to which they are attached, form substituted or unsubstituted nitrogen-containing 3- to 8-membered monocyclic heterocyclyl or substituted or unsubstituted 5- or 6-membered monocyclic heteroaryl; in the nitrogen-containing 3- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus;
in the groups described above, the substitutions each independently refer to that the groups described above are each independently substituted with 1, 2, 3, 4, 5, or 6 groups selected from group S1; the groups of group S1 are each independently selected from the following group consisting of: -SF₅, deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8 to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂; wherein, the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl; wherein, the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or,
each R^{a1} and R^{b1}, together with the nitrogen atom to which they are attached, form 3- to 20-membered heterocyclyl; wherein, each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, -C₁₋₄ alkyl-hydroxy, -C₁₋₄ alkyl-NR^{a1}R^{b1}, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, - C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₃₋₆ monocyclic cycloalkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, -SF₅, and -C₁₋₄ alkyl-hydroxy;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, phenyl, and C₃₋₆ monocyclic cycloalkyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein, when the ring atom is a sulfur atom, the sulfur atom is optionally substituted with one or two groups selected from oxo and =NR^{e}; R^{e} is as defined above;
wherein, when the ring atom is a phosphorus atom, the phosphorus atom is optionally substituted with one or two groups selected from oxo and =NR^{e}; R^{e} is as defined above.

2. The compound, or the stable deuterated derivative thereof, or the stereoisomer thereof, or the pharmaceutically acceptable salt thereof, the solvate thereof, or the prodrug thereof according to claim 1,
wherein, 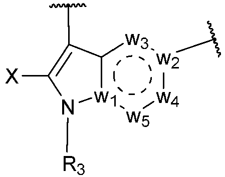 is selected from the following group consisting of: 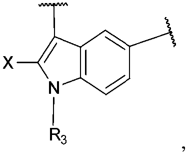 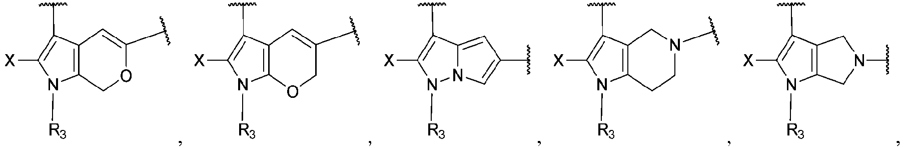
and 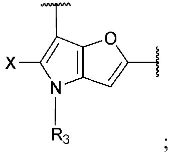 wherein, X is as defined above;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl.

3. The compound, or the stable deuterated derivative thereof, or the stereoisomer thereof, or the pharmaceutically acceptable salt thereof, the solvate thereof, or the prodrug thereof according to claim 1,
wherein, 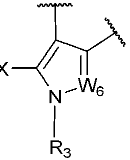 is selected from 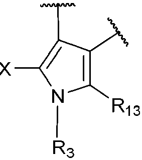 and 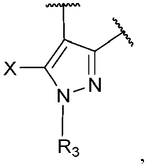 wherein, X is as defined above;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl; R₁₃ is hydrogen, halogen, substituted or unsubstituted C₁₋₆ alkoxy, or substituted or unsubstituted C₁₋₆ alkyl; or,
R₁₃ and R₃, together with the atom to which they are attached, form substituted or unsubstituted nitrogen-containing 3- to 8-membered monocyclic heterocyclyl or substituted or unsubstituted 5- or 6-membered monocyclic heteroaryl; in the nitrogen-containing 3- to 8-membered monocyclic heterocyclyl, one ring atom is a nitrogen atom, and optionally one or more (e.g., 1, 2, or 3) ring atoms are heteroatoms selected from nitrogen, oxygen, sulfur, and phosphorus.

4. The compound, or the stable deuterated derivative thereof, or the stereoisomer thereof, or the pharmaceutically acceptable salt thereof, the solvate thereof, or the prodrug thereof according to claim 1, wherein, the compound of formula (I) is represented by formula (A1), formula (A2), formula (A3), formula (A4), formula (A5), formula (A6), formula (B1), formula (B2), formula (B3), or formula (B4): 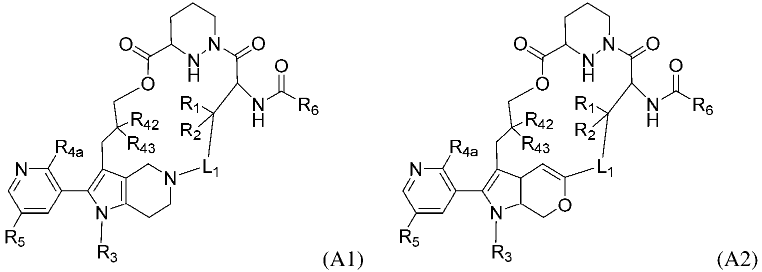 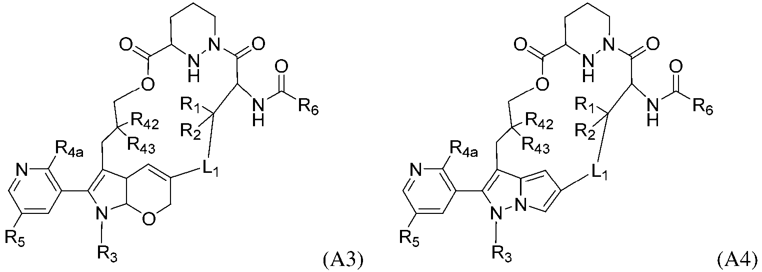 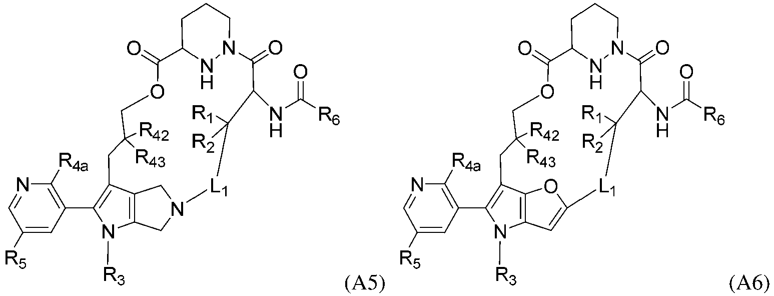 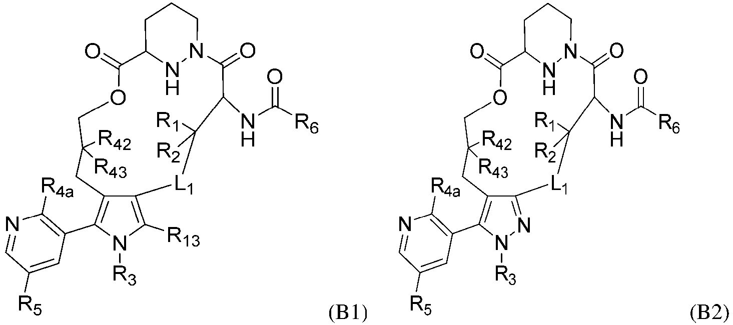 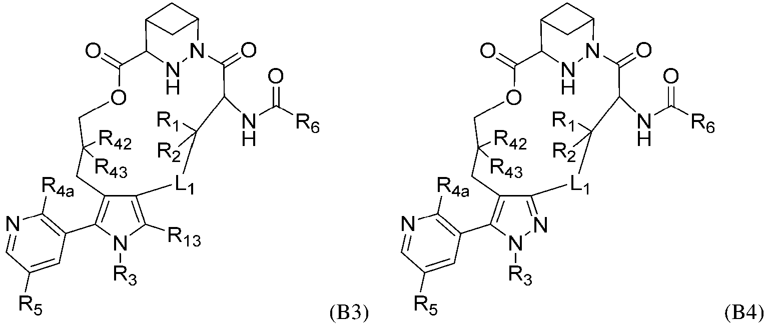 wherein,
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₃ is hydrogen, halogen, substituted or unsubstituted C₁₋₆ alkyl, or substituted or unsubstituted C₁₋₆ alkoxy; or
R₁₃ and R₃, together with the atom to which they are attached, form substituted or unsubstituted nitrogen-containing 3- to 8-membered monocyclic heterocyclyl;
R₄₂ is hydrogen, halogen, or substituted or unsubstituted C₁₋₆ alkyl; R₄₃ is hydrogen, halogen, or substituted or unsubstituted C₁₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, -N(R₀₀)C(=O)-O-, -C₁₋₄ alkyl-N(R₀₀)C(=O)-, -C(=O)N(R₀₀)-C₁₋₄ alkyl-, -C₁₋₄ alkyl-N(R₀₀)C(=S)-, substituted or unsubstituted C₁₋₆ alkyl, -substituted or unsubstituted C₁₋₆ alkyl-O-, -substituted or unsubstituted C₂₋₆ alkenyl-O-substituted or unsubstituted C₃₋₆ cycloalkyl, substituted or unsubstituted 3-to 8-membered heterocyclyl, substituted or unsubstituted C₆₋₁₀ aryl, substituted or unsubstituted 5- or 6-membered monocyclic heteroaryl, substituted or unsubstituted 8- to 10-membered bicyclic heteroaryl, -L₂-substituted or unsubstituted C₆₋₁₀ aryl, -L₂-substituted or unsubstituted 5- or 6-membered monocyclic heteroaryl, or -L₂-substituted or unsubstituted 3- to 8-membered heterocyclyl;
L₂ is -O-, -C₁₋₄ alkyl-, -N(R₀₀)-, -C(=O)-, -CR₀₉=CR₁₀-, -N(R₀₀)C(=O)-, or -N(R₀₀)C(=S)-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is -NR₀₁R₀₂ or -C₂₋₄ alkynyl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₀₉ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₁₀ is hydrogen, deuterium, halogen, or substituted or unsubstituted C₁₋₆ alkyl; the substitution refers to substitution with one or more (e.g., 1, 2, 3, or 4) groups selected from the following group consisting of: deuterium and halogen;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
in the groups described above, the substitutions each independently refer to that the groups described above are each independently substituted with 1, 2, 3, 4, 5, or 6 groups selected from group S1; the groups of group S1 are each independently selected from the following group consisting of: -SF₅, deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆₋₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂; wherein, the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl; wherein the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl; wherein, each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus;
wherein, when the ring atom is a sulfur atom, the sulfur atom is optionally substituted with one or two groups selected from oxo and =NR^{e}; R^{e} is as defined above;
wherein, when the ring atom is a phosphorus atom, the phosphorus atom is optionally substituted with one or two groups selected from oxo and =NR^{e}; R^{e} is as defined above.

5. The compound, or the stable deuterated derivative thereof, or the stereoisomer thereof, or the pharmaceutically acceptable salt thereof, the solvate thereof, or the prodrug thereof according to claim 1, wherein, the compound of formula (I) is represented by formula (C1): 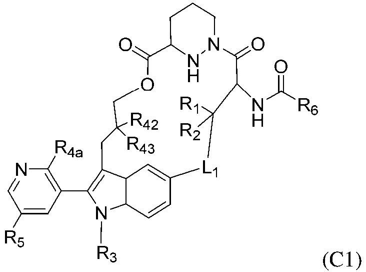
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-O-, -C_{1-A} alkyl-N(R₀₀)C(=O)-, -C(=O)N(R₀₀)-C₁₋₄ alkyl-, -C_{1-A} alkyl-N(R₀₀)C(=S)-, - substituted or unsubstituted C₁₋₆ alkyl-O-, or -substituted or unsubstituted C₂₋₆ alkenyl-O-;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is -NR₀₁R₀₂ or -C₂₋₄ alkynyl-C₁₋₄ alkyl-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted C₃₋₆ cycloalkyl, substituted or unsubstituted C₂₋₆ alkenyl, and substituted or unsubstituted C₂₋₆ alkynyl; or,
R₀₁ and R₀₂, together with the nitrogen atom to which they are attached, form substituted or unsubstituted 5-to 15-membered tricyclic heterocyclyl, substituted or unsubstituted 7- to 12-membered bicyclic heterocyclyl, or substituted or unsubstituted 3- to 8-membered monocyclic heterocyclyl;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
in the groups described above, the substitutions each independently refer to that the groups described above are each independently substituted with 1, 2, 3, 4, 5, or 6 groups selected from group S1; the groups of group S1 are each independently selected from the following group consisting of: -SF₅, deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkyl-C₃₋₂₀ cycloalkyl, -C≡C-C₁₋₄ alkyl-3- to 20-membered heterocyclyl, -C≡C-C₁₋₄ alkyl-C₆₋₁₄ aryl, -C≡C-C₁₋₄ alkyl-5- or 6-membered monocyclic heteroaryl, -C≡C-C₁₋₄ alkyl-8- to 10-membered bicyclic heteroaryl, -C₁₋₄ alkyl-hydroxy, -C₁₋₄ alkyl-cyano, - C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -O-C₁₋₄ alkyl-hydroxy, -O-C₁₋₄ alkyl-cyano, -O-C₁₋₄ alkyl-C₁₋₆ alkyl, -O-C₁₋₄ alkyl-C₁₋₆ alkoxy, -O-C₁₋₄ alkyl-C₃₋₂₀ cycloalkyl, -O-C₁₋₄ alkyl-O-C₃₋₂₀ cycloalkyl, -O-C₁₋₄ alkyl-3- to 20-membered heterocyclyl, -O-C₁₋₄ alkyl-O-3- to 20-membered heterocyclyl, -O-C₁₋₄ alkyl-C₆₋₁₄ aryl, -O-C₁₋₄ alkyl-O-C₆-₁₄ aryl, -O-C₁₋₄ alkyl-5- or 6-membered monocyclic heteroaryl, -O-C₁₋₄ alkyl-O-5- or 6-membered monocyclic heteroaryl, -O-C₁₋₄ alkyl-8- to 10-membered bicyclic heteroaryl, -O-C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, - C(=O)-C₆₋₁₄ aryl, -NR^{a1}R^{b1}, -C(=O)-NR^{a1}R^{b1}, -C(=O)-NR^{d1}-C₁₋₄ alkyl-R^{c1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, - C(=O)-C₁₋₆ alkyl-8- to 10-membered bicyclic heteroaryl, -C₁₋₄ alkyl-NR^{a1}R^{b1}, -C₁₋₄ alkyl-C(=O)-NR^{a1}R^{b1}, - C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, - P(=O)-(C₁₋₆ alkyl)₂, -C₁₋₄ alkyl-NR^{d1}-C(=O)-R^{c1}, -C₁₋₄ alkyl-NR^{dl1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-C₁₋₄ alkyl-R^{c1}, -NR^{d1}-C(=O)-NR^{a1}R^{b1}, -NR^{d1}-S(=O)₂-R^{c1}, -S(=O)₂-NR^{a1}R^{e1}, -NR^{d1}-S(=O)₂-NR^{a1}R^{b1}, and -P(=O)-(C₁₋₆ alkyl)₂; wherein, the C₁₋₆ alkyl, the C₁₋₆ alkoxy, the C₂₋₆ alkenyl, and the C₂₋₆ alkynyl are each independently and optionally substituted with 1, 2, or 3 groups selected from halogen, deuterium, cyano, and hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently and optionally substituted with 1, 2, 3, or 4 groups selected from group S2;
in the groups described above, each R^{a1} and R^{b1} are each independently H, C₁₋₆ alkyl, C₁₋₆ haloalkyl, deuterated C₁₋₆ alkyl, -C₁₋₄ alkyl-hydroxy, -C₁₋₄ alkyl-cyano, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3-to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, - S(=O)₂-3- to 6-membered monocyclic heterocyclyl, -C₁₋₄ alkyl-S(=O)₂-C₁₋₆ alkyl, -C₁₋₄ alkyl-S(=O)₂-C₃₋₆ monocyclic cycloalkyl, -C₁₋₄ alkyl-S(=O)₂-3- to 6-membered monocyclic heterocyclyl, -C(=O)-C₁₋₆ alkyl, - C(=O)-C₃₋₆ monocyclic cycloalkyl, or -C(=O)-3- to 6-membered monocyclic heterocyclyl; wherein, the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkyl-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl; wherein, each 3- to 20-membered heterocyclyl is independently and optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkyl-C₁₋₆ haloalkyl, -C₁₋₄ alkyl-deuterated C₁₋₆ alkyl, -C₁₋₄ alkyl-C₁₋₆ alkoxy, -C₁₋₄ alkyl-C₁₋₆ haloalkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3-to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or - C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, or the 8- to 10-membered bicyclic heteroaryl is optionally substituted with 1 or 2 groups selected from the following group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, and -P(=O)-(C₁₋₆ alkyl)₂;
in the groups described above, the groups of group S2 are each independently selected from the following group consisting of: deuterium, oxo (=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkyl-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkyl- is unsubstituted; or 1, 2, 3, or 4 hydrogen atoms on the -C₁₋₄ alkyl- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the C₁₋₄ alkyl are both substituted with -(CH₂)ⱼ- to form cycloalkyl, wherein j is 2, 3, 4, 5, or 6; or 2 hydrogen atoms on the same carbon atom of the C₁₋₄ alkyl are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) ring atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus;
wherein, when the ring atom is a sulfur atom, the sulfur atom is optionally substituted with one or two groups selected from oxo and =NR^{e}; R^{e} is as defined above;
wherein, when the ring atom is a phosphorus atom, the phosphorus atom is optionally substituted with one or two groups selected from oxo and =NR^{e}; R^{e} is as defined above.

6. The compound, or the stable deuterated derivative thereof, or the stereoisomer thereof, or the pharmaceutically acceptable salt thereof, the solvate thereof, or the prodrug thereof according to claim 1, wherein, R₄ₐ is -C₁₋₆ alkyl-C₁₋₆ alkoxy.

7. The compound, or the stable deuterated derivative thereof, or the stereoisomer thereof, or the pharmaceutically acceptable salt thereof, the solvate thereof, or the prodrug thereof according to claim 1, wherein, R₅ is selected from hydrogen

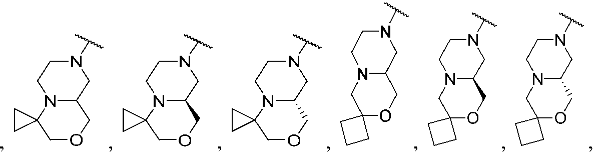

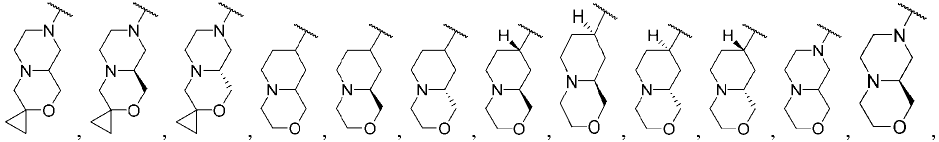

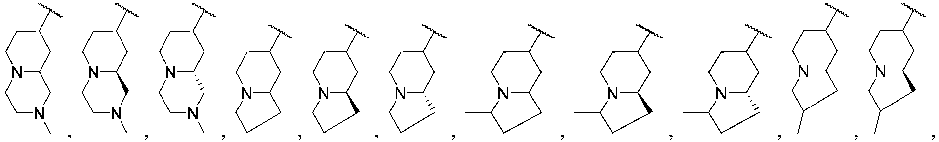

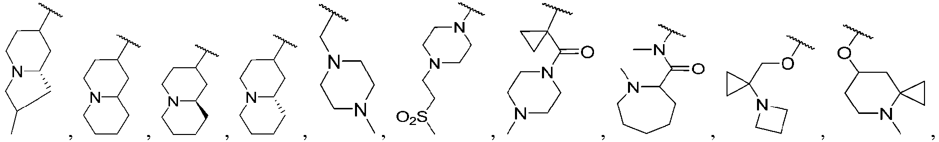

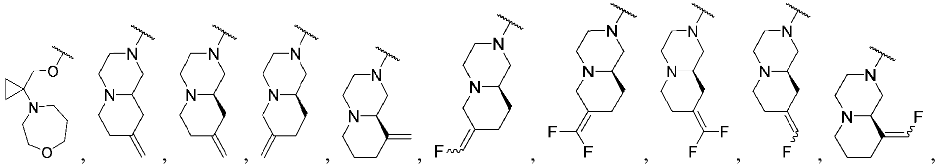

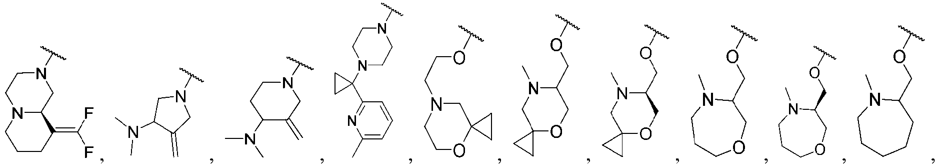

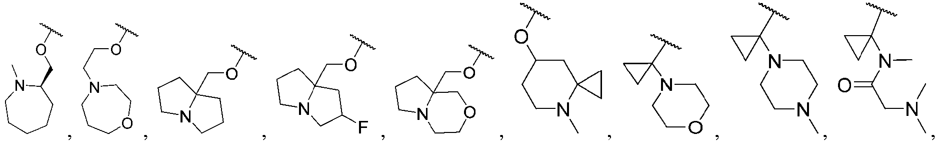

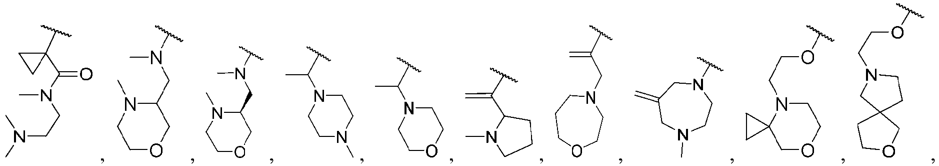

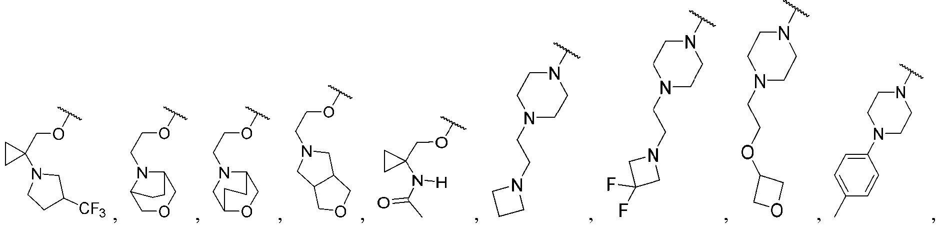

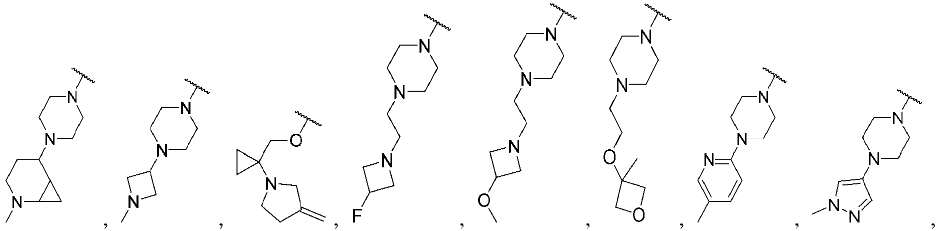

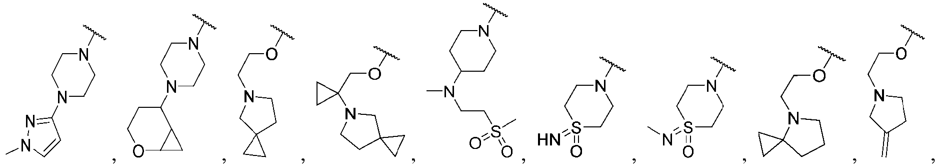

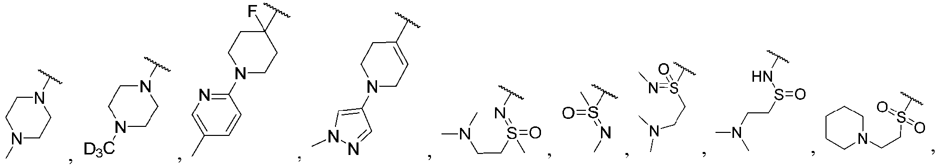

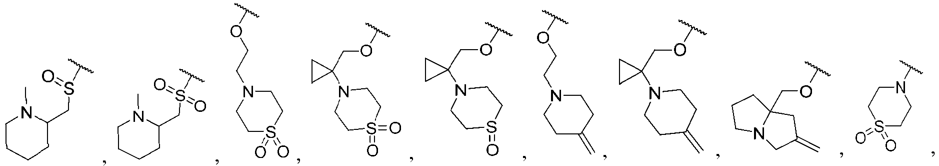

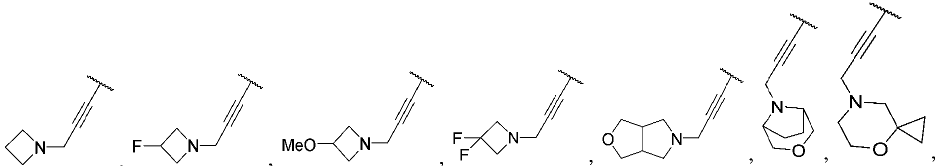

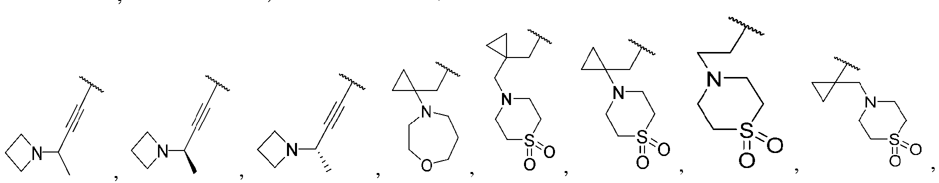

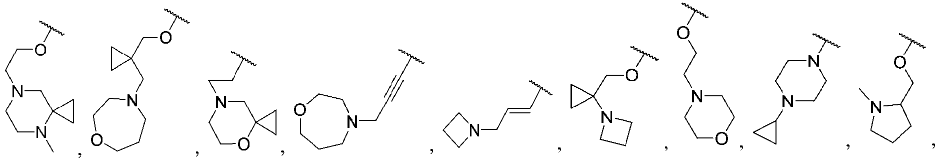

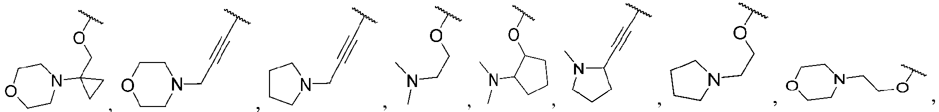

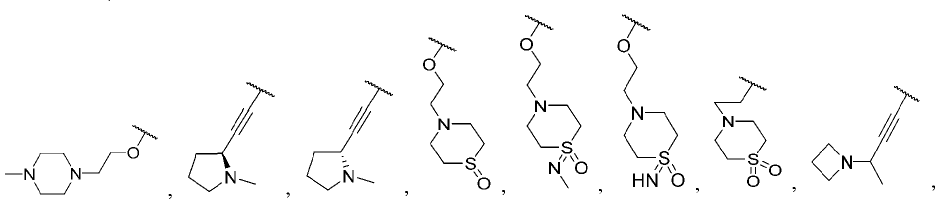

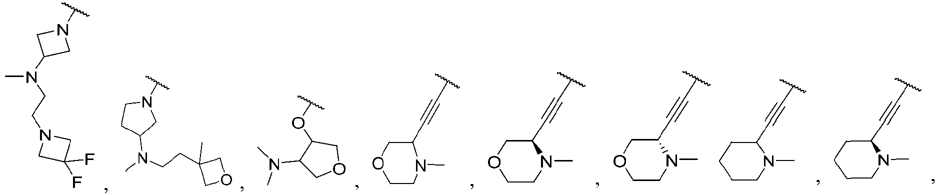

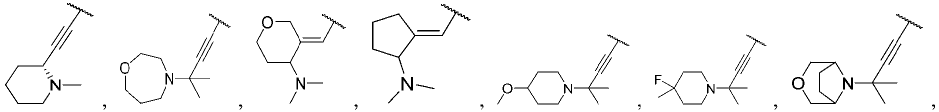

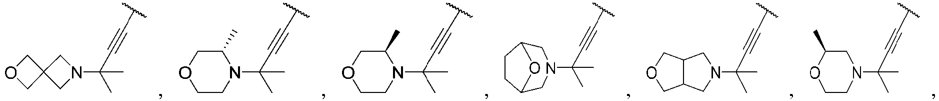

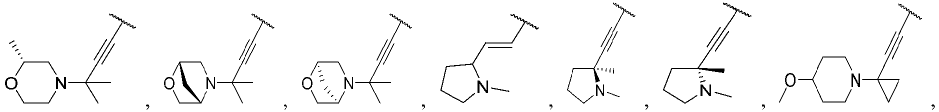

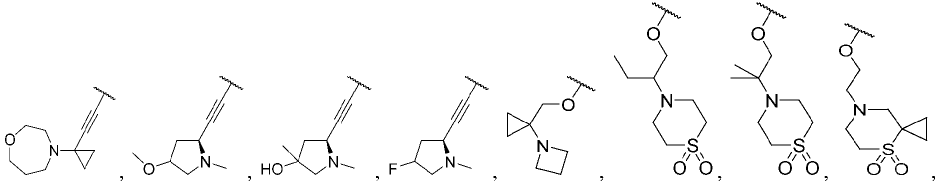

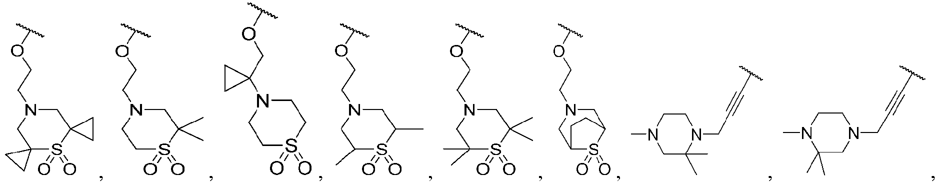

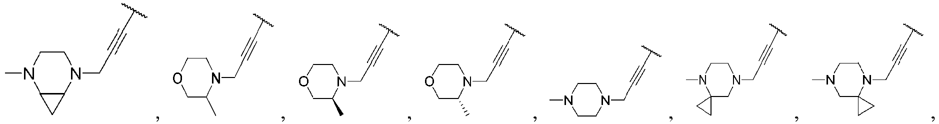

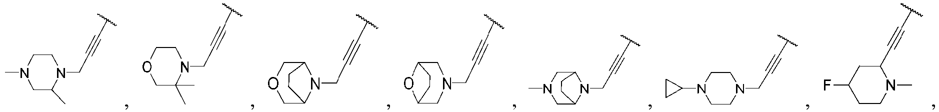

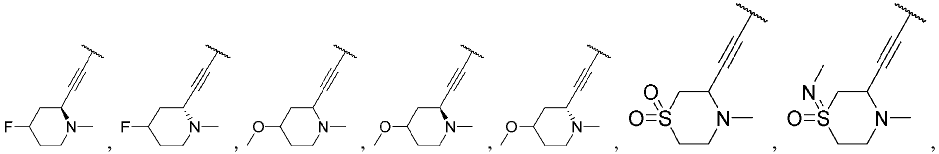

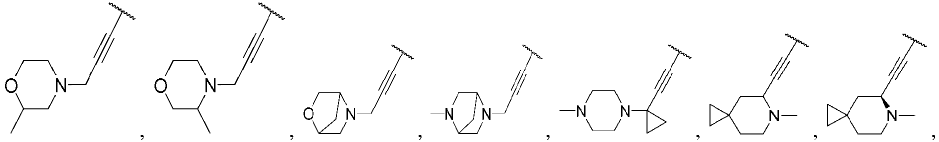

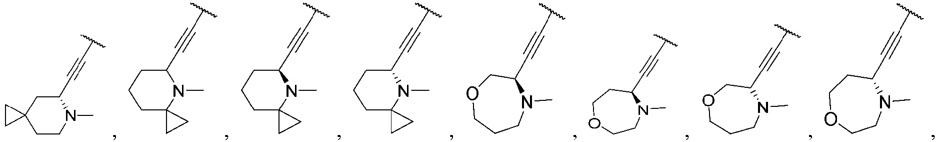

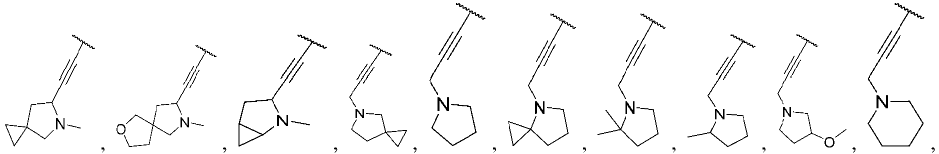

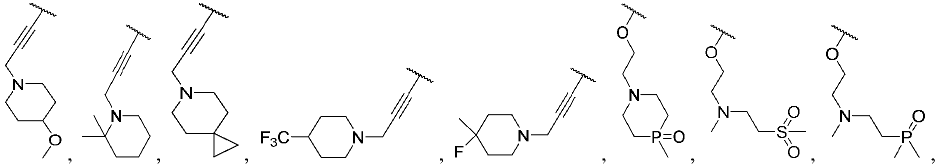

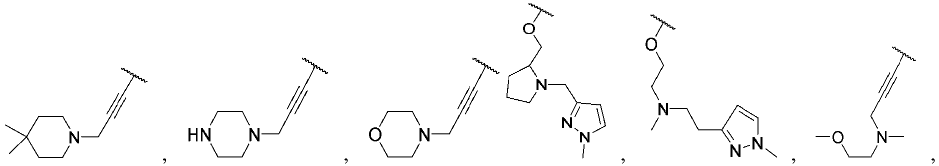

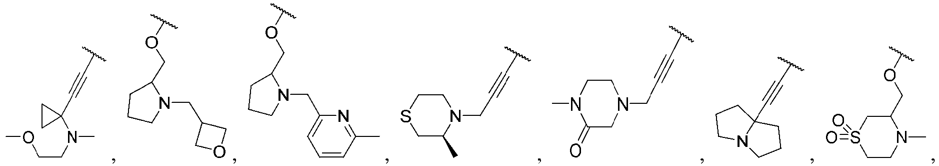

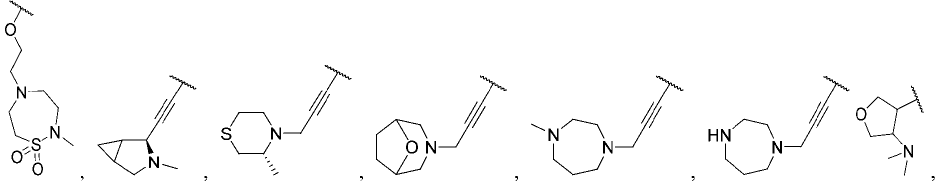

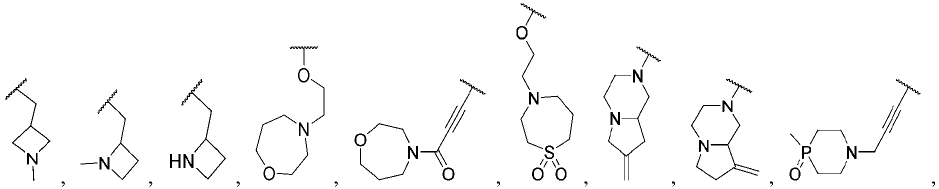

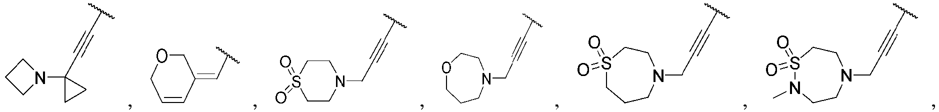

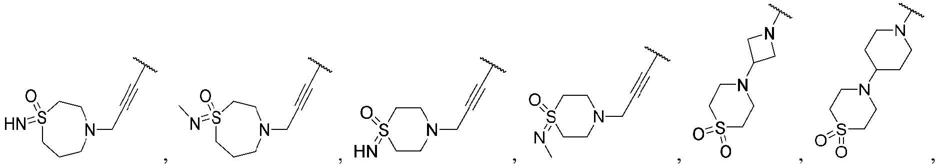

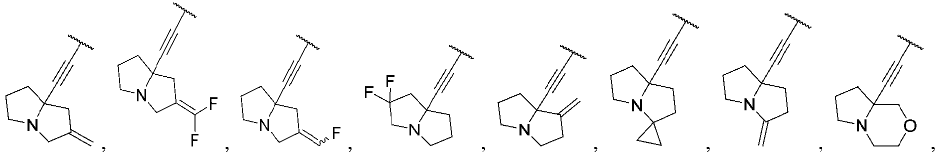

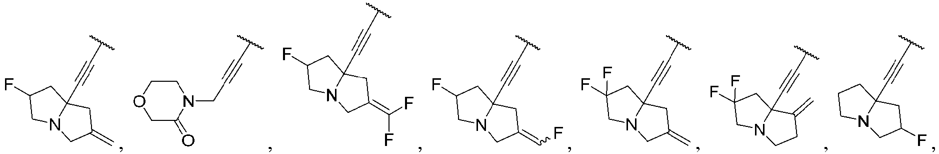

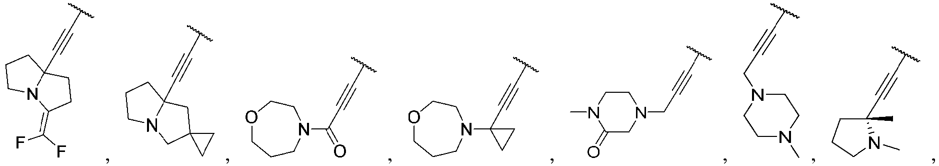

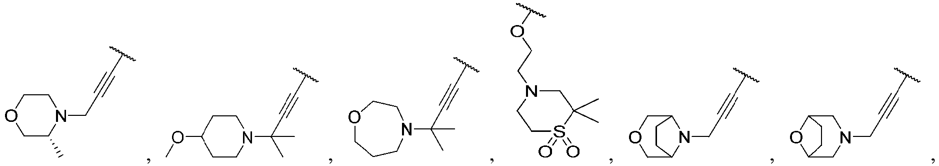

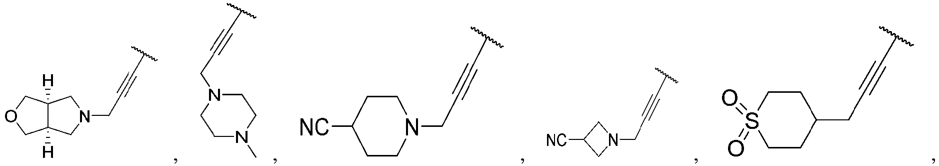

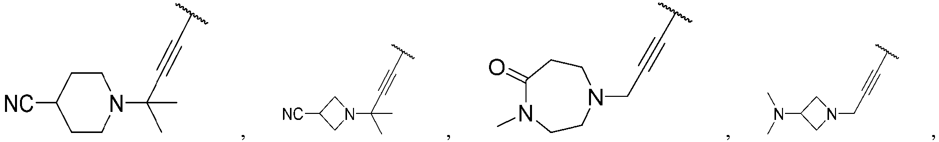

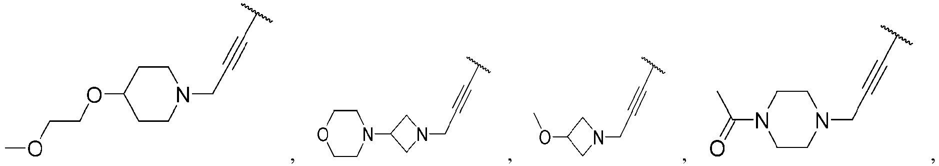

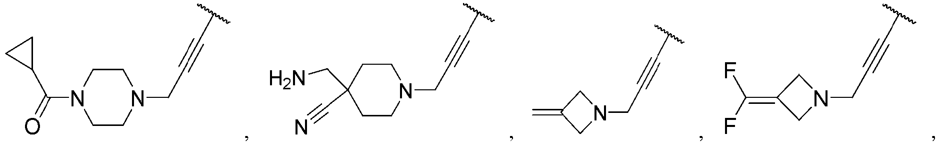

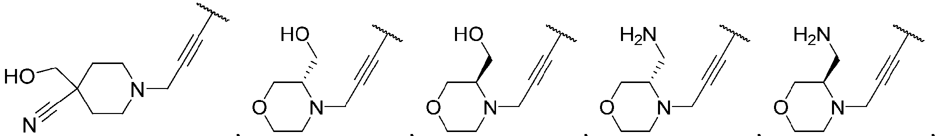

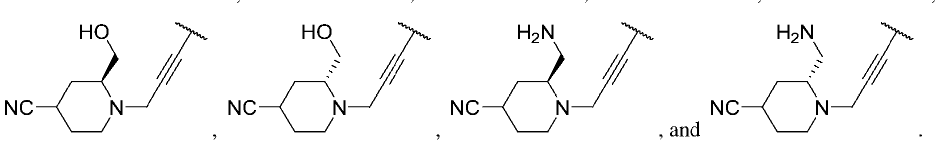

8. The compound, or the stable deuterated derivative thereof, or the stereoisomer thereof, or the pharmaceutically acceptable salt thereof, the solvate thereof, or the prodrug thereof according to claim 1, wherein, R₃ is hydrogen, C₁₋₆ alkyl, C₁₋₆ haloalkyl, deuterated C₁₋₆ alkyl, or C₃₋₆ cycloalkyl.

9. The compound, or the stable deuterated derivative thereof, or the stereoisomer thereof, or the pharmaceutically acceptable salt thereof, the solvate thereof, or the prodrug thereof according to claim 1, wherein, R₁ is hydrogen; R₂ is hydrogen.

10. The compound, or the stable deuterated derivative thereof, or the stereoisomer thereof, or the pharmaceutically acceptable salt thereof, the solvate thereof, or the prodrug thereof according to claim 1, wherein, L₁ is selected from

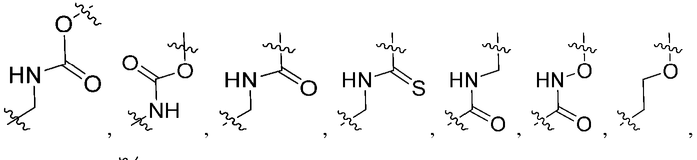

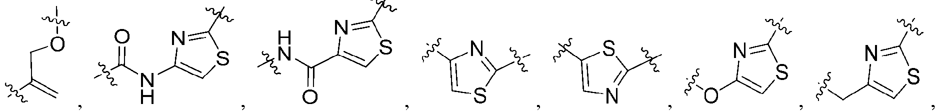

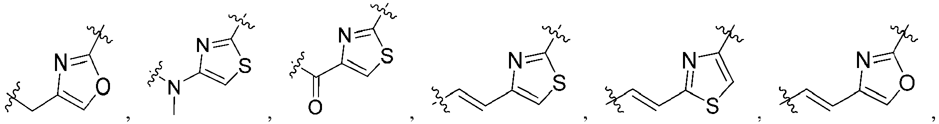

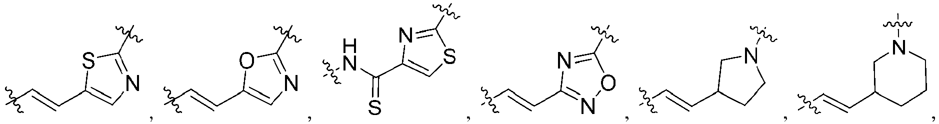

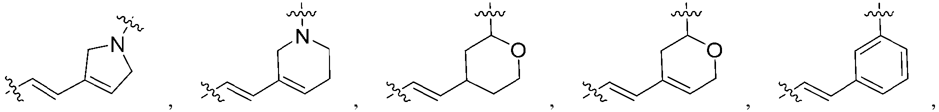

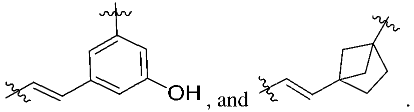

11. The compound, or the stable deuterated derivative thereof, or the stereoisomer thereof, or the pharmaceutically acceptable salt thereof, the solvate thereof, or the prodrug thereof according to claim 1, wherein, R₆ is selected from

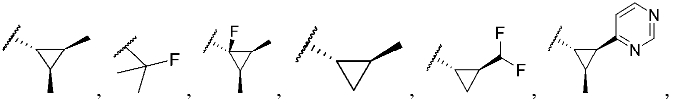

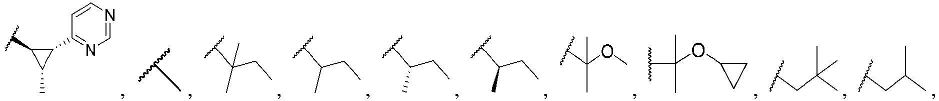

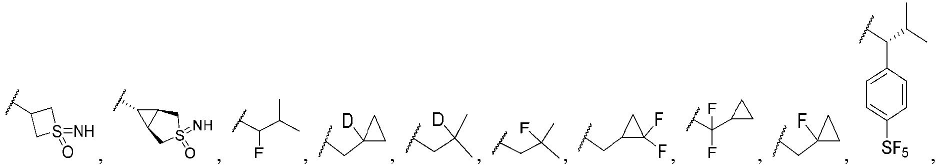

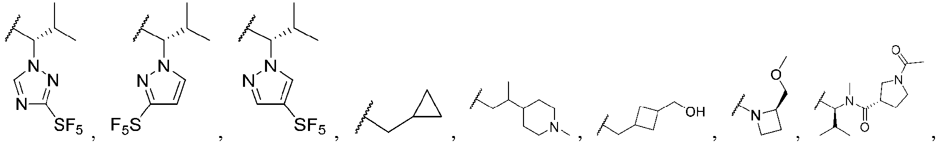

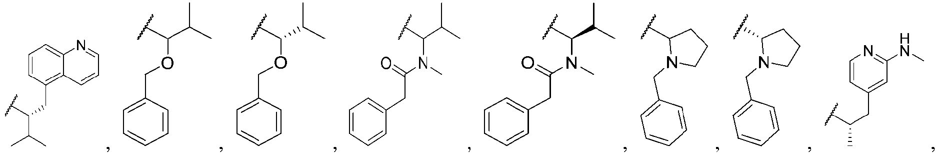

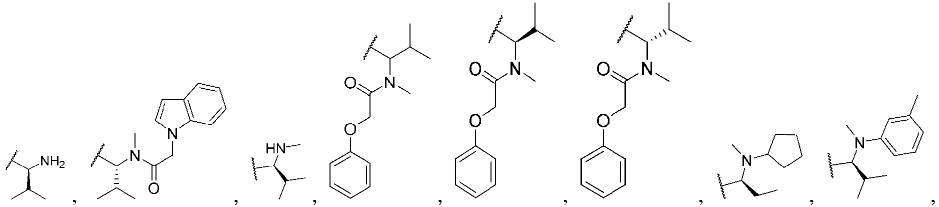

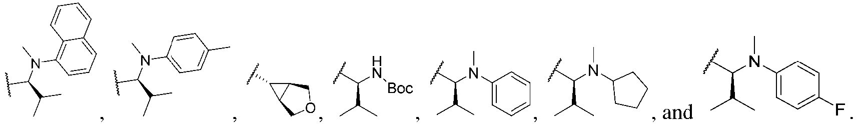

12. The compound, or the stable deuterated derivative thereof, or the stereoisomer thereof, or the pharmaceutically acceptable salt thereof, the solvate thereof, or the prodrug thereof according to claim 1, wherein, the compound is selected from Table A, Table B, and Table C.

13. A pharmaceutical composition, comprising the compound, or the stable deuterated derivative thereof, or the stereoisomer thereof, or the pharmaceutically acceptable salt thereof, the solvate thereof, or the prodrug thereof according to any one of claims 1 to 12, and a pharmaceutically acceptable carrier.

14. Use of the compound, or the stable deuterated derivative thereof, or the stereoisomer thereof, or the pharmaceutically acceptable salt thereof, the solvate thereof, or the prodrug thereof according to any one of claims 1 to 12, or the pharmaceutical composition according to claim 13 in the manufacture of a medicament for preventing and/or treating a disease or disorder associated with RAS protein activity.

15. The use according to claim 14, wherein, the disease or disorder associated with RAS protein activity is a cancer.

16. The use according to claim 15, wherein, the cancer is pancreatic cancer, colorectal cancer, non-small cell lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer.
